# EUROPEAN PATENT SPECIFICATION

(11) **EP 2 758 535 B1**
(45) Date of publication and mention of the grant of the patent: **09.11.2016**
(21) Application number: 12772122.3
(22) Date of filing: 18.09.2012
(51) Int. Cl.: C12N 15/85, A01K 67/027, C07K 16/46

(54) **ANTIBODIES, VARIABLE DOMAINS&CHAINS TAILORED FOR HUMAN USE**
MASSGESCHNEIDERTE ANTIKÖRPER, VARIABLE DOMÄNEN UND KETTEN FÜR MENSCHLICHE VERWENDUNG
ANTICORPS, DOMAINES VARIABLES&CHAÎNES ADAPTÉES POUR UNE UTILISATION HUMAINE

(30) Priority: 19.09.2011 GB 201116122; 19.09.2011 GB 201116120; 24.02.2012 GB 201203257; 15.03.2012 GB 201204592; 29.03.2012 GB 201205702; 18.05.2012 GB 201208749; 02.07.2012 GB 201211692
(43) Date of publication of application: 30.07.2014
(60) Divisional application: 16189625.3
(73) Proprietor: Kymab Limited, Cambridge CB22 3AT (GB)
(72) Inventor: BRADLEY, Allan, Cambridge, Cambridgeshire CB22 3AT (GB); FRIEDRICH, Glenn, Cambridge, Cambridgeshire CB22 3AT (GB); LEE, E-Chiang, Cambridge Cambridgeshire CB22 3AT (GB); STRIVENS, Mark, Cambridge, Cambridgeshire CB22 3AT (GB); ENGLAND, Nicholas, Cambridge, Cambridgeshire CB22 3AT (GB)
(74) Representative: Hollywood, Jane Constance
(86) International application number: PCT/GB2012/052296
(87) International publication number: WO 2013/041844

(56) References cited:
- WO-A1-02/066630
- WO-A1-2011/004192
- WO-A1-2012/141798
- WO-A2-02/053596
- WO-A2-2004/050838
- WO-A2-2008/054606
- WO-A2-2009/097006
- US-A1- 2006 021 074
- US-B1- 6 914 128
- US-B2- 6 596 541
- KRAUSE JENS C ET AL: "Epitope-Specific Human Influenza Antibody Repertoires Diversify by B Cell Intraclonal Sequence Divergence and Interclonal Convergence", JOURNAL OF IMMUNOLOGY, vol. 187, no. 7, 31 August 2011 (2011-08-31), pages 3704-3711, XP002689676, ISSN: 0022-1767
- BRUEGGEMANN M ET AL: "HUMAN ANTIBODY PRODUCTION IN TRANSGENIC MICE: EXPRESSION FROM 100 KB OF THE HUMAN IGH LOCUS", EUROPEAN JOURNAL OF IMMUNOLOGY, WILEY - V C H VERLAG GMBH & CO. KGAA, DE, vol. 21, 1 May 1991 (1991-05-01), pages 1323-1326, XP000944561, ISSN: 0014-2980, DOI: 10.1002/EJI.1830210535
- MENDEZ M J ET AL: "FUNCTIONAL TRANSPLANT OF MEGABASE HUMAN IMMUNOGLOBULIN LOCI RECAPITULATES HUMAN ANTIBODY RESPONSE IN MICE", NATURE GENETICS, NATURE PUBLISHING GROUP, NEW YORK, US, vol. 15, no. 2, 1 February 1997 (1997-02-01), pages 146-156, XP002067603, ISSN: 1061-4036, DOI: 10.1038/NG0297-146
- GREEN L L: "Antibody engineering via genetic engineering of the mouse: XenoMouse strains are a vehicle for the facile generation of therapeutic human monoclonal antibodies", JOURNAL OF IMMUNOLOGICAL METHODS, ELSEVIER SCIENCE PUBLISHERS B.V.,AMSTERDAM, NL, vol. 231, no. 1-2, 10 December 1999 (1999-12-10), pages 11-23, XP004187631, ISSN: 0022-1759, DOI: 10.1016/S0022-1759(99)00137-4
- RICHARD M. DURBIN ET AL: "A map of human genome variation from population-scale sequencing", NATURE, vol. 467, no. 7319, 28 October 2010 (2010-10-28), pages 1061-1073, XP055048531, ISSN: 0028-0836, DOI: 10.1038/nature09534
- MIR KALIM U: "Sequencing Genomes: From Individuals to Populations", BRIEFINGS IN FUNCTIONAL GENOMICS & PROTEOMICS, vol. 8, no. 5, September 2009 (2009-09), pages 367-378, XP002689677, ISSN: 1473-9550
- MILNER E C B ET AL: "Polymorphism and utilization of human V(H) genes", ANNALS OF THE NEW YORK ACADEMY OF SCIENCES 1995 US, vol. 764, 1995, pages 50-61, XP002696241, ISSN: 0077-8923
- PRAMANIK SREEMANTA ET AL: "Segmental duplication as one of the driving forces underlying the diversity of the human immunoglobulin heavy chain variable gene region", BMC GENOMICS, BIOMED CENTRAL LTD, LONDON, UK, vol. 12, no. 1, 27 January 2011 (2011-01-27), page 78, XP021086493, ISSN: 1471-2164, DOI: 10.1186/1471-2164-12-78
- LI HONGHUA ET AL: "Genetic diversity of the human immunoglobulin heavy chain VH region.", IMMUNOLOGICAL REVIEWS, vol. 190, December 2002 (2002-12), pages 53-68, XP002696242, ISSN: 0105-2896
- TOMIZUKA K ET AL: "Double trans-chromosomic mice: maintenance of two individual human chromosome fragments containing Ig heavy and k loci and expression of fully human antibodies", PROCEEDINGS OF THE NATIONAL ACADEMY OF SCIENCES, NATIONAL ACADEMY OF SCIENCES, US, vol. 97, no. 2, 18 January 2000 (2000-01-18), pages 722-727, XP002150871, ISSN: 0027-8424, DOI: 10.1073/PNAS.97.2.722
- LONBERG NILS: "Human antibodies from transgenic animals", NATURE BIOTECHNOLOGY, NATURE PUBLISHING GROUP, NEW YORK, NY, US, vol. 23, no. 9, 1 September 2005 (2005-09-01), pages 1117-1125, XP002442149, ISSN: 1087-0156, DOI: 10.1038/NBT1135 cited in the application
- DING LING ET AL: "Generation of High-Affinity Fully Human Anti-Interleukin-8 Antibodies from its cDNA by Two-Hybrid Screening and Affinity Maturation in Yeast", PROTEIN SCIENCE, vol. 19, no. 10, October 2010 (2010-10), pages 1957-1966, XP002696243, ISSN: 0961-8368
- OHLIN MATS ET AL: "The human antibody repertoire to infectious agents: Implications for disease pathogenesis.", MOLECULAR IMMUNOLOGY, vol. 40, no. 1, September 2003 (2003-09), pages 1-11, XP002696244, ISSN: 0161-5890
- WANG YAN ET AL: "Many human immunoglobulin heavy-chain IGHV gene polymorphisms have been reported in error", IMMUNOLOGY AND CELL BIOLOGY, vol. 86, no. 2, February 2008 (2008-02), pages 111-115, XP002696245, ISSN: 0818-9641
- LOVESLATI B YACOUBI ET AL: "A study of Gm allotypes and immunoglobulin heavy gamma IGHG genes in Berbers, Arabs and sub-Saharan Africans from Jerba Island, Tunisia", EUROPEAN JOURNAL OF IMMUNOGENETICS, vol. 28, no. 5, October 2001 (2001-10), pages 531-538, XP002696246, ISSN: 0960-7420
- SASSO ERIC H ET AL: "Ethnic differences in polymorphism of an immunoglobulin V-H3 gene", JOURNAL OF CLINICAL INVESTIGATION, vol. 96, no. 3, 1995, pages 1591-1600, XP002696247, ISSN: 0021-9738
- PONSEL DIRK ET AL: "High affinity, developability and functional size: the holy grail of combinatorial antibody library generation", MOLECULES: A JOURNAL OF SYNTHETIC ORGANIC AND NATURAL PRODUCT CHEMISTRY, M D P I AG, CH, vol. 16, no. 5, 1 January 2011 (2011-01-01), pages 3675-3700, XP009168724, ISSN: 1420-3049, DOI: 10.3390/MOLECULES16053675
- Declaration of Lynn E. McDonald
- L. E. Macdonald ET AL: "Precise and in situ genetic humanization of 6 Mb of mouse immunoglobulin genes", Proceedings of the National Academy of Sciences, vol. 111, no. 14, 25 March 2014 (2014-03-25), pages 5147-5152, XP055126064, ISSN: 0027-8424, DOI: 10.1073/pnas.1323896111
- GENBANK:X97051.1
- EPO BoA Decision T2220/14
- Lefranc & Lefranc: "The Immunoglobulin FactsBook", 1 January 2001 (2001-01-01), Academic Press pages 104-104,

### SEQUENCE LISTING

<110> Kymab Limited
<120> Synthetically -Extended & Ethnically-Diverse Superhuman Immunoglobin Gene Repertoires
<130> 25077.15
<160> 445
<170> PatentIn version 3.5
<210> 1
   <211> 49
   <212> DNA
   <213> homo sapiens;
<400> 1
   ttgaccaagc tggggacccc ggtcccttgg gaccagtggc agaggagtc 49
<210> 2
   <211> 61
   <212> DNA
   <213> homo sapiens;
<400> 2
   atgatgatga tgatgatgta cctgcagacc ccgtttccct ggtgccagtg gcagaggagt 60
   c 61
<210> 3
   <211> 52
   <212> DNA
   <213> homo sapiens;
<400> 3
   atgaccatga agctagagac cccggcaccg tgggaccagt gacagaggag tc 52
<210> 4
   <211> 61
   <212> DNA
   <213> homo sapiens;
<400> 4
   atgatgatga tgatgccata cctgcagacc ccggttccct ggtgccagtg gcagaggagt 60
   c 61
<210> 5
   <211> 49
   <212> DNA
   <213> homo sapiens;
<400> 5
   aactggttcg acccctgggg ccagggaacc ctggtcaccg tctcctcag 49
<210> 6
   <211> 16
   <212> PRT
   <213> homo sapiens;
<400> 6
   Asn Trp Phe Asp Pro Trp Gly Gln Gly Thr Leu Val Thr Val Ser Ser
   1 5 10 15
<210> 7
   <211> 61
   <212> DNA
   <213> homo sapiens;
<400> 7
   tactactact actactacat ggacgtctgg ggcaaaggga ccacggtcac cgtctcctca 60
   g 61
<210> 8
   <211> 20
   <212> PRT
   <213> homo sapiens;
<400> 8
   Tyr Tyr Tyr Tyr Tyr Tyr Met Asp Val Trp Gly Lys Gly Thr Thr Val
   1 5 10 15
   Thr Val Ser Ser
   20
<210> 9
   <211> 52
   <212> DNA
   <213> homo sapiens;
<400> 9
   tactggtact tcgatctctg gggccgtggc accctggtca ctgtctcctc ag 52
<210> 10
   <211> 17
   <212> PRT
   <213> homo sapiens;
<400> 10
   Tyr Trp Tyr Phe Asp Leu Trp Gly Arg Gly Thr Leu Val Thr Val Ser
   1               5                   10                  15
   Ser
<210> 11
   <211> 296
   <212> DNA
   <213> homo sapiens;
<400> 11
   caggtgcagc tggtgcagtc tggggctgag gtgaagaagc ctggggcctc agtgaaggtc      60
   tcctgcaagg cttctggata caccttcacc ggctactata tgcactgggt gcgacaggcc     120
   cctggacaag ggcttgagtg gatgggacgg atcaacccta acagtggtgg cacaaactat     180
   gcacagaagt ttcagggcag ggtcaccagt accagggaca cgtccatcag cacagcctac     240
   atggagctga gcaggctgag atctgacgac acggtcgtgt attactgtgc gagaga         296
<210> 12
   <211> 296
   <212> DNA
   <213> homo sapiens;
<400> 12
   caggtccagc ttgtgcagtc tggggctgag gtgaagaagc ctggggcctc agtgaaggtt      60
   tcctgcaagg cttctggata caccttcact agctatgcta tgcattgggt gcgccaggcc     120
   cccggacaaa ggcttgagtg gatgggatgg atcaacgctg gcaatggtaa cacaaaatat     180
   tcacagaagt tccagggcag agtcaccatt accagggaca catccgcgag cacagcctac     240
   atggagctga gcagcctgag atctgaagac acggctgtgt attactgtgc gagaga         296
<210> 13
   <211> 296
   <212> DNA
   <213> homo sapiens;
<400> 13
   caggtgcagc tggtgcagtc tggggctgag gtgaagaagc ctggggcctc agtgaaggtc 60
   tcctgcaagg cttctggata caccttcacc agttatgata tcaactgggt gcgacaggcc 120
   actggacaag ggcttgagtg gatgggatgg atgaacccta acagtggtaa cacaggctat 180
   gcacagaagt tccagggcag agtcaccatg accaggaaca cctccataag cacagcctac 240
   atggagctga gcagcctgag atctgaggac acggccgtgt attactgtgc gagagg 296
<210> 14
   <211> 296
   <212> DNA
   <213> homo sapiens;
<400> 14
   caggtccagc tggtacagtc tggggctgag gtgaagaagc ctggggcctc agtgaaggtc 60
   tcctgcaagg tttccggata caccctcact gaattatcca tgcactgggt gcgacaggct 120
   cctggaaaag ggcttgagtg gatgggaggt tttgatcctg aagatggtga aacaatctac 180
   gcacagaagt tccagggcag agtcaccatg accgaggaca catctacaga cacagcctac 240
   atggagctga gcagcctgag atctgaggac acggccgtgt attactgtgc aacaga 296
<210> 15
   <211> 296
   <212> DNA
   <213> homo sapiens;
<220>
   <221> misc_feature
   <222> (295)..(295)
   <223> n is a, c, g, or t
<400> 15
   cagatgcagc tggtgcagtc tggggctgag gtgaagaaga ctgggtcctc agtgaaggtt 60
   tcctgcaagg cttccggata caccttcacc taccgctacc tgcactgggt gcgacaggcc 120
   cccggacaag cgcttgagtg gatgggatgg atcacacctt tcaatggtaa caccaactac 180
   gcacagaaat tccaggacag agtcaccatt actagggaca ggtctatgag cacagcctac 240
   atggagctga gcagcctgag atctgaggac acagccatgt attactgtgc aagana 296
<210> 16
   <211> 296
   <212> DNA
   <213> homo sapiens;
<400> 16
   caggtgcagc tggtgcagtc tggggctgag gtgaagaagc ctggggcctc agtgaaggtt 60
   tcctgcaagg catctggata caccttcacc agctactata tgcactgggt gcgacaggcc 120
   cctggacaag ggcttgagtg gatgggaata atcaacccta gtggtggtag cacaagctac 180
   gcacagaagt tccagggcag agtcaccatg accagggaca cgtccacgag cacagtctac 240
   atggagctga gcagcctgag atctgaggac acggccgtgt attactgtgc gagaga 296
<210> 17
   <211> 296
   <212> DNA
   <213> homo sapiens;
<400> 17
   caaatgcagc tggtgcagtc tgggcctgag gtgaagaagc ctgggacctc agtgaaggtc 60
   tcctgcaagg cttctggatt cacctttact agctctgctg tgcagtgggt gcgacaggct 120
   cgtggacaac gccttgagtg gataggatgg atcgtcgttg gcagtggtaa cacaaactac 180
   gcacagaagt tccaggaaag agtcaccatt accagggaca tgtccacaag cacagcctac 240
   atggagctga gcagcctgag atccgaggac acggccgtgt attactgtgc ggcaga 296
<210> 18
   <211> 296
   <212> DNA
   <213> homo sapiens;
<400> 18
   caggtgcagc tggtgcagtc tggggctgag gtgaagaagc ctgggtcctc ggtgaaggtc 60
   tcctgcaagg cttctggagg caccttcagc agctatgcta tcagctgggt gcgacaggcc 120
   cctggacaag ggcttgagtg gatgggaggg atcatcccta tctttggtac agcaaactac 180
   gcacagaagt tccagggcag agtcacgatt accgcggacg aatccacgag cacagcctac 240
   atggagctga gcagcctgag atctgaggac acggccgtgt attactgtgc gagaga 296
<210> 19
   <211> 294
   <212> DNA
   <213> homo sapiens;
<400> 19
   caggtccagc tggtgcagtc ttgggctgag gtgaggaagt ctggggcctc agtgaaagtc 60
   tcctgtagtt tttctgggtt taccatcacc agctacggta tacattgggt gcaacagtcc 120
   cctggacaag ggcttgagtg gatgggatgg atcaaccctg gcaatggtag cccaagctat 180
   gccaagaagt ttcagggcag attcaccatg accagggaca tgtccacaac cacagcctac 240
   acagacctga gcagcctgac atctgaggac atggctgtgt attactatgc aaga 294
<210> 20
   <211> 294
   <212> DNA
   <213> homo sapiens;
<400> 20
   gaggtccagc tggtacagtc tggggctgag gtgaagaagc ctggggctac agtgaaaatc 60
   tcctgcaagg tttctggata caccttcacc gactactaca tgcactgggt gcaacaggcc 120
   cctggaaaag ggcttgagtg gatgggactt gttgatcctg aagatggtga aacaatatac 180
   gcagagaagt tccagggcag agtcaccata accgcggaca cgtctacaga cacagcctac 240
   atggagctga gcagcctgag atctgaggac acggccgtgt attactgtgc aaca 294
<210> 21
   <211> 302
   <212> DNA
   <213> homo sapiens;
<400> 21
   cagatcacct tgaaggagtc tggtcctacg ctggtgaaac ccacacagac cctcacgctg 60
   acctgcacct tctctgggtt ctcactcagc actagtggag tgggtgtggg ctggatccgt 120
   cagcccccag gaaaggccct ggagtggctt gcactcattt attggaatga tgataagcgc 180
   tacagcccat ctctgaagag caggctcacc atcaccaagg acacctccaa aaaccaggtg 240
   gtccttacaa tgaccaacat ggaccctgtg gacacagcca catattactg tgcacacaga 300
   cc 302
<210> 22
   <211> 301
   <212> DNA
   <213> homo sapiens;
<400> 22
   caggtcacct tgaaggagtc tggtcctgtg ctggtgaaac ccacagagac cctcacgctg 60
   acctgcaccg tctctgggtt ctcactcagc aatgctagaa tgggtgtgag ctggatccgt 120
   cagcccccag ggaaggccct ggagtggctt gcacacattt tttcgaatga cgaaaaatcc 180
   tacagcacat ctctgaagag caggctcacc atctccaagg acacctccaa aagccaggtg 240
   gtccttacca tgaccaacat ggaccctgtg gacacagcca catattactg tgcacggata 300
   c 301
<210> 23
   <211> 301
   <212> DNA
   <213> homo sapiens;
<400> 23
   caggtcacct tgagggagtc tggtcctgcg ctggtgaaac ccacacagac cctcacactg 60
   acctgcacct tctctgggtt ctcactcagc actagtggaa tgtgtgtgag ctggatccgt 120
   cagcccccag ggaaggccct ggagtggctt gcactcattg attgggatga tgataaatac 180
   tacagcacat ctctgaagac caggctcacc atctccaagg acacctccaa aaaccaggtg 240
   gtccttacaa tgaccaacat ggaccctgtg gacacagcca cgtattactg tgcacggata 300
   c 301
<210> 24
   <211> 296
   <212> DNA
   <213> homo sapiens;
<400> 24
   gaggtgcagc tggtggagtc tgggggaggc ttggtccagc ctggggggtc cctgagactc 60
   tcctgtgcag cctctggatt cacctttagt agctattgga tgagctgggt ccgccaggct 120
   ccagggaagg ggctggagtg ggtggccaac ataaagcaag atggaagtga gaaatactat 180
   gtggactctg tgaagggccg attcaccatc tccagagaca acgccaagaa ctcactgtat 240
   ctgcaaatga acagcctgag agccgaggac acggctgtgt attactgtgc gagaga 296
<210> 25
   <211> 298
   <212> DNA
   <213> homo sapiens;
<400> 25
   gaagtgcagc tggtggagtc tgggggaggc ttggtacagc ctggcaggtc cctgagactc 60
   tcctgtgcag cctctggatt cacctttgat gattatgcca tgcactgggt ccggcaagct 120
   ccagggaagg gcctggagtg ggtctcaggt attagttgga atagtggtag cataggctat 180
   gcggactctg tgaagggccg attcaccatc tccagagaca acgccaagaa ctccctgtat 240
   ctgcaaatga acagtctgag agctgaggac acggccttgt attactgtgc aaaagata 298
<210> 26
   <211> 296
   <212> DNA
   <213> homo sapiens;
<400> 26
   caggtgcagc tggtggagtc tgggggaggc ttggtcaagc ctggagggtc cctgagactc 60
   tcctgtgcag cctctggatt caccttcagt gactactaca tgagctggat ccgccaggct 120
   ccagggaagg ggctggagtg ggtttcatac attagtagta gtggtagtac catatactac 180
   gcagactctg tgaagggccg attcaccatc tccagggaca acgccaagaa ctcactgtat 240
   ctgcaaatga acagcctgag agccgaggac acggccgtgt attactgtgc gagaga 296
<210> 27
   <211> 293
   <212> DNA
   <213> homo sapiens;
<400> 27
   gaggtgcagc tggtggagtc tgggggaggc ttggtacagc ctggggggtc cctgagactc 60
   tcctgtgcag cctctggatt caccttcagt agctacgaca tgcactgggt ccgccaagct 120
   acaggaaaag gtctggagtg ggtctcagct attggtactg ctggtgacac atactatcca 180
   ggctccgtga agggccgatt caccatctcc agagaaaatg ccaagaactc cttgtatctt 240
   caaatgaaca gcctgagagc cggggacacg gctgtgtatt actgtgcaag aga 293
<210> 28
   <211> 302
   <212> DNA
   <213> homo sapiens;
<400> 28
   gaggtgcagc tggtggagtc tgggggaggc ttggtaaagc ctggggggtc ccttagactc 60
   tcctgtgcag cctctggatt cactttcagt aacgcctgga tgagctgggt ccgccaggct 120
   ccagggaagg ggctggagtg ggttggccgt attaaaagca aaactgatgg tgggacaaca 180
   gactacgctg cacccgtgaa aggcagattc accatctcaa gagatgattc aaaaaacacg 240
   ctgtatctgc aaatgaacag cctgaaaacc gaggacacag ccgtgtatta ctgtaccaca 300
   ga 302
<210> 29
   <211> 296
   <212> DNA
   <213> homo sapiens;
<400> 29
   gaggtacaac tggtggagtc tgggggaggc ttggtacagc ctggggggtc cctgagactc 60
   tcctgtgcag cctctggatt caccttcagt aacagtgaca tgaactgggc ccgcaaggct 120
   ccaggaaagg ggctggagtg ggtatcgggt gttagttgga atggcagtag gacgcactat 180
   gtggactccg tgaagcgccg attcatcatc tccagagaca attccaggaa ctccctgtat 240
   ctgcaaaaga acagacggag agccgaggac atggctgtgt attactgtgt gagaaa 296
<210> 30
   <211> 296
   <212> DNA
   <213> homo sapiens;
<400> 30
   acagtgcagc tggtggagtc tgggggaggc ttggtagagc ctggggggtc cctgagactc 60
   tcctgtgcag cctctggatt caccttcagt aacagtgaca tgaactgggt ccgccaggct 120
   ccaggaaagg ggctggagtg ggtatcgggt gttagttgga atggcagtag gacgcactat 180
   gcagactctg tgaagggccg attcatcatc tccagagaca attccaggaa cttcctgtat 240
   cagcaaatga acagcctgag gcccgaggac atggctgtgt attactgtgt gagaaa 296
<210> 31
   <211> 296
   <212> DNA
   <213> homo sapiens;
<400> 31
   gaggtgcagc tggtggagtc tgggggaggt gtggtacggc ctggggggtc cctgagactc 60
   tcctgtgcag cctctggatt cacctttgat gattatggca tgagctgggt ccgccaagct 120
   ccagggaagg ggctggagtg ggtctctggt attaattgga atggtggtag cacaggttat 180
   gcagactctg tgaagggccg attcaccatc tccagagaca acgccaagaa ctccctgtat 240
   ctgcaaatga acagtctgag agccgaggac acggccttgt atcactgtgc gagaga 296
<210> 32
   <211> 296
   <212> DNA
   <213> homo sapiens;
<400> 32
   gaggtgcagc tggtggagtc tgggggaggc ctggtcaagc ctggggggtc cctgagactc 60
   tcctgtgcag cctctggatt caccttcagt agctatagca tgaactgggt ccgccaggct 120
   ccagggaagg ggctggagtg ggtctcatcc attagtagta gtagtagtta catatactac 180
   gcagactcag tgaagggccg attcaccatc tccagagaca acgccaagaa ctcactgtat 240
   ctgcaaatga acagcctgag agccgaggac acggctgtgt attactgtgc gagaga 296
<210> 33
   <211> 296
   <212> DNA
   <213> homo sapiens;
<400> 33
   gaggtgcagc tgttggagtc tgggggaggc ttggtacagc ctggggggtc cctgagactc 60
   tcctgtgcag cctctggatt cacctttagc agctatgcca tgagctgggt ccgccaggct 120
   ccagggaagg ggctggagtg ggtctcagct attagtggta gtggtggtag cacatactac 180
   gcagactccg tgaagggccg gttcaccatc tccagagaca attccaagaa cacgctgtat 240
   ctgcaaatga acagcctgag agccgaggac acggccgtat attactgtgc gaaaga 296
<210> 34
   <211> 296
   <212> DNA
   <213> homo sapiens;
<400> 34
   caggtgcagc tggtggagtc tgggggaggc gtggtccagc ctgggaggtc cctgagactc 60
   tcctgtgcag cctctggatt caccttcagt agctatgcta tgcactgggt ccgccaggct 120
   ccaggcaagg ggctagagtg ggtggcagtt atatcatatg atggaagtaa taaatactac 180
   gcagactccg tgaagggccg attcaccatc tccagagaca attccaagaa cacgctgtat 240
   ctgcaaatga acagcctgag agctgaggac acggctgtgt attactgtgc gagaga 296
<210> 35
   <211> 294
   <212> DNA
   <213> homo sapiens;
<400> 35
   caggtgcagc tggtggagtc tgggggaggc gtggtccagc ctgggaggtc cctgagactc 60
   tcctgtgcag cctctggatt caccttcagt agctatgcta tgcactgggt ccgccaggct 120
   ccaggcaagg ggctggagtg ggtggcagtt atatcatatg atggaagcaa taaatactac 180
   gcagactccg tgaagggccg attcaccatc tccagagaca attccaagaa cacgctgtat 240
   ctgcaaatga acagcctgag agctgaggac acggctgtgt attactgtgc gaga 294
<210> 36
   <211> 296
   <212> DNA
   <213> homo sapiens;
<400> 36
   caggtgcagc tggtggagtc tgggggaggc gtggtccagc ctgggaggtc cctgagactc 60
   tcctgtgcag cgtctggatt caccttcagt agctatggca tgcactgggt ccgccaggct 120
   ccaggcaagg ggctggagtg ggtggcagtt atatggtatg atggaagtaa taaatactat 180
   gcagactccg tgaagggccg attcaccatc tccagagaca attccaagaa cacgctgtat 240
   ctgcaaatga acagcctgag agccgaggac acggctgtgt attactgtgc gagaga 296
<210> 37
   <211> 296
   <212> DNA
   <213> homo sapiens;
<400> 37
   gaggtgcagc tggtggagtc tgggggaggc ttggtacagc ctgggggatc cctgagactc 60
   tcctgtgcag cctctggatt caccttcagt aacagtgaca tgaactgggt ccatcaggct 120
   ccaggaaagg ggctggagtg ggtatcgggt gttagttgga atggcagtag gacgcactat 180
   gcagactctg tgaagggccg attcatcatc tccagagaca attccaggaa caccctgtat 240
   ctgcaaacga atagcctgag ggccgaggac acggctgtgt attactgtgt gagaaa 296
<210> 38
   <211> 292
   <212> DNA
   <213> homo sapiens;
<400> 38
   gaggtgcagc tggtggagtc tgggggaggc ttggtacagc ctagggggtc cctgagactc 60
   tcctgtgcag cctctggatt caccgtcagt agcaatgaga tgagctggat ccgccaggct 120
   ccagggaagg ggctggagtg ggtctcatcc attagtggtg gtagcacata ctacgcagac 180
   tccaggaagg gcagattcac catctccaga gacaattcca agaacacgct gtatcttcaa 240
   atgaacaacc tgagagctga gggcacggcc gcgtattact gtgccagata ta 292
<210> 39
   <211> 298
   <212> DNA
   <213> homo sapiens;
<400> 39
   gaagtgcagc tggtggagtc tgggggagtc gtggtacagc ctggggggtc cctgagactc 60
   tcctgtgcag cctctggatt cacctttgat gattatacca tgcactgggt ccgtcaagct 120
   ccggggaagg gtctggagtg ggtctctctt attagttggg atggtggtag cacatactat 180
   gcagactctg tgaagggccg attcaccatc tccagagaca acagcaaaaa ctccctgtat 240
   ctgcaaatga acagtctgag aactgaggac accgccttgt attactgtgc aaaagata 298
<210> 40
   <211> 291
   <212> DNA
   <213> homo sapiens;
<400> 40
   gaggatcagc tggtggagtc tgggggaggc ttggtacagc ctggggggtc cctgcgaccc 60
   tcctgtgcag cctctggatt cgccttcagt agctatgctc tgcactgggt tcgccgggct 120
   ccagggaagg gtctggagtg ggtatcagct attggtactg gtggtgatac atactatgca 180
   gactccgtga tgggccgatt caccatctcc agagacaacg ccaagaagtc cttgtatctt 240
   catatgaaca gcctgatagc tgaggacatg gctgtgtatt attgtgcaag a 291
<210> 41
   <211> 296
   <212> DNA
   <213> homo sapiens;
<400> 41
   gaggtgcagc tggtggagtc tgggggaggc ttggtacagc ctggggggtc cctgagactc 60
   tcctgtgcag cctctggatt caccttcagt agctatagca tgaactgggt ccgccaggct 120
   ccagggaagg ggctggagtg ggtttcatac attagtagta gtagtagtac catatactac 180
   gcagactctg tgaagggccg attcaccatc tccagagaca atgccaagaa ctcactgtat 240
   ctgcaaatga acagcctgag agccgaggac acggctgtgt attactgtgc gagaga 296
<210> 42
   <211> 302
   <212> DNA
   <213> homo sapiens;
<400> 42
   gaggtgcagc tggtggagtc tgggggaggc ttggtacagc cagggcggtc cctgagactc 60
   tcctgtacag cttctggatt cacctttggt gattatgcta tgagctggtt ccgccaggct 120
   ccagggaagg ggctggagtg ggtaggtttc attagaagca aagcttatgg tgggacaaca 180
   gaatacaccg cgtctgtgaa aggcagattc accatctcaa gagatggttc caaaagcatc 240
   gcctatctgc aaatgaacag cctgaaaacc gaggacacag ccgtgtatta ctgtactaga 300
   ga 302
<210> 43
   <211> 293
   <212> DNA
   <213> homo sapiens;
<400> 43
   gaggtgcagc tggtggagtc tggaggaggc ttgatccagc ctggggggtc cctgagactc 60
   tcctgtgcag cctctgggtt caccgtcagt agcaactaca tgagctgggt ccgccaggct 120
   ccagggaagg ggctggagtg ggtctcagtt atttatagcg gtggtagcac atactacgca 180
   gactccgtga agggccgatt caccatctcc agagacaatt ccaagaacac gctgtatctt 240
   caaatgaaca gcctgagagc cgaggacacg gccgtgtatt actgtgcgag aga 293
<210> 44
   <211> 296
   <212> DNA
   <213> homo sapiens;
<400> 44
   gaggtgcagc tggtggagtc tgggggaggc ttggtccagc ctggggggtc cctgagactc 60
   tcctgtgcag cctctggatt caccttcagt agctatgcta tgcactgggt ccgccaggct 120
   ccagggaagg gactggaata tgtttcagct attagtagta atgggggtag cacatattat 180
   gcaaactctg tgaagggcag attcaccatc tccagagaca attccaagaa cacgctgtat 240
   cttcaaatgg gcagcctgag agctgaggac atggctgtgt attactgtgc gagaga 296
<210> 45
   <211> 293
   <212> DNA
   <213> homo sapiens;
<400> 45
   gaggtgcagc tggtggagtc tgggggaggc ttggtccagc ctggggggtc cctgagactc 60
   tcctgtgcag cctctggatt caccgtcagt agcaactaca tgagctgggt ccgccaggct 120
   ccagggaagg ggctggagtg ggtctcagtt atttatagcg gtggtagcac atactacgca 180
   gactccgtga agggcagatt caccatctcc agagacaatt ccaagaacac gctgtatctt 240
   caaatgaaca gcctgagagc cgaggacacg gctgtgtatt actgtgcgag aga 293
<210> 46
   <211> 302
   <212> DNA
   <213> homo sapiens;
<400> 46
   gaggtgcagc tggtggagtc tgggggaggc ttggtccagc ctggagggtc cctgagactc 60
   tcctgtgcag cctctggatt caccttcagt gaccactaca tggactgggt ccgccaggct 120
   ccagggaagg ggctggagtg ggttggccgt actagaaaca aagctaacag ttacaccaca 180
   gaatacgccg cgtctgtgaa aggcagattc accatctcaa gagatgattc aaagaactca 240
   ctgtatctgc aaatgaacag cctgaaaacc gaggacacgg ccgtgtatta ctgtgctaga 300
   ga 302
<210> 47
   <211> 302
   <212> DNA
   <213> homo sapiens;
<400> 47
   gaggtgcagc tggtggagtc tgggggaggc ttggtccagc ctggggggtc cctgaaactc 60
   tcctgtgcag cctctgggtt caccttcagt ggctctgcta tgcactgggt ccgccaggct 120
   tccgggaaag ggctggagtg ggttggccgt attagaagca aagctaacag ttacgcgaca 180
   gcatatgctg cgtcggtgaa aggcaggttc accatctcca gagatgattc aaagaacacg 240
   gcgtatctgc aaatgaacag cctgaaaacc gaggacacgg ccgtgtatta ctgtactaga 300
   ca 302
<210> 48
   <211> 296
   <212> DNA
   <213> homo sapiens;
<400> 48
   gaggtgcagc tggtggagtc cgggggaggc ttagttcagc ctggggggtc cctgagactc 60
   tcctgtgcag cctctggatt caccttcagt agctactgga tgcactgggt ccgccaagct 120
   ccagggaagg ggctggtgtg ggtctcacgt attaatagtg atgggagtag cacaagctac 180
   gcggactccg tgaagggccg attcaccatc tccagagaca acgccaagaa cacgctgtat 240
   ctgcaaatga acagtctgag agccgaggac acggctgtgt attactgtgc aagaga 296
<210> 49
   <211> 288
   <212> DNA
   <213> homo sapiens;
<400> 49
   gaggtgcagc tggtggagtc tcggggagtc ttggtacagc ctggggggtc cctgagactc 60
   tcctgtgcag cctctggatt caccgtcagt agcaatgaga tgagctgggt ccgccaggct 120
   ccagggaagg gtctggagtg ggtctcatcc attagtggtg gtagcacata ctacgcagac 180
   tccaggaagg gcagattcac catctccaga gacaattcca agaacacgct gcatcttcaa 240
   atgaacagcc tgagagctga ggacacggct gtgtattact gtaagaaa 288
<210> 50
   <211> 293
   <212> DNA
   <213> homo sapiens;
<400> 50
   gaggtgcagc tggtggagtc tgggggaggc ttggtaaagc ctggggggtc cctgagactc 60
   tcctgtgcag cctctggatt caccttcagt gactactaca tgaactgggt ccgccaggct 120
   ccagggaagg ggctggagtg ggtctcatcc attagtagta gtagtaccat atactacgca 180
   gactctgtga agggccgatt caccatctcc agagacaacg ccaagaactc actgtatctg 240
   caaatgaaca gcctgagagc cgaggacacg gctgtgtatt actgtgcgag aga 293
<210> 51
   <211> 296
   <212> DNA
   <213> homo sapiens;
<400> 51
   caggtgcagc tgcaggagtc gggcccagga ctggtgaagc ctccggggac cctgtccctc 60
   acctgcgctg tctctggtgg ctccatcagc agtagtaact ggtggagttg ggtccgccag 120
   cccccaggga aggggctgga gtggattggg gaaatctatc atagtgggag caccaactac 180
   aacccgtccc tcaagagtcg agtcaccata tcagtagaca agtccaagaa ccagttctcc 240
   ctgaagctga gctctgtgac cgccgcggac acggccgtgt attgctgtgc gagaga 296
<210> 52
   <211> 296
   <212> DNA
   <213> homo sapiens;
<400> 52
   caggtgcagc tgcaggagtc gggcccagga ctggtgaagc cttcggacac cctgtccctc 60
   acctgcgctg tctctggtta ctccatcagc agtagtaact ggtggggctg gatccggcag 120
   cccccaggga agggactgga gtggattggg tacatctatt atagtgggag cacctactac 180
   aacccgtccc tcaagagtcg agtcaccatg tcagtagaca cgtccaagaa ccagttctcc 240
   ctgaagctga gctctgtgac cgccgtggac acggccgtgt attactgtgc gagaaa 296
<210> 53
   <211> 299
   <212> DNA
   <213> homo sapiens;
<400> 53
   cagctgcagc tgcaggagtc cggctcagga ctggtgaagc cttcacagac cctgtccctc 60
   acctgcgctg tctctggtgg ctccatcagc agtggtggtt actcctggag ctggatccgg 120
   cagccaccag ggaagggcct ggagtggatt gggtacatct atcatagtgg gagcacctac 180
   tacaacccgt ccctcaagag tcgagtcacc atatcagtag acaggtccaa gaaccagttc 240
   tccctgaagc tgagctctgt gaccgccgcg gacacggccg tgtattactg tgccagaga 299
<210> 54
   <211> 299
   <212> DNA
   <213> homo sapiens;
<400> 54
   caggtgcagc tgcaggagtc gggcccagga ctggtgaagc cttcacagac cctgtccctc 60
   acctgcactg tctctggtgg ctccatcagc agtggtgatt actactggag ttggatccgc 120
   cagcccccag ggaagggcct ggagtggatt gggtacatct attacagtgg gagcacctac 180
   tacaacccgt ccctcaagag tcgagttacc atatcagtag acacgtccaa gaaccagttc 240
   tccctgaagc tgagctctgt gactgccgca gacacggccg tgtattactg tgccagaga 299
<210> 55
   <211> 299
   <212> DNA
   <213> homo sapiens;
<400> 55
   caggtgcagc tgcaggagtc gggcccagga ctggtgaagc cttcacagac cctgtccctc 60
   acctgcactg tctctggtgg ctccatcagc agtggtggtt actactggag ctggatccgc 120
   cagcacccag ggaagggcct ggagtggatt gggtacatct attacagtgg gagcacctac 180
   tacaacccgt ccctcaagag tctagttacc atatcagtag acacgtctaa gaaccagttc 240
   tccctgaagc tgagctctgt gactgccgcg gacacggccg tgtattactg tgcgagaga 299
<210> 56
   <211> 293
   <212> DNA
   <213> homo sapiens;
<400> 56
   caggtgcagc tacagcagtg gggcgcagga ctgttgaagc cttcggagac cctgtccctc 60
   acctgcgctg tctatggtgg gtccttcagt ggttactact ggagctggat ccgccagccc 120
   ccagggaagg ggctggagtg gattggggaa atcaatcata gtggaagcac caactacaac 180
   ccgtccctca agagtcgagt caccatatca gtagacacgt ccaagaacca gttctccctg 240
   aagctgagct ctgtgaccgc cgcggacacg gctgtgtatt actgtgcgag agg 293
<210> 57
   <211> 299
   <212> DNA
   <213> homo sapiens;
<400> 57
   cagctgcagc tgcaggagtc gggcccagga ctggtgaagc cttcggagac cctgtccctc 60
   acctgcactg tctctggtgg ctccatcagc agtagtagtt actactgggg ctggatccgc 120
   cagcccccag ggaaggggct ggagtggatt gggagtatct attatagtgg gagcacctac 180
   tacaacccgt ccctcaagag tcgagtcacc atatccgtag acacgtccaa gaaccagttc 240
   tccctgaagc tgagctctgt gaccgccgca gacacggctg tgtattactg tgcgagaca 299
<210> 58
   <211> 296
   <212> DNA
   <213> homo sapiens;
<400> 58
   caggtgcagc tgcaggagtc gggcccagga ctggtgaagc cttcggagac cctgtccctc 60
   atctgcgctg tctctggtga ctccatcagc agtggtaact ggtgaatctg ggtccgccag 120
   cccccaggga aggggctgga gtggattggg gaaatccatc atagtgggag cacctactac 180
   aacccgtccc tcaagagtcg aatcaccatg tccgtagaca cgtccaagaa ccagttctac 240
   ctgaagctga gctctgtgac cgccgcggac acggccgtgt attactgtgc gagata 296
<210> 59
   <211> 293
   <212> DNA
   <213> homo sapiens;
<400> 59
   caggtgcagc tgcaggagtc gggcccagga ctggtgaagc cttcggagac cctgtccctc 60
   acctgcactg tctctggtgg ctccatcagt agttactact ggagctggat ccggcagccc 120
   ccagggaagg gactggagtg gattgggtat atctattaca gtgggagcac caactacaac 180
   ccctccctca agagtcgagt caccatatca gtagacacgt ccaagaacca gttctccctg 240
   aagctgagct ctgtgaccgc tgcggacacg gccgtgtatt actgtgcgag aga 293
<210> 60
   <211> 299
   <212> DNA
   <213> homo sapiens;
<400> 60
   caggtgcagc tgcaggagtc gggcccagga ctggtgaagc cttcggagac cctgtccctc 60
   acctgcactg tctctggtgg ctccgtcagc agtggtagtt actactggag ctggatccgg 120
   cagcccccag ggaagggact ggagtggatt gggtatatct attacagtgg gagcaccaac 180
   tacaacccct ccctcaagag tcgagtcacc atatcagtag acacgtccaa gaaccagttc 240
   tccctgaagc tgagctctgt gaccgctgcg gacacggccg tgtattactg tgcgagaga 299
<210> 61
   <211> 294
   <212> DNA
   <213> homo sapiens;
<400> 61
   caggtgcagc tgcaggagtc gggcccagga ctggtgaagc cttcggagac cctgtccctc 60
   acctgcgctg tctctggtta ctccatcagc agtggttact actggggctg gatccggcag 120
   cccccaggga aggggctgga gtggattggg agtatctatc atagtgggag cacctactac 180
   aacccgtccc tcaagagtcg agtcaccata tcagtagaca cgtccaagaa ccagttctcc 240
   ctgaagctga gctctgtgac cgccgcagac acggccgtgt attactgtgc gaga 294
<210> 62
   <211> 296
   <212> DNA
   <213> homo sapiens;
<400> 62
   gaggtgcagc tggtgcagtc tggagcagag gtgaaaaagc ccggggagtc tctgaagatc 60
   tcctgtaagg gttctggata cagctttacc agctactgga tcggctgggt gcgccagatg 120
   cccgggaaag gcctggagtg gatggggatc atctatcctg gtgactctga taccagatac 180
   agcccgtcct tccaaggcca ggtcaccatc tcagccgaca agtccatcag caccgcctac 240
   ctgcagtgga gcagcctgaa ggcctcggac accgccatgt attactgtgc gagaca 296
<210> 63
   <211> 294
   <212> DNA
   <213> homo sapiens;
<400> 63
   gaagtgcagc tggtgcagtc tggagcagag gtgaaaaagc ccggggagtc tctgaggatc 60
   tcctgtaagg gttctggata cagctttacc agctactgga tcagctgggt gcgccagatg 120
   cccgggaaag gcctggagtg gatggggagg attgatccta gtgactctta taccaactac 180
   agcccgtcct tccaaggcca cgtcaccatc tcagctgaca agtccatcag cactgcctac 240
   ctgcagtgga gcagcctgaa ggcctcggac accgccatgt attactgtgc gaga 294
<210> 64
   <211> 305
   <212> DNA
   <213> homo sapiens;
<400> 64
   caggtacagc tgcagcagtc aggtccagga ctggtgaagc cctcgcagac cctctcactc 60
   acctgtgcca tctccgggga cagtgtctct agcaacagtg ctgcttggaa ctggatcagg 120
   cagtccccat cgagaggcct tgagtggctg ggaaggacat actacaggtc caagtggtat 180
   aatgattatg cagtatctgt gaaaagtcga ataaccatca acccagacac atccaagaac 240
   cagttctccc tgcagctgaa ctctgtgact cccgaggaca cggctgtgta ttactgtgca 300
   agaga 305
<210> 65
   <211> 294
   <212> DNA
   <213> homo sapiens;
<400> 65
   caggtgcagc tggtgcaatc tgggtctgag ttgaagaagc ctggggcctc agtgaaggtt 60
   tcctgcaagg cttctggata caccttcact agctatgcta tgaattgggt gcgacaggcc 120
   cctggacaag ggcttgagtg gatgggatgg atcaacacca acactgggaa cccaacgtat 180
   gcccagggct tcacaggacg gtttgtcttc tccttggaca cctctgtcag cacggcatat 240
   ctgcagatct gcagcctaaa ggctgaggac actgccgtgt attactgtgc gaga 294
<210> 66
   <211> 296
   <212> DNA
   <213> homo sapiens;
<400> 66
   caggtgcagc tggtgcagtc tggccatgag gtgaagcagc ctggggcctc agtgaaggtc 60
   tcctgcaagg cttctggtta cagtttcacc acctatggta tgaattgggt gccacaggcc 120
   cctggacaag ggcttgagtg gatgggatgg ttcaacacct acactgggaa cccaacatat 180
   gcccagggct tcacaggacg gtttgtcttc tccatggaca cctctgccag cacagcatac 240
   ctgcagatca gcagcctaaa ggctgaggac atggccatgt attactgtgc gagata 296
<210> 67
   <211> 17
   <212> DNA
   <213> homo sapiens;
<400> 67
   ggtacaactg gaacgac 17
<210> 68
   <211> 17
   <212> DNA
   <213> homo sapiens;
<400> 68
   ggtataactg gaactac 17
<210> 69
   <211> 17
   <212> DNA
   <213> homo sapiens;
<400> 69
   ggtataaccg gaaccac 17
<210> 70
   <211> 17
   <212> DNA
   <213> homo sapiens;
<400> 70
   ggtataactg gaacgac 17
<210> 71
   <211> 20
   <212> DNA
   <213> homo sapiens;
<400> 71
   ggtatagtgg gagctactac 20
<210> 72
   <211> 31
   <212> DNA
   <213> homo sapiens;
<400> 72
   aggatattgt agtagtacca gctgctatgc c 31
<210> 73
   <211> 31
   <212> DNA
   <213> homo sapiens;
<400> 73
   aggatattgt actaatggtg tatgctatac c 31
<210> 74
   <211> 31
   <212> DNA
   <213> homo sapiens;
<400> 74
   aggatattgt agtggtggta gctgctactc c 31
<210> 75
   <211> 28
   <212> DNA
   <213> homo sapiens;
<400> 75
   agcatattgt ggtggtgatt gctattcc 28
<210> 76
   <211> 31
   <212> DNA
   <213> homo sapiens;
<400> 76
   gtattacgat ttttggagtg gttattatac c 31
<210> 77
   <211> 31
   <212> DNA
   <213> homo sapiens;
<400> 77
   gtattacgat attttgactg gttattataa c 31
<210> 78
   <211> 31
   <212> DNA
   <213> homo sapiens;
<400> 78
   gtattactat ggttcgggga gttattataa c 31
<210> 79
   <211> 37
   <212> DNA
   <213> homo sapiens;
<400> 79
   gtattatgat tacgtttggg ggagttatgc ttatacc 37
<210> 80
   <211> 31
   <212> DNA
   <213> homo sapiens;
<400> 80
   gtattactat gatagtagtg gttattacta c 31
<210> 81
   <211> 16
   <212> DNA
   <213> homo sapiens;
<400> 81
   tgactacagt aactac 16
<210> 82
   <211> 16
   <212> DNA
   <213> homo sapiens;
<400> 82
   tgactacagt aactac 16
<210> 83
   <211> 16
   <212> DNA
   <213> homo sapiens;
<400> 83
   tgactacggt gactac 16
<210> 84
   <211> 19
   <212> DNA
   <213> homo sapiens;
<400> 84
   tgactacggt ggtaactcc 19
<210> 85
   <211> 20
   <212> DNA
   <213> homo sapiens;
<400> 85
   gtggatacag ctatggttac 20
<210> 86
   <211> 23
   <212> DNA
   <213> homo sapiens;
<400> 86
   gtggatatag tggctacgat tac 23
<210> 87
   <211> 20
   <212> DNA
   <213> homo sapiens;
<400> 87
   gtggatacag ctatggttac 20
<210> 88
   <211> 20
   <212> DNA
   <213> homo sapiens;
<400> 88
   gtagagatgg ctacaattac 20
<210> 89
   <211> 18
   <212> DNA
   <213> homo sapiens;
<400> 89
   gagtatagca gctcgtcc 18
<210> 90
   <211> 21
   <212> DNA
   <213> homo sapiens;
<400> 90
   gggtatagca gcagctggta c 21
<210> 91
   <211> 21
   <212> DNA
   <213> homo sapiens;
<400> 91
   gggtatagca gtggctggta c 21
<210> 92
   <211> 18
   <212> DNA
   <213> homo sapiens;
<400> 92
   gggtatagca gcggctac 18
<210> 93
   <211> 11
   <212> DNA
   <213> homo sapiens;
<400> 93
   ctaactgggg a 11
<210> 94
   <211> 52
   <212> DNA
   <213> homo sapiens;
<400> 94
   gctgaatact tccagcactg gggccagggc accctggtca ccgtctcctc ag 52
<210> 95
   <211> 53
   <212> DNA
   <213> homo sapiens;
<400> 95
   ctactggtac ttcgatctct ggggccgtgg caccctggtc actgtctcct cag 53
<210> 96
   <211> 50
   <212> DNA
   <213> homo sapiens;
<400> 96
   tgatgctttt gatgtctggg gccaagggac aatggtcacc gtctcttcag 50
<210> 97
   <211> 48
   <212> DNA
   <213> homo sapiens;
<400> 97
   actactttga ctactggggc caaggaaccc tggtcaccgt ctcctcag 48
<210> 98
   <211> 51
   <212> DNA
   <213> homo sapiens;
<400> 98
   acaactggtt cgactcctgg ggccaaggaa ccctggtcac cgtctcctca g 51
<210> 99
   <211> 63
   <212> DNA
   <213> homo sapiens;
<400> 99
   attactacta ctactacggt atggacgtct gggggcaagg gaccacggtc accgtctcct 60
   cag 63
<210> 100
   <211> 287
   <212> DNA
   <213> homo sapiens;
<400> 100
   gacatccaga tgacccagtc tccttccacc ctgtctgcat ctgtaggaga cagagtcacc 60
   atcacttgcc gggccagtca gagtattagt agctggttgg cctggtatca gcagaaacca 120
   gggaaagccc ctaagctcct gatctatgat gcctccagtt tggaaagtgg ggtcccatca 180
   aggttcagcg gcagtggatc tgggacagaa ttcactctca ccatcagcag cctgcagcct 240
   gatgattttg caacttatta ctgccaacag tataatagtt attctcc 287
<210> 101
   <211> 287
   <212> DNA
   <213> homo sapiens;
<400> 101
   gccatccaga tgacccagtc tccatcctcc ctgtctgcat ctgtaggaga cagagtcacc 60
   atcacttgcc gggcaagtca gggcattaga aatgatttag gctggtatca gcagaaacca 120
   gggaaagccc ctaagctcct gatctatgct gcatccagtt tacaaagtgg ggtcccatca 180
   aggttcagcg gcagtggatc tggcacagat ttcactctca ccatcagcag cctgcagcct 240
   gaagattttg caacttatta ctgtctacaa gattacaatt accctcc 287
<210> 102
   <211> 287
   <212> DNA
   <213> homo sapiens;
<400> 102
   gccatccgga tgacccagtc tccatcctca ttctctgcat ctacaggaga cagagtcacc 60
   atcacttgtc gggcgagtca gggtattagc agttatttag cctggtatca gcaaaaacca 120
   gggaaagccc ctaagctcct gatctatgct gcatccactt tgcaaagtgg ggtcccatca 180
   aggttcagcg gcagtggatc tgggacagat ttcactctca ccatcagctg cctgcagtct 240
   gaagattttg caacttatta ctgtcaacag tattatagtt accctcc 287
<210> 103
   <211> 287
   <212> DNA
   <213> homo sapiens;
<400> 103
   gtcatctgga tgacccagtc tccatcctta ctctctgcat ctacaggaga cagagtcacc 60
   atcagttgtc ggatgagtca gggcattagc agttatttag cctggtatca gcaaaaacca 120
   gggaaagccc ctgagctcct gatctatgct gcatccactt tgcaaagtgg ggtcccatca 180
   aggttcagtg gcagtggatc tgggacagat ttcactctca ccatcagttg cctgcagtct 240
   gaagattttg caacttatta ctgtcaacag tattatagtt tccctcc 287
<210> 104
   <211> 287
   <212> DNA
   <213> homo sapiens;
<400> 104
   gacatccagt tgacccagtc tccatccttc ctgtctgcat ctgtaggaga cagagtcacc 60
   atcacttgcc gggccagtca gggcattagc agttatttag cctggtatca gcaaaaacca 120
   gggaaagccc ctaagctcct gatctatgct gcatccactt tgcaaagtgg ggtcccatca 180
   aggttcagcg gcagtggatc tgggacagaa ttcactctca caatcagcag cctgcagcct 240
   gaagattttg caacttatta ctgtcaacag cttaatagtt accctcc 287
<210> 105
   <211> 287
   <212> DNA
   <213> homo sapiens;
<400> 105
   gacatccaga tgacccagtc tccatcttcc gtgtctgcat ctgtaggaga cagagtcacc 60
   atcacttgtc gggcgagtca gggtattagc agctggttag cctggtatca gcagaaacca 120
   gggaaagccc ctaagctcct gatctatgct gcatccagtt tgcaaagtgg ggtcccatca 180
   aggttcagcg gcagtggatc tgggacagat ttcactctca ccatcagcag cctgcagcct 240
   gaagattttg caacttacta ttgtcaacag gctaacagtt tccctcc 287
<210> 106
   <211> 287
   <212> DNA
   <213> homo sapiens;
<400> 106
   gccatccagt tgacccagtc tccatcctcc ctgtctgcat ctgtaggaga cagagtcacc 60
   atcacttgcc gggcaagtca gggcattagc agtgctttag cctgatatca gcagaaacca 120
   gggaaagctc ctaagctcct gatctatgat gcctccagtt tggaaagtgg ggtcccatca 180
   aggttcagcg gcagtggatc tgggacagat ttcactctca ccatcagcag cctgcagcct 240
   gaagattttg caacttatta ctgtcaacag tttaataatt accctca 287
<210> 107
   <211> 287
   <212> DNA
   <213> homo sapiens;
<400> 107
   gacatccaga tgacccagtc tccatcctca ctgtctgcat ctgtaggaga cagagtcacc 60
   atcacttgtc gggcgagtca gggcattagc aattatttag cctggtttca gcagaaacca 120
   gggaaagccc ctaagtccct gatctatgct gcatccagtt tgcaaagtgg ggtcccatca 180
   aggttcagcg gcagtggatc tgggacagat ttcactctca ccatcagcag cctgcagcct 240
   gaagattttg caacttatta ctgccaacag tataatagtt accctcc 287
<210> 108
   <211> 287
   <212> DNA
   <213> homo sapiens;
<400> 108
   gacatccaga tgacccagtc tccatcctca ctgtctgcat ctgtaggaga cagagtcacc 60
   atcacttgtc gggcgagtca gggtattagc agctggttag cctggtatca gcagaaacca 120
   gagaaagccc ctaagtccct gatctatgct gcatccagtt tgcaaagtgg ggtcccatca 180
   aggttcagcg gcagtggatc tgggacagat ttcactctca ccatcagcag cctgcagcct 240
   gaagattttg caacttatta ctgccaacag tataatagtt accctcc 287
<210> 109
   <211> 287
   <212> DNA
   <213> homo sapiens;
<400> 109
   gacatccaga tgacccagtc tccatcctcc ctgtctgcat ctgtaggaga cagagtcacc 60
   atcacttgcc gggcaagtca gggcattaga aatgatttag gctggtatca gcagaaacca 120
   gggaaagccc ctaagcgcct gatctatgct gcatccagtt tgcaaagtgg ggtcccatca 180
   aggttcagcg gcagtggatc tgggacagaa ttcactctca caatcagcag cctgcagcct 240
   gaagattttg caacttatta ctgtctacag cataatagtt accctcc 287
<210> 110
   <211> 287
   <212> DNA
   <213> homo sapiens;
<400> 110
   aacatccaga tgacccagtc tccatctgcc atgtctgcat ctgtaggaga cagagtcacc 60
   atcacttgtc gggcgaggca gggcattagc aattatttag cctggtttca gcagaaacca 120
   gggaaagtcc ctaagcacct gatctatgct gcatccagtt tgcaaagtgg ggtcccatca 180
   aggttcagcg gcagtggatc tgggacagaa ttcactctca caatcagcag cctgcagcct 240
   gaagattttg caacttatta ctgtctacag cataatagtt accctcc 287
<210> 111
   <211> 287
   <212> DNA
   <213> homo sapiens;
<400> 111
   gacatccaga tgacccagtc tccatcctcc ctgtctgcat ctgtaggaga cagagtcacc 60
   atcacttgcc gggcgagtca gggcattagc aattatttag cctggtatca gcagaaacca 120
   gggaaagttc ctaagctcct gatctatgct gcatccactt tgcaatcagg ggtcccatct 180
   cggttcagtg gcagtggatc tgggacagat ttcactctca ccatcagcag cctgcagcct 240
   gaagatgttg caacttatta ctgtcaaaag tataacagtg cccctcc 287
<210> 112
   <211> 287
   <212> DNA
   <213> homo sapiens;
<400> 112
   gacatccaga tgacccagtc tccatcctcc ctgtctgcat ctgtaggaga cagagtcacc 60
   atcacttgcc aggcgagtca ggacattagc aactatttaa attggtatca gcagaaacca 120
   gggaaagccc ctaagctcct gatctacgat gcatccaatt tggaaacagg ggtcccatca 180
   aggttcagtg gaagtggatc tgggacagat tttactttca ccatcagcag cctgcagcct 240
   gaagatattg caacatatta ctgtcaacag tatgataatc tccctcc 287
<210> 113
   <211> 287
   <212> DNA
   <213> homo sapiens;
<400> 113
   gacatccaga tgacccagtc tccatcctcc ctgtctgcat ctgtaggaga cagagtcacc 60
   atcacttgcc aggcgagtca ggacattagc aactatttaa attggtatca gcagaaacca 120
   gggaaagccc ctaagctcct gatctacgat gcatccaatt tggaaacagg ggtcccatca 180
   aggttcagtg gaagtggatc tgggacagat tttactttca ccatcagcag cctgcagcct 240
   gaagatattg caacatatta ctgtcaacag tatgataatc tccctcc 287
<210> 114
   <211> 287
   <212> DNA
   <213> homo sapiens;
<400> 114
   gacatccagt tgacccagtc tccatcctcc ctgtctgcat ctgtaggaga cagagtcacc 60
   atcacttgcc gggtgagtca gggcattagc agttatttaa attggtatcg gcagaaacca 120
   gggaaagttc ctaagctcct gatctatagt gcatccaatt tgcaatctgg agtcccatct 180
   cggttcagtg gcagtggatc tgggacagat ttcactctca ctatcagcag cctgcagcct 240
   gaagatgttg caacttatta cggtcaacgg acttacaatg cccctcc 287
<210> 115
   <211> 287
   <212> DNA
   <213> homo sapiens;
<400> 115
   gacatccagt tgacccagtc tccatcctcc ctgtctgcat ctgtaggaga cagagtcacc 60
   atcacttgcc gggtgagtca gggcattagc agttatttaa attggtatcg gcagaaacca 120
   gggaaagttc ctaagctcct gatctatagt gcatccaatt tgcaatctgg agtcccatct 180
   cggttcagtg gcagtggatc tgggacagat ttcactctca ctatcagcag cctgcagcct 240
   gaagatgttg caacttatta cggtcaacgg acttacaatg cccctcc 287
<210> 116
   <211> 287
   <212> DNA
   <213> homo sapiens;
<400> 116
   gacatccaga tgacccagtc tccatcctcc ctgtctgcat ctgtaggaga cagagtcacc 60
   atcacttgcc gggcaagtca gagcattagc agctatttaa attggtatca gcagaaacca 120
   gggaaagccc ctaagctcct gatctatgct gcatccagtt tgcaaagtgg ggtcccatca 180
   aggttcagtg gcagtggatc tgggacagat ttcactctca ccatcagcag tctgcaacct 240
   gaagattttg caacttacta ctgtcaacag agttacagta cccctcc 287
<210> 117
   <211> 287
   <212> DNA
   <213> homo sapiens;
<400> 117
   gacatccaga tgacccagtc tccatcctcc ctgtctgcat ctgtaggaga cagagtcacc 60
   atcacttgcc gggcaagtca gagcattagc agctatttaa attggtatca gcagaaacca 120
   gggaaagccc ctaagctcct gatctatgct gcatccagtt tgcaaagtgg ggtcccatca 180
   aggttcagtg gcagtggatc tgggacagat ttcactctca ccatcagcag tctgcaacct 240
   gaagattttg caacttacta ctgtcaacag agttacagta cccctcc 287
<210> 118
   <211> 287
   <212> DNA
   <213> homo sapiens;
<400> 118
   gacatccaga tgatccagtc tccatctttc ctgtctgcat ctgtaggaga cagagtcagt 60
   atcatttgct gggcaagtga gggcattagc agtaatttag cctggtatct gcagaaacca 120
   gggaaatccc ctaagctctt cctctatgat gcaaaagatt tgcaccctgg ggtctcatcg 180
   aggttcagtg gcaggggatc tgggacggat ttcactctca ccatcatcag cctgaagcct 240
   gaagattttg cagcttatta ctgtaaacag gacttcagtt accctcc 287
<210> 119
   <211> 287
   <212> DNA
   <213> homo sapiens;
<400> 119
   gccatccgga tgacccagtc tccattctcc ctgtctgcat ctgtaggaga cagagtcacc 60
   atcacttgct gggccagtca gggcattagc agttatttag cctggtatca gcaaaaacca 120
   gcaaaagccc ctaagctctt catctattat gcatccagtt tgcaaagtgg ggtcccatca 180
   aggttcagcg gcagtggatc tgggacggat tacactctca ccatcagcag cctgcagcct 240
   gaagattttg caacttatta ctgtcaacag tattatagta cccctcc 287
<210> 120
   <211> 302
   <212> DNA
   <213> homo sapiens;
<400> 120
   gatattgtga tgacccagac tccactctcc tcacctgtca cccttggaca gccggcctcc 60
   atctcctgca ggtctagtca aagcctcgta cacagtgatg gaaacaccta cttgagttgg 120
   cttcagcaga ggccaggcca gcctccaaga ctcctaattt ataagatttc taaccggttc 180
   tctggggtcc cagacagatt cagtggcagt ggggcaggga cagatttcac actgaaaatc 240
   agcagggtgg aagctgagga tgtcggggtt tattactgca tgcaagctac acaatttcct 300
   ca 302
<210> 121
   <211> 302
   <212> DNA
   <213> homo sapiens;
<400> 121
   gatattgtga tgacccagac tccactctcc tcgcctgtca cccttggaca gccggcctcc 60
   atctccttca ggtctagtca aagcctcgta cacagtgatg gaaacaccta cttgagttgg 120
   cttcagcaga ggccaggcca gcctccaaga ctcctaattt ataaggtttc taaccggttc 180
   tctggggtcc cagacagatt cagtggcagt ggggcaggga cagatttcac actgaaaatc 240
   agcagggtgg aagctgagga tgtcggggtt tattactgca cgcaagctac acaatttcct 300
   ca 302
<210> 122
   <211> 302
   <212> DNA
   <213> homo sapiens;
<400> 122
   gatattgtga tgactcagtc tccactctcc ctgcccgtca cccctggaga gccggcctcc 60
   atctcctgca ggtctagtca gagcctcctg catagtaatg gatacaacta tttggattgg 120
   tacctgcaga agccagggca gtctccacag ctcctgatct atttgggttc taatcgggcc 180
   tccggggtcc ctgacaggtt cagtggcagt ggatcaggca cagattttac actgaaaatc 240
   agcagagtgg aggctgagga tgttggggtt tattactgca tgcaagctct acaaactcct 300
   cc 302
<210> 123
   <211> 302
   <212> DNA
   <213> homo sapiens;
<400> 123
   gatattgtga tgactcagtc tccactctcc ctgcccgtca cccctggaga gccggcctcc 60
   atctcctgca ggtctagtca gagcctcctg catagtaatg gatacaacta tttggattgg 120
   tacctgcaga agccagggca gtctccacag ctcctgatct atttgggttc taatcgggcc 180
   tccggggtcc ctgacaggtt cagtggcagt ggatcaggca cagattttac actgaaaatc 240
   agcagagtgg aggctgagga tgttggggtt tattactgca tgcaagctct acaaactcct 300
   cc 302
<210> 124
   <211> 302
   <212> DNA
   <213> homo sapiens;
<400> 124
   gatattgtga tgacccagac tccactctct ctgtccgtca cccctggaca gccggcctcc 60
   atctcctgca agtctagtca gagcctcctg catagtgatg gaaagaccta tttgtattgg 120
   tacctgcaga agccaggcca gtctccacag ctcctgatct atgaagtttc cagccggttc 180
   tctggagtgc cagataggtt cagtggcagc gggtcaggga cagatttcac actgaaaatc 240
   agccgggtgg aggctgagga tgttggggtt tattactgaa tgcaaggtat acaccttcct 300
   cc 302
<210> 125
   <211> 302
   <212> DNA
   <213> homo sapiens;
<400> 125
   gatattgtga tgacccagac tccactctct ctgtccgtca cccctggaca gccggcctcc 60
   atctcctgca agtctagtca gagcctcctg catagtgatg gaaagaccta tttgtattgg 120
   tacctgcaga agccaggcca gcctccacag ctcctgatct atgaagtttc caaccggttc 180
   tctggagtgc cagataggtt cagtggcagc gggtcaggga cagatttcac actgaaaatc 240
   agccgggtgg aggctgagga tgttggggtt tattactgca tgcaaagtat acagcttcct 300
   cc 302
<210> 126
   <211> 302
   <212> DNA
   <213> homo sapiens;
<400> 126
   gatgttgtga tgactcagtc tccactctcc ctgcccgtca cccttggaca gccggcctcc 60
   atctcctgca ggtctagtca aagcctcgta tacagtgatg gaaacaccta cttgaattgg 120
   tttcagcaga ggccaggcca atctccaagg cgcctaattt ataaggtttc taaccgggac 180
   tctggggtcc cagacagatt cagcggcagt gggtcaggca ctgatttcac actgaaaatc 240
   agcagggtgg aggctgagga tgttggggtt tattactgca tgcaaggtac acactggcct 300
   cc 302
<210> 127
   <211> 302
   <212> DNA
   <213> homo sapiens;
<400> 127
   gatgttgtga tgactcagtc tccactctcc ctgcccgtca cccttggaca gccggcctcc 60
   atctcctgca ggtctagtca aagcctcgta tacagtgatg gaaacaccta cttgaattgg 120
   tttcagcaga ggccaggcca atctccaagg cgcctaattt ataaggtttc taactgggac 180
   tctggggtcc cagacagatt cagcggcagt gggtcaggca ctgatttcac actgaaaatc 240
   agcagggtgg aggctgagga tgttggggtt tattactgca tgcaaggtac acactggcct 300
   cc 302
<210> 128
   <211> 305
   <212> DNA
   <213> homo sapiens;
<400> 128
   gatattgtga tgacccagac tccactctcc ctgcccgtca cccctggaga gccggcctcc 60
   atctcctgca ggtctagtca gagcctcttg gatagtgatg atggaaacac ctatttggac 120
   tggtacctgc agaagccagg gcagtctcca cagctcctga tctatacgct ttcctatcgg 180
   gcctctggag tcccagacag gttcagtggc agtgggtcag gcactgattt cacactgaaa 240
   atcagcaggg tggaggctga ggatgttgga gtttattact gcatgcaacg tatagagttt 300
   ccttc 305
<210> 129
   <211> 305
   <212> DNA
   <213> homo sapiens;
<400> 129
   gatattgtga tgacccagac tccactctcc ctgcccgtca cccctggaga gccggcctcc 60
   atctcctgca ggtctagtca gagcctcttg gatagtgatg atggaaacac ctatttggac 120
   tggtacctgc agaagccagg gcagtctcca cagctcctga tctatacgct ttcctatcgg 180
   gcctctggag tcccagacag gttcagtggc agtgggtcag gcactgattt cacactgaaa 240
   atcagcaggg tggaggctga ggatgttgga gtttattact gcatgcaacg tatagagttt 300
   ccttc 305
<210> 130
   <211> 290
   <212> DNA
   <213> homo sapiens;
<400> 130
   gaaattgtaa tgacacagtc tccacccacc ctgtctttgt ctccagggga aagagtcacc 60
   ctctcctgca gggccagtca gagtgttagc agcagctact taacctggta tcagcagaaa 120
   cctggccagg cgcccaggct cctcatctat ggtgcatcca ccagggccac tagcatccca 180
   gccaggttca gtggcagtgg gtctgggaca gacttcactc tcaccatcag cagcctgcag 240
   cctgaagatt ttgcagttta ttactgtcag caggatcata acttacctcc 290
<210> 131
   <211> 290
   <212> DNA
   <213> homo sapiens;
<400> 131
   gaaattgtaa tgacacagtc tccacccacc ctgtctttgt ctccagggga aagagtcacc 60
   ctctcctgca gggccagtca gagtgttagc agcagctact taacctggta tcagcagaaa 120
   cctggccagg cgcccaggct cctcatctat ggtgcatcca ccagggccac tagcatccca 180
   gccaggttca gtggcagtgg gtctgggaga gacttcactc tcaccatcag cagcctgcag 240
   cctgaagatt ttgcagttta ttactgtcag caggatcata acttacctcc 290
<210> 132
   <211> 290
   <212> DNA
   <213> homo sapiens;
<400> 132
   gaaattgtaa tgacacagtc tccagccacc ctgtctttgt ctccagggga aagagccacc 60
   ctctcctgca gggccagtca gagtgttagc agcagctact tatcctggta ccagcagaaa 120
   cctgggcagg ctcccaggct cctcatctat ggtgcatcca ccagggccac tggcatccca 180
   gccaggttca gtggcagtgg gtctgggaca gacttcactc tcaccatcag cagcctgcag 240
   cctgaagatt ttgcagttta ttactgtcag caggattata acttacctcc 290
<210> 133
   <211> 287
   <212> DNA
   <213> homo sapiens;
<400> 133
   gaaattgtgt tgacacagtc tccagccacc ctgtctttgt ctccagggga aagagccacc 60
   ctctcctgca gggccagtca gagtgttagc agctacttag cctggtacca acagaaacct 120
   ggccaggctc ccaggctcct catctatgat gcatccaaca gggccactgg catcccagcc 180
   aggttcagtg gcagtgggtc tgggacagac ttcactctca ccatcagcag cctagagcct 240
   gaagattttg cagtttatta ctgtcagcag cgtagcaact ggcctcc 287
<210> 134
   <211> 287
   <212> DNA
   <213> homo sapiens;
<400> 134
   gaaattgtgt tgacacagtc tccagccacc ctgtctttgt ctccagggga aagagccacc 60
   ctctcctgca gggccagtca gggtgttagc agctacttag cctggtacca gcagaaacct 120
   ggccaggctc ccaggctcct catctatgat gcatccaaca gggccactgg catcccagcc 180
   aggttcagtg gcagtgggcc tgggacagac ttcactctca ccatcagcag cctagagcct 240
   gaagattttg cagtttatta ctgtcagcag cgtagcaact ggcatcc 287
<210> 135
   <211> 287
   <212> DNA
   <213> homo sapiens;
<400> 135
   gaaatagtga tgacgcagtc tccagccacc ctgtctgtgt ctccagggga aagagccacc 60
   ctctcctgca gggccagtca gagtgttagc agcaacttag cctggtacca gcagaaacct 120
   ggccaggctc ccaggctcct catctatggt gcatccacca gggccactgg tatcccagcc 180
   aggttcagtg gcagtgggtc tgggacagag ttcactctca ccatcagcag cctgcagtct 240
   gaagattttg cagtttatta ctgtcagcag tataataact ggcctcc 287
<210> 136
   <211> 287
   <212> DNA
   <213> homo sapiens;
<400> 136
   gaaatagtga tgacgcagtc tccagccacc ctgtctgtgt ctccagggga aagagccacc 60
   ctctcctgca gggccagtca gagtgttagc agcaacttag cctggtacca gcagaaacct 120
   ggccaggctc ccaggctcct catctatggt gcatccacca gggccactgg catcccagcc 180
   aggttcagtg gcagtgggtc tgggacagag ttcactctca ccatcagcag cctgcagtct 240
   gaagattttg cagtttatta ctgtcagcag tataataact ggcctcc 287
<210> 137
   <211> 290
   <212> DNA
   <213> homo sapiens;
<400> 137
   gaaattgtgt tgacgcagtc tccaggcacc ctgtctttgt ctccagggga aagagccacc 60
   ctctcctgca gggccagtca gagtgttagc agcagctact tagcctggta ccagcagaaa 120
   cctggccagg ctcccaggct cctcatctat ggtgcatcca gcagggccac tggcatccca 180
   gacaggttca gtggcagtgg gtctgggaca gacttcactc tcaccatcag cagactggag 240
   cctgaagatt ttgcagtgta ttactgtcag cagtatggta gctcacctcc 290
<210> 138
   <211> 290
   <212> DNA
   <213> homo sapiens;
<400> 138
   gaaattgtgt tgacgcagtc tccagccacc ctgtctttgt ctccagggga aagagccacc 60
   ctctcctgcg gggccagtca gagtgttagc agcagctact tagcctggta ccagcagaaa 120
   cctggcctgg cgcccaggct cctcatctat gatgcatcca gcagggccac tggcatccca 180
   gacaggttca gtggcagtgg gtctgggaca gacttcactc tcaccatcag cagactggag 240
   cctgaagatt ttgcagtgta ttactgtcag cagtatggta gctcacctcc 290
<210> 139
   <211> 305
   <212> DNA
   <213> homo sapiens;
<400> 139
   gacatcgtga tgacccagtc tccagactcc ctggctgtgt ctctgggcga gagggccacc 60
   atcaactgca agtccagcca gagtgtttta tacagctcca acaataagaa ctacttagct 120
   tggtaccagc agaaaccagg acagcctcct aagctgctca tttactgggc atctacccgg 180
   gaatccgggg tccctgaccg attcagtggc agcgggtctg ggacagattt cactctcacc 240
   atcagcagcc tgcaggctga agatgtggca gtttattact gtcagcaata ttatagtact 300
   cctcc 305
<210> 140
   <211> 287
   <212> DNA
   <213> homo sapiens;
<400> 140
   gaaacgacac tcacgcagtc tccagcattc atgtcagcga ctccaggaga caaagtcaac 60
   atctcctgca aagccagcca agacattgat gatgatatga actggtacca acagaaacca 120
   ggagaagctg ctattttcat tattcaagaa gctactactc tcgttcctgg aatcccacct 180
   cgattcagtg gcagcgggta tggaacagat tttaccctca caattaataa catagaatct 240
   gaggatgctg catattactt ctgtctacaa catgataatt tccctct 287
<210> 141
   <211> 287
   <212> DNA
   <213> homo sapiens;
<400> 141
   gaaattgtgc tgactcagtc tccagacttt cagtctgtga ctccaaagga gaaagtcacc 60
   atcacctgcc gggccagtca gagcattggt agtagcttac actggtacca gcagaaacca 120
   gatcagtctc caaagctcct catcaagtat gcttcccagt ccttctcagg ggtcccctcg 180
   aggttcagtg gcagtggatc tgggacagat ttcaccctca ccatcaatag cctggaagct 240
   gaagatgctg caacgtatta ctgtcatcag agtagtagtt tacctca 287
<210> 142
   <211> 287
   <212> DNA
   <213> homo sapiens;
<400> 142
   gaaattgtgc tgactcagtc tccagacttt cagtctgtga ctccaaagga gaaagtcacc 60
   atcacctgcc gggccagtca gagcattggt agtagcttac actggtacca gcagaaacca 120
   gatcagtctc caaagctcct catcaagtat gcttcccagt ccttctcagg ggtcccctcg 180
   aggttcagtg gcagtggatc tgggacagat ttcaccctca ccatcaatag cctggaagct 240
   gaagatgctg caacgtatta ctgtcatcag agtagtagtt tacctca 287
<210> 143
   <211> 287
   <212> DNA
   <213> homo sapiens;
<400> 143
   gatgttgtga tgacacagtc tccagctttc ctctctgtga ctccagggga gaaagtcacc 60
   atcacctgcc aggccagtga aggcattggc aactacttat actggtacca gcagaaacca 120
   gatcaagccc caaagctcct catcaagtat gcttcccagt ccatctcagg ggtcccctcg 180
   aggttcagtg gcagtggatc tgggacagat ttcaccttta ccatcagtag cctggaagct 240
   gaagatgctg caacatatta ctgtcagcag ggcaataagc accctca 287
<210> 144
   <211> 296
   <212> DNA
   <213> homo sapiens;
<400> 144
   cagtctgtgc tgactcagcc accctcggtg tctgaagccc ccaggcagag ggtcaccatc 60
   tcctgttctg gaagcagctc caacatcgga aataatgctg taaactggta ccagcagctc 120
   ccaggaaagg ctcccaaact cctcatctat tatgatgatc tgctgccctc aggggtctct 180
   gaccgattct ctggctccaa gtctggcacc tcagcctccc tggccatcag tgggctccag 240
   tctgaggatg aggctgatta ttactgtgca gcatgggatg acagcctgaa tggtcc 296
<210> 145
   <211> 299
   <212> DNA
   <213> homo sapiens;
<400> 145
   cagtctgtgc tgacgcagcc gccctcagtg tctggggccc cagggcagag ggtcaccatc 60
   tcctgcactg ggagcagctc caacatcggg gcaggttatg atgtacactg gtaccagcag 120
   cttccaggaa cagcccccaa actcctcatc tatggtaaca gcaatcggcc ctcaggggtc 180
   cctgaccgat tctctggctc caagtctggc acctcagcct ccctggccat cactgggctc 240
   caggctgagg atgaggctga ttattactgc cagtcctatg acagcagcct gagtggttc 299
<210> 146
   <211> 296
   <212> DNA
   <213> homo sapiens;
<400> 146
   cagtctgtgt tgacgcagcc gccttcagtg tctgcggccc caggacagaa ggtcaccatc 60
   tcctgctctg gaagcagctc cgacatgggg aattatgcgg tatcctggta ccagcagctc 120
   ccaggaacag cccccaaact cctcatctat gaaaataata agcgaccctc agggattcct 180
   gaccgattct ctggctccaa gtctggcacc tcagccaccc tgggcatcac tggcctctgg 240
   cctgaggacg aggccgatta ttactgctta gcatgggata ccagcccgag agcttg 296
<210> 147
   <211> 296
   <212> DNA
   <213> homo sapiens;
<400> 147
   cagtctgtgc tgactcagcc accctcagcg tctgggaccc ccgggcagag ggtcaccatc 60
   tcttgttctg gaagcagctc caacatcgga agtaatactg taaactggta ccagcagctc 120
   ccaggaacgg cccccaaact cctcatctat agtaataatc agcggccctc aggggtccct 180
   gaccgattct ctggctccaa gtctggcacc tcagcctccc tggccatcag tgggctccag 240
   tctgaggatg aggctgatta ttactgtgca gcatgggatg acagcctgaa tggtcc 296
<210> 148
   <211> 296
   <212> DNA
   <213> homo sapiens;
<400> 148
   cagtctgtgc tgactcagcc accctcagcg tctgggaccc ccgggcagag ggtcaccatc 60
   tcttgttctg gaagcagctc caacatcgga agtaattatg tatactggta ccagcagctc 120
   ccaggaacgg cccccaaact cctcatctat aggaataatc agcggccctc aggggtccct 180
   gaccgattct ctggctccaa gtctggcacc tcagcctccc tggccatcag tgggctccgg 240
   tccgaggatg aggctgatta ttactgtgca gcatgggatg acagcctgag tggtcc 296
<210> 149
   <211> 299
   <212> DNA
   <213> homo sapiens;
<400> 149
   cagtctgtgc tgacgcagcc gccctcagtg tctggggccc cagggcagag ggtcaccatc 60
   tcctgcactg ggagcagctc caacattggg gcgggttatg ttgtacattg gtaccagcag 120
   cttccaggaa cagcccccaa actcctcatc tatggtaaca gcaatcggcc ctcaggggtc 180
   cctgaccaat tctctggctc caagtctggc acctcagcct ccctggccat cactggactc 240
   cagtctgagg atgaggctga ttattactgc aaagcatggg ataacagcct gaatgctca 299
<210> 150
   <211> 296
   <212> DNA
   <213> homo sapiens;
<400> 150
   cagtctgtgt tgacgcagcc gccctcagtg tctgcggccc caggacagaa ggtcaccatc 60
   tcctgctctg gaagcagctc caacattggg aataattatg tatcctggta ccagcagctc 120
   ccaggaacag cccccaaact cctcatttat gacaataata agcgaccctc agggattcct 180
   gaccgattct ctggctccaa gtctggcacg tcagccaccc tgggcatcac cggactccag 240
   actggggacg aggccgatta ttactgcgga acatgggata gcagcctgag tgctgg 296
<210> 151
   <211> 297
   <212> DNA
   <213> homo sapiens;
<400> 151
   cagtctgccc tgactcagcc tccctccgcg tccgggtctc ctggacagtc agtcaccatc 60
   tcctgcactg gaaccagcag tgacgttggt ggttataact atgtctcctg gtaccaacag 120
   cacccaggca aagcccccaa actcatgatt tatgaggtca gtaagcggcc ctcaggggtc 180
   cctgatcgct tctctggctc caagtctggc aacacggcct ccctgaccgt ctctgggctc 240
   caggctgagg atgaggctga ttattactgc agctcatatg caggcagcaa caatttc 297
<210> 152
   <211> 297
   <212> DNA
   <213> homo sapiens;
<400> 152
   cagtctgccc tgactcagcc tcgctcagtg tccgggtctc ctggacagtc agtcaccatc 60
   tcctgcactg gaaccagcag tgatgttggt ggttataact atgtctcctg gtaccaacag 120
   cacccaggca aagcccccaa actcatgatt tatgatgtca gtaagcggcc ctcaggggtc 180
   cctgatcgct tctctggctc caagtctggc aacacggcct ccctgaccat ctctgggctc 240
   caggctgagg atgaggctga ttattactgc tgctcatatg caggcagcta cactttc 297
<210> 153
   <211> 297
   <212> DNA
   <213> homo sapiens;
<400> 153
   cagtctgccc tgactcagcc tgcctccgtg tctgggtctc ctggacagtc gatcaccatc 60
   tcctgcactg gaaccagcag tgacgttggt ggttataact atgtctcctg gtaccaacag 120
   cacccaggca aagcccccaa actcatgatt tatgaggtca gtaatcggcc ctcaggggtt 180
   tctaatcgct tctctggctc caagtctggc aacacggcct ccctgaccat ctctgggctc 240
   caggctgagg acgaggctga ttattactgc agctcatata caagcagcag cactctc 297
<210> 154
   <211> 297
   <212> DNA
   <213> homo sapiens;
<400> 154
   cagtctgccc tgactcagcc tccctccgtg tccgggtctc ctggacagtc agtcaccatc 60
   tcctgcactg gaaccagcag tgacgttggt agttataacc gtgtctcctg gtaccagcag 120
   cccccaggca cagcccccaa actcatgatt tatgaggtca gtaatcggcc ctcaggggtc 180
   cctgatcgct tctctgggtc caagtctggc aacacggcct ccctgaccat ctctgggctc 240
   caggctgagg acgaggctga ttattactgc agcttatata caagcagcag cactttc 297
<210> 155
   <211> 298
   <212> DNA
   <213> homo sapiens;
<400> 155
   cagtctgccc tgactcagcc tgcctccgtg tctgggtctc ctggacagtc gatcaccatc 60
   tcctgcactg gaaccagcag tgatgttggg agttataacc ttgtctcctg gtaccaacag 120
   cacccaggca aagcccccaa actcatgatt tatgagggca gtaagcggcc ctcaggggtt 180
   tctaatcgct tctctggctc caagtctggc aacacggcct ccctgacaat ctctgggctc 240
   caggctgagg acgaggctga ttattactgc tgctcatatg caggtagtag cactttac 298
<210> 156
   <211> 297
   <212> DNA
   <213> homo sapiens;
<400> 156
   caatctgccc tgactcagcc tccttttgtg tccggggctc ctggacagtc ggtcaccatc 60
   tcctgcactg gaaccagcag tgacgttggg gattatgatc atgtcttctg gtaccaaaag 120
   cgtctcagca ctacctccag actcctgatt tacaatgtca atactcggcc ttcagggatc 180
   tctgacctct tctcaggctc caagtctggc aacatggctt ccctgaccat ctctgggctc 240
   aagtccgagg ttgaggctaa ttatcactgc agcttatatt caagtagtta cactttc 297
<210> 157
   <211> 285
   <212> DNA
   <213> homo sapiens;
<400> 157
   tcctatgagc tgactcagcc accctcagtg tccgtgtccc caggacagac agccagcatc 60
   acctgctctg gagataaatt gggggataaa tatgcttgct ggtatcagca gaagccaggc 120
   cagtcccctg tgctggtcat ctatcaagat agcaagcggc cctcagggat ccctgagcga 180
   ttctctggct ccaactctgg gaacacagcc actctgacca tcagcgggac ccaggctatg 240
   gatgaggctg actattactg tcaggcgtgg gacagcagca ctgca 285
<210> 158
   <211> 290
   <212> DNA
   <213> homo sapiens;
<400> 158
   tcctatgagc tgacacagcc accctcggtg tcagtgtccc caggacaaac ggccaggatc 60
   acctgctctg gagatgcatt gccaaaaaaa tatgcttatt ggtaccagca gaagtcaggc 120
   caggcccctg tgctggtcat ctatgaggac agcaaacgac cctccgggat ccctgagaga 180
   ttctctggct ccagctcagg gacaatggcc accttgacta tcagtggggc ccaggtggag 240
   gatgaagctg actactactg ttactcaaca gacagcagtg gtaatcatag 290
<210> 159
   <211> 290
   <212> DNA
   <213> homo sapiens;
<400> 159
   tcctatgagc tgactcagcc acactcagtg tcagtggcca cagcacagat ggccaggatc 60
   acctgtgggg gaaacaacat tggaagtaaa gctgtgcact ggtaccagca aaagccaggc 120
   caggaccctg tgctggtcat ctatagcgat agcaaccggc cctcagggat ccctgagcga 180
   ttctctggct ccaacccagg gaacaccacc accctaacca tcagcaggat cgaggctggg 240
   gatgaggctg actattactg tcaggtgtgg gacagtagta gtgatcatcc 290
<210> 160
   <211> 290
   <212> DNA
   <213> homo sapiens;
<400> 160
   tcctatgagc tgacacagcc accctcggtg tcagtgtccc taggacagat ggccaggatc 60
   acctgctctg gagaagcatt gccaaaaaaa tatgcttatt ggtaccagca gaagccaggc 120
   cagttccctg tgctggtgat atataaagac agcgagaggc cctcagggat ccctgagcga 180
   ttctctggct ccagctcagg gacaatagtc acattgacca tcagtggagt ccaggcagaa 240
   gacgaggctg actattactg tctatcagca gacagcagtg gtacttatcc 290
<210> 161
   <211> 290
   <212> DNA
   <213> homo sapiens;
<400> 161
   tcttctgagc tgactcagga ccctgctgtg tctgtggcct tgggacagac agtcaggatc 60
   acatgccaag gagacagcct cagaagctat tatgcaagct ggtaccagca gaagccagga 120
   caggcccctg tacttgtcat ctatggtaaa aacaaccggc cctcagggat cccagaccga 180
   ttctctggct ccagctcagg aaacacagct tccttgacca tcactggggc tcaggcggaa 240
   gatgaggctg actattactg taactcccgg gacagcagtg gtaaccatct 290
<210> 162
   <211> 290
   <212> DNA
   <213> homo sapiens;
<400> 162
   tcctatgtgc tgactcagcc accctcagtg tcagtggccc caggaaagac ggccaggatt 60
   acctgtgggg gaaacaacat tggaagtaaa agtgtgcact ggtaccagca gaagccaggc 120
   caggcccctg tgctggtcat ctattatgat agcgaccggc cctcagggat ccctgagcga 180
   ttctctggct ccaactctgg gaacacggcc accctgacca tcagcagggt cgaagccggg 240
   gatgaggccg actattactg tcaggtgtgg gacagtagta gtgatcatcc 290
<210> 163
   <211> 284
   <212> DNA
   <213> homo sapiens;
<400> 163
   tcctatgagc tgacacagct accctcggtg tcagtgtccc caggacagac agccaggatc 60
   acctgctctg gagatgtact gggggaaaat tatgctgact ggtaccagca gaagccaggc 120
   caggcccctg agttggtgat atacgaagat agtgagcggt accctggaat ccctgaacga 180
   ttctctgggt ccacctcagg gaacacgacc accctgacca tcagcagggt cctgaccgaa 240
   gacgaggctg actattactg tttgtctggg gatgaggaca atcc 284
<210> 164
   <211> 290
   <212> DNA
   <213> homo sapiens;
<400> 164
   tcctatgagc tgatgcagcc accctcggtg tcagtgtccc caggacagac ggccaggatc 60
   acctgctctg gagatgcatt gccaaagcaa tatgcttatt ggtaccagca gaagccaggc 120
   caggcccctg tgctggtgat atataaagac agtgagaggc cctcagggat ccctgagcga 180
   ttctctggct ccagctcagg gacaacagtc acgttgacca tcagtggagt ccaggcagaa 240
   gatgaggctg actattactg tcaatcagca gacagcagtg gtacttatcc 290
<210> 165
   <211> 284
   <212> DNA
   <213> homo sapiens;
<400> 165
   tcctatgagc tgacacagcc atcctcagtg tcagtgtctc cgggacagac agccaggatc 60
   acctgctcag gagatgtact ggcaaaaaaa tatgctcggt ggttccagca gaagccaggc 120
   caggcccctg tgctggtgat ttataaagac agtgagcggc cctcagggat ccctgagcga 180
   ttctccggct ccagctcagg gaccacagtc accttgacca tcagcggggc ccaggttgag 240
   gatgaggctg actattactg ttactctgcg gctgacaaca atct 284
<210> 166
   <211> 284
   <212> DNA
   <213> homo sapiens;
<400> 166
   tcctctgggc caactcaggt gcctgcagtg tctgtggcct tgggacaaat ggccaggatc 60
   acctgccagg gagacagcat ggaaggctct tatgaacact ggtaccagca gaagccaggc 120
   caggcccccg tgctggtcat ctatgatagc agtgaccggc cctcaaggat ccctgagcga 180
   ttctctggct ccaaatcagg caacacaacc accctgacca tcactggggc ccaggctgag 240
   gatgaggctg attattacta tcagttgata gacaaccatg ctac 284
<210> 167
   <211> 314
   <212> DNA
   <213> homo sapiens;
<400> 167
   ctgcctgtgc tgactcagcc cccgtctgca tctgccttgc tgggagcctc gatcaagctc 60
   acctgcaccc taagcagtga gcacagcacc tacaccatcg aatggtatca acagagacca 120
   gggaggtccc cccagtatat aatgaaggtt aagagtgatg gcagccacag caagggggac 180
   gggatccccg atcgcttcat gggctccagt tctggggctg accgctacct caccttctcc 240
   aacctccagt ctgacgatga ggctgagtat cactgtggag agagccacac gattgatggc 300
   caagtcggtt gagc 314
<210> 168
   <211> 297
   <212> DNA
   <213> homo sapiens;
<400> 168
   cagcctgtgc tgactcaatc atcctctgcc tctgcttccc tgggatcctc ggtcaagctc 60
   acctgcactc tgagcagtgg gcacagtagc tacatcatcg catggcatca gcagcagcca 120
   gggaaggccc ctcggtactt gatgaagctt gaaggtagtg gaagctacaa caaggggagc 180
   ggagttcctg atcgcttctc aggctccagc tctggggctg accgctacct caccatctcc 240
   aacctccagt tagaggatga ggctgattat tactgtgaga cctgggacag taacact 297
<210> 169
   <211> 299
   <212> DNA
   <213> homo sapiens;
<400> 169
   cagcttgtgc tgactcaatc gccctctgcc tctgcctccc tgggagcctc ggtcaagctc 60
   acctgcactc tgagcagtgg gcacagcagc tacgccatcg catggcatca gcagcagcca 120
   gagaagggcc ctcggtactt gatgaagctt aacagtgatg gcagccacag caagggggac 180
   gggatccctg atcgcttctc aggctccagc tctggggctg agcgctacct caccatctcc 240
   agcctccagt ctgaggatga ggctgactat tactgtcaga cctggggcac tggcattca 299
<210> 170
   <211> 312
   <212> DNA
   <213> homo sapiens;
<400> 170
   cagcctgtgc tgactcagcc accttcctcc tccgcatctc ctggagaatc cgccagactc 60
   acctgcacct tgcccagtga catcaatgtt ggtagctaca acatatactg gtaccagcag 120
   aagccaggga gccctcccag gtatctcctg tactactact cagactcaga taagggccag 180
   ggctctggag tccccagccg cttctctgga tccaaagatg cttcagccaa tacagggatt 240
   ttactcatct ccgggctcca gtctgaggat gaggctgact attactgtat gatttggcca 300
   agcaatgctt ct 312
<210> 171
   <211> 312
   <212> DNA
   <213> homo sapiens;
<400> 171
   caggctgtgc tgactcagcc ggcttccctc tctgcatctc ctggagcatc agccagtctc 60
   acctgcacct tgcgcagtgg catcaatgtt ggtacctaca ggatatactg gtaccagcag 120
   aagccaggga gtcctcccca gtatctcctg aggtacaaat cagactcaga taagcagcag 180
   ggctctggag tccccagccg cttctctgga tccaaagatg cttcggccaa tgcagggatt 240
   ttactcatct ctgggctcca gtctgaggat gaggctgact attactgtat gatttggcac 300
   agcagcgctt ct 312
<210> 172
   <211> 312
   <212> DNA
   <213> homo sapiens;
<400> 172
   cagcctgtgc tgactcagcc aacttccctc tcagcatctc ctggagcatc agccagactc        60
   acctgcacct tgcgcagtgg catcaatctt ggtagctaca ggatattctg gtaccagcag       120
   aagccagaga gccctccccg gtatctcctg agctactact cagactcaag taagcatcag       180
   ggctctggag tccccagccg cttctctgga tccaaagatg cttcgagcaa tgcagggatt       240
   ttagtcatct ctgggctcca gtctgaggat gaggctgact attactgtat gatttggcac       300
   agcagtgctt ct                                                           312
<210> 173
   <211> 317
   <212> DNA
   <213> homo sapiens;
<400> 173
   cagcctgtgc tgactcagcc atcttcccat tctgcatctt ctggagcatc agtcagactc        60
   acctgcatgc tgagcagtgg cttcagtgtt ggggacttct ggataaggtg gtaccaacaa       120
   aagccaggga accctccccg gtatctcctg tactaccact cagactccaa taagggccaa       180
   ggctctggag ttcccagccg cttctctgga tccaacgatg catcagccaa tgcagggatt       240
   ctgcgtatct ctgggctcca gcctgaggat gaggctgact attactgtgg tacatggcac       300
   agcaactcta agactca                                                      317
<210> 174
   <211> 296
   <212> DNA
   <213> homo sapiens;
<400> 174
   aattttatgc tgactcagcc ccactctgtg tcggagtctc cggggaagac ggtaaccatc        60
   tcctgcaccc gcagcagtgg cagcattgcc agcaactatg tgcagtggta ccagcagcgc       120
   ccgggcagtt cccccaccac tgtgatctat gaggataacc aaagaccctc tggggtccct       180
   gatcggttct ctggctccat cgacagctcc tccaactctg cctccctcac catctctgga       240
   ctgaagactg aggacgaggc tgactactac tgtcagtctt atgatagcag caatca           296
<210> 175
   <211> 294
   <212> DNA
   <213> homo sapiens;
<400> 175
   cagactgtgg tgactcagga gccctcactg actgtgtccc caggagggac agtcactctc 60
   acctgtgctt ccagcactgg agcagtcacc agtggttact atccaaactg gttccagcag 120
   aaacctggac aagcacccag ggcactgatt tatagtacaa gcaacaaaca ctcctggacc 180
   cctgcccggt tctcaggctc cctccttggg ggcaaagctg ccctgacact gtcaggtgtg 240
   cagcctgagg acgaggctga gtattactgc ctgctctact atggtggtgc tcag 294
<210> 176
   <211> 294
   <212> DNA
   <213> homo sapiens;
<400> 176
   caggctgtgg tgactcagga gccctcactg actgtgtccc caggagggac agtcactctc 60
   acctgtggct ccagcactgg agctgtcacc agtggtcatt atccctactg gttccagcag 120
   aagcctggcc aagcccccag gacactgatt tatgatacaa gcaacaaaca ctcctggaca 180
   cctgcccggt tctcaggctc cctccttggg ggcaaagctg ccctgaccct ttcgggtgcg 240
   cagcctgagg atgaggctga gtattactgc ttgctctcct atagtggtgc tcgg 294
<210> 177
   <211> 296
   <212> DNA
   <213> homo sapiens;
<400> 177
   cagactgtgg tgacccagga gccatcgttc tcagtgtccc ctggagggac agtcacactc 60
   acttgtggct tgagctctgg ctcagtctct actagttact accccagctg gtaccagcag 120
   accccaggcc aggctccacg cacgctcatc tacagcacaa acactcgctc ttctggggtc 180
   cctgatcgct tctctggctc catccttggg aacaaagctg ccctcaccat cacgggggcc 240
   caggcagatg atgaatctga ttattactgt gtgctgtata tgggtagtgg catttc 296
<210> 178
   <211> 317
   <212> DNA
   <213> homo sapiens;
<400> 178
   cagcctgtgc tgactcagcc accttctgca tcagcctccc tgggagcctc ggtcacactc 60
   acctgcaccc tgagcagcgg ctacagtaat tataaagtgg actggtacca gcagagacca 120
   gggaagggcc cccggtttgt gatgcgagtg ggcactggtg ggattgtggg atccaagggg 180
   gatggcatcc ctgatcgctt ctcagtcttg ggctcaggcc tgaatcggta cctgaccatc 240
   aagaacatcc aggaagagga tgagagtgac taccactgtg gggcagacca tggcagtggg 300
   agcaacttcg tgtaacc 317
<210> 179
   <211> 296
   <212> DNA
   <213> homo sapiens;
<400> 179
   caggcagggc tgactcagcc accctcggtg tccaagggct tgagacagac cgccacactc 60
   acctgcactg ggaacagcaa caatgttggc aaccaaggag cagcttggct gcagcagcac 120
   cagggccacc ctcccaaact cctatcctac aggaataaca accggccctc agggatctca 180
   gagagattat ctgcatccag gtcaggaaac acagcctccc tgaccattac tggactccag 240
   cctgaggacg aggctgacta ttactgctca gcatgggaca gcagcctcag tgctca 296
<210> 180
   <211> 312
   <212> DNA
   <213> homo sapiens;
<400> 180
   cggcccgtgc tgactcagcc gccctctctg tctgcatccc cgggagcaac agccagactc 60
   ccctgcaccc tgagcagtga cctcagtgtt ggtggtaaaa acatgttctg gtaccagcag 120
   aagccaggga gctctcccag gttattcctg tatcactact cagactcaga caagcagctg 180
   ggacctgggg tccccagtcg agtctctggc tccaaggaga cctcaagtaa cacagcgttt 240
   ttgctcatct ctgggctcca gcctgaggac gaggccgatt attactgcca ggtgtacgaa 300
   agtagtgcta at 312
<210> 181
   <211> 38
   <212> DNA
   <213> homo sapiens;
<400> 181
   gtggacgttc ggccaaggga ccaaggtgga aatcaaac 38
<210> 182
   <211> 39
   <212> DNA
   <213> homo sapiens;
<400> 182
   tgtacacttt tggccagggg accaagctgg agatcaaac 39
<210> 183
   <211> 38
   <212> DNA
   <213> homo sapiens;
<400> 183
   attcactttc ggccctggga ccaaagtgga tatcaaac 38
<210> 184
   <211> 38
   <212> DNA
   <213> homo sapiens;
<400> 184
   gctcactttc ggcggaggga ccaaggtgga gatcaaac 38
<210> 185
   <211> 38
   <212> DNA
   <213> homo sapiens;
<400> 185
   gatcaccttc ggccaaggga cacgactgga gattaaac 38
<210> 186
   <211> 38
   <212> DNA
   <213> homo sapiens;
<400> 186
   ttatgtcttc ggaactggga ccaaggtcac cgtcctag 38
<210> 187
   <211> 38
   <212> DNA
   <213> homo sapiens;
<400> 187
   tgtggtattc ggcggaggga ccaagctgac cgtcctag 38
<210> 188
   <211> 38
   <212> DNA
   <213> homo sapiens;
<400> 188
   tgtggtattc ggcggaggga ccaagctgac cgtcctag 38
<210> 189
   <211> 38
   <212> DNA
   <213> homo sapiens;
<400> 189
   ttttgtattt ggtggaggaa cccagctgat cattttag 38
<210> 190
   <211> 38
   <212> DNA
   <213> homo sapiens;
<400> 190
   ctgggtgttt ggtgagggga ccgagctgac cgtcctag 38
<210> 191
   <211> 38
   <212> DNA
   <213> homo sapiens;
<400> 191
   taatgtgttc ggcagtggca ccaaggtgac cgtcctcg 38
<210> 192
   <211> 38
   <212> DNA
   <213> homo sapiens;
<400> 192
   tgctgtgttc ggaggaggca cccagctgac cgtcctcg 38
<210> 193
   <211> 302
   <212> DNA
   <213> homo sapiens;
<400> 193
   gagattgtga tgacccagac tccactctcc ttgtctatca cccctggaga gcaggcctcc        60
   atctcctgca ggtctagtca gagcctcctg catagtgatg gatacaccta tttgtattgg       120
   tttctgcaga aagccaggcc agtctccaca ctcctgatct atgaagtttc caaccggttc       180
   tctggagtgc cagataggtt cagtggcagc gggtcaggga cagatttcac actgaaaatc       240
   agccgggtgg aggctgagga ttttggagtt tattactgca tgcaagatgc acaagatcct       300
   cc                                                                      302
<210> 194
   <211> 296
   <212> DNA
   <213> homo sapiens;
<400> 194
   cagtctgtgc tgactcagcc accctcggtg tctgaagccc ccaggcagag ggtcaccatc 60
   tcctgttctg gaagcagctc caacatcgga aataatgctg taaactggta ccagcagctc 120
   ccaggaaagg ctcccaaact cctcatctat tatgatgatc tgctgccctc aggggtctct 180
   gaccgattct ctggctccaa gtctggcacc tcagcctccc tggccatcag tgggctccag 240
   tctgaggatg aggctgatta ttactgtgca gcatgggatg acagcctgaa tggtcc 296
<210> 195
   <211> 296
   <212> DNA
   <213> homo sapiens;
<400> 195
   cagtctgtgc tgactcagcc accctcagcg tctgggaccc ccgggcagag ggtcaccatc 60
   tcttgttctg gaagcagctc caacatcgga agtaattatg tatactggta ccagcagctc 120
   ccaggaacgg cccccaaact cctcatctat aggaataatc agcggccctc aggggtccct 180
   gaccgattct ctggctccaa gtctggcacc tcagcctccc tggccatcag tgggctccgg 240
   tccgaggatg aggctgatta ttactgtgca gcatgggatg acagcctgag tggtcc 296
<210> 196
   <211> 299
   <212> DNA
   <213> homo sapiens;
<400> 196
   cagtctgtgc tgacgcagcc gccctcagtg tctggggccc cagggcagag ggtcaccatc 60
   tcctgcactg ggagcagctc caacattggg gcgggttatg ttgtacattg gtaccagcag 120
   cttccaggaa cagcccccaa actcctcatc tatggtaaca gcaatcggcc ctcaggggtc 180
   cctgaccaat tctctggctc caagtctggc acctcagcct ccctggccat cactggactc 240
   cagtctgagg atgaggctga ttattactgc aaagcatggg ataacagcct gaatgctca 299
<210> 197
   <211> 296
   <212> DNA
   <213> homo sapiens;
<400> 197
   cagtctgtgt tgacgcagcc gccctcagtg tctgcggccc caggacagaa ggtcaccatc 60
   tcctgctctg gaagcagctc caacattggg aataattatg tatcctggta ccagcagctc 120
   ccaggaacag cccccaaact cctcatttat gacaataata agcgaccctc agggattcct 180
   gaccgattct ctggctccaa gtctggcacg tcagccaccc tgggcatcac cggactccag 240
   actggggacg aggccgatta ttactgcgga acatgggata gcagcctgag tgctgg 296
<210> 198
   <211> 296
   <212> DNA
   <213> homo sapiens;
<400> 198
   caggcagggc tgactcagcc accctcggtg tccaagggct tgagacagac cgccacactc 60
   acctgcactg ggaacagcaa caatgttggc aaccaaggag cagcttggct gcagcagcac 120
   cagggccacc ctcccaaact cctatcctac aggaataaca accggccctc agggatctca 180
   gagagattat ctgcatccag gtcaggaaac acagcctccc tgaccattac tggactccag 240
   cctgaggacg aggctgacta ttactgctca gcatgggaca gcagcctcag tgctca 296
<210> 199
   <211> 312
   <212> DNA
   <213> homo sapiens;
<400> 199
   cggcccgtgc tgactcagcc gccctctctg tctgcatccc cgggagcaac agccagactc 60
   ccctgcaccc tgagcagtga cctcagtgtt ggtggtaaaa acatgttctg gtaccagcag 120
   aagccaggga gctctcccag gttattcctg tatcactact cagactcaga caagcagctg 180
   ggacctgggg tccccagtcg agtctctggc tccaaggaga cctcaagtaa cacagcgttt 240
   ttgctcatct ctgggctcca gcctgaggac gaggccgatt attactgcca ggtgtacgaa 300
   agtagtgcta at 312
<210> 200
   <211> 297
   <212> DNA
   <213> homo sapiens;
<400> 200
   cagtctgccc tgactcagcc tgcctccgtg tctgggtctc ctggacagtc gatcaccatc 60
   tcctgcactg gaaccagcag tgacgttggt ggttataact atgtctcctg gtaccaacag 120
   cacccaggca aagcccccaa actcatgatt tatgaggtca gtaatcggcc ctcaggggtt 180
   tctaatcgct tctctggctc caagtctggc aacacggcct ccctgaccat ctctgggctc 240
   caggctgagg acgaggctga ttattactgc agctcatata caagcagcag cactctc 297
<210> 201
   <211> 297
   <212> DNA
   <213> homo sapiens;
<400> 201
   cagtctgccc tgactcagcc tccctccgtg tccgggtctc ctggacagtc agtcaccatc 60
   tcctgcactg gaaccagcag tgacgttggt agttataacc gtgtctcctg gtaccagcag 120
   cccccaggca cagcccccaa actcatgatt tatgaggtca gtaatcggcc ctcaggggtc 180
   cctgatcgct tctctgggtc caagtctggc aacacggcct ccctgaccat ctctgggctc 240
   caggctgagg acgaggctga ttattactgc agcttatata caagcagcag cactttc 297
<210> 202
   <211> 298
   <212> DNA
   <213> homo sapiens;
<400> 202
   cagtctgccc tgactcagcc tgcctccgtg tctgggtctc ctggacagtc gatcaccatc 60
   tcctgcactg gaaccagcag tgatgttggg agttataacc ttgtctcctg gtaccaacag 120
   cacccaggca aagcccccaa actcatgatt tatgagggca gtaagcggcc ctcaggggtt 180
   tctaatcgct tctctggctc caagtctggc aacacggcct ccctgacaat ctctgggctc 240
   caggctgagg acgaggctga ttattactgc tgctcatatg caggtagtag cactttac 298
<210> 203
   <211> 297
   <212> DNA
   <213> homo sapiens;
<400> 203
   cagtctgccc tgactcagcc tccctccgcg tccgggtctc ctggacagtc agtcaccatc 60
   tcctgcactg gaaccagcag tgacgttggt ggttataact atgtctcctg gtaccaacag 120
   cacccaggca aagcccccaa actcatgatt tatgaggtca gtaagcggcc ctcaggggtc 180
   cctgatcgct tctctggctc caagtctggc aacacggcct ccctgaccgt ctctgggctc 240
   caggctgagg atgaggctga ttattactgc agctcatatg caggcagcaa caatttc 297
<210> 204
   <211> 285
   <212> DNA
   <213> homo sapiens;
<400> 204
   tcctatgagc tgactcagcc accctcagtg tccgtgtccc caggacagac agccagcatc 60
   acctgctctg gagataaatt gggggataaa tatgcttgct ggtatcagca gaagccaggc 120
   cagtcccctg tgctggtcat ctatcaagat agcaagcggc cctcagggat ccctgagcga 180
   ttctctggct ccaactctgg gaacacagcc actctgacca tcagcgggac ccaggctatg 240
   gatgaggctg actattactg tcaggcgtgg gacagcagca ctgca 285
<210> 205
   <211> 290
   <212> DNA
   <213> homo sapiens;
<400> 205
   tcctatgagc tgactcagcc acactcagtg tcagtggcca cagcacagat ggccaggatc 60
   acctgtgggg gaaacaacat tggaagtaaa gctgtgcact ggtaccagca aaagccaggc 120
   caggaccctg tgctggtcat ctatagcgat agcaaccggc cctcagggat ccctgagcga 180
   ttctctggct ccaacccagg gaacaccacc accctaacca tcagcaggat cgaggctggg 240
   gatgaggctg actattactg tcaggtgtgg gacagtagta gtgatcatcc 290
<210> 206
   <211> 290
   <212> DNA
   <213> homo sapiens;
<400> 206
   tcttctgagc tgactcagga ccctgctgtg tctgtggcct tgggacagac agtcaggatc 60
   acatgccaag gagacagcct cagaagctat tatgcaagct ggtaccagca gaagccagga 120
   caggcccctg tacttgtcat ctatggtaaa aacaaccggc cctcagggat cccagaccga 180
   ttctctggct ccagctcagg aaacacagct tccttgacca tcactggggc tcaggcggaa 240
   gatgaggctg actattactg taactcccgg gacagcagtg gtaaccatct 290
<210> 207
   <211> 290
   <212> DNA
   <213> homo sapiens;
<400> 207
   tcctatgtgc tgactcagcc accctcagtg tcagtggccc caggaaagac ggccaggatt 60
   acctgtgggg gaaacaacat tggaagtaaa agtgtgcact ggtaccagca gaagccaggc 120
   caggcccctg tgctggtcat ctattatgat agcgaccggc cctcagggat ccctgagcga 180
   ttctctggct ccaactctgg gaacacggcc accctgacca tcagcagggt cgaagccggg 240
   gatgaggccg actattactg tcaggtgtgg gacagtagta gtgatcatcc 290
<210> 208
   <211> 284
   <212> DNA
   <213> homo sapiens;
<400> 208
   tcctatgagc tgacacagct accctcggtg tcagtgtccc caggacagac agccaggatc 60
   acctgctctg gagatgtact gggggaaaat tatgctgact ggtaccagca gaagccaggc 120
   caggcccctg agttggtgat atacgaagat agtgagcggt accctggaat ccctgaacga 180
   ttctctgggt ccacctcagg gaacacgacc accctgacca tcagcagggt cctgaccgaa 240
   gacgaggctg actattactg tttgtctggg gatgaggaca atcc 284
<210> 209
   <211> 290
   <212> DNA
   <213> homo sapiens;
<400> 209
   tcctatgagc tgatgcagcc accctcggtg tcagtgtccc caggacagac ggccaggatc 60
   acctgctctg gagatgcatt gccaaagcaa tatgcttatt ggtaccagca gaagccaggc 120
   caggcccctg tgctggtgat atataaagac agtgagaggc cctcagggat ccctgagcga 180
   ttctctggct ccagctcagg gacaacagtc acgttgacca tcagtggagt ccaggcagaa 240
   gatgaggctg actattactg tcaatcagca gacagcagtg gtacttatcc 290
<210> 210
   <211> 297
   <212> DNA
   <213> homo sapiens;
<400> 210
   cagcctgtgc tgactcaatc atcctctgcc tctgcttccc tgggatcctc ggtcaagctc 60
   acctgcactc tgagcagtgg gcacagtagc tacatcatcg catggcatca gcagcagcca 120
   gggaaggccc ctcggtactt gatgaagctt gaaggtagtg gaagctacaa caaggggagc 180
   ggagttcctg atcgcttctc aggctccagc tctggggctg accgctacct caccatctcc 240
   aacctccagt tagaggatga ggctgattat tactgtgaga cctgggacag taacact 297
<210> 211
   <211> 312
   <212> DNA
   <213> homo sapiens;
<400> 211
   cagcctgtgc tgactcagcc accttcctcc tccgcatctc ctggagaatc cgccagactc 60
   acctgcacct tgcccagtga catcaatgtt ggtagctaca acatatactg gtaccagcag 120
   aagccaggga gccctcccag gtatctcctg tactactact cagactcaga taagggccag 180
   ggctctggag tccccagccg cttctctgga tccaaagatg cttcagccaa tacagggatt 240
   ttactcatct ccgggctcca gtctgaggat gaggctgact attactgtat gatttggcca 300
   agcaatgctt ct 312
<210> 212
   <211> 312
   <212> DNA
   <213> homo sapiens;
<400> 212
   caggctgtgc tgactcagcc ggcttccctc tctgcatctc ctggagcatc agccagtctc 60
   acctgcacct tgcgcagtgg catcaatgtt ggtacctaca ggatatactg gtaccagcag 120
   aagccaggga gtcctcccca gtatctcctg aggtacaaat cagactcaga taagcagcag 180
   ggctctggag tccccagccg cttctctgga tccaaagatg cttcggccaa tgcagggatt 240
   ttactcatct ctgggctcca gtctgaggat gaggctgact attactgtat gatttggcac 300
   agcagcgctt ct 312
<210> 213
   <211> 312
   <212> DNA
   <213> homo sapiens;
<400> 213
   cagcctgtgc tgactcagcc aacttccctc tcagcatctc ctggagcatc agccagactc 60
   acctgcacct tgcgcagtgg catcaatctt ggtagctaca ggatattctg gtaccagcag 120
   aagccagaga gccctccccg gtatctcctg agctactact cagactcaag taagcatcag 180
   ggctctggag tccccagccg cttctctgga tccaaagatg cttcgagcaa tgcagggatt 240
   ttagtcatct ctgggctcca gtctgaggat gaggctgact attactgtat gatttggcac 300
   agcagtgctt ct 312
<210> 214
   <211> 296
   <212> DNA
   <213> homo sapiens;
<400> 214
   aattttatgc tgactcagcc ccactctgtg tcggagtctc cggggaagac ggtaaccatc 60
   tcctgcaccc gcagcagtgg cagcattgcc agcaactatg tgcagtggta ccagcagcgc 120
   ccgggcagtt cccccaccac tgtgatctat gaggataacc aaagaccctc tggggtccct 180
   gatcggttct ctggctccat cgacagctcc tccaactctg cctccctcac catctctgga 240
   ctgaagactg aggacgaggc tgactactac tgtcagtctt atgatagcag caatca 296
<210> 215
   <211> 294
   <212> DNA
   <213> homo sapiens;
<400> 215
   caggctgtgg tgactcagga gccctcactg actgtgtccc caggagggac agtcactctc 60
   acctgtggct ccagcactgg agctgtcacc agtggtcatt atccctactg gttccagcag 120
   aagcctggcc aagcccccag gacactgatt tatgatacaa gcaacaaaca ctcctggaca 180
   cctgcccggt tctcaggctc cctccttggg ggcaaagctg ccctgaccct ttcgggtgcg 240
   cagcctgagg atgaggctga gtattactgc ttgctctcct atagtggtgc tcgg 294
<210> 216
   <211> 296
   <212> DNA
   <213> homo sapiens;
<400> 216
   cagactgtgg tgacccagga gccatcgttc tcagtgtccc ctggagggac agtcacactc 60
   acttgtggct tgagctctgg ctcagtctct actagttact accccagctg gtaccagcag 120
   accccaggcc aggctccacg cacgctcatc tacagcacaa acactcgctc ttctggggtc 180
   cctgatcgct tctctggctc catccttggg aacaaagctg ccctcaccat cacgggggcc 240
   caggcagatg atgaatctga ttattactgt gtgctgtata tgggtagtgg catttc 296
<210> 217
   <211> 1134
   <212> DNA
   <213> homo sapiens;
<220>
   <221> misc_feature
   <222> (1)..(1)
   <223> n is a, c, g, or t
<400> 217
   ncttccacca agggcccatc ggtcttcccc ctggcgccct gctccaggag cacctctggg 60
   ggcacagcgg ccctgggctg cctggtcaag gactacttcc cagaaccggt gacggtgtcg 120
   tggaactcag gcgccctgac cagcggcgtg cacaccttcc cggctgtcct acagtcctca 180
   ggactctact ccctcagcag cgtggtgacc gtgccctcca gcagcttggg cacccagacc 240
   tacacctgca acgtgaatca caagcccagc aacaccaagg tggacaagag agttgagctc 300
   aaaaccccac ttggtgacac aactcacaca tgcccacggt gcccagagcc caaatcttgt 360
   gacacacctc ccccgtgccc acggtgccca gagcccaaat cttgtgacac acctccccca 420
   tgcccacggt gcccagagcc caaatcttgt gacacacctc ccccgtgccc aaggtgccca 480
   gcacctgaac tcctgggagg accgtcagtc ttcctcttcc ccccaaaacc caaggatacc 540
   cttatgattt cccggacccc tgaggtcacg tgcgtggtgg tggacgtgag ccacgaagac 600
   cccgaggtcc agttcaagtg gtacgtggac ggcgtggagg tgcataatgc caagacaaag 660
   ccgcgggagg agcagtacaa cagcacgttc cgtgtggtca gcgtcctcac cgtcctgcac 720
   caggactggc tgaacggcaa ggagtacaag tgcaaggtct ccaacaaagc cctcccagcc 780
   cccatcgaga aaaccatctc caaaaccaaa ggacagcccc gagaaccaca ggtgtacacc 840
   ctgcccccat cccgggagga gatgaccaag aaccaggtca gcctgacctg cctggtcaaa 900
   ggcttctacc ccagcgacat cgccgtggag tgggagagca gcgggcagcc ggagaacaac 960
   tacaacacca cgcctcccat gctggactcc gacggctcct tcttcctcta cagcaagctc 1020
   accgtggaca agagcaggtg gcagcagggg aacatcttct catgctccgt gatgcatgag 1080
   gctctgcaca accgcttcac gcagaagagc ctctccctgt ctccgggtaa atga 1134
<210> 218
   <211> 1023
   <212> DNA
   <213> homo sapiens;
<400> 218
   gcatccccga ccagccccaa ggtcttcccg ctgagcctcg acagcacccc ccaagatggg      60
   aacgtggtcg tcgcatgcct ggtccagggc ttcttccccc aggagccact cagtgtgacc     120
   tggagcgaaa gcggacagaa cgtgaccgcc agaaacttcc cacctagcca ggatgcctcc     180
   ggggacctgt acaccacgag cagccagctg accctgccgg ccacacagtg cccagacggc     240
   aagtccgtga catgccacgt gaagcactac acgaattcca gccaggatgt gactgtgccc     300
   tgccgagttc ccccacctcc cccatgctgc cacccccgac tgtcgctgca ccgaccggcc     360
   ctcgaggacc tgctcttagg ttcagaagcg aacctcacgt gcacactgac cggcctgaga     420
   gatgcctctg gtgccacctt cacctggacg ccctcaagtg ggaagagcgc tgttcaagga     480
   ccacctgagc gtgacctctg tggctgctac agcgtgtcca gtgtcctgcc tggctgtgcc     540
   cagccatgga accatgggga gaccttcacc tgcactgctg cccaccccga gttgaagacc     600
   ccactaaccg ccaacatcac aaaatccgga aacacattcc ggcccgaggt ccacctgctg     660
   ccgccgccgt cggaggagct ggccctgaac gagctggtga cgctgacgtg cctggcacgt     720
   ggcttcagcc ccaaggatgt gctggttcgc tggctgcagg ggtcacagga gctgccccgc     780
   gagaagtacc tgacttgggc atcccggcag gagcccagcc agggcaccac cacctacgct     840
   gtaaccagca tactgcgcgt ggcagctgag gactggaaga agggggagac cttctcctgc     900
   atggtgggcc acgaggccct gccgctggcc ttcacacaga agaccatcga ccgcatggcg     960
   ggtaaaccca cccacatcaa tgtgtctgtt gtcatggcgg aggcggatgg cacctgctac    1020
   tga                                                                  1023
<210> 219
   <211> 981
   <212> DNA
   <213> homo sapiens;
<400> 219
   gcctccacca agggcccatc ggtcttcccc ctggcgccct gctccaggag cacctccgag        60
   agcacagcgg ccctgggctg cctggtcaag gactacttcc ccgaaccggt gacggtgtcg       120
   tggaactcag gcgctctgac cagcggcgtg cacaccttcc cggctgtcct acagtcctca       180
   ggactctact ccctcagcag cgtggtgacc gtgccctcca gcaacttcgg cacccagacc       240
   tacacctgca acgtagatca caagcccagc aacaccaagg tggacaagac agttgagcgc       300
   aaatgttgtg tcgagtgccc accgtgccca gcaccacctg tggcaggacc gtcagtcttc       360
   ctcttccccc caaaacccaa ggacaccctc atgatctccc ggacccctga ggtcacgtgc       420
   gtggtggtgg acgtgagcca cgaagacccc gaggtccagt tcaactggta cgtggacggc       480
   gtggaggtgc ataatgccaa gacaaagcca cgggaggagc agttcaacag cacgttccgt       540
   gtggtcagcg tcctcaccgt cgtgcaccag gactggctga acggcaagga gtacaagtgc       600
   aaggtctcca acaaaggcct cccagccccc atcgagaaaa ccatctccaa aaccaaaggg       660
   cagccccgag aaccacaggt gtacaccctg cccccatccc gggaggagat gaccaagaac       720
   caggtcagcc tgacctgcct ggtcaaaggc ttctacccca gcgacatctc cgtggagtgg       780
   gagagcaatg ggcagccgga gaacaactac aagaccacac ctcccatgct ggactccgac       840
   ggctccttct tcctctacag caagctcacc gtggacaaga gcaggtggca gcaggggaac       900
   gtcttctcat gctccgtgat gcatgaggct ctgcacaacc actacacaca gaagagcctc       960
   tccctgtctc cgggtaaatg a                                                 981
<210> 220
   <211> 1287
   <212> DNA
   <213> homo sapiens;
<400> 220
   gcctccacac agagcccatc cgtcttcccc ttgacccgct gctgcaaaaa cattccctcc 60
   aatgccacct ccgtgactct gggctgcctg gccacgggct acttcccgga gccggtgatg 120
   gtgacctggg acacaggctc cctcaacggg acaactatga ccttaccagc caccaccctc 180
   acgctctctg gtcactatgc caccatcagc ttgctgaccg tctcgggtgc gtgggccaag 240
   cagatgttca cctgccgtgt ggcacacact ccatcgtcca cagactgggt cgacaacaaa 300
   accttcagcg tctgctccag ggacttcacc ccgcccaccg tgaagatctt acagtcgtcc 360
   tgcgacggcg gcgggcactt ccccccgacc atccagctcc tgtgcctcgt ctctgggtac 420
   accccaggga ctatcaacat cacctggctg gaggacgggc aggtcatgga cgtggacttg 480
   tccaccgcct ctaccacgca ggagggtgag ctggcctcca cacaaagcga gctcaccctc 540
   agccagaagc actggctgtc agaccgcacc tacacctgcc aggtcaccta tcaaggtcac 600
   acctttgagg acagcaccaa gaagtgtgca gattccaacc cgagaggggt gagcgcctac 660
   ctaagccggc ccagcccgtt cgacctgttc atccgcaagt cgcccacgat cacctgtctg 720
   gtggtggacc tggcacccag caaggggacc gtgaacctga cctggtcccg ggccagtggg 780
   aagcctgtga accactccac cagaaaggag gagaagcagc gcaatggcac gttaaccgtc 840
   acgtccaccc tgccggtggg cacccgagac tggatcgagg gggagaccta ccagtgcagg 900
   gtgacccacc cccacctgcc cagggccctc atgcggtcca cgaccaagac cagcggcccg 960
   cgtgctgccc cggaagtcta tgcgtttgcg acgccggagt ggccggggag ccgggacaag 1020
   cgcaccctcg cctgcctgat ccagaacttc atgcctgagg acatctcggt gcagtggctg 1080
   cacaacgagg tgcagctccc ggacgcccgg cacagcacga cgcagccccg caagaccaag 1140
   ggctccggct tcttcgtctt cagccgcctg gaggtgacca gggccgaatg ggagcagaaa 1200
   gatgagttca tctgccgtgc agtccatgag gcagcaagcc cctcacagac cgtccagcga 1260
   gcggtgtctg taaatcccgg taaatga 1287
<210> 221
   <211> 30
   <212> DNA
   <213> homo sapiens;
<400> 221
   tccggcttct tcgtcttcag ccgcctggag 30
<210> 222
   <211> 1062
   <212> DNA
   <213> homo sapiens;
<400> 222
   gcatccccga ccagccccaa ggtcttcccg ctgagcctct gcagcaccca gccagatggg 60
   aacgtggtca tcgcctgcct ggtccagggc ttcttccccc aggagccact cagtgtgacc 120
   tggagcgaaa gcggacaggg cgtgaccgcc agaaacttcc cacccagcca ggatgcctcc 180
   ggggacctgt acaccacgag cagccagctg accctgccgg ccacacagtg cctagccggc 240
   aagtccgtga catgccacgt gaagcactac acgaatccca gccaggatgt gactgtgccc 300
   tgcccagttc cctcaactcc acctacccca tctccctcaa ctccacctac cccatctccc 360
   tcatgctgcc acccccgact gtcactgcac cgaccggccc tcgaggacct gctcttaggt 420
   tcagaagcga acctcacgtg cacactgacc ggcctgagag atgcctcagg tgtcaccttc 480
   acctggacgc cctcaagtgg gaagagcgct gttcaaggac cacctgagcg tgacctctgt 540
   ggctgctaca gcgtgtccag tgtcctgccg ggctgtgccg agccatggaa ccatgggaag 600
   accttcactt gcactgctgc ctaccccgag tccaagaccc cgctaaccgc caccctctca 660
   aaatccggaa acacattccg gcccgaggtc cacctgctgc cgccgccgtc ggaggagctg 720
   gccctgaacg agctggtgac gctgacgtgc ctggcacgcg gcttcagccc caaggatgtg 780
   ctggttcgct ggctgcaggg gtcacaggag ctgccccgcg agaagtacct gacttgggca 840
   tcccggcagg agcccagcca gggcaccacc accttcgctg tgaccagcat actgcgcgtg 900
   gcagccgagg actggaagaa gggggacacc ttctcctgca tggtgggcca cgaggccctg 960
   ccgctggcct tcacacagaa gaccatcgac cgcttggcgg gtaaacccac ccatgtcaat 1020
   gtgtctgttg tcatggcgga ggtggacggc acctgctact ga 1062
<210> 223
   <211> 888
   <212> DNA
   <213> homo sapiens;
<400> 223
   gcctccacca agggcccatc ggtcttcccc ctggcaccct cctccaagag cacctctggg 60
   ggcacagcag ccctgggctg cctggtcaag gactacttcc ccgaaccggt gacggtgtcg 120
   tggaactcag gcgccctgac cagcggcgtg cacaccttcc cggctgtcct acagtcctca 180
   ggactctact ccctcagcag cgtggtgacc gtgccctcca gcagcttggg cacccagacc 240
   tacatctgca acgtgaatca caagcccagc aacaccaagg tggacaagaa agttgagccc 300
   aaatcttgtg acaaaactca cacatgccca ccgtgcccag cacctgaact cctgggggga 360
   ccgtcagtct tcctcttccc cccaaaaccc aaggacaccc tcatgatctc ccggacccct 420
   gaggtcacat gcgtggtggt ggacgtgagc cacgaagacc ctgaggtcaa gttcaactgg 480
   tacgtggacg gcgtggagta caagtgcaag gtctccaaca aagccctccc agcccccatc 540
   gagaaaacca tctccaaagc caaagggcag ccccgagaac cacaggtgta caccctgccc 600
   ccatcccggg atgagctgac caagaaccag gtcagcctga cctgcctggt caaaggcttc 660
   tatcccagcg acatcgccgt ggagtgggag agcaatgggc agccggagaa caactacaag 720
   accacgcctc ccgtgctgga ctccgacggc tccttcttcc tctacagcaa gctcaccgtg 780
   gacaagagca ggtggcagca ggggaacgtc ttctcatgct ccgtgatgca tgaggctctg 840
   cacaaccact acacacagaa gagcctctcc ctgtctccgg gtaaatga 888
<210> 224
   <211> 993
   <212> DNA
   <213> homo sapiens;
<400> 224
   gcctccacca agggcccatc ggtcttcccc ctggcaccct cctccaagag cacctctggg 60
   ggcacagcag ccctgggctg cctggtcaag gactacttcc ccgaaccggt gacggtgtcg 120
   tggaactcag gcgccctgac cagcggcgtg cacaccttcc cggctgtcct acagtcctca 180
   ggactctact ccctcagcag cgtggtgacc gtgccctcca gcagcttggg cacccagacc 240
   tacatctgca acgtgaatca caagcccagc aacaccaagg tggacaagaa agttgagccc 300
   aaatcttgtg acaaaactca cacatgccca ccgtgcccag cacctgaact cctgggggga 360
   ccgtcagtct tcctcttccc cccaaaaccc aaggacaccc tcatgatctc ccggacccct 420
   gaggtcacat gcgtggtggt ggacgtgagc cacgaagacc ctgaggtcaa gttcaactgg 480
   tacgtggacg gcgtggaggt gcataatgcc aagacaaagc cgcgggagga gcagtacaac 540
   agcacgtacc gtgtggtcag cgtcctcacc gtcctgcacc aggactggct gaatggcaag 600
   gagtacaagt gcaaggtctc caacaaagcc ctcccagccc ccatcgagaa aaccatctcc 660
   aaagccaaag ggcagccccg agaaccacag gtgtacaccc tgcccccatc ccgggatgag 720
   ctgaccaaga accaggtcag cctgacctgc ctggtcaaag gcttctatcc cagcgacatc 780
   gccgtggagt gggagagcaa tgggcagccg gagaacaact acaagaccac gcctcccgtg 840
   ctggactccg acggctcctt cttcctctac agcaagctca ccgtggacaa gagcaggtgg 900
   cagcagggga acgtcttctc atgctccgtg atgcatgagg ctctgcacaa ccactacaca 960
   cagaagagcc tctccctgtc tccgggtaaa tga 993
<210> 225
   <211> 1362
   <212> DNA
   <213> homo sapiens;
<400> 225
   gggagtgcat ccgccccaac ccttttcccc ctcgtctcct gtgagaattc cccgtcggat      60
   acgagcagcg tggccgttgg ctgcctcgca caggacttcc ttcccgactc catcactttc     120
   tcctggaaat acaagaacaa ctctgacatc agcagcaccc ggggcttccc atcagtcctg     180
   agagggggca agtacgcagc cacctcacag gtgctgctgc cttccaagga cgtcatgcag     240
   ggcacagacg aacacgtggt gtgcaaagtc cagcacccca acggcaacaa agaaaagaac     300
   gtgcctcttc cagtgattgc cgagctgcct cccaaagtga gcgtcttcgt cccaccccgc     360
   gacggcttct tcggcaaccc ccgcaagtcc aagctcatct gccaggccac gggtttcagt     420
   ccccggcaga ttcaggtgtc ctggctgcgc gaggggaagc aggtggggtc tggcgtcacc     480
   acggaccagg tgcaggctga ggccaaagag tctgggccca cgacctacaa ggtgaccagc     540
   acactgacca tcaaagagag cgactggctc agccagagca tgttcacctg ccgcgtggat     600
   cacaggggcc tgaccttcca gcagaatgcg tcctccatgt gtggccccga tcaagacaca     660
   gccatccggg tcttcgccat ccccccatcc tttgccagca tcttcctcac caagtccacc     720
   aagttgacct gcctggtcac agacctgacc acctatgaca gcgtgaccat ctcctggacc     780
   cgccagaatg gcgaagctgt gaaaacccac accaacatct ccgagagcca ccccaatgcc     840
   actttcagcg ccgtgggtga ggccagcatc tgcgaggatg actggaattc cggggagagg     900
   ttcacgtgca ccgtgaccca cacagacctg ccctcgccac tgaagcagac catctcccgg     960
   cccaaggggg tggccctgca caggcccgat gtctacttgc tgccaccagc ccgggagcag    1020
   ctgaacctgc gggagtcggc caccatcacg tgcctggtga cgggcttctc tcccgcggac    1080
   gtcttcgtgc agtggatgca gagggggcag cccttgtccc cggagaagta tgtgaccagc    1140
   gccccaatgc ctgagcccca ggccccaggc cggtacttcg cccacagcat cctgaccgtg    1200
   tccgaagagg aatggaacac gggggagacc tacacctgcg tggtggccca tgaggccctg    1260
   cccaacaggg tcaccgagag gaccgtggac aagtccaccg gtaaacccac cctgtacaac    1320
   gtgtccctgg tcatgtccga cacagctggc acctgctact ga                       1362
<210> 226
   <211> 1200
   <212> DNA
   <213> homo sapiens;
<400> 226
   gcctccacca agggcccatc ggtcttcccc ctggcaccct cctccaagag cacctctggg 60
   ggcacagcag ccctgggctg cctggtcaag gactacttcc ccgaaccggt gacggtgtcg 120
   tggaactcag gcgccctgac cagcggcgtg cacaccttcc cggctgtcct acagtcctca 180
   ggactctact ccctcagcag cgtggtgacc gtgccctcca gcagcttggg cacccagacc 240
   tacatctgca acgtgaatca caagcccagc aacaccaagg tggacaagaa agttgagccc 300
   aaatcttgtg acaaaactca cacatgccca ccgtgcccag cacctgaact cctgggggga 360
   ccgtcagtct tcctcttccc cccaaaaccc aaggacaccc tcatgatctc ccggacccct 420
   gaggtcacat gcgtggtggt ggacgtgagc cacgaagacc ctgaggtcaa gttcaactgg 480
   tacgtggacg gcgtggaggt gcataatgcc aagacaaagc cgcgggagga gcagtacaac 540
   agcacgtacc gtgtggtcag cgtcctcacc gtcctgcacc aggactggct gaatggcaag 600
   gagtacaagt gcaaggtctc caacaaagcc ctcccagccc ccatcgagaa aaccatctcc 660
   aaagccaaag ggcagccccg agaaccacag gtgtacaccc tgcccccatc ccgggatgag 720
   ctgaccaaga accaggtcag cctgacctgc ctggtcaaag gcttctatcc cagcgacatc 780
   gccgtggagt gggagagcaa tgggcagccg gagaacaact acaagaccac gcctcccgtg 840
   ctggactccg acggctcctt cttcctctac agcaagctca ccgtggacaa gagcaggtgg 900
   cagcagggga acgtcttctc atgctccgtg atgcatgagg ctctgcacaa ccactacaca 960
   cagaagagcc tctccctgtc tccggagctg caactggagg agagctgtgc ggaggcgcag 1020
   gacggggagc tggacgggct gtggacgacc atcaccatct tcatcacact cttcctgtta 1080
   agcgtgtgct acagtgccac cgtcaccttc ttcaaggtga agtggatctt ctcctcggtg 1140
   gtggacctga agcagaccat catccccgac tacaggaaca tgatcggaca gggggcctag 1200
<210> 227
   <211> 1293
   <212> DNA
   <213> homo sapiens;
<220>
   <221> misc_feature
   <222> (1)..(1)
   <223> n is a, c, g, or t
<400> 227
   ncacccacca aggctccgga tgtgttcccc atcatatcag ggtgcagaca cccaaaggat 60
   aacagccctg tggtcctggc atgcttgata actgggtacc acccaacgtc cgtgactgtc 120
   acctggtaca tggggacaca gagccagccc cagagaacct tccctgagat acaaagacgg 180
   gacagctact acatgacaag cagccagctc tccacccccc tccagcagtg gcgccaaggc 240
   gagtacaaat gcgtggtcca gcacaccgcc agcaagagta agaaggagat cttccgctgg 300
   ccagagtctc caaaggcaca ggcctcctca gtgcccactg cacaacccca agcagagggc 360
   agcctcgcca aggcaaccac agccccagcc accacccgta acacaggaag aggaggagaa 420
   gagaagaaga aggagaagga gaaagaggaa caagaagaga gagagacaaa gacaccagag 480
   tgtccgagcc acacccagcc tcttggcgtc tacctgctaa cccctgcagt gcaggacctg 540
   tggctccggg acaaagccac cttcacctgc ttcgtggtgg gcagtgacct gaaggatgct 600
   cacctgacct gggaggtggc cgggaaggtc cccacagggg gcgtggagga agggctgctg 660
   gagcggcaca gcaacggctc ccagagccag cacagccgtc tgaccctgcc caggtccttg 720
   tggaacgcgg ggacctccgt cacctgcaca ctgaaccatc ccagcctccc accccagagg 780
   ttgatggcgc tgagagaacc cgctgcgcag gcacccgtca agctttccct gaacctgctg 840
   gcctcgtctg accctcccga ggcggcctcg tggctcctgt gtgaggtgtc tggcttctcg 900
   ccccccaaca tcctcctgat gtggctggag gaccagcgtg aggtgaacac ttctgggttt 960
   gcccccgcac gcccccctcc acagcccggg agcaccacgt tctgggcctg gagtgtgctg 1020
   cgtgtcccag ccccgcccag ccctcagcca gccacctaca cgtgtgtggt cagccacgag 1080
   gactcccgga ctctgctcaa cgccagccgg agcctagaag tcagctacct ggccatgacc 1140
   cccctgatcc ctcagagcaa ggatgagaac agcgatgact acacgacctt tgatgatgtg 1200
   ggcagcctgt ggaccgccct gtccacgttt gtggccctct tcatcctcac cctcctctac 1260
   agcggcattg tcactttcat caaggtgaag tag 1293
<210> 228
   <211> 984
   <212> DNA
   <213> homo sapiens;
<400> 228
   gcttccacca agggcccatc ggtcttcccc ctggcgccct gctccaggag cacctccgag 60
   agcacagccg ccctgggctg cctggtcaag gactacttcc ccgaaccggt gacggtgtcg 120
   tggaactcag gcgccctgac cagcggcgtg cacaccttcc cggctgtcct acagtcctca 180
   ggactctact ccctcagcag cgtggtgacc gtgccctcca gcagcttggg cacgaagacc 240
   tacacctgca acgtagatca caagcccagc aacaccaagg tggacaagag agttgagtcc 300
   aaatatggtc ccccatgccc atcatgccca gcacctgagt tcctgggggg accatcagtc 360
   ttcctgttcc ccccaaaacc caaggacact ctcatgatct cccggacccc tgaggtcacg 420
   tgcgtggtgg tggacgtgag ccaggaagac cccgaggtcc agttcaactg gtacgtggat 480
   ggcgtggagg tgcataatgc caagacaaag ccgcgggagg agcagttcaa cagcacgtac 540
   cgtgtggtca gcgtcctcac cgtcctgcac caggactggc tgaacggcaa ggagtacaag 600
   tgcaaggtct ccaacaaagg cctcccgtcc tccatcgaga aaaccatctc caaagccaaa 660
   gggcagcccc gagagccaca ggtgtacacc ctgcccccat cccaggagga gatgaccaag 720
   aaccaggtca gcctgacctg cctggtcaaa ggcttctacc ccagcgacat cgccgtggag 780
   tgggagagca atgggcagcc ggagaacaac tacaagacca cgcctcccgt gctggactcc 840
   gacggctcct tcttcctcta cagcaggctc accgtggaca agagcaggtg gcaggagggg 900
   aatgtcttct catgctccgt gatgcatgag gctctgcaca accactacac acagaagagc 960
   ctctccctgt ctctgggtaa atga 984
<210> 229
   <211> 228
   <212> DNA
   <213> homo sapiens;
<400> 229
   tccggcttct tcgtcttcag ccgcctggag gtgaccaggg ccgaatggga gcagaaagat 60
   gagttcatct gccgtgcagt ccatgaggca gcaagcccct cacagaccgt ccagcgagcg 120
   gtgtctgtaa atcccgagct ggacgtgtgc gtggaggagg ccgagggcga ggcgccgtgg 180
   acgtggaccg gcctctgcat cttcgccgca ctcttcctgc tcagcgtg 228
<210> 230
   <211> 324
   <212> DNA
   <213> homo sapiens;
<220>
   <221> misc_feature
   <222> (1)..(1)
   <223> n is a, c, g, or t
<400> 230
   ngaactgtgg ctgcaccatc tgtcttcatc ttcccgccat ctgatgagca gttgaaatct      60
   ggaactgcct ctgttgtgtg cctgctgaat aacttctatc ccagagaggc caaagtacag     120
   tggaaggtgg ataacgccct ccaatcgggt aactcccagg agagtgtcac agagcaggac     180
   agcaaggaca gcacctacag cctcagcagc accctgacgc tgagcaaagc agactacgag     240
   aaacacaaag tctacgcctg cgaagtcacc catcagggcc tgagctcgcc cgtcacaaag     300
   agcttcaaca ggggagagtg ttag                                            324
<210> 231
   <211> 321
   <212> DNA
   <213> homo sapiens;
<220>
   <221> misc_feature
   <222> (1)..(1)
   <223> n is a, c, g, or t
<400> 231
   ngtcagccca aggctgcccc ctcggtcact ctgttcccgc cctcctctga ggagcttcaa      60
   gccaacaagg ccacactggt gtgtctcata agtgacttct acccgggagc cgtgacagtg     120
   gcctggaagg cagatagcag ccccgtcaag gcgggagtgg agaccaccac accctccaaa     180
   caaagcaaca acaagtacgc ggccagcagc tatctgagcc tgacgcctga gcagtggaag     240
   tcccacagaa gctacagctg ccaggtcacg catgaaggga gcaccgtgga gaagacagtg     300
   gcccctacag aatgttcata g                                               321
<210> 232
   <211> 321
   <212> DNA
   <213> homo sapiens;
<220>
   <221> misc_feature
   <222> (1)..(1)
   <223> n is a, c, g, or t
<400> 232
   ngtcagccca aggccaaccc cactgtcact ctgttcccgc cctcctctga ggagctccaa 60
   gccaacaagg ccacactagt gtgtctgatc agtgacttct acccgggagc tgtgacagtg 120
   gcctggaagg cagatggcag ccccgtcaag gcgggagtgg agaccaccaa accctccaaa 180
   cagagcaaca acaagtacgc ggccagcagc tacctgagcc tgacgcccga gcagtggaag 240
   tcccacagaa gctacagctg ccaggtcacg catgaaggga gcaccgtgga gaagacagtg 300
   gcccctacag aatgttcata g 321
<210> 233
   <211> 321
   <212> DNA
   <213> homo sapiens;
<220>
   <221> misc_feature
   <222> (1)..(1)
   <223> n is a, c, g, or t
<400> 233
   ngtcagccca aggctgcccc ctcggtcact ctgttcccgc cctcctctga ggagcttcaa 60
   gccaacaagg ccacactggt gtgtctcata agtgacttct acccgggagc cgtgacagtg 120
   gcctggaagg cagatagcag ccccgtcaag gcgggagtgg agaccaccac accctccaaa 180
   caaagcaaca acaagtacgc ggccagcagc tacctgagcc tgacgcctga gcagtggaag 240
   tcccacagaa gctacagctg ccaggtcacg catgaaggga gcaccgtgga gaagacagtg 300
   gcccctacag aatgttcata g 321
<210> 234
   <211> 321
   <212> DNA
   <213> homo sapiens;
<220>
   <221> misc_feature
   <222> (1)..(1)
   <223> n is a, c, g, or t
<400> 234
   ngtcagccca aggctgcccc ctcggtcact ctgttcccac cctcctctga ggagcttcaa    60
   gccaacaagg ccacactggt gtgtctcgta agtgacttct acccgggagc cgtgacagtg   120
   gcctggaagg cagatggcag ccccgtcaag gtgggagtgg agaccaccaa accctccaaa   180
   caaagcaaca acaagtatgc ggccagcagc tacctgagcc tgacgcccga gcagtggaag   240
   tcccacagaa gctacagctg ccgggtcacg catgaaggga gcaccgtgga gaagacagtg   300
   gcccctgcag aatgctctta g                                             321
<210> 235
   <211> 61
   <212> DNA
   <213> homo sapiens;
<400> 235
   tactactact actacggtat ggacgtctgg ggccaaggga ccacggtcac cgtctcctca    60
   g                                                                    61
<210> 236
   <211> 20
   <212> PRT
   <213> homo sapiens;
<400> 236
   Tyr Tyr Tyr Tyr Tyr Gly Met Asp Val Trp Gly Gln Gly Thr Thr Val
   1               5                   10                  15
   Thr Val Ser Ser
               20
<210> 237
   <211> 101
   <212> DNA
   <213> homo sapiens;
<400> 237
   ggtttttgtg gggtgaggat ggacattctg ccattgtgat tactactact actacggtat    60
   ggacgtctgg ggccaaggga ccacggtcac cgtctcctca g                      101
<210> 238
   <211> 38
   <212> DNA
   <213> homo sapiens;
<400> 238
   ggtttttgtg gggtgaggat ggacattctg ccattgtg 38
<210> 239
   <211> 52
   <212> DNA
   <213> homo sapiens;
<400> 239
   gctgaatact tccagcactg gggccagggc accctggtca ccgtctcctc ag 52
<210> 240
   <211> 52
   <212> DNA
   <213> homo sapiens;
<400> 240
   tactggtact tcgatctctg gggccgtggc accctggtca ctgtctcctc ag 52
<210> 241
   <211> 49
   <212> DNA
   <213> homo sapiens;
<400> 241
   gatgcttttg atatctgggg ccaagggaca atggtcaccg tctcttcag 49
<210> 242
   <211> 46
   <212> DNA
   <213> homo sapiens;
<400> 242
   tactttgact actggggcca gggaaccctg gtcaccgtct cctcag 46
<210> 243
   <211> 49
   <212> DNA
   <213> homo sapiens;
<400> 243
   aactggttcg acccctgggg ccagggaacc ctggtcaccg tctcctcag 49
<210> 244
   <211> 61
   <212> DNA
   <213> homo sapiens;
<400> 244
   tactactact actactacat ggacgtctgg ggcaaaggga ccacggtcac cgtctcctca 60
   g 61
<210> 245
   <211> 494
   <212> DNA
   <213> homo sapiens;
<400> 245
   gcatcaccca aaaaccacac ccctccttgg gagaatcccc tagatcacag ctcctcacca 60
   tggactggac ctggagcatc cttttcttgg tggcagcagc aacaggtaac ggactcccca 120
   gtcccagggc tgagagagaa accaggccag tcatgtgaga cttcacccac tcctgtgtcc 180
   tctccacagg tgcccactcc caggttcagc tggtgcagtc tggagctgag gtgaagaagc 240
   ctggggcctc agtgaaggtc tcctgcaagg cttctggtta cacctttacc agctatggta 300
   tcagctgggt gcgacaggcc cctggacaag ggcttgagtg gatgggatgg atcagcgctt 360
   acaatggtaa cacaaactat gcacagaagc tccagggcag agtcaccatg accacagaca 420
   catccacgag cacagcctac atggagctga ggagcctgag atctgacgac acggccgtgt 480
   attactgtgc gaga 494
<210> 246
   <211> 500
   <212> DNA
   <213> homo sapiens;
<400> 246
   gagagcatca cccagcaacc acatctgtcc tctagagaat cccctgagag ctccgttcct 60
   caccatggac tggacctgga ggatcctctt cttggtggca gcagccacag gtaagaggct 120
   ccctagtccc agtgatgaga aagagattga gtccagtcca gggagatctc atccacttct 180
   gtgttctctc cacaggagcc cactcccagg tgcagctggt gcagtctggg gctgaggtga 240
   agaagcctgg ggcctcagtg aaggtctcct gcaaggcttc tggatacacc ttcaccggct 300
   actatatgca ctgggtgcga caggcccctg gacaagggct tgagtggatg ggatggatca 360
   accctaacag tggtggcaca aactatgcac agaagtttca gggcagggtc accatgacca 420
   gggacacgtc catcagcaca gcctacatgg agctgagcag gctgagatct gacgacacgg 480
   ccgtgtatta ctgtgcgaga 500
<210> 247
   <211> 496
   <212> DNA
   <213> homo sapiens;
<400> 247
   accatcacac aacagccaca tccctcccct acagaagccc ccagagcgca gcacctcacc 60
   atggactgca cctggaggat cctcttcttg gtggcagcag ctacaggcaa gagaatcctg 120
   agttccaggg ctgatgaggg gactgggtcc agttaagtgg tgtctcatcc actcctctgt 180
   cctctccaca ggcacccacg cccaggtcca gctggtacag tctggggctg aggtgaagaa 240
   gcctggggcc tcagtgaagg tctcctgcaa ggtttccgga tacaccctca ctgaattatc 300
   catgcactgg gtgcgacagg ctcctggaaa agggcttgag tggatgggag gttttgatcc 360
   tgaagatggt gaaacaatct acgcacagaa gttccagggc agagtcacca tgaccgagga 420
   cacatctaca gacacagcct acatggagct gagcagcctg agatctgagg acacggccgt 480
   gtattactgt gcaaca 496
<210> 248
   <211> 478
   <212> DNA
   <213> homo sapiens;
<400> 248
   ccacatccct cctcagaagc ccccagagca caactcctca ccatggactg gacctggagg 60
   atcctctttt tggtggcagc agccacaggt aaggggctgc caaatcccag tgaggaggaa 120
   gggatcgaag ccagtcaagg gggcttccat ccactcctgt gtcttctcta caggtgtcca 180
   ctcccaggtt cagctggtgc agtctggggc tgaggtgaag aagcctgggg cctcagtgaa 240
   ggtttcctgc aaggcttctg gatacacctt cactagctat gctatgcatt gggtgcgcca 300
   ggcccccgga caaaggcttg agtggatggg atggagcaac gctggcaatg gtaacacaaa 360
   atattcacag gagttccagg gcagagtcac cattaccagg gacacatccg cgagcacagc 420
   ctacatggag ctgagcagcc tgagatctga ggacatggct gtgtattact gtgcgaga 478
<210> 249
   <211> 494
   <212> DNA
   <213> homo sapiens;
<400> 249
   atcacccaac aaccacatcc ctcctctaga gaatcccctg aaagcacagc tcctcaccat 60
   ggactggacc tggagaatcc tcttcttggt ggcagcagcc acaggtaagg ggctcccaag 120
   tcccagtgat gaggagggga ttgagtccag tcaaggtggc ttttatccac tcctgtgtcc 180
   cctccacaga tgcctactcc cagatgcagc tggtgcagtc tggggctgag gtgaagaaga 240
   ctgggtcctc agtgaaggtt tcctgcaagg cttccggata caccttcacc taccgctacc 300
   tgcactgggt gcgacaggcc cccggacaag cgcttgagtg gatgggatgg atcacacctt 360
   tcaatggtaa caccaactac gcacagaaat tccaggacag agtcaccatt accagggaca 420
   ggtctatgag cacagcctac atggagctga gcagcctgag atctgaggac acagccatgt 480
   attactgtgc aaga 494
<210> 250
   <211> 740
   <212> DNA
   <213> homo sapiens;
<400> 250
   atctgtgggg acttgttctt cagtgaaagg atcctgtccg caaacagaaa tggagcagga 60
   catgcatttc ttcaagcagg attagggctt ggaccatcag catcccactc ctgtgtggca 120
   gatgggacat ctatcttctt tctcaacctc gatcaggctt tgaggtatga aataatctgt 180
   ctcatgaata tgcaaataac cttagatcta ctgaggtaaa tatggataca tctgggccct 240
   gaaagcatca tccaacaacc acatcccttc tctacagaag cctctgagag gaaagttctt 300
   caccatggac tggacctgga gggtcttctg cttgctggct gtagctccag gtaaagggcc 360
   aactggttcc agggctgagg aagggatttt ttccagttta gaggactgtc attctctact 420
   gtgtcctctc cgcaggtgct cactcccagg tgcagctggt gcagtctggg gctgaggtga 480
   agaagcctgg ggcctcagtg aaggtttcct gcaaggcatc tggatacacc ttcaccagct 540
   actatatgca ctgggtgcga caggcccctg gacaagggct tgagtggatg ggaataatca 600
   accctagtgg tggtagcaca agctacgcac agaagttcca gggcagagtc accatgacca 660
   gggacacgtc cacgagcaca gtctacatgg agctgagcag cctgagatct gaggacacgg 720
   ccgtgtatta ctgtgcgaga 740
<210> 251
   <211> 497
   <212> DNA
   <213> homo sapiens;
<400> 251
   agcatcatcc agaaaccaca tccctccgct agagaagccc ctgacggcac agttcctcac 60
   tatggactgg atttggaggg tcctcttctt ggtgggagca gcgacaggca aggagatgcc 120
   aagtcccagt gatgaggagg ggattgagtc cagtcaaggt ggctttcatc cactcctgtg 180
   ttctctccac aggtgcccac tcccaaatgc agctggtgca gtctgggcct gaggtgaaga 240
   agcctgggac ctcagtgaag gtctcctgca aggcttctgg attcaccttt actagctctg 300
   ctatgcagtg ggtgcgacag gctcgtggac aacgccttga gtggatagga tggatcgtcg 360
   ttggcagtgg taacacaaac tacgcacaga agttccagga aagagtcacc attaccaggg 420
   acatgtccac aagcacagcc tacatggagc tgagcagcct gagatccgag gacacggccg 480
   tgtattactg tgcggca 497
<210> 252
   <211> 498
   <212> DNA
   <213> homo sapiens;
<400> 252
   agcatcacat aacaaccaca ttcctcctct gaagaagccc ctgggagcac agctcatcac 60
   catggactgg acctggaggt tcctctttgt ggtggcagca gctacaggta aggggcttcc 120
   tagtcctaag gctgaggaag ggatcctggt ttagttaaag aggattttat tcacccctgt 180
   gtcctctcca caggtgtcca gtcccaggtg cagctggtgc agtctggggc tgaggtgaag 240
   aagcctgggt cctcggtgaa ggtctcctgc aaggcttctg gaggcacctt cagcagctat 300
   gctatcagct gggtgcgaca ggcccctgga caagggcttg agtggatggg agggatcatc 360
   cctatctttg gtacagcaaa ctacgcacag aagttccagg gcagagtcac gattaccgcg 420
   gacaaatcca cgagcacagc ctacatggag ctgagcagcc tgagatctga ggacacggcc 480
   gtgtattact gtgcgaga 498
<210> 253
   <211> 499
   <212> DNA
   <213> homo sapiens;
<400> 253
   gagcatcact caacaaccac atctgtcctc tagagaaaac cctgtgagca cagctcctca        60
   ccatggactg gacctggagg atcctcttct tggtggcagc agctacaagt aaggggcttc       120
   ctagtctcaa agctgaggaa cggatcctgg ttcagtcaaa gaggatttta ttctctcctg       180
   tgttctctcc acaggtgccc actcccaggt gcagctggtg cagtctgggg ctgaggtgaa       240
   gaagcctggg gcctcagtga aggtctcctg caaggcttct ggatacacct tcaccagtta       300
   tgatatcaac tgggtgcgac aggccactgg acaagggctt gagtggatgg gatggatgaa       360
   ccctaacagt ggtaacacag gctatgcaca gaagttccag ggcagagtca ccatgaccag       420
   gaacacctcc ataagcacag cctacatgga gctgagcagc ctgagatctg aggacacggc       480
   cgtgtattac tgtgcgaga                                                    499
<210> 254
   <211> 467
   <212> DNA
   <213> homo sapiens;
<400> 254
   gctcagtgac tcctgtgccc caccatggac acactttgct acacactcct gctgctgacc        60
   accccttcct gtgagtgctg tggtcaggga cttcctcaga agtgaaacat cagttgtctc      120
   ctttgtgggc ttcatcttct tatgtcttct ccacaggggt cttgtcccag gtcaccttga       180
   aggagtctgg tcctgtgctg gtgaaaccca cagagaccct cacgctgacc tgcaccgtct       240
   ctgggttctc actcagcaat gctagaatgg gtgtgagctg gatccgtcag cccccaggga       300
   aggccctgga gtggcttgca cacatttttt cgaatgacga aaaatcctac agcacatctc       360
   tgaagagcag gctcaccatc tccaaggaca cctccaaaag ccaggtggtc cttaccatga       420
   ccaacatgga ccctgtggac acagccacat attactgtgc acggata                     467
<210> 255
   <211> 463
   <212> DNA
   <213> homo sapiens;
<400> 255
   agtgactcct gtgccccacc atggacacac tttgctccac gctcctgctg ctgaccatcc 60
   cttcatgtga gtgctgtggt cagggactcc ttcacgggtg aaacatcagt tttcttgttt 120
   gtgggcttca tcttcttatg ctttctccac aggggtcttg tcccagatca ccttgaagga 180
   gtctggtcct acgctggtga aacccacaca gaccctcacg ctgacctgca ccttctctgg 240
   gttctcactc agcactagtg gagtgggtgt gggctggatc cgtcagcccc caggaaaggc 300
   cctggagtgg cttgcactca tttattggaa tgatgataag cgctacagcc catctctgaa 360
   gagcaggctc accatcacca aggacacctc caaaaaccag gtggtcctta caatgaccaa 420
   catggaccct gtggacacag ccacatatta ctgtgcacac aga 463
<210> 256
   <211> 519
   <212> DNA
   <213> homo sapiens;
<400> 256
   atctccacca gctccaccct cccctgggtt caaaagacga ggacagggcc tcgctcagtg 60
   aatcctgctc tccaccatgg acatactttg ttccacgctc ctgctactga ctgtcccgtc 120
   ctgtgagtgc tgtggtcagg tagtacttca gaagcaaaaa atctattctc tcctttgtgg 180
   gcttcatctt cttatgtctt ctccacaggg gtcttatccc aggtcacctt gagggagtct 240
   ggtcctgcgc tggtgaaacc cacacagacc ctcacactga cctgcacctt ctctgggttc 300
   tcactcagca ctagtggaat gtgtgtgagc tggatccgtc agcccccagg gaaggccctg 360
   gagtggcttg cactcattga ttgggatgat gataaatact acagcacatc tctgaagacc 420
   aggctcacca tctccaagga cacctccaaa aaccaggtgg tccttacaat gaccaacatg 480
   gaccctgtgg acacagccac gtattattgt gcacggata 519
<210> 257
   <211> 568
   <212> DNA
   <213> homo sapiens;
<400> 257
   ctccctctgc tgataaaaac cagccgagcc cagaccctgc agctctggga gaagagcccc 60
   agccccagaa ttcccaggag tttccattcg gtgatcagca ctgaacacag aggactcacc 120
   atggagtttg ggctgagctg ggttttcctt gttgctatta taaaaggtga tttatggaga 180
   actagagaca ttgagtggac gtgagtgaga taagcagtga atatatgtgg cagtttctga 240
   ctaggttgtc tctgtgtttg caggtgtcca gtgtcaggtg cagctggtgg agtctggggg 300
   aggcttggtc aagcctggag ggtccctgag actctcctgt gcagcctctg gattcacctt 360
   cagtgactac tacatgagct ggatccgcca ggctccaggg aaggggctgg agtgggtttc 420
   atacattagt agtagtggta gtaccatata ctacgcagac tctgtgaagg gccgattcac 480
   catctccagg gacaacgcca agaactcact gtatctgcaa atgaacagcc tgagagccga 540
   ggacacggcc gtgtattact gtgcgaga 568
<210> 258
   <211> 531
   <212> DNA
   <213> homo sapiens;
<400> 258
   agctctggga gtggagcccc agccttggga ttcccaagtg tttgtattca gtgatcagga 60
   ctgaacacac aggactcacc atggagttgg ggctgagctg ggttttcctt gttgctatat 120
   tagaaggtga ttcatggaga actagagata ttgagtgtga atgggcatga atgagagaaa 180
   cagtgggtat gtgtggcaat ttctgacttt tgtgtctctg tgtttgcagg tgtccagtgt 240
   gaggtgcagc tggtggagtc tgggggaggc ttggtacagc ctggggggtc cctgagactc 300
   tcctgtgcag cctctggatt caccttcagt agctacgaca tgcactgggt ccgccaagct 360
   acaggaaaag gtctggagtg ggtctcagct attggtactg ctggtgacac atactatcca 420
   ggctccgtga agggccgatt caccatctcc agagaaaatg ccaagaactc cttgtatctt 480
   caaatgaaca gcctgagagc cggggacacg gctgtgtatt actgtgcaag a 531
<210> 259
   <211> 540
   <212> DNA
   <213> homo sapiens;
<400> 259
   agctctggga gaggagcccc agccttggga ttcccaagtg ttttcattca gtgatcagga 60
   ctgaacacag aggactcacc atggagtttg ggctgagctg gattttcctt gctgctattt 120
   taaaaggtga tttatggaga actagagaga ttaagtgtga gtggacgtga gtgagagaaa 180
   cagtggatat gtgtggcagt ttctgatctt agtgtctctg tgtttgcagg tgtccagtgt 240
   gaggtgcagc tggtggagtc tgggggaggc ttggtaaagc ctggggggtc ccttagactc 300
   tcctgtgcag cctctggatt cactttcagt aacgcctgga tgagctgggt ccgccaggct 360
   ccagggaagg ggctggagtg ggttggccgt attaaaagca aaactgatgg tgggacaaca 420
   gactacgctg cacccgtgaa aggcagattc accatctcaa gagatgattc aaaaaacacg 480
   ctgtatctgc aaatgaacag cctgaaaacc gaggacacag ccgtgtatta ctgtaccaca 540
<210> 260
   <211> 526
   <212> DNA
   <213> homo sapiens;
<400> 260
   agccctggga gagaagcccc agccctggga ttctcaggtg tttctattgg gtcaacagca 60
   ataaacaaat taccatggaa tttgggctga gctgggtttt tcttgctggt attttaaaag 120
   gtgattcatg gagaactaag gatattgagt gagtggacat gagtgagaga aacagtggat 180
   atgtgtggca gtttctgacc agggtgtctc tgtgtttgca ggtgtccagt gtgaggtgca 240
   gctggtggag tctgggggag gcttggtaca gcctgggggg tccctgagac tctcctgtgc 300
   agcctctgga ttcaccttca gtaacagtga catgaactgg gcccgcaagg ctccaggaaa 360
   ggggctggag tgggtatcgg gtgttagttg gaatggcagt aggacgcact atgtggactc 420
   cgtgaagcgc cgattcatca tctccagaga caattccagg aactccctgt atctgcaaaa 480
   gaacagacgg agagccgagg acatggctgt gtattactgt gtgaga 526
<210> 261
   <211> 515
   <212> DNA
   <213> homo sapiens;
<400> 261
   ccagccctga gattcccacg tgtttccatt cagtgatcag cactgaacac agaggactcg 60
   ccatggagtt tgggctgagc tgggttttcc ttgttgctat tttaaaaggt gattcatgga 120
   tcaatagaga tgttgagtgt gagtgaacac gagtgagaga aacagtggat ttgtgtggca 180
   gtttctgacc aggtgtctct gtgtttgcag gtgtccagtg tgaggtgcag ctggtggagt 240
   ctgggggagg tgtggtacgg cctggggggt ccctgagact ctcctgtgca gcctctggat 300
   tcacctttga tgattatggc atgagctggg tccgccaagc tccagggaag gggctggagt 360
   gggtctctgg tattaattgg aatggtggta gcacaggtta tgcagactct gtgaagggcc 420
   gattcaccat ctccagagac aacgccaaga actccctgta tctgcaaatg aacagtctga 480
   gagccgagga cacggccttg tatcactgtg cgaga 515
<210> 262
   <211> 531
   <212> DNA
   <213> homo sapiens;
<400> 262
   agctctgaga gaggagcctt agccctggat tccaaggcct atccacttgg tgatcagcac 60
   tgagcaccga ggattcacca tggaactggg gctccgctgg gttttccttg ttgctatttt 120
   agaaggtgaa tcatggaaaa gtagagagat ttagtgtgtg tggatatgag tgagagaaac 180
   ggtggatgtg tgtgacagtt tctgaccaat gtctctctgt ttgcaggtgt ccagtgtgag 240
   gtgcagctgg tggagtctgg gggaggcctg gtcaagcctg gggggtccct gagactctcc 300
   tgtgcagcct ctggattcac cttcagtagc tatagcatga actgggtccg ccaggctcca 360
   gggaaggggc tggagtgggt ctcatccatt agtagtagta gtagttacat atactacgca 420
   gactcagtga agggccgatt caccatctcc agagacaacg ccaagaactc actgtatctg 480
   caaatgaaca gcctgagagc cgaggacacg gctgtgtatt actgtgcgag a 531
<210> 263
   <211> 533
   <212> DNA
   <213> homo sapiens;
<400> 263
   agctctgaga gaggagccca gccctgggat tttcaggtgt tttcatttgg tgatcaggac 60
   tgaacagaga gaactcacca tggagtttgg gctgagctgg ctttttcttg tggctatttt 120
   aaaaggtaat tcatggagaa atagaaaaat tgagtgtgaa tggataagag tgagagaaac 180
   agtggatacg tgtggcagtt tctgaccagg gtttcttttt gtttgcaggt gtccagtgtg 240
   aggtgcagct gttggagtct gggggaggct tggtacagcc tggggggtcc ctgagactct 300
   cctgtgcagc ctctggattc acctttagca gctatgccat gagctgggtc cgccaggctc 360
   cagggaaggg gctggagtgg gtctcagcta ttagtggtag tggtggtagc acatactacg 420
   cagactccgt gaagggccgg ttcaccatct ccagagacaa ttccaagaac acgctgtatc 480
   tgcaaatgaa cagcctgaga gccgaggaca cggccgtata ttactgtgcg aaa 533
<210> 264
   <211> 532
   <212> DNA
   <213> homo sapiens;
<400> 264
   cagctctggg agaggagccc agcactagaa gtcggcggtg tttccattcg gtgatcagca 60
   ctgaacacag aggactcacc atggagtttg ggctgagctg ggttttcctc gttgctcttt 120
   taagaggtga ttcatggaga aatagagaga ctgagtgtga gtgaacatga gtgagaaaaa 180
   ctggatttgt gtggcatttt ctgataacgg tgtccttctg tttgcaggtg tccagtgtca 240
   ggtgcagctg gtggagtctg ggggaggcgt ggtccagcct gggaggtccc tgagactctc 300
   ctgtgcagcc tctggattca ccttcagtag ctatggcatg cactgggtcc gccaggctcc 360
   aggcaagggg ctggagtggg tggcagttat atcatatgat ggaagtaata aatactatgc 420
   agactccgtg aagggccgat tcaccatctc cagagacaat tccaagaaca cgctgtatct 480
   gcaaatgaac agcctgagag ctgaggacac ggctgtgtat tactgtgcga ga 532
<210> 265
   <211> 532
   <212> DNA
   <213> homo sapiens;
<400> 265
   cagctctggg agaggagccc agcactagaa gtcggcggtg tttccattcg gtgatcagca       60
   ctgaacacag aggactcacc atggagtttg ggctgagctg ggttttcctc gttgctcttt      120
   taagaggtga ttcatggaga aatagagaga ctgagtgtga gtgaacatga gtgagaaaaa      180
   ctggatttgt gtggcatttt ctgataacgg tgtccttctg tttgcaggtg tccagtgtca      240
   ggtgcagctg gtggagtctg ggggaggcgt ggtccagcct gggaggtccc tgagactctc      300
   ctgtgcagcg tctggattca ccttcagtag ctatggcatg cactgggtcc gccaggctcc      360
   aggcaagggg ctggagtggg tggcagttat atggtatgat ggaagtaata aatactatgc      420
   agactccgtg aagggccgat tcaccatctc cagagacaat tccaagaaca cgctgtatct      480
   gcaaatgaac agcctgagag ccgaggacac ggctgtgtat tactgtgcga ga              532
<210> 266
   <211> 467
   <212> DNA
   <213> homo sapiens;
<400> 266
   aacaaacaaa ttaccatgga atttgggctg agctgggttt ttcttgctgc tattttaaaa       60
   ggtgattcat gaagaactaa ggatattgag tgagtggaca tgagtgagag aaacagtgga      120
   tttgtgtggc agtttctgac cagggtgtct ctgtgtttgc aggtgtccag tgtgaggtgc      180
   agctggtgga gtctggggga ggcttggtac agcctggggg atccctgaga ctctcctgtg      240
   cagcctctgg attcaccttc agtaacagtg acatgaactg ggtccatcag gctccaggaa      300
   aggggctgga gtgggtatcg ggtgttagtt ggaatggcag taggacgcac tatgcagact      360
   ctgtgaaggg ccgattcatc atctccagag acaattccag gaacaccctg tatctgcaaa      420
   cgaatagcct gagggccgag gacacggctg tgtattactg tgtgaga                    467
<210> 267
   <211> 529
   <212> DNA
   <213> homo sapiens;
<400> 267
   ctctgggagt ggagccccag ccttgggatt cccaggtgtt tcccttcagt gatcaggact 60
   gaacacacac aactcatcat gcagtttgtg ctgagctggg ttttccttgt tggtatttta 120
   aaaggtgatt catggagaac tacagatgtt gagtgtgagt ggacatgagt gagcaaaaca 180
   gtgggtttgt gtggcagttt ctgaccttgg tgtctctgtg tttgcaggtg tccagtgtga 240
   ggtgcagctg gtggagtctg ggggaggctt ggtacagcct agggggtccc tgagactctc 300
   ctgtgcagcc tctggattca ccgtcagtag caatgagatg agctggatcc gccaggctcc 360
   agggaagggg ctggagtggg tctcatccat tagtggtggt agcacatact acgcagactc 420
   caggaagggc agattcacca tctccagaga caattccaag aacacgctgt atcttcaaat 480
   gaacaacctg agagctgagg gcacggccgt gtattactgt gccagatat 529
<210> 268
   <211> 537
   <212> DNA
   <213> homo sapiens;
<400> 268
   agctctggga gaggagcccc agccctgaga ttcccaggtg tttccattcg gtgatcagca 60
   ctgaacacag agaacgcacc atggagtttg gactgagctg ggttttcctt gttgctattt 120
   taaaaggtga ttcatggata aatagagatg ttgagtgtga gtgaacatga gtgagagaaa 180
   cagtggatat gtgtggcagt gtctgaccag ggtgtctctg tgtttgcagg tgtccagtgt 240
   gaagtgcagc tggtggagtc tgggggagtc gtggtacagc ctggggggtc cctgagactc 300
   tcctgtgcag cctctggatt cacctttgat gattatacca tgcactgggt ccgtcaagct 360
   ccggggaagg gtctggagtg ggtctctctt attagttggg atggtggtag cacatactat 420
   gcagactctg tgaagggccg attcaccatc tccagagaca acagcaaaaa ctccctgtat 480
   ctgcaaatga acagtctgag aactgaggac accgccttgt attactgtgc aaaagat 537
<210> 269
   <211> 533
   <212> DNA
   <213> homo sapiens;
<400> 269
   agctctcaga gaggtgcctt agccctggat tccaaggcat ttccacttgg tgatcagcac 60
   tgaacacaga ggactcacca tggagttggg gctgtgctgg gttttccttg ttgctatttt 120
   agaaggtgat tcatggaaaa ctagagagat ttagtgtgtg tggatatgag tgagagaaac 180
   agtggatatg tgtggcagtt tctgaccttg gtgtctcttt gtttgcaggt gtccagtgtg 240
   aggtgcagct ggtggagtct gggggaggct tggtacagcc tggggggtcc ctgagactct 300
   cctgtgcagc ctctggattc accttcagta gctatagcat gaactgggtc cgccaggctc 360
   cagggaaggg gctggagtgg gtttcataca ttagtagtag tagtagtacc atatactacg 420
   cagactctgt gaagggccga ttcaccatct ccagagacaa tgccaagaac tcactgtatc 480
   tgcaaatgaa cagcctgaga gacgaggaca cggctgtgta ttactgtgcg aga 533
<210> 270
   <211> 540
   <212> DNA
   <213> homo sapiens;
<400> 270
   agctctggga gaggagcccc agccgtgaga ttcccaggag tttccacttg gtgatcagca 60
   ctgaacacag accaccaacc atggagtttg ggcttagctg ggttttcctt gttgctattt 120
   taaaaggtaa ttcatggtgt actagagata ctgagtgtga ggggacatga gtggtagaaa 180
   cagtggatat gtgtggcagt ttctgacctt ggtgtttctg tgtttgcagg tgtccaatgt 240
   gaggtgcagc tggtggagtc tgggggaggc ttggtacagc cagggcggtc cctgagactc 300
   tcctgtacag cttctggatt cacctttggt gattatgcta tgagctggtt ccgccaggct 360
   ccagggaagg ggctggagtg ggtaggtttc attagaagca aagcttatgg tgggacaaca 420
   gaatacgccg cgtctgtgaa aggcagattc accatctcaa gagatgattc caaaagcatc 480
   gcctatctgc aaatgaacag cctgaaaacc gaggacacag ccgtgtatta ctgtactaga 540
<210> 271
   <211> 670
   <212> DNA
   <213> homo sapiens;
<400> 271
   aataccaatc tcccccagga cacttcatct gcacggagcc cggcctctcc tcagatgtcc 60
   caccccagag cttgctatat agtcggggac atccaaatag ggccctccct ctgctgatga 120
   aaaccagccc agctgaccct gcagctctgg gagaggagcc cagcactggg attccgaggt 180
   gtttccattc ggtgatcagc actgaacaca gaggactcac catggagttt tggctgagct 240
   gggttttcct tgttgctatt ttaaaaggtg attcatggag aactagagat attgagtgtg 300
   agtgaacacg agtgagagaa acagtggata tgtgtggcag tttctaacca atgtctctgt 360
   gtttgcaggt gtccagtgtg aggtgcagct ggtggagtct ggaggaggct tgatccagcc 420
   tggggggtcc ctgagactct cctgtgcagc ctctgggttc accgtcagta gcaactacat 480
   gagctgggtc cgccaggctc cagggaaggg gctggagtgg gtctcagtta tttatagcgg 540
   tggtagcaca tactacgcag actccgtgaa gggccgattc accatctcca gagacaattc 600
   caagaacacg ctgtatcttc aaatgaacag cctgagagcc gaggacacgg ccgtgtatta 660
   ctgtgcgaga 670
<210> 272
   <211> 534
   <212> DNA
   <213> homo sapiens;
<400> 272
   agctctggga gaggagcccc cgccctggga ttcccaggtg ttttcatttg gtgatcagca 60
   ctgaacacag aagagtcatg atggagtttg ggctgagctg ggttttcctt gttgctattt 120
   ttaaaggtga ttcatgagga aatagagata ttgagtgtga gtggacatga gtgagagaaa 180
   cagtggattt gtgtggcagt ttctgacctt ggtgtctctg tgtttgcagg tgtccagtgt 240
   gaggtgcagc tggtggagtc tggggaaggc ttggtccagc ctggggggtc cctgagactc 300
   tcctgtgcag cctctggatt caccttcagt agctatgcta tgcactgggt ccgccaggct 360
   ccagggaagg gactggaata tgtttcagct attagtagta atgggggtag cacatattat 420
   gcagactctg tgaagggcag attcaccatc tccagagaca attccaagaa cacgctgtat 480
   cttcaaatgg gcagcctgag agctgaggac atggctgtgt attactgtgc gaga 534
<210> 273
   <211> 528
   <212> DNA
   <213> homo sapiens;
<400> 273
   agctctggga gaggagccca gcactgggat tccgaggtgt ttccattcag tgatctgcac 60
   tgaacacaga ggactcgcca tggagtttgg gctgagctgg gttttccttg ttgctatttt 120
   aaaaggtgat tcatggagaa ctagagatat tgagtgtgag tgaacacgag tgagagaaac 180
   agtggatatg tgtggcagtt tctaaccaat gtctctgtgt ttgcaggtgt ccagtgtgag 240
   gtgcagctgg tggagtctgg aggaggcttg atccagcctg gggggtccct gagactctcc 300
   tgtgcagcct ctgggttcac cgtcagtagc aactacatga gctgggtccg ccaggctcca 360
   gggaaggggc tggagtgggt ctcagttatt tatagctgtg gtagcacata ctacgcagac 420
   tccgtgaagg gccgattcac catctccaga gacaattcca agaacacgct gtatcttcaa 480
   atgaacagcc tgagagctga ggacacggct gtgtattact gtgcgaga 528
<210> 274
   <211> 533
   <212> DNA
   <213> homo sapiens;
<400> 274
   aggtctcaga gaggagcctt agccctggac tccaaggcct ttccacttgg tgatcagcac 60
   tgagcacaga ggactcacca tggaattggg gctgagctgg gttttccttg ttgctatttt 120
   agaaggtgat tcatggaaaa ctaggaagat tgagtgtgtg tggatatgag tgtgagaaac 180
   agtggatttg tgtggcagtt tctgaccttg gtgtctcttt gtttgcaggt gtccagtgtg 240
   aggtgcagct ggtggagtct gggggaggct tggtccagcc tggggggtcc ctgagactct 300
   cctgtgcagc ctctggattc acctttagta gctattggat gagctgggtc cgccaggctc 360
   cagggaaggg gctggagtgg gtggccaaca taaagcaaga tggaagtgag aaatactatg 420
   tggactctgt gaagggccga ttcaccatct ccagagacaa cgccaagaac tcactgtatc 480
   tgcaaatgaa cagcctgaga gccgaggaca cggctgtgta ttactgtgcg aga 533
<210> 275
   <211> 540
   <212> DNA
   <213> homo sapiens;
<400> 275
   agctctgaga gcggagcccc agccccagaa ttcccaggtg ttttcatttg gtgatcagca 60
   ctgaacacag aggactcacc atggagtttg ggctgagctg ggttttcctt gttgttattt 120
   tacaaggtga tttatggaga actagagatg ttaagtgtga gtggacgtga gtgagagaaa 180
   cagtggattt gtgtgacagt ttctgaccag ggtgtctctg tgtttgcagg tgtccagtgt 240
   gaggtgcagc tggtggagtc tgggggaggc ttggtccagc ctggagggtc cctgagactc 300
   tcctgtgcag cctctggatt caccttcagt gaccactaca tggactgggt ccgccaggct 360
   ccagggaagg ggctggagtg ggttggccgt actagaaaca aagctaacag ttacaccaca 420
   gaatacgccg cgtctgtgaa aggcagattc accatctcaa gagatgattc aaagaactca 480
   ctgtatctgc aaatgaacag cctgaaaacc gaggacacgg ccgtgtatta ctgtgctaga 540
<210> 276
   <211> 540
   <212> DNA
   <213> homo sapiens;
<400> 276
   agctctggga gaggagctcc agccttggga ttcccagctg tctccactcg gtgatcggca 60
   ctgaatacag gagactcacc atggagtttg ggctgagctg ggttttcctt gttgctattt 120
   taaaaggtga ttcatgggga actagagata ctgagtgtga gtggacatga gtgagagaaa 180
   cagtggacgt gtgtggcact ttctgaccag ggtgtctctg tgtttgcagg tgtccagtgt 240
   gaggtgcagc tggtggagtc cgggggaggc ttggtccagc ctggggggtc cctgaaactc 300
   tcctgtgcag cctctgggtt caccttcagt ggctctgcta tgcactgggt ccgccaggct 360
   tccgggaaag ggctggagtg ggttggccgt attagaagca aagctaacag ttacgcgaca 420
   gcatatgctg cgtcggtgaa aggcaggttc accatctcca gagatgattc aaagaacacg 480
   gcgtatctgc aaatgaacag cctgaaaacc gaggacacgg ccgtgtatta ctgtactaga 540
<210> 277
   <211> 690
   <212> DNA
   <213> homo sapiens;
<400> 277
   aatttctcaa atcccattgt tgtcacccat cttcctcagg acactttcat ctgccctggg 60
   tcctgctctt tcttcaggtg tctcacccca gagcttgata tatagtagga gacatgcaaa 120
   tagggccctc actctgctga agaaaaccag ccctgcagct ctgggagagg agccccagcc 180
   ctgggattcc cagctgtttc tgcttgctga tcaggactgc acacagagaa ctcaccatgg 240
   agtttgggct gagctgggtt ttccttgttg ctattttaaa aggtgattca tggagaactg 300
   gagatatgga gtgtgaatgg acatgagtga gataagcagt ggatgtgtgt ggcagtttct 360
   gaccagggtg tctctgtgtt tgcaggtgtc cagtgtgagg tgcagctggt ggagtccggg 420
   ggaggcttag ttcagcctgg ggggtccctg agactctcct gtgcagcctc tggattcacc 480
   ttcagtagct actggatgca ctgggtccgc caagctccag ggaaggggct ggtgtgggtc 540
   tcacgtatta atagtgatgg gagtagcaca agctacgcgg actccgtgaa gggccgattc 600
   accatctcca gagacaacgc caagaacacg ctgtatctgc aaatgaacag tctgagagcc 660
   gaggacacgg ctgtgtatta ctgtgcaaga 690
<210> 278
   <211> 525
   <212> DNA
   <213> homo sapiens;
<400> 278
   agctctggga gaggagcccc agccctgaga ttcccaggtg tttccattca gtgatcagca 60
   ctgaacacag aggactcacc atggagttgg gactgagctg gattttcctt ttggctattt 120
   taaaaggtga ttcatggaga aatagagaga ttgagtgtga gtggacatga gtggatttgt 180
   gtggcagttt ctgaccttgg tgtctctgtg tttgcaggtg tccagtgtga agtgcagctg 240
   gtggagtctg ggggaggctt ggtacagcct ggcaggtccc tgagactctc ctgtgcagcc 300
   tctggattca cctttgatga ttatgccatg cactgggtcc ggcaagctcc agggaagggc 360
   ctggagtggg tctcaggtat tagttggaat agtggtagca taggctatgc ggactctgtg 420
   aagggccgat tcaccatctc cagagacaac gccaagaact ccctgtatct gcaaatgaac 480
   agtctgagag ctgaggacac ggccttgtat tactgtgcaa aagat 525
<210> 279
   <211> 505
   <212> DNA
   <213> homo sapiens;
<400> 279
   atttccttaa attcagggtc ctgctcacat gggaaatact ttctgagagt cctggacctc 60
   ctgtgcaaga acatgaaaca cctgtggttc ttcctcctgc tggtggcagc tcccagatgt 120
   gagtgtctca aggctgcaga catggagata tgggaggtgc ctctgagccc agggctcact 180
   gtgggtctct ctgttcacag tggtcctgtc ccaggtgcag ctgcaggagt cgggcccagg 240
   actggtgaag ccttcggaca ccctgtccct cacctgcgct gtctctggtt actccatcag 300
   cagtagtaac tggtggggct ggatccggca gcccccaggg aagggactgg agtggattgg 360
   gtacatctat tatagtggga gcacctacta caacccgtcc ctcaagagtc gagtcaccat 420
   gtcagtagac acgtccaaga accagttctc cctgaagctg agctctgtga ccgccgtgga 480
   cacggccgtg tattactgtg cgaga 505
<210> 280
   <211> 508
   <212> DNA
   <213> homo sapiens;
<400> 280
   atttccttaa attcagggtc ctgctcacat gggaaatact ttctgagagt cctggacctc 60
   ctgtgcaaga acatgaaaca cctgtggttc ttcctcctgc tggtggcagc tcccagatgt 120
   gagtgtctca aggctgcaga catggagata tgggaggtgc ctctgatccc agggctcact 180
   gtgtgtctct ctgttcacag gggtcctgcc ccaggtgcag ctgcaggagt cgggcccagg 240
   actggtgaag ccttcacaga ccctgtccct cacctgtact gtctctggtg gctccatcag 300
   cagtggtggt tactactgga gctggatccg ccagcaccca gggaagggcc tggagtggat 360
   tgggtacatc tattacagtg ggagcaccta ctacaacccg tccctcaaga gtcgagttac 420
   catatcagta gacacgtcta agaaccagtt ctccctgaag ctgagctctg tgactgccgc 480
   ggacacggcc gtgtattact gtgcgaga 508
<210> 281
   <211> 483
   <212> DNA
   <213> homo sapiens;
<400> 281
   cagctcacat gggaagtgct ttctgagagt catggacctc ctgcacaaga acatgaaaca 60
   cctgtggttc ttcctcctcc tggtggcagc tcccagatgt gagtgtctca ggaatgcgga 120
   tatgaagata tgagatgctg cctctgatcc cagggctcac tgtgggtttc tctgttcaca 180
   ggggtcctgt cccaggtgca gctacagcag tggggcgcag gactgttgaa gccttcggag 240
   accctgtccc tcacctgcgc tgtctatggt gggtccttca gtggttacta ctggagctgg 300
   atccgccagc ccccagggaa ggggctggag tggattgggg aaatcaatca tagtggaagc 360
   accaactaca acccgtccct caagagtcga gtcaccatat cagtagacac gtccaagaac 420
   cagttctccc tgaagctgag ctctgtgacc gccgcggaca cggctgtgta ttactgtgcg 480
   aga 483
<210> 282
   <211> 508
   <212> DNA
   <213> homo sapiens;
<400> 282
   atttccttaa attcaggtcc aactcataag ggaaatgctt tctgagagtc atggatctca 60
   tgtgcaagaa aatgaagcac ctgtggttct tcctcctgct ggtggcggct cccagatgtg 120
   agtgtttcta ggatgcagac atggagatat gggaggctgc ctctgatccc agggctcact 180
   gtgggttttt ctgttcacag gggtcctgtc ccagctgcag ctgcaggagt cgggcccagg 240
   actggtgaag ccttcggaga ccctgtccct cacctgcact gtctctggtg gctccatcag 300
   cagtagtagt tactactggg gctggatccg ccagccccca gggaaggggc tggagtggat 360
   tgggagtatc tattatagtg ggagcaccta ctacaacccg tccctcaaga gtcgagtcac 420
   catatccgta gacacgtcca agaaccagtt ctccctgaag ctgagctctg tgaccgccgc 480
   agacacggct gtgtattact gtgcgaga 508
<210> 283
   <211> 494
   <212> DNA
   <213> homo sapiens;
<400> 283
   aaattcaggg tccagctcac atgggaaata ctttctgaga ctcatggacc tcctgcacaa 60
   gaacatgaaa cacctgtggt tcttcctcct gctggtggca gctcccagat gtgagtgtct 120
   caaggctgca gacatgggga tatgggaggt gcctctgatc ccagggctca ctgtgggtct 180
   ctctgttcac aggggtcctg tcccaggtgc agctgcagga gtcgggccca ggactggtga 240
   agccttcgga gaccctgtcc ctcacctgca ctgtctctgg tggctccatc agtagttact 300
   actggagctg gatccggcag cccgccggga agggactgga gtggattggg cgtatctata 360
   ccagtgggag caccaactac aacccctccc tcaagagtcg agtcaccatg tcagtagaca 420
   cgtccaagaa ccagttctcc ctgaagctga gctctgtgac cgccgcggac acggccgtgt 480
   attactgtgc gaga 494
<210> 284
   <211> 2025
   <212> DNA
   <213> homo sapiens;
<400> 284
   ttttcacctc tccatacaaa ggcaccaccc acatgcaaat cctcacttaa gcacccacag 60
   gaaaccacca cacatttcct taaattcagg ttccagctca catgggaaat actttctgag 120
   agtcctggac ctcctgtgca agaacatgaa acatctgtgg ttcttccttc tcctggtggc 180
   agctcccaga tgtgagtatc tcagggatcc agacatgggg atatgggagg tgcctctgat 240
   cccagggctc actgtgggtc tctctgttca caggggtcct gtcccaggtg cagctgcagg 300
   agtcgggccc aggactggtg aagccttcgg agaccctgtc cctcacctgc actgtctctg 360
   gtggctccat cagtagttac tactggagct ggatccggca gcccccaggg aagggactgg 420
   agtggattgg gtatatctat tacagtggga gcaccaacta caacccctcc ctcaagagtc 480
   gagtcaccat atcagtagac acgtccaaga accagttctc cctgaagctg agctctgtga 540
   ccgctgcgga cacggccgtg tattactgtg cgagagacac agtgagggga ggtgagtgtg 600
   agcccagaca aaaacctccg tgcagggagg cggaggggac cggcgcaggt gctgctcagc 660
   gccagcaggg ggcgcgcggg gcccacagag caggaggccc ggtcaggagc aggtgcaggg 720
   agggcggggc ttcctcatct gctcagtggt ctccctcctc gccagcacct cagctgtccc 780
   caggggtcct ctttctttat tatctgtggt tctgcttcct cacattcttg tgccaagaaa 840
   gaaatgagga agacaaattt tcgtctgtag ttgaagtttc accaattact aggaactttc 900
   ctagaagttc ctgcatggcc cattatagct tacagattaa atatatatca agcttctcat 960
   ctcttgattt gtgtcatcaa ctgaattgtg ccctctttga aattcatatg cagaaacctt 1020
   aaattcaatt gatgtatatt ggaattttaa tgaaataatt aaggttaaat gtggtcataa 1080
   gtgtaagact ctaattcaac agacgtgtcg tctttataag aagaggaaga gacaccagag 1140
   acctctcact tttcacgtgc aggcagagaa gaggccatgt ggagacgtaa tgcactagaa 1200
   ggtggcccag tgcaagccag gaagaagcct caccaagaac caaccctgcc agaacattga 1260
   tcttcaacat tcagactgca gaattttaag aaaatcaata tttgttgttt aagccaccca 1320
   ctcctgttgt cttcttatga agatccagac agactaatac cacataactc tgttagcgct 1380
   gtcccctgga tgcagaatca gcccgctggg gctgggcaca tctctcagat ttccacataa 1440
   agtaggcaaa aaatagtagt tctgatataa aaatttgtca tgtccctgtt ggccaatttc 1500
   tgggcaaggt cttttaaaga agccctgggg gctttgtcac aaaagttgcc ttttatcatt 1560
   tattaggaca taactgatga acaatgagta ccagttggat ggagactgac cactgaccat 1620
   cttctgctgt ctcctaagta tgccacagaa aaccacacca acattactct atgtcttcaa 1680
   ctttctaaat ttgcactgat tggtatttaa ggcaggccca gcgttgaata actcctttag 1740
   tttttgcttc tctgggaaag gtcttatcta tcctggcctt ggtcttcaag tttcagcaat 1800
   tctgggaagc caaggacgcc tctatctcct cctccatgct ctgcaactca cctgagaaca 1860
   gctttctcat tggaatgtct tctgtttaag gaataagagt ccctgtttca ggcttgggtg 1920
   cctgagtaca cctactggat ccagcccagg attggagaaa ctttccagaa cacatcacct 1980
   gagaaatgac cagtcacact gttacacttt cacaatttcc gcttc 2025
<210> 285
   <211> 537
   <212> DNA
   <213> homo sapiens;
<400> 285
   acttaagcac ccacaggaaa ccaccacaca tttccttaaa ttcaggttcc agctcacatg 60
   ggaaatactt tctgagagtc ctggacctcc tgtgcaagaa catgaaacac ctgtggttct 120
   tcctcctcct ggtggcagct cccagatgtg agtgtctcag ggatccagac atgggggtat 180
   gggaggtgcc tctgatccca gggctcactg tgggtctctc tgttcacagg ggtcctgtcc 240
   caggtgcagc tgcaggagtc gggcccagga ctggtgaagc cttcggagac cctgtccctc 300
   acctgcactg tctctggtgg ctccgtcagc agtggtggtt actactggag ctggatccgg 360
   cagcccccag ggaagggact ggagtggatt gggtatatct attacagtgg gagcaccaac 420
   tacaacccct ccctcaagag tcgagtcacc atatcagtag acacgtccaa gaaccagttc 480
   tccctgaagc tgagctctgt gaccgctgcg gacacggccg tgtattactg tgcgaga 537
<210> 286
   <211> 493
   <212> DNA
   <213> homo sapiens;
<400> 286
   tgagtctccc tcactgccca gctgggatct cagggcttca ttttctgtcc tccaccatca 60
   tggggtcaac cgccatcctc gccctcctcc tggctgttct ccaaggtcag tcctgccgag 120
   ggcttgaggt cacagaggag aacgggtgga aaggagcccc tgattcaaat tttgtgtctc 180
   ccccacagga gtctgtgccg aggtgcagct ggtgcagtct ggagcagagg tgaaaaagcc 240
   cggggagtct ctgaagatct cctgtaaggg ttctggatac agctttacca gctactggat 300
   cggctgggtg cgccagatgc ccgggaaagg cctggagtgg atggggatca tctatcctgg 360
   tgactctgat accagataca gcccgtcctt ccaaggccag gtcaccatct cagccgacaa 420
   gtccatcagc accgcctacc tgcagtggag cagcctgaag gcctcggaca ccgccatgta 480
   ttactgtgcg aga 493
<210> 287
   <211> 498
   <212> DNA
   <213> homo sapiens;
<400> 287
   gcagagcctg ctgaattctg gctgaccagg gcagtcacca gagctccaga caatgtctgt 60
   ctccttcctc atcttcctgc ccgtgctggg cctcccatgg ggtcagtgtc agggagatgc 120
   cgtattcaca gcagcattca cagactgagg ggtgtttcac tttgctgttt ccttttgtct 180
   ccaggtgtcc tgtcacaggt acagctgcag cagtcaggtc caggactggt gaagccctcg 240
   cagaccctct cactcacctg tgccatctcc ggggacagtg tctctagcaa cagtgctgct 300
   tggaactgga tcaggcagtc cccatcgaga ggccttgagt ggctgggaag gacatactac 360
   aggtccaagt ggtataatga ttatgcagta tctgtgaaaa gtcgaataac catcaaccca 420
   gacacatcca agaaccagtt ctccctgcag ctgaactctg tgactcccga ggacacggct 480
   gtgtattact gtgcaaga 498
<210> 288
   <211> 489
   <212> DNA
   <213> homo sapiens;
<400> 288
   acccaacaac aacatccctc cttgggagaa tcccctagag cacagctcct caccatggac 60
   tggacctgga gcatcctctt cttggtggca gcagcaacag gtaaggggct ccccagtctc 120
   ggggttgagg cagaaaccag gccactcaag tgaggcttta cccacccctg tgtcctctcc 180
   acaggtacct actcccaggt gcagctggtg cagtctggcc atgaggtgaa gcagcctggg 240
   gcctcagtga aggtctcctg caaggcttct ggttacagtt tcaccaccta tggtatgaat 300
   tgggtgccac aggcccctgg acaagggctt gagtggatgg gatggttcaa cacctacact 360
   gggaacccaa catatgccca gggcttcaca ggacggtttg tcttctccat ggacacctct 420
   gccagcacag catacctgca gatcagcagc ctaaaggctg aggacatggc catgtattac 480
   tgtgcgaga 489
<210> 289
   <211> 38
   <212> DNA
   <213> homo sapiens;
<400> 289
   gtacactttt ggccagggga ccaagctgga gatcaaac 38
<210> 290
   <211> 38
   <212> DNA
   <213> homo sapiens;
<400> 290
   attcactttc ggccctggga ccaaagtgga tatcaaac 38
<210> 291
   <211> 37
   <212> DNA
   <213> homo sapiens;
<400> 291
   ctcactttcg gcggagggac caaggtggag atcaaac 37
<210> 292
   <211> 38
   <212> DNA
   <213> homo sapiens;
<400> 292
   gatcaccttc ggccaaggga cacgactgga gattaaac 38
<210> 293
   <211> 503
   <212> DNA
   <213> homo sapiens;
<400> 293
   aggaatcaga cccagtcagg acacagcatg gacatgagag tcctcgctca gctcctgggg      60
   ctcctgctgc tctgtttccc aggtaaggat ggagaacact agcagtttac tcagcccagg     120
   gtgctcagta ctgctttact attcagggaa attctcttac aacatgatta attgtgtgga     180
   catttgtttt tatgtttcca atctcaggtg ccagatgtga catccagatg acccagtctc     240
   catcctcact gtctgcatct gtaggagaca gagtcaccat cacttgtcgg gcgagtcagg     300
   gcattagcaa ttatttagcc tggtttcagc agaaaccagg gaaagcccct aagtccctga     360
   tctatgctgc atccagtttg caaagtgggg tcccatcaaa gttcagcggc agtggatctg     420
   ggacagattt cactctcacc atcagcagcc tgcagcctga agattttgca acttattact     480
   gccaacagta taatagttac cct                                             503
<210> 294
   <211> 503
   <212> DNA
   <213> homo sapiens;
<400> 294
   aggaatcagt cccactcagg acacagcatg gacatgaggg tccccgctca gctcctgggg 60
   ctcctgctgc tctggttccc aggtaaggat ggagaacact agcagtttac tcagcccaga 120
   gtgctcagta ctgctttact gttcagggaa attctcttac aacatgatta attgtgtgga 180
   catttgtttt tatgtttcca atctcaggtg ccaggtgtga catccagatg acccagtctc 240
   catcctccct gtctgcatct gtaggagaca gagtcaccat cacttgccgg gcaagtcagg 300
   gcattagaaa tgatttaggc tggtatcagc agaaaccagg gaaagcccct aagcgcctga 360
   tctatgctgc atccagtttg caaagtgggg tcccatcaag gttcagcggc agtggatctg 420
   ggacagaatt cactctcaca atcagcagcc tgcagcctga agattttgca acttattact 480
   gtctacagca taatagttac cct 503
<210> 295
   <211> 657
   <212> DNA
   <213> homo sapiens;
<400> 295
   gggacacctg gggacactga gctggtgctg agttactgag atgagccagc tctgcagctg 60
   tgcccagcct gccccatccc ctgctcattt gcatgttccc agagcacaac ctcctgccct 120
   gaagccttat taataggctg gtcacacttt gtgcaggagt cagacccagt caggacacag 180
   catggacatg agggtccccg ctcagctcct ggggctcctg ctgctctggc tcccaggtaa 240
   ggaaggagaa cactaggaat ttactcagcc cagtgtgctc agtactgcct ggttattcag 300
   ggaagtcttc ctataatatg atcaatagta tgaatatttg tgtttctatt tccaatctca 360
   ggtgccaaat gtgacatcca gatgacccag tctccttcca ccctgtctgc atctgtagga 420
   gacagagtca ccatcacttg ccgggccagt cagagtatta gtagctggtt ggcctggtat 480
   cagcagaaac cagggaaagc ccctaagctc ctgatctata aggcgtctag tttagaaagt 540
   ggggtcccat caaggttcag cggcagtgga tctgggacag aattcactct caccatcagc 600
   agcctgcagc ctgatgattt tgcaacttat tactgccaac agtataatag ttattct 657
<210> 296
   <211> 506
   <212> DNA
   <213> homo sapiens;
<400> 296
   gcaggagtca gacccactca ggacacagca tggacatgag ggtccccgct cagctcctgg        60
   ggctcctgct gctctggctc ccaggtaagg atggagaaca ctggcagttt actcagccca       120
   gggtgctcag cacagcctgg ctattcaggg aaattctctt actacatgat taattgtgtg       180
   gaccatttgt ttttgtgttt ccaatctcag gtgccagatg tgccatccag atgacccagt       240
   ctccatcctc cctgtctgca tctgtaggag acagagtcac catcacttgc cgggcaagtc       300
   agggcattag aaatgattta ggctggtatc agcagaaacc agggaaagcc cctaagctcc       360
   tgatctatgc tgcatccagt ttacaaagtg gggtcccatc aaggttcagc ggcagtggat       420
   ctggcacaga tttcactctc accatcagca gcctgcagcc tgaagatttt gcaacttatt       480
   actgtctaca agattacaat taccct                                            506
<210> 297
   <211> 523
   <212> DNA
   <213> homo sapiens;
<400> 297
   aggctggaca cacttcatgc aggagtcaga ccctgtcagg acacagcata gacatgaggg        60
   tccccgctca gctcctgggg ctcctgctgc tctggctccc aggtaaggaa ggagaacact       120
   aggaatttac tcagcccagt gtgcttggta cagcctggcc cttcagggaa gttctcttac       180
   aacatgatta attgtatgga catttgtttt tatgtttcca atctcaggtg ccagatgtgc       240
   catccggatg acccagtctc catcctcatt ctctgcatct acaggagaca gagtcaccat       300
   cacttgtcgg gcgagtcagg gtattagcag ttatttagcc tggtatcagc aaaaaccagg       360
   gaaagcccct aagctcctga tctatgctgc atccactttg caaagtgggg tcccatcaag       420
   gttcagcggc agtggatctg ggacagattt cactctcacc atcagctgcc tgcagtctga       480
   agattttgca acttattact gtcaacagta ttatagttac cct                         523
<210> 298
   <211> 534
   <212> DNA
   <213> homo sapiens;
<400> 298
   agacttctta ataggctggt cacacctgtg caggagtcag tcccagtcag gacacagcat 60
   ggacatgagg gtccccgctc agctcctggg gctcctgctg ctctggctcc caggtaagga 120
   aggagaacac taggaattta ctcagcccag tgtgttccgt acagcctggc tcttgaggga 180
   agttctctta caacatgatt aattctatgg acatttgtgt ttatatttcc aatctcaggt 240
   gccagatgtg acatccagtt gacccagtct ccatccttcc tgtctgcatc tgtaggagac 300
   agagtcacca tcacttgccg ggccagtcag ggcattagca gttatttagc ctggtatcag 360
   caaaaaccag ggaaagcccc taagctcctg atctatgctg catccacttt gcaaagtggg 420
   gtcccatcaa ggttcagcgg cagtggatct gggacagaat tcactctcac aatcagcagc 480
   ctgcagcctg aagattttgc aacttattac tgtcaacagc ttaatagtta ccct 534
<210> 299
   <211> 656
   <212> DNA
   <213> homo sapiens;
<400> 299
   gggacacctg gggacactga gctggtgctg agttactgag atgagccagc cctgcagctg 60
   cgcccagcct gccccatccc ctgctcattt gcatgttccc agagcacagt ctcctgacct 120
   gaagacttat taacaggctg atcacaccct gtgcaggagt cagacccagt caggacacag 180
   catggacatg agggtccccg ctcagctcct ggggctcctg ctgctctggt tcccaggtaa 240
   gaaaggagaa cactaggatt atactcggtc agtgtgctga gtactgcttt actattcagg 300
   gaacttctct tacagcatga ttaattgtgt ggacatttgt ttttatgttt ccaatctcag 360
   gttccagatg cgacatccag atgacccagt ctccatcttc tgtgtctgca tctgtaggag 420
   acagagtcac catcacttgt cgggcgagtc agggtattag cagctggtta gcctggtatc 480
   agcagaaacc agggaaagcc cctaagctcc tgatctatgc tgcatccagt ttgcaaagtg 540
   gggtcccatc aaggttcagc ggcagtggat ctgggacaga tttcactctc actatcagca 600
   gcctgcagcc tgaagatttt gcaacttact attgtcaaca ggctaacagt ttccct 656
<210> 300
   <211> 503
   <212> DNA
   <213> homo sapiens;
<400> 300
   aggaatcaga cccagtcagg acacagcatg gacatgaggg tcctcgctca gctcctgggg 60
   ctcctgctgc tctgtttccc aggtaaggat ggagaacact agcagtttac tcagcccagg      120
   gtgctcagta ctgctttact attcagggaa attctcttac aacatgatta attgtgtgga      180
   catttgtttt tatgtttcca atctcaggtg ccagatgtga catccagatg acccagtctc      240
   catcctcact gtctgcatct gtaggagaca gagtcaccat cacttgtcgg gcgagtcagg      300
   gtattagcag ctggttagcc tggtatcagc agaaaccaga gaaagcccct aagtccctga      360
   tctatgctgc atccagtttg caaagtgggg tcccatcaag gttcagcggc agtggatctg      420
   ggacagattt cactctcacc atcagcagcc tgcagcctga agattttgca acttattact      480
   gccaacagta taatagttac cct                                               503
<210> 301
   <211> 657
   <212> DNA
   <213> homo sapiens;
<400> 301
   gggacacctg gggacactga gctgctgctg agttactgag atgagccagc cctgcagctg       60
   cgcccagcct gccccatccc ctgctcattt gcatgttccc agagcatagc ctcctgccct      120
   gaagccttat taataggctg gacacacttc atggaggaat cagtcccact caggacacag      180
   catggacatg agggtccctg ctcagctcct ggggctcctg ctgctctggt tcccaggtaa      240
   ggatggagaa cactaacagt ttactcagcc cagagtgctc agtactgctt tactgttcag      300
   ggaaattctc ttacaacatg attaattgtg tggacatttg tttttatgtt tccaatctca      360
   ggtgccagat gtaacatcca gatgacccag tctccatctg ccatgtctgc atctgtagga      420
   gacagagtca ccatcacttg tcgggcgagg cagggcatta gcaattattt agcctggttt      480
   cagcagaaac cagggaaagt ccctaagcac ctgatctatg ctgcatccag tttgcaaagt      540
   ggggtcccat caaggttcag cggcagtgga tctgggacag aattcactct cacaatcagc      600
   agcctgcagc ctgaagattt tgcaacttat tactgtctac agcataatag ttaccct         657
<210> 302
   <211> 487
   <212> DNA
   <213> homo sapiens;
<400> 302
   agtcccagtc aggacacagc atggacatga gggtccccgc tcagctcctg gggctcctgc 60
   tgctctggct cccaggtaag gaaggagaac actaggaatt ttcttagccc actgtgctct 120
   ggcacttctg ggaagttctc ttataccatg attcatggtg tggatatttg tttttatgtt 180
   tccaatctca ggtgtcagat ttgacatcca gatgatccag tctccatctt tcctgtctgc 240
   atctgtagga gacagagtca gtatcatttg ctgggcaagt gagggcatta gcagtaattt 300
   agcctggtat ctgcagaaac cagggaaatc ccctaagctc ttcctctatg atgcaaaaga 360
   tttgcaccct ggggtctcat cgaggttcag tggcagggga tctgggacgg atttcactct 420
   caccatcatc agcctgaagc ctgaagattt tgcagcttat tactgtaaac aggacttcag 480
   ttaccct 487
<210> 303
   <211> 657
   <212> DNA
   <213> homo sapiens;
<400> 303
   gggacacctg gggacactga gctggtgctg agttactgag atgaaccagc cctgcagctg 60
   tgcccagcct gccttgcccc ctgctaattt gcatgttccc agagcacatc ctcctaccct 120
   gaagacttat taatgcgctg gtcacacttc atgcaggagt cagacccagt caggacacag 180
   catggacatg agggtgcccg ctcagcgcct ggggctcctg ctgctctggt tcccaggtaa 240
   ggaaggagaa ccctagcagt ttactcagcc cagtgtgttc cgtacagcct ggctcttgag 300
   ggaagttctc ttacaacatg attaattgta tggacatttg tgtttatatt tccaatctca 360
   ggtgccagat gtgccatccg gatgacccag tctccattct ccctgtctgc atctgtagga 420
   gacagagtca ccatcacttg ctgggccagt cagggcatta gcagttattt agcctggtat 480
   cagcaaaaac cagcaaaagc ccctaagctc ttcatctatt atgcatccag tttgcaaagt 540
   ggggtcccat caaggttcag cggcagtgga tctgggacgg attacactct caccatcagc 600
   agcctgcagc ctgaagattt tgcaacttat tactgtcaac agtattatag tacccct 657
<210> 304
   <211> 656
   <212> DNA
   <213> homo sapiens;
<400> 304
   gggacacctg gggacactga gctggtgctg agttactgag atgagccagc tctgcagctg 60
   tgcccagtca gccccatccc ctgctcattt gcatgttccc agagcacaac ctcctgcact 120
   gaagccttat taataggctg gccacacttc atgcaggagt cagacccagt caggacacag 180
   catggacatg agggtccccg ctcagctcct ggggctcctg ctgctctggc tcccaggtaa 240
   ggaaggagaa cactatgaat ttactcagcc aatgtgctca gtacagcctg gcccttcagg 300
   gaaattctct tactacatga ttaattgtat ggatatttgt ttttatgttt ccaatctcag 360
   gtgccagatg tgtcatctgg atgacccagt ctccatcctt actctctgca tctacaggag 420
   acagagtcac catcagttgt cggatgagtc agggcattag cagttattta gcctggtatc 480
   agcaaaaacc agggaaagcc cctgagctcc tgatctatgc tgcatccact ttgcaaagtg 540
   gggtcccatc aaggttcagt ggcagtggat ctgggacaga tttcactctc accatcagtt 600
   gcctgcagtc tgaagatttt gcaacttatt actgtcaaca gtattatagt ttccct 656
<210> 305
   <211> 833
   <212> DNA
   <213> homo sapiens;
<400> 305
   aattaggact cctcaggtca ccttctcaca atgaggctcc ttgctcagct tctggggctg 60
   ctaatgctct gggtccctgg tgaggacaga agagagatga gggaggagaa tggggtggga 120
   gggtgaactc tgggggcccc attgcctccc atgtgtgttc tgtcctcatg ttagatgtgt 180
   acgtcttgta ctccaggatg gggcttgtaa cttttatatc tgcgtgagta aggcatgtga 240
   ggtttagatc tgtaagaatg aggaagattc cagaaggaac aaagaccagt gctccggtga 300
   agactctaac agagaaagag ggaatggtag aggaaacttc tagcactcaa agcactctgc 360
   tgtgctttga aaatatgttt ttattttgaa attatatatt actagggtct gaatcaaatt 420
   ataaaaattg atttagcctg aaataaataa cagaagaaaa attattttaa aattgtgctt 480
   aaagtttcta cataaccttg cacttctctc tcattatttc aggatccagt ggggatattg 540
   tgatgaccca gactccactc tcctcacctg tcacccttgg acagccggcc tccatctcct 600
   gcaggtctag tcaaagcctc gtacacagtg atggaaacac ctacttgagt tggcttcagc 660
   agaggccagg ccagcctcca agactcctaa tttataagat ttctaaccgg ttctctgggg 720
   tcccagacag attcagtggc agtggggcag ggacagattt cacactgaaa atcagcaggg 780
   tggaagctga ggatgtcggg gtttattact gcatgcaagc tacacaattt cct 833
<210> 306
   <211> 816
   <212> DNA
   <213> homo sapiens;
<400> 306
   gatcaggact cctcagttca ccttctcaca atgaggctcc ctgctcagct cctggggctg 60
   ctaatgctct gggtcccagg taagggtaga agggagatga gggaggagaa tggcatggaa 120
   cggtgagttc tggggcccca ctgcctctaa caacagtgat ctctgggggt ctcactacac 180
   tcctatgtgt gttcctttcc tgtattggac atgcacatgt tgtcctccag agtggggcat 240
   gtgatgatca gatctgtgag agtgaggaag attcaagcag aaacaaggat ctgtgctctg 300
   gggaagactg acacagaaag gggatggtgt ggggtcttct ggagacccct ttgagccttg 360
   gatcccttga gttccatttt gaaactgtgt atttttgaaa tatgaacaaa tacatatata 420
   gcctgaaata aacaacaaat caaaatttat gaaaattaca cataaacttt atacataacc 480
   ttgctcttct ttctatttat ttcaggatcc agtggggatg ttgtgatgac tcagtctcca 540
   ctctccctgc ccgtcaccct tggacagccg gcctccatct cctgcaggtc tagtcaaagc 600
   ctcgtataca gtgatggaaa cacctacttg aattggtttc agcagaggcc aggccaatct 660
   ccaaggcgcc taatttataa ggtttctaac cgggactctg gggtcccaga cagattcagc 720
   ggcagtgggt caggcactga tttcacactg aaaatcagca gggtggaggc tgaggatgtt 780
   ggggtttatt actgcatgca aggtacacac tggcct 816
<210> 307
   <211> 833
   <212> DNA
   <213> homo sapiens;
<400> 307
   aattaggact cctcaggtca ccttctcaca atgaggctcc ttgctcagct tctggggctg      60
   ctaatgctct gggtccctgg tgaggacaga agagagatga gggaggagaa tggggtggga     120
   gggtgaactc tgggggcccc attgcctccc atgtgtgttc tgtcctcatg ttagatgtgt     180
   acgtcttgta ctccaggatg gggcttgtaa cttttatatc tgcgtgagta aggcatgtga     240
   ggtttagatc tgtaagaatg aggaagattc cagaaggaac aaagaccagt gctccggtga     300
   agactctaac agagaaagag ggaatggtag aggaaacttc tagcactcaa agcactctgc     360
   tgtgctttga aaatatgttt ttattttgaa attatatatt actagggtct gaatcaaatt     420
   ataaaaattg atttagcctg aaataaataa cagaagaaaa attattttaa aattgtgctt     480
   aaagtttcta cataaccttg cacttctctc tcattatttc aggatccagt ggggatattg     540
   tgatgaccca gactccactc tcctcgcctg tcacccttgg acagccggcc tccatctcct     600
   tcaggtctag tcaaagcctc gtacacagtg atggaaacac ctacttgagt tggcttcagc     660
   agaggccagg ccagcctcca agactcctaa tttataaggt ttctaaccgg ttctctgggg     720
   tcccagacag attcagtggc agtggggcag ggacagattt cacactgaaa atcagcaggg     780
   tggaagctga ggatgtcggg gtttattact gcacgcaagc tacacaattt cct            833
<210> 308
   <211> 781
   <212> DNA
   <213> homo sapiens;
<400> 308
   gatcaggact cctcagttca ccttctcact atgaggctcc ctgctcagct cttggggctg      60
   ctaatgctct gggtccctgg taaggacaga aggagatgag ggaggagaat ggggtgggaa     120
   ggtaagcctg gggaccccac tgccttccat gtgtgttctg ccctgcccat gtgttagatg     180
   tacaggtctt gttctccagg atggggaatg tgaggtttaa atctgtgaga gtgaggacga     240
   ttcaaaaaga agcaaggacc tgtgtgctct ggtgaatatc gtcacacaga gaaagggagg     300
   tggtgtaggt gacttctaga atcccctttg cagcttgcaa atttggaata tgtttagtgt     360
   ataaatacaa acaacaaaaa attatatagc ctgaaataaa aaatgaaaat ttatgataaa 420
   tgacacatga tatttgtaca tatccttcca cttctttcta tctattttag gatccagtgc 480
   agagattgtg atgacccaga ctccactctc cttgtctatc acccctggag agcaggcctc 540
   catgtcctgc aggtctagtc agagcctcct gcatagtgat ggatacacct atttgtattg 600
   gtttctgcag aaagccaggc cagtctccac gctcctgatc tatgaagttt ccaaccggtt 660
   ctctggagtg ccagataggt tcagtggcag cgggtcaggg acagatttca cactgaaaat 720
   cagccgggtg gaggctgagg attttggagt ttattactgc atgcaagatg cacaagatcc 780
   t 781
<210> 309
   <211> 758
   <212> DNA
   <213> homo sapiens;
<400> 309
   gatcaggact tctcagttca tcttctcacc atgaggctcc ctgctcagct cctggggctg 60
   ctaatgctct ggatacctgg taaggatgga aggagatgag ggaggaggag ggggtgggaa 120
   gctgagctct ggcggcccca ctgattcccg tgtttattct aaccatgtgt taaaggaata 180
   tggcctatgc tccagggaga ggaattcata ttttgccctg atgatgattt gaaaactcct 240
   aaaagcagtg ctctgaataa tatcttgaga aatgaaagaa ctcttgtgcc tatttaataa 300
   agggttcatt taaagagttt gtttttatga tatgaataca aatttgtaaa aataaaagat 360
   tagccataaa tcaataccat aaggcaaatc tcaaaagttg ttcattatgc tttcacataa 420
   ccttgcactt ctctctcata atttcaggat ccagtgcaga tattgtgatg acccagactc 480
   cactctctct gtccgtcacc cctggacagc cggcctccat ctcctgcaag tctagtcaga 540
   gcctcctgca tagtgatgga aagacctatt tgtattggta cctgcagaag ccaggccagc 600
   ctccacagct cctgatctat gaagtttcca accggttctc tggagtgcca gataggttca 660
   gtggcagcgg gtcagggaca gatttcacac tgaaaatcag ccgggtggag gctgaggatg 720
   ttggggttta ttactgcatg caaagtatac agcttcct 758
<210> 310
   <211> 821
   <212> DNA
   <213> homo sapiens;
<400> 310
   tgactgatca ggactcctca gttcaccttc tcacaatgag gctccctgct cagctcctgg 60
   ggctgctaat gctctgggtc ccaggtaagg gtagaaggga gatgagggag gagaatggca 120
   tggaacggtg agttctgggg ccccactgcc tctaacaaca gtgatctctg ggggtctcac 180
   tacactccta tgtgtgttcc tttcctgtat tggacatgca catgttgtcc tccagaatgg 240
   ggcatgtgat gatcagatct gtgagagtca ggaagattca agaagaaaca aggatctgtg 300
   ctctggggaa gactgacaca gaaaggggat ggtgtggggt cttctggaga cccctttgag 360
   ccttggatcc cttgagttcc attttgaaac tgtatatttt tgaaatatga acaaatacat 420
   atatagcctg agataaacaa caaatcaaaa tttatgaaaa ttacacataa actttataca 480
   taaccttgct cttctttcta tttatttcag gatccagtgg ggatgttgtg atgactcagt 540
   ctccactctc cctgcccgtc acccttggac agccggcctc catctcctgc aggtctagtc 600
   aaagcctcgt atacagtgat ggaaacacct acttgaattg gtttcagcag aggccaggcc 660
   aatctccaag gcgcctaatt tataaggttt ctaactggga ctctggggtc ccagacagat 720
   tcagcggcag tgggtcaggc actgatttca cactgaaaat cagcagggtg gaggctgagg 780
   atgttggggt ttattactgc atgcaaggta cacactggcc t 821
<210> 311
   <211> 561
   <212> DNA
   <213> homo sapiens;
<400> 311
   gtcagagccc tggggaggaa ctgctcagtt aggacccaga gggaaccatg gaagccccag 60
   ctcagcttct cttcctcctg ctactctggc tcccaggtga ggggaacatg aggtggtttt 120
   gcacattagt gaaaactctt gccacctctg ctcagcaaga aatataatta aaattcaaag 180
   tatatcaaca attttggctc tactcaaaga cagttggttt gatcttgatt acatgagtgc 240
   atttctgttt tatttccaat ttcagatacc accggagaaa ttgtgttgac acagtctcca 300
   gccaccctgt ctttgtctcc aggggaaaga gccaccctct cctgcagggc cagtcagagt 360
   gttagcagct acttagcctg gtaccaacag aaacctggcc aggctcccag gctcctcatc 420
   tatgatgcat ccaacagggc cactggcatc ccagccaggt tcagtggcag tgggtctggg 480
   acagacttca ctctcaccat cagcagccta gagcctgaag attttgcagt ttattactgt 540
   cagcagcgta gcaactggcc t 561
<210> 312
   <211> 587
   <212> DNA
   <213> homo sapiens;
<400> 312
   cctgggtcag agctctggag aagagctgct cagttaggac ccagagggaa ccatggaaac      60
   cccagcgcag cttctcttcc tcctgctact ctggctccca ggtgagggga acatgggatg     120
   gttttgcatg tcagtgaaaa ccctctcaag tcctgttacc tggcaactct gctcagtcaa     180
   tacaataatt aaagctcaat ataaagcaat aattctggct cttctgggaa gacaatgggt     240
   ttgatttaga ttacatgggt gacttttctg ttttatttcc aatctcagat accaccggag     300
   aaattgtgtt gacgcagtct ccaggcaccc tgtctttgtc tccaggggaa agagccaccc     360
   tctcctgcag ggccagtcag agtgttagca gcagctactt agcctggtac cagcagaaac     420
   ctggccaggc tcccaggctc ctcatctatg gtgcatccag cagggccact ggcatcccag     480
   acaggttcag tggcagtggg tctgggacag acttcactct caccatcagc agactggagc     540
   ctgaagattt tgcagtgtat tactgtcagc agtatggtag ctcacct                   587
<210> 313
   <211> 614
   <212> DNA
   <213> homo sapiens;
<400> 313
   gcatgtccct cccagctgcc ctaccttcca gagcccatat caatgcctgg gtcagagccc      60
   tgggaaggaa ctgctcagtt aggacccaga cggaaccatg gaagccccag ctcagcttct     120
   cttcctcctg ctactctggc tcccaggtga ggggaacatg aggtggtttt gcacatcagt     180
   gaaaactcct gccacctctg ctcagcaaga aatataatta aaattcaatg tagatcaaca     240
   attttggctc tactcaaaga cagctggttt gatctagatt acatgagtgc atttctgttt     300
   tatttccaat cttggatacc accagagaaa ttgtaatgac acagtctcca cccaccctgt     360
   ctttgtctcc aggggaaaga gtcaccctct cctgcagggc cagtcagagt gttagcagca     420
   gctacttaac ctggtatcag cagaaacctg gccaggcgcc caggctcctc atctatggtg     480
   catccaccag ggccactagc atcccagcca ggttcagtgg cagtgggtct gggacagact     540
   tcactctcac catcagcagc ctgcagcctg aagattttgc agtttattac tgtcagcagg     600
   attataactt acct                                                      614
<210> 314
   <211> 611
   <212> DNA
   <213> homo sapiens;
<400> 314
   gcatgtccct cccagctgcc ctaccttcca gagcccatat caatgcctgg gtcagagctc 60
   tggggaggaa ctgctcagtt aggacccaga cggaaccatg gaagccccag cgcagcttct 120
   cttcctcctg ctactctggc tcccaggtga ggggaatatg aggtgtcttt gcacatcagt 180
   gaaaactcct gccacctctg ctcagcaaga aatataatta aaattcaaaa tagatcaaca 240
   attttggctc tactcaaaga cagtgggttt gattttgatt acatgagtgc atttctgttt 300
   tatttccaat ttcagatacc accggagaaa ttgtgttgac acagtctcca gccaccctgt 360
   ctttgtctcc aggggaaaga gccaccctct cctgcagggc cagtcagggt gttagcagct 420
   acttagcctg gtaccagcag aaacctggcc aggctcccag gctcctcatc tatgatgcat 480
   ccaacagggc cactggcatc ccagccaggt tcagtggcag tgggcctggg acagacttca 540
   ctctcaccat cagcagccta gagcctgaag attttgcagt ttattactgt cagcagcgta 600
   gcaactggca t 611
<210> 315
   <211> 563
   <212> DNA
   <213> homo sapiens;
<400> 315
   gggtcagagc tctggggagg aactgctcag ttaggaccca gacggaacca tggaagcccc 60
   agcgcagctt ctcttcctcc tgctactctg gctcccaggt gaggggaata tgaggtggtt 120
   ttgcacatca gtgaaaactc ctgccacctc tgctcagcaa gaaatataat taaaattcaa 180
   tgtagatcaa caattttggc tctacttaaa gacagtgggt ttgattttga ttacatgagt 240
   gcatttctgt tttatttcca atttcagata ccactggaga aatagtgatg acgcagtctc 300
   cagccaccct gtctgtgtct ccaggggaaa gagccaccct ctcctgcagg gccagtcaga 360
   gtgttagcag caacttagcc tggtaccagc agaaacctgg ccaggctccc aggctcctca 420
   tctatggtgc atccaccagg gccactggca tcccagccag gttcagtggc agtgggtctg 480
   ggacagagtt cactctcacc atcagcagcc tgcagtctga agattttgca gtttattact 540
   gtcagcagta taataactgg cct 563
<210> 316
   <211> 632
   <212> DNA
   <213> homo sapiens;
<400> 316
   gcatgtccct cccagccgcc ctgcagtcca gagcccatat caatgcctgg gtcagagctc 60
   tggggaggaa ctgctcagtt aggacccaga gggaaccatg gaaaccccag cgcagcttct 120
   cttcctcctg ctactctggc tcccaggtga ggggaacatg ggatggtttt gcatgtcagt 180
   gaaaaccctc tcaagtcctg ttacctggca actctgctga atcaatacaa taattaaagc 240
   tcaatataaa gcaataattc tggctcttct gggaagacag tgggtttgat ttagattaca 300
   tgggtgactt ttctatttta tttccaatct cagataccac cggagaaatt gtgttgacgc 360
   agtctccagc caccctgtct ttgtctccag gggaaagagc caccctctcc tgcggggcca 420
   gtcagagtgt tagcagcagc tacttagcct ggtaccagca gaaacctggc ctggcgccca 480
   ggctcctcat ctatgatgca tccagcaggg ccactggcat cccagacagg ttcagtggca 540
   gtgggtctgg gacagacttc actctcacca tcagcagact ggagcctgaa gattttgcag 600
   tgtattactg tcagcagtat ggtagctcac ct 632
<210> 317
   <211> 757
   <212> DNA
   <213> homo sapiens;
<400> 317
   tttggctctt gatttacatt gggtactttc acaacccact gctcatgaaa tttgcttttg 60
   tactcactgg ttgtttttgc ataggcccct ccaggccacg accagctgtt tggattttat 120
   aaacgggccg tttgcattgt gaactgagct acaacaggca ggcaggggca gcaagatggt 180
   gttgcagacc caggtcttca tttctctgtt gctctggatc tctggtgagg aattaaaaag 240
   tgccacagtc ttttcagagt aatatctgtg tagaaataaa aaaaattaag atatagttgg 300
   aaataatgac tatttccaat atggatccaa ttatctgctg acttataata ctactagaaa 360
   gcaaatttaa atgacatatt tcaattatat ctgagacagc gtgtataagt ttatgtataa 420
   tcattgtcca ttactgacta caggtgccta cggggacatc gtgatgaccc agtctccaga 480
   ctccctggct gtgtctctgg gcgagagggc caccatcaac tgcaagtcca gccagagtgt 540
   tttatacagc tccaacaata agaactactt agcttggtac cagcagaaac caggacagcc 600
   tcctaagctg ctcatttact gggcatctac ccgggaatcc ggggtccctg accgattcag 660
   tggcagcggg tctgggacag atttcactct caccatcagc agcctgcagg ctgaagatgt 720
   ggcagtttat tactgtcagc aatattatag tactcct 757
<210> 318
   <211> 553
   <212> DNA
   <213> homo sapiens;
<400> 318
   ataaaatctg tgctgtcaaa ctgattagga actgactacc acctgcaggt cagggccaag      60
   gttatggggt cccaggttca cctcctcagc ttcctcctcc tttggatctc tggtaagaga     120
   aacacttcct ctcctctgtg ccaccaagtc ccctgcatat ccacaaaaat aatatatttt     180
   cataaggaat tgattttcct cattctctgc aaatatgatg catttgattt atgtttttta     240
   ctttgctcca taatcagata ccagggcaga aacgacactc acgcagtctc cagcattcat     300
   gtcagcgact ccaggagaca aagtcaacat ctcctgcaaa gccagccaag acattgatga     360
   tgatatgaac tggtaccaac agaaaccagg agaagctgct attttcatta ttcaagaagc     420
   tactactctc gttcctggaa tcccacctcg attcagtggc agcgggtatg gaacagattt     480
   taccctcaca attaataaca tagaatctga ggatgctgca tattacttct gtctacaaca     540
   tgataatttc cct                                                        553
<210> 319
   <211> 616
   <212> DNA
   <213> homo sapiens;
<400> 319
   atcttaaaag aggttctttc tctgggatgt ggcatgagca aaactgacaa gtcaaggcag      60
   gaagatgttg ccatcacaac tcattgggtt tctgctgctc tgggttccag gtgagaatat     120
   ttccacaaac ctaggcggag atattctttc aatctgtaat ttctttcatt ggggactctg     180
   caataggtga tttttggctt gattttaaaa tcctaatttt aaaaatgtaa tgcatattct     240
   ttcttcatgt ctagcaagat taaaggtgat tttcatacac agatatttat gttgtactga     300
   tgtttgctgt atattttcag cctccagggg tgaaattgtg ctgactcagt ctccagactt     360
   tcagtctgtg actccaaagg agaaagtcac catcacctgc cgggccagtc agagcattgg     420
   tagtagctta cactggtacc agcagaaacc agatcagtct ccaaagctcc tcatcaagta     480
   tgcttcccag tccttctcag gggtcccctc gaggttcagt ggcagtggat ctgggacaga      540
   tttcaccctc accatcaata gcctggaagc tgaagatgct gcaacgtatt actgtcatca     600
   gagtagtagt ttacct                                                     616
<210> 320
   <211> 619
   <212> DNA
   <213> homo sapiens;
<400> 320
   ggtatcttaa aagaggttct ttctctggga tgtggcatga gcaaaactga caagtcaagg 60
   caggaagatg tcgccatcac aactcattgg gtttctgctg ctctgggttc caggtgagaa 120
   tatttccaca aacctaggcg gagatattct ttcaatctgt aatttctttc attggggact 180
   ctgcaatagg tgatttttgg cttgatttta aaatcctaat tttaaaaatg taatgcatat 240
   tctttcttca tgtctagcaa gattaaaggt gattttcata cacagatatt tatgttgtac 300
   tgatgtttgc tgtatatttt cagcctccag gggtgaaatt gtgctgactc agtctccaga 360
   ctttcagtct gtgactccaa aggagaaagt caccatcacc tgccgggcca gtcagagcat 420
   tggtagtagc ttacactggt accagcagaa accagatcag tctccaaagc tcctcatcaa 480
   gtatgcttcc cagtccatct caggggtccc ctcgaggttc agtggcagtg gatctgggac 540
   agatttcacc ctcaccatca atagcctgga agctgaagat gctgcagcgt attactgtca 600
   tcagagtagt agtttacct 619
<210> 321
   <211> 577
   <212> DNA
   <213> homo sapiens;
<400> 321
   agcaaaactg aagtcaaaac actgagatgg tgtccccgtt gcaattcctg cggcttctgc 60
   tcctctgggt tccaggtgag aatatttaga aaaagctaaa actaattctt tgaaccatta 120
   attttcttaa ttaggaacct ggcaccatat ggaacttggc ttgtttttaa atgtgatttt 180
   tttttaagta atgcgtattc tttcatcttg tgctactaga ttagtggtga tttcattaag 240
   cagatgctta tattgtgcta atgtttgctg tatggtttca gcctccaggg gtgatgttgt 300
   gatgacacag tctccagctt tcctctctgt gactccaggg gagaaagtca ccatcacctg 360
   ccaggccagt gaaggcattg gcaactactt atactggtac cagcagaaac cagatcaagc 420
   cccaaagctc ctcatcaagt atgcttccca gtccatctca ggggtcccct cgaggttcag 480
   tggcagtgga tctgggacag atttcacctt taccatcagt agcctggaag ctgaagatgc 540
   tgcaacatat tactgtcagc agggcaataa gcaccct 577
<210> 322
   <211> 127
   <212> DNA
   <213> homo sapiens;
<400> 322
   cccagcaggc tcctgctcca gcccagcccc cagagagcag accccaggtg ctggccccgg 60
   gggttttggt ctgagcctca gtcactgtgt tatgtcttcg gaactgggac caaggtcacc 120
   gtcctag 127
<210> 323
   <211> 175
   <212> DNA
   <213> homo sapiens;
<400> 323
   gtgtgggggc catgtggact ccctcatgag cagatgccac cagggccact ggccccagct 60
   tcctccttca cagctgcagt gggggctggg gctggggcat cccagggagg gtttttgtat 120
   gagcctgtgt cacagtgtgt ggtattcggc ggagggacca agctgaccgt cctag 175
<210> 324
   <211> 176
   <212> DNA
   <213> homo sapiens;
<400> 324
   gtgtgggggc catgtggact ccctcatgag cagatgccac caggaccact ggccccagct 60
   tcctccttca cagctgcagt gggggctggg gctaggggca tcccagggag ggtttttgta 120
   tgagcctgtg tcacagtgtt gggtgttcgg cggagggacc aagctgaccg tcctag 176
<210> 325
   <211> 72
   <212> DNA
   <213> homo sapiens;
<400> 325
   cagagagggt ttttgtatga gcctgtgtca cagcactggg tgtttggtga ggggacggag 60
   ctgaccgtcc ta 72
<210> 326
   <211> 70
   <212> DNA
   <213> homo sapiens;
<400> 326
   ggagggtttg tgtgcagggt tatatcacag tgtaatgtgt tcggcagtgg caccaaggtg 60
   accgtcctcg 70
<210> 327
   <211> 46
   <212> DNA
   <213> homo sapiens;
<400> 327
   tcactgtgtg ctgtgttcgg aggaggcacc cagctgaccg ccctcg 46
<210> 328
   <211> 477
   <212> DNA
   <213> homo sapiens;
<400> 328
   gggaatctgc accatgccct gggctctgct cctcctgacc ctcctcactc actctgcagg     60
   tgagagtgga ccttacccag ggatctgcac ccacctctgc tccagcttct ccactccctg    120
   gctcagtgga ctctgatcct gctctcacat tcctttctgt cccctctaca gtgtcagtgg    180
   tccaggcagg gctgactcag ccaccctcgg tgtccaaggg cttgagacag accgccacac    240
   tcacctgcac tgggaacagc aacattgttg gcaaccaagg agcagcttgg ctgcagcagc    300
   accagggcca ccctcccaaa ctcctatcct acaggaataa caaccggccc tcagggatct    360
   cagagagatt ctctgcatcc aggtcaggaa acacagcctc cctgaccatt actggactcc    420
   agcctgagga cgaggctgac tattactgct cagcattgga cagcagcctc agtgctc       477
<210> 329
   <211> 544
   <212> DNA
   <213> homo sapiens;
<400> 329
   gctgtgtcca ctatggccct gactcctctc ctcctcctgc tcctctctca ctgcacaggt     60
   agggacaggg ctcagagccc agggtggtcc ccagcctgat ctgtccctca tggctcagat    120
   ccctcagcag ctgcgccctg accctgctcc tcactgtgct gtgtctgtgt ctgcaggttc    180
   cctctcccgg cccgtgctga ctcagccgcc ctctctgtct gcatccccgg gagcaacagc    240
   cagactcccc tgcaccctga gcagtgacct cagtgttggt ggtaaaaaca tgttctggta    300
   ccagcagaag ccagggagct ctcccaggtt attcctgtat cactactcag actcagacaa    360
   gcagctggga cctggggtcc ccagtcgagt ctctggctcc aaggagacct caagtaacac    420
   agcgtttttg ctcatctctg ggctccagcc tgaggacgag gccgattatt actgccaggt    480
   gtacgaaagt agtgctaatc acagtgagac agatgaggaa gtcggacaaa aaccaaggtt    540
   ttaa                                                                 544
<210> 330
   <211> 507
   <212> DNA
   <213> homo sapiens;
<400> 330
   gctgcgggta gagaagacag gactcaggac aatctccagc atggcctggt cccctctctt      60
   cctcaccctc atcactcact gtgcaggtga caggatgggg accaagagag aggccctggg     120
   aagcccatgc gaccctgctt tctcctcttg tctccttttg tctcttgtca atcaccatgt     180
   ctgtgtctct ctcacttcca gggtcctggg cccagtctgt gctgactcag ccaccctcgg     240
   tgtctgaagc ccccaggcag agggtcacca tctcctgttc tggaagcagc tccaacatcg      300
   gaaataatgc tgtaaactgg taccagcagc tcccaggaaa ggctcccaaa ctcctcatct     360
   attatgatga tctgctgccc tcaggggtct ctgaccgatt ctctggctcc aagtctggca     420
   cctcagcctc cctggccatc agtgggctcc agtctgagga tgaggctgat tattactgtg     480
   cagcatggga tgacagcctg aatggtc                                         507
<210> 331
   <211> 517
   <212> DNA
   <213> homo sapiens;
<400> 331
   gctctgcttc agctgtgggc acaagaggca gcactcagga caatctccag catggcctgg      60
   tctcctctcc tcctcactct cctcgctcac tgcacaggtg actggataca ggtccagggg     120
   aggggccctg ggaagcctat ggattcttgc tttctcctgt tgtctctaga agccgaataa     180
   tgatgcctgt gtctctccca cttccagggt cctgggccca gtctgtgctg acgcagccgc     240
   cctcagtgtc tggggcccca gggcagaggg tcaccatctc ctgcactggg agcagctcca     300
   acatcggggc aggttatgat gtacactggt accagcagct tccaggaaca gcccccaaac     360
   tcctcatcta tggtaacagc aatcggccct caggggtccc tgaccgattc tctggctcca     420
   agtctggcac ctcagcctcc ctggccatca ctgggctcca ggctgaggat gaggctgatt     480
   attactgcca gtcctatgac agcagcctga gtggttc                              517
<210> 332
   <211> 581
   <212> DNA
   <213> homo sapiens;
<400> 332
   ctgatttgca tggatggact ctccccctct cagagtatga agagagggag agatctgggg 60
   gaagctcagc ttcagctgtg ggtagagaag acaggactca ggacaatctc cagcatggcc 120
   agcttccctc tcctcctcac cctcctcact cactgtgcag gtgacaggat ggggaccaag 180
   aaaggggccc tgggaagccc atggggccct gctttctcct cttgtctcct tttgtctctt 240
   gtcaatcacc atgtctgtgt ctctctcact tccagggtcc tgggcccagt ctgtgctgac 300
   tcagccaccc tcagcgtctg ggacccccgg gcagagggtc accatctctt gttctggaag 360
   cagctccaac atcggaagta atactgtaaa ctggtaccag cagctcccag gaacggcccc 420
   caaactcctc atctatagta ataatcagcg gccctcaggg gtccctgacc gattctctgg 480
   ctccaagtct ggcacctcag cctccctggc catcagtggg ctccagtctg aggatgaggc 540
   tgattattac tgtgcagcat gggatgacag cctgaatggt c 581
<210> 333
   <211> 522
   <212> DNA
   <213> homo sapiens;
<400> 333
   ggggaagctc agcttcagct gtggtagaga agacaggatt caggacaatc tccagcatgg 60
   ccggcttccc tctcctcctc accctcctca ctcactgtgc aggtgacagg atggggacca 120
   agagaggggc cctgggaagc ccatggggcc ctgctttctc ctcttgtctc ctttcgtctc 180
   ttgtcaatca ccatgtctgt gtctctctca cttccagggt cctgggccca gtctgtgctg 240
   actcagccac cctcagcgtc tgggaccccc gggcagaggg tcaccatctc ttgttctgga 300
   agcagctcca acatcggaag taattatgta tactggtacc agcagctccc aggaacggcc 360
   cccaaactcc tcatctatag taataatcag cggccctcag gggtccctga ccgattctct 420
   ggctccaagt ctggcacctc agcctccctg gccatcagtg ggctccggtc cgaggatgag 480
   gctgattatt actgtgcagc atgggatgac agcctgagtg gt 522
<210> 334
   <211> 515
   <212> DNA
   <213> homo sapiens;
<400> 334
   gctctgcttc agctgtgggc acaggaggca gcactcagga caatctccag catggcctgg 60
   tcttctctcc tcctcactct cctcgctcac tgcacaggtg actggatgca gatcgagggg 120
   agggtccctg ggaagcctat ggattcttgc tttctcctct tgtctctaga agcagaatca      180
   tgatgcctgt gtctctccca cttccagggt cctgggccca gtctgtgctg acgcagccgc      240
   cctcagtgtc tggggcccca gggcagaggg tcaccatctc ctgcactggg agcagctcca      300
   acattggggc gggttatgtt gtacattggt accagcagct tccaggaaca gcccccaaac      360
   tcctcatcta tggtaacagc aatcggccct caggggtccc tgaccaattc tctggctcca      420
   agtctggcac ctcagcctcc ctggccatca ctggactcca gtctgaggat gaggctgatt      480
   attactgcaa agcatgggat aacagcctga atgct                                  515
<210> 335
   <211> 509
   <212> DNA
   <213> homo sapiens;
<400> 335
   tgagcgcaga aggcaggact cgggacaatc ttcatcatga cctgctcccc tctcctcctc       60
   acccttctca ttcactgcac aggtgcccag acacagggtc aggggagggg tccaggaagc      120
   ccatgaggcc ctgctttctc cttctctctc tagaccaaga atcaccgtgt ctgtgtctct      180
   cctgcttcca gggtcctggg cccagtctgt gttgacgcag ccgccctcag tgtctgcggc      240
   cccaggacag aaggtcacca tctcctgctc tggaagcagc tccaacattg ggaataatta      300
   tgtatcctgg taccagcagc tcccaggaac agcccccaaa ctcctcattt atgacaataa      360
   taagcgaccc tcagggattc ctgaccgatt ctctggctcc aagtctggca cgtcagccac      420
   cctgggcatc accggactcc agactgggga cgaggccgat tattactgcg gaacatggga      480
   tagcagcctg agtgctggca cagtgctcc                                        509
<210> 336
   <211> 517
   <212> DNA
   <213> homo sapiens;
<400> 336
   tgctggggtc tcaggaggca gcactctcgg gacgtctcca ccatggcctg ggctctgctc 60
   ctcctcagcc tcctcactca gggcacaggt gacacctcca gggaaagggt cacaggggtc 120
   tctgggctga tccttggtct cctgctcctc aggctcacct gggcccagca ctgactcact 180
   agagtgtgtt tctccctctt tccaggatcc tgggctcagt ctgccctgac tcagcctcgc 240
   tcagtgtccg ggtctcctgg acagtcagtc accatctcct gcactggaac cagcagtgat 300
   gttggtggtt ataactatgt ctcctggtac caacagcacc caggcaaagc ccccaaactc 360
   atgatttatg atgtcagtaa gcggccctca ggggtccctg atcgcttctc tggctccaag 420
   tctggcaaca cggcctccct gaccatctct gggctccagg ctgaggatga ggctgattat 480
   tactgctgct catatgcagg cagctacact ttccaca 517
<210> 337
   <211> 519
   <212> DNA
   <213> homo sapiens;
<400> 337
   gctggggtct caggaggcag cgctctcagg acatctccac catggcctgg gctctgctgc 60
   tcctcaccct cctcactcag ggcacaggtg acgcctccag ggaaggggct tcagggacct 120
   ctgggctgat ccttggtctc ctgctcctca ggctcaccgg ggcccagcac tgactcactg 180
   gcatgtgttt ctccctcttt ccagggtcct gggcccagtc tgccctgact cagcctgcct 240
   ccgtgtctgg gtctcctgga cagtcgatca ccatctcctg cactggaacc agcagtgacg 300
   ttggtggtta taactatgtc tcctggtacc aacagcaccc aggcaaagcc cccaaactca 360
   tgatttatga ggtcagtaat cggccctcag gggtttctaa tcgcttctct ggctccaagt 420
   ctggcaacac ggcctccctg accatctctg ggctccaggc tgaggacgag gctgattatt 480
   actgcagctc atatacaagc agcagcactc tccacagtg 519
<210> 338
   <211> 490
   <212> DNA
   <213> homo sapiens;
<400> 338
   gaatatctcc accatggcct gggctctgct cctcctcacc ctcctcactc agggcacagg 60
   tgaggcctcc agggaagggg cttcggggac ctctgggctg atccttaact cctgctcctc 120
   aggctcacct gggcccagca ctgacttact aaaatgtgtt tcttcctttt tccaggatcc 180
   tgggctcagt ctgccctgac tcagcctccc tccgtgtccg ggtctcctgg acagtcagtc 240
   accatctcct gcactggaac cagcagtgac gttggtagtt ataaccgtgt ctcctggtac 300
   cagcagcccc caggcacagc ccccaaactc atgatttatg aggtcagtaa tcggccctca 360
   ggggtccctg atcgcttctc tgggtccaag tctggcaaca cggcctccct gaccatctct 420
   gggctccagg ctgaggacga ggctgattat tactgcagct tatatacaag cagcagcact 480
   ttccacagag 490
<210> 339
   <211> 619
   <212> DNA
   <213> homo sapiens;
<400> 339
   tctctgagcc caggcccacg tgagggtggg gtgaggagag gagcccagga tgctgatttt 60
   catggaggcc ccgccctcct ctgaggcaaa ggggataaga cagggctggg gcagggccag 120
   tgctggggtc acaagaggca gcgctctcgg gacgtctcca ccatggcctg ggctctgctg 180
   ctcctcactc tcctcactca ggacacaggt gacgcctcca gggaaggggt cttggggacc 240
   tctgggctga tccttggtct cctgctcctc aggctcaccg gggcccagca ctgactcact 300
   ggcatgtgtt tctccctctt tccagggtcc tgggcccagt ctgccctgac tcagcctgcc 360
   tccgtgtctg ggtctcctgg acagtcgatc accatctcct gcactggaac cagcagtgat 420
   gttgggagtt ataaccttgt ctcctggtac caacagcacc caggcaaagc ccccaaactc 480
   atgatttatg agggcagtaa gcggccctca ggggtttcta atcgcttctc tggctccaag 540
   tctggcaaca cggcctccct gacaatctct gggctccagg ctgaggacga ggctgattat 600
   tactgctgct catatgcag 619
<210> 340
   <211> 520
   <212> DNA
   <213> homo sapiens;
<400> 340
   ggctagaggc aggcccggtg ctggggtctc aaggcagcgc tctcgggaca tctccaccat 60
   ggcctgggct ctgctcctcc tcaccctcct cactcagggc acaggtgaca cctccaggga 120
   aatggccttg gggacctctg agctaatgct tggtcttctg ctcctgctcc tcagggtcac 180
   tggacccagt actgacccag tagagtgtgt ttctccctct ttccagggtc ctgggcccaa 240
   tctgccctga ctcagcctcc ttttgtgtcc ggggctcctg gacagtcggt caccatctcc 300
   tgcactggaa ccagcagtga cgttggggat tatgatcatg tcttctggta ccaaaagcgt 360
   ctcagcacta cctccagact cctgatttac aatgtcaata ctcggccttc agggatctct 420
   gacctcttct caggctccaa gtctggcaac atggcttccc tgaccatctc tgggctcaag 480
   tccgaggttg aggctaatta tcactgcagc ttatattcaa 520
<210> 341
   <211> 635
   <212> DNA
   <213> homo sapiens;
<400> 341
   tctctaagcc caggcccaag tgagggtggg gtgagaagag gagctcagga tgcagatttg 60
   catggaggtc ccgcccttct ctgaggcaga gggataagac agggctgggg gcaggcccag 120
   tgctggggtc tcaggaggca gcgctctcag gacgtcacca ccatggcctg ggctctgctc 180
   ctcctcaccc tcctcactca gggcacaggt gatgcctcca gggaaggggc cacagggacc 240
   tctgggctga tccttggtct cctgctcctc aggctcacct gggcccagca ctgactcact 300
   agactgtgtt tctccctttc cagggtcctg ggcccagtct gccctgactc agcctccctc 360
   cgcgtccggg tctcctggac agtcagtcac catctcctgc actggaacca gcagtgacgt 420
   tggtggttat aactatgtct cctggtacca acagcaccca ggcaaagccc ccaaactcat 480
   gatttatgag gtcagtaagc ggccctcagg ggtccctgat cgcttctctg gctccaagtc 540
   tggcaacacg gcctccctga ccgtctctgg gctccaggct gaggatgagg ctgattatta 600
   ctgcagctca tatgcaggca gcaacaattt ccaca 635
<210> 342
   <211> 691
   <212> DNA
   <213> homo sapiens;
<400> 342
   aagaacctgc ccagcctggg cctcaggaag cagcatcgga ggtgcctcag ccatggcatg 60
   gatccctctc ttcctcggcg tccttgctta ctgcacaggt gctgccccta gggtcctagc 120
   cactggtcca gtcccagggc tctgggtcca gcctggccct gactctgagc tcagcagggc 180
   ccccgcctgt ggtgggcagg atgctcatga ccctgctgca ggtggatggg ctcggcgggg 240
   ctgaaatccc cccacacagt gctcatgtgc tcacactgcc ttagggctct ttcatccctg 300
   gatctgtgtc caggccaggc acgtgggaag atttacttgg agttcagctc ctcagtttca 360
   agccttttct ctcccgtttt ctctcctgta ggatccgtgg cctcctatga gctgactcag 420
   ccaccctcag tgtccgtgtc cccaggacag acagccagca tcacctgctc tggagataaa 480
   ttgggggata aatatgcttg ctggtatcag cagaagccag gccagtcccc tgtgctggtc 540
   atctatcaag atagcaagcg gccctcaggg atccctgagc gattctctgg ctccaactct 600
   gggaacacag ccactctgac catcagcggg acccaggcta tggatgaggc tgactattac 660
   tgtcaggcgt gggacagcag cactgcacac a 691
<210> 343
   <211> 539
   <212> DNA
   <213> homo sapiens;
<400> 343
   gtgggctcag gaggcagagc tctgggaatc tcaccatggc ctggacccct ctcctgctcc 60
   ccctcctcac tttctgcaca ggtgcttctc ccaggccctg ccccaggctc agtgcccata 120
   gaccccaagt tggccctgcc ctgaaccctg tgcaaagccc agacacagtc ttagggtagg 180
   acccctggga atgggctctt gatcttcaag ccccctctcc tgttttcctt gcagtctctg 240
   aggcctccta tgagctgaca cagccaccct cggtgtcagt gtccccagga caaacggcca 300
   ggatcacctg ctctggagat gcattgccaa aaaaatatgc ttattggtac cagcagaagt 360
   caggccaggc ccctgtgctg gtcatctatg aggacagcaa acgaccctcc gggatccctg 420
   agagattctc tggctccagc tcagggacaa tggccacctt gactatcagt ggggcccagg 480
   tggaggatga agctgactac tactgttact caacagacag cagtggtaat catagcaca 539
<210> 344
   <211> 763
   <212> DNA
   <213> homo sapiens;
<400> 344
   gcctcagcca tggcctggac ccctctcctc ctcagcctcc tcgctcactg cacaggtgct 60
   ctgcccaggg tatcaccaac ctgcccatcc ccagggctct gggtccagtg tggccatgac 120
   tatgagctca ggagggccct gcctgtggtg ggcaggatgc tcatgaccct gctgcagggt 180
   gagggactgg cggagctgaa gtcccctcaa actctgctca gaggcttgtg agagcctgag 240
   gggctgcacc tgccaggaga gagtactggg ttttcagttc aaaggctcca tgcagaggga 300
   aagtccatgg gccactgggg ctagggctga ttgcagggga taccctgagg gttcacagac 360
   tctctgaagc ttttccagga cagcagggca ggggatttca tacggatctt ttacctaaaa 420
   gccatcctct cctttttttt tttttttaat ctttgcaggc tctgcgacct cctatgagct 480
   gactcagcca cactcagtgt cagtggccac agcacagatg gccaggatca cctgtggggg 540
   aaacaacatt ggaagtaaag ctgtgcactg gtaccagcaa aagccaggcc aggaccctgt 600
   gctggtcatc tatagcgata gcaaccggcc ctcagggatc cctgagcgat tctctggctc 660
   caacccaggg aacaccgcca ccctaaccat cagcaggatc gaggctgggg atgaggctga 720
   ctattactgt caggtgtggg acagtagtag tgatcatccc acg 763
<210> 345
   <211> 529
   <212> DNA
   <213> homo sapiens;
<400> 345
   tctgtgggtc caggaggcac agctctggga atctcaccat ggcctggatc cctctcctgc 60
   tccccctcct cactctctgc acaggtgctg accccaggcc cttccccagg ctcagtcccc 120
   acagattcca agttgagcct gacctgaatc ctgagcaaag cccagacaca gcctctgggt 180
   gggactcctg gaaatgggtc ctttgtcttc aagccccctc tcttgttctt ccttgcaggc 240
   tctgaggcct cctatgagct gacacagcca ccctcggtgt cagtgtccct aggacagatg 300
   gccaggatca cctgctctgg agaagcattg ccaaaaaaat atgcttattg gtaccagcag 360
   aagccaggcc agttccctgt gctggtgata tataaagaca gcgagaggcc ctcagggatc 420
   cctgagcgat tctctggctc cagctcaggg acaatagtca cattgaccat cagtggagtc 480
   caggcagaag acgaggctga ctattactgt ctatcagcag acagcagtg 529
<210> 346
   <211> 523
   <212> DNA
   <213> homo sapiens;
<400> 346
   agctgtgggc tcagaagcag agttctgggg tgtctccacc atggcctgga cccctctctg 60
   gctcactctc ctcactcttt gcataggtgc tgcctcccag ggctcaaccc catattatca 120
   tgctagctgt gccaacctgg ccctgagctt cggctcaaca cagggagtag tgtagggtgt 180
   gggactctag gcgtgaaacc cttatcctca cctcttctgt cctcttttgc aggttctgtg 240
   gtttcttctg agctgactca ggaccctgct gtgtctgtgg ccttgggaca gacagtcagg 300
   atcacatgcc aaggagacag cctcagaagc tattatgcaa gctggtacca gcagaagcca 360
   ggacaggccc ctgtacttgt catctatggt aaaaacaacc ggccctcagg gatcccagac 420
   cgattctctg gctccagctc aggaaacaca gcttccttga ccatcactgg ggctcaggcg 480
   gaagatgagg ctgactatta ctgtaactcc cgggacagca gtg 523
<210> 347
   <211> 1515
   <212> DNA
   <213> homo sapiens;
<400> 347
   gcacagagga gctgtgccct ggaatggggc ctgtacctgt ccaaggcttg tgccgtcccc 60
   tgtgggagat gagaagcgtc cctgcattgg gctcttgggg acccgtcttg gacatgagtg 120
   agaatgaaga gggtccctgc attgggctct ggcatgtgac tttaaatgga tttaggcctg 180
   taccagacat ctcatgtctg acataaaata tttacaatca ggacattact agagaagcag 240
   aaaaaagcta accacctccc tcctgagcca ggatggaatg aaggagggga ctgtggaccc 300
   cagataattc ccctgtcacc actgtgactc taacaacctc ttaaatcacg gccaacatct 360
   atcccatagg aaggtcttta tatcccctag aaaatacaga ggaagtcagc tctgagcttt 420
   tccacgacca acccagccaa ggagcaaggc tgggcacaac ctgggtaaag atgtgagccc 480
   agaccatggg accagtgggt gaaggaaaat cgcatgggct gagggggtgg gtaagcaggg 540
   gccagccctc ctctctctgt ttcctttggg gctgagtcct tctctggaaa ccacagatct 600
   cctccagcag cagcctctga ctctgctgat ttgcatcatg ggccgctctc tccagcaagg 660
   ggataagaga ggcctgggag gaacctgctc agtctgggcc taaggaagca gcactggtgg 720
   tgcctcagcc atggcctgga ccgttctcct cctcggcctc ctctctcact gcacaggtga 780
   tccccccagg gtctcaccaa cctgcccagc ccaagggttc tgggtccagc gtgtccttga 840
   ttctgagctc aggagggccc ttcctgtggt gggcaggatg ctcatgaccc tgctgcaggg 900
   tgggaggctg gtggggctga actcccccca aactgtgctc aaaggcttgt gagagcctga 960
   gggactgcac ctgccaggag agagtagtga gttttcagtt caaagtctcc atacaacagg 1020
   aaagtcatgg gccactgggg ctggggctga ttgcagggga taccctgagg gttcacagac 1080
   tctctggagc ttgtctggga cagcagggca agggatttca taagaagcat ctttcacctg 1140
   caagccaacc tctctcttat ttatttattt atttatttat ttatttattt atttattttt 1200
   atctttgcag gctctgtgac ctcctatgtg ctgactcagc caccctcggt gtcagtggcc 1260
   ccaggacaga cggccaggat tacctgtggg ggaaacaaca ttggaagtaa aagtgtgcac 1320
   tggtaccagc agaagccagg ccaggcccct gtgctggtcg tctatgatga tagcgaccgg 1380
   ccctcaggga tccctgagcg attctctggc tccaactctg ggaacacggc caccctgacc 1440
   atcagcaggg tcgaagccgg ggatgaggcc gactattact gtcaggtgtg ggatagtagt 1500
   agtgatcatc ccacg 1515
<210> 348
   <211> 558
   <212> DNA
   <213> homo sapiens;
<400> 348
   aagagaggcc tgggaagccc agctgtgctg tgggctcagg aggcagagct gtgggtgtct 60
   caccatggca tgggccacac tcctgctccc actcctcaac ctctacacag gtgctgcccc 120
   cagaccctgc cccaggctca gccctcctaa gcccctggtc ttaccctgaa ccctgagctc 180
   agcccaggca tagcctcagg gcgatactac tggaatgggt ttgttatctt caagccccct 240
   ctcttgtcct ctcttgcagg ctctgttgcc tcctatgagc tgacacagct accctcggtg 300
   tcagtgtccc caggacagac agccaggatc acctgctctg gagatgtact gggggaaaat 360
   tatgctgact ggtaccagca gaagccaggc caggcccctg agttggtgat atacgaagat 420
   agtgagcggt accctggaat ccctgaacga ttctctgggt ccacctcagg gaacacgacc 480
   accctgacca tcagcagggt cctgaccgaa gacgaggctg actattactg tttgtctggg 540
   gatgaggaca atccctca 558
<210> 349
   <211> 546
   <212> DNA
   <213> homo sapiens;
<400> 349
   gctgtgctgt gggtccagga ggcagaactc tgggtgtctc accatggcct ggatccctct 60
   acttctcccc ctcttcactc tctgcacagg tgctgtcccc aggccctgct ccaggccctg 120
   ctccagtctt attccccaca gatcccaagt tgagcctgcc ctgaatcccg agcaaagccc 180
   agacgcagcc tctgggtgcg actcctggga atgggtcctt tgtcttcaag ccccctctct 240
   tgttcttcct tgcaggctct gaggcctcct atgagctgac acagccaccc tcggtgtcag 300
   tgtccccagg acagacggcc aggatcacct gctctggaga tgcattgcca aagcaatatg 360
   cttattggta ccagcagaag ccaggccagg cccctgtgct ggtgatatat aaagacagtg 420
   agaggccctc agggatccct gagcgattct ctggctccag ctcagggaca acagtcacgt 480
   tgaccatcag tggagtccag gcagaagatg aggctgacta ttactgtcaa tcagcagaca 540
   gcagtg 546
<210> 350
   <211> 519
   <212> DNA
   <213> homo sapiens;
<400> 350
   gctgtaggct caggaggcag agctctgaat gtctcaccat ggcctggatc cctctcctgc 60
   tccccctcct cattctctgc acaggtgctg cccctaggct cagtctccac agaccccaag 120
   ttgagcctga cctgaatcct gagcaaagcc ctgccactgc ctctgggggg gattcctggc 180
   aatgcgtcct ttgtcctcaa gccccctctc ctgtcttttc ttgcagtctc tgtggcctcc 240
   tatgagctga cacagccatc ctcagtgtca gtgtctccgg gacagacagc caggatcacc 300
   tgctcaggag atgtactggc aaaaaaatat gctcggtggt tccagcagaa gccaggccag 360
   gcccctgtgc tggtgattta taaagacagt gagcggccct cagggatccc tgagcgattc 420
   tccggctcca gctcagggac cacagtcacc ttgaccatca gcggggccca ggttgaggat 480
   gaggctgact attactgtta ctctgcggct gacaacaat 519
<210> 351
   <211> 504
   <212> DNA
   <213> homo sapiens;
<400> 351
   gctgtggact cagaggcaga gctctggggc atttccatta tggcctggac ccctcccctg 60
   ctcgtcctca ctctctgcac aggtgctgcc tcccagggct cagcccccag tgggatcaag 120
   atcagcctgg ccctgacctt caactcaaca tagggagtga tgcagggtgt ggggttctgg 180
   gaatgaggcc ctcatcctca gactcacctc tcctgtcctc tcttgtgggc tccgttattt 240
   cctctgggcc aactcaggtg cctgcagtgt ctgtggcctt gggacaaatg gccaggatca 300
   cctgccaggg agacagcatg gaaggctctt atgaacactg gtaccagcag aagccaggcc 360
   aggcccccgt gctggtcatc tatgatagca gtgaccggcc ctcaaggatc cctgagcgat 420
   tctctggctc caaatcaggc aacacaacca ccctgaccat cactggggcc caggctgagg 480
   atgaggctga ttattactat cagt 504
<210> 352
   <211> 747
   <212> DNA
   <213> homo sapiens;
<400> 352
   cttgactctg ctgatttgca tcacaggctg ctctcttcag caaggggata agagagggct 60
   ggaaggaacc tgcccagcct gggcctcagg aagcagcatc gggggtgccg cagccatggc 120
   ctggaccgct ctccttctga gcctccttgc tcactttaca ggtgctgccc ccagtgtccc 180
   agccacctac ccagctccaa ggctctgggt ccagcctggc ctgacagtga tctcagcagg 240
   gccctgcctg tggtgtgcag gatgctcatg atcctgctgc agggggaggg gctgctggag 300
   gtgaaatccc cccacactgt tcttctgtgc tcatggtccc ctgaggacac ttctattcct 360
   gaaactcagg ccaggcaggt gggaaggcat tgttgggttg agcctctcag tttcaagtct 420
   attctattct ctcccctttt cttgcaggtt ctgtggcctc ctatgagctg actcagccac 480
   tctcagtgtc agtggccctg ggacagacgg ccaggattac ctgtggggga aacaacattg 540
   gaagtaaaaa tgtgcactgg taccagcaga agccaggcca ggcccctgtg ctggtcatct 600
   atagggatag caaccggccc tctgggatcc ctgagcgatt ctctggctcc aactcgggga 660
   acacggccac cctgaccatc agcagagccc aagccgggga tgaggctgac tattactgtc 720
   aggtgtggga cagcagcact gcacaca 747
<210> 353
   <211> 529
   <212> DNA
   <213> homo sapiens;
<400> 353
   ctcgaataga gctcttggaa gtccctccaa ccatggcctg ggtctccttc tacctactgc 60
   ccttcatttt ctccacaggt cagaacatcc cagggaattc agggaaatgt tttcactgct 120
   attttcccat gagcaccagt cctcaggggc attctttcca gttcttctgt gcattcagca 180
   tcattcatga cattctgttt acaggtctct gtgctctgcc tgtgctgact cagcccccgt 240
   ctgcatctgc cttgctggga gcctcgatca agctcacctg caccctaagc agtgagcaca 300
   gcacctacac catcgaatgg tatcaacaga gaccagggag gtccccccag tatataatga 360
   aggttaagag tgatggcagc cacagcaagg gggacgggat ccccgatcgc ttcatgggct 420
   ccagttctgg ggctgaccgc tacctcacct tctccaacct ccagtctgac gatgaggctg 480
   agtatcactg tggagagagc cacacgattg atggccaagt cggttgagc 529
<210> 354
   <211> 483
   <212> DNA
   <213> homo sapiens;
<400> 354
   atggcctgga ccccactcct cctcctcttc cctctcctcc tccactgcac aggtcaggag 60
   gaccctcagc atcctcatgc cccagctcac tgacaccatc tcccaaactc ataccagaaa 120
   tgttgtttgc tcttgtcctt ccttcaggcc ataatgagcg tctctgtttt cagggtctct 180
   ctcccagcct gtgctgactc aatcatcctc tgcctctgct tccctgggat cctcggtcaa 240
   gctcacctgc actctgagca gtgggcacag tagctacatc atcgcatggc atcagcagca 300
   gccagggaag gcccctcggt acttgatgaa gcttgaaggt agtggaagct acaacaaggg 360
   gagcggagtt cctgatcgct tctcaggctc cagctctggg gctgaccgct acctcaccat 420
   ctccaacctc cagtttgagg atgaggctga ttattactgt gagacctggg acagtaacac 480
   tca 483
<210> 355
   <211> 539
   <212> DNA
   <213> homo sapiens;
<400> 355
   agggtgggta agaaatacct gcaactgtca gcctcagcag agctctgggg agtctgcacc 60
   atggcttgga ccccactcct cttcctcacc ctcctcctcc actgcacagg tcaggatggc 120
   cctcagcacc ctgacctcca gctcactgat accacctccc aaacttatgc caggaatgtc 180
   cttccctctt ttcttgactc cagccggtaa tgggtgtctg tgttttcagg gtctctctcc 240
   cagcttgtgc tgactcaatc gccctctgcc tctgcctccc tgggagcctc ggtcaagctc 300
   acctgcactc tgagcagtgg gcacagcagc tacgccatcg catggcatca gcagcagcca 360
   gagaagggcc ctcggtactt gatgaagctt aacagtgatg gcagccacag caagggggac 420
   gggatccctg atcgcttctc aggctccagc tctggggctg agcgctacct caccatctcc 480
   agcctccagt ctgaggatga ggctgactat tactgtcaga cctggggcac tggcattca 539
<210> 356
   <211> 496
   <212> DNA
   <213> homo sapiens;
<400> 356
   ccaccatggc ctggactcct cttcttctct tgctcctctc tcactgcaca ggtagggaca 60
   ggcctcagag atcagggcca gccacccaac ctgattctgg ctcttctggt aaagatccct 120
   gaaaaacctc accctgaacc ctgcccatca accatgagtg tctgtgtttg caggttccct 180
   ctcccagcct gtgctgactc agccaccttc ctcctccgca tctcctggag aatccgccag 240
   actcacctgc accttgccca gtgacatcaa tgttggtagc tacaacatat actggtacca 300
   gcagaagcca gggagccctc ccaggtatct cctgtactac tactcagact cagataaggg 360
   ccagggctct ggagtcccca gccgcttctc tggatccaaa gatgcttcag ccaatacagg 420
   gattttactc atctccgggc tccagtctga ggatgaggct gactattact gtatgatttg 480
   gccaagcaat gcttct 496
<210> 357
   <211> 520
   <212> DNA
   <213> homo sapiens;
<400> 357
   actgcggggg taagaggttg tgtccaccat ggcctggact cctctcctcc tcctgttcct        60
   ctctcactgc acaggtagga atagacttca gagaccaggg tcagccaccc agcctgattc       120
   tgactcttct ggcaaagatc cctgaaaaac tttaccctgg tttctgcctt agcacccatt       180
   aatgtctgtg tttccaggtt ccctctcgca ggctgtgctg actcagccgt cttccctctc       240
   tgcatctcct ggagcatcag ccagtctcac ctgcaccttg cgcagtggca tcaatgttgg       300
   tacctacagg atatactggt accagcagaa gccagggagt cctccccagt atctcctgag       360
   gtacaaatca gactcagata agcagcaggg ctctggagtc cccagccgct tctctggatc       420
   caaagatgct tcggccaatg cagggatttt actcatctct gggctccagt ctgaggatga       480
   ggctgactat tactgtatga tttggcacag cagcgcttct                             520
<210> 358
   <211> 493
   <212> DNA
   <213> homo sapiens;
<400> 358
   atggcctgga atcctctcct cctcctgttc ctctctcact gcacaggtag gaaaaggcct        60
   cagagaccag ggtcagccac acagcctgat tctgactctt gtgtcaaaga tcactaaaaa       120
   aaatattacc ttggtttctg tcttaaagcc tatatatgcc tgtgttccag gttccctctc       180
   gcagcctgtg ctgactcagc caacttccct ctcagcatct cctggagcat cagccagact       240
   cacctgcacc ttgcgcagtg gcatcaatct tggtagctac aggatattct ggtaccagca       300
   gaagccagag agccctcccc ggtatctcct gagctactac tcagactcaa gtaagcatca       360
   gggctctgga gtccccagcc gcttctctgg atccaaagat gcttcgagca atgcagggat       420
   tttagtcatc tctgggctcc agtctgagga tgaggctgac tattactgta tgatttggca       480
   cagcagtgct tct                                                          493
<210> 359
   <211> 500
   <212> DNA
   <213> homo sapiens;
<400> 359
   ccaccatggc ctggactctt ctccttctcg tgctcctctc tcactgcaca ggtagggaaa        60
   gtccttataa actgagtctc agtgtccaac ctacaccatc ccctgtggct cagacctaca       120
   agaagcttta ccctgggaac tgccttatca cccatgatgt ctgtgttttc aggttccctc 180
   tcccagcctg tgctgactca gccatcttcc cattctgcat cttctggagc atcagtcaga 240
   ctcacctgca tgctgagcag tggcttcagt gttggggact tctggataag gtggtaccaa 300
   caaaagccag ggaaccctcc ccggtatctc ctgtactacc actcagactc caataagggc 360
   caaggctctg gagttcccag ccgcttctct ggatccaacg atgcatcagc caatgcaggg 420
   attctgcgta tctctgggct ccagcctgag gatgaggctg actattactg tggtacatgg 480
   cacagcaact ctaagactca 500
<210> 360
   <211> 574
   <212> DNA
   <213> homo sapiens;
<400> 360
   tctgaggata cgcgtgacag ataagaaggg ctggtgggat cagtcctggt ggtagctcag 60
   gaagcagagc ctggagcatc tccactatgg cctgggctcc actacttctc accctcctcg 120
   ctcactgcac aggtggctgc ctgcaaggaa ttcagggagc gttcctggat gtcacctggg 180
   ctgatgatct gttcctcctg cctgggaacc agtcttcatc tctcccgact gatctctgtg 240
   ttgctctctt cttgcaggtt cttgggccaa ttttatgctg actcagcccc actctgtgtc 300
   ggagtctccg gggaagacgg taaccatctc ctgcacccgc agcagtggca gcattgccag 360
   caactatgtg cagtggtacc agcagcgccc gggcagttcc cccaccactg tgatctatga 420
   ggataaccaa agaccctctg gggtccctga tcggttctct ggctccatcg acagctcctc 480
   caactctgcc tccctcacca tctctggact gaagactgag gacgaggctg actactactg 540
   tcagtcttat gatagcagca atcacacagt gctc 574
<210> 361
   <211> 472
   <212> DNA
   <213> homo sapiens;
<400> 361
   tctggcgcca ggggtccctt ccaatatcag caccatggcc tggactcctc tctttctgtt 60
   cctcctcact tgctgcccag gttaagagag atttcaaata ccagcctttg gagggatcct 120
   tctgtctgcc cttctaattt ctaacatgtg tctgtttttt gtttcagggt ccaattctca 180
   gactgtggtg actcaggagc cctcactgac tgtgtcccca ggagggacag tcactctcac 240
   ctgtgcttcc agcactggag cagtcaccag tggttactat ccaaactggt tccagcagaa 300
   acctggacaa gcacccaggg cactgattta tagtacaagc aacaaacact cctggacccc 360
   tgcccggttc tcaggctccc tccttggggg caaagctgcc ctgacactgt caggtgtgca 420
   gcctgaggac gaggctgagt attactgcct gctctactat ggtggtgctc ag 472
<210> 362
   <211> 473
   <212> DNA
   <213> homo sapiens;
<400> 362
   tctggcacca ggggtccctt ccaatatcag caccatggcc tggactcctc tctttctgtt 60
   cctcctcact tgctgcccag gttaagagag atttcaaata ccagcctttg gagggatccc 120
   tttttctccc tttctaattc ctaatatatg tctgtttttt ttgtttcagg gtccaattcc 180
   caggctgtgg tgactcagga gccctcactg actgtgtccc caggagggac agtcactctc 240
   acctgtggct ccagcactgg agctgtcacc agtggtcatt atccctactg gttccagcag 300
   aagcctggcc aagcccccag gacactgatt tatgatacaa gcaacaaaca ctcctggaca 360
   cctgcccggt tctcaggctc cctccttggg ggcaaagctg ccctgaccct tttgggtgcg 420
   cagcctgagg atgaggctga gtattactgc ttgctctcct atagtggtgc tcg 473
<210> 363
   <211> 513
   <212> DNA
   <213> homo sapiens;
<400> 363
   gaggaaaaca aaccccagct gggaagcctg agaacactta gccttcatga gtgtccccac 60
   catggcctgg atgatgcttc tcctcggact ccttgcttat ggatcaggtc aggggaaggg 120
   actctatccc tgggggacca cagaaaacag ggtccaggtt actctcatcc tcatgatcat 180
   aactgtgtct ctcctgttcg ttttaggagt ggattctcag actgtggtga cccaggagcc 240
   atcgttctca gtgtcccctg gagggacagt cacactcact tgtggcttga gctctggctc 300
   agtctctact agttactacc ccagctggta ccagcagacc ccaggccagg ctccacgcac 360
   gctcatctac agcacaaaca ctcgctcttc tggggtccct gatcgcttct ctggctccat 420
   ccttgggaac aaagctgccc tcaccatcac gggggcccag gcagatgatg aatctgatta 480
   ttactgtgtg ctgtatatgg gtagtggcat ttc 513
<210> 364
   <211> 546
   <212> DNA
   <213> homo sapiens;
<400> 364
   gagagactga agaacccagc attgcagcag ctccaccatg gcctgggctc ctctgctcct 60
   caccctcctc agtctcctca caggtcaggg tgggcagtgg gctgggcccc caaagggacc 120
   cccacctccc agcctccatc tccccatccc tgctcttcct cctccaacag ctcatcagcc 180
   acccaccaac aggagccctc atgggtgtct gtgtttccag ggtccctctc ccagcctgtg 240
   ctgactcagc caccttctgc atcagcctcc ctgggagcct cggtcacact cacctgcacc 300
   ctgagcagcg gctacagtaa ttataaagtg gactggtacc agcagagacc agggaagggc 360
   ccccggtttg tgatgcgagt gggcactggt gggattgtgg gatccaaggg ggatggcatc 420
   cctgatcgct tctcagtctt gggctcaggc ctgaatcggt acctgaccat caagaacatc 480
   caggaagaag atgagagtga ctaccactgt ggggcagacc atggcagtgg gagcaacttc 540
   gtgtaa 546
<210> 365
   <211> 6729
   <212> DNA
   <213> homo sapiens;
<400> 365
   gcctccacca agggcccatc ggtcttcccc ctggcaccct cctccaagag cacctctggg 60
   ggcacagcag ccctgggctg cctggtcaag gactacttcc ccgaaccggt gacggtgtcg 120
   tggaactcag gcgccctgac cagcggcgtg cacaccttcc cggctgtcct acagtcctca 180
   ggactctact ccctcagcag cgtggtgacc gtgccctcca gcagcttggg cacccagacc 240
   tacatctgca acgtgaatca caagcccagc aacaccaagg tggacaagaa agttggtgag 300
   aggccagcac agggagggag ggtgtctgct ggaagccagg ctcagcgctc ctgcctggac 360
   gcatcccggc tatgcagccc cagtccaggg cagcaaggca ggccccgtct gcctcttcac 420
   ccggaggcct ctgcccgccc cactcatgct cagggagagg gtcttctggc tttttcccca 480
   ggctctgggc aggcacaggc taggtgcccc taacccaggc cctgcacaca aaggggcagg 540
   tgctgggctc agacctgcca agagccatat ccgggaggac cctgcccctg acctaagccc 600
   accccaaagg ccaaactctc cactccctca gctcggacac cttctctcct cccagattcc 660
   agtaactccc aatcttctct ctgcagagcc caaatcttgt gacaaaactc acacatgccc 720
   accgtgccca ggtaagccag cccaggcctc gccctccagc tcaaggcggg acaggtgccc 780
   tagagtagcc tgcatccagg gacaggcccc agccgggtgc tgacacgtcc acctccatct 840
   cttcctcagc acctgaactc ctggggggac cgtcagtctt cctcttcccc ccaaaaccca 900
   aggacaccct catgatctcc cggacccctg aggtcacatg cgtggtggtg gacgtgagcc 960
   acgaagaccc tgaggtcaag ttcaactggt acgtggacgg cgtggaggtg cataatgcca 1020
   agacaaagcc gcgggaggag cagtacaaca gcacgtaccg tgtggtcagc gtcctcaccg 1080
   tcctgcacca ggactggctg aatggcaagg agtacaagtg caaggtctcc aacaaagccc 1140
   tcccagcccc catcgagaaa accatctcca aagccaaagg tgggacccgt ggggtgcgag 1200
   ggccacatgg acagaggccg gctcggccca ccctctgccc tgagagtgac cgctgtacca 1260
   acctctgtcc ctacagggca gccccgagaa ccacaggtgt acaccctgcc cccatcccgg 1320
   gatgagctga ccaagaacca ggtcagcctg acctgcctgg tcaaaggctt ctatcccagc 1380
   gacatcgccg tggagtggga gagcaatggg cagccggaga acaactacaa gaccacgcct 1440
   cccgtgctgg actccgacgg ctccttcttc ctctacagca agctcaccgt ggacaagagc 1500
   aggtggcagc aggggaacgt cttctcatgc tccgtgatgc atgaggctct gcacaaccac 1560
   tacacacaga agagcctctc cctgtctccg ggtaaatgag tgccacggcc ggcaagcccc 1620
   cgctccccag gctctcgggg tcgcgcgagg atgcttggca cgtaccccgt gtacatactt 1680
   cccaggcacc cagcatggaa ataaagcacc cagcgcttcc ctgggcccct gcgagactgt 1740
   gatggttctt tccacgggtc aggccgagtc tgaggcctga gtggcatgag ggaggcagag 1800
   tgggtcccac tgtccccaca ctggcccagg ctgtgcaggt gtgcctgggc cgcctagggt 1860
   ggggctcagc caggggctgc cctcggcagg gtgggggatt tgccagcgtg gccctccctc 1920
   cagcagcagc tgccctgggc tgggccacga gaagccctag gagcccctgg ggacagacac 1980
   acagcccctg cctctgtagg agactgtcct gttctgtgag cgccctgtcc tccgacccgc 2040
   atgcccactc gggggcatgc ctagtccatg tgcgtaggga caggccctcc ctcacccatc 2100
   tacccccacg gcactaaccc ctggcagccc tgcccagcct cgcacccgca tggggacaca 2160
   accgactccg gggacatgca ctctcgggcc ctgtggagag actggtccag atgcccacac 2220
   acacactcag cccagacccg ttcaacaaac cccgcactga ggttggccgg ccacacggcc 2280
   accacacaca cacgtgcacg cctcacacac ggagcctcac ccgggcgaac cgcacagcac 2340
   ccagaccaga gcaaggtcct cgcacacgtg aacactcctc ggacacaggc ccccacgagc 2400
   cccacgcggc acctcaaggc ccacgagccg ctcggcagct tctccacatg ctgacctgct 2460
   cagacaaacc cagccctcct ctcacaaggt gcccctgcag ccgccacaca cacacagggg 2520
   atcacacacc acgtcacgtc cctggccctg gcccacttcc cagtgccgcc cttccctgca 2580
   gctggggtca catgaggtgt gggcttcacc atcctcctgc cctctgggcc tcagggaggg 2640
   acacgggaga cggggagcgg gtcctgctga gggccaggtc gctatctagg gccgggtgtc 2700
   tggctgagcc ccggggccaa agctggtgcc cagggcgggc agctgtgggg agctgacctc 2760
   aggacattgt tggcccatcc cggccgggcc ctacatcctg ggtcctgcca cagagggaat 2820
   cacccccaga ggcccaagcc cagggggaca cagcactgac cacccccttc ctgtccagag 2880
   ctgcaactgg aggagagctg tgcggaggcg caggacgggg agctggacgg gctgtggacg 2940
   accatcacca tcttcatcac actcttcctg ttaagcgtgt gctacagtgc caccgtcacc 3000
   ttcttcaagg tcggccgcac gttgtcccca gctgtccttg acattgtccc ccatgctgtc 3060
   acaaactgtc tctgacactg tcccacaggc tgtccccacc tgtccctgac gctgtccccc 3120
   atgctctcac aaactgtccc tgacattgtc cccaatgctg cccccacctg tccaacagtg 3180
   tcccccaggc tctccccaca tgtccccgac actgtccccc atgctgtccc catctgtccc 3240
   caacactgtc ccccaccctg tccccctttg tccccaacac tgtcccccac agtttccacc 3300
   tgtccctgac actgtccccc atgctttccc cacctgtccc tgacaccatc ccccactctg 3360
   tcccctatag ttcctggccc tgtcccccac gctgtcccct acagtacctg gcactgtccc 3420
   ccatgctgtc ccctcctgta tgaaaccctg tcccacatgc tgtccccacc tgtccgtgac 3480
   aatatccccc acactgtccc cacctgtccc cgacactctc ctccacgttg ttcttaccta 3540
   aacccgacac tttcctccat gctgtcccca cccatctccg acactgtacc ccacgttgtc 3600
   cccacctgtc ctcaacactg tcccccatgc tgtccccacc tgtccccaac actctcctcc 3660
   atgctgtccc cacctgtccc tgatattgtc ccccatgcag tctccacctg tccccaatgc 3720
   tgtcccccag gctgtaccta ccagtacaac actgtccccc atgctgtccc cacctgtccc 3780
   tgacactgtc ccccacgctg tcccctcctg tccccgacac tgtcccccac actgtcccca 3840
   cctgtcccca acactatcct ccatgctgtc ccctcctgtc cccacctgtc ccctacactg 3900
   tcccccatgc tgtccccacc agtccccaaa actttcctcc acactgtccc cacctgtccc 3960
   caacactgtc ccccacgcta tcccccctgt ccccgacaat gtccccactg tttcctcctg 4020
   ttccctccta tccctgacac tgtccgccat gctgtcccca cctgtccctg acactgtctc 4080
   ccactctgtc ccctataatc cctgacactg tcccccacgc cgtcccctcc cgtatgcacc 4140
   actgtccccc aagctgtccc cacctgtcct caacacagtc ccccatgctg tccccacctg 4200
   tccccaacac tctcctccat gtccccacct gtccctgata ttgtccccca tgcagtcccc 4260
   acctgtcccc gatgctgtcc cccgggctgt acctaccagt ccaacactgt cccccacact 4320
   ctccccacct gtccctgata ctgtccccca tgctgtcccc acctgtcccg gacactgttc 4380
   tccacgctct cccctcctgt ccctgacact gtcccccaca ctgtccccac ctgtccccaa 4440
   cactatcctc catcctgtcc caacctgtct cctacactgt cccccatgct gtccccacca 4500
   gtccccaaca ctgtcctcca tgctgtcccc catgtcccca acactgtccc ccatgctatc 4560
   tcccctgtcc ctgacaatgt ccccactgtt tcctgtcccc tcctatccct gacactgtcc 4620
   cccatgctgt ccccacctgt cccccacatg gtctccaccg gtccctgaca ctgtctccca 4680
   ctctgtcccc tataatccct gacactgtcc cccacaccgt cccctcctgt atgcaccact 4740
   gtcccccatg ctgtccccac ctgtccctga tgctgtcctc cacacagtcc ccacctctcc 4800
   ctgacactgt ccccatctct ccccaacact ctcctccatg ctgtccttaa ctgtccccaa 4860
   cactcttcca cactctgtct ccacctgtcc ctgacactgt cccccacact gtcctcacct 4920
   gtgtctgaca ctgtccccca cgctgtcccc acctgtccct gacgctgtct tctgtgctgt 4980
   ccacatgctg ttggtgccct ggctctgctc tctatcacca agcctcagag caggcagtgg 5040
   tgaggccatg gcacctgggt ggcatgaggg gccggatggg cctcaggggc agggctgtgg 5100
   cctgcgtgga ctgacgggtg ggtgggcctt gggggcagag aggtggcctc agtgccctga 5160
   ggggtgggtg gggctcgggg gcagggctgt ggcctcgctc acccctgtgc tgtgccttgc 5220
   ctacaggtga agtggatctt ctcctcggtg gtggacctga agcagaccat catccccgac 5280
   tacaggaaca tgatcggaca gggggcctag ggccaccctc tgcggggtgt ccagggccgc 5340
   ccagacccca cacaccagcc atgggccatg ctcagccacc acccaggcca cacctgcccc 5400
   cgacctcacc gccctcaacc ccatgactct ctggcctcgc agttgccctc tgaccctgac 5460
   acacctgaca cgcccccctt ccagaccctg tgcatagcag gtctacccca gacctccgct 5520
   gcttggtgca tgcagggcac tgggggccag gtgtcccctc agcaggacgt ccttgccctc 5580
   cggaccacaa ggtgctcaca caaaaggagg cagtgaccgg tatcccaggc ccccacccag 5640
   gcaggacctc gccctggagc caaccccgtc cacgccagcc tcctgaacac aggcgtggtt 5700
   tccagatggt gagtgggagc gtcagccgcc aaggtaggga agccacagca ccatcaggcc 5760
   ctgttgggga ggcttccgag agctgcgaag gctcactcag acggccttcc tcccagcccg 5820
   cagccagcca gcctccattc cgggcactcc cgtgaactcc tgacatgagg aatgaggttg 5880
   ttctgatttc aagcaaagaa cgctgctctc tggctcctgg gaacagtctc agtgccagca 5940
   ccaccccttg gctgcctgcc cacactgctg gattctcggg tggaactgga cccgcaggga 6000
   cagccagccc cagagtccgc actggggaga gaaggggcca ggcccaggac actgccacct 6060
   cccacccact ccagtccacc gagatcactc agagaagagc ctgggccatg tggccgctgc 6120
   aggagcccca cagtgcaagg gtgaggatag cccaaggaag ggctgggcat ctgcccagac 6180
   aggcctccca gagaaggctg gtgaccaggt cccaggcggg caagactcag ccttggtggg 6240
   gcctgaggac agaggaggcc caggagcatc ggggagagag gtggagggac accgggagag 6300
   ccaggagcgt ggacacagcc agaactcatc acagaggctg gcgtccagcc ccgggtcacg 6360
   tgcagcagga acaagcagcc actctggggg caccaggtgg agaggcaaga cgacaaagag 6420
   ggtgcccgtg ttcttgcgaa agcagggctg ctggccacga gtgctggaca gaggccccca 6480
   cgctctgctg cccccatcac gccgttccgt gactgtcacg cagaatctgc agacaggaag 6540
   ggagactcga gcgggagtgc ggccagcgcc tgcctcggcc gtcagggagg actcctgggc 6600
   tcactcgaag gaggtgccac catttcagct ttggtagctt ttcttcttct tttaaatttt 6660
   ctaaagctca ttaattgtct ttgatgtttc ttttgtgatg acaataaaat atccttttta 6720
   agtcttgta 6729
<210> 366
   <211> 888
   <212> DNA
   <213> homo sapiens;
<400> 366
   gcctccacca agggcccatc ggtcttcccc ctggcaccct cctccaagag cacctctggg      60
   ggcacagcag ccctgggctg cctggtcaag gactacttcc ccgaaccggt gacggtgtcg     120
   tggaactcag gcgccctgac cagcggcgtg cacaccttcc cggctgtcct acagtcctca     180
   ggactctact ccctcagcag cgtggtgacc gtgccctcca gcagcttggg cacccagacc     240
   tacatctgca acgtgaatca caagcccagc aacaccaagg tggacaagaa agttgagccc     300
   aaatcttgtg acaaaactca cacatgccca ccgtgcccag cacctgaact cctgggggga     360
   ccgtcagtct tcctcttccc cccaaaaccc aaggacaccc tcatgatctc ccggacccct     420
   gaggtcacat gcgtggtggt ggacgtgagc cacgaagacc ctgaggtcaa gttcaactgg     480
   tacgtggacg gcgtggagta caagtgcaag gtctccaaca aagccctccc agcccccatc     540
   gagaaaacca tctccaaagc caaagggcag ccccgagaac cacaggtgta caccctgccc     600
   ccatcccggg atgagctgac caagaaccag gtcagcctga cctgcctggt caaaggcttc     660
   tatcccagcg acatcgccgt ggagtgggag agcaatgggc agccggagaa caactacaag     720
   accacgcctc ccgtgctgga ctccgacggc tccttcttcc tctacagcaa gctcaccgtg     780
   gacaagagca ggtggcagca ggggaacgtc ttctcatgct ccgtgatgca tgaggctctg     840
   cacaaccact acacacagaa gagcctctcc ctgtctccgg gtaaatga                  888
<210> 367
   <211> 993
   <212> DNA
   <213> homo sapiens;
<400> 367
   gcctccacca agggcccatc ggtcttcccc ctggcaccct cctccaagag cacctctggg 60
   ggcacagcag ccctgggctg cctggtcaag gactacttcc ccgaaccggt gacggtgtcg 120
   tggaactcag gcgccctgac cagcggcgtg cacaccttcc cggctgtcct acagtcctca 180
   ggactctact ccctcagcag cgtggtgacc gtgccctcca gcagcttggg cacccagacc 240
   tacatctgca acgtgaatca caagcccagc aacaccaagg tggacaagaa agttgagccc 300
   aaatcttgtg acaaaactca cacatgccca ccgtgcccag cacctgaact cctgggggga 360
   ccgtcagtct tcctcttccc cccaaaaccc aaggacaccc tcatgatctc ccggacccct 420
   gaggtcacat gcgtggtggt ggacgtgagc cacgaagacc ctgaggtcaa gttcaactgg 480
   tacgtggacg gcgtggaggt gcataatgcc aagacaaagc cgcgggagga gcagtacaac 540
   agcacgtacc gtgtggtcag cgtcctcacc gtcctgcacc aggactggct gaatggcaag 600
   gagtacaagt gcaaggtctc caacaaagcc ctcccagccc ccatcgagaa aaccatctcc 660
   aaagccaaag ggcagccccg agaaccacag gtgtacaccc tgcccccatc ccgggatgag 720
   ctgaccaaga accaggtcag cctgacctgc ctggtcaaag gcttctatcc cagcgacatc 780
   gccgtggagt gggagagcaa tgggcagccg gagaacaact acaagaccac gcctcccgtg 840
   ctggactccg acggctcctt cttcctctac agcaagctca ccgtggacaa gagcaggtgg 900
   cagcagggga acgtcttctc atgctccgtg atgcatgagg ctctgcacaa ccactacaca 960
   cagaagagcc tctccctgtc tccgggtaaa tga 993
<210> 368
   <211> 1200
   <212> DNA
   <213> homo sapiens;
<400> 368
   gcctccacca agggcccatc ggtcttcccc ctggcaccct cctccaagag cacctctggg      60
   ggcacagcag ccctgggctg cctggtcaag gactacttcc ccgaaccggt gacggtgtcg     120
   tggaactcag gcgccctgac cagcggcgtg cacaccttcc cggctgtcct acagtcctca     180
   ggactctact ccctcagcag cgtggtgacc gtgccctcca gcagcttggg cacccagacc     240
   tacatctgca acgtgaatca caagcccagc aacaccaagg tggacaagaa agttgagccc     300
   aaatcttgtg acaaaactca cacatgccca ccgtgcccag cacctgaact cctgggggga     360
   ccgtcagtct tcctcttccc cccaaaaccc aaggacaccc tcatgatctc ccggacccct     420
   gaggtcacat gcgtggtggt ggacgtgagc cacgaagacc ctgaggtcaa gttcaactgg     480
   tacgtggacg gcgtggaggt gcataatgcc aagacaaagc cgcgggagga gcagtacaac     540
   agcacgtacc gtgtggtcag cgtcctcacc gtcctgcacc aggactggct gaatggcaag     600
   gagtacaagt gcaaggtctc caacaaagcc ctcccagccc ccatcgagaa aaccatctcc     660
   aaagccaaag ggcagccccg agaaccacag gtgtacaccc tgcccccatc ccgggatgag     720
   ctgaccaaga accaggtcag cctgacctgc ctggtcaaag gcttctatcc cagcgacatc     780
   gccgtggagt gggagagcaa tgggcagccg gagaacaact acaagaccac gcctcccgtg     840
   ctggactccg acggctcctt cttcctctac agcaagctca ccgtggacaa gagcaggtgg     900
   cagcagggga acgtcttctc atgctccgtg atgcatgagg ctctgcacaa ccactacaca     960
   cagaagagcc tctccctgtc tccggagctg caactggagg agagctgtgc ggaggcgcag    1020
   gacggggagc tggacgggct gtggacgacc atcaccatct tcatcacact cttcctgtta    1080
   agcgtgtgct acagtgccac cgtcaccttc ttcaaggtga agtggatctt ctcctcggtg    1140
   gtggacctga agcagaccat catccccgac tacaggaaca tgatcggaca gggggcctag    1200
<210> 369
   <211> 1739
   <212> DNA
   <213> homo sapiens;
<400> 369
   gcctccacca agggcccatc ggtcttcccc ctggcgccct gctccaggag cacctccgag 60
   agcacagcgg ccctgggctg cctggtcaag gactacttcc ccgaaccggt gacggtgtcg 120
   tggaactcag gcgctctgac cagcggcgtg cacaccttcc cggctgtcct acagtcctca 180
   ggactctact ccctcagcag cgtggtgacc gtgccctcca gcaacttcgg cacccagacc 240
   tacacctgca acgtagatca caagcccagc aacaccaagg tggacaagac agttggtgag 300
   aggccagctc agggagggag ggtgtctgct ggaagccagg ctcagccctc ctgcctggac 360
   gcaccccggc tgtgcagccc cagcccaggg cagcaaggca ggccccatct gtctcctcac 420
   ccggaggcct ctgcccgccc cactcatgct cagggagagg gtcttctggc tttttccacc 480
   aggctccagg caggcacagg ctgggtgccc ctaccccagg cccttcacac acaggggcag 540
   gtgcttggct cagacctgcc aaaagccata tccgggagga ccctgcccct gacctaagcc 600
   gaccccaaag gccaaactgt ccactccctc agctcggaca ccttctctcc tcccagatcc 660
   gagtaactcc caatcttctc tctgcagagc gcaaatgttg tgtcgagtgc ccaccgtgcc 720
   caggtaagcc agcccaggcc tcgccctcca gctcaaggcg ggacaggtgc cctagagtag 780
   cctgcatcca gggacagacc ccagctgggt gctgacacgt ccacctccat ctcttcctca 840
   gcaccacctg tggcaggacc gtcagtcttc ctcttccccc caaaacccaa ggacaccctc 900
   atgatctccc ggacccctga ggtcacgtgc gtggtggtgg acgtgagcca cgaagacccc 960
   gaggtccagt tcaactggta cgtggacggc gtggaggtgc ataatgccaa gacaaagcca 1020
   cgggaggagc agttcaacag cacgttccgt gtggtcagcg tcctcaccgt cgtgcaccag 1080
   gactggctga acggcaagga gtacaagtgc aaggtctcca acaaaggcct cccagccccc 1140
   atcgagaaaa ccatctccaa aaccaaaggt gggacccgcg gggtatgagg gccacatgga 1200
   cagaggccgg ctcggcccac cctctgccct gggagtgacc gctgtgccaa cctctgtccc 1260
   tacagggcag ccccgagaac cacaggtgta caccctgccc ccatcccggg aggagatgac 1320
   caagaaccag gtcagcctga cctgcctggt caaaggcttc taccccagcg acatctccgt 1380
   ggagtgggag agcaatgggc agccggagaa caactacaag accacacctc ccatgctgga 1440
   ctccgacggc tccttcttcc tctacagcaa gctcaccgtg gacaagagca ggtggcagca 1500
   ggggaacgtc ttctcatgct ccgtgatgca tgaggctctg cacaaccact acacacagaa 1560
   gagcctctcc ctgtctccgg gtaaatgagt gccacggccg gcaagccccc gctccccagg 1620
   ctctcggggt cgcgcgagga tgcttggcac gtaccccgtc tacatacttc ccgggcaccc 1680
   agcatggaaa taaagcaccc agcgctgccc tgggcccctg cgagactgtg atggttctt 1739
<210> 370
   <211> 981
   <212> DNA
   <213> homo sapiens;
<400> 370
   gcctccacca agggcccatc ggtcttcccc ctggcgccct gctccaggag cacctccgag      60
   agcacagcgg ccctgggctg cctggtcaag gactacttcc ccgaaccggt gacggtgtcg     120
   tggaactcag gcgctctgac cagcggcgtg cacaccttcc cggctgtcct acagtcctca     180
   ggactctact ccctcagcag cgtggtgacc gtgccctcca gcaacttcgg cacccagacc     240
   tacacctgca acgtagatca caagcccagc aacaccaagg tggacaagac agttgagcgc     300
   aaatgttgtg tcgagtgccc accgtgccca gcaccacctg tggcaggacc gtcagtcttc     360
   ctcttccccc caaaacccaa ggacaccctc atgatctccc ggacccctga ggtcacgtgc     420
   gtggtggtgg acgtgagcca cgaagacccc gaggtccagt tcaactggta cgtggacggc     480
   gtggaggtgc ataatgccaa gacaaagcca cgggaggagc agttcaacag cacgttccgt     540
   gtggtcagcg tcctcaccgt cgtgcaccag gactggctga acggcaagga gtacaagtgc     600
   aaggtctcca acaaaggcct cccagccccc atcgagaaaa ccatctccaa aaccaaaggg     660
   cagccccgag aaccacaggt gtacaccctg cccccatccc gggaggagat gaccaagaac     720
   caggtcagcc tgacctgcct ggtcaaaggc ttctacccca gcgacatctc cgtggagtgg     780
   gagagcaatg ggcagccgga gaacaactac aagaccacac ctcccatgct ggactccgac     840
   ggctccttct tcctctacag caagctcacc gtggacaaga gcaggtggca gcaggggaac     900
   gtcttctcat gctccgtgat gcatgaggct ctgcacaacc actacacaca gaagagcctc     960
   tccctgtctc cgggtaaatg a                                                981
<210> 371
   <211> 981
   <212> DNA
   <213> homo sapiens;
<400> 371
   gcctccacca agggcccatc ggtcttcccc ctggcgccct gctccaggag cacctccgag 60
   agcacagcgg ccctgggctg cctggtcaag gactacttcc ccgaaccggt gacggtgtcg 120
   tggaactcag gcgctctgac cagcggcgtg cacaccttcc cggctgtcct acagtcctca 180
   ggactctact ccctcagcag cgtggtgacc gtgacctcca gcaacttcgg cacccagacc 240
   tacacctgca acgtagatca caagcccagc aacaccaagg tggacaagac agttgagcgc 300
   aaatgttgtg tcgagtgccc accgtgccca gcaccacctg tggcaggacc gtcagtcttc 360
   ctcttccccc caaaacccaa ggacaccctc atgatctccc ggacccctga ggtcacgtgc 420
   gtggtggtgg acgtgagcca cgaagacccc gaggtccagt tcaactggta cgtggacggc 480
   atggaggtgc ataatgccaa gacaaagcca cgggaggagc agttcaacag cacgttccgt 540
   gtggtcagcg tcctcaccgt cgtgcaccag gactggctga acggcaagga gtacaagtgc 600
   aaggtctcca acaaaggcct cccagccccc atcgagaaaa ccatctccaa aaccaaaggg 660
   cagccccgag aaccacaggt gtacaccctg cccccatccc gggaggagat gaccaagaac 720
   caggtcagcc tgacctgcct ggtcaaaggc ttctacccca gcgacatctc cgtggagtgg 780
   gagagcaatg ggcagccgga gaacaactac aagaccacac ctcccatgct ggactccgac 840
   ggctccttct tcctctacag caagctcacc gtggacaaga gcaggtggca gcaggggaac 900
   gtcttctcat gctccgtgat gcatgaggct ctgcacaacc actacacaca gaagagcctc 960
   tccctgtctc cgggtaaatg a 981
<210> 372
   <211> 1739
   <212> DNA
   <213> homo sapiens;
<400> 372
   gcctccacca agggcccatc ggtcttcccc ctggcgccct gctccaggag cacctccgag 60
   agcacagcgg ccctgggctg cctggtcaag gactacttcc ccgaaccggt gacggtgtcg 120
   tggaactcag gcgctctgac cagcggcgtg cacaccttcc cggctgtcct acagtcctca 180
   ggactctact ccctcagcag cgtggtgacc gtgacctcca gcaacttcgg cacccagacc 240
   tacacctgca acgtagatca caagcccagc aacaccaagg tggacaagac agttggtgag 300
   aggccagctc agggagggag ggtgtctgct ggaagccagg ctcagccctc ctgcctggac 360
   gcaccccggc tgtgcagccc cagcccaggg cagcaaggca ggccccatct gtctcctcac 420
   ccggaggcct ctgcccgccc cactcatgct cagggagagg gtcttctggc tttttccacc 480
   aggctccagg caggcacagg ctgggtgccc ctaccccagg cccttcacac acaggggcag 540
   gtgcttggct cagacctgcc aaaagccata tccgggagga ccctgcccct gacctaagcc 600
   gaccccaaag gccaaactgt ccactccctc agctcggaca ccttctctcc tcccagatcc 660
   gagtaactcc caatcttctc tctgcagagc gcaaatgttg tgtcgagtgc ccaccgtgcc 720
   caggtaagcc agcccaggcc tcgccctcca gctcaaggcg ggacaggtgc cctagagtag 780
   cctgcatcca gggacagacc ccagctgggt gctgacacgt ccacctccat ctcttcctca 840
   gcaccacctg tggcaggacc gtcagtcttc ctcttccccc caaaacccaa ggacaccctc 900
   atgatctccc ggacccctga ggtcacgtgc gtggtggtgg acgtgagcca cgaagacccc 960
   gaggtccagt tcaactggta cgtggacggc atggaggtgc ataatgccaa gacaaagcca 1020
   cgggaggagc agttcaacag cacgttccgt gtggtcagcg tcctcaccgt cgtgcaccag 1080
   gactggctga acggcaagga gtacaagtgc aaggtctcca acaaaggcct cccagccccc 1140
   atcgagaaaa ccatctccaa aaccaaaggt gggacccgcg gggtatgagg gccacatgga 1200
   cagaggccgg ctcggcccac cctctgccct gggagtgacc gctgtgccaa cctctgtccc 1260
   tacagggcag ccccgagaac cacaggtgta caccctgccc ccatcccggg aggagatgac 1320
   caagaaccag gtcagcctga cctgcctggt caaaggcttc taccccagcg acatctccgt 1380
   ggagtgggag agcaatgggc agccggagaa caactacaag accacacctc ccatgctgga 1440
   ctccgacggc tccttcttcc tctacagcaa gctcaccgtg gacaagagca ggtggcagca 1500
   ggggaacgtc ttctcatgct ccgtgatgca tgaggctctg cacaaccact acacacagaa 1560
   gagcctctcc ctgtctccgg gtaaatgagt gccacggccg gcaagccccc gctccccagg 1620
   ctctcggggt cgcgcgagga tgcttggcac gtaccccgtc tacatacttc ccgggcaccc 1680
   agcatggaaa taaagcaccc agcgctgccc tgggcccctg cgagactgtg atggttctt 1739
<210> 373
   <211> 2304
   <212> DNA
   <213> homo sapiens;
<400> 373
   cttccaccaa gggcccatcg gtcttccccc tggcgccctg ctccaggagc acctctgggg      60
   gcacagcggc cctgggctgc ctggtcaagg actacttccc agaaccggtg acggtgtcgt     120
   ggaactcagg cgccctgacc agcggcgtgc acaccttccc ggctgtccta cagtcctcag     180
   gactctactc cctcagcagc gtggtgaccg tgccctccag cagcttgggc acccagacct     240
   acacctgcaa cgtgaatcac aagcccagca acaccaaggt ggacaagaga gttggtgaga     300
   ggccagcgca gggagggagg gtgtctgctg gaagccaggc tcagccctcc tgcctggacg     360
   catcccggct gtgcagtccc agcccagggc accaaggcag gccccgtctg actcctcacc     420
   cggaggcctc tgcccgcccc actcatgctc agggagaggg tcttctggct ttttccacca     480
   ggctccgggc aggcacaggc tggatgcccc taccccaggc ccttcacaca caggggcagg     540
   tgctgcgctc agagctgcca agagccatat ccaggaggac cctgcccctg acctaagccc     600
   accccaaagg ccaaactctc tactcactca gctcagatac cttctctctt cccagatctg     660
   agtaactccc aatcttctct ctgcagagct caaaacccca cttggtgaca caactcacac     720
   atgcccacgg tgcccaggta agccagccca ggcctcgccc tccagctcaa ggcgggacaa     780
   gagccctaga gtggcctgag tccagggaca ggccccagca gggtgctgac gcatccacct     840
   ccatcccaga tccccgtaac tcccaatctt ctctctgcag agcccaaatc ttgtgacaca     900
   cctcccccgt gcccacggtg cccaggtaag ccagcccagg cctcgccctc cagctcaagg     960
   caggacaaga gccctagagt ggcctgagtc cagggacagg ccccagcagg gtgctgacgc    1020
   gtccacctcc atcccagatc cccgtaactc ccaatcttct ctctgcagag cccaaatctt    1080
   gtgacacacc tcccccatgc ccacggtgcc caggtaagcc agcccaggcc tcgccctcca    1140
   gctcaaggcg ggacaagagc cctagagtgg cctgagtcca gggacaggcc ccagcagggt    1200
   gctgacgcat ccacctccat cccagatccc cgtaactccc aatcttctct ctgcagagcc 1260
   caaatcttgt gacacacctc ccccgtgccc aaggtgccca ggtaagccag cccaggcctc 1320
   gccctccagc tcaaggcagg acaggtgccc tagagtggcc tgcatccagg gacaggtccc 1380
   agtcgggtgc tgacacatct gcctccatct cttcctcagc acctgaactc ctgggaggac 1440
   cgtcagtctt cctcttcccc ccaaaaccca aggataccct tatgatttcc cggacccctg 1500
   aggtcacgtg cgtggtggtg gacgtgagcc acgaagaccc cgaggtccag ttcaagtggt 1560
   acgtggacgg cgtggaggtg cataatgcca agacaaagcc gcgggaggag cagtacaaca 1620
   gcacgttccg tgtggtcagc gtcctcaccg tcctgcacca ggactggctg aacggcaagg 1680
   agtacaagtg caaggtctcc aacaaagccc tcccagcccc catcgagaaa accatctcca 1740
   aaaccaaagg tgggacccgc ggggtatgag ggccacatgg acagaggcca gcttgaccca 1800
   ccctctgccc tgggagtgac cgctgtgcca acctctgtcc ctacaggaca gccccgagaa 1860
   ccacaggtgt acaccctgcc cccatcccgg gaggagatga ccaagaacca ggtcagcctg 1920
   acctgcctgg tcaaaggctt ctaccccagc gacatcgccg tggagtggga gagcagcggg 1980
   cagccggaga acaactacaa caccacgcct cccatgctgg actccgacgg ctccttcttc 2040
   ctctacagca agctcaccgt ggacaagagc aggtggcagc aggggaacat cttctcatgc 2100
   tccgtgatgc atgaggctct gcacaaccgc ttcacgcaga agagcctctc cctgtctccg 2160
   ggtaaatgag tgcgacggcc ggcaagcccc cgctccccgg gctctcgggg tcgcgcgagg 2220
   atgcttggca cgtaccccgt gtacatactt cccgggcacc cagcatggaa ataaagcacc 2280
   cagcgctgcc ctgggcccct gcga 2304
<210> 374
   <211> 1134
   <212> DNA
   <213> homo sapiens;
<220>
   <221> misc_feature
   <222> (1)..(1)
   <223> n is a, c, g, or t
<400> 374
   ncttccacca agggcccatc ggtcttcccc ctggcgccct gctccaggag cacctctggg      60
   ggcacagcgg ccctgggctg cctggtcaag gactacttcc cagaaccggt gacggtgtcg     120
   tggaactcag gcgccctgac cagcggcgtg cacaccttcc cggctgtcct acagtcctca     180
   ggactctact ccctcagcag cgtggtgacc gtgccctcca gcagcttggg cacccagacc     240
   tacacctgca acgtgaatca caagcccagc aacaccaagg tggacaagag agttgagctc     300
   aaaaccccac ttggtgacac aactcacaca tgcccacggt gcccagagcc caaatcttgt     360
   gacacacctc ccccgtgccc acggtgccca gagcccaaat cttgtgacac acctccccca     420
   tgcccacggt gcccagagcc caaatcttgt gacacacctc ccccgtgccc aaggtgccca     480
   gcacctgaac tcctgggagg accgtcagtc ttcctcttcc ccccaaaacc caaggatacc     540
   cttatgattt cccggacccc tgaggtcacg tgcgtggtgg tggacgtgag ccacgaagac     600
   cccgaggtcc agttcaagtg gtacgtggac ggcgtggagg tgcataatgc caagacaaag     660
   ccgcgggagg agcagtacaa cagcacgttc cgtgtggtca gcgtcctcac cgtcctgcac     720
   caggactggc tgaacggcaa ggagtacaag tgcaaggtct ccaacaaagc cctcccagcc     780
   cccatcgaga aaaccatctc caaaaccaaa ggacagcccc gagaaccaca ggtgtacacc     840
   ctgcccccat cccgggagga gatgaccaag aaccaggtca gcctgacctg cctggtcaaa     900
   ggcttctacc ccagcgacat cgccgtggag tgggagagca gcgggcagcc ggagaacaac     960
   tacaacacca cgcctcccat gctggactcc gacggctcct tcttcctcta cagcaagctc    1020
   accgtggaca agagcaggtg gcagcagggg aacatcttct catgctccgt gatgcatgag    1080
   gctctgcaca accgcttcac gcagaagagc ctctccctgt ctccgggtaa atga          1134
<210> 375
   <211> 1134
   <212> DNA
   <213> homo sapiens;
<220>
   <221> misc_feature
   <222> (1)..(1)
   <223> n is a, c, g, or t
<400> 375
   ncttccacca agggcccatc ggtcttcccc ctggcgccct gctccaggag cacctctggg      60
   ggcacagcgg ccctgggctg cctggtcaag gactacttcc cagaaccggt gacggtgtcg     120
   tggaactcag gcgccctgac cagcggcgtg cacaccttcc cggctgtcct acagtcctca     180
   ggactctact ccctcagcag cgtggtgacc gtgccctcca gcagcttggg cacccagacc     240
   tacacctgca acgtgaatca caagcccagc aacaccaagg tggacaagag agttgagctc     300
   aaaaccccac ttggtgacac aactcacaca tgcccacggt gcccagagcc caaatcttgt     360
   gacacacctc ccccgtgccc acggtgccca gagcccaaat cttgtgacac acctccccca     420
   tgcccacggt gcccagagcc caaatcttgt gacacacctc ccccgtgccc aaggtgccca     480
   gcacctgaac tcctgggagg accgtcagtc ttcctcttcc ccccaaaacc caaggatacc     540
   cttatgattt cccggacccc tgaggtcacg tgcgtggtgg tggacgtgag ccacgaagac     600
   cccgaggtcc agttcaagtg gtacgtggac ggcgtggagg tgcataatgc caagacaaag     660
   ctgcgggagg agcagtacaa cagcacgttc cgtgtggtca gcgtcctcac cgtcctgcac     720
   caggactggc tgaacggcaa ggagtacaag tgcaaggtct ccaacaaagc cctcccagcc     780
   cccatcgaga aaaccatctc caaaaccaaa ggacagcccc gagaaccaca ggtgtacacc     840
   ctgcccccat cccgggagga gatgaccaag aaccaggtca gcctgacctg cctggtcaaa     900
   ggcttctacc ccagcgacat cgccgtggag tgggagagca gcgggcagcc ggagaacaac     960
   tacaacacca cgcctcccat gctggactcc gacggctcct tcttcctcta cagcaagctc    1020
   accgtggaca agagcaggtg gcagcagggg aacatcttct catgctccgt gatgcatgag    1080
   gctctgcaca accgctacac gcagaagagc ctctccctgt ctccgggtaa atga          1134
<210> 376
   <211> 2304
   <212> DNA
   <213> homo sapiens;
<400> 376
   cttccaccaa gggcccatcg gtcttccccc tggcgccctg ctccaggagc acctctgggg      60
   gcacagcggc cctgggctgc ctggtcaagg actacttccc agaaccggtg acggtgtcgt     120
   ggaactcagg cgccctgacc agcggcgtgc acaccttccc ggctgtccta cagtcctcag     180
   gactctactc cctcagcagc gtggtgaccg tgccctccag cagcttgggc acccagacct     240
   acacctgcaa cgtgaatcac aagcccagca acaccaaggt ggacaagaga gttggtgaga     300
   ggccagcgca gggagggagg gtgtctgctg gaagccaggc tcagccctcc tgcctggacg     360
   catcccggct gtgcagtccc agcccagggc accaaggcag gccccgtctg actcctcacc     420
   cggaggcctc tgcccgcccc actcatgctc agggagaggg tcttctggct ttttccacca     480
   ggctccgggc aggcacaggc tggatgcccc taccccaggc ccttcacaca caggggcagg     540
   tgctgcgctc agagctgcca agagccatat ccaggaggac cctgcccctg acctaagccc     600
   accccaaagg ccaaactctc tactcactca gctcagatac cttctctctt cccagatctg     660
   agtaactccc aatcttctct ctgcagagct caaaacccca cttggtgaca caactcacac     720
   atgcccacgg tgcccaggta agccagccca ggcctcgccc tccagctcaa ggcgggacaa     780
   gagccctaga gtggcctgag tccagggaca ggccccagca gggtgctgac gcatccacct     840
   ccatcccaga tccccgtaac tcccaatctt ctctctgcag agcccaaatc ttgtgacaca     900
   cctcccccgt gcccacggtg cccaggtaag ccagcccagg cctcgccctc cagctcaagg     960
   caggacaaga gccctagagt ggcctgagtc cagggacagg ccccagcagg gtgctgacgc    1020
   gtccacctcc atcccagatc cccgtaactc ccaatcttct ctctgcagag cccaaatctt    1080
   gtgacacacc tcccccatgc ccacggtgcc caggtaagcc agcccaggcc tcgccctcca    1140
   gctcaaggcg ggacaagagc cctagagtgg cctgagtcca gggacaggcc ccagcagggt    1200
   gctgacgcat ccacctccat cccagatccc cgtaactccc aatcttctct ctgcagagcc    1260
   caaatcttgt gacacacctc ccccgtgccc aaggtgccca ggtaagccag cccaggcctc 1320
   gccctccagc tcaaggcagg acaggtgccc tagagtggcc tgcatccagg gacaggtccc 1380
   agtcgggtgc tgacacatct gcctccatct cttcctcagc acctgaactc ctgggaggac 1440
   cgtcagtctt cctcttcccc ccaaaaccca aggataccct tatgatttcc cggacccctg 1500
   aggtcacgtg cgtggtggtg gacgtgagcc acgaagaccc cgaggtccag ttcaagtggt 1560
   acgtggacgg cgtggaggtg cataatgcca agacaaagct gcgggaggag cagtacaaca 1620
   gcacgttccg tgtggtcagc gtcctcaccg tcctgcacca ggactggctg aacggcaagg 1680
   agtacaagtg caaggtctcc aacaaagccc tcccagcccc catcgagaaa accatctcca 1740
   aaaccaaagg tgggacccgc ggggtatgag ggccacatgg acagaggcca gcttgaccca 1800
   ccctctgccc tgggagtgac cgctgtgcca acctctgtcc ctacaggaca gccccgagaa 1860
   ccacaggtgt acaccctgcc cccatcccgg gaggagatga ccaagaacca ggtcagcctg 1920
   acctgcctgg tcaaaggctt ctaccccagc gacatcgccg tggagtggga gagcagcggg 1980
   cagccggaga acaactacaa caccacgcct cccatgctgg actccgacgg ctccttcttc 2040
   ctctacagca agctcaccgt ggacaagagc aggtggcagc aggggaacat cttctcatgc 2100
   tccgtgatgc atgaggctct gcacaaccgc tacacgcaga agagcctctc cctgtctccg 2160
   ggtaaatgag tgcgacggcc ggcaagcccc cgctccccgg gctctcgggg tcgcgcgagg 2220
   atgcttggca cgtaccccgt gtacatactt cccgggcacc cagcatggaa ataaagcacc 2280
   cagcgctgcc ctgggcccct gcga 2304
<210> 377
   <211> 1134
   <212> DNA
   <213> homo sapiens;
<220>
   <221> misc_feature
   <222> (1)..(1)
   <223> n is a, c, g, or t
<400> 377
   ncttccacca agggcccatc ggtcttcccc ctggcgccct gctccaggag cacctctggg      60
   ggcacagcgg ccctgggctg cctggtcaag gactacttcc cagaaccggt gacggtgtcg     120
   tggaactcag gcgccctgac cagcggcgtg cacaccttcc cggctgtcct acagtcctca     180
   ggactctact ccctcagcag cgtggtgacc gtgccctcca gcagcttggg cacccagacc     240
   tacacctgca acgtgaatca caagcccagc aacaccaagg tggacaagag agttgagctc     300
   aaaaccccac ttggtgacac aactcacaca tgcccacggt gcccagagcc caaatcttgt     360
   gacacacctc ccccgtgccc acggtgccca gagcccaaat cttgtgacac acctccccca     420
   tgcccacggt gcccagagcc caaatcttgt gacacacctc ccccgtgccc aaggtgccca     480
   gcacctgaac tcctgggagg accgtcagtc ttcctcttcc ccccaaaacc caaggatacc     540
   cttatgattt cccggacccc tgaggtcacg tgcgtggtgg tggacgtgag ccacgaagac     600
   cccgaggtcc agttcaagtg gtacgtggac ggcgtggagg tgcataatgc caagacaaag     660
   ccgcgggagg agcagttcaa cagcacgttc cgtgtggtca gcgtcctcac cgtcctgcac     720
   caggactggc tgaacggcaa ggagtacaag tgcaaggtct ccaacaaagc cctcccagcc     780
   cccatcgaga aaaccatctc caaaaccaaa ggacagcccc gagaaccaca ggtgtacacc     840
   ctgcccccat cccgggagga gatgaccaag aaccaggtca gcctgacctg cctggtcaaa     900
   ggcttctacc ccagcgacat cgccgtggag tgggagagca gcgggcagcc ggagaacaac     960
   tacaacacca cgcctcccat gctggactcc gacggctcct tcttcctcta cagcaagctc    1020
   accgtggaca agagcaggtg gcagcagggg aacatcttct catgctccgt gatgcatgag    1080
   gctctgcaca accgcttcac gcagaagagc ctctccctgt ctccgggtaa atga          1134
<210> 378
   <211> 2304
   <212> DNA
   <213> homo sapiens;
<400> 378
   cttccaccaa gggcccatcg gtcttccccc tggcgccctg ctccaggagc acctctgggg 60
   gcacagcggc cctgggctgc ctggtcaagg actacttccc agaaccggtg acggtgtcgt 120
   ggaactcagg cgccctgacc agcggcgtgc acaccttccc ggctgtccta cagtcctcag 180
   gactctactc cctcagcagc gtggtgaccg tgccctccag cagcttgggc acccagacct 240
   acacctgcaa cgtgaatcac aagcccagca acaccaaggt ggacaagaga gttggtgaga 300
   ggccagcgca gggagggagg gtgtctgctg gaagccaggc tcagccctcc tgcctggacg 360
   catcccggct gtgcagtccc agcccagggc accaaggcag gccccgtctg actcctcacc 420
   cggaggcctc tgcccgcccc actcatgctc agggagaggg tcttctggct ttttccacca 480
   ggctccgggc aggcacaggc tggatgcccc taccccaggc ccttcacaca caggggcagg 540
   tgctgcgctc agagctgcca agagccatat ccaggaggac cctgcccctg acctaagccc 600
   accccaaagg ccaaactctc tactcactca gctcagatac cttctctctt cccagatctg 660
   agtaactccc aatcttctct ctgcagagct caaaacccca cttggtgaca caactcacac 720
   atgcccacgg tgcccaggta agccagccca ggcctcgccc tccagctcaa ggcgggacaa 780
   gagccctaga gtggcctgag tccagggaca ggccccagca gggtgctgac gcatccacct 840
   ccatcccaga tccccgtaac tcccaatctt ctctctgcag agcccaaatc ttgtgacaca 900
   cctcccccgt gcccacggtg cccaggtaag ccagcccagg cctcgccctc cagctcaagg 960
   caggacaaga gccctagagt ggcctgagtc cagggacagg ccccagcagg gtgctgacgc 1020
   gtccacctcc atcccagatc cccgtaactc ccaatcttct ctctgcagag cccaaatctt 1080
   gtgacacacc tcccccatgc ccacggtgcc caggtaagcc agcccaggcc tcgccctcca 1140
   gctcaaggcg ggacaagagc cctagagtgg cctgagtcca gggacaggcc ccagcagggt 1200
   gctgacgcat ccacctccat cccagatccc cgtaactccc aatcttctct ctgcagagcc 1260
   caaatcttgt gacacacctc ccccgtgccc aaggtgccca ggtaagccag cccaggcctc 1320
   gccctccagc tcaaggcagg acaggtgccc tagagtggcc tgcatccagg gacaggtccc 1380
   agtcgggtgc tgacacatct gcctccatct cttcctcagc acctgaactc ctgggaggac 1440
   cgtcagtctt cctcttcccc ccaaaaccca aggataccct tatgatttcc cggacccctg 1500
   aggtcacgtg cgtggtggtg gacgtgagcc acgaagaccc cgaggtccag ttcaagtggt 1560
   acgtggacgg cgtggaggtg cataatgcca agacaaagcc gcgggaggag cagttcaaca 1620
   gcacgttccg tgtggtcagc gtcctcaccg tcctgcacca ggactggctg aacggcaagg 1680
   agtacaagtg caaggtctcc aacaaagccc tcccagcccc catcgagaaa accatctcca 1740
   aaaccaaagg tgggacccgc ggggtatgag ggccacatgg acagaggcca gcttgaccca 1800
   ccctctgccc tgggagtgac cgctgtgcca acctctgtcc ctacaggaca gccccgagaa 1860
   ccacaggtgt acaccctgcc cccatcccgg gaggagatga ccaagaacca ggtcagcctg 1920
   acctgcctgg tcaaaggctt ctaccccagc gacatcgccg tggagtggga gagcagcggg 1980
   cagccggaga acaactacaa caccacgcct cccatgctgg actccgacgg ctccttcttc 2040
   ctctacagca agctcaccgt ggacaagagc aggtggcagc aggggaacat cttctcatgc 2100
   tccgtgatgc atgaggctct gcacaaccgc ttcacgcaga agagcctctc cctgtctccg 2160
   ggtaaatgag tgcgacggcc ggcaagcccc cgctccccgg gctctcgggg tcgcgcgagg 2220
   atgcttggca cgtaccccgt gtacatactt cccgggcacc cagcatggaa ataaagcacc 2280
   cagcgctgcc ctgggcccct gcga 2304
<210> 379
   <211> 1717
   <212> DNA
   <213> homo sapiens;
<400> 379
   gcttccacca agggcccatc ggtcttcccc ctggcgccct gctccaggag cacctccgag 60
   agcacagccg ccctgggctg cctggtcaag gactacttcc ccgaaccggt gacggtgtcg 120
   tggaactcag gcgccctgac cagcggcgtg cacaccttcc cggctgtcct acagtcctca 180
   ggactctact ccctcagcag cgtggtgacc gtgccctcca gcagcttggg cacgaagacc 240
   tacacctgca acgtagatca caagcccagc aacaccaagg tggacaagag agttggtgag 300
   aggccagcac agggagggag ggtgtctgct ggaagccagg ctcagccctc ctgcctggac 360
   gcaccccggc tgtgcagccc cagcccaggg cagcaaggca ggccccatct gtctcctcac 420
   ccggaggcct ctgaccaccc cactcatgct cagggagagg gtcttctgga tttttccacc 480
   aggctccggg cagccacagg ctggatgccc ctaccccagg ccctgcgcat acaggggcag 540
   gtgctgcgct cagacctgcc aagagccata tccgggagga ccctgcccct gacctaagcc 600
   caccccaaag gccaaactct ccactccctc agctcagaca ccttctctcc tcccagatct 660
   gagtaactcc caatcttctc tctgcagagt ccaaatatgg tcccccatgc ccatcatgcc 720
   caggtaagcc aacccaggcc tcgccctcca gctcaaggcg ggacaggtgc cctagagtag 780
   cctgcatcca gggacaggcc ccagccgggt gctgacgcat ccacctccat ctcttcctca 840
   gcacctgagt tcctgggggg accatcagtc ttcctgttcc ccccaaaacc caaggacact 900
   ctcatgatct cccggacccc tgaggtcacg tgcgtggtgg tggacgtgag ccaggaagac 960
   cccgaggtcc agttcaactg gtacgtggat ggcgtggagg tgcataatgc caagacaaag 1020
   ccgcgggagg agcagttcaa cagcacgtac cgtgtggtca gcgtcctcac cgtcctgcac 1080
   caggactggc tgaacggcaa ggagtacaag tgcaaggtct ccaacaaagg cctcccgtcc 1140
   tccatcgaga aaaccatctc caaagccaaa ggtgggaccc acggggtgcg agggccacat 1200
   ggacagaggt cagctcggcc caccctctgc cctgggagtg accgctgtgc caacctctgt 1260
   ccctacaggg cagccccgag agccacaggt gtacaccctg cccccatccc aggaggagat 1320
   gaccaagaac caggtcagcc tgacctgcct ggtcaaaggc ttctacccca gcgacatcgc 1380
   cgtggagtgg gagagcaatg ggcagccgga gaacaactac aagaccacgc ctcccgtgct 1440
   ggactccgac ggctccttct tcctctacag caggctcacc gtggacaaga gcaggtggca 1500
   ggaggggaat gtcttctcat gctccgtgat gcatgaggct ctgcacaacc actacacaca 1560
   gaagagcctc tccctgtctc tgggtaaatg agtgccaggg ccggcaagcc cccgctcccc 1620
   gggctctcgg ggtcgcgcga ggatgcttgg cacgtacccc gtgtacatac ttcccgggcg 1680
   cccagcatgg aaataaagca cccagcgctg ccctggg 1717
<210> 380
   <211> 984
   <212> DNA
   <213> homo sapiens;
<400> 380
   gcttccacca agggcccatc ggtcttcccc ctggcgccct gctccaggag cacctccgag      60
   agcacagccg ccctgggctg cctggtcaag gactacttcc ccgaaccggt gacggtgtcg     120
   tggaactcag gcgccctgac cagcggcgtg cacaccttcc cggctgtcct acagtcctca     180
   ggactctact ccctcagcag cgtggtgacc gtgccctcca gcagcttggg cacgaagacc     240
   tacacctgca acgtagatca caagcccagc aacaccaagg tggacaagag agttgagtcc     300
   aaatatggtc ccccatgccc atcatgccca gcacctgagt tcctgggggg accatcagtc     360
   ttcctgttcc ccccaaaacc caaggacact ctcatgatct cccggacccc tgaggtcacg     420
   tgcgtggtgg tggacgtgag ccaggaagac cccgaggtcc agttcaactg gtacgtggat     480
   ggcgtggagg tgcataatgc caagacaaag ccgcgggagg agcagttcaa cagcacgtac     540
   cgtgtggtca gcgtcctcac cgtcctgcac caggactggc tgaacggcaa ggagtacaag     600
   tgcaaggtct ccaacaaagg cctcccgtcc tccatcgaga aaaccatctc caaagccaaa     660
   gggcagcccc gagagccaca ggtgtacacc ctgcccccat cccaggagga gatgaccaag     720
   aaccaggtca gcctgacctg cctggtcaaa ggcttctacc ccagcgacat cgccgtggag     780
   tgggagagca atgggcagcc ggagaacaac tacaagacca cgcctcccgt gctggactcc     840
   gacggctcct tcttcctcta cagcaggctc accgtggaca agagcaggtg gcaggagggg     900
   aatgtcttct catgctccgt gatgcatgag gctctgcaca accactacac acagaagagc     960
   ctctccctgt ctctgggtaa atga                                            984
<210> 381
   <211> 984
   <212> DNA
   <213> homo sapiens;
<400> 381
   gcttccacca agggcccatc ggtcttcccc ctggcgccct gctccaggag cacctccgag      60
   agcacagccg ccctgggctg cctggtcaag gactacttcc ccgaaccggt gacggtgtcg     120
   tggaactcag gcgccctgac cagcggcgtg cacaccttcc cggctgtcct acagtcctca     180
   ggactctact ccctcagcag cgtggtgacc gtgccctcca gcagcttggg cacgaagacc     240
   tacacctgca acgtagatca caagcccagc aacaccaagg tggacaagag agttgagtcc     300
   aaatatggtc ccccatgccc atcatgccca gcacctgagt tcctgggggg accatcagtc     360
   ttcctgttcc ccccaaaacc caaggacact ctcatgatct cccggacccc tgaggtcacg     420
   tgcgtggtgg tggacgtgag ccaggaagac cccgaggtcc agttcaactg gtacgtggat     480
   ggcgtggagg tgcataatgc caagacaaag ccgcgggagg agcagttcaa cagcacgtac     540
   cgtgtggtca gcgtcctcac cgtcgtgcac caggactggc tgaacggcaa ggagtacaag     600
   tgcaaggtct ccaacaaagg cctcccgtcc tccatcgaga aaaccatctc caaagccaaa     660
   gggcagcccc gagagccaca ggtgtacacc ctgcccccat cccaggagga gatgaccaag     720
   aaccaggtca gcctgacctg cctggtcaaa ggcttctacc ccagcgacat cgccgtggag     780
   tgggagagca atgggcagcc ggagaacaac tacaagacca cgcctcccgt gctggactcc     840
   gacggctcct tcttcctcta cagcaggctc accgtggaca agagcaggtg gcaggagggg     900
   aatgtcttct catgctccgt gatgcatgag gctctgcaca accactacac acagaagagc     960
   ctctccctgt ctctgggtaa atga                                            984
<210> 382
   <211> 1717
   <212> DNA
   <213> homo sapiens;
<400> 382
   gcttccacca agggcccatc ggtcttcccc ctggcgccct gctccaggag cacctccgag      60
   agcacagccg ccctgggctg cctggtcaag gactacttcc ccgaaccggt gacggtgtcg     120
   tggaactcag gcgccctgac cagcggcgtg cacaccttcc cggctgtcct acagtcctca     180
   ggactctact ccctcagcag cgtggtgacc gtgccctcca gcagcttggg cacgaagacc     240
   tacacctgca acgtagatca caagcccagc aacaccaagg tggacaagag agttggtgag     300
   aggccagcac agggagggag ggtgtctgct ggaagccagg ctcagccctc ctgcctggac     360
   gcaccccggc tgtgcagccc cagcccaggg cagcaaggca ggccccatct gtctcctcac     420
   ccggaggcct ctgaccaccc cactcatgct cagggagagg gtcttctgga tttttccacc     480
   aggctccggg cagccacagg ctggatgccc ctaccccagg ccctgcgcat acaggggcag     540
   gtgctgcgct cagacctgcc aagagccata tccgggagga ccctgcccct gacctaagcc     600
   caccccaaag gccaaactct ccactccctc agctcagaca ccttctctcc tcccagatct     660
   gagtaactcc caatcttctc tctgcagagt ccaaatatgg tcccccatgc ccatcatgcc     720
   caggtaagcc aacccaggcc tcgccctcca gctcaaggcg ggacaggtgc cctagagtag     780
   cctgcatcca gggacaggcc ccagccgggt gctgacgcat ccacctccat ctcttcctca     840
   gcacctgagt tcctgggggg accatcagtc ttcctgttcc ccccaaaacc caaggacact     900
   ctcatgatct cccggacccc tgaggtcacg tgcgtggtgg tggacgtgag ccaggaagac     960
   cccgaggtcc agttcaactg gtacgtggat ggcgtggagg tgcataatgc caagacaaag    1020
   ccgcgggagg agcagttcaa cagcacgtac cgtgtggtca gcgtcctcac cgtcgtgcac    1080
   caggactggc tgaacggcaa ggagtacaag tgcaaggtct ccaacaaagg cctcccgtcc    1140
   tccatcgaga aaaccatctc caaagccaaa ggtgggaccc acggggtgcg agggccacat    1200
   ggacagaggt cagctcggcc caccctctgc cctgggagtg accgctgtgc caacctctgt    1260
   ccctacaggg cagccccgag agccacaggt gtacaccctg cccccatccc aggaggagat    1320
   gaccaagaac caggtcagcc tgacctgcct ggtcaaaggc ttctacccca gcgacatcgc    1380
   cgtggagtgg gagagcaatg ggcagccgga gaacaactac aagaccacgc ctcccgtgct    1440
   ggactccgac ggctccttct tcctctacag caggctcacc gtggacaaga gcaggtggca    1500
   ggaggggaat gtcttctcat gctccgtgat gcatgaggct ctgcacaacc actacacaca    1560
   gaagagcctc tccctgtctc tgggtaaatg agtgccaggg ccggcaagcc cccgctcccc    1620
   gggctctcgg ggtcgcgcga ggatgcttgg cacgtacccc gtgtacatac ttcccgggcg    1680
   cccagcatgg aaataaagca cccagcgctg ccctggg                              1717
<210> 383
   <211> 1546
   <212> DNA
   <213> homo sapiens;
<400> 383
   gcatccccga ccagccccaa ggtcttcccg ctgagcctct gcagcaccca gccagatggg      60
   aacgtggtca tcgcctgcct ggtccagggc ttcttccccc aggagccact cagtgtgacc     120
   tggagcgaaa gcggacaggg cgtgaccgcc agaaacttcc cacccagcca ggatgcctcc     180
   ggggacctgt acaccacgag cagccagctg accctgccgg ccacacagtg cctagccggc     240
   aagtccgtga catgccacgt gaagcactac acgaatccca gccaggatgt gactgtgccc     300
   tgcccaggtc agagggcagg ctggggagtg gggcggggcc accccgtcgt gccctgacac      360
   tgcgcctgca cccgtgttcc ccacagggag ccgccccttc actcacacca gagtggaccg      420
   cgggccgagc cccaggaggt ggtggtggac aggccaggag gggcgaggcg ggggcatggg     480
   gaagtatgtg ctgaccagct caggccatct ctccactcca gttccctcaa ctccacctac     540
   cccatctccc tcaactccac ctaccccatc tccctcatgc tgccaccccc gactgtcact     600
   gcaccgaccg gccctcgagg acctgctctt aggttcagaa gcgaacctca cgtgcacact     660
   gaccggcctg agagatgcct caggtgtcac cttcacctgg acgccctcaa gtgggaagag     720
   cgctgttcaa ggaccacctg agcgtgacct ctgtggctgc tacagcgtgt ccagtgtcct     780
   gccgggctgt gccgagccat ggaaccatgg gaagaccttc acttgcactg ctgcctaccc     840
   cgagtccaag accccgctaa ccgccaccct ctcaaaatcc ggtgggtcca gaccctgctc     900
   ggggccctgc tcagtgctct ggtttgcaaa gcatattcct ggcctgcctc ctccctccca     960
   atcctgggct ccagtgctca tgccaagtac agagggaaac tgaggcaggc tgaggggcca    1020
   ggacacagcc cagggtgccc accagagcag aggggctctc tcatcccctg cccagccccc    1080
   tgacctggct ctctaccctc caggaaacac attccggccc gaggtccacc tgctgccgcc    1140
   gccgtcggag gagctggccc tgaacgagct ggtgacgctg acgtgcctgg cacgcggctt    1200
   cagccccaag gatgtgctgg ttcgctggct gcaggggtca caggagctgc cccgcgagaa    1260
   gtacctgact tgggcatccc ggcaggagcc cagccagggc accaccacct tcgctgtgac    1320
   cagcatactg cgcgtggcag ccgaggactg gaagaagggg gacaccttct cctgcatggt    1380
   gggccacgag gccctgccgc tggccttcac acagaagacc atcgaccgct tggcgggtaa    1440
   acccacccat gtcaatgtgt ctgttgtcat ggcggaggtg gacggcacct gctactgagc    1500
   cgcccgcctg tccccacccc tgaataaact ccatgctccc ccaagc                   1546
<210> 384
   <211> 1062
   <212> DNA
   <213> homo sapiens;
<400> 384
   gcatccccga ccagccccaa ggtcttcccg ctgagcctct gcagcaccca gccagatggg      60
   aacgtggtca tcgcctgcct ggtccagggc ttcttccccc aggagccact cagtgtgacc     120
   tggagcgaaa gcggacaggg cgtgaccgcc agaaacttcc cacccagcca ggatgcctcc     180
   ggggacctgt acaccacgag cagccagctg accctgccgg ccacacagtg cctagccggc     240
   aagtccgtga catgccacgt gaagcactac acgaatccca gccaggatgt gactgtgccc     300
   tgcccagttc cctcaactcc acctacccca tctccctcaa ctccacctac cccatctccc    360
   tcatgctgcc acccccgact gtcactgcac cgaccggccc tcgaggacct gctcttaggt     420
   tcagaagcga acctcacgtg cacactgacc ggcctgagag atgcctcagg tgtcaccttc     480
   acctggacgc cctcaagtgg gaagagcgct gttcaaggac cacctgagcg tgacctctgt     540
   ggctgctaca gcgtgtccag tgtcctgccg ggctgtgccg agccatggaa ccatgggaag     600
   accttcactt gcactgctgc ctaccccgag tccaagaccc cgctaaccgc caccctctca     660
   aaatccggaa acacattccg gcccgaggtc cacctgctgc cgccgccgtc ggaggagctg     720
   gccctgaacg agctggtgac gctgacgtgc ctggcacgcg gcttcagccc caaggatgtg     780
   ctggttcgct ggctgcaggg gtcacaggag ctgccccgcg agaagtacct gacttgggca     840
   tcccggcagg agcccagcca gggcaccacc accttcgctg tgaccagcat actgcgcgtg     900
   gcagccgagg actggaagaa gggggacacc ttctcctgca tggtgggcca cgaggccctg     960
   ccgctggcct tcacacagaa gaccatcgac cgcttggcgg gtaaacccac ccatgtcaat    1020
   gtgtctgttg tcatggcgga ggtggacggc acctgctact ga                       1062
<210> 385
   <211> 1546
   <212> DNA
   <213> homo sapiens;
<400> 385
   gcatccccga ccagccccaa ggtcttcccg ctgagcctct gcagcaccca gccagatggg      60
   aacgtggtca tcgcctgcct ggtccagggc ttcttccccc aggagccact cagtgtgacc     120
   tggagcgaaa gcggacaggg cgtgaccgcc agaaacttcc cacccagcca ggatgcctcc     180
   ggggacctgt acaccacgag cagccagctg accctgccgg ccacacagtg cctagccggc     240
   aagtccgtga catgccacgt gaagcactac acgaatccca gccaggatgt gactgtgccc     300
   tgcccaggtc agagggcagg ctggggagtg gggcggggcc accccgtcgt gccctgacac 360
   tgcgcctgca cccgtgttcc ccacagggag ccgccccttc actcacacca gagtggaccg 420
   cgggccgagc cccaggaggt ggtggtggac aggccaggag gggcgaggcg ggggcatggg 480
   gaagtatgtg ctgaccagct caggccatct ctccactcca gttccctcaa ctccacctac 540
   cccatctccc tcaactccac ctaccccatc tccctcatgc tgccaccccc gactgtcact 600
   gcaccgaccg gccctcgagg acctgctctt aggttcagaa gcgaacctca cgtgcacact 660
   gaccggcctg agagatgcct caggtgtcac cttcacctgg acgccctcaa gtgggaagag 720
   cgctgttcaa ggaccacctg accgtgacct ctgtggctgc tacagcgtgt ccagtgtcct 780
   gccgggctgt gccgagccat ggaaccatgg gaagaccttc acttgcactg ctgcctaccc 840
   cgagtccaag accccgctaa ccgccaccct ctcaaaatcc ggtgggtcca gaccctgctc 900
   ggggccctgc tcagtgctct ggtttgcaaa gcatattcct ggcctgcctc ctccctccca 960
   atcctgggct ccagtgctca tgccaagtac agagggaaac tgaggcaggc tgaggggcca 1020
   ggacacagcc cagggtgccc accagagcag aggggctctc tcatcccctg cccagccccc 1080
   tgacctggct ctctaccctc caggaaacac attccggccc gaggtccacc tgctgccgcc 1140
   gccgtcggag gagctggccc tgaacgagct ggtgacgctg acgtgcctgg cacgcggctt 1200
   cagccccaag gatgtgctgg ttcgctggct gcaggggtca caggagctgc cccgcgagaa 1260
   gtacctgact tgggcatccc ggcaggagcc cagccagggc accaccacct tcgctgtgac 1320
   cagcatactg cgcgtggcag ccgaggactg gaagaagggg gacaccttct cctgcatggt 1380
   gggccacgag gccctgccgc tggccttcac acagaagacc atcgaccgct tggcgggtaa 1440
   acccacccat gtcaatgtgt ctgttgtcat ggcggaggtg gacggcacct gctactgagc 1500
   cgcccgcctg tccccacccc tgaataaact ccatgctccc ccaagc 1546
<210> 386
   <211> 1062
   <212> DNA
   <213> homo sapiens;
<400> 386
   gcatccccga ccagccccaa ggtcttcccg ctgagcctct gcagcaccca gccagatggg      60
   aacgtggtca tcgcctgcct ggtccagggc ttcttccccc aggagccact cagtgtgacc     120
   tggagcgaaa gcggacaggg cgtgaccgcc agaaacttcc cacccagcca ggatgcctcc     180
   ggggacctgt acaccacgag cagccagctg accctgccgg ccacacagtg cctagccggc     240
   aagtccgtga catgccacgt gaagcactac acgaatccca gccaggatgt gactgtgccc     300
   tgcccagttc cctcaactcc acctacccca tctccctcaa ctccacctac cccatctccc     360
   tcatgctgcc acccccgact gtcactgcac cgaccggccc tcgaggacct gctcttaggt     420
   tcagaagcga acctcacgtg cacactgacc ggcctgagag atgcctcagg tgtcaccttc     480
   acctggacgc cctcaagtgg gaagagcgct gttcaaggac cacctgaccg tgacctctgt     540
   ggctgctaca gcgtgtccag tgtcctgccg ggctgtgccg agccatggaa ccatgggaag     600
   accttcactt gcactgctgc ctaccccgag tccaagaccc cgctaaccgc caccctctca     660
   aaatccggaa acacattccg gcccgaggtc cacctgctgc cgccgccgtc ggaggagctg     720
   gccctgaacg agctggtgac gctgacgtgc ctggcacgcg gcttcagccc caaggatgtg     780
   ctggttcgct ggctgcaggg gtcacaggag ctgccccgcg agaagtacct gacttgggca     840
   tcccggcagg agcccagcca gggcaccacc accttcgctg tgaccagcat actgcgcgtg     900
   gcagccgagg actggaagaa gggggacacc ttctcctgca tggtgggcca cgaggccctg     960
   ccgctggcct tcacacagaa gaccatcgac cgcttggcgg gtaaacccac ccatgtcaat    1020
   gtgtctgttg tcatggcgga ggtggacggc acctgctact ga                       1062
<210> 387
   <211> 1507
   <212> DNA
   <213> homo sapiens;
<400> 387
   gcatccccga ccagccccaa ggtcttcccg ctgagcctcg acagcacccc ccaagatggg 60
   aacgtggtcg tcgcatgcct ggtccagggc ttcttccccc aggagccact cagtgtgacc 120
   tggagcgaaa gcggacagaa cgtgaccgcc agaaacttcc cacctagcca ggatgcctcc 180
   ggggacctgt acaccacgag cagccagctg accctgccgg ccacacagtg cccagacggc 240
   aagtccgtga catgccacgt gaagcactac acgaattcca gccaggatgt gactgtgccc 300
   tgccgaggtc agagggcagg ctggggagtg gggcggggcc accccgtcct gccctgacac 360
   tgcgcctgca cccgtgttcc ccacagggag ccgccccttc actcacacca gagtggaccg 420
   cgggccgagc cccaggaggt ggtggtggac aggccaggag gggcgaggcg gggcacgggg 480
   gaagggcgtt ctgaccagct caggccatct ctccactcca gttcccccac ctcccccatg 540
   ctgccacccc cgactgtcgc tgcaccgacc ggccctcgag gacctgctct taggttcaga 600
   agcgaacctc acgtgcacac tgaccggcct gagagatgcc tctggtgcca ccttcacctg 660
   gacgccctca agtgggaaga gcgctgttca aggaccacct gagcgtgacc tctgtggctg 720
   ctacagcgtg tccagtgtcc tgcctggctg tgcccagcca tggaaccatg gggagacctt 780
   cacctgcact gctgcccacc ccgagttgaa gaccccacta accgccaaca tcacaaaatc 840
   cggtgggtcc agaccctgct cggggccctg ctcagtgctc tggtttgcaa agcatattcc 900
   cggcctgcct cctccctccc aatcctgggc tccagtgctc atgccaagta cagagggaaa 960
   ctgaggcagg ctgaggggcc aggacacagc ccagggtgcc caccagagca gaggggctct 1020
   ctcatcccct gcccagcccc ctgacctggc tctctaccct ccaggaaaca cattccggcc 1080
   cgaggtccac ctgctgccgc cgccgtcgga ggagctggcc ctgaacgagc tggtgacgct 1140
   gacgtgcctg gcacgtggct tcagccccaa ggatgtgctg gttcgctggc tgcaggggtc 1200
   acaggagctg ccccgcgaga agtacctgac ttgggcatcc cggcaggagc ccagccaggg 1260
   caccaccacc tacgctgtaa ccagcatact gcgcgtggca gctgaggact ggaagaaggg 1320
   ggagaccttc tcctgcatgg tgggccacga ggccctgccg ctggccttca cacagaagac 1380
   catcgaccgc atggcgggta aacccaccca catcaatgtg tctgttgtca tggcggaggc 1440
   ggatggcacc tgctactgag ccgcccgcct gtccccaccc ctgaataaac tccatgctcc 1500
   cccaagc 1507
<210> 388
   <211> 1023
   <212> DNA
   <213> homo sapiens;
<400> 388
   gcatccccga ccagccccaa ggtcttcccg ctgagcctcg acagcacccc ccaagatggg      60
   aacgtggtcg tcgcatgcct ggtccagggc ttcttccccc aggagccact cagtgtgacc     120
   tggagcgaaa gcggacagaa cgtgaccgcc agaaacttcc cacctagcca ggatgcctcc     180
   ggggacctgt acaccacgag cagccagctg accctgccgg ccacacagtg cccagacggc     240
   aagtccgtga catgccacgt gaagcactac acgaattcca gccaggatgt gactgtgccc     300
   tgccgagttc ccccacctcc cccatgctgc cacccccgac tgtcgctgca ccgaccggcc     360
   ctcgaggacc tgctcttagg ttcagaagcg aacctcacgt gcacactgac cggcctgaga     420
   gatgcctctg gtgccacctt cacctggacg ccctcaagtg ggaagagcgc tgttcaagga     480
   ccacctgagc gtgacctctg tggctgctac agcgtgtcca gtgtcctgcc tggctgtgcc     540
   cagccatgga accatgggga gaccttcacc tgcactgctg cccaccccga gttgaagacc     600
   ccactaaccg ccaacatcac aaaatccgga aacacattcc ggcccgaggt ccacctgctg     660
   ccgccgccgt cggaggagct ggccctgaac gagctggtga cgctgacgtg cctggcacgt     720
   ggcttcagcc ccaaggatgt gctggttcgc tggctgcagg ggtcacagga gctgccccgc     780
   gagaagtacc tgacttgggc atcccggcag gagcccagcc agggcaccac cacctacgct     840
   gtaaccagca tactgcgcgt ggcagctgag gactggaaga agggggagac cttctcctgc     900
   atggtgggcc acgaggccct gccgctggcc ttcacacaga agaccatcga ccgcatggcg     960
   ggtaaaccca cccacatcaa tgtgtctgtt gtcatggcgg aggcggatgg cacctgctac    1020
   tga                                                                  1023
<210> 389
   <211> 1507
   <212> DNA
   <213> homo sapiens;
<400> 389
   gcatccccga ccagccccaa ggtcttcccg ctgagcctcg acagcacccc ccaagatggg 60
   aacgtggtcg tcgcatgcct ggtccagggc ttcttccccc aggagccact cagtgtgacc 120
   tggagcgaaa gcggacagaa cgtgaccgcc agaaacttcc cacctagcca ggatgcctcc 180
   ggggacctgt acaccacgag cagccagctg accctgccgg ccacacagtg cccagacggc 240
   aagtccgtga catgccacgt gaagcactac acgaatccca gccaggatgt gactgtgccc 300
   tgcccaggtc agagggcagg ctggggagtg gggcggggcc accccgtcct gccctgacac 360
   tgcgcctgca cccgtgttcc ccacagggag ccgccccttc actcacacca gagtggaccg 420
   cgggccgagc cccaggaggt ggtggtggac aggccaggag gggcgaggcg ggggcacggg 480
   gaagggcgtt ctgaccagct caggccatct ctccactcca gttcccccac ctcccccatg 540
   ctgccacccc cgactgtcgc tgcaccgacc ggccctcgag gacctgctct taggttcaga 600
   agcgaacctc acgtgcacac tgaccggcct gagagatgcc tctggtgcca ccttcacctg 660
   gacgccctca agtgggaaga gcgctgttca aggaccacct gagcgtgacc tctgtggctg 720
   ctacagcgtg tccagtgtcc tgcctggctg tgcccagcca tggaaccatg gggagacctt 780
   cacctgcact gctgcccacc ccgagttgaa gaccccacta accgccaaca tcacaaaatc 840
   cggtgggtcc agaccctgct cggggccctg ctcagtgctc tggtttgcaa agcatattcc 900
   cggcctgcct cctccctccc aatcctgggc tccagtgctc atgccaagta cagagggaaa 960
   ctgaggcagg ctgaggggcc aggacacagc ccagggtgcc caccagagca gaggggctct 1020
   ctcatcccct gcccagcccc ctgacctggc tctctaccct ccaggaaaca cattccggcc 1080
   cgaggtccac ctgctgccgc cgccgtcgga ggagctggcc ctgaacgagc tggtgacgct 1140
   gacgtgcctg gcacgtggct tcagccccaa ggatgtgctg gttcgctggc tgcaggggtc 1200
   acaggagctg ccccgcgaga agtacctgac ttgggcatcc cggcaggagc ccagccaggg 1260
   caccaccacc ttcgctgtaa ccagcatact gcgcgtggca gctgaggact ggaagaaggg 1320
   ggacaccttc tcctgcatgg tgggccacga ggccctgccg ctggccttca cacagaagac 1380
   catcgaccgc atggcgggta aacccaccca catcaatgtg tctgttgtca tggcggaggt 1440
   ggatggcacc tgctactgag ccgcccgcct gtccccaccc ctgaataaac tccatgctcc 1500
   cccaagc 1507
<210> 390
   <211> 1022
   <212> DNA
   <213> homo sapiens;
<400> 390
   gcatccccga ccagccccaa ggtcttcccg ctgagcctcg acagcacccc ccaagatggg 60
   aacgtggtcg tcgcatgcct ggtccagggc ttcttccccc aggagccact cagtgtgacc 120
   tggagcgaaa gcggacagaa cgtgaccgcc agaaacttcc cacctagcca ggatgcctcc 180
   ggggacctgt acaccacgag cagccagctg accctgccgg ccacacagtg cccagacggc 240
   aagtccgtga catgccacgt gaagcactac acgaatccca gccaggatgt gactgtgccc     300
   tgccagttcc cccacctccc ccatgctgcc acccccgact gtcgctgcac cgaccggccc     360
   tcgaggacct gctcttaggt tcagaagcga acctcacgtg cacactgacc ggcctgagag 420
   atgcctctgg tgccaccttc acctggacgc cctcaagtgg gaagagcgct gttcaaggac 480
   cacctgagcg tgacctctgt ggctgctaca gcgtgtccag tgtcctgcct ggctgtgccc 540
   agccatggaa ccatggggag accttcacct gcactgctgc ccaccccgag ttgaagaccc 600
   cactaaccgc caacatcaca aaatccggaa acacattccg gcccgaggtc cacctgctgc 660
   cgccgccgtc ggaggagctg gccctgaacg agctggtgac gctgacgtgc ctggcacgtg 720
   gcttcagccc caaggatgtg ctggttcgct ggctgcaggg gtcacaggag ctgccccgcg 780
   agaagtacct gacttgggca tcccggcagg agcccagcca gggcaccacc accttcgctg 840
   taaccagcat actgcgcgtg gcagctgagg actggaagaa gggggacacc ttctcctgca 900
   tggtgggcca cgaggccctg ccgctggcct tcacacagaa gaccatcgac cgcatggcgg 960
   gtaaacccac ccacatcaat gtgtctgttg tcatggcgga ggtggatggc acctgctact 1020
   ga 1022
<210> 391
   <211> 1507
   <212> DNA
   <213> homo sapiens;
<400> 391
   gcatccccga ccagccccaa ggtcttcccg ctgagcctcg acagcacccc ccaagatggg 60
   aacgtggtcg tcgcatgcct ggtccagggc ttcttccccc aggagccact cagtgtgacc 120
   tggagcgaaa gcggacagaa cgtgaccgcc agaaacttcc cacctagcca ggatgcctcc 180
   ggggacctgt acaccacgag cagccagctg accctgccgg ccacacagtg cccagacggc 240
   aagtccgtga catgccacgt gaagcactac acgaatccca gccaggatgt gactgtgccc 300
   tgcccaggtc agagggcagg ctggggagtg gggcggggcc accccgtcct gccctgacac 360
   tgcgcctgca cccgtgttcc ccacagggag ccgccccttc actcacacca gagtggaccg 420
   cgggccgagc cccaggaggt ggtggtggac aggccaggag gggcgaggcg ggggcacggg 480
   gaagggcgtt ctgaccagct caggccatct ctccactcca gttcccccac ctcccccatg 540
   ctgccacccc cgactgtcgc tgcaccgacc ggccctcgag gacctgctct taggttcaga 600
   agcgaacctc acgtgcacac tgaccggcct gagagatgcc tctggtgcca ccttcacctg 660
   gacgccctca agtgggaaga gcgctgttca aggaccacct gagcgtgacc tctgtggctg 720
   ctacagcgtg tccagtgtcc tgcctggctg tgcccagcca tggaaccatg gggagacctt 780
   cacctgcact gctgcccacc ccgagttgaa gaccccacta accgccaaca tcacaaaatc 840
   cggtgggtcc agaccctgct cggggccctg ctcagtgctc tggtttgcaa agcatattcc 900
   cggcctgcct cctccctccc aatcctgggc tccagtgctc atgccaagta cagagggaaa 960
   ctgaggcagg ctgaggggcc aggacacagc ccagggtgcc caccagagca gaggggctct 1020
   ctcatcccct gcccagcccc ctgacctggc tctctaccct ccaggaaaca cattccggcc 1080
   cgaggtccac ctgctgccgc cgccgtcgga ggagctggcc ctgaacgagc tggtgacgct 1140
   gacgtgcctg gcacgtggct tcagccccaa ggatgtgctg gttcgctggc tgcaggggtc 1200
   acaggagctg ccccgcgaga agtacctgac ttgggcatcc cggcaggagc ccagccaggg 1260
   caccaccacc tacgctgtaa ccagcatact gcgcgtggca gctgaggact ggaagaaggg 1320
   ggagaccttc tcctgcatgg tgggccacga ggccctgccg ctggccttca cacagaagac 1380
   catcgaccgc atggcgggta aacccaccca catcaatgtg tctgttgtca tggcggaggc 1440
   ggatggcacc tgctactgag ccgcccgcct gtccccaccc ctgaataaac tccatgctcc 1500
   cccaagc 1507
<210> 392
   <211> 1022
   <212> DNA
   <213> homo sapiens;
<400> 392
   gcatccccga ccagccccaa ggtcttcccg ctgagcctcg acagcacccc ccaagatggg 60
   aacgtggtcg tcgcatgcct ggtccagggc ttcttccccc aggagccact cagtgtgacc 120
   tggagcgaaa gcggacagaa cgtgaccgcc agaaacttcc cacctagcca ggatgcctcc 180
   ggggacctgt acaccacgag cagccagctg accctgccgg ccacacagtg cccagacggc 240
   aagtccgtga catgccacgt gaagcactac acgaatccca gccaggatgt gactgtgccc 300
   tgccagttcc cccacctccc ccatgctgcc acccccgact gtcgctgcac cgaccggccc 360
   tcgaggacct gctcttaggt tcagaagcga acctcacgtg cacactgacc ggcctgagag 420
   atgcctctgg tgccaccttc acctggacgc cctcaagtgg gaagagcgct gttcaaggac 480
   cacctgagcg tgacctctgt ggctgctaca gcgtgtccag tgtcctgcct ggctgtgccc 540
   agccatggaa ccatggggag accttcacct gcactgctgc ccaccccgag ttgaagaccc 600
   cactaaccgc caacatcaca aaatccggaa acacattccg gcccgaggtc cacctgctgc 660
   cgccgccgtc ggaggagctg gccctgaacg agctggtgac gctgacgtgc ctggcacgtg 720
   gcttcagccc caaggatgtg ctggttcgct ggctgcaggg gtcacaggag ctgccccgcg 780
   agaagtacct gacttgggca tcccggcagg agcccagcca gggcaccacc acctacgctg 840
   taaccagcat actgcgcgtg gcagctgagg actggaagaa gggggagacc ttctcctgca 900
   tggtgggcca cgaggccctg ccgctggcct tcacacagaa gaccatcgac cgcatggcgg 960
   gtaaacccac ccacatcaat gtgtctgttg tcatggcgga ggcggatggc acctgctact 1020
   ga 1022
<210> 393
   <211> 8912
   <212> DNA
   <213> homo sapiens;
<400> 393
   cacccaccaa ggctccggat gtgttcccca tcatatcagg gtgcagacac ccaaaggata      60
   acagccctgt ggtcctggca tgcttgataa ctgggtacca cccaacgtcc gtgactgtca     120
   cctggtacat ggggacacag agccagcccc agagaacctt ccctgagata caaagacggg     180
   acagctacta catgacaagc agccagctct ccacccccct ccagcagtgg cgccaaggcg      240
   agtacaaatg cgtggtccag cacaccgcca gcaagagtaa gaaggagatc ttccgctggc     300
   caggtaggtc gcaccggaga tcacccagaa gggcccccca ggacccccag caccttccac     360
   tcagggcctg accacaaaga cagaagcaag ggctgggctg tgaggcaacc cccacctccc     420
   cctcagagca cgttcctccc ccttcaccct gtatccaccc ctccggaccc tccccatctc     480
   agtccctccg ctccctctct ctgaggccca tctcccaata cccagatcac tttccttcca     540
   gacccttccc tcagtgtgca cggaggcagc ttgcccagca aaggtgactg tctagtgggc     600
   ttcccacagc caagctccca ccccatgctg cggcccttcc cttcttcctg cttggctgcc     660
   tgtgcccccc acctgcctgt ccacaaccca gcctctggta catccatgcc ctctgccctc     720
   agcctcacct gcacttttcc ttggatttca gagtctccaa aggcacaggc ctcctcagtg     780
   cccactgcac aaccccaagc agagggcagc ctcgccaagg caaccacagc cccagccacc     840
   acccgtaaca caggtgagaa gccccttccc tgcacactcc acccccaccc acctgctcat    900
   tcctcagccg cctcctccag gcagcccttc ataactcctt gtctgagtct ccaagtcaca     960
   ctttggtaag gagagggaca ctgaacggac ctctaacaaa cacctactgc cagccagccc    1020
   cagtctgggg gccagcagat gccaaacagc cagcagactc ccagagcaga cctgggccgg    1080
   ctccctggcc catggaccca gctctgcctc gctgagctga ggcatgggct ctcagcgcag    1140
   cctcacatag agccaccctg ccgaggcagt ccggcttgca gactcacagg tcacttgggc    1200
   cgcagcagcc cctccccgtg accctcgcct cccgcccgcc ccagcctggc tctctccaag    1260
   tgttggatct tggtggccag cctgcttctc accctcaccc tgcctgccac ctcagaatgg    1320
   caggggaaag agggccctca ccaagaactt tatctgagga gtctgaggct tgtgactctg 1380
   acctgcctga gatgtccatg tggccggggg gacgggttca gtgttcggga gaactcgggt 1440
   acgtgcctga ctttctctga gtagggcagg aagctgttag gagaagcagc agtgaggtgg 1500
   gctggaccaa caggcagaat gactgtccct cagccaccct ctgggatgtg ggtcaagctc 1560
   tgacaaaggc atggcacagc catggtggcc cctgcttgga tgagtggcca cggtgccctc 1620
   accctgggcc agaatctgcc tccactctgc aggtgcagaa acacgacatt cccgtctcta 1680
   aacacaccta gctcctaggc ttggggtggg cctatcaaat gcagggagat ggacacagca 1740
   caagggccag agcttcccat gagaaaggtg agggcagctg ctccctgacc cgggcatctg 1800
   cacttgtccc tctccaccct cctcatgggc agtggagact cagcaacaaa acaagttgag 1860
   tgcattagca gccagctctg gagccaagtc actcacccca cggccttggc tgctggtgga 1920
   ggggccttcc cctgggcagc ctccaagaag acggccaagt gctcttactc agaccacggc 1980
   gctgcttcct ggcacctcga tttcccacaa caacatgggg tgcagacagg ctagggcccc 2040
   ctgccctggg gcctggacgg catccagtta aagatgaccc ttcacgggcg gtgcctgagg 2100
   tgtgctgacc tcagcagcta agccctcagg tctggtctgc actgccccac ctggaggacc 2160
   caactgaccc agacacagcc agggttatgg catgaccccg tggacggtga cccacaggcc 2220
   agatgcagcc aggggctgtt ttgtgtggcc tagaaatgtc tttacagttg tagtgggatg 2280
   gaggaggaag aggaagagag gaggggagag aaaagcaggg aaggggaaaa agaggagttc 2340
   aatgcaaccc caaaagccag aacagttttg agctgaaaga acaaggcagg aaacatccca 2400
   gtacctgact tcaaaacata ctataaagca gttgtaatca aaacaggatc ataaaaacag 2460
   acacacagac ccatggaaca gaaaagcgag cccagaaata aatctacatg cttgcagtcc 2520
   attgattttc aacaaaggca ccaggaaaac acaatgggga gaggacagtt tcctcaataa 2580
   atagtgctgg ggaaactgga tatccatgtg cagactaatg aaactacaca aaaatcaatt 2640
   gaaaacagtc taggccaggc gcggtggctc atgccggtaa tcccagcact ttgggaggcc 2700
   gagacaggcg gatcacctga ggtcaggagt tcgagaccag cttggccaac atggcgaaac 2760
   ccggtctcca ctaaaaatac aaaaattagc acatggtggc ctacgtctgt tatcccagct 2820
   tttcaggagg ctgaggcagg agaatcgctt gaatccggga ggtgaaggtt gcagggagcc 2880
   aagattgcgc cactgcattc cagcctgggc aatggagcga gactgtctca aaaaaaaaaa 2940
   aaaaaagaaa agaaaacagt ctaaaggttt aactgaacag ataaagctac tagaagaaaa 3000
   cataggggga aaactccatg acattagtct gagcaacgat ttttggatat gatcccaaaa 3060
   gctcaggcag cactagtcac aaaagccaag atacagaacc aacctaagca cccctcagca 3120
   gatgcacagg taaagaaaat gtggtacgta tggggcacaa tggaatacga ttcagccttt 3180
   aaaaacagtg aaattctgtc attggcaaca atgtagatga acctgaagga cacttatgct 3240
   aagtgaaata agccaggcac agaaggagca atactgcatg attgcactta catctggcag 3300
   gttaaaaagg caaactctta gaggcagaca gtagagaggt ggtgccaggg agcgggcact 3360
   ggtggctggg gagatgttgg tcaaagggca caaaactgca gttgggagga attagttcag 3420
   gacatccctt gtacatgggg acagtggtta gtaacaacgg attgtatcct tgaaaaccgc 3480
   taagaaaata gtttttaagt gttcttgaca caaaaagtga cacgtatgtg agatactgca 3540
   tggtcattag ctggatttag ccattccaca atgtacacat atttcaaaca ttgtgttgta 3600
   tatgataaac atgtataatt tttgtcaatt aaaaattttt aggaagagga ggagaagaga 3660
   agaagaagga gaaggagaaa gaggaacaag aagagagaga gacaaagaca ccaggttttt 3720
   tctgacccct gggctatcaa aacacctatt gcccaataac tagttggccg ttggtgccct 3780
   aaactattga agcgattgct gttatgtgga tgggccccgg acacttagaa actcgtgacc 3840
   cctgaggacc cccacgagga cagtcagggt ccccccgaac tcagggagca ctgaggaagg 3900
   agctcttaga ggcgtggggc ccctcaggcc cctcagaggg ctctgccaca tgggtcaggg 3960
   gcaggctgag ggggagtccc aggctccatg cccagcctct gtgcctctga ccagggtgtc 4020
   ccccacaccg cctcctcccc agtgccctcc actggccaca cctggccaga agctggggag 4080
   aggagagcac agtggttaag tcagtccctg cagggagacg gcaccagaaa aacctggcct 4140
   gtggatgagt cccggcctgg cagccacaga gcagagagct ctagaagcaa cgaaggcccg 4200
   agtctgctca gggaagagcg ggcagcagcc ccagggccgg acagtgacca agagtggcac 4260
   cgcccatggc tcaacgggtc tttgcccaca gatcccccag cccctggaga cagggtctgt 4320
   gtgcctggcc gtgcaggcag gcaccacact cagggggagg ccactgtgga gctctgtgca 4380
   gagccccggg cgggagccta ctgctcccga aggtccggcc acagctgctc tcgtttgctc 4440
   tcccctgcag agtgtccgag ccacacccag cctcttggcg tctacctgct aacccctgca 4500
   gtgcaggacc tgtggctccg ggacaaagcc accttcacct gcttcgtggt gggcagtgac 4560
   ctgaaggatg ctcacctgac ctgggaggtg gccgggaagg tccccacagg gggcgtggag 4620
   gaagggctgc tggagcggca cagcaacggc tcccagagcc agcacagccg tctgaccctg 4680
   cccaggtcct tgtggaacgc ggggacctcc gtcacctgca cactgaacca tcccagcctc 4740
   ccaccccaga ggttgatggc gctgagagaa cccggtgagc ctggctccca ggtggggaga 4800
   cgagggtgcc cacagcctgc tgacccctac gcctgcccca gggccatgac cccagctggg 4860
   ccccagcagc accggtcatc ctccacagga aaggagaagg gaggcaccag caccctggcc 4920
   ggccccactt ctctcccagt gcccccgtgg ccagaggctg acagcctccc ccacctcccc 4980
   gcagctgcgc aggcacccgt caagctttcc ctgaacctgc tggcctcgtc tgaccctccc 5040
   gaggcggcct cgtggctcct gtgtgaggtg tctggcttct cgccccccaa catcctcctg 5100
   atgtggctgg aggaccagcg tgaggtgaac acttctgggt ttgcccccgc acgcccccct 5160
   ccacagcccg ggagcaccac gttctgggcc tggagtgtgc tgcgtgtccc agccccgccc 5220
   agccctcagc cagccaccta cacgtgtgtg gtcagccacg aggactcccg gactctgctc 5280
   aacgccagcc ggagcctaga agtcagctgt gagtcacccc caggcccagg gttgggacgg 5340
   ggactctgag gggggccata aggagctgga atccatacta ggcaggggtg ggcactgggc 5400
   aggggcgggg ctaggctgtc ctgggcacac aggccccttc tcggtgtccg gcaggagcac 5460
   agacttccca gtactcctgg gccatggatg tcccagcgtc catccttgct gtccacacca 5520
   cgtgctggcc caggctggct ggcacagtgt aagaggtgga tacaacccct cgccgtgccc 5580
   tgaggagtgg cggtttcctc ccaagacatt ccccacggct gggtgctggg cacaggcctt 5640
   ccctggtgtg accgtgaatg tggtcaccct gaacagctgc cctctctggg gacatctgac 5700
   tgtccaagac cacagtcagc acctctggga gccagagggg tctccagaga cccccagatg 5760
   tcaggcttgg gctcagtgcc cagcgaaagg tcagccccac acatgcccat aatgggcgcc 5820
   cacccagagt gacagccccc agcctcctgc caggcccacc cttttccgcc cccttgaggc 5880
   atggcacaca gaccagtgcg cccactgccc gagcatggcc ccagtgggat gtggtggcca 5940
   cgaggggctg tacacacagc aggaggctgt ccgccctgct cagggcctgc tgcctatgcc 6000
   ccagctgtcc aaccaaggga ggcatggaag ggcccctggt gtaagctgga gccaggcacc 6060
   caggcccccg gccaccctgc agagccaagg aaaggaagac acccaagtca acaaggggca 6120
   gggctgaggg ctgtcccagg ctcttttggc ccgaggggct gccagcagcc ctgacccggc 6180
   atgggccttc cccaaaagcg accctgtgag gtggcctcac agagaacccc ctctgaggac 6240
   agtgtctgac cctgcctgcc tcacacagat gggccccaca gcagtgggca acctgggggg 6300
   cagcagccca acctgaccct gcagggactg ccccctgcag cagcagctgc ttctcagtcc 6360
   cccaacctcc ctgtccccgc cagagggtct tccccgaagc tgcagcccca acccatggct 6420
   gcccacctgg aaccgggact ccctgtccac tgccccctcc ccttcggggc cccatctgtg 6480
   ctggggccca ggttcggcct acagattccc atcattgcca tggcctcctg accttgccta 6540
   tccaccccca accaccggct ccatgctgac cctcccccag gctcccacgc ccagctggcc 6600
   ggccatcccc aggcacagac agtctgggat ctcacaggtt agcctggacc atccacctgg 6660
   ccagacctgg gagaggctgg aagctgccct gccaccatgc tccagggccc caggttgcag 6720
   tactatgggg tgagggtgtg tgtgcacacc tgtgtgtacc taggatatcc gagtgtaccc 6780
   ttgtgccccc aagcacaagt ctccctccca ggcagtgagg cccagatggt gcagtggtta 6840
   gagctgaggc ttatcccaca gagaaccctg gcgccttggt caaggaagcc cctatgcctt 6900
   tcttgcctcg atttcccctc ttgtctgctg agccagcagg ggccacgtcc tgggctgctg 6960
   tgaggaggaa gcaagttggt gctaggaggg gctcctgtgt gtgcatgggc gggaggggtg 7020
   caggtatctg agcaccccgg tctccacttg agagagcagg gcaggagctc cctgacccac 7080
   ccagactaca cacgctgtgt ccacgtgtct cccattatct gtggcagagg atccggcttc 7140
   tttctcaatt tccagttctt cacaaagcaa tgcctttgta aaatgcaata agaaatacta 7200
   gaaaaatgat atgaacagaa agacacgccg attttttgtt attagatgta acagaccatg 7260
   gccccatgaa atgatcccgg accagatccg tccacacccg ccactcagca gctctggccg 7320
   agctcacagt acaaccacaa taaactcttg ttgaatgaac tctaggaagt ctgtgacgtg 7380
   gctggttctt gtcaatgctt cctgcctgcc cacaggctct tcctcgtgga tggggctgtg 7440
   cttgccacgg aagcgcgttt ttcccggcct aggcttgcct tgggccccac tgccgtctcc 7500
   agctggagat gaccttctat acacacattt gctcatgaca gacccttgct tagccccctt 7560
   ccatggctcc ctcctgctgc tgggataaaa tcaccttgcc tggatatccc ctcctgggcc 7620
   cctttccacc ctccttagtc agcaccccca gttcagggca cctgctttcc ccgctgcgga 7680
   gaagccactc tctccttgct gcccggctgt gtcttgcctt ccacaccttg tcacagtggc 7740
   cacttcctaa ggaaggcctc cctgtgtgca ggtgtgcaga agtgccccag cctcccgtca 7800
   cctttgtcac gggagcccaa tccatgagag tctatggttc tgtctgtctg ccccactcag 7860
   ggcagcgaca agtccaggcg gggaggacac agtaggcaga gatttgtcga ggggacatat 7920
   gagcaagagg gtgaggctgg gagctccctg gagataacca cgcctcctgg gaagactcgc 7980
   cgtcatttca gctccacgct gtgcgggggt gggtggaggg gtagcctggc cctcatgacc 8040
   agggagcttc tcactcagcc cctgttcctc cccagacctg gccatgaccc ccctgatccc 8100
   tcagagcaag gatgagaaca gcgatgacta cacgaccttt gatgatgtgg gcagcctgtg 8160
   gaccgccctg tccacgtttg tggccctctt catcctcacc ctcctctaca gcggcattgt 8220
   cactttcatc aaggtcaggg gagcggccag gctctcagtg accctcgggg tgggtgtggg 8280
   gcaaggtgcc cttccagggg acatgccaga gctggtccag ggatcctgga ccaggcagag 8340
   gcagggctga gggagcctgg aggacatgca ggccctctgt ggcctgtgga cactgtcgaa 8400
   ggccctcttg accctgtgga taaaggacaa caccccctcc cctgctcctc tgtctcccct 8460
   gcccctccac ccctcaggct tctagccccc tgtctgaccc caggggctgt ctttcaggtg 8520
   aagtagcccc agaagagcag gacgccctgt acctgcagag aagggaagca gcctctgtac 8580
   ctcatctgtg gctaccagag agcagaaagg acccaccctg gactcttctg tgtgcaggaa 8640
   gatgcgccag cccctgcccc cggctcccct ctgtccgcca cagaatccag tcttctagac 8700
   cagggggacg ggcacccatc actccgcagg cgaatcagag cccccctgcc ccggccctaa 8760
   cccctgtgcc tccttcccgt gcttccccca gagccagcta cacccctgcc ccggccctaa 8820
   cccccatgcc tccttcctgt gcttccccca gagccagcta gtcccacctg cagcccgctg 8880
   gcctccccat aaacacgctt tggttcattt ca 8912
<210> 394
   <211> 1293
   <212> DNA
   <213> homo sapiens;
<220>
   <221> misc_feature
   <222> (1)..(1)
   <223> n is a, c, g, or t
<400> 394
   ncacccacca aggctccgga tgtgttcccc atcatatcag ggtgcagaca cccaaaggat 60
   aacagccctg tggtcctggc atgcttgata actgggtacc acccaacgtc cgtgactgtc 120
   acctggtaca tggggacaca gagccagccc cagagaacct tccctgagat acaaagacgg 180
   gacagctact acatgacaag cagccagctc tccacccccc tccagcagtg gcgccaaggc 240
   gagtacaaat gcgtggtcca gcacaccgcc agcaagagta agaaggagat cttccgctgg 300
   ccagagtctc caaaggcaca ggcctcctca gtgcccactg cacaacccca agcagagggc 360
   agcctcgcca aggcaaccac agccccagcc accacccgta acacaggaag aggaggagaa 420
   gagaagaaga aggagaagga gaaagaggaa caagaagaga gagagacaaa gacaccagag 480
   tgtccgagcc acacccagcc tcttggcgtc tacctgctaa cccctgcagt gcaggacctg 540
   tggctccggg acaaagccac cttcacctgc ttcgtggtgg gcagtgacct gaaggatgct 600
   cacctgacct gggaggtggc cgggaaggtc cccacagggg gcgtggagga agggctgctg 660
   gagcggcaca gcaacggctc ccagagccag cacagccgtc tgaccctgcc caggtccttg 720
   tggaacgcgg ggacctccgt cacctgcaca ctgaaccatc ccagcctccc accccagagg 780
   ttgatggcgc tgagagaacc cgctgcgcag gcacccgtca agctttccct gaacctgctg 840
   gcctcgtctg accctcccga ggcggcctcg tggctcctgt gtgaggtgtc tggcttctcg 900
   ccccccaaca tcctcctgat gtggctggag gaccagcgtg aggtgaacac ttctgggttt 960
   gcccccgcac gcccccctcc acagcccggg agcaccacgt tctgggcctg gagtgtgctg 1020
   cgtgtcccag ccccgcccag ccctcagcca gccacctaca cgtgtgtggt cagccacgag 1080
   gactcccgga ctctgctcaa cgccagccgg agcctagaag tcagctacct ggccatgacc 1140
   cccctgatcc ctcagagcaa ggatgagaac agcgatgact acacgacctt tgatgatgtg 1200
   ggcagcctgt ggaccgccct gtccacgttt gtggccctct tcatcctcac cctcctctac 1260
   agcggcattg tcactttcat caaggtgaag tag 1293
<210> 395
   <211> 3842
   <212> DNA
   <213> homo sapiens;
<400> 395
   gcctccacac agagcccatc cgtcttcccc ttgacccgct gctgcaaaaa cattccctcc 60
   aatgccacct ccgtgactct gggctgcctg gccacgggct acttcccgga gccggtgatg 120
   gtgacctggg acacaggctc cctcaacggg acaactatga ccttaccagc caccaccctc 180
   acgctctctg gtcactatgc caccatcagc ttgctgaccg tctcgggtgc gtgggccaag 240
   cagatgttca cctgccgtgt ggcacacact ccatcgtcca cagactgggt cgacaacaaa 300
   accttcagcg gtaagagagg gccaagctca gagaccacag ttcccaggag tgccaggctg 360
   agggctggca gagtgggcag gggttgaggg ggtgggtggg ctcaaacgtg ggaacaccca 420
   gcatgcctgg ggacccgggc caggacgcgg gggcaagagg agggcacaca gagctcagag 480
   aggccaacaa ccctcatgac caccagctct cccccagtct gctccaggga cttcaccccg 540
   cccaccgtga agatcttaca gtcgtcctgc gacggcggcg ggcacttccc cccgaccatc 600
   cagctcctgt gcctcgtctc tgggtacacc ccagggacta tcaacatcac ctggctggag 660
   gacgggcagg tcatggacgt ggacttgtcc accgcctcta ccacgcagga gggtgagctg 720
   gcctccacac aaagcgagct caccctcagc cagaagcact ggctgtcaga ccgcacctac 780
   acctgccagg tcacctatca aggtcacacc tttgaggaca gcaccaagaa gtgtgcaggt 840
   acgttcccac ctgccctggt ggccgccacg gaggccagag aagaggggcg ggtgggcctc 900
   acacagccct ccggtgtacc acagattcca acccgagagg ggtgagcgcc tacctaagcc 960
   ggcccagccc gttcgacctg ttcatccgca agtcgcccac gatcacctgt ctggtggtgg 1020
   acctggcacc cagcaagggg accgtgaacc tgacctggtc ccgggccagt gggaagcctg 1080
   tgaaccactc caccagaaag gaggagaagc agcgcaatgg cacgttaacc gtcacgtcca 1140
   ccctgccggt gggcacccga gactggatcg agggggagac ctaccagtgc agggtgaccc 1200
   acccccacct gcccagggcc ctcatgcggt ccacgaccaa gaccagcggt gagccatggg 1260
   caggccgggg tcgtggggga agggagggag cgagtgagcg gggcccgggc tgaccccacg 1320
   tctggccaca ggcccgcgtg ctgccccgga agtctatgcg tttgcgacgc cggagtggcc 1380
   ggggagccgg gacaagcgca ccctcgcctg cctgatccag aacttcatgc ctgaggacat 1440
   ctcggtgcag tggctgcaca acgaggtgca gctcccggac gcccggcaca gcacgacgca 1500
   gccccgcaag accaagggct ccggcttctt cgtcttcagc cgcctggagg tgaccagggc 1560
   cgaatgggag cagaaagatg agttcatctg ccgtgcagtc catgaggcag caagcccctc 1620
   acagaccgtc cagcgagcgg tgtctgtaaa tcccggtaaa tgacgtactc ctgcctccct 1680
   ccctcccagg gctccatcca gctgtgcagt ggggaggact ggccagacct tctgtccact 1740
   gttgcaatga ccccaggaag ctacccccaa taaactgtgc ctgctcagag ccccaggtac 1800
   acccattctt gggagcgggc agggctgtgg gcaggtgcat cttggcacag aggaatgggc 1860
   cccccaggag gggcagtggg aggaggtggg cagggctgag tccccccagg agaggcggtg 1920
   ggaggaggtg ggcagggctg aggtgccact catccatctg ccttcgtgtc agggttattt 1980
   gtcaaacagc atatctgcag ggactcatca cagctacccc gggccctctc tgcccccact 2040
   ctgggtctac cccctccaag gagtccaaag acccagggga ggtcctcagg gaaggggcaa 2100
   gggagccccc acagccctct ctcttggggg cttggcttct acccccctgg acaggagccc 2160
   ctgcaccccc aggtatagat gggcacacag gcccctccag gtggaaaaac agccctaagt 2220
   gaaaccccca cacagacaca cacgacccga cagccctcgc ccaagtctgt gccactggcg 2280
   ttcgcctctc tgccctgtcc cgccttgccg agtcctggcc ccagcaccgg ggccggtgga 2340
   gccgagccca ctcacacccc gcagcctccg ccaccctgcc ctgtgggcac accaggccca 2400
   ggtcagcagc caggccccct ctcctactgc cccccaccgc cccttggtcc atcctgaatc 2460
   ggcccccagg ggatcgccag cctcacacac ccagtctcgc ccactcacgc ctcactcaag 2520
   gcacagctgt gcacacacta ggccccatag caactccaca gcaccctgta ccaccaccag 2580
   ggcgccatag acaccccaca cgtggtcaca cgtggcccac actccgcctc tcacgctgcc 2640
   tccagcgagg ctactgccaa gcccttcctc tgagccatac ctgggccgct ggatcccaga 2700
   gagaaatgga gaggccctca cgtggtgtcc tccagtccaa ccctccctgt caccctgtca 2760
   gcagcagcac cccacagcca aacacaggat ggatgcgtgg gctccatccc ccactcaccc 2820
   acaccggaac cccagagcag gctacgtgcc cctcacagac ctcaaaccca catgtgcatc 2880
   tgacacccca gatccaaacg ctccccccgg tcatgcacac caagggcaca gcacccacca 2940
   aatccacacg gaaacacggg caccgggcac cccatgagca caaagcccct ccatgtctga 3000
   agacagtccc tgcacaccgt cacagccata cattcagctt cactctcacg tcccagccca 3060
   cctgcaccca gctctgggcc tggagcagca gaaagaggtg tgagggcccg aggcgggacc 3120
   tgcacctgct gatgacccgg gaccagcagg cagctcacgg tgttggggaa gggagtggag 3180
   ggcacccagg gcaggagcca gagggaccag gctggtgggc ggggccgggc cggggtaggg 3240
   ccaggaggca gctctggaca cccacaggcc tgggctcata gtccacacca ggacagcccc 3300
   tcagagcacc catgcagtga gtcccaggtc ttgggagcca ggccgcagag ctcacgcatc 3360
   cttccgaggg ccctgagtga ggcggccact gctgtgccga ggggttgggt ccttctctgg 3420
   ggagggcgtg gggtctagag aggcggagtg gaggtaacca gaggtcagga gagaagccgt 3480
   aaggaacaga gggaaaatgg ggccagagtc ggggcgcagg gacgagaggt caggagtggt 3540
   cggcctggct ctgggccgtt gactgactcg ggacctgggt gcccaccctc agggctggct 3600
   ggcggctccg cgcagtccca gagggccccg gatagggtgc tctgccactc cggacagcag 3660
   cagggactgc cgagagcagc aggaggctct gtcccccacc cccgctgcca ctgtggagcc 3720
   gggagggctg actggccagg tcccccagag ctggacgtgt gcgtggagga ggccgagggc 3780
   gaggcgccgt ggacgtggac cggcctctgc atcttcgccg cactcttcct gctcagcgtg 3840
   ag 3842
<210> 396
   <211> 1287
   <212> DNA
   <213> homo sapiens;
<400> 396
   gcctccacac agagcccatc cgtcttcccc ttgacccgct gctgcaaaaa cattccctcc 60
   aatgccacct ccgtgactct gggctgcctg gccacgggct acttcccgga gccggtgatg 120
   gtgacctggg acacaggctc cctcaacggg acaactatga ccttaccagc caccaccctc 180
   acgctctctg gtcactatgc caccatcagc ttgctgaccg tctcgggtgc gtgggccaag 240
   cagatgttca cctgccgtgt ggcacacact ccatcgtcca cagactgggt cgacaacaaa 300
   accttcagcg tctgctccag ggacttcacc ccgcccaccg tgaagatctt acagtcgtcc 360
   tgcgacggcg gcgggcactt ccccccgacc atccagctcc tgtgcctcgt ctctgggtac 420
   accccaggga ctatcaacat cacctggctg gaggacgggc aggtcatgga cgtggacttg 480
   tccaccgcct ctaccacgca ggagggtgag ctggcctcca cacaaagcga gctcaccctc 540
   agccagaagc actggctgtc agaccgcacc tacacctgcc aggtcaccta tcaaggtcac 600
   acctttgagg acagcaccaa gaagtgtgca gattccaacc cgagaggggt gagcgcctac 660
   ctaagccggc ccagcccgtt cgacctgttc atccgcaagt cgcccacgat cacctgtctg 720
   gtggtggacc tggcacccag caaggggacc gtgaacctga cctggtcccg ggccagtggg 780
   aagcctgtga accactccac cagaaaggag gagaagcagc gcaatggcac gttaaccgtc 840
   acgtccaccc tgccggtggg cacccgagac tggatcgagg gggagaccta ccagtgcagg 900
   gtgacccacc cccacctgcc cagggccctc atgcggtcca cgaccaagac cagcggcccg 960
   cgtgctgccc cggaagtcta tgcgtttgcg acgccggagt ggccggggag ccgggacaag 1020
   cgcaccctcg cctgcctgat ccagaacttc atgcctgagg acatctcggt gcagtggctg 1080
   cacaacgagg tgcagctccc ggacgcccgg cacagcacga cgcagccccg caagaccaag 1140
   ggctccggct tcttcgtctt cagccgcctg gaggtgacca gggccgaatg ggagcagaaa 1200
   gatgagttca tctgccgtgc agtccatgag gcagcaagcc cctcacagac cgtccagcga 1260
   gcggtgtctg taaatcccgg taaatga 1287
<210> 397
   <211> 30
   <212> DNA
   <213> homo sapiens;
<400> 397
   tccggcttct tcgtcttcag ccgcctggag 30
<210> 398
   <211> 228
   <212> DNA
   <213> homo sapiens;
<400> 398
   tccggcttct tcgtcttcag ccgcctggag gtgaccaggg ccgaatggga gcagaaagat 60
   gagttcatct gccgtgcagt ccatgaggca gcaagcccct cacagaccgt ccagcgagcg 120
   gtgtctgtaa atcccgagct ggacgtgtgc gtggaggagg ccgagggcga ggcgccgtgg 180
   acgtggaccg gcctctgcat cttcgccgca ctcttcctgc tcagcgtg 228
<210> 399
   <211> 1975
   <212> DNA
   <213> homo sapiens;
<400> 399
   gggagtgcat ccgccccaac ccttttcccc ctcgtctcct gtgagaattc cccgtcggat 60
   acgagcagcg tggccgttgg ctgcctcgca caggacttcc ttcccgactc catcactttc 120
   tcctggaaat acaagaacaa ctctgacatc agcagcaccc ggggcttccc atcagtcctg 180
   agagggggca agtacgcagc cacctcacag gtgctgctgc cttccaagga cgtcatgcag 240
   ggcacagacg aacacgtggt gtgcaaagtc cagcacccca acggcaacaa agaaaagaac 300
   gtgcctcttc caggtgaggg ccgggcccag ccaccgggac agagagggag ccgaaggggg 360
   cgggagtggc gggcaccggg ctgacacgtg tccctcactg cagtgattgc cgagctgcct 420
   cccaaagtga gcgtcttcgt cccaccccgc gacggcttct tcggcaaccc ccgcaagtcc 480
   aagctcatct gccaggccac gggtttcagt ccccggcaga ttcaggtgtc ctggctgcgc 540
   gaggggaagc aggtggggtc tggcgtcacc acggaccagg tgcaggctga ggccaaagag 600
   tctgggccca cgacctacaa ggtgaccagc acactgacca tcaaagagag cgactggctc 660
   agccagagca tgttcacctg ccgcgtggat cacaggggcc tgaccttcca gcagaatgcg 720
   tcctccatgt gtggccccgg tgagtgacct gtccccaggg gcagcaccca ccgacacaca 780
   ggggtccact cgggtctggc attcgccacc ccggatgcag ccatctactc cctgagcctt 840
   ggcttcccag agcggccaag ggcaggggct cgggcggcag gacccctggg ctcggcagag 900
   gcagttgcta ctctttgggt gggaaccatg cctccgccca catccacacc tgccccacct 960
   ctgactccct tctcttgact ccagatcaag acacagccat ccgggtcttc gccatccccc 1020
   catcctttgc cagcatcttc ctcaccaagt ccaccaagtt gacctgcctg gtcacagacc 1080
   tgaccaccta tgacagcgtg accatctcct ggacccgcca gaatggcgaa gctgtgaaaa      1140
   cccacaccaa catctccgag agccacccca atgccacttt cagcgccgtg ggtgaggcca      1200
   gcatctgcga ggatgactgg aattccgggg agaggttcac gtgcaccgtg acccacacag      1260
   acctgccctc gccactgaag cagaccatct cccggcccaa gggtaggccc cactcttgcc      1320
   cctcttcctg cactccctgg gacctccctt ggcctctggg gcatggtgga aagcacccct      1380
   cactcccccg ttgtctgggc aactggggaa aaggggactc aaccccagcc cacaggctgg      1440
   tccccccact gccccgccct caccaccatc tctgttcaca ggggtggccc tgcacaggcc      1500
   cgatgtctac ttgctgccac cagcccggga gcagctgaac ctgcgggagt cggccaccat      1560
   cacgtgcctg gtgacgggct tctctcccgc ggacgtcttc gtgcagtgga tgcagagggg      1620
   gcagcccttg tccccggaga agtatgtgac cagcgcccca atgcctgagc cccaggcccc      1680
   aggccggtac ttcgcccaca gcatcctgac cgtgtccgaa gaggaatgga acacggggga      1740
   gacctacacc tgcgtggtgg cccatgaggc cctgcccaac agggtcaccg agaggaccgt      1800
   ggacaagtcc accggtaaac ccaccctgta caacgtgtcc ctggtcatgt ccgacacagc      1860
   tggcacctgc tactgaccct gctggcctgc ccacaggctc ggggcggctg gccgctctgt      1920
   gtgtgcatgc aaactaaccg tgtcaacggg gtgagatgtt gcatcttata aaatt           1975
<210> 400
   <211> 1362
   <212> DNA
   <213> homo sapiens;
<400> 400
   gggagtgcat ccgccccaac ccttttcccc ctcgtctcct gtgagaattc cccgtcggat      60
   acgagcagcg tggccgttgg ctgcctcgca caggacttcc ttcccgactc catcactttc     120
   tcctggaaat acaagaacaa ctctgacatc agcagcaccc ggggcttccc atcagtcctg     180
   agagggggca agtacgcagc cacctcacag gtgctgctgc cttccaagga cgtcatgcag     240
   ggcacagacg aacacgtggt gtgcaaagtc cagcacccca acggcaacaa agaaaagaac     300
   gtgcctcttc cagtgattgc cgagctgcct cccaaagtga gcgtcttcgt cccaccccgc     360
   gacggcttct tcggcaaccc ccgcaagtcc aagctcatct gccaggccac gggtttcagt     420
   ccccggcaga ttcaggtgtc ctggctgcgc gaggggaagc aggtggggtc tggcgtcacc     480
   acggaccagg tgcaggctga ggccaaagag tctgggccca cgacctacaa ggtgaccagc     540
   acactgacca tcaaagagag cgactggctc agccagagca tgttcacctg ccgcgtggat     600
   cacaggggcc tgaccttcca gcagaatgcg tcctccatgt gtggccccga tcaagacaca     660
   gccatccggg tcttcgccat ccccccatcc tttgccagca tcttcctcac caagtccacc     720
   aagttgacct gcctggtcac agacctgacc acctatgaca gcgtgaccat ctcctggacc     780
   cgccagaatg gcgaagctgt gaaaacccac accaacatct ccgagagcca ccccaatgcc     840
   actttcagcg ccgtgggtga ggccagcatc tgcgaggatg actggaattc cggggagagg     900
   ttcacgtgca ccgtgaccca cacagacctg ccctcgccac tgaagcagac catctcccgg     960
   cccaaggggg tggccctgca caggcccgat gtctacttgc tgccaccagc ccgggagcag    1020
   ctgaacctgc gggagtcggc caccatcacg tgcctggtga cgggcttctc tcccgcggac    1080
   gtcttcgtgc agtggatgca gagggggcag cccttgtccc cggagaagta tgtgaccagc    1140
   gccccaatgc ctgagcccca ggccccaggc cggtacttcg cccacagcat cctgaccgtg    1200
   tccgaagagg aatggaacac gggggagacc tacacctgcg tggtggccca tgaggccctg    1260
   cccaacaggg tcaccgagag gaccgtggac aagtccaccg gtaaacccac cctgtacaac    1320
   gtgtccctgg tcatgtccga cacagctggc acctgctact ga                       1362
<210> 401
   <211> 1975
   <212> DNA
   <213> homo sapiens;
<400> 401
   gggagtgcat ccgccccaac ccttttcccc ctcgtctcct gtgagaattc cccgtcggat 60
   acgagcagcg tggccgttgg ctgcctcgca caggacttcc ttcccgactc catcactttc 120
   tcctggaaat acaagaacaa ctctgacatc agcagcaccc ggggcttccc atcagtcctg 180
   agagggggca agtacgcagc cacctcacag gtgctgctgc cttccaagga cgtcatgcag 240
   ggcacagacg aacacgtggt gtgcaaagtc cagcacccca acggcaacaa agaaaagaac 300
   gtgcctcttc caggtgaggg ccgggcccag ccaccgggac agagagggag ccgaaggggg 360
   cgggagtggc gggcaccggg ctgacacgtg tccctcactg cagtgattgc cgagctgcct 420
   cccaaagtga gcgtcttcgt cccaccccgc gacggcttct tcggcaaccc ccgcaagtcc 480
   aagctcatct gccaggccac gggtttcagt ccccggcaga ttcaggtgtc ctggctgcgc 540
   gaggggaagc aggtggggtc tggcgtcacc acggaccagg tgcaggctga ggccaaagag 600
   tctgggccca cgacctacaa ggtgaccagc acactgacca tcaaagagag cgactggctc 660
   agccagagca tgttcacctg ccgcgtggat cacaggggcc tgaccttcca gcagaatgcg 720
   tcctccatgt gtgtccccgg tgagtgacct gtccccaggg gcagcaccca ccgacacaca 780
   ggggtccact cgggtctggc attcgccacc ccggatgcag ccatctactc cctgagcctt 840
   ggcttcccag agcggccaag ggcaggggct cgggcggcag gacccctggg ctcggcagag 900
   gcagttgcta ctctttgggt gggaaccatg cctccgccca catccacacc tgccccacct 960
   ctgactccct tctcttgact ccagatcaag acacagccat ccgggtcttc gccatccccc 1020
   catcctttgc cagcatcttc ctcaccaagt ccaccaagtt gacctgcctg gtcacagacc 1080
   tgaccaccta tgacagcgtg accatctcct ggacccgcca gaatggcgaa gctgtgaaaa 1140
   cccacaccaa catctccgag agccacccca atgccacttt cagcgccgtg ggtgaggcca 1200
   gcatctgcga ggatgactgg aattccgggg agaggttcac gtgcaccgtg acccacacag 1260
   acctgccctc gccactgaag cagaccatct cccggcccaa gggtaggccc cactcttgcc 1320
   cctcttcctg cactccctgg gacctccctt ggcctctggg gcatggtgga aagcacccct 1380
   cactcccccg ttgtctgggc aactggggaa aaggggactc aaccccagcc cacaggctgg 1440
   tccccccact gccccgccct caccaccatc tctgttcaca ggggtggccc tgcacaggcc 1500
   cgatgtctac ttgctgccac cagcccggga gcagctgaac ctgcgggagt cggccaccat 1560
   cacgtgcctg gtgacgggct tctctcccgc ggacgtcttc gtgcagtgga tgcagagggg 1620
   gcagcccttg tccccggaga agtatgtgac cagcgcccca atgcctgagc cccaggcccc 1680
   aggccggtac ttcgcccaca gcatcctgac cgtgtccgaa gaggaatgga acacggggga 1740
   gacctacacc tgcgtggtgg cccatgaggc cctgcccaac agggtcaccg agaggaccgt 1800
   ggacaagtcc accggtaaac ccaccctgta caacgtgtcc ctggtcatgt ccgacacagc 1860
   tggcacctgc tactgaccct gctggcctgc ccacaggctc ggggcggctg gccgctctgt 1920
   gtgtgcatgc aaactaaccg tgtcaacggg gtgagatgtt gcatcttata aaatt 1975
<210> 402
   <211> 1362
   <212> DNA
   <213> homo sapiens;
<400> 402
   gggagtgcat ccgccccaac ccttttcccc ctcgtctcct gtgagaattc cccgtcggat 60
   acgagcagcg tggccgttgg ctgcctcgca caggacttcc ttcccgactc catcactttc 120
   tcctggaaat acaagaacaa ctctgacatc agcagcaccc ggggcttccc atcagtcctg 180
   agagggggca agtacgcagc cacctcacag gtgctgctgc cttccaagga cgtcatgcag 240
   ggcacagacg aacacgtggt gtgcaaagtc cagcacccca acggcaacaa agaaaagaac 300
   gtgcctcttc cagtgattgc cgagctgcct cccaaagtga gcgtcttcgt cccaccccgc 360
   gacggcttct tcggcaaccc ccgcaagtcc aagctcatct gccaggccac gggtttcagt 420
   ccccggcaga ttcaggtgtc ctggctgcgc gaggggaagc aggtggggtc tggcgtcacc 480
   acggaccagg tgcaggctga ggccaaagag tctgggccca cgacctacaa ggtgaccagc 540
   acactgacca tcaaagagag cgactggctc agccagagca tgttcacctg ccgcgtggat 600
   cacaggggcc tgaccttcca gcagaatgcg tcctccatgt gtgtccccga tcaagacaca 660
   gccatccggg tcttcgccat ccccccatcc tttgccagca tcttcctcac caagtccacc 720
   aagttgacct gcctggtcac agacctgacc acctatgaca gcgtgaccat ctcctggacc 780
   cgccagaatg gcgaagctgt gaaaacccac accaacatct ccgagagcca ccccaatgcc 840
   actttcagcg ccgtgggtga ggccagcatc tgcgaggatg actggaattc cggggagagg 900
   ttcacgtgca ccgtgaccca cacagacctg ccctcgccac tgaagcagac catctcccgg 960
   cccaaggggg tggccctgca caggcccgat gtctacttgc tgccaccagc ccgggagcag 1020
   ctgaacctgc gggagtcggc caccatcacg tgcctggtga cgggcttctc tcccgcggac 1080
   gtcttcgtgc agtggatgca gagggggcag cccttgtccc cggagaagta tgtgaccagc 1140
   gccccaatgc ctgagcccca ggccccaggc cggtacttcg cccacagcat cctgaccgtg 1200
   tccgaagagg aatggaacac gggggagacc tacacctgcg tggtggccca tgaggccctg 1260
   cccaacaggg tcaccgagag gaccgtggac aagtccaccg gtaaacccac cctgtacaac 1320
   gtgtccctgg tcatgtccga cacagctggc acctgctact ga 1362
<210> 403
   <211> 1975
   <212> DNA
   <213> homo sapiens;
<400> 403
   gggagtgcat ccgccccaac ccttttcccc ctcgtctcct gtgagaattc cccgtcggat 60
   acgagcagcg tggccgttgg ctgcctcgca caggacttcc ttcccgactc catcactttc 120
   tcctggaaat acaagaacaa ctctgacatc agcagcaccc ggggcttccc atcagtcctg 180
   agagggggca agtacgcagc cacctcacag gtgctgctgc cttccaagga cgtcatgcag 240
   ggcacagacg aacacgtggt gtgcaaagtc cagcacccca acggcaacaa agaaaagaac 300
   gtgcctcttc caggtgaggg ccgggcccag ccaccgggac agagagggag ccgaaggggg 360
   cgggagtggc gggcaccggg ctgacacgtg tccctcactg cagtgattgc cgagctgcct 420
   cccaaagtga gcgtcttcgt cccaccccgc gacggcttct tcggcaaccc ccgcaagtcc 480
   aagctcatct gccaggccac gggtttcagt ccccggcaga ttcaggtgtc ctggctgcgc 540
   gaggggaagc aggtggggtc tggcgtcacc acggaccagg tgcaggctga ggccaaagag 600
   tctgggccca cgacctacaa ggtgaccagc acactgacca tcaaagagag cgactggctc 660
   ggccagagca tgttcacctg ccgcgtggat cacaggggcc tgaccttcca gcagaatgcg 720
   tcctccatgt gtgtccccgg tgagtgacct gtccccaggg gcagcaccca ccgacacaca 780
   ggggtccact cgggtctggc attcgccacc ccggatgcag ccatctactc cctgagcctt 840
   ggcttcccag agcggccaag ggcaggggct cgggcggcag gacccctggg ctcggcagag 900
   gcagttgcta ctctttgggt gggaaccatg cctccgccca catccacacc tgccccacct 960
   ctgactccct tctcttgact ccagatcaag acacagccat ccgggtcttc gccatccccc 1020
   catcctttgc cagcatcttc ctcaccaagt ccaccaagtt gacctgcctg gtcacagacc 1080
   tgaccaccta tgacagcgtg accatctcct ggacccgcca gaatggcgaa gctgtgaaaa     1140
   cccacaccaa catctccgag agccacccca atgccacttt cagcgccgtg ggtgaggcca     1200
   gcatctgcga ggatgactgg aattccgggg agaggttcac gtgcaccgtg acccacacag     1260
   acctgccctc gccactgaag cagaccatct cccggcccaa gggtaggccc cactcttgcc     1320
   cctcttcctg cactccctgg gacctccctt ggcctctggg gcatggtgga aagcacccct     1380
   cactcccccg ttgtctgggc aactggggaa aaggggactc aaccccagcc cacaggctgg     1440
   tccccccact gccccgccct caccaccatc tctgttcaca ggggtggccc tgcacaggcc     1500
   cgatgtctac ttgctgccac cagcccggga gcagctgaac ctgcgggagt cggccaccat     1560
   cacgtgcctg gtgacgggct tctctcccgc ggacgtcttc gtgcagtgga tgcagagggg     1620
   gcagcccttg tccccggaga agtatgtgac cagcgcccca atgcctgagc cccaggcccc     1680
   aggccggtac ttcgcccaca gcatcctgac cgtgtccgaa gaggaatgga acacggggga     1740
   gacctacacc tgcgtggtgg cccatgaggc cctgcccaac agggtcaccg agaggaccgt     1800
   ggacaagtcc accggtaaac ccaccctgta caacgtgtcc ctggtcatgt ccgacacagc     1860
   tggcacctgc tactgaccct gctggcctgc ccacaggctc ggggcggctg gccgctctgt     1920
   gtgtgcatgc aaactaaccg tgtcaacggg gtgagatgtt gcatcttata aaatt          1975
<210> 404
   <211> 1362
   <212> DNA
   <213> homo sapiens;
<400> 404
   gggagtgcat ccgccccaac ccttttcccc ctcgtctcct gtgagaattc cccgtcggat 60
   acgagcagcg tggccgttgg ctgcctcgca caggacttcc ttcccgactc catcactttc 120
   tcctggaaat acaagaacaa ctctgacatc agcagcaccc ggggcttccc atcagtcctg 180
   agagggggca agtacgcagc cacctcacag gtgctgctgc cttccaagga cgtcatgcag 240
   ggcacagacg aacacgtggt gtgcaaagtc cagcacccca acggcaacaa agaaaagaac 300
   gtgcctcttc cagtgattgc cgagctgcct cccaaagtga gcgtcttcgt cccaccccgc 360
   gacggcttct tcggcaaccc ccgcaagtcc aagctcatct gccaggccac gggtttcagt 420
   ccccggcaga ttcaggtgtc ctggctgcgc gaggggaagc aggtggggtc tggcgtcacc 480
   acggaccagg tgcaggctga ggccaaagag tctgggccca cgacctacaa ggtgaccagc 540
   acactgacca tcaaagagag cgactggctc ggccagagca tgttcacctg ccgcgtggat 600
   cacaggggcc tgaccttcca gcagaatgcg tcctccatgt gtgtccccga tcaagacaca 660
   gccatccggg tcttcgccat ccccccatcc tttgccagca tcttcctcac caagtccacc 720
   aagttgacct gcctggtcac agacctgacc acctatgaca gcgtgaccat ctcctggacc 780
   cgccagaatg gcgaagctgt gaaaacccac accaacatct ccgagagcca ccccaatgcc 840
   actttcagcg ccgtgggtga ggccagcatc tgcgaggatg actggaattc cggggagagg 900
   ttcacgtgca ccgtgaccca cacagacctg ccctcgccac tgaagcagac catctcccgg 960
   cccaaggggg tggccctgca caggcccgat gtctacttgc tgccaccagc ccgggagcag 1020
   ctgaacctgc gggagtcggc caccatcacg tgcctggtga cgggcttctc tcccgcggac 1080
   gtcttcgtgc agtggatgca gagggggcag cccttgtccc cggagaagta tgtgaccagc 1140
   gccccaatgc ctgagcccca ggccccaggc cggtacttcg cccacagcat cctgaccgtg 1200
   tccgaagagg aatggaacac gggggagacc tacacctgcg tggtggccca tgaggccctg 1260
   cccaacaggg tcaccgagag gaccgtggac aagtccaccg gtaaacccac cctgtacaac 1320
   gtgtccctgg tcatgtccga cacagctggc acctgctact ga 1362
<210> 405
   <211> 11
   <212> DNA
   <213> homo sapiens;
<400> 405
   ctaactgggg a 11
<210> 406
   <211> 31
   <212> DNA
   <213> homo sapiens;
<400> 406
   aggatattgt agtggtggta gctgctactc c 31
<210> 407
   <211> 37
   <212> DNA
   <213> homo sapiens;
<400> 407
   gtattatgat tacgtttggg ggagttatcg ttatacc 37
<210> 408
   <211> 18
   <212> DNA
   <213> homo sapiens;
<400> 408
   gagtatagca gctcgtcc 18
<210> 409
   <211> 20
   <212> DNA
   <213> homo sapiens;
<400> 409
   gtggatacag ctatggttac 20
<210> 410
   <211> 31
   <212> DNA
   <213> homo sapiens;
<400> 410
   aggatattgt agtagtacca gctgctatgc c 31
<210> 411
   <211> 16
   <212> DNA
   <213> homo sapiens;
<400> 411
   tgactacagt aactac 16
<210> 412
   <211> 23
   <212> DNA
   <213> homo sapiens;
<400> 412
   gtggatatag tggctacgat tac 23
<210> 413
   <211> 31
   <212> DNA
   <213> homo sapiens;
<400> 413
   gtattacgat ttttggagtg gttattatac c 31
<210> 414
   <211> 31
   <212> DNA
   <213> homo sapiens;
<400> 414
   aggatattgt actaatggtg tatgctatac c 31
<210> 415
   <211> 16
   <212> DNA
   <213> homo sapiens;
<400> 415
   tgactacagt aactac 16
<210> 416
   <211> 19
   <212> DNA
   <213> homo sapiens;
<400> 416
   tgactacggt ggtaactcc 19
<210> 417
   <211> 17
   <212> DNA
   <213> homo sapiens;
<400> 417
   ggtataaccg gaaccac 17
<210> 418
   <211> 31
   <212> DNA
   <213> homo sapiens;
<400> 418
   gtattactat ggttcgggga gttattataa c 31
<210> 419
   <211> 20
   <212> DNA
   <213> homo sapiens;
<400> 419
   ggtatagtgg gagctactac 20
<210> 420
   <211> 31
   <212> DNA
   <213> homo sapiens;
<400> 420
   gtattacgat attttgactg gttattataa c 31
<210> 421
   <211> 17
   <212> DNA
   <213> homo sapiens;
<400> 421
   ggtacaactg gaacgac 17
<210> 422
   <211> 18
   <212> DNA
   <213> homo sapiens;
<400> 422
   gggtatagca gcggctac 18
<210> 423
   <211> 20
   <212> DNA
   <213> homo sapiens;
<400> 423
   gtagagatgg ctacaattac 20
<210> 424
   <211> 28
   <212> DNA
   <213> homo sapiens;
<400> 424
   agcatattgt ggtggtgact gctattcc 28
<210> 425
   <211> 17
   <212> DNA
   <213> homo sapiens;
<400> 425
   ggtataactg gaacgac 17
<210> 426
   <211> 21
   <212> DNA
   <213> homo sapiens;
<400> 426
   gggtatagca gcagctggta c 21
<210> 427
   <211> 16
   <212> DNA
   <213> homo sapiens;
<400> 427
   tgactacggt gactac 16
<210> 428
   <211> 31
   <212> DNA
   <213> homo sapiens;
<400> 428
   gtattactat gatagtagtg gttattacta c 31
<210> 429
   <211> 20
   <212> DNA
   <213> homo sapiens;
<400> 429
   gtggatacag ctatggttac 20
<210> 430
   <211> 21
   <212> DNA
   <213> homo sapiens;
<400> 430
   gggtatagca gtggctggta c 21
<210> 431
   <211> 17
   <212> DNA
   <213> homo sapiens;
<400> 431
   ggtataactg gaactac 17
<210> 432
   <211> 6
   <212> PRT
   <213> Oryctolagus cuniculus;
<400> 432
   Tyr Tyr Gly Met Asp Leu
   1 5
<210> 433
   <211> 20
   <212> DNA
   <213> Oryctolagus cuniculus;
<400> 433
   attactacgg catggacctc 20
<210> 434
   <211> 6
   <212> PRT
   <213> Ovis aries;
<400> 434
   Tyr Tyr Gly Val Asp Val
   1 5
<210> 435
   <211> 20
   <212> DNA
   <213> Ovis aries;
<400> 435
   attactacgg tgtagatgtc 20
<210> 436
   <211> 6
   <212> PRT
   <213> Bos taurus;
<400> 436
   Tyr Tyr Gly Val Asp Val
   1 5
<210> 437
   <211> 20
   <212> DNA
   <213> Bos taurus;
<400> 437
   attactacgg tgtagatgtc 20
<210> 438
   <211> 6
   <212> PRT
   <213> Canis familiaris;
<400> 438
   Tyr Tyr Gly Met Asp Tyr
   1 5
<210> 439
   <211> 20
   <212> DNA
   <213> Canis familiaris;
<400> 439
   attactatgg tatggactac 20
<210> 440
   <211> 9
   <212> PRT
   <213> homo sapiens;
<400> 440
   Tyr Tyr Tyr Tyr Tyr Gly Val Asp Val
   1 5
<210> 441
   <211> 29
   <212> DNA
   <213> homo sapiens;
<400> 441
   attactacta ctactacggt atggacgtc 29
<210> 442
   <211> 57
   <212> DNA
   <213> Homo sapiens
<400> 442
   atgggctggt cctgcatcat cctgtttctg gtggccaccg ccaccggcgt gcacagc 57
<210> 443
   <211> 19
   <212> PRT
   <213> Homo sapiens
<400> 443
   Met Gly Trp Ser Cys Ile Ile Leu Phe Leu Val Ala Thr Ala Thr Gly
   1               5                   10                  15
   Val His Ser
<210> 444
   <211> 21
   <212> DNA
   <213> Artificial Sequence
<220>
   <223> Primer
<400> 444
   aggccagcag agggttccat g 21
<210> 445
   <211> 22
   <212> DNA
   <213> Artificial Sequence
<220>
   <223> Primer
<400> 445
   ggctcccaga tcctcaaggc ac 22

## Description

### FIELD OF THE INVENTION

The present invention relates to the provision of antibody therapeutics and prophylactics that are tailored specifically for human use.

Herein described are libraries, vertebrates and cells, such as transgenic mice or rats or transgenic mouse or rat cells. The invention relates to methods of using the vertebrates to isolate a heavy chain, VH domain or antibodies. Antibodies, heavy chains, polypeptides, nucleotide sequences, pharmaceutical compositions are also described. Uses are also provided by the invention.

### BACKGROUND

The state of the art provides non-human vertebrates (eg, mice and rats) and cells comprising transgenic immunoglobulin loci, such loci comprising human variable (V), diversity (D) and/or joining (J) segments, and optionally human constant regions. Alternatively, endogenous constant regions of the host vertebrate (eg, mouse or rat constant regions) are provided in the transgenic loci. Methods of constructing such transgenic vertebrates and use of these to generate antibodies and nucleic acids thereof following antigen immunisation are known in the art, eg, see US7501552 (Medarex), US5939598 (Abgenix), US6130364 (Abgenix), WO02/066630 (Regeneron), WO2011004192 (Genome Research Limited), WO2009076464, WO2009143472 and WO2010039900 (Ablexis). Such transgenic loci in the art include varying amounts of the human V(D)J repertoire. Existing transgenic immunoglobulin loci are based on a single human DNA source. The potential diversity of human antibody variable regions in non-human vertebrates bearing such transgenic loci is thus confined.

The inventors considered that it would be desirable to tailor the genomes of these transgenic non-human vertebrates (and thus antibody and antibody chain products of these) to address the variability - and commonality - in the natural antibody gene usage of humans. The inventors wanted to do this in order to better address human use of antibody-based therapeutic and prophylactic drugs.

It would be desirable also to provide for novel and potentially expanded repertoire and diversity of human variable regions in transgenic immunoglobulin loci and non-human vertebrates harbouring these, as well as in antibodies produced following immunisation of such animals.

### SUMMARY OF THE INVENTION

The invention is as set out in the claims.

The present invention has been developed from extensive bioinformatics analysis of natural antibody gene segment distributions across a myriad of different human populations and across more than two thousand samples from human individuals. The inventors have undertaken this huge task to more thoroughly understand and design non-human vertebrate systems and resultant antibodies to better address human medical therapeutics as a whole, as well as to enable rational design to address specific ethnic populations of humans. Using such rational design, the inventors have constructed transgenic non-human vertebrates and isolated antibodies, antibody chains and cells expressing these in a way that yields products that utilise gene segments that have been purposely included on the basis of the human bioinformatics analysis. The examples illustrate worked experiments where the inventors isolated many cells and antibodies to this effect.

The disclosure relates to synthetically-extended & ethnically-diverse superhuman immunoglobulin gene repertoires. The disclosure thus provides for novel and potentially expanded synthetic immunoglobulin diversities, thus providing a pool of diversity from which human antibody therapeutic leads can be selected. This expanded pool is useful when seeking to find antibodies with desirable characteristics, such as relatively high affinity to target antigen without the need for further affinity maturation (eg, using laborious in vitro techniques such as phage or ribosome display), or improved biophysical characteristics, or to address targets and new epitopes that have previously been difficult to address with antibodies are not reached by prior antibody binding sites.

The disclosure also provides for diversity that is potentially biased towards variable gene usage common to members of a specific human population, which is useful for generating antibodies for treating and/or preventing diseases or conditions within such population. This ability to bias the antibody repertoire allows one to tailor antibody therapeutics with the aim of more effectively treating and/or preventing disease or medical conditions in specific human populations.

The present inventors realised the possibility of providing immunoglobulin gene segments from disparate sources in transgenic loci, in order to provide for novel and potentially-expanded antibody diversities from which antibody therapeutics (and antibody tool reagents) could be generated. This- opens up the potential of transgenic human-mouse/rat technologies to the possibility of interrogating different and possibly larger antibody sequence-spaces than has hitherto been possible.

In rationally designing transgenic antibody loci, as well as antibodies and antibody chains, the inventors also realised that a relatively long HCDR3 length (at least 20 amino acids) is often desirable to address epitopes. For example, naturally-occurring antibodies have been isolated from humans infected with infectious disease pathogens, such antibodies having a long HCDR3 length. Neutralising antibodies have been found in this respect. A long HCDR3 length would be desirable to address other antigens (eg, receptor clefts or enzyme active sites), not just limited to infectious disease pathogens, and thus the inventors realised the general desirability of the possibility of engineering transgenic loci to be able to produce long HCDR3 antibodies and heavy chains. The inventors, through laborious execution of bioinformatics on in excess of 2000 human DNA samples via the 1000 Genomes project together with rational sequence choices, identified that the inclusion of the specific human gene segment variant JH6*02 is desirable for producing long HCDR3 antibodies and chains.

Additional rational design and bioinformatics has led the inventors to realise that specific human constant region variants are conserved across many diverse human populations. The inventors realised that this opens up the possibility of making a choice to humanise antibodies, chains and variable domains by using such specific constant regions in products, rather than arbitrarily choosing the human constant region (or a synthetic version of a human constant region). This aspect of the disclosure also enables one to tailor antibody-based drugs to specific human ethnic populations, thereby more closely matching drug to patient (and thus disease setting) than has hitherto been performed. It can be a problem in the state of the art that antibodies are humanised with an arbitrary choice of human constant region (presumably derived from one (often unknown) ethnic population or non-naturally occurring) that does not function as well in patients of a different human ethnic population. This is important, since the constant region has the major role in providing antibody effector functions, eg, for antibody recycling, cellular and complement recruitment and for cell killing.

Disclosed herein is:

### First Configuration

A non-human vertebrate or vertebrate cell (optionally an ES cell or antibody-producing cell) comprising a genome having a superhuman immunoglobulin heavy chain human VH and/or D and/or J gene repertoire.

A non-human vertebrate or vertebrate cell (optionally an ES cell or antibody-producing cell) comprising a genome having a superhuman immunoglobulin light chain human VL gene repertoire; optionally wherein the vertebrate or cell is according to the first configuration.

A non-human vertebrate or vertebrate cell (optionally an ES cell or antibody-producing cell) whose genome comprises a transgenic immunoglobulin locus (eg, a heavy chain locus or a light chain locus), said locus comprising immunoglobulin gene segments according to the first and second human immunoglobulin gene segments (optionally V segments) as mentioned below operably connected upstream of an immunoglobulin constant region; optionally wherein the genome is homozygous for said transgenic immunoglobulin locus; optionally wherein the immunoglobulin locus comprises more than the natural human complement of functional V gene segments; and/or optionally wherein the immunoglobulin locus comprises more than the natural human complement of functional D gene segments; and/or optionally wherein the immunoglobulin locus comprises more than the natural human complement of functional J gene segments.

A transgenic non-human vertebrate (eg, a mouse or rat) or vertebrate cell (optionally an ES cell or antibody-producing cell) whose genome comprises a transgenic immunoglobulin locus comprising a plurality of human immunoglobulin gene segments operably connected upstream of a non-human vertebrate constant region for the production of a repertoire of chimaeric antibodies, or chimaeric light or heavy chains, having a non-human vertebrate constant region and a human variable region; wherein the transgenic locus comprises one or more human immunoglobulin V gene segments, one or more human J gene segments and optionally one or more human D gene segments, a first (optionally a V segment) of said gene segments and a second (optionally a V segment) of said gene segments being different and derived from the genomes of first and second human individuals respectively, wherein the individuals are different; and optionally not related;
optionally wherein the immunoglobulin locus comprises more than the natural human complement of functional V gene segments; and/or
optionally wherein the immunoglobulin locus comprises more than the natural human complement of functional D gene segments; and/or
optionally wherein the immunoglobulin locus comprises more than the natural human complement of functional J gene segments.

A transgenic non-human vertebrate (eg, a mouse or rat) or vertebrate cell (optionally an ES cell or antibody-producing cell) whose genome comprises first and second transgenic immunoglobulin loci, each locus comprising a plurality of human immunoglobulin gene segments operably connected upstream of a non-human vertebrate constant region for the production of a repertoire of chimaeric antibodies, or chimaeric light or heavy chains, having a non-human vertebrate constant region and a human variable region;
wherein (i) the first transgenic locus comprises one or more human immunoglobulin V gene segments, one or more human J gene segments and optionally one or more human D gene segments, (ii) the second transgenic locus comprises one or more human immunoglobulin V gene segments, one or more human J gene segments and optionally one or more human D gene segments; and (iii) wherein a first (optionally a V) gene segment of said first locus and a second (optionally a V) gene segment of said second gene locus are different and derived from the genomes of first and second human individuals respectively, wherein the individuals are different; and optionally not related;
optionally wherein the first and second loci are on different chromosomes (optionally chromosomes with the same chromosome number) in said genome;
optionally wherein each immunoglobulin locus comprises more than the natural human complement of functional V gene segments; and/or
optionally wherein each immunoglobulin locus comprises more than the natural human complement of functional D gene segments; and/or
optionally wherein each immunoglobulin locus comprises more than the natural human complement of functional J gene segments.

A method of constructing a cell (eg, an ES cell) according to the disclosure, the method comprising
(a) identifying functional V and J (and optionally D) gene segments of the genome sequence of a (or said) first human individual;
(b) identifying one or more functional V and/or D and/or J gene segments of the genome sequence of a (or said) second human individual, wherein these additional gene segments are not found in the genome sequence of the first individual;
(c) and constructing a transgenic immunoglobulin locus in the cell, wherein the gene segments of (a) and (b) are provided in the locus operably connected upstream of a constant region.

In one embodiment, the gene segment(s) in step (b) are identified from an immunoglobulin gene database selected from the 1000 Genomes, Ensembl, Genbank and IMGT databases.

Throughout this text, Genbank is a reference to Genbank release number 185.0 or 191.0; the 1000 Genomes database is Phase 1, release v3, 16th March 2012; the Ensembl database is assembly GRCh37.p8 (10/04/2012); the IMGT database is available at www.imgt.org .

In one embodiment, the first and second human individuals are members of first and second ethnic populations respectively, wherein the populations are different, optionally wherein the human immunoglobulin gene segment derived from the genome sequence of the second individual is low- frequency (optionally rare) within the second ethnic population.

This configuration of the disclosure also provides a method of making a transgenic non-human vertebrate (eg, a mouse or rat), the method comprising
(a) constructing an ES cell (eg, a mouse C57BL/6N, C57BL/6J, 129S5 or 129Sv strain ES cell) by carrying out the method above;
(b) injecting the ES cell into a donor non-human vertebrate blastocyst (eg, a mouse C57BL/6N, C57BL/6J, 129S5 or 129Sv strain blastocyst);
(c) implanting the blastocyst into a foster non-human vertebrate mother (eg, a C57BL/6N, C57BL/6J, 129S5 or 129Sv strain mouse); and
(d) obtaining a child from said mother, wherein the child genome comprises a transgenic immunoglobulin locus.

In one embodiment, the invention provides a method of isolating an antibody that binds a predetermined antigen (eg, a bacterial or viral pathogen antigen), the method comprising immunising a non-human vertebrate according to the disclosure.

### Second Configuration

A library of antibody-producing transgenic cells whose genomes collectively encode a repertoire of antibodies, wherein
(a) a first transgenic cell expresses a first antibody having a chain encoded by a first immunoglobulin gene, the gene comprising a first variable domain nucleotide sequence produced following recombination of a first human unrearranged immunoglobulin gene segment;
(b) a second transgenic cell expresses a second antibody having a chain encoded by a second immunoglobulin gene, the second gene comprising a second variable domain nucleotide sequence produced following recombination of a second human unrearranged immunoglobulin gene segment, the first and second antibodies being non-identical;
(c) the first and second gene segments are different and derived from the genome sequences of first and second human individuals respectively, wherein the individuals are different; and optionally not related;
(d) wherein the cells are non-human vertebrate (eg, mouse or rat) cells.

In one embodiment, the first and second human individuals are members of first and second ethnic populations respectively, wherein the populations are different; optionally wherein the ethnic populations are selected from those identified in the 1000 Genomes database.

In another embodiment, the second human immunoglobulin gene segment is a polymorphic variant of the first human immunoglobulin gene segment; optionally wherein the second gene segment is selected from the group consisting of a gene segment in any of Tables 1 to 7 and 9 to 14 below (eg, selected from Table 13 or Table 14), eg, the second gene segment is a polymorphic variant of VH1- 69.

### Third Configuration

An isolated antibody having
(a) a heavy chain encoded by a nucleotide sequence produced following recombination in a transgenic non-human vertebrate cell of an unrearranged human immunoglobulin V gene segment with a human D and human J segment, optionally with affinity maturation in said cell, wherein one of the gene segments is derived from the genome of an individual from a first human ethnic population; and the other two gene segments are derived from the genome of an individual from a second, different, human ethnic population, and wherein the antibody comprises heavy chain constant regions of said non-human vertebrate (eg, rodent, mouse or rat heavy chain constant regions); and/or
(b) a light chain encoded by a nucleotide sequence produced following recombination in a transgenic non-human vertebrate cell of an unrearranged human immunoglobulin V gene segment with a human J segment, optionally with affinity maturation in said cell, wherein one of the gene segments is derived from the genome of an individual from a first human ethnic population (optionally the same as the first population in (a)); and the other gene segment is derived from the genome of an individual from a second, different, human ethnic population (optionally the same as the second population in (a)), and wherein the antibody comprises light chain constant regions of said non-human vertebrate (eg, rodent, mouse or rat heavy light constant regions);
(c) Optionally wherein each variable domain of the antibody is a human variable domain.
(d) Optionally wherein the heavy chain constant regions are gamma-type constant regions.

The disclosure also provides an isolated nucleotide sequence encoding the antibody, optionally wherein the sequence is provided in an antibody expression vector, optionally in a host cell.

The invention also provides a method of producing a human antibody, the method comprising replacing the non-human vertebrate constant regions of the antibody of the third configuration with human antibody constant regions.

The disclosure also provides a pharmaceutical composition comprising an antibody according to the third configuration, or an antibody produced according to the method above and a diluent, excipient or carrier; optionally wherein the composition is provided in a container connected to an IV needle or syringe or in an IV bag.

The disclosure also provides an antibody-producing cell that expresses the second antibody recited in any one of the configurations.

In an alternative configuration, the disclosure contemplates the combination of nucleotide sequences of first and second immunoglobulin gene segments (eg, two or more polymorphic variants of a particular human germline VH or VL gene segment) to provide a synthetic gene segment. Such synthetic gene segment is used, in one embodiment, to build a transgenic immunoglobulin locus, wherein the synthetic gene segment is provided in combination with one or more human variable and J regions (and optionally one or more human D regions) operably connected upstream of a constant region. When provided in the genome of a non-human vertebrate or cell (eg, mouse or rat cell, eg, ES cell), the disclosure provides for superhuman gene segment diversity. The sequences to be combined can be selected from gene segments that have been observed to be commonly used in human antibodies raised against a particular antigen (eg, a flu antigen, such as haemaglutinin). By combining the sequences, the synthetic gene segment may recombine in vivo to produce an antibody that is well suited to the treatment and/or prevention of a disease or condition (eg, influenza) mediated by said antigen.

### Fourth Configuration

A non-human vertebrate (optionally a mouse or a rat) or vertebrate cell whose genome comprises an immunoglobulin heavy chain locus comprising human gene segment JH6*02, one or more VH gene segments and one or more D gene segments upstream of a constant region; wherein the gene segments in the heavy chain locus are operably linked to the constant region thereof so that the mouse is capable of producing an antibody heavy chain produced by recombination of the human JH6*02 with a D segment and a VH segment.

A non-human vertebrate cell (optionally a mouse cell or a rat cell) whose genome comprises an immunoglobulin heavy chain locus comprising human gene segment JH6*02, one or more VH gene segments and one or more D gene segments upstream of a constant region; wherein the gene segments in the heavy chain locus are operably linked to the constant region thereof for producing (eg, in a subsequent progeny cell) an antibody heavy chain produced by recombination of the human JH6*02 with a D segment and a VH segment.

A heavy chain (eg, comprised by an antibody) isolated from a vertebrate of the invention wherein the heavy chain comprises a HCDR3 of at least 20 amino acids.

A method for producing a heavy chain, VH domain or an antibody specific to a target antigen, the method comprising immunizing a non-human vertebrate according to the disclosure with the antigen and isolating the heavy chain, VH domain or an antibody specific to a target antigen or a cell producing the heavy chain, VH domain or an antibody, wherein the heavy chain, VH domain or an antibody comprises a HCDR3 that is derived from the recombination of human JH6*02 with a VH gene segment and a D gene segment.

A heavy chain, VH domain or an antibody produced by the method.

A B-cell or hybridoma expressing a heavy chain VH domain that is identical to the VH domain of the heavy chain.

A nucleic acid encoding the VH domain of the heavy chain disclosed herein, or encoding the heavy chain.

A vector (eg, a CHO cell or HEK293 cell vector) comprising the nucleic acid; optionally wherein the vector is in a host cell (eg, a CHO cell or HEK293 cell).

A pharmaceutical composition comprising the antibody, heavy chain or VH domain (eg, comprised by an antibody), together with a pharmaceutically-acceptable excipient, diluent or a medicament (eg, a further antigen-specific variable domain, heavy chain or antibody).

The antibody, heavy chain or VH domain (eg, comprised by an antibody) as above for use in medicine.

The use of an antibody, heavy chain or VH domain (eg, comprised by an antibody) as above in the manufacture of a medicament for treating and/or preventing a medical condition in a human.

### Fifth Configuration

A method of producing an antibody heavy chain, the method comprising
(a) providing an antigen-specific heavy chain variable domain; and
(b) combining the variable domain with a human heavy chain constant region to produce an antibody heavy chain comprising (in N- to C-terminal direction) the variable domain and the constant region;
wherein the human heavy chain constant region is an IGHG1ref, IGHG2ref, IGHG2a, IGHG3ref, IGHG3a, IGHG3b, IGHG4ref or IGHG4a constant region.

An antibody comprising a human heavy chain, the heavy chain comprising a variable domain that is specific for an antigen and a constant region that is an IGHG1ref, IGHG2ref, IGHG2a, IGHG3ref, IGHG3a, IGHG3b, IGHG4ref or IGHG4a constant region. Optionally, the variable domain comprises mouse-pattern AID somatic mutations.

A polypeptide comprising (in N- to C- terminal direction) a leader sequence, a human variable domain that is specific for an antigen and a human constant region that is an IGHG1ref, IGHG2ref, IGHG2a, IGHG3ref, IGHG3a, IGHG3b, IGHG4ref or IGHG4a constant region wherein (i) the leader sequence is not the native human variable domain leader sequence; and/or (ii) the variable domain comprises mouse AID-pattern somatic mutations and/or mouse Terminal deoxynucleotidyl transferase (TdT)- pattern junctional mutations.

A nucleotide sequence encoding (in 5' to 3' direction) a leader sequence and a human antibody heavy chain, the heavy chain comprising a variable domain that is specific for an antigen and a constant region that is an IGHG1ref, IGHG2ref, IGHG2a, IGHG3ref, IGHG3a, IGHG3b, IGHG4ref or IGHG4a constant region; and the leader sequence being operable for expression of the heavy chain and wherein the leader sequence is not the native human variable domain leader sequence.

A nucleotide sequence encoding (in 5' to 3' direction) a promoter and a human antibody heavy chain, the heavy chain comprising a variable domain that is specific for an antigen and a constant region that is an IGHG1ref, IGHG2ref, IGHG2a, IGHG3ref, IGHG3a, IGHG3b, IGHG4ref or IGHG4a constant region; and the promoter being operable for expression of the heavy chain and wherein the promoter is not the native human promoter.

A vector (eg, a CHO cell or HEK293 cell vector) comprising a IGHG1ref, IGHG2ref, IGHG2a, IGHG3ref, IGHG3a, IGHG3b, IGHG4ref or IGHG4a constant region nucleotide sequence that is 3' of a cloning site for the insertion of a human antibody heavy chain variable domain nucleotide sequence, such that upon insertion of such a variable domain sequence the vector comprises (in 5' to 3' direction) a promoter, a leader sequence, the variable domain sequence and the constant region sequence so that the vector is capable of expressing a human antibody heavy chain when present in a host cell.

### Sixth Configuration

A non-human vertebrate (eg, a mouse or rat) or a non-human vertebrate cell (eg, an ES cell or a B- cell) having a genome comprising at least 3 human variable region gene segments of the same type (eg, at least 3 human VH6-1 gene segments, at least 3 human JH6 gene segments, at least 3 human Vκ1-39 gene segments, at least 3 human D2-2 gene segments or at least 3 human JK1 gene segments), wherein at least two of the human gene segments are variants that are not identical to each other.

A non-human vertebrate (eg, a mouse or rat) or a non-human vertebrate cell (eg, an ES cell or a B- cell) having a genome comprising at least 2 different non-endogenous variable region gene segments of the same type (eg, at least 2 human VH6-1 gene segments, at least 3 human JH6 gene segments, at least 2 human Vκ1-39 gene segments, at least 2 human D2-2 gene segments or at least 2 human Jκ1 gene segments) cis at the same Ig locus.

A non-human vertebrate (eg, a mouse or rat) or a non-human vertebrate cell (eg, an ES cell or a B- cell) having a genome comprising at least 2 different human variable region gene segments of the same type (eg, at least 2 human VH6-1 gene segments, at least 2 human JH6 gene segments, at least 2 human Vκ1-39 gene segments, at least 2 human D2-2 gene segments or at least 2 human Jκ1 gene segments) trans at the same Ig locus; and optionally a third human gene segment of the same type, wherein the third gene segment is cis with one of said 2 different gene segments.

A population of non-human vertebrates (eg, mice or rats) comprising a repertoire of human variable region gene segments, wherein the plurality comprises at least 2 human variable region gene segments of the same type (eg, at least 2 human VH6-1 gene segments, at least 2 human JH6 gene segments, at least 2 human Vκ1-39 gene segments, at least 2 human D2-2 gene segments or at least 2 human Jκ1 gene segments), a first of said different gene segments is provided in the genome of a first vertebrate of the population, and a second of said different gene segments being provided in the genome of a second vertebrate of the population, wherein the genome of the first vertebrate does not comprise the second gene segment.

A non-human vertebrate (eg, a mouse or rat) or a non-human vertebrate cell (eg, an ES cell or a B- cell) having a genome comprising at least 2 different non-endogenous variable region gene segments of the same type (eg, at least 2 human VH6-1 gene segments, at least 2 human JH6 gene segments, at least 2 human Vκ1-39 gene segments, at least 2 human D2-2 gene segments or at least 2 human Jκ1 gene segments), wherein the gene segments are derived from the genome sequence of different human individuals that are not genetically related over at least 3 generations.

A method of enhancing the human immunoglobulin gene diversity of a non-human vertebrate (eg, a mouse or rat), the method comprising providing the vertebrate with a genome comprising at least 3 human variable region gene segments of the same type (eg, at least 3 human VH6-1 gene segments, at least 3 human JH6 gene segments, at least 3 human Vκ1-39 gene segments, at least 3 human D2-2 gene segments or at least 3 human Jκ1 gene segments), wherein at least two of the human gene segments are variants that are not identical to each other.

A method of enhancing the immunoglobulin gene diversity of a non-human vertebrate (eg, a mouse or rat), the method comprising providing the vertebrate with a genome comprising at least 2 different non-endogenous variable region gene segments of the same type (eg, at least 2 human VH6-1 gene segments, at least 2 human JH6 gene segments, at least 2 human Vκ1-39 gene segments, at least 2 human D2-2 gene segments or at least 2 human Jκ1 gene segments) cis at the same Ig locus.

A method of enhancing the immunoglobulin gene diversity of a non-human vertebrate (eg, a mouse or rat), the method comprising providing the vertebrate with a genome comprising at least 2 different human variable region gene segments of the same type (eg, at least 2 human VH6-1 gene segments, at least 2 human JH6 gene segments, at least 2 human Vκ1-39 gene segments, at least 2 human D2-2 gene segments or at least 2 human Jκ1 gene segments) trans at the same Ig locus; and optionally a third human gene segment of the same type, wherein the third gene segment is cis with one of said 2 different gene segments.

A method of providing an enhanced human immunoglobulin variable region gene segment repertoire, the method comprising providing a population of non-human vertebrates (eg, a mouse or rat) comprising a repertoire of human variable region gene segments, wherein the method comprises providing at least 2 different human variable region gene segments of the same type (eg, at least 2 human VH6-1 gene segments, at least 2 human JH6 gene segments, at least 2 human Vκ1- 39 gene segments, at least 2 human D2-2 gene segments or at least 2 human Jκ1 gene segments), wherein a first of said different gene segments is provided in the genome of a first vertebrate of the population, and a second of said different gene segments is provided in the genome of a second vertebrate of the population, wherein the genome of the first vertebrate does not comprise the second gene segment.

A method of enhancing the human immunoglobulin gene diversity of a non-human vertebrate (eg, a mouse or rat), the method comprising providing the vertebrate with a genome comprising at least 2 different non-endogenous variable region gene segments of the same type (eg, at least 2 human VH6-1 gene segments, at least 2 human JH6 gene segments, at least 2 human Vκ1-39 gene segments, at least 2 human D2-2 gene segments or at least 2 human Jκ1 gene segments), wherein the gene segments are derived from the genome sequence of different human individuals that are not genetically related over at least 3 generations.

A method of enhancing the human immunoglobulin gene diversity of a non-human vertebrate (eg, a mouse or rat), the method comprising providing the vertebrate with a genome comprising at least 2 human variable region gene segments of the same type (eg, at least 2 human VH6-1 gene segments, at least 2 human JH6 gene segments, at least 2 human Vκ1-39 gene segments, at least 2 human D2-2 gene segments or at least 2 human Jκ1 gene segments), wherein the gene segments are derived from the genome sequence of different human individuals that are not genetically related over at least 3 generations; optionally wherein at least 2 or 3 of said different gene segments are provided at the same Ig locus in said genome.

A non-human vertebrate (eg, a mouse or rat) or a non-human vertebrate cell (eg, an ES cell or a B- cell) having a genome comprising first and second human Ig locus gene segments of the same type (eg, first and second human JH6 gene segments; or first and second IgG2 gene segments; or first and second human Jλ7 gene segments), wherein the first gene segment is a gene segment selected from any one of Tables 1 and 9 to 14 (eg, selected from Table 13 or Table 14) (eg, IGHJ6-a) and the second gene segment is the corresponding reference sequence.

A population of non-human vertebrates (eg, mice or rats) comprising first and second human Ig locus gene segments of the same type (eg, first and second human JH6 gene segments; or first and second IgG2 gene segments; or first and second human Jλ7 gene segments), wherein the first gene segment is a gene segment selected from any one of Tables 1 and 9 to 14 (eg, selected from Table 13 or Table 14) (eg, IGHJ6-a) and the second gene segment is the corresponding reference sequence, wherein the first gene segment is provided in the genome of a first vertebrate of the population, and the second gene segment is provided in the genome of a second vertebrate of the population.

A method of enhancing the human immunoglobulin gene diversity of a non-human vertebrate (eg, a mouse or rat), the method comprising providing the vertebrate with a genome comprising first and second human Ig locus gene segments of the same type (eg, first and second human JH6 gene segments; or first and second IgG2 gene segments; or first and second human Jλ7 gene segments), wherein the first gene segment is a gene segment selected from any one of Tables 1 and 9 to 14 (eg, selected from Table 13 or Table 14) (eg, IGHJ6-a) and the second gene segment is the corresponding reference sequence.

In one aspect of this configuration, the disclosure relates to human D gene segment variants as described further below.

In one aspect of this configuration, the disclosure relates to human V gene segment variants as described further below.

In one aspect of this configuration, the disclosure relates to human J gene segment variants as described further below.

### BRIEF DESCRIPTION OF THE FIGURES

Figures 1 to 3: Schematic illustrating a protocol for producing recombineered BAC vectors to add V gene segments into a mouse genome;
Figure 4: Schematic illustrating a protocol for adding V gene segments to a mouse genome using sequential recombinase mediated cassette exchange (sRMCE); and
Figure 5 (in 4 parts): Alignment of 13 IGHV1-69 variants showing the variable (V) coding region only. Nucleotides that differ from VH1-69 variant *01 are indicated at the appropriate position whereas identical nucleotides are marked with a dash. Where nucleotide changes result in amino acid differences, the encoded amino acid is shown above the corresponding triplet. Boxed regions correspond to CDR1, CDR2 and CDR3 as indicated.
Figure 6 is a schematic illustrating gene segment diversity and the effect of including variant variants in *cis* according to the disclosure :-
   (a) Situation in a normal person: Recombination on the same chromosome limits combinations of variants, for instance the antibody gene V4-4 can only be recombined within variant 1 to form for instance for instance V4-4-D-J6 or V4-4-D-J2^{A}. Similarly the variant V4-4^{A} can't be recombined with either J6 or J2^{A} from variant 1 and can only be joined with J-genes from variant 2 to form V4-4^{A}-D-J6^{A} and V4-4^{A}-D-J2. V4-4-J2/J6 complexity = 4.
   (b) Situation in a transgenic mouse: Only one variant is provided so the genome is limited. V4- 4-J6/J2 complexity = 2.
   (c) Supra mouse of the disclosure: The variants are added in cis and thus can be recombined in every combination, expanding the repertoire. For instance V4-4 can be combined with J6A, J6, J2A or J2 and similarly V4-4A can be recombined with these same J-genes. The V4-4-J6/J2 complexity = 8, which in this simple example is double that of a person and 4X that of a mouse with a single variant.

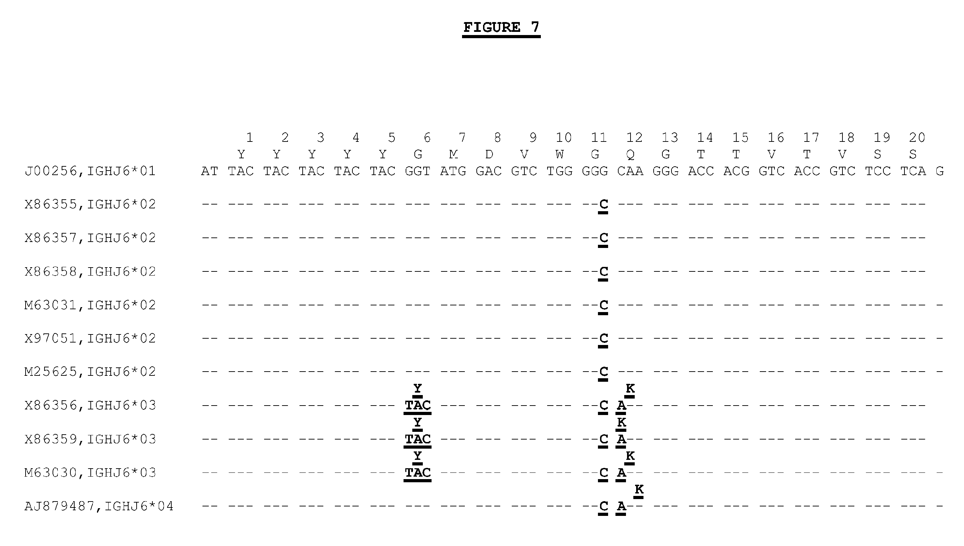

Figure 7: Alignment of human JH6^{*}02 variants. Nucleotides that differ from JH6 *01 are indicated at the appropriate position whereas identical nucleotides are marked with a dash. Where nucleotide changes result in amino acid differences, the encoded amino acid is shown above. Accession numbers (eg, J00256) are shown to the left of the IMGT variant name.

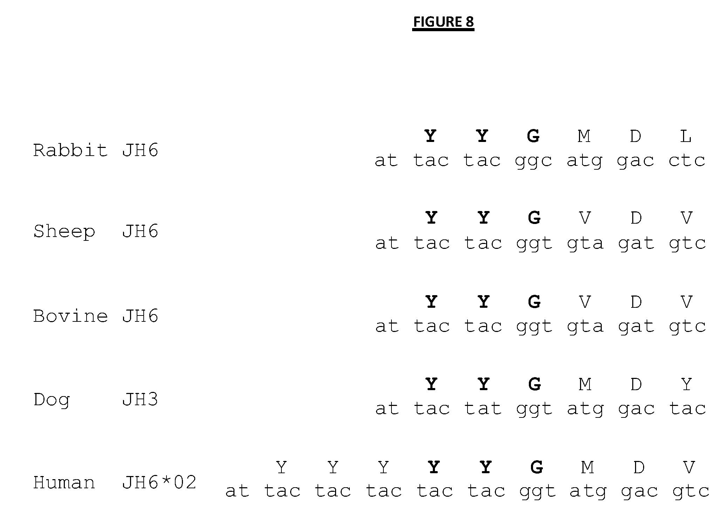

Figure 8: Alignment of JH sequences from various species.

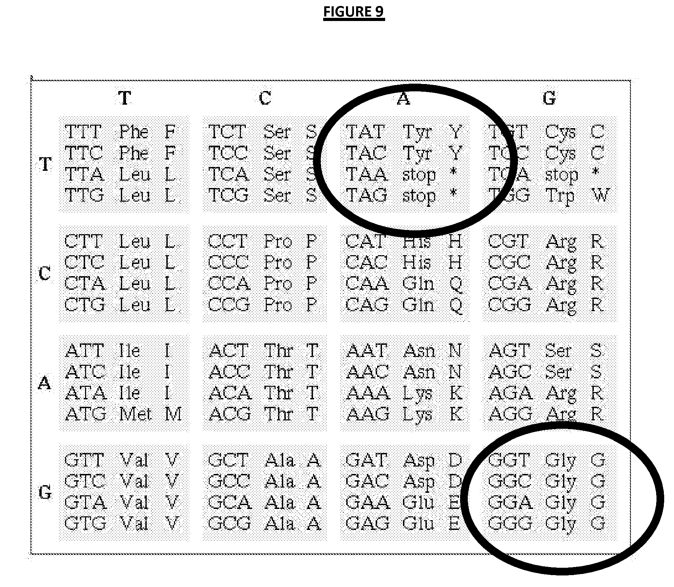

Figure 9: Codon Table

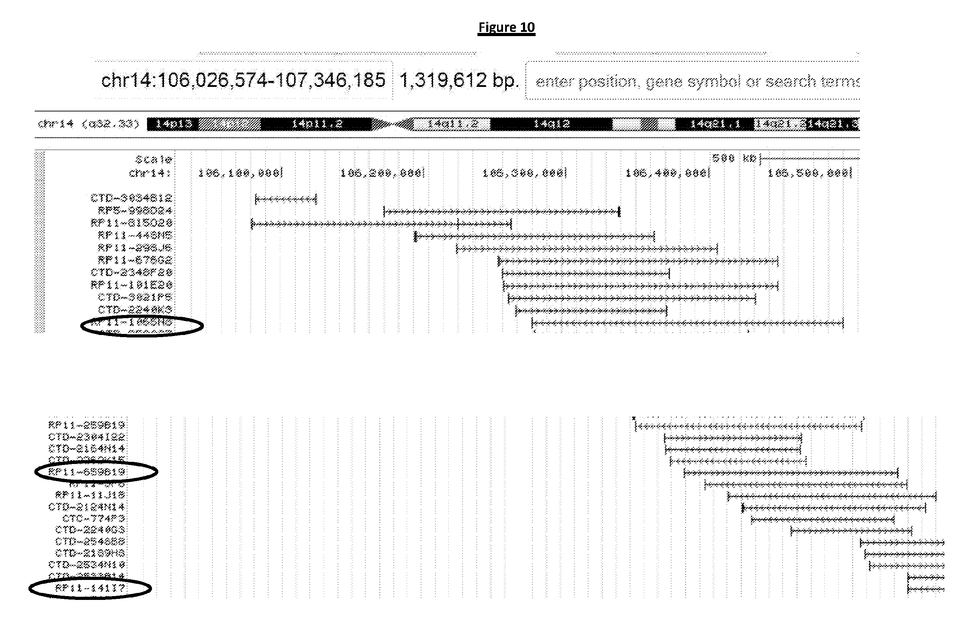

Figure 10: BAC database extract

### BRIEF DESCRIPTION OF THE TABLES

**Table 1:** Human IgH V Polymorphic Variants
**Table 2:** Human IgH D Polymorphic Variants
**Table 3:** Human IgH J Polymorphic Variants
**Table 4:** Human Ig Vκ Polymorphic Variants
**Table 5:** Human Ig Vλ Polymorphic Variants
**Table 6:** Human IgH Jκ Polymorphic Variants
**Table 7:** Human IgH Jλ Polymorphic Variants
**Table 8:** 1000 Genomes Project Human Populations
**Table 9:** Immunoglobulin Gene Usage in Human Antibody Responses to Infectious Disease Pathogens
**Table 10A:** Human IgH JH5 Variant Occurrences
**Table 10B:** Non-Synonymous Human IgH JH5 Variants
**Table 11A:** Human IgH JH6 Variant Occurrences
**Table 11 B:** Non-Synonymous Human IgH JH6 Variants
**Table 12A:** Human IgH JH2 Variant Occurrences
**Table 12B:** Non-Synonymous Human IgH JH2 Variants
**Table 13:** Variant Frequency Analyses & Human Population Distributions
**Table 14:** Frequent Human Variant Distributions
**Table 15:** Human Gene Segment Usage: Heavy Chain Repertoires From Naive Non-Human Vertebrates
**Table 16:** Human Gene Segment Usage: Heavy Chain Repertoires From Immunised Non-Human Vertebrates
**Table 17:** Human Gene Segment Usage: Heavy Chain Repertoires From Antigen-Specific Hybridomas
**Table 18:** Sequence Correlation Table
**Table 19:** Summary Of Function Correlated With Human Gamma Constant Region SubType
**Table 20:** Gene Segments Prevalent In Few Human Populations
**Table 21:** Genomic and sequence information

### DETAILED DESCRIPTION OF THE INVENTION

A suitable source of JH6*02 and other human DNA sequences for use in the invention will be readily apparent to the skilled person. For example, it is possible to collect a DNA sample from a consenting human donor (eg, a cheek swab sample as per the Example herein) from which can be obtained suitable DNA sequences for use in constructing a locus of the invention. Other sources of human DNA are commercially available, as will be known to the skilled person. Alternatively, the skilled person is able to construct gene segment sequence by referring to one or more databases of human Ig gene segment sequences disclosed herein.

An example source for human V, D and J gene segments according to the invention are Bacterial Artificial Chromosomes (RPCl-11 BACs) obtained from Roswell Park Cancer Institute (RPCl)/Invitrogen. See http://bacpac.chori.org/hmale11.htm, which describes the BACs as follows:-

### "RPCl-11 Human Male BAC Library

*The RPCl-11 Human Male BAC Library (Osoegawa et al., 2001) was constructed using improved cloning techniques (Osoegawa et al.,* 1998) *developed by Kazutoyo Osoegawa. The library was generated by Kazutoyo Osoegawa. Construction was funded by a grant from the National Human Genome Research Institute (NHGRI, NIH) (#1R01RG01165-03). This library was generated according to the new NHGRI*/*DOE "Guidance on Human Subjects in Large-Scale DNA Sequencing...*

*"Male blood was obtained via a double-blind selection protocol. Male blood DNA was isolated from one randomly chosen donor (out of 10 male donors)".*
- Osoegawa K, Mammoser AG, Wu C, Frengen E, Zeng C, Catanese JJ, de Jong PJ; Genome Res. 2001 Mar;11(3):483-96; "A bacterial artificial chromosome library for sequencing the complete human genome";
- Osoegawa, K., Woon, P.Y., Zhao, B., Frengen, E., Tateno, M., Catanese, J.J, and de Jong, P.J. (1998); "An Improved Approach for Construction of Bacterial Artificial Chromosome Libraries"; Genomics 52, 1-8.

### Superhuman Immunoglobulin Gene Repertoires

The disclosure relates to synthetically-extended & ethnically-diverse superhuman immunoglobulin gene repertoires. The human immunoglobulin repertoires are beyond those found in nature (ie, "Superhuman"), for example, they are more diverse than a natural human repertoire or they comprise combinations of human immunoglobulin gene segments from disparate sources in a way that is non-natural. Thus, the repertoires described herein are "superhuman" immunoglobulin repertoires, and the disclosurerelates to the application of these in transgenic cells and non-human vertebrates for utility in producing chimaeric antibodies (with the possibility of converting these into fully-human, isolated antibodies using recombinant DNA technology). The present disclosure thus provides for novel and potentially expanded synthetic immunoglobulin diversities, which provides for a pool of diversity from which antibody therapeutic leads (antibody therapeutics and antibody tool reagents) can be selected. This opens up the potential of transgenic human-mouse/rat technologies to the possibility of interrogating different and possibly larger antibody sequence- spaces than has hitherto been possible. To this end, in one embodiment, the disclosure provides a SUPERHUMAN MOUSE™ (aka SUPRA-MOUSE™) and a SUPERHUMAN RAT™ (aka SUPRA-RAT™).

In developing this thinking, the present inventors have realised the possibility of mining the huge genetics resources now available to the skilled person thanks to efforts such as the HapMap Project, 1000 Genomes Project and sundry other immunoglobulin gene databases (see below for more details). Thus, in some embodiments, the inventors realised the application of these genome sequencing developments in the present disclosure to generate synthetically-produced and ethnically-diverse artificial immunoglobulin gene repertoires. In one aspect, the inventors realised that such repertoires are useful for the production of antibodies having improved affinity and/or biophysical characteristics, and/or wherein the range of epitope specificities produced by means of such repertoire is novel, provides for antibodies to epitopes that have hitherto been intractable by prior transgenic immunoglobulin loci or difficult to address.

The present disclosure provides libraries, vertebrates and cells, such as transgenic mice or rats or transgenic mouse or rat cells. Furthermore, the invention relates to methods of using the vertebrates to isolate antibodies or nucleotide sequences encoding antibodies. Also disclosed are antibodies, nucleotide sequences and pharmaceutical compositions. Uses are also provided by the invention.

### Variation Analysis

The present inventors have realized methods and antibody loci designs that harness the power of genetic variation analysis. The reference human genome provides a foundation for experimental work and genetic analysis of human samples. The reference human is a compilation of the genomes from a small number of individuals and for any one segment of the genome a high quality single reference genome for one of the two chromosomes is available. Because the reference genome was assembled from a series of very large insert clones, the identity of these clones is known. Accordingly, experimental work with human genomic DNA is usually conducted on the clones from which the reference sequence was derived.

Individual humans differ in their sequence and recently several individuals have had their genomes sequenced, for instance James Watson and Craig Venter. Comparison of the genome sequence of these individuals has revealed differences between their sequences and the reference genome in both coding and non-coding parts of the genome, approximately 1 in 1000 bases are different. Some variants will be significant and contribute to differences between individuals. In extreme cases these will result in genetic disease. Variation can be implicated in differing responses to drugs administered to human patients, eg, yielding an undesirable lowering of patient response to treatment.

The 1000-Genomes Project has the objective of identifying the most frequent variations in the human genome. This public domain project involved sequencing the genomes of more than 1000 individuals from diverse ethnic groups, comparing these sequences to the reference and assembling a catalogue of variants. This has enabled the annotation of variants in coding regions, but because this sequence wasn't derived from large clones of DNA, the analysis of the sequence from diploid individuals can't discriminate the distribution of the variation between the maternal and paternally inherited chromosomes. Where more than one variant is identified in a protein coding gene, it is not possible to illuminate the distribution of the pattern of variants in each version of the protein. For example, if two variants are detected in different positions of the same protein in an individual, this could have resulted from one copy with two variants and none in the other or each copy could have just one variant. To illuminate the sequence of real proteins, the 1000-Genome Project has sequenced mother-father-child trios. This allows one to "phase" the sequence variants, in other words identify blocks of sequence that are inherited from one or other parent and deconvolute the variants.

To further understand the variation within the 1000-genome set a tool has been developed that can identify the significant variants (defined as non-synonymous amino acid changes) from a region of DNA from the phased data in the 1000-genome data set. This tool has been made available online http://www.1000genomes.org/variation-pattern-finder. This tool allows an investigator to download non-synonymous variation delimited between specific coordinates. The downloaded files are configured as individual genotypes, but the data is phased so the haplotype information and the frequencies of specific haplotypes in different populations can be extracted.

The inventors' analysis of the 1000-genome data for the individual human coding segments of the C, V D and J genes from the heavy and light chains reveals that there is significant variation in these segments. Individuals will usually have two different heavy chain alleles and also different light chain alleles at both kappa and lambda loci. The repertoire of antibodies that can be generated from each allele will be different. This variation will contribute to a better or differing immune response to certain antigens.

Humanized mice that have hitherto been generated with immunoglobulin heavy and light chain loci contain just one type of immunoglobulin locus. Even if these mice contain a full human heavy chain locus, the variation will be less than contained in a typical human because only one set of C, V, D and J genes are available, while a typical human would have two sets.

The inventors have devised ways to improve on this limitation when constructing transgenic non-human vertebrates and cells for human antibody and variable region production in vivo.

Mice can be generated with two different loci, each engineered to have a different repertoire of V, D and J segments. This could be in a single mouse or two or more separate mouse strains and would be analogous to or beyond the repertoire found in a normal human. The engineering of such a mouse would go beyond the repertoire described in humanized mice to date which only have one set of alleles.

However, the inventors also realized that this also has limitations, because the different loci would not normally interact to shuffle V, D and J variants between loci. This same limitation is also inherent in a human, thus this system does not utilize the advantage of recombining variants in all combinations.

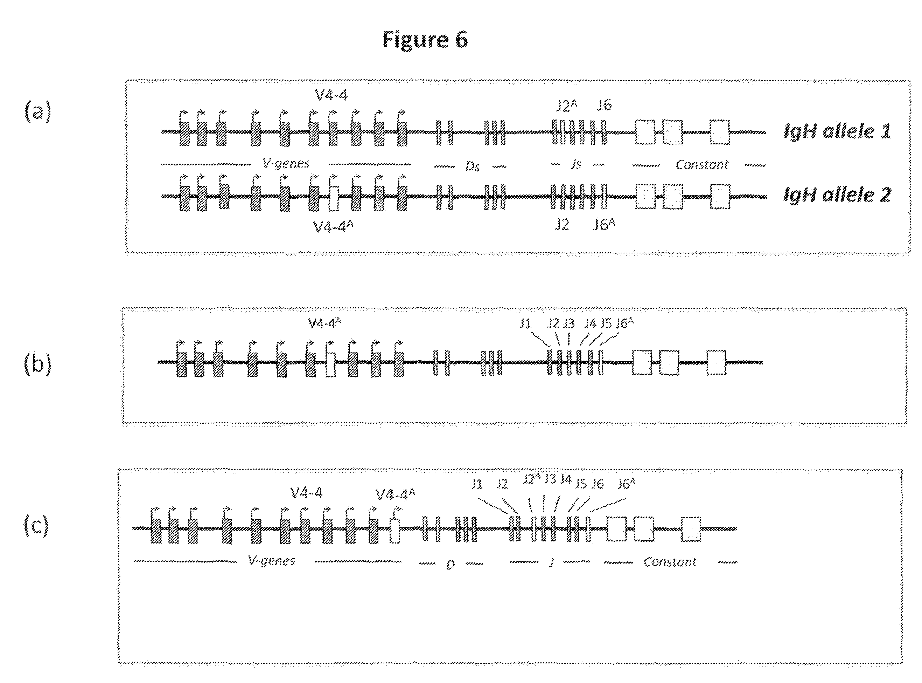

To go beyond the normal repertoire in humans and take advantage of combinations of C, V, D and J variants the inventors decided, in one embodiment, to provide these on the same chromosome in *cis.* See figure 6. These loci would be characterized by having more than the normal number of J, D or V genes. For example n=6 for the J genes, but including one J6 variant and one J2 variant would increase this to n=8. This could be combined with additional variants for the D and V genes, for example. By detailed analysis of the 1000- Genomes database, the inventors have devised a collection of candidate polymorphic human variant gene segments, eg, JH gene segments (eg, see the examples), that can be built into the design of transgenic heavy and light chain loci in mice for expressing increasingly diverse and new, synthetic repertoires of human variable regions. Moreover, by utilizing naturally-occurring human variant gene segments, as per embodiments of the invention, this addresses compatibility with human patients since the inventor's analysis has drawn out candidate variants that are naturally conserved and sometimes very prevalent amongst human ethnic populations. Additionally this enables one to tailor the configurations of the disclosure to provide for antibody-based drugs that better address specific human ethnic populations.

In an example according to any configuration of the disclosure, loci (and cells and vertebrates comprising these) are provided in which gene segments from different human populations are used. This is desirable to increase antibody gene diversity to better address more diverse human patients. In an example, the gene segments are from first and second different human populations respectively, and thus the second gene segment is found in the second human population, but not so (or rarely) in the first human population. Rarely means, for example, that the gene segment is found in 5, 4, 3, 2, or 1 or zero individuals in the first population in the 1000 Genomes database. For example, the first gene segment may be shown as present in a first population by reference to Table 13 or 14 herein, the second gene segment may be shown as present in the second population by reference to Table 13 and not in the first population. Optionally, the first gene segment may also be shown as being present in the second population by reference to Table 13 or 14.

In any configuration or aspect of the invention, where a V gene segment is used, this may be used optionally with the native leader sequence. For example, use of genomic DNA (eg, from BACs as in the examples) will mean that the native leader will be used for each V gene segment incorporated into the locus and genomes of the disclosure. In an alternative, the skilled person may wish to inert a non-native leader sequence together with one or more of the V gene segments. Similarly, in any configuration or aspect of the invention, where a V gene segment is used, this may be used optionally with the native 5' UTR sequence. For example, use of genomic DNA (eg, from BACs as in the examples) will mean that the native 5' UTR sequence will be used for each V gene segment incorporated into the locus and genomes of the disclosure. In an alternative, the skilled person may wish to exclude the native 5' UTR sequence.

### Disclosed herein is, in a first configuration

### (a) Superhuman heavy chain gene repertoires

A non-human vertebrate or vertebrate cell (optionally an ES cell or antibody-producing cell) comprising a genome having a superhuman immunoglobulin heavy chain human VH and/or D and/or J gene repertoire.

In one aspect the cell of the disclosure is an embryonic stem cell. For example, the ES cell is derived from the mouse C57BL/6N, C57BL/6J, 129S5 or 129Sv strain. In one aspect the non-human vertebrate is a rodent, suitably a mouse, and cells of the disclosure, are rodent cells or ES cells, suitably mouse ES cells. The ES cells of the present disclosure can be used to generate animals using techniques well known in the art, which comprise injection of the ES cell into a blastocyst followed by implantation of chimaeric blastocystys into females to produce offspring which can be bred and selected for homozygous recombinants having the required insertion. In one aspect the disclosure relates to a transgenic animal comprised of ES cell-derived tissue and host embryo derived tissue. In one aspect the disclosure relates to genetically-altered subsequent generation animals, which include animals having a homozygous recombinants for the VDJ and/or VJ regions.

The natural human immunoglobulin gene segment repertoire consists of (see eg, www.imgt.orq):-
VH: total-125 ; functional-41
DH: total-27; functional-23
JH: total-8; functional-6
Vk: total-77; functional-38
Jk: total-5; functional-5
V lambda: total-75; functional-31
J lambda: total-7; functional-5

In one embodiment, the vertebrate or cell genome comprises a transgenic immunoglobulin heavy chain locus comprising a plurality of human immunoglobulin VH gene segments, one or more human D gene segments and one or more human J gene segments, wherein the plurality of VH gene segments consists of more than the natural human repertoire of functional VH gene segments; optionally wherein the genome is homozygous for said transgenic heavy chain locus.

In one embodiment of the vertebrate or cell, the VH gene repertoire consists of a plurality of VH gene segments derived from the genome sequence of a first human individual, supplemented with one or more different VH gene segments derived from the genome sequence of a second, different human individual. Optionally the D and J segments are derived from the genome sequence of the first human individual. Optionally the VH gene segments from the genome sequence of the second individual are selected from the VH gene segments listed in Table 1, 13 or 14. In this way, the locus provides a superhuman repertoire of D gene segments.

Optionally the individuals are not related. Individuals are "not related" in the context of any configuration or aspect of the disclosure, for example, if one of the individuals does not appear in a family tree of the other individual in the same generation or going back one, two, three or four generations. Alternatively, are not related, for example, if they do not share a common ancestor in the present generation or going back one, two, three or four generations.

In one embodiment of the vertebrate or cell, the transgenic locus comprises more than 41 functional human VH gene segment species, and thus more than the natural human functional repertoire. Optionally the locus comprises at least 42, 43, 44, 45, 46, 47, 48, 49 or 50 functional human VH gene segment species (eg, wherein the locus comprises the full functional VH repertoire of said first individual supplemented with one or more VH gene segments derived from the genome sequence of the second human individual and optionally with one or more VH gene segments derived from the genome sequence of a third human individual). In this way, the locus provides a superhuman repertoire of VH gene segments that is useful for generating a novel gene and antibody diversity for use in therapeutic and tool antibody selection.

In one embodiment of the vertebrate or cell, the transgenic locus comprises a first VH gene segment derived from the genome sequence of the first individual and a second VH gene segment derived from the genome sequence of the second individual, wherein the second VH gene segment is a polymorphic variant of the first VH gene segment. For example, the VH gene segments are polymorphic variants of VH1-69 as illustrated in the examples below. Optionally the locus comprises a further polymorphic variant of the first VH gene segment (eg, a variant derived from the genome sequence of a third human individual). In this way, the locus provides a superhuman repertoire of VH gene segments.

In one embodiment of the vertebrate or cell, the genome (alternatively or additionally to the superhuman VH diversity) comprises a transgenic immunoglobulin heavy chain locus comprising a plurality of human immunoglobulin VH gene segments, a plurality of human D gene segments and one or more human J gene segments, wherein the plurality of D gene segments consists of more than the natural human repertoire of functional D gene segments. Optionally the genome is homozygous for said transgenic heavy chain locus.

In one embodiment of the vertebrate or cell, the D gene repertoire consists of a plurality of D gene segments derived from the genome sequence of a (or said) first human individual, supplemented with one or more different D gene segments derived from the genome sequence of a (or said) second, different human individual. Optionally the individuals are not related. Optionally the J segments are derived from the genome sequence of the first human individual. Optionally the D gene segments from the genome sequence of the second individual are selected from the D gene segments listed in Table 2, 13 or 14. In this way, the locus provides a superhuman repertoire of D gene segments.

In one embodiment of the vertebrate or cell, the transgenic locus comprises more than 23 functional human D gene segment species; optionally wherein the locus comprises at least 24, 25, 26, 27, 28, 29, 30 or 31 functional human D gene segment species (eg, wherein the locus comprises the full functional D repertoire of said first individual supplemented with one or more D gene segments derived from the genome sequence of the second human individual and optionally with one or more D gene segments derived from the genome sequence of a third human individual). In this way, the locus provides a superhuman repertoire of D gene segments.

In one embodiment of the vertebrate or cell, the transgenic locus comprises a first D gene segment derived from the genome sequence of the first individual and a second D gene segment derived from the genome sequence of the second individual, wherein the second D gene segment is a polymorphic variant of the first D gene segment. Optionally the locus comprises a further polymorphic variant of the first D gene segment (eg, a variant derived from the genome sequence of a third human individual). In this way, the locus provides a superhuman repertoire of D gene segments.

In one embodiment of the vertebrate or cell (alternatively or additionally to the superhuman VH and/or JH diversity), the genome comprises a (or said) transgenic immunoglobulin heavy chain locus comprising a plurality of human immunoglobulin VH gene segments, one or more human D gene segments and a plurality of human JH gene segments, wherein the plurality of J gene segments consists of more than the natural human repertoire of functional J gene segments; optionally wherein the genome is homozygous for said transgenic heavy chain locus.

In one embodiment of the vertebrate or cell, the JH gene repertoire consists of a plurality of J gene segments derived from the genome sequence of a (or said) first human individual, supplemented with one or more different J gene segments derived from the genome sequence of a (or said) second, different human individual. Optionally the individuals are not related. Optionally D segments are derived from the genome sequence of the first human individual. Optionally the J gene segments from the genome sequence of the second individual are selected from the J gene segments listed in Table 3 13 or 14. In this way, the locus provides a superhuman repertoire of JH gene segments.

In one embodiment of the vertebrate or cell, the transgenic locus comprises more than 6 functional human JH gene segment segments. Optionally the locus comprises at least 7, 8, 9, 10, 11, 12, 13, 14, 15, or 16 functional human JH gene segments (eg, wherein the locus comprises the full functional JH repertoire of said first individual supplemented with one or more JH gene segments derived from the genome sequence of the second human individual and optionally with one or more JH gene segments derived from the genome sequence of a third human individual). In this way, the locus provides a superhuman repertoire of JH gene segments.

In one embodiment of the vertebrate or cell, the transgenic locus comprises a first JH gene segment derived from the genome sequence of the first individual and a second JH gene segment derived from the genome sequence of the second individual, wherein the second JH gene segment is a polymorphic variant of the first JH gene segment. Optionally the locus comprises a further polymorphic variant of the first JH gene segment (eg, a variant derived from the genome sequence of a third human individual). In this way, the locus provides a superhuman repertoire of JH gene segments.

### (b) Superhuman light chain gene repertoires

### The first configuration of the disclosure also provides:-

A non-human vertebrate or vertebrate cell (optionally an ES cell or antibody-producing cell) comprising a genome having a superhuman immunoglobulin light chain human VL gene repertoire. Optionally the vertebrate or cell comprises a heavy chain transgene according to aspect (a) of the first configuration. Thus, superhuman diversity is provided in both the heavy and light chain immunoglobulin gene segments in the cell and vertebrate. For example, the genome of the cell or vertebrate is homozygous for the heavy and light chain transgenes and endogenous antibody expression is inactivated. Such a vertebrate is useful for immunisation with a predetermined antigen to produce one or more selected antibodies that bind the antigen and have human variable regions resulting from recombination within the superhuman gene segment repertoire. This provides potentially for a novel antibody and gene sequence space from which to select therapeutic, prophylactic and tool antibodies.

In one embodiment of aspect (b) of the first configuration, the vertebrate or cell genome comprises
(i) a transgenic immunoglobulin kappa light chain locus comprising a plurality of human immunoglobulin Vκ gene segments and one or more human J gene segments, wherein the plurality of Vκ gene segments consists of more than the natural human repertoire of functional Vκ gene segments; optionally wherein the genome is homozygous for said transgenic kappa light chain locus; and/or
(ii) a transgenic immunoglobulin lambda light chain locus comprising a plurality of human immunoglobulin Vλ gene segments and one or more human J gene segments, wherein the plurality of Vλ gene segments consists of more than the natural human repertoire of functional Vλ gene segments; optionally wherein the genome is homozygous for said transgenic lambda light chain locus.

In this way, the locus provides a superhuman repertoire of VL gene segments.
In one embodiment of the vertebrate or cell,
(i) the Vκ gene repertoire consists of a plurality of Vκ gene segments derived from the genome sequence of a first human individual, supplemented with one or more Vκ gene segments derived from the genome sequence of a second, different human individual; optionally wherein the individuals are not related; optionally wherein the J segments are derived from the genome sequence of the first human individual; and optionally wherein the Vκ gene segments from the genome sequence of the second individual are selected from the Vκ gene segments listed in Table 4, 13 or 14; and
(i) the Vλ gene repertoire consists of a plurality of Vλ gene segments derived from the genome sequence of a first human individual, supplemented with one or more Vλ gene segments derived from the genome sequence of a second, different human individual; optionally wherein the individuals are not related; optionally wherein the J segments are derived from the genome sequence of the first human individual; and optionally wherein the Vλ gene segments from the genome sequence of the second individual are selected from the Vλ gene segments listed in Table 5, 13 or 14.

In this way, the locus provides a superhuman repertoire of VL gene segments.

In one embodiment of the vertebrate or cell,
- the kappa light transgenic locus comprises more than 38 functional human Vκ gene segment species; optionally wherein the locus comprises at least 39, 40, 41, 42, 43, 44, 45, 46, 47 or 48 functional human Vκ gene segment species (eg, wherein the locus comprises the full functional Vκ repertoire of said first individual supplemented with one or more Vκ gene segments derived from the genome sequence of the second human individual and optionally with one or more Vκ gene segments derived from the genome sequence of a third human individual); and
- the lambda light transgenic locus comprises more than 31 functional human Vλ gene segment species; optionally wherein the locus comprises at least 32, 33, 34, 35, 36, 37, 38, 39, 40 or 41 functional human Vλ gene segment species (eg, wherein the locus comprises the full functional Vλ repertoire of said first individual supplemented with one or more Vλ gene segments derived from the genome sequence of the second human individual and optionally with one or more Vλ gene segments derived from the genome sequence of a third human individual).

In this way, the locus provides a superhuman repertoire of VL gene segments.

In one embodiment of the vertebrate or cell,
- the kappa light transgenic locus comprises a first Vκ gene segment derived from the genome sequence of the first individual and a second Vκ gene segment derived from the genome sequence of the second individual, wherein the second Vκ gene segment is a polymorphic variant of the first Vκ gene segment; optionally wherein the locus comprises a further polymorphic variant of the first Vκ gene segment (eg, a variant derived from the genome sequence of a third human individual); and
- the lambda light transgenic locus comprises a first Vλ gene segment derived from the genome sequence of the first individual and a second Vλ gene segment derived from the genome sequence of the second individual, wherein the second Vλ gene segment is a polymorphic variant of the first Vλ gene segment; optionally wherein the locus comprises a further polymorphic variant of the first Vλ gene segment (eg, a variant derived from the genome sequence of a third human individual).
In this way, the locus provides a superhuman repertoire of VL gene segments.

In one embodiment of the vertebrate or cell, the genome comprises a (or said) transgenic immunoglobulin light chain locus comprising a plurality of human immunoglobulin VL gene segments and a plurality of human JL gene segments, wherein the plurality of J gene segments consists of more than the natural human repertoire of functional J gene segments; optionally wherein the genome is homozygous for said transgenic heavy chain locus.

In one embodiment of the vertebrate or cell,
(i) the Jκ gene repertoire consists of a plurality of Jκ gene segments derived from the genome sequence of a (or said) first human individual, supplemented with one or more Jκ gene segments derived from the genome sequence of a (or said) second, different human individual; optionally wherein the individuals are not related; optionally wherein the Vκ segments are derived from the genome sequence of the first human individual; optionally wherein the Jκ gene segments from the genome sequence of the second individual are selected from the Jκ gene segments listed in Table 6, 13 or 14;and
(ii) the Jκ gene repertoire consists of a plurality of Jλgene segments derived from the genome sequence of a (or said) first human individual, supplemented with one or more Jλ gene segments derived from the genome sequence of a (or said) second, different human individual; optionally wherein the individuals are not related; optionally wherein the Vλ segments are derived from the genome sequence of the first human individual; optionally wherein the Jλ gene segments from the genome sequence of the second individual are selected from the Jλ gene segments listed in Table 7, 13 or 14.

In this way, the locus provides a superhuman repertoire of JL gene segments.

In one embodiment of the vertebrate or cell,
(i) the transgenic light chain locus comprises more than 5 functional human Jκ gene segment species; optionally wherein the locus comprises at least 6, 7, 8, 9, 10, 11, 12, 13, 14 or 15 functional human Jκ gene segment species (eg, wherein the locus comprises the full functional Jκ repertoire of said first individual supplemented with one or more Jκ gene segments derived from the genome sequence of the second human individual and optionally with one or more Jκ gene segments derived from the genome sequence of a third human individual); and/or
(i) the transgenic light chain locus comprises more than 5 functional human Jλ gene segment species; optionally wherein the locus comprises at least 6, 7, 8, 9, 10, 11, 12, 13, 14 or 15 functional human Jλ gene segment species (eg, wherein the locus comprises the full functional Jλ repertoire of said first individual supplemented with one or more Jλ gene segments derived from the genome sequence of the second human individual and optionally with one or more Jλ gene segments derived from the genome sequence of a third human individual).

In this way, the locus provides a superhuman repertoire of JL gene segments.

In one embodiment of the vertebrate or cell,
(i) the kappa light transgenic locus comprises a first Jκ gene segment derived from the genome sequence of the first individual and a second Jκ gene segment derived from the genome sequence of the second individual, wherein the second Jκ gene segment is a polymorphic variant of the first Jκ gene segment; optionally wherein the locus comprises a further polymorphic variant of the first Jκ gene segment (eg, a variant derived from the genome sequence of a third human individual); and
(ii) the lambda light transgenic locus comprises a first Jλ gene segment derived from the genome sequence of the first individual and a second Jλ gene segment derived from the genome sequence of the second individual, wherein the second Jκ gene segment is a polymorphic variant of the first Jλ gene segment; optionally wherein the locus comprises a further polymorphic variant of the first Jλ gene segment (eg, a variant derived from the genome sequence of a third human individual).

In this way, the locus provides a superhuman repertoire of JL gene segments.

Further aspects of the first configuration are described below.

### Disclosed herein is, in a second configuration

A library of antibody-producing transgenic cells whose genomes collectively encode a repertoire of antibodies, wherein
(a) a first transgenic cell expresses a first antibody having a chain (eg, heavy chain) encoded by a first immunoglobulin gene, the gene comprising a first variable domain nucleotide sequence produced following recombination of a first human unrearranged immunoglobulin gene segment (eg, a VH);
(b) a second transgenic cell expresses a second antibody having a chain (eg, a heavy chain) encoded by a second immunoglobulin gene, the second gene comprising a second variable domain nucleotide sequence produced following recombination of a second human unrearranged immunoglobulin gene segment (eg, a VH), the first and second antibodies being non-identical;
(c) the first and second gene segments are different and derived from the genome sequences of first and second human individuals respectively, wherein the individuals are different; and optionally not related;
(d) wherein the cells are non-human vertebrate (eg, mouse or rat) cells (eg, B-cells or hybridomas).

In one embodiment, the library is provided in vitro. In another embodiment, the library is provided in vivo by one or a plurality of transgenic non-human vertebrates. For example, the or each vertebrate is according to any aspect of the first configuration of the disclosure.

In one embodiment, the library encodes an antibody repertoire of from 10 to 10⁹ antibodies, for example, 10, 20, 30, 40, 50, 100 or 1000 to 10⁸; or 10, 20, 30, 40, 50, 100 or 1000 to 10⁷; or 10, 20, 30, 40, 50, 100 or 1000 to 10⁶; or 10, 20, 30, 40, 50, 100 or 1000 to 10⁵; or 10, 20, 30, 40, 50, 100 or 1000 to 10⁴ antibodies. In an example, library encodes an antibody repertoire of at least 10³, 10⁴, 10⁵, 10⁶, 10⁷, 10⁸, 10⁹, or 10¹⁰ antibodies.

The first variable domain nucleotide sequence is produced following recombination of the first human unrearranged immunoglobulin gene segment with one or more other immunoglobulin gene segments (for example, human immunoglobulin gene segments). For example, where the first gene segment is a VH, the first variable domain nucleotide sequence (a VH domain) is produced following recombination of the VH with a human D and JH segments in vivo, optionally with somatic hypermutation, in the first transgenic cell or an ancestor thereof. For example, where the first gene segment is a VL, the first variable domain nucleotide sequence (a VL domain) is produced following recombination of the VL with a human JL segment in vivo, optionally with somatic hypermutation, in the first transgenic cell or an ancestor thereof.

The second variable domain nucleotide sequence is produced following recombination of the second human unrearranged immunoglobulin gene segment with one or more other immunoglobulin gene segments (for example, human immunoglobulin gene segments). For example, where the second gene segment is a VH, the second variable domain nucleotide sequence (a VH domain) is produced following recombination of the VH with a human D and JH segments in vivo, optionally with somatic hypermutation, in the second transgenic cell or an ancestor thereof. For example, where the second gene segment is a VL, the second variable domain nucleotide sequence (a VL domain) is produced following recombination of the VL with a human JL segment in vivo, optionally with somatic hypermutation, in the second transgenic cell or an ancestor thereof.

The first and second gene segments are respectively derived from genome sequences of first and second human individuals. In one example, such a gene segment is isolated or cloned from a sample cell taken from said individual using standard molecular biology techniques as known to the skilled person. The sequence of the gene segment may be mutated (eg, by the introduction of up to 5, 6, 7, 8, 9, 10, 11, 12, 13, 14 or 15 nucleotide changes) prior to use in the present disclosure. In another example, a gene segment is derived by identifying a candidate human immunoglobulin gene segment in a database (see guidance below) and a nucleotide sequence encoding a gene segment for use in the present disclosure is made by reference (eg, to be identical or a mutant with up to 5, 6, 7, 8, 9, 10, 11, 12, 13, 14 or 15 nucleotide changes to the reference sequence) to the database sequence. The skilled person will be aware of methods of obtaining nucleotide sequences by reference to databases or by obtaining from cellular samples.

In one embodiment of the vertebrate, cell or library of any configuration of the disclosure, the first and second human individuals are members of first and second ethnic populations respectively, wherein the populations are different. This, therefore, provides for superhuman gene diversity in transgenic loci, cells and vertebrates as per the disclosure.

### Human Populations

Optionally the ethnic populations are selected from those identified in the 1000 Genomes Project of database. In this respect, see Table 8 which provides details of the ethnic populations on which the 1000 Genomes database is based.

N A Rosenberg et al (Science 20 December 2002: vol. 298 no. 5602 2342-2343) studied the genetic structure of human populations of differing geographical ancestry. In total, 52 populations were sampled, these being populations with:
*African ancestry*
   (Mbuti Pygmies, Biaka Pygmies, San peoples, and speakers of Niger-Kordofanian languages (Bantu, Yoruba or Mandenka populations),
*Eurasian ancestry*
   (European ancestry (Orcadian, Adygei, Basque, French, Russians, Italians, Sardinian, Tuscan), Middle Eastern ancestry (Mozabite, Bedouin, Druze, Palestinians),
   Central/South Asian ancestry (Balochl, Brahul, Makrani, Sindhi, Pathan, Burusho, Hazara, Uygur, Kalash)),
*East Asian ancestry*
   (Han, Dal, Daur, Hezhen, Lahu, Miao, Oroqen, She, Tujia, Tu, Xibo, Yi, Mongola, Naxi, Cambodian, Japanese, Yakut), Oceanic ancestry (Melanesian, Papuan); or
*Americas ancestry*
   (Karitiana, Surui, Colombian, Maya, Pima).

The International HapMap Project, Nature, 2003 Dec 18;426(6968):789-96, discloses that goal of the HapMap Project: to determine the common patterns of DNA sequence variation in the human genome by determining the genotypes of one million or more sequence variants, their frequencies and the degree of association between them in DNA samples from populations with ancestry from parts of Africa, Asia and Europe. The relevant human populations of differing geographical ancestry include Yoruba, Japanese, Chinese, Northern European and Western European populations. More specifically:-

Utah population with Northern or Western European ancestry (samples collected in 1980 by the Centre d'Etude du Polymorphisme Humain (CEPH));
population with ancestry of Yoruba people from Ibadan, Nigeria;
population with Japanese ancestry; and
population with ancestry of Han Chinese from China.

The authors, citing earlier publications, suggest that ancestral geography is a reasonable basis for sampling human populations.

A suitable sample of human populations from which the populations used in the present disclosure are selected is as follows:-
(a) *European **ancestry***
(b) Northern European ancestry; Western European ancestry; Toscani ancestry; British ancestry, Finnish ancestry or Iberian ancestry.
(c) More specifically, population of Utah residents with Northern and/or Western European ancestry; Toscani population in Italia; British population in England and/or Scotland; Finnish population in Finland; or Iberian population in Spain.

(a) *East Asian **ancestry***
(b) Japanese ancestry; Chinese ancestry or Vietnamese ancestry.
(c) More specifically, Japanese population in Tokyo, Japan; Han Chinese population in Beijing, China; Chinese Dai population in Xishuangbanna; Kinh population in Ho Chi Minh City, Vietnam; or Chinese population in Denver, Colorado, USA.

(a) ***West African ancestry***
(b) Yoruba ancestry; Luhya ancestry; Gambian ancestry; or Malawian ancestry.
(c) More specifically, Yoruba population in Ibadan, Nigeria; Luhya population in Webuye, Kenya; Gambian population in Western Division, The Gambia; or Malawian population in Blantyre, Malawi.

(a) ***Population of The Americas***
(b) Native American ancestry; Afro-Caribbean ancestry; Mexican ancestry; Puerto Rican ancestry; Columbian ancestry; or Peruvian ancestry.
(c) More specifically, population of African Ancestry in Southwest US; population of African American in Jackson, MS; population of African Caribbean in Barbados; population of Mexican Ancestry in Los Angeles, CA; population of Puerto Rican in Puerto Rico; population of Colombian in Medellin, Colombia; or population of Peruvian in Lima, Peru.

(a) ***South Asian ancestry***
(b) Ahom ancestry; Kayadtha ancestry; Reddy ancestry; Maratha; or Punjabi ancestry.
(c) More specifically, Ahom population in the State of Assam, India; Kayadtha population in Calcutta, India; Reddy population in Hyderabad, India; Maratha population in Bombay, India; or Punjabi population in Lahore, Pakistan.

In any configuration of the disclosure, in one embodiment, each human population is selected from a population marked "(a)" above.

In any configuration of the disclosure, in another embodiment, each human population is selected from a population marked "(b)" above.

In any configuration of the disclosure, in another embodiment, each human population is selected from a population marked "(c)" above.

In one embodiment of the library of the vertebrate, cell or library of the disclosure, the first and second ethnic populations are selected from the group consisting of an ethnic population with European ancestry, an ethnic population with East Asian, an ethnic population with West African ancestry, an ethnic population with Americas ancestry and an ethnic population with South Asian ancestry.

In one embodiment of the library of the vertebrate, cell or library of the disclosure, the first and second ethnic populations are selected from the group consisting of an ethnic population with Northern European ancestry; or an ethnic population with Western European ancestry; or an ethnic population with Toscani ancestry; or an ethnic population with British ancestry; or an ethnic population with Icelandic ancestry; or an ethnic population with Finnish ancestry; or an ethnic population with Iberian ancestry; or an ethnic population with Japanese ancestry; or an ethnic population with Chinese ancestry; or an ethnic population Vietnamese ancestry; or an ethnic population with Yoruba ancestry; or an ethnic population with Luhya ancestry; or an ethnic population with Gambian ancestry; or an ethnic population with Malawian ancestry; or an ethnic population with Native American ancestry; or an ethnic population with Afro-Caribbean ancestry; or an ethnic population with Mexican ancestry; or an ethnic population with Puerto Rican ancestry; or an ethnic population with Columbian ancestry; or an ethnic population with Peruvian ancestry; or an ethnic population with Ahom ancestry; or an ethnic population with Kayadtha ancestry; or an ethnic population with Reddy ancestry; or an ethnic population with Maratha; or an ethnic population with Punjabi ancestry.

In one embodiment of any configuration of the vertebrate, cell or library of the disclosure, the human immunoglobulin gene segment derived from the genome sequence of the second individual is low-frequency (optionally rare) within the second ethnic population. Optionally human immunoglobulin gene segment has a Minor Allele Frequency (MAF) (cumulative frequency) of between 0.5% - 5%, optionally less than 0.5%, in the second human population, eg, as in the 1000 Genomes database.

In one embodiment of any configuration of the vertebrate, cell or library of the disclosure, the first variable region nucleotide sequence is produced by recombination of the first human immunoglobulin gene segment with a first J gene segment and optionally a first D gene segment, wherein the first human immunoglobulin gene segment is a V gene segment and the V, D and J segments are derived from the first human population, optionally from the genome of one individual of the first human population.

In one embodiment of the library of the vertebrate, cell or library of the disclosure, the second variable region nucleotide sequence is produced by recombination of the second human immunoglobulin gene segment with a second J gene segment and optionally a second D gene segment, wherein the second human immunoglobulin gene segment is a V gene segment derived from the second population and the D and/or J segments are derived from the first human population, optionally the D and J gene segments being from the genome of one individual of the first human population.

In one embodiment of the library of the vertebrate, cell or library of the disclosure, all of the D and J segments that have been recombined with the first and second V gene segments are D and J segments derived from the first human population, optionally the D and J gene segments being from the genome of one individual of the first human population.

In one embodiment of the library, the second human immunoglobulin gene segment is a polymorphic variant of the first human immunoglobulin gene segment; optionally wherein the second gene segment is selected from the group consisting of a gene segment in any of Tables 1 to 7 and 9 to 14 (eg, selected from Table 13 or 14).

In one embodiment of the library, the first and second human immunoglobulin gene segments are both (i) VH gene segments; (ii) D segments; (iii) J segments (optionally both J_{H} segments, both J_{κ} segments or both J_{λ} segments); (iv) constant regions (optionally both a gamma constant region, optionally both a C gamma-1 constant region); (v) CH1 regions; (vi) CH2 regions; or (vii) CH3 regions.

The library is, for example, a naive and optionally has a library size of from 10 or 10² to 10⁹ cells. For example, from 10, 20, 30, 40, 50, 100 or 1000 to 10⁸; or 10, 20, 30, 40, 50, 100 or 1000 to 10⁷; or 10, 20, 30, 40, 50, 100 or 1000 to 10⁶; or 10, 20, 30, 40, 50, 100 or 1000 to 10⁵; or 10, 20, 30, 40, 50, 100 or 1000 to 10⁴ cells.

The library has, for example, been selected against a predetermined antigen and optionally has a library size of from 10 or 10² to 10⁹ cells. For example, from 10, 20, 30, 40, 50, 100 or 1000 to 10⁸; or 10, 20, 30, 40, 50, 100 or 1000 to 10⁷; or 10, 20, 30, 40, 50, 100 or 1000 to 10⁶; or 10, 20, 30, 40, 50, 100 or 1000 to 10⁵; or 10, 20, 30, 40, 50, 100 or 1000 to 10⁴ cells.

In one embodiment of the library of the disclosure, said first and second cells are progeny of first and second ancestor non-human vertebrate cells respectively, wherein the first ancestor cell comprises a genome comprising said first human immunoglobulin gene segment; and the second ancestor cell comprises a genome comprising said second human immunoglobulin gene segment.

The disclosure further provides a library of antibody-producing transgenic cells whose genomes collectively encode a repertoire of antibodies, wherein the library comprises the first and second ancestor cells described above.

The disclosure further provides a library of hybridoma cells produced by fusion of the library of the disclosure (eg, a B-cell library) with fusion partner cells and optionally has a library size of from 10 or 102 to 109 cells. For example, from 10, 20, 30, 40, 50, 100 or 1000 to 10⁸; or 10, 20, 30, 40, 50, 100 or 1000 to 10⁷; or 10, 20, 30, 40, 50, 100 or 1000 to 10⁶; or 10, 20, 30, 40, 50, 100 or 1000 to 10⁵; or 10, 20, 30, 40, 50, 100 or 1000 to 10⁴ cells. Production of hybridomas is well known to the skilled person. Examples of fusion partners are SP2/0-g14 (obtainable from ECACC), P3XS3-Ag8.S53 (obtainable from LGC Standards; CRL-1580), NS1 and NS0 cells. PEG fusion or electrofusion can be carried out, as is conventional.

### The disclosure provides, in a third configuration:-

An isolated antibody having
(a) a heavy chain encoded by a nucleotide sequence produced following recombination in a transgenic non-human vertebrate cell of an unrearranged human immunoglobulin V gene segment with a human D and human J segment, optionally with affinity maturation in said cell, wherein one of the gene segments (eg, VH) is derived from the genome of an individual from a first human ethnic population; and the other two gene segments (eg, D and JH) are derived from the genome of an individual from a second (eg, a second and third respectively), different, human ethnic population,
   and wherein the antibody comprises heavy chain constant regions (eg, C gamma) of said non-human vertebrate (eg, rodent, mouse or rat heavy chain constant regions); and/or
(b) a light chain encoded by a nucleotide sequence produced following recombination in a transgenic non-human vertebrate cell of an unrearranged human immunoglobulin V gene segment with a human J segment, optionally with affinity maturation in said cell, wherein one of the gene segments (eg, VL) is derived from the genome of an individual from a first human ethnic population (optionally the same as the first population in (a)); and the other gene segment (eg, JL) is derived from the genome of an individual from a second, different, human ethnic population (optionally the same as the second population in (a)), and wherein the antibody comprises light chain constant regions of said non-human vertebrate (eg, rodent, mouse or rat heavy light constant regions);
(c) Optionally wherein each variable domain of the antibody is a human variable domain.
(d) Optionally wherein the heavy chain constant regions are mu- or gamma-type constant regions.

The disclosure also provides an isolated nucleotide sequence encoding the antibody of the third configuration, optionally wherein the sequence is provided in an antibody expression vector, optionally in a host cell. Suitable vectors are mammalian expression vectors (eg, CHO cell vectors or HEK293 cell vectors), yeast vectors (eg, a vector for expression in Picchia pastoris, or a bacterial expression vector, eg, a vector for E. coli expression.

The invention also provides a method of producing a human antibody, the method comprising replacing the non-human vertebrate constant regions of the antibody of the third configuration with human antibody constant regions (eg, a C variant disclosed in table 13 or 18). The skilled person will be aware of standard molecular biology techniques to do this. For example, see Harlow, E. & Lane, D. 1998, 5th edition, Antibodies: A Laboratory Manual, Cold Spring Harbor Lab. Press, Plainview, NY; and Pasqualini and Arap, Proceedings of the National Academy of Sciences (2004) 101:257-259 for standard immunisation. Joining of the variable regions of an antibody to a human constant region can be effected by techniques readily available in the art, such as using conventional recombinant DNA and RNA technology as will be apparent to the skilled person. See e.g. Sambrook, J and Russell, D. (2001, 3'd edition) Molecular Cloning: A Laboratory Manual (Cold Spring Harbor Lab. Press, Plainview, NY).

In one embodiment, the method comprises further making a mutant or derivative of the antibody.

The disclosure also provides a pharmaceutical composition comprising an antibody according to the third configuration, or a human antibody produced according to the invention and a diluent, excipient or carrier; optionally wherein the composition is provided in a container connected to an IV needle or syringe or in an IV bag.

The disclosure also provides an antibody-producing cell (eg, a mammalian cell, eg, CHO or HEK293; a yeast cell, eg, *P pastoris;* a bacterial cell, eg, *E coli*; a B-cell; or a hybridoma) that expresses the second antibody of the third configuration or the isolated antibody produced according to the invention.

### The first configuration of the disclosure also provides:-

A non-human vertebrate or vertebrate cell (optionally an ES cell or antibody-producing cell) whose genome comprises a transgenic immunoglobulin locus (eg, a heavy chain locus or a light chain locus), said locus comprising immunoglobulin gene segments according to the first and second human immunoglobulin gene segments (optionally V segments) described above in connection with the third configuration. The gene segments are operably connected upstream of an immunoglobulin constant region; optionally wherein the genome is homozygous for said transgenic immunoglobulin locus.

Optionally the immunoglobulin locus comprises more than the natural human complement of functional V gene segments; and/or

Optionally wherein the immunoglobulin locus comprises more than the natural human complement of functional D gene segments; and/or

Optionally wherein the immunoglobulin locus comprises more than the natural human complement of functional J gene segments.

In this way, a superhuman immunoglobulin gene repertoire is provided in a transgenic non-human vertebrate or vertebrate cell according to the disclosure.

### The first configuration also provides:-

A transgenic non-human vertebrate (eg, a mouse or rat) or vertebrate cell (optionally an ES cell or antibody-producing cell) whose genome comprises a transgenic immunoglobulin locus comprising a plurality of human immunoglobulin gene segments operably connected upstream of a non-human vertebrate constant region for the production of a repertoire of chimaeric antibodies, or chimaeric light or heavy chains, having a non-human vertebrate constant region and a human variable region; wherein the transgenic locus comprises one or more human immunoglobulin V gene segments, one or more human J gene segments and optionally one or more human D gene segments, a first (optionally a V segment) of said gene segments and a second (optionally a V segment) of said gene segments being different and derived from the genomes of first and second human individuals respectively, wherein the individuals are different; and optionally not related;
optionally wherein the immunoglobulin locus comprises more than the natural human complement of functional V gene segments; and/or
optionally wherein the immunoglobulin locus comprises more than the natural human complement of functional D gene segments; and/or
optionally wherein the immunoglobulin locus comprises more than the natural human complement of functional J gene segments.

In this way, a superhuman immunoglobulin gene repertoire is provided in a transgenic non-human vertebrate or vertebrate cell according to the disclosure.

### The first configuration also provides:-

A transgenic non-human vertebrate (eg, a mouse or rat) or vertebrate cell (optionally an ES cell or antibody-producing cell) whose genome comprises first and second transgenic immunoglobulin loci, each locus comprising a plurality of human immunoglobulin gene segments operably connected upstream of a non-human vertebrate constant region for the production of a repertoire of chimaeric antibodies, or chimaeric light or heavy chains, having a non-human vertebrate constant region and a human variable region;
wherein (i) the first transgenic locus comprises one or more human immunoglobulin V gene segments, one or more human J gene segments and optionally one or more human D gene segments, (ii) the second transgenic locus comprises one or more human immunoglobulin V gene segments, one or more human J gene segments and optionally one or more human D gene segments; and (iii) wherein a first (optionally a V) gene segment of said first locus and a second (optionally a V) gene segment of said second gene locus are different and derived from the genomes of first and second human individuals respectively, wherein the individuals are different; and optionally not related;
optionally wherein the first and second loci are on different chromosomes (optionally chromosomes with the same chromosome number) in said genome;
optionally wherein each immunoglobulin locus comprises more than the natural human complement of functional V gene segments; and/or
optionally wherein each immunoglobulin locus comprises more than the natural human complement of functional D gene segments; and/or
optionally wherein each immunoglobulin locus comprises more than the natural human complement of functional J gene segments.

In this way, a superhuman immunoglobulin gene repertoire is provided in a transgenic non-human vertebrate or vertebrate cell according to the disclosure.

In these embodiments of the first configuration, the immunoglobulin gene segments are optionally as described for the third configuration.

In these embodiments of the first configuration, the genome optionally comprises a third immunoglobulin gene segment (optionally a V segment), the third gene segment being derived from a human individual that is different from the individual from which the first (and optionally also the second) gene segment is derived; optionally wherein the first, second and third gene segments are polymorphic variants of a human immunoglobulin gene segment (eg, VH1-69 - see the examples for further description).

In these embodiments of the first configuration, the genome of the vertebrate or cell is optionally homozygous for the first, second and optional third gene segment, wherein a copy of the first, second and optional third gene segments are provided together on the same chromosome operably connected upstream of a common non-human vertebrate constant region.

For example, each first, second and optional third gene segment is a V gene segment.

In one example, the library of the disclosure is provided by a collection of non-human vertebrates (optionally a collection of rodents, mice or rats); optionally, wherein a first member of said collection produces said first antibody but not said second antibody, and a second member of the collection produces said second antibody (but optionally not said first antibody). It is therefore contemplated to make non-human vertebrates where different human genomes have been used as a source for building the transgenic loci in the vertebrates. For example, a first vertebrate comprises a transgenic heavy chain locus having gene segments only from a first (and optionally a second) human population or individual; a second vertebrate comprises a transgenic heavy chain locus having gene segments only from a third (and optionally a fourth) human population or individual; and optionally third and more vertebrates can be built similarly based on unique or overlapping human population genomes. However, when provided as a mixed population of transgenic vertebrates, the mixed population provides a collective pool of human immunoglobulin genes that is greater than found in a natural human repertoire. This is useful to extend the antibody and gene sequence space beyond those possible with prior transgenic mice and rats bearing human immunoglobulin loci. As explained above, these have been based on a single human genome.

In one embodiment, the collection of non-human vertebrates bear human immunoglobulin genes confined to human populations that are together grouped under the same population genus "(a)" mentioned above. This provides for a gene repertoire that is biased to producing human antibody variable regions prevalent in the population genus (a) and thus useful for generating antibody therapeutics/prophylactics for members of said population. Alternatively, where gene segments from different human populations are provided in a single transgene according to the disclosure (not necessarily in a collection of vertebrates), the different human populations are for example together grouped under the same population genus "(a)" mentioned above.

The disclosure also provides a repertoire of antibodies expressed from a library of cells according to the disclosure.

In the non-human vertebrate or cell of any configuration of the disclosure, the constant region of the transgenic locus is, in one example, an endogenous constant region of said vertebrate (eg, endogenous mouse or rat constant region, eg, from the same strain of mouse or rat as the non-human vertebrate itself).

The disclosure also provides a method of constructing a cell (eg, an ES cell) according to the invention, the method comprising
(a) identifying functional V and J (and optionally D) gene segments of the genome sequence of a (or said) first human individual;
(b) identifying one or more functional V and/or D and/or J gene segments of the genome sequence of a (or said) second human individual, wherein these additional gene segments are not found in the genome sequence of the first individual;
(c) and constructing a transgenic immunoglobulin locus in the cell, wherein the gene segments of (a) and (b) are provided in the locus operably connected upstream of a constant region.

Optionally the cell comprises a heavy chain locus constructed according to steps (a) to (c) and/or a light chain locus (kappa and/or lambda loci) constructed according to steps (a) to (c).

Optionally the cell is homozygous for the or each transgenic locus; optionally wherein antibody expression from loci endogenous to said cell has been inactivated. This is useful for confining the functional antibody gene repertoire, and thus antibody production, to antibodies bearing human variable regions.

Optionally the gene segment(s) in step (b) are identified from an immunoglobulin gene database selected from the 1000 Genomes, Ensembl, Genbank and IMGT databases.

Optionally the first and second human individuals are members of first and second ethnic populations respectively, wherein the populations are different, optionally wherein the human immunoglobulin gene segment derived from the genome sequence of the second individual is low- frequency (optionally rare) within the second ethnic population.

The disclosure also provides a method of making a transgenic non-human vertebrate (eg, a mouse or rat), the method comprising
(a) constructing an ES cell (eg, a mouse C57BL/6N, C57BL/6J, 129S5 or 129Sv strain ES cell) by carrying out the method above;
(b) injecting the ES cell into a donor non-human vertebrate blastocyst (eg, a mouse C57BL/6N, C57BL/6J, 129S5 or 129Sv strain blastocyst);
(c) implanting the blastocyst into a foster non-human vertebrate mother (eg, a C57BL/6N, C57BL/6J, 129S5 or 129Sv strain mouse); and
(d) obtaining a child from said mother, wherein the child genome comprises a transgenic immunoglobulin locus.

The disclosure provides a transgenic non-human vertebrate (eg, a mouse or rat) made by the method or a progeny thereof. The disclosure also provides a population of such non-human vertebrates.

Microinjection of ES cells into blastocysts and generation of transgenic mice thereafter are conventional practices in the state of the art, and the skilled person is aware of techniques useful to effect this. C57BL/6N, C57BL/6J, 129S5 or 129Sv mouse strains and ES cells are readily and publicly available.

The invention also provides a method of isolating an antibody that binds a predetermined antigen (eg, a bacterial or viral pathogen antigen), the method comprising
(a) providing a vertebrate (optionally a mouse or rat) according to claim 1;
(b) immunising (eg, using a standard prime-boost method) said vertebrate with said antigen (optionally wherein the antigen is an antigen of an infectious disease pathogen);
(c) removing B lymphocytes from the vertebrate and selecting one or more B lymphocytes expressing antibodies that bind to the antigen;
(d) optionally immortalising said selected B lymphocytes or progeny thereof, optionally by producing hybridomas therefrom; and
(e) isolating an antibody (eg, and IgG-type antibody) expressed by the B lymphocytes; and
(f) optionally producing a derivative or variant of the antibody.

This method optionally further comprises after step (e) the step of isolating from said B lymphocytes nucleic acid encoding said antibody that binds said antigen; optionally exchanging the heavy chain constant region nucleotide sequence of the antibody with a nucleotide sequence encoding a human or humanised heavy chain constant region and optionally affinity maturing the variable region of said antibody; and optionally inserting said nucleic acid into an expression vector and optionally a host.

### Bioinformatics Analysis & Selection of Immunoglobulin Gene Segments

See also the discussion on variation analysis above.

The skilled person will know of sources of human antibody gene sequences, such as IMGT (www.imgt.org), GenBank (www.ncbi.nlm.nih.gov/genbank) Bioinformatics tools for database manipulation are also readily available and known to the skilled person, eg, as publicly available from the 1000 Genomes Project/EBI (www.1000genomes.org)

As a source of antibody gene segment sequences, the skilled person will also be aware of the following available databases and resources (including updates thereof):-
1.1. The Kabat Database (G. Johnson and T. T.Wu, 2002; http://www.kabatdatabase.com). Created by E. A. Kabat and T. T. Wu in 1966, the Kabat database publishes aligned sequences of antibodies, T-cell receptors, major histocompatibility complex (MHC) class I and II molecules, and other proteins of immunological interest. A searchable interface is provided by the Seqhuntll tool, and a range of utilities is available for sequence alignment, sequence subgroup classification, and the generation of variability plots. See also Kabat, E. A.,Wu, T. T., Perry, H., Gottesman, K., and Foeller, C. (1991) Sequences of Proteins of Immunological Interest, 5th ed., NIH Publication No. 91- 3242,Bethesda, MD, in particular with reference to human gene segments for use in the present invention.
1.2. KabatMan (A. C. R. Martin, 2002; http://www.bioinf.org.uk/abs/simkab.html). This is a web interface to make simple queries to the Kabat sequence database.
1.3. *IMGT, the International ImMunoGeneTics Information System*®; M.-P. Lefranc, 2002; http://imgt.cines.fr). IMGT is an integrated information system that specializes in antibodies, T cell receptors, and MHC molecules of all vertebrate species. It provides a common portal to standardized data that include nucleotide and protein sequences, oligonucleotide primers, gene maps, genetic polymorphisms, specificities, and two-dimensional (2D) and three-dimensional (3D) structures. IMGT includes three sequence databases (IMGT/LIGM-DB, IMGT/MHC-DB, IMGT/PRIMERDB), one genome database (IMGT/GENE-DB), one 3D structure database (IMGT/3Dstructure-DB), and a range of web resources ("IMGT Marie-Paule page") and interactive tools.
1.4. *V-BASE* (I. M. Tomlinson, 2002;http://www.mrc-cpe.cam.ac.uk/vbase). V-BASE is a comprehensive directory of all human antibody germline variable region sequences compiled from more than one thousand published sequences. It includes a version of the alignment software DNAPLOT (developed by Hans-Helmar Althaus and Werner Muller) that allows the assignment of rearranged antibody V genes to their closest germline gene segments.
1.5. *Antibodies-Structure and Sequence(A.* C. R. Martin, 2002; http://www.bioinf.org.uk/abs). This page summarizes useful information on antibody structure and sequence. It provides a query interface to the Kabat antibody sequence data, general information on antibodies, crystal structures, and links to other antibody-related information. It also distributes an automated summary of all antibody structures deposited in the Protein Databank (PDB). Of particular interest is a thorough description and comparison of the various numbering schemes for antibody variable regions.
1.6. *AAAAA-AHo's Amazing Atlas of Antibody Anatomy* (A. Honegger, 2001; http://www.unizh.ch/∼antibody). This resource includes tools for structural analysis, modelling, and engineering. It adopts a unifying scheme for comprehensive structural alignment of antibody and T- cell-receptor sequences, and includes Excel macros for antibody analysis and graphical representation.
1.7. *WARM- Web Antibody Modeling* (N. Whitelegg and A. R. Rees, 2001; http://antibody.bath.ac.uk). Hosted by the Centre for Protein Analysis and Design at the University of Bath, United Kingdom. Based on the AbM package (formerly marketed by Oxford Molecular) to construct 3D models of antibody Fv sequences using a combination of established theoretical methods, this site also includes the latest antibody structural information.
1.8. *Mike's Immunoglobulin Structure*/*Function Page* (M. R. Clark, 2001; http://www.path.cam.ac.uk/∼mrc7/mikeimages.html). These pages provide educational materials on immunoglobulin structure and function, and are illustrated by many colour images, models, and animations. Additional information is available on antibody humanization and Mike Clark's Therapeutic Antibody Human Homology Project, which aims to correlate clinical efficacy and anti-immunoglobulin responses with variable region sequences of therapeutic antibodies.
1.9. *The Antibody Resource Page* (The Antibody Resource Page, 2000; http://www.antibodyresource.com). This site describes itself as the "complete guide to antibody research and suppliers." Links to amino acid sequencing tools, nucleotide antibody sequencing tools, and hybridoma/cell-culture databases are provided.
1.9. Humanization *bYDesign* (J. Saldanha, 2000; http://people.cryst.bbk.ac.uk/∼ubcg07s). This resource provides an overview on antibody humanization technology. The most useful feature is a searchable database (by sequence and text) of more than 40 published humanized antibodies including information on design issues, framework choice, framework back-mutations, and binding affinity of the humanized constructs. See also Antibody Engineering Methods and Protocols, Ed. Benny K C Lo, Methods in Molecular Biology™, Human Press. Also at http://www.blogsua.com/pdf/antibody-engineering-methods-and-protocolsantibody-engineering-methods-and-protocols.pdf

As a source of genomic sequence variation data, the skilled person will also be aware of the following available databases and resources (including updates thereof):-
1. HapMap (The International HapMap Consortium. 2003; http://hapmap.ncbi.nlm.nih.gov/index.html.en). The HapMap Project is an international project that aims to compare the genetic sequences of different individuals to identify chromosomal regions containing shared genetic variants. The HapMap www site provides tools to identify chromosomal regions and the variant therein, with options to drill down to population level frequency data.
2. 1000 Genomes (The 1000 Genomes Project Consortium 2010; http://www.1000genomes.org/). This resource provides complete genomic sequence for 2500 unidentified individuals from one of 25 distinct population groups, with the aim of identifying genomic variants of >1%. The site provides the ability to interrogate data utilizing online tools (e.g. 'Variation Pattern Finder') and to download variant data for individual population groups.
3. Japanese SNP Database (H.Haga et al. 2002; http://snp.ims.u-tokyo.ac.jp/index.html). Based on a study identifying 190,562 human genetic variants this site catalogues genomic variants with useful features for searching and summarizing data.

It is possible to identify variants in immunoglobulin genes classed as low-frequency or rare variants that segregate with specific human ethnic populations. For the purpose of this analysis, a low- frequency immunoglobulin gene segment is classed as one with 'Minor Allele Frequency' (MAF) (cumulative frequency) of between 0.5% - 5%, rare variants are those classed as having a MAF of less than 0.5% in a particular human population.

The following bioinformatics protocol is envisaged to identify human immunoglobulin gene segments for use in the present disclosure:
(a) Identify one or more genomic regions containing gene segments of interest ('target genomic regions') and calculate the genomic coordinates, using coordinates that match the sequence assembly build used by either the 1000 Genomes project or International HapMap project (or another selected human gene database of choice).
(b) Identify genomic variants mapped to the genomic regions previously identified in (a). Retrieve variant frequencies for variants for each super population and preferably subpopulation where such data is available. Tools readily available on the HapMap WWW site and the VWC tools for the 1000Genomes Project are useful for this step.
(c) Filter list of genomic variants from target genomic regions to contain only variants classed as either 'Non-synonymous' single nucleotide polymorphisms (SNPs) or genomic 'insertions or deletions' (indels). Filter further to include those that are present in exonic sequences only.
(d) Correlate population frequency data for each of the identified variants for each of the super populations (for example 'European Ancestry', 'East Asian ancestry', 'West African ancestry', 'Americas', and 'South Asian ancestry') to identify those variants that segregate with less than two super-populations. Further correlate all identified variants with each of the sub- populations (for example, 'European ancestry' super-population might be subdivided into groups such as 'CEU - Utah residents with Northern or Western European ancestry', 'TSI Toscani in Italia' and 'British from England and Scotland') and produce a second score for rarity of variants in within a super-population.
(e) Collect one or more gene segments that show segregation to specific sub-populations for construction of synthetic loci according to the disclosure.

In one embodiment throughout the present text, "germline" refers to the canonical germline gene segment sequence.

By detailed analysis of the 1000 Genomes database, the inventors have devised a collection of candidate polymorphic antibody gene segment variants, eg, human variant JH gene segments (eg, see Example 4), that can be built into the design of transgenic heavy chain loci in mice for expressing increasingly diverse and new, synthetic repertoires of human variable regions. To this end, the invention provides the following embodiments.

### The present invention provides -

### Selection of Human JH6*02 Variant

### Transgenic IgH Loci, Non-Human Vertebrates, Cells & Antibodies Based on Human JH6*02

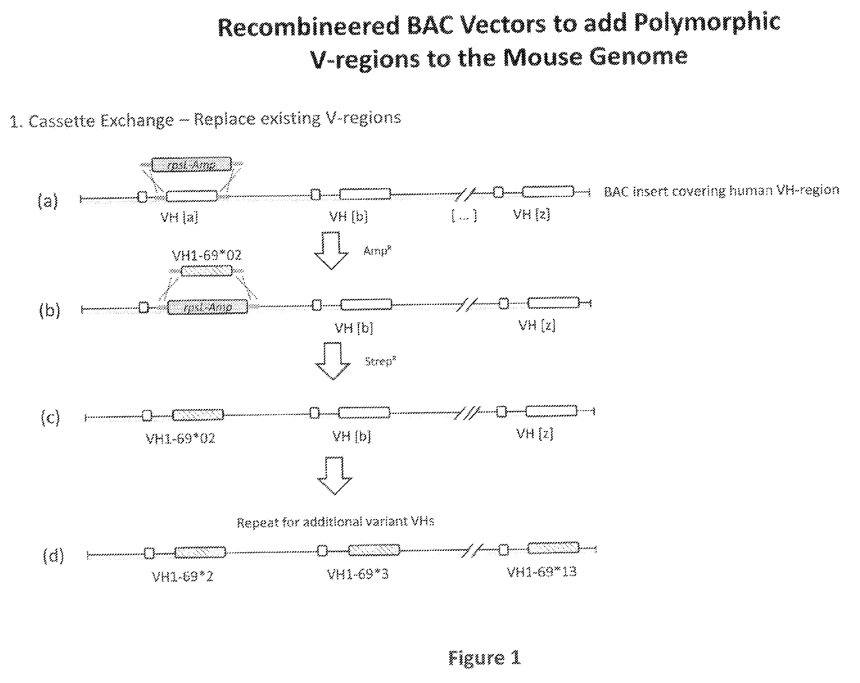

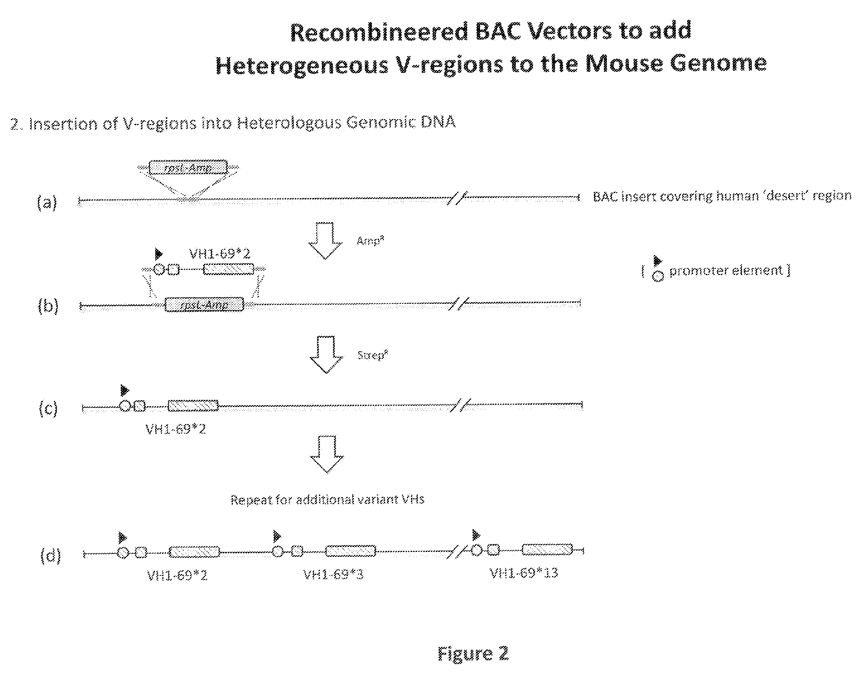

As explained above, in designing transgenic Ig heavy chain loci the present inventors have considered the huge amount of data available from the 1000 Genomes project (see www.1000genomes.org) that analyses gene distributions amongst many human populations, and in particular data on Ig gene segments. The inventors were also aware of human gene segments disclosed in the IMGT database (see www.imgt.org) and in Ensembl (see www.ensembl.org). The inventors needed to make choices about which human gene segments to include amongst the large number of human gene segments presented in these databases and the other sources of human Ig gene segment information known in the art, including those other databases disclosed herein. When choosing human JH gene segments, the inventors were aware that human JH6 encodes a relatively long amino acid sequence, and thus the inventors thought it desirable to include this for increasing the chances of producing IgH chains with relatively long HCDR3 regions. Antibodies with long HCDR3 (at least 20 amino acids according to IMGT nomenclature) have been shown to neutralise a variety of pathogens effectively including HIV, Influenza virus, malaria and Africa trypanosomes. Reference is also made to naturally-occurring Camelid (eg, Ilama or camel) heavy chain-only antibodies which bear long HCDR3s for reaching relatively inaccessible epitopes (see, eg, EP0937140). Long HCDR3s can form unique stable subdomains with extended loop structure that towers above the antibody surface to confer fine specificity. In some cases, the long HCDR3 itself is sufficient for epitope binding and neutralization (Liu, L et al; Journal of Virology. 2011. 85: 8467-8476). The unique structure of the long HCDR3 allows it to bind to cognate epitopes within inaccessible structure or extensive glycosylation on a pathogen surface. In human peripheral blood, there is around 3.5% of naive B antibodies or 1.9% of memory B IgG antibodies containing the HCDR3s with lengths of more than 24 amino acids (PLoS One. 2012;7(5):e36750. Epub 2012 May 9; "Human peripheral blood antibodies with long HCDR3s are established primarily at original recombination using a limited subset of germline genes"; Briney BS *et al*) (Fig. 1). The usage analysis indicates that these antibodies have the preference to use human JH6 with human D2-2, D3-3 or D2-15 (Brinley, BS et al, Figs. 2-5). See also PLoS One. 2011 Mar 30;6(3):e16857; Comparison of antibody repertoires produced by HIV-1 infection, other chronic and acute infections, and systemic autoimmune disease"; Breden F et *al.* Around 20% of all HCDR3 of antibodies use JH6. However, in those antibodies with HCDR3 of more than 24 amino acids, 70% use JH6 (Brinley, BS et al, Fig.2).

There is a need in the art for genetically modified non-human vertebrates and cells that can make antibodies and heavy chains that have long human HCDR3s, as well as antibodies, chains and VH domains that can be selected from such vertebrates and cells wherein these can address target epitopes better accessed by long HCDR3s.

The inventors, therefore, chose in this configuration of the invention to include a human JH6 gene segment as a mandatory human gene segment in their IgH locus design. Several different naturally- occurring human JH6 variants are known (eg, JH6*01 to *04 as well as others; IMGT nomenclature). The inventors considered this when deciding upon which human JH6 variant should be included in the transgenic IgH locus design. An alignment of some human JH6 variants is shown in Figure 7 (from www.imgt.org; dashes indicate identical nucleotides; nucleotide changes versus the *01 variant are shown by underlined nucleotides and corresponding amino acid changes are shown by underlined amino acids; Genbank accession numbers (release 185.0) are shown prefixed by J, X, M or A). The inventors used sequencing of human genomic DNA samples, inspection of public IgH DNA databases as well as informed choices on the basis of variant sequences as means to arrive at a rational choice of which JH6 variant to use.

The 1000 Genomes database uses human JH6*03 as the reference sequence, which would be a possible choice for the skilled person wishing to construct a transgenic IgH locus. The inventors noticed (eg, Figure 7 herein) that position 6 in JH6*03 is a tyrosine (Y) encoded by a TAC codon, whereas some other naturally-occurring human variants have a glycine (G) encoded by a GGT codon (the glycine being present as a YYG motif, forming part of a larger YYGXDX motif). To understand the potential significance of this, the inventors carried out analysis of JH sequences from other vertebrate species. The inventors surprisingly noticed that YYG and YYGXDX motifs are conserved across many vertebrate species (see Figures 7 & 8). This suggested to the inventors, therefore, that preservation of this motif might be desirable, which could guide the choice of JH6 variant for use in the present invention.

Another pointer arose when the inventors considered the TAC codon versus the GGT codon encoding Y or G respectively. The inventors considered the impact of these nucleotide sequences on the action of activation-induced cytidine deaminase (AID). The inventors knew that activation- induced cytidine deaminase (AID) is believed to initiate Ig somatic hypermutation (SHM) in a multi- step mechanism and they addressed this activity when rationally designing the locus. AID catalyses the deamination of C to U in DNA, generating mutations at C bases. Cytidines located within hotspot motifs are preferentially deaminated. Certain motifs are hotspots for AID activity (DGYW, WRC, WRCY, WRCH, RGYW, AGY, TAC, WGCW, wherein W=A or T, Y=C or T, D=A, G or T, H=A or C or T, and R=A or G). The presence of a TAC codon encoding Y at position 6 in JH6*03 creates AID mutation hotspots (the cytidine being the substrate of AID), these hotspots being the underlined motifs in the previous sentence. The inventors considered the impact of this and in doing so they considered possible mutants created by AID activity at the cytidine. Reference is made to Figure 9. The inventors noticed that a mutation at the third base of the TAC codon would yield 3 possible outcomes: Y, stop or stop. Thus, out of the three stop codons possible in the genetic code (the other being encoded by TGA - see Figure 9), two of them would be provided by mutation of the cytidine in the TAC codon encoding position 6 in JH6*03. The inventors, therefore, considered that this might increase the chances of non-productive IgH variable region production in transgenic loci based on JH6*03. Moreover, the inventors noticed that provision of a GGT codon instead (as per the other human JH6 variants) seemed preferable since mutation of the third base would never yield a stop codon (see Figure 9), and furthermore would retain coding, and thus conservation, of glycine at position 6, which the inventors also noticed was is in the YYG and YYGXDX motifs conserved across species.

Having decided against using JH6*03, the inventors needed to make a choice from other possible human variants. The MDV motif is at the C-terminus of HCDR3 based on human JH6, the adjacent framework 4 (FW4) starting with the WGQ motif (with reference to the sequence shown encoded by JH6*01; Figure 7). In making their choices for locus design, the inventors wished to maximise conservation of this HCDR3/FW4 junction in product IgH chains and antibodies including these. The inventors believed this to be desirable for heavy chain variable domain functionality and conformation. The inventors thought that this might in some cases be desirable to minimise immunogenicity (suitable for human pharmaceutical use). Consistent with these considerations, the inventors wanted to make a choice that would minimise mutation around the HCDR3/FW4 junction as a result of SHM in vivo to conserve junction configuration. See Rogozin & Diaz; "Cutting Edge: DGYW/WRCH Is a Better Predictor of Mutability at G:C Bases in Ig Hypermutation Than the Widely Accepted RGYW/WRCY Motif and Probably Reflects a Two-Step Activation-Induced Cytidine Deaminase-Triggered Process"; Journal of Immunology; March 15, 2004 vol. 172 no. 6 3382-3384. An example of a DGYW motif is GGCA. The inventors had this in mind when analysing the variant sequences.

With these considerations in mind, the inventors decided specifically to use human JH6*02 as the mandatory human JH6 for their IgH locus design. JH6*01 was rejected as the mandatory JH6 gene segment since the nucleotide sequence GGG CAA (encoding G and Q) contains a GGCA motif which is an AID recognition hotspot. The inventors realised that JH6*04 also contains such a motif due to the presence of the sequence GGC AAA encoding G and K (positions 11 and 12 respectively). The inventors also realised that the *02 variant has a C instead of a G that is in the *01 variant, the C desirably being a synonymous change (ie, not changing the encoded amino acid sequence around the CDR3/FW4 junction) and also this does not provide a GGCA AID hotspot motif. The inventors, therefore, decided that the mandatory JH6 should have this C base and this too pointed them to using the human JH6*02 variant.

In one example of any configuration of the invention herein, the only JH6 species included in the locus or genome is human JH6*02.

The inventors obtained 9 anonymised DNA samples from cheek swabs of 9 consenting human adults. Sequencing was performed on IgH locus DNA to confirm natural JH6 variant usage. It was found that the genome of all 9 humans contained a JH6*02 variant gene segment. In 7 out of the 9 humans, the genome was homozygous for JH6*02 (ie, each chromosome 14 had JH6*02 as its JH6 gene segment in the IgH locus). The inventors also inspected the publicly-available sequence information from the genomes of well-known scientists Craig Venter and Jim Watson. Both of these genomes contain JH6*02 too. This indicated to the inventors that this variant is common in humans.

So, the inventors made a choice of human JH6*02 on the basis of
(i) Containing the YYG and YYGXDX motifs that is conserved across several vertebrate species;
(ii) Provision of one less TAC codon (an AID hotspot that risks stop codons) and a choice instead of a codon that preserves the YYG and YYGXDX motifs;
(iii) Avoidance of a GGCA AID hotspot in the region of the HCDR3/FW4 junction; and
(iv) Common occurrence (and thus conservation and acceptability) in humans of the JH6*02 variant.

This rationale was tested by the inventors in laboratory examples, in order to see if human JH6*02 could desirably participate in antibody gene segment recombination and heavy chain production in a foreign (non-human vertebrate) setting, and moreover to assess if long HCDR3s based on human JH6*02 could be produced in vivo (in naive and immunised settings) in such non-human systems. It was noted that in some non-human settings, such as a mouse, the YYG and YYGXDX motifs are not conserved, and thus the inventors decided that it was important to test whether or not JH6*02 (having the YYG and YYGXDX motifs) could function properly in such a foreign setting to participate in VDJ recombination and selection against antigen.

Thus, as explained further in the examples, the inventors constructed transgenic JH6*02-containing IgH loci in ES cells, generated transgenic non-human vertebrates from the ES cells (both naive and immunised with a range of different target antigen types), isolated antibodies and heavy chain sequences based on JH6*02 as well as B-cells expressing these and made hybridomas expressing antigen-specific antibodies that are based on the chosen JH6*02 variant. The inventors found that the JH6*02 variant was extensively used and could contribute to the production of HCDR3 of at least 20 amino acids in many different heavy chains (including antigen-specific heavy chains). The chosen variant was preferably used over other JH gene segments in all settings (naive, immunised and antigen-specific) for the production of HCDR3 of at least 20 amino acids.

Thus, the present disclosure provides an IgH locus including human JH6*02 (IMGT nomenclature) as a mandatory JH gene segment. In one embodiment, the locus comprises non-human vertebrate (eg, mouse or rat) constant region gene segments downstream (ie, 3' of) the human JH6*02; and one or more VH gene segments (eg, a plurality of human VH gene segments) and one or more D gene segments (eg, a plurality of human D gene segments) upstream of (ie, 5' of) the human JH6*02. For example, the locus is comprised by a vector (eg, a DNA vector, eg, a yeast artificial chromosome (YAC), BAC or PAC). Such a vector (eg, YAC) can be introduced into a non-human vertebrate (eg, mouse or rat) cell using standard techniques (eg, pronuclear injection) so that the locus is integrated into the cell genome for expression of IgH chains comprising at least one chain whose variable domain is a product of the recombination of human JH6*02 with a VH and a D gene segment.

In another example, the locus (eg, with a completely human, rat or mouse constant region, or a human/mouse chimaeric constant region) can be provided in the genome of a non-human vertebrate (eg, mouse or rat) cell. For example, the cell is an ES cell or an antibody-producing cell (eg, an isolated B-cell, an iPS cell or a hybridoma).

In another example, the disclosure provides a non-human vertebrate (eg, a mouse or a rat) comprising an IgH locus of the invention which comprises a human JH6*02 gene segment, wherein the locus can express an IgH chain whose variable domain is a product of the recombination of human JH6*02 with a VH and a D gene segment. As shown in the examples, the inventors have successfully produced such mice which produce such IgH chains with VH domains based on human JH6*02. The inventors isolated and sequenced IgH chains from the mice before (naive) and after (immunised) exposure to a range of target antigens and confirmed by comparison to IMGT IgH gene segment sequences that the isolated chains (and antibodies containing these) were produced based on JH6*02. Such chains were found in naive mice, as well as in antigen-specific antibodies from immunised mice. B-cells were isolated from immunised mice, wherein the B-cells express antibodies based on JH6*02 and hybridomas were generated from the B-cells, the hybridomas expressing antigen-specific antibodies based on JH6*02. The inventors, therefore, provided the locus, vertebrate, cell and hybridoma of the disclosure based on the use of human JH6*02 and showed that antibodies based on JH6*02 and B-cells expressing these can be successfully produced and isolated following immunisation of the vertebrates, corresponding hybridomas being a good source of antibodies whose VH domains are based on JH6*02, eg for administration to a patient, eg, for human medicine. Furthermore, it was found possible to produce and isolated antigen-specific antibodies whose VH domains are based on JH6*02 and which had a relatively long HCDR3 (eg, 20 amino acids).

Thus, the present disclosure provides embodiments as in the following clauses:-
1. A non-human vertebrate (optionally a mouse or a rat) or vertebrate cell whose genome comprises an immunoglobulin heavy chain locus comprising human gene segment JH6*02, one or more VH gene segments and one or more D gene segments upstream of a constant region; wherein the gene segments in the heavy chain locus are operably linked to the constant region thereof so that the mouse is capable of producing an antibody heavy chain produced by recombination of the human JH6*02 with a D segment and a VH segment.
   In another example, the disclosure provides
   A non-human vertebrate (optionally a mouse or a rat) or vertebrate cell whose genome comprises an immunoglobulin heavy chain locus comprising one, more or all of human IGHV gene segments selected from V3-21, V3-13, V3-7, V6-1, V1-8, V1-2, V7-4-1, V1-3, V1-18, V4-4, V3-9, V3-23, V3-11 and V3-20 (eg, one, more or all of V3-21*03, V3-13*01, V3-7*01, V6-1*01, V1-8*01, V1-2*02, V7-4-1*01, V1-3*01, V1-18*01, V4-4*01, V3-9*01 and V3-23*04). These segments were found in naive repertoires to be productive to produce HCDR3s of at least 20 amino acids in length. In an embodiment, the locus comprises a human JH6, eg, JH6*02.
   The invention also provides a HCDR3, VH domain, antibody heavy chain or antibody having a HCDR3 size of at least 20 amino acids. Optionally, the HCDR3 or VH domain (or VH domain of the heavy chain or antibody) comprises mouse AID-pattern somatic hypermutations and/or mouse dTd-pattern mutations. This can be provided, for example, wherein VH domain is produced in a mouse comprising mouse AID and/or mouse TdT (eg, endogenous AID or TdT). See also Annu. Rev. Biochem. 2007. 76:1-22; Javier M. Di Noia and Michael S. Neuberger, "Molecular Mechanisms of Antibody Somatic Hypermutation" (in particular figure 1 and associated discussion on AID hotspots in mouse); and Curr Opin Immunol. 1995 Apr;7(2):248-54, "Somatic hypermutation", Neuberger MS and Milstein C (in particular, discussion on hotspots in mouse).
   These segments were found in naive repertoires to be productive in recombination with human JH6*02 to produce HCDR3s of at least 20 amino acids in length.
   In an example, the vertebrate is naive. In another embodiment, the vertebrate instead is immunised with a target antigen.
   In an example, the vertebrate or cell mentioned below is capable of so producing an antibody heavy chain upon immunisation with a target antigen. In an example, the vertebrate is an immunised vertebrate that produces antibody heavy chains specific for a target antigen and wherein the variable domains of the heavy chains are the product of recombination between a VH, D and JH6*02. For example, the D is selected from human D3-3, D2-15, D3-9; D4-17; D3-10; D2-2; D5-24; D6-19; D3-22; D6-13; D5-12; D1-26; D1-20; D5-18; D3-16; D2-21; D1-14; D7-27; D1- 1; D6-25; D2-14 and D4-23 (eg, selected from D3-9*01; D4-17*01; D3-10*01; D2-2*02; D5- 24*01; D6-19*01; D3-22*01; D6-13*01; D5-12*01; D1-26*01; D1-20*01; D5-18*01; D3-16*02; D2-21*02; D1-14*01; D7-27*02; D1-1*01; D6-25*01; D2-15*01; and D4-23*01). For example, the D is human D3-9 or D3-10. In an example, the HCDR3 length is at least 20 amino acids (eg, 20, 21, 23 or 24).
   In an example of the vertebrate or cell, the genome comprises additional human JH gene segments (eg, JH2, 3, 4 and 5 gene segments).
   In an example of the vertebrate or cell, the genome comprises an immunoglobulin light chain locus comprising one or more human V gene segments and one or more human J gene segments upstream of a constant region (eg, a human or a mouse lambda or kappa constant region).
   For rearrangement and expression of heavy chains, the locus comprises control elements, such as an Eµ and Sµ between the J gene segment(s) and the constant region as is known by the skilled person. In one example, a mouse Eµ and Sµ is included in the heavy chain locus between the JH6*02 and the constant region (ie, in 5' to 3' order the locus comprises the JH6*02, Eµ and Sµ and constant region). In an example, the Eµ and Sµ are Eµ and Sµ of a mouse 129-derived genome (eg, a 129Sv-derived genome, eg, 129Sv/EV (such as 129S7Sv/Ev (such as from AB2.1 or AB2.2 cells obtainable from Baylor College of Medicine, Texas, USA) or 129S6Sv/Ev))); in another example, the Eµ and Sµ are Eµ and Sµ of a mouse C57BL/6 -derived genome. In this respect, the locus can be constructed in the IgH locus of the genome of a cell selected from AB2.1, AB2.2, VGF1, CJ7 and FH14. VGF1 cells were established and described in Auerbach W, Dunmore JH, Fairchild-Huntress V, et al; Establishment and chimera analysis of 129/SvEv- and C57BL/6-derived mouse embryonic stem cell lines. Biotechniques 2000; 29:1024-8, 30, 32,.
   Additionally or alternatively, the constant region (or at least a Cµ or Cµ and gamma constant regions thereof) is a constant region (or Cµ or Cµ and gamma constant regions thereof) is of a genome described in the paragraph immediately above.
   A suitable source of JH6*02 and other human DNA sequences will be readily apparent to the skilled person. For example, it is possible to collect a DNA sample from a consenting human donor (eg, a cheek swab sample as per the Example herein) from which can be obtained suitable DNA sequences for use in constructing a locus of the invention. Other sources of human DNA are commercially available, as will be known to the skilled person. Alternatively, the skilled person is able to construct gene segment sequence by referring to one or more databases of human Ig gene segment sequences disclosed herein.
2. The vertebrate of clause 1, wherein the vertebrate has been immunised with a target antigen and wherein the variable domain of the heavy chain is the product of recombination between a VH, D and JH6*02 and wherein the HCDR3 length is at least 20 amino acids (eg, 20, 21, 23 or 24).
   Optionally, the immunised vertebrate produces an antibody heavy chain specific for a target antigen and wherein the variable domain of the heavy chain is the product of recombination between a VH, D and JH6*02 and wherein the HCDR3 length is at least 20 amino acids (eg, 20, 21, 23 or 24).
3. A non-human vertebrate cell (optionally a mouse cell or a rat cell) whose genome comprises an immunoglobulin heavy chain locus comprising human gene segment JH6*02, one or more VH gene segments and one or more D gene segments upstream of a constant region; wherein the gene segments in the heavy chain locus are operably linked to the constant region thereof for producing (eg, in a subsequent progeny cell) an antibody heavy chain produced by recombination of the human JH6*02 with a D segment and a VH segment.
4. The cell of clause 3, which is an ES cell capable of differentiation into a progeny antibody- producing cell that expresses said heavy chain.
5. The vertebrate or cell of any preceding clause, wherein the heavy chain locus comprises a human JH6*02 recombination signal sequence (RSS) operably connected 5' to the JH6*02 gene segment.
   For example, the native RSS-JH6*02 sequence can be used to advantageously maintain the natural pairing between RSS and this JH gene segment. In this respect, the following sequence is used:-
   ggttttt**ggggtgaggatggacattctg**ccattgtgattactactactactacggtatggacgtctggggccaagggacca cggtcaccg tctcctcag (SEQ ID NO: 238)
   RSSs have a common architecture: 9mer (eg, first underlined sequence above) followed by a 22bp spacer and then a 7mer (eg, second underlined sequence above). Spacers are 23bp +/-1 normally, while the 9 and 7mer are more conserved.
6. The vertebrate or cell of clause 5, wherein the RSS is SEQ ID NO: 238 or a sequence having an identical 9mer and 7mer sequence flanking a sequence that is at least 70% identical to the 22mer sequence of SEQ ID NO: 238.
7. The vertebrate or cell of clause 6, wherein the RSS and JH6*02 are provided as SEQ ID NO: 237.
8. The vertebrate or cell of any preceding clause, wherein the JH6*02 is the only JH6-type gene segment in the genome.
9. The vertebrate or cell of any preceding clause, wherein the JH6*02 is the closest JH gene segment to the constant region in the locus.
10. The vertebrate or cell of any preceding clause, wherein the locus comprises one, more or all human D gene segments D3-9; D4-17; D3-10; D2-2; D5-24; D6-19; D3-22; D6-13; D5-12; D1-26; D1-20; D5-18; D3-16; D2-21; D1-14; D7-27; D1-1; D6-25; D2-14; and D4-23.
   For example, the locus comprises one, more or all of human D gene segments D3-9*01; D4-17*01; D3-10*01; D2-2*02; D5-24*01; D6-19*01; D3-22*01; D6-13*01; D5-12*01; D1-26*01; D1-20*01; D5-18*01; D3-16*02; D2-21*02; D1-14*01; D7-27*02; D1-1*01; D6-25*01; D2-15*01; and D4-23*01.
11. The vertebrate or cell of clause 10, wherein the locus comprises one, more or all human D gene segments D3-9, D3-10, D6-19, D4-17, D6-13, D3-22, D2-2, D2-25 and D3-3.
   These D segments were found to be productive in recombination with human JH6*02 to produce HCDR3s of at least 20 amino acids in length.
   In an example, the locus comprises one, more or all human D gene segments D3-9, D3-10, D6- 19, D4-17, D6-13 and D3-22 (for example one, more or all of D3-9*01, D3-10*01, D6-19*01, D4- 17*01, D6-13*01 and D3-22*01 These D segments were found in naive repertoires to be productive in recombination with human JH6*02 to produce HCDR3s of at least 20 amino acids in length.
   In an example, the locus comprises one, more or all human D gene segments D3-10, D6-19 and D1-26 (for example, one, more or all of D3-10*01, D6-19*01 and D1-26*01). These D segments were found in immunised repertoires to be productive in recombination with human JH6*02 to produce HCDR3s of at least 20 amino acids in length.
   In an example, the locus comprises one, more or all human D gene segments D3-9 and D3-10 (for example, one, more or all of D3-9*01 and D3-10*01 These D segments were found in antigen-specific repertoires to be productive in recombination with human JH6*02 to produce HCDR3s of at least 20 amino acids in length.
12. The vertebrate or cell of any preceding clause, wherein the locus comprises a plurality of human D gene segments and the JH6*02 is in human germline configuration with respect to the 3'-most human D gene segment (or all of the human D segments comprised by the locus).
   In an example, the 3'-most D gene segment is D7-27. In an example, the locus comprises all of human D gene segments from D1-1 to D7-27 as present in a germline human IgH locus (eg, as shown in the IMGT database).
   Alternatively or additionally, the JH6*02 is in human germline configuration with respect to one, more or all of the Eµ Sµ and constant region (eg, Cµ)
13. The vertebrate or cell of any preceding clause, wherein the locus comprises one, more or all of IGHV gene segments selected from V3-21, V3-13, V3-7, V6-1, V1-8, V1-2, V7-4-1, V1-3, V1-18, V4-4, V3-9, V3-23, V3-11 and V3-20.
   In an example, the locus comprises one, more or all human IGHV gene segments V3-21, V3-13, V3-7, V6-1, V1-8, V1-2, V7-4-1, V1-3, V1-18, V4-4, V3-9, V3-23 (for example, one, more or all of V3-21*03, V3-13*01, V3-7*01, V6-1*01, V1-8*01, V1-2*02, V7-4-1*01, V1-3*01, V1-18*01, V4- 4*01, V3-9*01 and V3-23*04). These segments were found in naive repertoires to be productive in recombination with human JH6*02 to produce HCDR3s of at least 20 amino acids in length.
   In an example, the locus comprises one, more or all human IGHV gene segments V3-7, V3-11 and V4-4 (for example, one, more or all of V3-7*01, V3-11*01 and V4-4*02). These segments were found in immunised repertoires to be productive in recombination with human JH6*02 to produce HCDR3s of at least 20 amino acids in length.
   In an example, the locus comprises one, more or all human IGHV gene segments V4-4, V1-8, V3- 9, V3-11 and V3-20 (for example, one, more or all of V4-4*02, V1-8*01, V3-9*01, V3-11*01 and V3-20 (eg, *d01). These segments were found in antigen-specific repertoires to be productive in recombination with human JH6*02 to produce HCDR3s of at least 20 amino acids in length.
14. The vertebrate or cell of any preceding clause, wherein the locus comprises one, more or all of human D3-9*01, D3-10*01, D6-19*01, D6-13*01, D1-26*01, IGHV1-8*01, IGHV4-61*01, IGHV6- 1*01, IGHV4-4*02, IGHV1-3*01, IGHV3-66*03, IGHV3-7*01 and IGHV3-9*01.
   These are gene segments that very frequently combine with JH6*02 to produce productive heavy chains and antibodies.
   For example, the locus comprises one, more or all of human IGHV1-8*01, D3-9*01 and D3- 10*01. These gene segments were productive with JH6*02 to produce HCDR3s of at least 20 amino acids in more than 10 antibodies.
15. An antibody-producing cell (eg, a B-cell) that is a progeny of the cell of any one of clauses 3 to 14, wherein the antibody-producing cell comprises a heavy chain locus comprising a rearranged variable region produced by recombination of human JH6*02 with a D segment and a VH segment (eg, JH6*02 with human VH3-11 (eg, VH3-11*01) and D3-9; VH3-20 (eg, VH3-20*01 ) and D3-10; VH4-4 (eg, VH4-4*02) and D3-10; VH3-9 (eg, VH3-9*01) and D3-10; or VH1-8 (eg, VH1-8*01) and D310).
   Such a variable region would be the product of in vivo somatic hypermutation in a non-human vertebrate or cell of the invention.
16. The cell of clause 15, which is a B-cell or hybridoma that expresses a target antigen-specific antibody comprising a heavy chain that comprises a rearranged variable region produced by recombination of human JH6*02 with a D segment and a VH segment (eg, JH6*02 with human VH3-11 (eg, VH3-11*01) and D3-9; VH3-20 (eg, VH3-20*01) and D3-10; VH4-4 (eg, VH4-4*02) and D3-10; VH3-9 (eg, VH3-9*01) and D3-10; or VH1-8 (eg, VH1-8*01) and D310).
   Such a variable region would be the product of in vivo somatic hypermutation in a non-human vertebrate or cell of the invention
17. The vertebrate or cell of any preceding clause, wherein the antibody heavy chain specifically binds a target antigen.
18. The vertebrate or cell of any preceding clause, wherein the antibody heavy chain has a HCDR3 length of at least 20 amino acids.
   Optionally, the HCDR3 length is at least 21, 22, 23, 24, 25, 26, 27, 28, 29 or 30 amino acids. Additionally, in one example the length is no more than 35, 34, 33, 32 or 31 amino acids. For example, the HCDR3 length is 20, 21, 22, 23 or 24 amino acids.
19. The vertebrate or cell of any preceding clause, wherein the antibody heavy chain is a product of the recombination of JH6*02 with a human VH gene segment recited in clause 13 or 14 and/or a D gene segment recited in clause 10, 11 or 14.
20. The vertebrate or cell of any preceding clause, wherein all endogenous non-human vertebrate heavy chain variable region gene segments have been inactivated in the genome (Eg, by gene segment deletion or inversion).
21. The vertebrate or cell of any preceding clause, wherein the genome is homozygous for said heavy chain locus.
22. A heavy chain (eg, comprised by an antibody) isolated from a vertebrate of any one of clauses 1, 2, 5 to 14 and 17 to 21 wherein the heavy chain comprises a HCDR3 of at least 20 amino acids.
23. The heavy chain of clause 22, wherein the HCDR3 is the product of recombination of human JH6*02 with a human VH gene segment recited in clause 13 or 14 and/or a D gene segment recited in clause 10, 11 or 14.
   In an example, the heavy chain is chimaeric where the C region is non-human. In an example, the heavy chain is human where the C region is human.
24. A heavy chain (eg, comprised by an antibody) whose VH variable domain is identical to the VH variable domain of the heavy chain of clause 22 or 23, and which comprises a human constant region or a human-mouse chimaeric constant region (eg, CH1 is human and the other constant domains are mouse).
25. The heavy chain of clause 22, 23 or 24, whose VH variable domain is specific for a target antigen.
26. A method for producing a heavy chain, VH domain or an antibody specific to a target antigen, the method comprising immunizing a non-human vertebrate according to any one of clauses 1, 2, 5 to 14 and 17 to 21 with the antigen and isolating the heavy chain, VH domain or an antibody specific to a target antigen or a cell producing the heavy chain, VH domain or an antibody, wherein the heavy chain, VH domain or an antibody comprises a HCDR3 that is derived from the recombination of human JH6*02 with a VH gene segment and a D gene segment.
27. A method for producing a human heavy chain or antibody comprising carrying out the method of clause 26, wherein the constant region of the locus is a non-human vertebrate (eg, mouse or rat) constant region, and then replacing the non-human constant region of the isolated heavy chain or antibody with a human constant region (eg, by engineering of the nucleic acid encoding the antibody).
28. A heavy chain, VH domain or an antibody produced by the method of clause 26 or 27.
   Optionally the HCDR3 length is at least 20 amino acids as herein described.
29. A B-cell or hybridoma expressing a heavy chain VH domain that is identical to the VH domain of the heavy chain of clause 22, 23 or 28.
30. A nucleic acid encoding the VH domain of the heavy chain of clause 22, 23 or 28, or encoding the heavy chain of clause 22, 23, 24, 25 or 28.
31. A vector (eg, a CHO cell or HEK293 cell vector) comprising the nucleic acid of clause 30; optionally wherein the vector is in a host cell (eg, a CHO cell or HEK293 cell).
32. A pharmaceutical composition comprising the antibody, heavy chain or VH domain (eg, comprised by an antibody) of any one of clauses 22 to 25 and 28, together with a pharmaceutically-acceptable excipient, diluent or a medicament (eg, a further antigen-specific variable domain, heavy chain or antibody).
33. The antibody, heavy chain or VH domain (eg, comprised by an antibody) of any one of clauses 22 to 25 and 28 for use in medicine (eg, human medicine).

For example, the locus comprises the following human VH gene segments
IGHV6-1
IGHV3-7
IGHV1-8
IGHV3-9
IGHV3-11
IGHV3-13
IGHV1-18
IGHV3-30
IGHV4-31
IGHV4-39
IGHV4-59

Optionally also (i) and/or (ii)
(i)
   IGHV1-2
   IGHV2-5 and
   IGHV3-21
(ii)

   IGHV1-2
   IGHV2-5
   IGHV3-21
   IGHV1-24

For example, the locus comprises the following human VH gene segment variants
IGHV6-1*01
IGHV3-7*01
IGHV1-8*01
IGHV3-9*01
IGHV3-11*01
IGHV3-13*01
IGHV1-18*01
IGHV3-30*18
IGHV4-31*03
IGHV4-39*01 and
IGHV4-59*01;

Optionally also (iii) or (iv)
(ii)
   IGHV1-2*04
   IGHV2-5*10 and
   IGHV3-21*03
(iv)
   IGHV1-2*02
   IGHV2-5*01
   IGHV3-21*01 and
   IGHV1-24*01

For example, the locus comprises the following human JH gene segment variants
IGHJ2*01
IGHJ3*02
IGHJ4*02
IGHJ5*02 and
GHJ6*02

For example, the locus comprises the following human D gene segments
IGHD1-1
IGHD2-2
IGHD3-9
IGHD3-10
IGHD5-12
IGHD6-13
IGHD1-14
IGHD2-15
IGHD3-16
IGHD4-17
IGHD6-19
IGHD2-21
IGHD5-24
IGHD1-26 and
IGHD7-27
and optionally also (v) or (vi)
(v)
   IGHD3-3
(vi)
   IGHD3-3
   IGHD4-4
   IGHD5-5
   IGHD6-6
   IGHD1-7
   IGHD2-8 and
   IGHD2-8

### The present disclosure provides in a fifth configuration-

### Constant Regions Tailored to Human Use & Antibody Humanisation

Additional rational design and bioinformatics has led the inventors to realise that specific human constant region variants are conserved across many diverse human populations. The inventors realised that this opens up the possibility of making a choice to humanise antibodies, chains and variable domains by using such specific constant regions in products, rather than arbitrarily choosing the human constant region (or a synthetic version of a human constant region). This aspect of the disclosure also enables one to tailor antibody-based drugs to specific human ethnic populations, thereby more closely matching drug to patient (and thus disease setting) than has hitherto been performed. It can be a problem in the state of the art that antibodies are humanised with an arbitrary choice of human constant region (presumably derived from one (often unknown) ethnic population or non-naturally occurring) that does not function as well in patients of a different human ethnic population. This is important, since the constant region has the major role in providing antibody effector functions, eg, for antibody recycling, cellular and complement recruitment and for cell killing.

As discussed further in WO2011066501, human IgG sub-types IgG1, IgG2, gG3 and IgG4 exhibit differential capacity to recruit immune functions, such as antibody-dependent cellular cytotoxicity (ADCC, e.g., IgG1 and IgG3), antibody-dependent cellular phagocytosis (ADCP, e.g., IgG1, IgG2, IgG3 and IgG4), and complement dependent cytotoxicity (CDC, e.g., IgG1, IgG3). Sub-type-specific engagement of such immune functions is based on selectivity for Fc receptors on distinct immune cells and the ability to bind C1q and activate the assembly of a membrane attack complex (MAC). Among the various types, relative affinity for FcY receptors (e.g., FcγRI, FcγRIIa/b/c, FcγRIIIa/b) is high for IgG1 and IgG3, however, there is minimal affinity for IgG2 (restricted to the FcγRIIa 131 H polymorphism), and IgG4 only has measurable affinity for FcγRI. Using comparative sequence analysis and co-crystal structures, the key contact residues for receptor binding have been mapped to the amino acid residues spanning the lower hinge and CH2 region. Using standard protein engineering techniques, some success in enhancing or reducing the affinity of an antibody preparation for Fc receptors and the C1q component of complement has been achieved.

Among the isotypes, IgG2 is least capable of binding the family of Fc receptors. Using IgG2 as the starting point, efforts have been made to find a mutant with diminished effector functions but which retains FcRn binding, prolonged stability, and low immunogenicity. Improved mutants of this nature may provide improved antibody therapeutics with retained safety. Human IgG1 therapeutic antibodies that bind to cell surface targets are able to engage effector cells that may mediate cell lysis of the target cell by antibody-dependent cellular cytotoxicity (ADCC) or complement dependent cytotoxicity (CDC). These mechanisms occur through interaction of the CH2 region of the antibody Fc domain to FcγR receptors on immune effector cells or with C1q, the first component of the complement cascade. Table 19 shows the activities of different human gamma sub-types. The skilled person may choose accordingly to promote or dampen-down activity depending upon the disease setting in humans of interest. For example, use of a human gamma-1 constant region is desirable when one wishes to isolated totally human heavy chains and antibodies that have relatively high complement activation activity by the classical pathway and FcγR1 recognition in human patients. See also Mol Immunol. 2003 Dec;40(9):585-93; "Differential binding to human Fcgamma Rlla and Fcgamma Rllb receptors by human IgG wild type and mutant antibodies"; Armour KL et al.

IgG2 constant regions are well suited to producing antibodies and heavy chains according to the invention for binding to cytokines or soluble targets in humans, since IgG2 is essentially FcγRI,III- silent, FcγRIIa-active and has little Complement activity.

IgG1 constant regions have wide utility for human therapeutics, since IgG1 antibodies and heavy chains are FcγRI,II,III- active and have complement activity. This can be enhanced by using a human gamma-1 constant region that has been activated by engineering as is known in the art.

The work of the inventors has therefore identified a collection of human constant region of different isotypes from which an informed choice can be made when humanising chimaeric antibody chains (or conjugating V domains, such as dAbs or Camelid VHH, to constant regions). The collection was identified on the basis of bioinformatics analysis of the 1000 Genomes database, the inventors selecting constant region variants that are frequently occurring across several human ethnic populations, as well as those that appear with relatively high frequency within individual populations (as assessed by the number of individuals whose genomes comprise the variant). By sorting through the myriad possible sequences on this basis, the inventors have provided a collection of human constant region variants that are naturally-occurring and which can be used when rationally designing antibodies, heavy chains and other antibody-based formats that bear a human constant region. In particular, this is useful when humanising chimaeric heavy chains to produce totally human chains in which both the variable and constant regions are human. This is useful for compatibility with human patients receiving antibody-based drugs.

To this end, the disclosure provides the following aspects:-
1. A method of producing an antibody heavy chain, the method comprising
   (a) providing an antigen-specific heavy chain variable domain (eg, VH (such as a human VH or dAb) or VHH or a humanised heavy chain variable domain); and
   (b) combining the variable domain with a human heavy chain constant region to produce an antibody heavy chain comprising (in N- to C-terminal direction) the variable domain and the constant region;
   wherein
   the human heavy chain constant region is an IGHAref, IGHA1a, IGHA2a, IGHA2b, IGHG1ref, IGHG2ref, IGHG2a, IGHG3ref, IGHG3a, IGHG3b, IGHG4ref, IGHG4a, IGHDref, IGHEref, IGHMref, IGHMa or IGHMb constant region.
   Step (b) can be carried out, eg, using recombinant DNA technology using the corresponding nucleotide sequences.
   For the constant region according to any aspect of this configuration, either genomic DNA or equivalent (ie, having introns and exons and optionally also 5' UTR sequences, eg, with native or a non-native leader sequence) can be used for the constant region. For example, any of the "GENOMIC" sequences disclosed as SEQ ID NO: 365 onwards herein. Alternatively, an intronless sequence can be used, for example any of the "CDS" sequences disclosed as SEQ ID NO: 365 onwards herein (eg, with native or a non-native leader sequence).
   Optionally for any aspect of this configuration of the disclosure, the human heavy chain constant region is an IGHAref constant region.
   Optionally for any aspect of this configuration of the disclosure, the human heavy chain constant region is an IGHA1a constant region.
   Optionally for any aspect of this configuration of the disclosuren, the human heavy chain constant region is an IGHA2a constant region.
   Optionally for any aspect of this configuration of the disclosure, the human heavy chain constant region is an IGHA2b constant region.
   Optionally for any aspect of this configuration of the disclosure, the human heavy chain constant region is IGHG1ref constant region.
   Optionally for any aspect of this configuration of the disclosure, the human heavy chain constant region is an IGHG2ref constant region.
   Optionally for any aspect of this configuration of the disclosure, the human heavy chain constant region is an IGHG2a constant region.
   Optionally for any aspect of this configuration of the disclosure, the human heavy chain constant region is an IGHG3ref constant region.
   Optionally for any aspect of this configuration of the disclosure, the human heavy chain constant region is an IGHG3a constant region.
   Optionally for any aspect of this configuration of the disclosure, the human heavy chain constant region is an IGHG3b constant region.
   Optionally for any aspect of this configuration of the disclosure, the human heavy chain constant region is an IGHG4ref constant region.
   Optionally for any aspect of this configuration of the disclosure, the human heavy chain constant region is an IGHG4a constant region.
   Optionally for any aspect of this configuration of the disclosure, the human heavy chain constant region is an IGHDref constant region.
   Optionally for any aspect of this configuration of the disclosure, the human heavy chain constant region is an IGHEref constant region.
   Optionally for any aspect of this configuration of the disclosure, the human heavy chain constant region is an IGHMref constant region.
   Optionally for any aspect of this configuration of the disclosure, the human heavy chain constant region is an IGHMa constant region.
   Optionally for any aspect of this configuration of the disclosure, the human heavy chain constant region is an IGHMb constant region.
   Optionally, a derivative (eg, a mutant or conjugate) of the heavy chain or an antibody containing the heavy chain is produced. For example, a toxic payload can be conjugated (eg, for oncology applications). For example, one or more mutations can be introduced, as is known in the art, to inactivate or enhance Fc effector function.
2. The method of aspect 1, wherein the variable domain is a human variable domain.
   A human variable domain is, for example, the product of recombination in a transgenic non- human vertebrate of human VH, D and JH gene segments. Alternatively, the variable domain is identified using *in vitro* display technology from a human VH library, eg, using phage display, ribosome display or yeast display, as is known in the art.
   In another embodiment, the variable domain is a humanised variable domain, eg, comprising human frameworks with non-human (eg, mouse or rat) CDRs). Humanisation technology is conventional in the art, and will be readily known to the skilled person.
3. The method of any preceding aspect, wherein the variable domain has previously been selected from a non-human vertebrate that has been immunised with the antigen.
   For example, the vertebrate (such as a mouse or rat) genome comprises a chimaeric heavy chain locus comprising a human variable region (human V, D and JH gene segments) operably connected upstream of a non-human vertebrate constant region so that the locus is able to rearrange for the expression of heavy chains comprising human variable domains and non- human vertebrate constant regions.
   In alternative embodiments, the variable domain is selected using an *in vitro* technology such as phage display, ribosome display or yeast display. In this case the variable domain may be displayed with or without a constant region, provided that it is later combined with a human constant region as per the disclosure.
4. The method of any preceding aspect, comprising providing an expression vector (Eg, a mammalian expression vector, such as a CHO or HEK293 vector) comprising a nucleotide sequence encoding the constant region; inserting a nucleotide sequence encoding the variable domain into the vector 5' of the constant region sequence; inserting the vector into a host cell and expressing the heavy chain by the host cell; the method further comprising isolating a heavy chain (eg, as part of an antibody) comprising the variable domain and the human constant region.
   The vector comprises regulatory elements sufficient to effect expression of the heavy chain when the vector is harboured by a host cell, eg, a CHO or HEK293 cell.
5. The method of any preceding aspect, further comprising obtaining a nucleotide sequence encoding the heavy chain.
6. An antibody comprising a human heavy chain, the heavy chain comprising a variable domain that is specific for an antigen and a constant region that is an IGHAref, IGHA1a, IGHA2a, IGHA2b, IGHG1ref, IGHG2ref, IGHG2a, IGHG3ref, IGHG3a, IGHG3b, IGHG4ref, IGHG4a, IGHDref, IGHEref, IGHMref, IGHMa or IGHMb constant region.
7. A polypeptide comprising (in N- to C- terminal direction) a leader sequence, a human variable domain that is specific for an antigen and a human constant region that is an IGHAref, IGHA1a, IGHA2a, IGHA2b, IGHG1ref, IGHG2ref, IGHG2a, IGHG3ref, IGHG3a, IGHG3b, IGHG4ref, IGHG4a, IGHDref, IGHEref, IGHMref, IGHMa or IGHMb constant region; wherein (i) the leader sequence is not the native human variable domain leader sequence (eg, the leader sequence is another human leader sequence or a non-human leader sequence); and/or (ii) the variable domain comprises mouse AID-pattern somatic mutations or mouse terminal deoxynucleotidyl transferase (TdT)- pattern junctional mutations.
8. A nucleotide sequence encoding (in 5' to 3' direction) a leader sequence and a human antibody heavy chain, the heavy chain comprising a variable domain that is specific for an antigen and a constant region that is an IGHAref, IGHA1a, IGHA2a, IGHA2b, IGHG1ref, IGHG2ref, IGHG2a, IGHG3ref, IGHG3a, IGHG3b, IGHG4ref, IGHG4a, IGHDref, IGHEref, IGHMref, IGHMa or IGHMb constant region; and the leader sequence being operable for expression (eg, in a mammalian CHO or HEK293 cell) of the heavy chain and wherein the leader sequence is not the native human variable domain leader sequence (eg, the leader sequence is another human leader sequence or a non-human leader sequence).
   In an example, the leader sequence is
   ATGGGCTGGTCCTGCATCATCCTGTTTCTGGTGGCCACCGCCACCGGCGTGCACAG C

   Which translates to
   MGWSCIILFLVATATGVHS
9. A nucleotide sequence encoding (in 5' to 3' direction) a promoter and a human antibody heavy chain, the heavy chain comprising a variable domain that is specific for an antigen and a constant region that is an IGHAref, IGHA1a, IGHA2a, IGHA2b, IGHG1ref, IGHG2ref, IGHG2a, IGHG3ref,
   IGHG3a, IGHG3b, IGHG4ref, IGHG4a, IGHDref, IGHEref, IGHMref, IGHMa or IGHMb constant region; and the promoter being operable for expression (eg, in a mammalian CHO or HEK293 cell) of the heavy chain and wherein the promoter is not the native human promoter.
   In one embodiment, the promoter sequence is a human IGK 3-15 promoter.
10. The antibody, polypeptide or nucleotide sequence of any one of aspects 6 to 9, wherein the variable domain comprises mouse AID-pattern somatic mutations and/or mouse terminal deoxynucleotidyl transferase (TdT)- pattern junctional mutations.
   For example, one way, in any aspect of this configuration of the disclosure, to provide mouse AID-pattern somatic mutations and/or mouse terminal deoxynucleotidyl transferase (TdT)- pattern junctional mutations is to select a variable domain from a non-human vertebrate or cell. For example, a vertebrate or cell as disclosed herein.
11. A vector (eg, a CHO cell or HEK293 cell vector) comprising the nucleic acid of aspect 8, 9 or 10; optionally wherein the vector is in a host cell (eg, a CHO cell or HEK293 cell).
12. A pharmaceutical composition comprising the antibody or polypeptide of any one of aspects 6, 7 and 10, together with a pharmaceutically-acceptable excipient, diluent or a medicament (eg, a further antigen-specific variable domain, antibody chain or antibody).
13. The antibody or polypeptide of any one of aspects 6, 7 and 10 for use in treating and/or preventing a medical condition in a human patient.
14. Use of the antibody or polypeptide of any one of aspects 6, 7 and 10 for the manufacture of a medicament for treating and/or preventing a medical condition in a human patient.
15. The antibody, polypeptide or use of aspect 13 or 14, wherein the human is a member of a human population selected from population numbers 1-14, wherein the populations are numbered as follows (population labels being according to 1000 Genomes Project nomenclature)
   1= ASW;
   2= CEU;
   3=CHB;
   4=CHS;
   5=CLM;
   6=FIN;
   7=GBR;
   8=IBS;
   9=JPT;
   10=LWK;
   11=MXL;
   12=PUR;
   13=TSI;
   14=YRI.
16. The antibody, polypeptide or use of aspect 15, wherein the constant region is a
   (i) IGHA1a constant region and the human population is selected from any population number 1-14;
   (ii) IGHA2a constant region and the human population is selected from any population number 1-14;
   (iii) IGHA2b constant region and the human population is selected from any population number 1-14;
   (iv) IGHG2a constant region and the human population is selected from any population number 1-9 and 11-13;
   (v) IGHG3a constant region and the human population is selected from any population number 1-14;
   (vi) IGHG3b constant region and the human population is selected from any population number 1-8 and 11-13;
   (vii) IGHG4a constant region and the human population is selected from any population number 1-9 and 11-13;
   (viii) IGHMa constant region and the human population is selected from any population number 1-14; or
   (ix) IGHMb constant region and the human population is selected from any population number 1-14;

   Wherein the populations are numbered as follows (population labels being according to 1000 Genomes Project nomenclature)
   1= ASW;
   2= CEU;
   3=CHB;
   4=CHS;
   5=CLM;
   6=FIN;
   7=GBR;
   8=IBS;
   9=JPT;
   10=LWK;
   11=MXL;
   12=PUR;
   13=TSI;
   14=YRI.
17. A vector (eg, a CHO cell or HEK293 cell vector) comprising a IGHG1ref, IGHG2ref, IGHG2a, IGHG3ref, IGHG3a, IGHG3b, IGHG4ref or IGHG4a constant region nucleotide sequence that is 3' of a cloning site for the insertion of a human antibody heavy chain variable domain nucleotide sequence, such that upon insertion of such a variable domain sequence the vector comprises (in 5' to 3' direction) a promoter, a leader sequence, the variable domain sequence and the constant region sequence so that the vector is capable of expressing a human antibody heavy chain when present in a host cell.

### The present disclosure provides in a Sixth configuration-

### Multiple Variants in the Same Genome Cis or Trans

The inventors' analysis has revealed groupings of naturally-occurring human antibody gene segment variants as set out in Table 13 and Table 14. This revealed the possibility of producing transgenic genomes in non-human vertebrates and cells wherein the genomes contain more than the natural human complement of specific human gene segments. In one example, this can be achieved by providing more than the natural human complement of a specific gene segment type on one or both of the respective Ig locus (eg, one or both chromosomes harbouring IgH in a mouse genome or mouse cell genome).

To this end, this configuration of the disclosure provides the following (as set out in numbered paragraphs):-
1. A non-human vertebrate (eg, a mouse or rat) or a non-human vertebrate cell (eg, an ES cell or a B-cell) having a genome comprising at least 3 human variable region gene segments of the same type (eg, at least 3 human VH6-1 gene segments, at least 3 human JH6 gene segments, at least 3 human Vκ1-39 gene segments, at least 3 human D2-2 gene segments or at least 3 human Jκ1 gene segments), wherein at least two of the human gene segments are variants that are not identical to each other.
   For example, the genome comprises a variable region that comprises V, D and J gene segments (for the variable region of a heavy chain locus) or V and J gene segments (for the variable region of a light chain locus) upstream of a constant region for expression of heavy or light chains respectively.
   In an alternative, the skilled person can choose to provide more than the wild type human complement of a specific gene segment type by providing several copies of one variant type of the human gene segment. Thus, there is provided
   A non-human vertebrate (eg, a mouse or rat) or a non-human vertebrate cell (eg, an ES cell or a B-cell) having a genome comprising at least 3 human variable region gene segments of the same type (eg, at least 3 human VH6-1 gene segments, at least 3 human JH6 gene segments, at least 3 human Vκ1-39 gene segments, at least 3 human D2-2 gene segments or at least 3 human Jκ1 gene segments), wherein the human gene segments are identical variants.
   For example, the genome comprises a variable region that comprises V, D and J gene segments (for the variable region of a heavy chain locus) or V and J gene segments (for the variable region of a light chain locus) upstream of a constant region for expression of heavy or light chains respectively.
2. A non-human vertebrate (eg, a mouse or rat) or a non-human vertebrate cell (eg, an ES cell or a B-cell) having a genome comprising at least 2 different non-endogenous variable region gene segments of the same type (eg, at least 2 human VH6-1 gene segments, at least 3 human JH6 gene segments, at least 2 human Vκ1-39 gene segments, at least 2 human D2-2 gene segments or at least 2 human Jκ1 gene segments) cis at the same Ig locus.
   In an alternative, the skilled person can choose to provide more than the wild type human complement of a specific gene segment type by providing several copies of one variant type of the human gene segment. Thus, there is provided
   A non-human vertebrate (eg, a mouse or rat) or a non-human vertebrate cell (eg, an ES cell or a B-cell) having a genome comprising at least 2 non-endogenous variable region gene segments of the same variant type (eg, at least 2 human JH6*02 gene segments) *cis* at the same Ig locus.
3. A non-human vertebrate (eg, a mouse or rat) or a non-human vertebrate cell (eg, an ES cell or a B-cell) having a genome comprising at least 2 different human variable region gene segments of the same type (eg, at least 2 human VH6-1 gene segments, at least 2 human JH6 gene segments, at least 2 human Vκ1-39 gene segments, at least 2 human D2-2 gene segments or at least 2 human Jκ1 gene segments) *trans* at the same Ig locus; and optionally a third human gene segment of the same type, wherein the third gene segment is *cis* with one of said 2 different gene segments.
   In an alternative, the skilled person can choose to provide more than the wild type human complement of a specific gene segment type by providing several copies of one variant type of the human gene segment. Thus, there is provided
   A non-human vertebrate (eg, a mouse or rat) or a non-human vertebrate cell (eg, an ES cell or a B-cell) having a genome comprising at least 2 different human variable region gene segments of the same variant type (eg, at least 2 human JH6*02 gene segments) *trans* at the same Ig locus; and optionally a third human gene segment of the same variant type, wherein the third gene segment is *cis* with one of said 2 different gene segments.
4. A population of non-human vertebrates (eg, mice or rats) comprising a repertoire of human variable region gene segments, wherein the plurality comprises at least 2 human variable region gene segments of the same type (eg, at least 2 human VH6-1 gene segments, at least 2 human JH6 gene segments, at least 2 human Vκ1-39 gene segments, at least 2 human D2-2 gene segments or at least 2 human Jκ1 gene segments), a first of said different gene segments is provided in the genome of a first vertebrate of the population, and a second of said different gene segments being provided in the genome of a second vertebrate of the population, wherein the genome of the first vertebrate does not comprise the second gene segment.
5. A non-human vertebrate (eg, a mouse or rat) or a non-human vertebrate cell (eg, an ES cell or a B-cell) having a genome comprising at least 2 different non-endogenous variable region gene segments of the same type (eg, at least 2 human VH6-1 gene segments, at least 2 human JH6 gene segments, at least 2 human Vκ1-39 gene segments, at least 2 human D2-2 gene segments or at least 2 human Jκ1 gene segments), wherein the gene segments are derived from the genome sequence of different human individuals that are not genetically related over at least 3 generations.
6. A method of enhancing the human immunoglobulin gene diversity of a non-human vertebrate (eg, a mouse or rat), the method comprising providing the vertebrate with a genome comprising at least 3 human variable region gene segments of the same type (eg, at least 3 human VH6-1 gene segments, at least 3 human JH6 gene segments, at least 3 human Vκ1-39 gene segments, at least 3 human D2-2 gene segments or at least 3 human Jκ1 gene segments), wherein at least two of the human gene segments are variants that are not identical to each other.
7. A method of enhancing the immunoglobulin gene diversity of a non-human vertebrate (eg, a mouse or rat), the method comprising providing the vertebrate with a genome comprising at least 2 different non-endogenous variable region gene segments of the same type (eg, at least 2 human VH6-1 gene segments, at least 2 human JH6 gene segments, at least 2 human Vκ1-39 gene segments, at least 2 human D2-2 gene segments or at least 2 human Jκ1 gene segments) cis at the same Ig locus.
8. A method of enhancing the immunoglobulin gene diversity of a non-human vertebrate (eg, a mouse or rat), the method comprising providing the vertebrate with a genome comprising at least 2 different human variable region gene segments of the same type (eg, at least 2 human VH6-1 gene segments, at least 2 human JH6 gene segments, at least 2 human Vκ1-39 gene segments, at least 2 human D2-2 gene segments or at least 2 human Jκ1 gene segments) κ at the same Ig locus; and optionally a third human gene segment of the same type, wherein the third gene segment is κ with one of said 2 different gene segments.
9. A method of providing an enhanced human immunoglobulin variable region gene segment repertoire, the method comprising providing a population of non-human vertebrates (eg, a mouse or rat) comprising a repertoire of human variable region gene segments, wherein the method comprises providing at least 2 different human variable region gene segments of the same type (eg, at least 2 human VH6-1 gene segments, at least 2 human JH6 gene segments, at least 2 human Vκ1-39 gene segments, at least 2 human D2-2 gene segments or at least 2 human Jκ1 gene segments), wherein a first of said different gene segments is provided in the genome of a first vertebrate of the population, and a second of said different gene segments is provided in the genome of a second vertebrate of the population, wherein the genome of the first vertebrate does not comprise the second gene segment.
10. A method of enhancing the human immunoglobulin gene diversity of a non-human vertebrate (eg, a mouse or rat), the method comprising providing the vertebrate with a genome comprising at least 2 different non-endogenous variable region gene segments of the same type (eg, at least 2 human VH6-1 gene segments, at least 2 human JH6 gene segments, at least 2 human Vκ1-39 gene segments, at least 2 human D2-2 gene segments or at least 2 human Jκ1 gene segments), wherein the gene segments are derived from the genome sequence of different human individuals that are not genetically related over at least 3 generations.
11. The vertebrate, cell or method of any preceding paragraph, wherein at least 2 or 3 of said different gene segments are provided *cis* at the same Ig locus in said genome.
12. The vertebrate, cell or method of any preceding paragraph, wherein the gene segments are derived from the genome sequence of different human individuals that are not genetically
   related over at least 3 generations.
13. The vertebrate, cell or method of any preceding paragraph, wherein the gene segments are derived from the genome sequence of two or more different human individuals; optionally wherein the different human individuals are from different human populations.
14. The vertebrate, cell or method of paragraph 13, wherein the individuals are not genetically related.
15. A method of enhancing the human immunoglobulin gene diversity of a non-human vertebrate (eg, a mouse or rat), the method comprising providing the vertebrate with a genome comprising at least 2 human variable region gene segments of the same type (eg, at least 2 human VH6-1 gene segments, at least 2 human JH6 gene segments, at least 2 human Vκ1-39 gene segments, at least 2 human D2-2 gene segments or at least 2 human Jκ1 gene segments), wherein the gene segments are derived from the genome sequence of different human individuals that are not genetically related over at least 3 generations; optionally wherein at least 2 or 3 of said different gene segments are provided at the same Ig locus in said genome.
16. The method of paragraph 15, wherein the different human individuals are from different human populations.
17. The method of paragraph 15, wherein the individuals are not genetically related.
18. The vertebrate, cell or method of preceding paragraph, wherein at least one of the different segments is a synthetic mutant of a human germline gene segment.
19. The vertebrate, cell or method of any preceding paragraph, wherein each of said gene segments occurs in 10 or more different human populations.
20. The vertebrate, cell or method of preceding paragraph, wherein each of said gene segments has a human frequency of 5% or greater (eg, 10, 20, 25, 30, 35, 40, 45, 50, 55, 60, 65, 70, 75, 80, 85, 90, or 95% or greater).
   In this respect, the skilled person can be guided by the information provided in Table 14.
   Frequency can, for example, be cumulative frequency in the 1000 Genomes database.
21. The vertebrate, cell or method of paragraph 20, wherein each of said gene segments occurs in 10 or more different human populations.
22. The vertebrate, cell or method of any preceding paragraph, wherein each of said gene segments occurs in the 1000 Genomes database in more than 50 individuals.
23. The vertebrate, cell or method of preceding paragraph, wherein each of said gene segments (i) has a human frequency of 5% or greater (eg, 10, 20, 25, 30, 35, 40, 45, 50, 55, 60, 65, 70, 75, 80, 85, 90, or 95% or greater); and (ii) occurs in 10 or more different human populations.
   In this respect, the skilled person can be guided by the information provided in Table 14.
   Frequency can, for example, be cumulative frequency in the 1000 Genomes database.
24. A non-human vertebrate (eg, a mouse or rat) or a non-human vertebrate cell (eg, an ES cell or a B-cell) having a genome comprising first and second human Ig locus gene segments of the same type (eg, first and second human JH6 gene segments; or first and second IgG2 gene segments; or first and second human Jλ7 gene segments), wherein the first gene segment is a gene segment selected from Table 14 (eg, IGHJ6-a) and the second gene segment is the corresponding reference sequence (eg, IGHJ6 ref; SEQ ID NO: 244).
   Table 14 lists commonly-occurring natural human variants. It can be seen that these occur across many human populations and thus usefully have wide applicability for human antibody- based drugs.
   For example, the gene segments are provided as targeted insertions into an endogenous non- human vertebrate Ig locus. Alternatively, random integration (eg, using YACs) as is known in the art can be performed.
   For example, the genome comprises a variable region that comprises V, D and J gene segments (for the variable region of a heavy chain locus) or V and J gene segments (for the variable region of a light chain locus) upstream of a constant region for expression of heavy or light chains respectively.
   In another embodiment, the disclosure enables the skilled person to select two or more different naturally-occurring human gene segment variants for combination into the genome of a non- human vertebrate or cell. A reference sequence need not be included. It may be desirable to use one or more rare gene segments to increase diversity of the repertoire. Additionally or alternatively, it may be desirable to include a mixture of frequent and rare variants of the same type to provide repertoire diversity. The variants may be chosen additionally or alternatively to tailor the gene segment inclusion to one or more specific human populations as indicated by the information provided in Table 13 or Table 14.
   Thus, the disclosure provides
   A non-human vertebrate (eg, a mouse or rat) or a non-human vertebrate cell (eg, an ES cell or a B-cell) having a genome comprising first and second human Ig locus gene segments of the same type (eg, first and second human JH6 gene segments; or first and second IgG2 gene segments; or first and second human Jλ7 gene segments), wherein the gene segments are gene segments selected from Table 13 or Table 14; and optionally wherein one or more of the gene segments appears in Table 14 (eg, IGHJ6-a) or is a reference sequence (eg, IGHJ6 ref; SEQ ID NO: 244).
25. The vertebrate or cell of paragraph 24, wherein the genome comprises a third human gene segment of said type, the third gene segment being different from the first and second gene segments.
26. The vertebrate or cell of paragraph 24 or 25, wherein the first and second gene segments are cis on the same chromosome; and optionally the third gene segment is also cis on said chromosome.
27. The vertebrate or cell of paragraph 26, wherein the gene segments are targeted insertions into an endogenous non-human Ig locus.
   For example, the gene segments are heavy chain gene segments and the non-human locus is an IgH locus. For example, the gene segments are light chain (kappa or lambda) gene segments and the non-human locus is an IgL locus.
28. The vertebrate or cell of paragraph 24 or 25, wherein the first and second gene segments are *trans* on different chromosomes.
   Thus, the chromosomes are the same type (eg, both mouse chromosome 6 or rat chromosome 4).
29. The vertebrate or cell of any one of paragraphs 24 to 28, wherein the first gene segment is a gene segment selected from any one of Tables 1 to 7 and 9 to 14 (eg, selected from Table 13 or 14) and the second gene segment is the corresponding reference sequence.
30. A population of non-human vertebrates (eg, mice or rats) comprising first and second human Ig locus gene segments of the same type (eg, first and second human JH6 gene segments; or first and second IgG2 gene segments; or first and second human Jλ7 gene segments), wherein the first gene segment is a gene segment selected from any one of Tables 1 to 7 and 9 to 14 (eg, Table 13 or 14) (eg, IGHJ6-a) and the second gene segment is the corresponding reference sequence (eg, SEQ ID NO: 7), wherein the first gene segment is provided in the genome of a first vertebrate of the population, and the second gene segment is provided in the genome of a second vertebrate of the population.
31. The population of paragraph 30, wherein the genome of the first vertebrate does not comprise the second gene segment.
32. The population of paragraph 30 or 31, wherein the population comprises a third human gene segment of said type, the third gene segment being different from the first and second gene segments and optionally wherein the first and third gene segments are present in the genome of the first vertebrate.
33. The population of paragraph 30, 31 or 32, wherein the gene segments are targeted insertions into an endogenous non-human Ig locus in the respective genome.
   For example, the gene segments are heavy chain gene segments and the non-human locus is an IgH locus. For example, the gene segments are light chain (kappa or lambda) gene segments and the non-human locus is an IgL locus.
34. The population of any one of paragraphs 30 to 33, wherein the first gene segment is a gene segment selected from any one of Tables 1 to 7 and 9 to 14 (eg, Table 13 or 14) and the second gene segment is the corresponding reference sequence.
35. A method of enhancing the human immunoglobulin gene diversity of a non-human vertebrate (eg, a mouse or rat), the method comprising providing the vertebrate with a genome comprising first and second human Ig locus gene segments of the same type (eg, first and second human JH6 gene segments; or first and second IgG2 gene segments; or first and second human Jλ7 gene segments), wherein the first gene segment is a gene segment selected from any one of Tables 1 to 7 and 9 to 14 (eg, Table 13 or 14) (eg, IGHJ6-a) and the second gene segment is the corresponding reference sequence (eg, SEQ ID NO: 7).
36. A method of providing an enhanced human immunoglobulin gene segment repertoire, the method comprising providing a population according to any one of paragraphs 30 to 33.

### Variants Prevalent in Few Populations

In another aspect, it is of note that certain human gene segment variants may appear relatively frequently in one or a small number of populations, but is not found prevalently across many different human populations. There is thinking that specific germline gene segment repertoires have evolved in individual human ethnic populations due to iterative exposure to antigens (eg, disease pathogen antigens) to which the population is often exposed. Repeated exposure and mutation may have led to the evolution of gene segment variants that can provide an effective response to the antigen (pathogen) in the population, and this may explain the conservation of the gene segments in those populations (as opposed to other human ethnic populations that may not have frequently encountered the antigen). With this in mind, the inventors identified gene segment variants from their analysis that are relatively prevalent in a small number of human populations, and not across many populations. The inventors realized that inclusion of one or more of such gene segments in the configurations of the disclosure (eg, in transgenic Ig loci, vertebrates and cells) would be useful for producing antibodies, Ig chains and variable domains that can address antigens (eg, disease- causing antigens or pathogens) to which the small number of human populations may become exposed. Such products would be useful for treating and/or preventing disease or medical conditions in members of such a population. This aspect could also be useful for addressing infectious disease pathogens that may have been common in the small number of populations, but which in the future or relatively recently in evolution has become a more prevalent disease-causing pathogen in other human populations (ie, those not listed in Table 13 against the gene segment variant(s) in question). To this end, from the 1000 Genomes database the inventors have identified the gene segment variants listed in Table 20.

Thus, according to any configuration or aspect described herein, one, more or all of the gene segments used in the present disclosure can be a gene segment listed in Table 20A, 20B, 20C or 20D.

### Multiple JH Gene Segment Variants

A specific application of this configuration is the provision of multiple human JH gene segments as follows.

A non-human vertebrate (eg, a mouse or rat) or a non-human vertebrate cell (eg, an ES cell or a B- cell) having a genome comprising at least 3 human JH gene segments of the same type (JH1, JH2, JH3, JH4, JH5 or JH6), wherein at least two of the human JH gene segments are variants that are not identical to each other.

In an example, any cell of the disclosure is an isolated cell. An "isolated" cell is one that has been identified, separated and/or recovered from a component of its production environment (eg, naturally or recombinantly). Preferably, the isolated cell is free of association with all other components from its production environment, eg, so that the cell can produce an antibody to an FDA-approvable or approved standard. Contaminant components of its production environment, such as that resulting from recombinant transfected cells, are materials that would typically interfere with research, diagnostic or therapeutic uses for the resultant antibody, and may include enzymes, hormones, and other proteinaceous or non-proteinaceous solutes. In preferred embodiments, the polypeptide will be purified: (1) to greater than 95% by weight of antibody as determined by, for example, the Lowry method, and in some embodiments, to greater than 99% by weight; (2) to a degree sufficient to obtain at least 15 residues of N-terminal or internal amino acid sequence by use of a spinning cup sequenator, or (3) to homogeneity by SDS-PAGE under non- reducing or reducing conditions using Coomassie blue or, preferably, silver stain. Ordinarily, however, an isolated cell will be prepared by at least one purification step.

A non-human vertebrate (eg, a mouse or rat) or a non-human vertebrate cell (eg, an ES cell or a B- cell) having a genome comprising at least 2 different non-endogenous JH gene segments (eg, human gene segments) of the same type (JH1, JH2, JH3, JH4, JH5 or JH6) *cis* at the same Ig (eg, IgH, eg, endogenous IgH, eg, mouse or rat IgH) locus. In an example, the genome comprises a human VH, D and JH repertoire comprising said different JH gene segments. Optionally the non-endogenous JH gene segments are non-mouse or non-rat, eg, human JH gene segments. In an example one or more or all of the non-endogenous gene segments are synthetic.

A non-human vertebrate (eg, a mouse or rat) or a non-human vertebrate cell (eg, an ES cell or a B- cell) having a genome comprising at least 2 different human JH gene segments of the same type (JH1, JH2, JH3, JH4, JH5 or JH6) *trans* at the same Ig (eg, IgH, eg, endogenous IgH, eg, mouse or rat IgH) locus; and optionally a third human JH gene segments of the same type, wherein the third JH is cis with one of said 2 different JH gene segments.

A population of non-human vertebrates (eg, mice or rats) comprising a repertoire of human JH gene segments, wherein the plurality comprises at least 2 different human JH gene segments of the same type (JH1, JH2, JH3, JH4, JH5 or JH6), a first of said different JH gene segments is provided in the genome of a first vertebrate of the population, and a second of said different JH gene segments being provided in the genome of a second vertebrate of the population, wherein the genome of the first vertebrate does not comprise the second JH gene segment.

A non-human vertebrate (eg, a mouse or rat) or a non-human vertebrate cell (eg, an ES cell or a B- cell) having a genome comprising at least 2 different non-endogenous (eg, human) JH gene segments of the same type (JH1, JH2, JH3, JH4, JH5 or JH6), wherein the JH gene segments are derived from the genome sequence of different human individuals that are not genetically related over at least 3 generations (eg, 3, 4, 5 or 6 generations). Optionally the non-endogenous JH gene segments are human JH gene segments. In an example one or more or all of the non-endogenous gene segments are synthetic.

A method of enhancing the human immunoglobulin gene diversity of a non-human vertebrate (eg, a mouse or rat), the method comprising providing the vertebrate with a genome comprising at least 3 human JH gene segments of the same type (JH1, JH2, JH3, JH4, JH5 or JH6), wherein at least two of the human JH gene segments are variants that are not identical to each other.

A method of enhancing the immunoglobulin gene diversity of a non-human vertebrate (eg, a mouse or rat), the method comprising providing the vertebrate with a genome comprising at least 2 different non-endogenous (eg, human) JH gene segments of the same type (JH1, JH2, JH3, JH4, JH5 or JH6) *cis* at the same Ig (eg, IgH, eg, endogenous IgH, eg, mouse or rat IgH) locus). Optionally the non-endogenous JH gene segments are non-mouse or non-rat, eg, human JH gene segments. In an example one or more or all of the non-endogenous gene segments are synthetic.

A method of enhancing the immunoglobulin gene diversity of a non-human vertebrate (eg, a mouse or rat), the method comprising providing the vertebrate with a genome comprising at least 2 different human JH gene segments of the same type (JH1, JH2, JH3, JH4, JH5 or JH6) *trans* at the same Ig (eg, IgH, eg, endogenous IgH, eg, mouse or rat IgH) locus; and optionally a third human JH gene segments of the same type, wherein the third JH is *cis* with one of said 2 different JH gene segments.

A method of providing an enhanced human immunoglobulin JH gene segment repertoire, the method comprising providing a population of non-human vertebrates (eg, a mouse or rat) comprising a repertoire of human JH gene segments, wherein the method comprises providing at least 2 different human JH gene segments of the same type (JH1, JH2, JH3, JH4, JH5 or JH6), wherein a first of said different JH gene segments is provided in the genome of a first vertebrate of the population, and a second of said different JH gene segments is provided in the genome of a second vertebrate of the population, wherein the genome of the first vertebrate does not comprise the second JH gene segment.

A method of enhancing the human immunoglobulin gene diversity of a non-human vertebrate (eg, a mouse or rat), the method comprising providing the vertebrate with a genome comprising at least 2 different non-endogenous (eg, human) JH gene segments of the same type (JH1, JH2, JH3, JH4, JH5 or JH6), wherein the JH gene segments are derived from the genome sequence of different human individuals that are not genetically related over at least 3 generations (eg, 3, 4, 5, or 6 generations). Optionally the non-endogenous JH gene segments are human JH gene segments. In an example one or more or all of the non-endogenous gene segments are synthetic.

In an example of the vertebrate or cell or the method of the disclosure at least 2 or 3 of said different gene segments are provided *cis* at the same Ig locus in said genome.

In an example of the vertebrate or cell or the method of the disclosure the JH gene segments are derived from the genome sequence of different human individuals that are not genetically related over at least 3 generations (eg, 3, 4, 5, or 6 generations).

In an example of the vertebrate or cell or the method of the disclosure the JH gene segments are derived from the genome sequence of two or more different human individuals; optionally wherein the different human individuals are from different human populations.

In an example of the vertebrate or cell or the method of the disclosure the individuals are not genetically related (eg, going back 3, 4, 5, or 6 generations).

In an example of the vertebrate or cell or the method of the disclosure at least one of the different JH segments is a synthetic mutant of a human germline JH gene segment.

The disclosure also provides a method of enhancing the human immunoglobulin gene diversity of a non-human vertebrate (eg, a mouse or rat), the method comprising providing the vertebrate with a genome comprising at least 2 human JH gene segments of the same type (JH1, JH2, JH3, JH4, JH5 or JH6), wherein the JH gene segments are derived from the genome sequence of different human individuals that are not genetically related over at least 3 generations (eg, 3, 4, 5, or 6 generations); optionally wherein at least 2 or 3 of said different gene segments are provided at the same IgH locus in said genome.

In an example of the vertebrate or cell or the method of this embodiment of the disclosure the genome comprises a substantially complete functional repertoire of human JH gene segment types supplemented with one, two or more human JH gene segments, wherein said substantially complete functional repertoire and the supplementary JH gene segments are not found together in the germline genome of a human individual.

In an example of the population of the disclosure, the population comprises a substantially complete functional repertoire of human JH gene segment types supplemented with one, two or more human JH gene segments, wherein said substantially complete functional repertoire and the supplementary JH gene segments are not found together in the germline genome of a human individual.

A non-human vertebrate (eg, a mouse or rat) or a non-human cell (eg, an ES cell or a B-cell) having a genome comprising a substantially complete functional repertoire of human JH gene segment types supplemented with one, two or more human JH gene segments, wherein said substantially complete functional repertoire and the supplementary JH gene segments are not found together in the germline genome of a human individual.

A population of non-human vertebrates (eg, mice or rats) comprising a substantially complete functional repertoire of human JH gene segment types supplemented with one, two or more human JH gene segments, wherein said substantially complete functional repertoire and the supplementary JH gene segments are not found together in the germline genome of a human individual.

In an example of the vertebrate or the population, at least one of said JH gene segments is SEQ ID NO: 1, 2, 3 or 4. For example, at least one of said JH gene segments is SEQ ID NO: 1 and at least one, two or more of said supplementary JH gene segments is a variant according to any example above. For example, at least one of said JH gene segments is SEQ ID NO: 2 and at least one, two or more of said supplementary JH gene segments is a variant according to any one of the examples above. For example, at least one of said JH gene segments is SEQ ID NO: 2 and at least one, two or more of said supplementary JH gene segments is a variant according to any one of the examples above.

In an embodiment, the non-human vertebrate or vertebrate cell of the disclosure comprises a genome that comprises VH, D and JH gene repertoires comprising human gene segments, the JH gene repertoire (eg, a human JH gene segment repertoire) comprising
a plurality of JH1 gene segments provided by at least 2 different JH1 gene segments in *cis* at the same Ig locus in said genome;
a plurality of JH2 gene segments provided by at least 2 different JH2 gene segments in *cis* at the same Ig locus in said genome;
a plurality of JH3 gene segments provided by at least 2 different JH3 gene segments in *cis* at the same Ig locus in said genome;
a plurality of JH4 gene segments provided by at least 2 different JH4 gene segments in *cis* at the same Ig locus in said genome;
a plurality of JH5 gene segments provided by at least 2 different JH5 gene segments in *cis* at the same Ig locus in said genome; and/or
a plurality of JH6 gene segments provided by at least 2 different JH6 gene segments in *cis* at the same Ig locus in said genome;
optionally wherein the JH gene segments are derived from the genome sequence of two or more different human individuals.

Optionally said at least 2 different JH gene segments are human gene segments or synthetic gene segments derived from human gene segments.

Optionally, the Ig locus is a IgH locus, eg, an endogenous locus, eg, a mouse or rat IgH locus.

In an embodiment, the non-human vertebrate or vertebrate cell of the disclosure comprises a genome that comprises VH, D and JH gene repertoires comprising human gene segments, the JH gene repertoire (eg, a human JH gene segment repertoire) comprising
a plurality of JH1 gene segments provided by at least 3 different JH1 gene segments;
a plurality of JH2 gene segments provided by at least 3 different JH2 gene segments;
a plurality of JH3 gene segments provided by at least 3 different JH3 gene segments;
a plurality of JH4 gene segments provided by at least 3 different JH4 gene segments;
a plurality of JH5 gene segments provided by at least 3 different JH5 gene segments; and/or
a plurality of JH6 gene segments provided by at least 3 different JH6 gene segments;
optionally wherein the JH gene segments are derived from the genome sequence of two or three different human individuals;
optionally wherein at least 2 or 3 of said different gene segments are provided in *cis* at the same Ig locus in said genome.

Optionally said at least 3 different JH gene segments are human gene segments or synthetic gene segments derived from human gene segments.

Optionally, the Ig locus is a IgH locus, eg, an endogenous locus, eg, a mouse or rat IgH locus.

Optionally in the vertebrate or cell the different human individuals are from different human populations.

Optionally in the vertebrate or cell the individuals are not genetically related (eg, Going back 3, 4, 5 or 6generations).

Optionally in the vertebrate or cell at least one of the different JH segments is a synthetic mutant of a human germline JH gene segment.

In an embodiment of a non-human vertebrate or vertebrate cell (optionally an ES cell or B-cell) according to the disclosure, the vertebrate or cell genome comprises human VH, D and JH gene repertoires, the JH gene repertoire (eg, a human JH gene repertoire) comprising
a plurality of JH1 gene segments provided by at least 2 different human JH1 gene segments, optionally in *cis* at the same Ig locus in said genome;
a plurality of JH2 gene segments provided by at least 2 different human JH2 gene segments, optionally in *cis* at the same Ig locus in said genome;
a plurality of JH3 gene segments provided by at least 2 different human JH3 gene segments, optionally in *cis* at the same Ig locus in said genome;
a plurality of JH4 gene segments provided by at least 2 different human JH4 gene segments, optionally in *cis* at the same Ig locus in said genome;
a plurality of JH5 gene segments provided by at least 2 different human JH5 gene segments, optionally in *cis* at the same Ig locus in said genome; and/or
a plurality of JH6 gene segments provided by at least 2 different human JH6 gene segments, optionally in *cis* at the same Ig locus in said genome;
wherein the JH gene segments are derived from the genome sequence of different human individuals that are not genetically related over at least 3 generations (eg, 3, 4, 5 or 6 generations).

Optionally said at least 2 different JH gene segments are human gene segments or synthetic gene segments derived from human gene segments.

Optionally, the Ig locus is a IgH locus, eg, an endogenous locus, eg, a mouse or rat IgH locus. Optionally in the vertebrate or cell the human individuals are from different human populations.

### JH5

An embodiment provides a vertebrate, cell or population of the disclosure whose genome comprises a plurality of JH5 gene segments, wherein the plurality comprises a human JH5 gene variant of SEQ ID NO: 1, wherein the variant comprises a nucleotide mutation at one or more positions
corresponding to positions
106,330,024
106,330,027
106,330,032
106,330,041
106.330.44
106.330.45
106.330.62
106.330.63
106.330.65
106.330.66
106.330.67
106.330.68 and
106,330,071
on human chromosome 14.

In the vertebrate, cell or population optionally the plurality comprises a human JH5 gene variant of SEQ ID NO: 1, wherein the variant comprises a guanine at a position corresponding to position 106,330,067 on human chromosome 14; and optionally no further mutation from the sequence of SEO ID NO: 1.

Optionally the variant comprises additionally a mutation at a position corresponding to (i) position 106,330,071 on human chromosome 14 (optionally the additional mutation being a guanine); (ii) position 106,330,066 on human chromosome 14 (optionally the additional mutation being a guanine); and/or (iii) position 106,330,068 on human chromosome 14 (optionally the additional mutation being a thymine).

Optionally the plurality comprises a human JH5 gene variant of SEQ ID NO: 1, wherein the variant comprises a guanine at a position corresponding to position 106,330,071 on human chromosome 14; and optionally no further mutation from the sequence of SEQ ID NO: 1.

Optionally the variant comprises additionally a mutation at a position corresponding to (i) position 106,330,063 on human chromosome 14 (optionally the additional mutation being an adenine); and/or (ii) position 106,330,067 on human chromosome 14 (optionally the additional mutation being a guanine).

Optionally the plurality comprises a human JH5 gene variant of SEQ ID NO: 1, wherein the variant comprises a cytosine at a position corresponding to position 106,330,045 on human chromosome 14; and optionally no further mutation from the sequence of SEQ ID NO: 1.

Optionally the plurality comprises a human JH5 gene variant of SEQ ID NO: 1, wherein the variant comprises an adenine at a position corresponding to position 106,330,044 on human chromosome 14; and optionally no further mutation from the sequence of SEQ ID NO: 1.

Optionally the variant comprises additionally a mutation at a position corresponding to (i) position 106.330.66 on human chromosome 14 (optionally the additional mutation being a guanine); and/or (ii) position 106,330,068 on human chromosome 14 (optionally the additional mutation being a thymine).

Optionally the plurality comprises a human JH5 gene variant of SEQ ID NO: 1, wherein the variant comprises a guanine at a position corresponding to position 106,330,066 on human chromosome 14; and optionally no further mutation from the sequence of SEQ ID NO: 1.

Optionally the variant comprises additionally a mutation at a position corresponding to (i) position 106.330.67 on human chromosome 14 (optionally the additional mutation being a guanine); and/or (ii) position 106,330,068 on human chromosome 14 (optionally the additional mutation being a thymine).

Optionally the plurality comprises a human JH5 gene variant of SEQ ID NO: 1, wherein the variant comprises a thymine at a position corresponding to position 106,330,068 on human chromosome 14; and optionally no further mutation from the sequence of SEQ ID NO: 1.

Optionally the variant comprises additionally a mutation at a position corresponding to (i) position 106,330,067 on human chromosome 14 (optionally the additional mutation being a guanine); and/or (ii) position 106,330,066 on human chromosome 14 (optionally the additional mutation being a guanine).

Optionally the plurality comprises a human JH5 gene variant of SEQ ID NO: 1, wherein the variant comprises a cytosine at a position corresponding to position 106,330,027 on human chromosome 14; and optionally no further mutation from the sequence of SEQ ID NO: 1.

Optionally the plurality comprises a human JH5 gene variant of SEQ ID NO: 1, wherein the variant comprises an adenine at a position corresponding to position 106,330,024 on human chromosome 14; and optionally no further mutation from the sequence of SEQ ID NO: 1.

Optionally the plurality comprises a human JH5 gene variant of SEQ ID NO: 1, wherein the variant comprises a thymine at a position corresponding to position 106,330,032 on human chromosome 14; and optionally no further mutation from the sequence of SEQ ID NO: 1.

Optionally the plurality comprises a human JH5 gene variant of SEQ ID NO: 1, wherein the variant comprises a thymine at a position corresponding to position 106,330,041 on human chromosome 14; and optionally no further mutation from the sequence of SEQ ID NO: 1.

Optionally the plurality comprises a human JH5 gene variant of SEQ ID NO: 1, wherein the variant comprises an adenine or thymine at a position corresponding to position 106,330,063 on human chromosome 14; and optionally no further mutation from the sequence of SEQ ID NO: 1.

Optionally the variant comprises additionally a mutation at a position corresponding to position 106,330,071 on human chromosome 14 (optionally the additional mutation being a guanine).

Optionally the plurality comprises a human JH5 gene variant of SEQ ID NO: 1, wherein the variant comprises a cytosine at a position corresponding to position 106,330,062 on human chromosome
14; and optionally no further mutation from the sequence of SEQ ID NO: 1.

Optionally the genome comprises SEQ ID NO:1; optionally in cis at the same Ig locus as one, two or more of the variants.

### JH6

An embodiment provides a vertebrate, cell or population of the disclosure whose genome comprises a plurality of JH6 gene segments, wherein the plurality comprises a human JH6 gene variant of SEQ ID NO: 2, wherein the variant comprises a nucleotide mutation at one or more positions corresponding to positions
106,329,411
106.329.413
106.329.414
106,329,417
106,329,419
106,329,426
106,329,434
106,329,435, and
106,329,468
on human chromosome 14.

Optionally the genome of the vertebrate, cell or population comprises a plurality of JH6 gene segments, wherein the plurality comprises a human JH6 gene variant of SEQ ID NO: 2, wherein the variant comprises a guanine at a position corresponding to position 106,329,435 on human chromosome 14; and optionally no further mutation from the sequence of SEQ ID NO: 2.

Optionally the variant comprises additionally a mutation at a position corresponding to (i) position 106,329,468 on human chromosome 14 (optionally the additional mutation being a guanine); (ii) position 106,329,419 on human chromosome 14 (optionally the additional mutation being an adenine); (iii) position 106,329,434 on human chromosome 14 (optionally the additional mutation being a cytosine) and/or position 106,329,414 on human chromosome 14 (optionally the additional mutation being a guanine); (iv) position 106,329,426 on human chromosome 14 (optionally the additional mutation being an adenine); (v) position 106,329,413 on human chromosome 14 (optionally the additional mutation being an adenine); (vi) position 106,329,417 on human chromosome 14 (optionally the additional mutation being a thymine); (vii) position 106,329,411 on human chromosome 14 (optionally the additional mutation being a thymine); (viii) position 106,329,451 on human chromosome 14 (optionally the additional mutation being an adenine); (ix) position 106,329,452 on human chromosome 14 (optionally the additional mutation being a cytosine); and/or (x) position 106,329,453 on human chromosome 14 (optionally the additional mutation being a cytosine).

Optionally the variant comprises additionally mutations at positions corresponding to position 106.329.451 on human chromosome 14, the additional mutation being an adenine; position 106.329.452 on human chromosome 14, the additional mutation being a cytosine; and position 106.329.453 on human chromosome 14, the additional mutation being a cytosine.

The vertebrate, cell or population optionally comprises a plurality of JH6 gene segments" wherein the plurality comprises a human JH6 gene variant of SEQ ID NO: 2, wherein the variant comprises a guanine at a position corresponding to position 106,329,468 on human chromosome 14; and optionally no further mutation from the sequence of SEQ ID NO: 2.

Optionally the variant comprises additionally a mutation at a position corresponding to position 106,329,435 on human chromosome 14 (optionally the additional mutation being a guanine).

Optionally the vertebrate, cell or population comprises a plurality of JH6 gene segments,, wherein the plurality comprises a human JH6 gene variant of SEQ ID NO: 2, wherein the variant comprises a thymine at a position corresponding to position 106,329,417 on human chromosome 14; and optionally no further mutation from the sequence of SEQ ID NO: 2.

Optionally the variant comprises additionally a mutation at a position corresponding to position 106,329,435 on human chromosome 14 (optionally the additional mutation being a guanine).

Optionally the vertebrate, cell or population comprises a plurality of JH6 gene segments,, wherein the plurality comprises a human JH6 gene variant of SEQ ID NO: 2, wherein the variant comprises a cytosine at a position corresponding to position 106,329,434 on human chromosome 14; and optionally no further mutation from the sequence of SEQ ID NO: 2.

Optionally the variant comprises additionally a mutation at a position corresponding to (i) position 106,329,414 on human chromosome 14 (optionally the additional mutation being a guanine); and/or (ii) position 106,329,435 on human chromosome 14 (optionally the additional mutation being a guanine).

Optionally the vertebrate, cell or population comprises a plurality of JH6 gene segments, wherein the plurality comprises a human JH6 gene variant of SEQ ID NO: 2, wherein the variant comprises a thymine at a position corresponding to position 106,329,411 on human chromosome 14; and optionally no further mutation from the sequence of SEQ ID NO: 2.

Optionally the variant comprises additionally a mutation at a position corresponding to position 106,329,435 on human chromosome 14 (optionally the additional mutation being a guanine).

Optionally the vertebrate, cell or population comprises a plurality of JH6 gene segments, wherein the plurality comprises a human JH6 gene variant that is an antisense sequence of a variant described above.

Optionally the genome comprises SEQ ID NO:2; optionally *cis* at the same Ig locus as one, two or more of the JH6 variants.

### JH2

An embodiment provides a vertebrate, cell or population of the disclosure whose genome comprises a plurality of JH2 gene segments, wherein the plurality comprises a human JH2 gene variant of SEQ ID NO: 3, wherein the variant comprises a nucleotide mutation at one or more positions corresponding to positions
106,331,455
106,331,453, and
106,331,409
on human chromosome 14.

Optionally the vertebrate, cell or population comprises said plurality of JH2 gene segments, wherein the plurality comprises a human JH2 gene variant of SEQ ID NO: 3, wherein the variant comprises a guanine at a position corresponding to position 106,331,455 on human chromosome 14; and optionally no further mutation from the sequence of SEQ ID NO: 3.

Optionally the variant comprises additionally a mutation at a position corresponding to (i) position 106,331,453 on human chromosome 14 (optionally the additional mutation being an adenine); and/or (ii) position 106,331,409 on human chromosome 14 (optionally the additional mutation being an adenine); (iii) position 106,329,434 on human chromosome 14 (optionally the additional mutation being an adenine).

Optionally the vertebrate, cell or population comprises a plurality of JH2 gene segments, wherein the plurality comprises a human JH2 gene variant of SEQ ID NO: 3, wherein the variant comprises an adenine at a position corresponding to position 106,331,453 on human chromosome 14; and optionally no further mutation from the sequence of SEQ ID NO: 3.

Optionally the variant comprises additionally a mutation at a position corresponding to position 106,331,409 on human chromosome 14 (optionally the additional mutation being an adenine).

Optionally the vertebrate, cell or population comprises a plurality of JH2 gene segments, wherein the plurality comprises a human JH2 gene variant of SEQ ID NO: 3, wherein the variant comprises an adenine at a position corresponding to position 106,331,409 on human chromosome 14; and optionally no further mutation from the sequence of SEQ ID NO: 3.

Optionally the vertebrate, cell or population comprises a plurality of JH2 gene segments, wherein the plurality comprises a human JH2 gene variant that is an antisense sequence of a variant described above.

Optionally the genome comprises SEQ ID NO:3; optionally *cis* at the same Ig locus as one, two or more of the JH2 variants.

Optionally the vertebrate, cell or population genome comprises two or more different JH gene segments selected from SEQ ID NOs: 1 to 3 and variants described above; optionally wherein said JH gene segments are *cis* at the same immunoglobulin Ig locus.

### Multiple Human D Gene Segment Variants

A specific application of this configuration is the provision of multiple human D gene segments as follows (as set out in numbered clauses, starting at clause number 154).
154. A non-human vertebrate (eg, a mouse or rat) or a non-human vertebrate cell (eg, an ES cell or a B-cell) having a genome comprising at least 3 human D gene segments of the same type (eg, D2-2 gene segments), wherein at least two of the human D gene segments are variants that are not identical to each other (eg, D2-2ref and D2-2a).
   In an example of any aspect of the sixth configuration of the disclosure (V, D, J or C), one or more or all of the variants are naturally-occurring human gene segments.
   In an example of any aspect of the sixth configuration of the disclosure (V, D, J or C), one or more of the variants may be a synthetic variant of a human gene segment.
155. A non-human vertebrate (eg, a mouse or rat) or a non-human vertebrate cell (eg, an ES cell or a B-cell) having a genome comprising at least 2 different non-endogenous D gene segments of the same type (eg, D2-2ref and D2-2a) *cis* at the same Ig locus.
156. A non-human vertebrate (eg, a mouse or rat) or a non-human vertebrate cell (eg, an ES cell or a B-cell) having a genome comprising at least 2 different human D gene segments of the same type (eg, D2-2ref and D2-2a) *trans* at the same Ig locus; and optionally a third human D gene segment (eg, (eg, D2-2ref, D2-2a or D2-2b) of the same type, wherein the third D is cis with one of said 2 different D gene segments.
157. A population of non-human vertebrates (eg, mice or rats) comprising a repertoire of human D gene segments, wherein the plurality comprises at least 2 different human D gene segments of the same type (eg, D2-2 gene segments), a first of said different D gene segments (eg, D2-2ref) is provided in the genome of a first vertebrate of the population, and a second of said different D gene segment (eg, D2-2a) being provided in the genome of a second vertebrate of the population, wherein the genome of the first vertebrate does not comprise the second D gene segment.
158. A non-human vertebrate (eg, a mouse or rat) or a non-human vertebrate cell (eg, an ES cell or a B-cell) having a genome comprising at least 2 different non-endogenous D gene segments of the same type (eg, human D2-2 gene segments), wherein the D gene segments are derived from the genome sequence of different human individuals that are not genetically related over at least 3 generations.
159. A method of enhancing the human immunoglobulin gene diversity of a non-human vertebrate (eg, a mouse or rat), the method comprising providing the vertebrate with a genome comprising at least 3 human D gene segments of the same type (eg, D2-2 gene segments), wherein at least two of the human D gene segments are variants that are not identical to each other (eg, D2-2ref and D2-2a).
160. A method of enhancing the immunoglobulin gene diversity of a non-human vertebrate (eg, a mouse or rat), the method comprising providing the vertebrate with a genome comprising at least 2 different non-endogenous D gene segments of the same type (eg, human D2-2 gene segments) *cis* at the same Ig locus.
161. A method of enhancing the immunoglobulin gene diversity of a non-human vertebrate (eg, a mouse or rat), the method comprising providing the vertebrate with a genome comprising at least 2 different human D gene segments of the same type (eg, D2-2ref and D2-2a) *trans* at the same Ig locus; and optionally a third human D gene segment (eg, D2-2ref, D2-2a or D2-2b) of the same type, wherein the third D is *cis* with one of said 2 different D gene segments.
162. A method of providing an enhanced human immunoglobulin D gene segment repertoire, the method comprising providing a population of non-human vertebrates (eg, a mouse or rat) comprising a repertoire of human D gene segments, wherein the method comprises providing at least 2 different human D gene segments of the same type (eg, D2-2ref and D2-2a), wherein a first of said different D gene segments is provided in the genome of a first vertebrate of the population, and a second of said different D gene segments is provided in the genome of a second vertebrate of the population, wherein the genome of the first vertebrate does not comprise the second D gene segment.
163. A method of enhancing the human immunoglobulin gene diversity of a non-human vertebrate (eg, a mouse or rat), the method comprising providing the vertebrate with a genome comprising at least 2 different non-endogenous D gene segments of the same type (eg, D2-2ref and D2-2a), wherein the D gene segments are derived from the genome sequence of different human individuals that are not genetically related over at least 3 generations.
164. The vertebrate or cell of clause 154, 156 or 158, or the method of clause 159, 161 or 163, wherein at least 2 or 3 of said different gene segments are provided *cis* at the same Ig locus in said genome.
165. The vertebrate or cell of clause 154, 155 or 156, or the method of any one of clauses 159 to 162 and 164, wherein the D gene segments are derived from the genome sequence of different human individuals that are not genetically related over at least 3 generations.
166. The vertebrate or cell of any one of clauses 154 to 157, or the method of any one of clauses 159 to 162 and 165, wherein the D gene segments are derived from the genome sequence of two or more different human individuals; optionally wherein the different human individuals are from different human populations.
167. The vertebrate, cell or method of clause 166, wherein the individuals are not genetically related.
168. The vertebrate, cell or method of any one of clauses 154 to 167, wherein at least one of the different D segments is a synthetic mutant of a human germline D gene segment.
169. A method of enhancing the human immunoglobulin gene diversity of a non-human vertebrate (eg, a mouse or rat), the method comprising providing the vertebrate with a genome comprising at least 2 human D gene segments of the same type (eg, D2-2ref and D2-2a), wherein the D gene segments are derived from the genome sequence of different human individuals that are not genetically related over at least 3 generations; optionally wherein at least 2 or 3 of said different gene segments are provided at the same IgH locus in said genome.
170. The vertebrate or cell of any one of clauses 154 to 158 and 164 to 168, wherein the genome comprises a substantially complete functional repertoire of human D gene segment types supplemented with one, two or more variant human D gene segments, wherein said substantially complete functional repertoire and the supplementary D gene segments are not found together in the germline genome of a human individual.
171. The population of clause 157, wherein the population comprises a substantially complete functional repertoire of human D gene segment types supplemented with one, two or more variant human D gene segments, wherein said substantially complete functional repertoire and the supplementary D gene segments are not found together in the germline genome of a human individual.
172. A non-human vertebrate (eg, a mouse or rat) or a non-human cell (eg, an ES cell or a B-cell) having a genome comprising a substantially complete functional repertoire of human D gene segment types supplemented with one, two or more variant human D gene segments, wherein said substantially complete functional repertoire and the supplementary D gene segments are not found together in the germline genome of a human individual.
173. A population of non-human vertebrates (eg, mice or rats) comprising a substantially complete functional repertoire of human JH gene segment types supplemented with one, two or more variant human D gene segments, wherein said substantially complete functional repertoire and the supplementary D gene segments are not found together in the germline genome of a human individual.
174. The vertebrate or cell of clause 172 or the population of clause 173, comprising first and second D gene segments selected from
   D2-2ref and D2-2a; or
   D2-21ref and D2-21a; or
   D3-10refand D3-10a; or
   D3-16refand D3-16a; or
   D2-8ref and D2-8a; or
   D3-3ref and D3-3a; or
   D4-23ref and D4-23a; or
   D6-13refand D6-13a; or
   D3-9ref and D3-9a; or
   D4-4ref and D4-4a; or
   D7-27ref and D7-27a;
   Optionally wherein the first and/or second D gene segment is present in two or more copies.
   For example, there are provided two or three copies of the first gene segment, optionally with one, two or three copies of the second gene segment. Copies can be arranged in *cis* or *trans.*
175. The vertebrate, cell or population of clause 174, comprising human gene segments D2-2ref and D2-2a; and D3-3ref and D3-3a; and optionally also D2-15.
   In an example, the vertebrate, cell or population comprises one or more D segments selected from human D3-3, D2-15, D3-9; D4-17; D3-10; D2-2; D5-24; D6-19; D3-22; D6-13; D5-12; D1-26; D1-20; D5-18; D3-16; D2-21; D1-14; D7-27; D1-1; D6-25; D2-14 and D4-23 (eg, selected from D3- 9*01; D4-17*01; D3-10*01; D2-2*02; D5-24*01; D6-19*01; D3-22*01; D6-13*01; D5-12*01; D1- 26*01; D1-20*01; D5-18*01; D3-16*02; D2-21*02; D1-14*01; D7-27*02; D1-1*01; D6-25*01; D2-15*01; and D4-23*01 together with the reference sequence(s) of said selected segment(s). These were found in variable domains having a HCDR3 length of at least 20 amino acids (see examples herein).
176. A non-human vertebrate or vertebrate cell according to clause 155, comprising a genome that comprises VH, D and JH gene repertoires comprising human gene segments, the D gene repertoire comprising one or more of
   a plurality of D2-2 gene segments provided by at least 2 different D2-2 gene segments in *cis* at the same Ig locus in said genome;
   a plurality of D2-21 gene segments provided by at least 2 different D2-21 gene segments in *cis* at the same Ig locus in said genome;
   a plurality of D3-10 gene segments provided by at least 2 different D3-10 gene segments in *cis* at the same Ig locus in said genome;
   a plurality of D3-16 gene segments provided by at least 2 different D3-16 gene segments in *cis* at the same Ig locus in said genome;
   a plurality of D2-8 gene segments provided by at least 2 different D2-8 gene segments in *cis* at the same Ig locus in said genome;
   a plurality of D3-3 gene segments provided by at least 2 different D3-3 gene segments in *cis* at the same Ig locus in said genome;
   a plurality of D4-23 gene segments provided by at least 2 different D4-23 gene segments in *cis* at the same Ig locus in said genome;
   a plurality of D6-13 gene segments provided by at least 2 different D6-13 gene segments in *cis* at the same Ig locus in said genome;
   a plurality of D3-9 gene segments provided by at least 2 different D3-9 gene segments in *cis* at the same Ig locus in said genome;
   a plurality of D4-4 gene segments provided by at least 2 different D4-4 gene segments in *cis* at the same Ig locus in said genome; and
   a plurality of D7-27 gene segments provided by at least 2 different D7-27 gene segments in *cis* at the same Ig locus in said genome;
   optionally wherein the D gene segments are derived from the genome sequence of two or more different human individuals.
177. A non-human vertebrate or vertebrate cell according to clause 155, comprising a genome that comprises VH, D and JH gene repertoires comprising human gene segments, the D gene repertoire comprising one or more of
   a plurality of D2-2 gene segments provided by at least 2 different D2-2 gene segments in *trans* in said genome;
   a plurality of D2-21 gene segments provided by at least 2 different D2-21 gene segments in *trans* in said genome;
   a plurality of D3-10 gene segments provided by at least 2 different D3-10 gene segments in *trans* in said genome;
   a plurality of D3-16 gene segments provided by at least 2 different D3-16 gene segments in *trans* in said genome;
   a plurality of D2-8 gene segments provided by at least 2 different D2-8 gene segments in *trans* in said genome;
   a plurality of D3-3 gene segments provided by at least 2 different D3-3 gene segments in *trans* in said genome;
   a plurality of D4-23 gene segments provided by at least 2 different D4-23 gene segments in *trans* in said genome;
   a plurality of D6-13 gene segments provided by at least 2 different D6-13 gene segments in *trans* in said genome;
   a plurality of D3-9 gene segments provided by at least 2 different D3-9 gene segments in *trans* in said genome;
   a plurality of D4-4 gene segments provided by at least 2 different D4-4 gene segments in *trans* in said genome; and
   a plurality of D7-27 gene segments provided by at least 2 different D7-27 gene segments in *trans* in said genome;
   optionally wherein the D gene segments are derived from the genome sequence of two or more different human individuals.
178. A non-human vertebrate or vertebrate cell (optionally an ES cell or B-cell), according to clause 154, comprising a genome that comprises VH, D and JH gene repertoires comprising human gene segments, the D gene repertoire comprising one or more of
   a plurality of D2-2 gene segments provided by at least 3 different D2-2 gene segments;
   a plurality of D2-21 gene segments provided by at least 3 different D2-21 gene segments;
   a plurality of D3-10 gene segments provided by at least 3 different D3-10 gene segments;
   a plurality of D3-16 gene segments provided by at least 3 different D3-16 gene segments;
   a plurality of D2-8 gene segments provided by at least 3 different D2-8 gene segments;
   a plurality of D3-3 gene segments provided by at least 3 different D3-3 gene segments;
   a plurality of D4-23 gene segments provided by at least 3 different D4-23 gene segments;
   a plurality of D6-13 gene segments provided by at least 3 different D6-13 gene segments;
   a plurality of D3-9 gene segments provided by at least 3 different D3-9 gene segments;
   a plurality of D4-4 gene segments provided by at least 3 different D4-4 gene segments; and
   a plurality of D7-27 gene segments provided by at least 3 different D7-27 gene segments;
   optionally wherein the D gene segments are derived from the genome sequence of two or three different human individuals;
   optionally wherein at least 2 or 3 of said different gene segments are provided in *cis* at the same
   Ig locus in said genome.
179. The vertebrate or cell of clause 176, 177 or 178, wherein the different human individuals are from different human populations.
180. The vertebrate or cell of any one of clauses 176 to 179, wherein the individuals are not genetically related.
181. The vertebrate or cell of any one of clauses 176 to 180, wherein at least one of the different D segments is a synthetic mutant of a human germline D gene segment.
182. A non-human vertebrate or vertebrate cell (optionally an ES cell or B-cell) according to clause 158, comprising a genome comprising human VH, D and JH gene repertoires, the D gene repertoire comprising of one or more of
   a plurality of D2-2 gene segments provided by at least 2 different D2-2 gene ; optionally in *cis* in said genome;
   a plurality of D2-21 gene segments provided by at least 2 different D2-21 gene ;
   optionally in *cis* in said genome;
   a plurality of D3-10 gene segments provided by at least 2 different D3-10 gene ; optionally in *cis* in said genome;
   a plurality of D3-16 gene segments provided by at least 2 different D3-16 gene ; optionally in *cis* in said genome;
   a plurality of D2-8 gene segments provided by at least 2 different D2-8 gene ; optionally in *cis* in said genome;
   a plurality of D3-3 gene segments provided by at least 2 different D3-3 gene ; optionally in *cis* in said genome;
   a plurality of D4-23 gene segments provided by at least 2 different D4-23 gene ; optionally in *cis* in said genome;
   a plurality of D6-13 gene segments provided by at least 2 different D6-13 gene ; optionally in *cis* in said genome;
   a plurality of D3-9 gene segments provided by at least 2 different D3-9 gene ; optionally in *cis* in said genome;
   a plurality of D4-4 gene segments provided by at least 2 different D4-4 gene ; optionally in *cis* in
   said genome; and
   a plurality of D7-27 gene segments provided by at least 2 different D7-27 gene ; optionally in *cis* in said genome;
   wherein the D gene segments are derived from the genome sequence of different human individuals that are not genetically related over at least 3 generations.
183. The vertebrate or cell of clause 182, wherein the human individuals are from different human populations.
184. The vertebrate, cell or population of any one of clauses 154 to 183, wherein one or more of the D gene segments is a variant of a human germline D gene segment, wherein the variant gene segment encodes an amino acid sequence that differs by 1, 2 or 3 amino acids from the corresponding amino acid sequence encoded by the human germline D gene segment, provided in that said amino acid sequence encoded by the variant does not include a stop codon when said corresponding amino acid sequence does not include a stop codon.
   Optionally, the variant and germline D gene segments encode the respective amino acid sequences in reading frame 2 (IMGT numbering). See Briney et al 2012.
185. The vertebrate, cell or population of clause 184, wherein said corresponding amino acid sequence encoded by the human germline D gene segment is a hydrophilic or hydrophobic sequence (according to J Mol Biol. 1997 Jul 25;270(4):587-97; Corbett SJ et al; Table 2).
186. The vertebrate, cell or population of clause 184 or 185, comprising said variant and said germline human D gene segments; optionally wherein the variant and germline human D gene segments are *cis* on the same chromosome.
187. The vertebrate, cell or population of any one of clauses 184 to 186, wherein germline human D gene segment is a D2, D3, D5 or D6 family gene segment; optionally a D2-2, D2-15, D3-3, D3-9, D3-10, D3-22, D5-5, D5-18, D6-6, D6-13, D6-19 gene segment.
   These D segments are usable in all three reading frames.
   Optionally a variant of 1, 2, 3, 4, 5, 6, 7, 8, 9, 10 or all of these human germline D gene segments is used.
188. The vertebrate, cell or population of any one of clauses 154 to 187, comprising a plurality of D2-2 gene segments, wherein the plurality comprises D2-2 gene segments that vary from each other at one or more nucleotide positions corresponding to positions
   106,382,687 and
   106,382,711
   on human chromosome 14.
189. The vertebrate, cell or population of clause 188, wherein the plurality comprises a human D2-2 gene segment ((optionally two copies and/or in homozygous state) comprising a thymine at a position corresponding to position 106,382,687 on human chromosome 14; and optionally no further mutation from the sequence of D2-2ref.
190. The vertebrate, cell or population of clause 188 or 189, wherein the plurality comprises a human D2-2 gene segment comprising a cytosine at a position corresponding to position 106,382,687 on human chromosome 14; and optionally no further mutation from the sequence of D2-2a.
191. The vertebrate, cell or population of any one of clauses 188 to 190, wherein the plurality comprises a human D2-2 gene segment comprising an adenine at a position corresponding to position 106,382,711 on human chromosome 14; and optionally no further mutation from the sequence of D2-2b.
192. The vertebrate, cell or population of any one of clauses 188 to 191, wherein the plurality comprises a human D2-2 gene segment comprising an thymine at a position corresponding to position 106,382,711 on human chromosome 14; and optionally no further mutation from the sequence of D2-2ref.
193. The vertebrate, cell or population of any one of clauses 154 to 192, comprising a plurality of D7-27 gene segments, wherein the plurality comprises D7-27 gene segments that vary from each other at a nucleotide position corresponding to position 106,331,767 on human chromosome 14.
194. The vertebrate, cell or population of clause 193, wherein the plurality comprises a human D7-27 gene segment (optionally two copies and/or in homozygous state) comprising a cytosine at a position corresponding to position 106,331,767 on human chromosome 14; and optionally no further mutation from the sequence of D7-27ref.
195. The vertebrate, cell or population of clause 193 or 194, wherein the plurality comprises a human D7-27 gene segment comprising a guanine at a position corresponding to position 106,331,767 on human chromosome 14; and optionally no further mutation from the sequence of D7-27a.
196. The vertebrate, cell or population of any one of clauses 154 to 195, comprising a plurality of D4-23 gene segments, wherein the plurality comprises D4-23 gene segments that vary from each other at a nucleotide position corresponding to position 106,350,740 on human chromosome 14.
197. The vertebrate, cell or population of clause 196, wherein the plurality comprises a human D4-23 gene segment (optionally two copies and/or in homozygous state) comprising an adenine at a position corresponding to position 106,350,740 on human chromosome 14; and optionally no further mutation from the sequence of D4-23ref.
198. The vertebrate, cell or population of clause 196 or 197, wherein the plurality comprises a human D4-23 gene segment (optionally two copies and/or in homozygous state) comprising an guanine at a position corresponding to position 106,350,740 on human chromosome 14; and optionally no further mutation from the sequence of D4-23a.
199. The vertebrate, cell or population of any one of clauses 154 to 197, comprising a plurality of D2-21 gene segments, wherein the plurality comprises D2-21 gene segments that vary from each other at a nucleotide position corresponding to position 106,354,418 on human chromosome 14.
200. The vertebrate, cell or population of clause 199, wherein the plurality comprises a human D2-21 gene segment (optionally two copies and/or in homozygous state) comprising an adenine
   at a position corresponding to position 106,354,418 on human chromosome 14; and optionally no further mutation from the sequence of D2-21 ref.
201. The vertebrate, cell or population of clause 199 or 200, wherein the plurality comprises a human D2-21 gene segment (optionally two copies and/or in homozygous state) comprising a guanine at a position corresponding to position 106,354,418 on human chromosome 14; and optionally no further mutation from the sequence of D2-21a.
202. The vertebrate, cell or population of any one of clauses 154 to 201, comprising a plurality of D3-16 gene segments, wherein the plurality comprises D3-16 gene segments that vary from each other at a nucleotide position corresponding to position 106,354,418 on human chromosome 14.
203. The vertebrate, cell or population of clause 202, wherein the plurality comprises a human D3-16 gene segment (optionally two copies and/or in homozygous state) comprising a thymine at a position corresponding to position 106,361,515 on human chromosome 14; and optionally no further mutation from the sequence of D3-16ref.
204. The vertebrate, cell or population of clause 202 or 203, wherein the plurality comprises a human D3-16 gene segment (optionally two copies and/or in homozygous state) comprising a cytosine at a position corresponding to position 106,361,515 on human chromosome 14; and optionally no further mutation from the sequence of D3-16a.
205. The vertebrate, cell or population of any one of clauses 154 to 204, comprising a plurality of D6-13 gene segments, wherein the plurality comprises D6-13 gene segments that vary from each other at a nucleotide position corresponding to position 106,367,013 on human chromosome 14.
206. The vertebrate, cell or population of clause 205, wherein the plurality comprises a human D6-13 gene segment (optionally two copies and/or in homozygous state) comprising a thymine at a position corresponding to position 106,367,013 on human chromosome 14; and optionally no further mutation from the sequence of D6-13ref.
207. The vertebrate, cell or population of clause 205 or 206, wherein the plurality comprises a human D6-13 gene segment (optionally two copies and/or in homozygous state) comprising a cytosine at a position corresponding to position 106,367,013 on human chromosome 14; and optionally no further mutation from the sequence of D6-13a.
208. The vertebrate, cell or population of any one of clauses 154 to 207, comprising a plurality of D3-10 gene segments, wherein the plurality comprises D3-10 gene segments that vary from each other at one or more nucleotide positions corresponding to positions
   106.370.370 and
   106.370.371
   on human chromosome 14.
209. The vertebrate, cell or population of clause 208, wherein the plurality comprises a human D3-10 gene segment (optionally two copies and/or in homozygous state) comprising a thymine at a position corresponding to position 106,370,370 on human chromosome 14; and optionally no further mutation from the sequence of D3-10ref.
210. The vertebrate, cell or population of clause 208 or 209, wherein the plurality comprises a human D3-10 gene segment (optionally two copies and/or in homozygous state) comprising a cytosine at a position corresponding to position 106,370,370 on human chromosome 14; and optionally no further mutation from the sequence of D3-10a.
211. The vertebrate, cell or population of clause 208, 209 or 210 wherein the plurality comprises a human D3-10 gene segment (optionally two copies and/or in homozygous state) comprising an adenine at a position corresponding to position 106,370,371 on human chromosome 14; and optionally no further mutation from the sequence of D3-10ref.
212. The vertebrate, cell or population of any one of clauses 208 to 211, wherein the plurality comprises a human D3-10 gene segment (optionally two copies and/or in homozygous state) comprising a guanine at a position corresponding to position 106,370,371 on human chromosome 14; and optionally no further mutation from the sequence of D3-10b.
213. The vertebrate, cell or population of any one of clauses 154 to 212, comprising a plurality of D3-9 gene segments, wherein the plurality comprises D3-9 gene segments that vary from each other at a nucleotide position corresponding to position 106,370,567 on human chromosome 14.
214. The vertebrate, cell or population of clause 213, wherein the plurality comprises a human D3-9 gene segment (optionally two copies and/or in homozygous state) comprising an adenine at a position corresponding to position 106,370,567 on human chromosome 14; and optionally no further mutation from the sequence of D3-9ref.
215. The vertebrate, cell or population of clause 213 or 214, wherein the plurality comprises a human D3-9 gene segment (optionally two copies and/or in homozygous state) comprising a thymine at a position corresponding to position 106,370,567 on human chromosome 14; and optionally no further mutation from the sequence of D3-9a.
216. The vertebrate, cell or population of any one of clauses 154 to 215, comprising a plurality of D2-8 gene segments, wherein the plurality comprises D2-8 gene segments that vary from each other at one or more nucleotide positions corresponding to positions
   106,373,085;
   106,373,086 and
   106,373,089
   on human chromosome 14.
217. The vertebrate, cell or population of clause 216, wherein the plurality comprises a human D2-8 gene segment (optionally two copies and/or in homozygous state) comprising a cytosine at a position corresponding to position 106,373,085 on human chromosome 14.
218. The vertebrate, cell or population of clause 216 or 217, wherein the plurality comprises a human D2-8 gene segment (optionally two copies and/or in homozygous state) comprising a thymine at a position corresponding to position 106,373,085 on human chromosome 14; and optionally no further mutation from the sequence of D2-8b.
219. The vertebrate, cell or population of clause 216, 217 or 218 wherein the plurality comprises a human D2-8 gene segment (optionally two copies and/or in homozygous state) comprising a cytosine at a position corresponding to position 106,373,086 on human chromosome 14; and
   optionally no further mutation from the sequence of D2-8ref.
220. The vertebrate, cell or population of any one of clauses 216 to 219, wherein the plurality comprises a human D2-8 gene segment comprising a thymine at a position corresponding to position 106,373,086 on human chromosome 14; and optionally no further mutation from the sequence of D2-8ref.
221. The vertebrate, cell or population of any one of clauses 154 to 220, comprising a plurality of D4-4 gene segments, wherein the plurality comprises D4-4 gene segments that vary from each other at one or more nucleotide positions corresponding to positions
   106,379,086; and
   106,379,089
   on human chromosome 14.
222. The vertebrate, cell or population of clause 221, wherein the plurality comprises a D4-4 gene segment (optionally two copies and/or in homozygous state) comprising a cytosine at a position corresponding to position 106,379,086 on human chromosome 14; and optionally no further mutation from the sequence of D4-4ref.
223. The vertebrate, cell or population of clause 221 or 222, wherein the plurality comprises a human D4-4 gene segment (optionally two copies and/or in homozygous state) comprising a thymine at a position corresponding to position 106,379,086 on human chromosome 14; and optionally no further mutation from the sequence of D4-4a.
224. The vertebrate, cell or population of clause 221, 222 or 223 wherein the plurality comprises a human D4-4 gene segment (optionally two copies and/or in homozygous state) comprising a cytosine at a position corresponding to position 106,379,089 on human chromosome 14; and optionally no further mutation from the sequence of D4-4ref or a cytosine at a position corresponding to position 106,379,086 on human chromosome 14.
225. The vertebrate, cell or population of any one of clauses 221 to 224, wherein the plurality comprises a human D4-4 gene segment (optionally two copies and/or in homozygous state) comprising a thymine at a position corresponding to position 106,373,089 on human chromosome 14; and optionally no further mutation from the sequence of D4-4a.
226. The vertebrate, cell or population of any one of clauses 154 to 225, comprising a plurality of D3-3 gene segments, wherein the plurality comprises D3-3 gene segments that vary from each other at one or more nucleotide positions corresponding to positions
   106,380,241; and
   106,380,246
   on human chromosome 14.
227. The vertebrate, cell or population of clause 226, wherein the plurality comprises a D3-3 gene segment (optionally two copies and/or in homozygous state) comprising a thymine at a position corresponding to position 106,380,241 on human chromosome 14; and optionally no further mutation from the sequence of D3-3ref.
228. The vertebrate, cell or population of clause 226 or 227, wherein the plurality comprises a human D3-3 gene segment (optionally two copies and/or in homozygous state) comprising a cytosine at a position corresponding to position 106,380,241 on human chromosome 14; and optionally no further mutation from the sequence of D3-3a.
229. The vertebrate, cell or population of clause 226, 227 or 228 wherein the plurality comprises a human D3-3 gene segment (optionally two copies and/or in homozygous state) comprising an adenine at a position corresponding to position 106,380,246 on human chromosome 14; and optionally no further mutation from the sequence of D3-3ref.
230. The vertebrate, cell or population of any one of clauses 226 to 229, wherein the plurality comprises a human D3-3 gene segment (optionally two copies and/or in homozygous state) comprising a thymine at a position corresponding to position 106,380,246 on human chromosome 14; and optionally no further mutation from the sequence of D3-3a.

### Multiple Human JL Gene Segment Variants

A specific application of this configuration is the provision of multiple human JL gene segments (Jκ and/or Jλ) as follows (as set out in numbered paragraphs, starting at paragraph number 80).
80. A non-human vertebrate (eg, a mouse or rat) or a non-human vertebrate cell (eg, an ES cell or a B-cell) having a genome comprising at least 3 human JL gene segments of the same type (eg, Jκ1), wherein at least two of the human JL gene segments are variants that are not identical to each other.
81. A non-human vertebrate (eg, a mouse or rat) or a non-human vertebrate cell (eg, an ES cell or a B-cell) having a genome comprising at least 2 different non-endogenous JL gene segments of the same type (eg, Jκ1) *cis* at the same Ig locus.
82. A non-human vertebrate (eg, a mouse or rat) or a non-human vertebrate cell (eg, an ES cell or a B-cell) having a genome comprising at least 2 different human JL gene segments of the same type (eg, JK1) *trans* at the same Ig locus; and optionally a third human JLgene segment of the same type, wherein the third JL is *cis* with one of said 2 different JL gene segments.
83. A population of non-human vertebrates (eg, mice or rats) comprising a repertoire of human JL gene segments, wherein the plurality comprises at least 2 different human JL gene segments of the same type (eg, Jκ1), a first of said different JL gene segments is provided in the genome of a first vertebrate of the population, and a second of said different JL gene segments being provided in the genome of a second vertebrate of the population, wherein the genome of the first vertebrate does not comprise the second JL gene segment.
84. A non-human vertebrate (eg, a mouse or rat) or a non-human vertebrate cell (eg, an ES cell or a B-cell) having a genome comprising at least 2 different non-endogenous JL gene segments of the same type (eg, Jκ1 wherein the JL gene segments are derived from the genome sequence of different human individuals that are not genetically related over at least 3 generations.
85. A method of enhancing the human immunoglobulin gene diversity of a non-human vertebrate (eg, a mouse or rat), the method comprising providing the vertebrate with a genome comprising at least 3 human JL gene segments of the same type (eg, Jκ1), wherein at least two of the human JL gene segments are variants that are not identical to each other.
86. A method of enhancing the immunoglobulin gene diversity of a non-human vertebrate (eg, a mouse or rat), the method comprising providing the vertebrate with a genome comprising at least 2 different non-endogenous JL gene segments of the same type (eg, Jκ1 ) *cis* at the same Ig locus.
87. A method of enhancing the immunoglobulin gene diversity of a non-human vertebrate (eg, a mouse or rat), the method comprising providing the vertebrate with a genome comprising at least 2 different human JL gene segments of the same type (eg, Jκ1 ) *trans* at the same Ig locus; and optionally a third human JL gene segment of the same type, wherein the third JL is *cis* with one of said 2 different JL gene segments.
88. A method of providing an enhanced human immunoglobulin JL gene segment repertoire, the method comprising providing a population of non-human vertebrates (eg, a mouse or rat) comprising a repertoire of human JL gene segments, wherein the method comprises providing at least 2 different human JL gene segments of the same type (eg, Jκ1), wherein a first of said different JL gene segments is provided in the genome of a first vertebrate of the population, and a second of said different JL gene segments is provided in the genome of a second vertebrate of the population, wherein the genome of the first vertebrate does not comprise the second JL gene segment.
89. A method of enhancing the human immunoglobulin gene diversity of a non-human vertebrate (eg, a mouse or rat), the method comprising providing the vertebrate with a genome comprising at least 2 different non-endogenous JL gene segments of the same type (eg, Jκ1 wherein the JL gene segments are derived from the genome sequence of different human individuals that are not genetically related over at least 3 generations.
90. The vertebrate or cell of paragraph 80, 82 or 84, or the method of paragraph 85, 82 or 89, wherein at least 2 or 3 of said different gene segments are provided *cis* at the same Ig locus in said genome.
91. The vertebrate or cell of paragraph 80, 81 or 82, or the method of paragraph 85, 86 or 87, wherein the JL gene segments are derived from the genome sequence of different human individuals that are not genetically related over at least 3 generations.
92. The vertebrate or cell of paragraph 80, 81 or 82, or the method of paragraph 85, 86 or 87, wherein the JL gene segments are derived from the genome sequence of two or more different human individuals; optionally wherein the different human individuals are from different human populations.
93. The vertebrate, cell or method of paragraph 92, wherein the individuals are not genetically related.
94. The vertebrate, cell or method of any one of paragraphs 80 to 93, wherein at least one of the different JL segments is a synthetic mutant of a human germline JL gene segment.
95. A method of enhancing the human immunoglobulin gene diversity of a non-human vertebrate (eg, a mouse or rat), the method comprising providing the vertebrate with a genome comprising at least 2 human JL gene segments of the same type (eg, Jκ1), wherein the JL gene segments are derived from the genome sequence of different human individuals that are not genetically related over at least 3 generations; optionally wherein at least 2 or 3 of said different gene segments are provided at the same IgL locus in said genome.
96. The vertebrate or cell of any one of paragraphs paragraph 80 to 82 and 84, wherein the genome comprises a substantially complete functional repertoire of human Jκ and/or Jλ gene segment types supplemented with one, two or more human Jκ and/or Jλ gene segments respectively, wherein said substantially complete functional repertoire and the supplementary gene segments are not found together in the germline genome of a human individual.
97. The population of paragraph 83, wherein the population comprises a substantially complete functional repertoire of human JL gene segment types supplemented with one, two or more human Jκ and/or Jλ gene segments respectively, wherein said substantially complete functional repertoire and the supplementary gene segments are not found together in the germline genome of a human individual.
98. A non-human vertebrate (eg, a mouse or rat) or a non-human cell (eg, an ES cell or a B-cell) having a genome comprising a substantially complete functional repertoire of human Jκ and/or Jλ gene segment types supplemented with one, two or more human Jκ and/or Jλ gene segments respectively, wherein said substantially complete functional repertoire and the supplementary gene segments are not found together in the germline genome of a human individual.
99. A population of non-human vertebrates (eg, mice or rats) comprising a substantially complete functional repertoire of human Jκ and/or Jλ gene segment types supplemented with one, two or more human Jκ and/or Jλ gene segments respectively, wherein said substantially complete functional repertoire and the supplementary gene segments are not found together in the germline genome of a human individual.
100. A non-human vertebrate or vertebrate cell according to paragraph 81, comprising a genome that comprises VL and JL gene repertoires comprising human gene segments, the JL gene repertoire comprising
   a plurality of human Jκ1 gene segments provided by at least 2 different human Jκ1 gene segments in *cis* at the same Ig locus in said genome;
   a plurality of human Jκ2 gene segments provided by at least 2 different human Jκ1 gene segments in *cis* at the same Ig locus in said genome;
   a plurality of human Jκ3 gene segments provided by at least 2 different human Jκ1 gene segments in *cis* at the same Ig locus in said genome;
   a plurality of human Jκ4 gene segments provided by at least 2 different human Jκ1 gene segments in *cis* at the same Ig locus in said genome;
   a plurality of human Jκ5 gene segments provided by at least 2 different human Jκ1 gene segments in *cis* at the same Ig locus in said genome;
   a plurality of human Jλ1 gene segments provided by at least 2 different human Jλ1 gene segments in *cis* at the same Ig locus in said genome;
   a plurality of human Jλ2 gene segments provided by at least 2 different human Jλ2 gene segments in *cis* at the same Ig locus in said genome;
   a plurality of human Jλ3 gene segments provided by at least 2 different human Jλ3 gene segments in *cis* at the same Ig locus in said genome;
   a plurality of human Jλ4 gene segments provided by at least 2 different human Jλ4 gene segments in *cis* at the same Ig locus in said genome;
   a plurality of human Jλ5 gene segments provided by at least 2 different human Jλ5 gene segments in *cis* at the same Ig locus in said genome;
   a plurality of human Jλ6 gene segments provided by at least 2 different human Jλ6 gene segments in *cis* at the same Ig locus in said genome; or
   a plurality of human Jλ7 gene segments provided by at least 2 different human Jλ7 gene segments in *cis* at the same Ig locus in said genome;
   optionally wherein the JL gene segments are derived from the genome sequence of two or more different human individuals.
101. A non-human vertebrate or vertebrate cell (optionally an ES cell or B-cell), according to paragraph 80, comprising a genome that comprises VL and JL gene repertoires comprising human gene segments, the JL gene repertoire comprising
   a plurality of human Jκ1 gene segments provided by at least 3 (eg, 3, 4, 5, 6, or 7) different human Jκ1 gene segments;
   a plurality of human Jκ2 gene segments provided by at least 3 (eg, 3, 4, 5, 6, or 7) different human Jκ2 gene segments;
   a plurality of human Jκ3 gene segments provided by at least 3 (eg, 3, 4, 5, 6, or 7) different human Jκ1 gene segments;
   a plurality of human Jκ4 gene segments provided by at least 3 (eg, 3, 4, 5, 6, or 7) different human Jκ1 gene segments;
   a plurality of human Jκ5 gene segments provided by at least 3 (eg, 3, 4, 5, 6, or 7) different human Jκ1 gene segments;
   a plurality of human Jκ1 gene segments provided by at least 3 (eg, 3, 4, 5, 6, or 7) different human Jλ1 gene segments;
   a plurality of human Jλ2 gene segments provided by at least 3 (eg, 3, 4, 5, 6, or 7) different human Jλ2 gene segments;
   a plurality of human Jλ3 gene segments provided by at least 3 (eg, 3, 4, 5, 6, or 7) different human Jλ3 gene segments;
   a plurality of human Jλ4 gene segments provided by at least 3 (eg, 3, 4, 5, 6, or 7) different human Jλ4 gene segments;
   a plurality of human Jλ5 gene segments provided by at least 3 (eg, 3, 4, 5, 6, or 7) different human Jλ5 gene segments;
   a plurality of human Jλ6 gene segments provided by at least 3 (eg, 3, 4, 5, 6, or 7) different human Jλ6 gene segments; or
   a plurality of human Jλ7 gene segments provided by at least 3 (eg, 3, 4, 5, 6, or 7) different human Jλ7 gene segments;
   optionally wherein the JL gene segments are derived from the genome sequence of two or three different human individuals;
   optionally wherein at least 2 or 3 of said different gene segments are provided in cis at the same Ig locus in said genome.
102. The vertebrate or cell of paragraph 104 or 105, wherein the different human individuals are from different human populations.
103. The vertebrate or cell of any one of paragraphs 104 to 106, wherein the individuals are not genetically related.
104. The vertebrate or cell of any one of paragraphs 104 to 107, wherein at least one of the different JL segments is a synthetic mutant of a human germline JL gene segment.
105. A non-human vertebrate or vertebrate cell (optionally an ES cell or B-cell) according to paragraph 84, comprising a genome comprising human VL and JL gene repertoires, the JL gene repertoire comprising
   a plurality of human Jκ1 gene segments provided by at least 2 different human Jκ1 gene segments, optionally in *cis* at the same Ig locus in said genome;
   a plurality of human Jκ2 gene segments provided by at least 2 different human Jκ1 gene segments, optionally in *cis* at the same Ig locus in said genome;
   a plurality of human Jκ3 gene segments provided by at least 2 different human Jκ1 gene segments, optionally in *cis* at the same Ig locus in said genome;
   a plurality of human Jκ4 gene segments provided by at least 2 different human Jκ1 gene segments, optionally in *cis* at the same Ig locus in said genome;
   a plurality of human Jκ5 gene segments provided by at least 2 different human Jκ1 gene segments, optionally in *cis* at the same Ig locus in said genome;
   a plurality of human Jλ1 gene segments provided by at least 2 different human Jλ1 gene segments, optionally in *cis* at the same Ig locus in said genome;
   a plurality of human Jλ2 gene segments provided by at least 2 different human Jλ2 gene segments, optionally in *cis* at the same Ig locus in said genome;
   a plurality of human Jλ3 gene segments provided by at least 2 different human Jλ3 gene segments, optionally in *cis* at the same Ig locus in said genome;
   a plurality of human Jλ4 gene segments provided by at least 2 different human Jλ4 gene segments, optionally in *cis* at the same Ig locus in said genome;
   a plurality of human Jλ5 gene segments provided by at least 2 different human Jλ5 gene segments, optionally in *cis* at the same Ig locus in said genome;
   a plurality of human Jλ6 gene segments provided by at least 2 different human Jλ6 gene segments, optionally in *cis* at the same Ig locus in said genome; or a plurality of human Jλ7 gene segments provided by at least 2 different human Jλ7 gene segments, optionally in *cis* at the same Ig locus in said genome;
   wherein the JL gene segments are derived from the genome sequence of different human individuals that are not genetically related over at least 3 generations.
106. The vertebrate or cell of paragraph 109, wherein the human individuals are from different human populations.

The skilled person will realise that standard molecular biology techniques can be used to provide vectors comprising synthetic combinations of immunoglobulin gene segments (eg, V, D and/or J) for use in the invention, such that the vectors can be used to build a transgenic immunoglobulin locus (eg, using homologous recombination and/or recombinase mediated cassette exchange as known in the art, eg, see US7501552 (Medarex), US5939598 (Abgenix), US6130364 (Abgenix), WO02/066630 (Regeneron), WO2011004192 (Genome Research Limited), WO2009076464, WO2009143472 and WO2010039900 (Ablexis. For example, such synthetic combinations of gene segments can be made using standard recombineering techniques in E coli to construct BAC vectors harbouring the synthetic combination prior to insertion in embryonic stem cells using homologous recombination or RMCE (eg, using cre/lox site-specific recombination). Details of recombineering can be found at www.genebridges.com and in EP1034260 and EP1204740.

In one embodiment, it is useful to bias the immune response of the vertebrate (and thus resultant lead antibodies) to a predetermined gene segment, eg, one known to be commonly used in natural human immune responses to antigens, such as antigens of infectious disease pathogens. For example, VH1-69 is commonly used to produce antibodies in humans against Influenza virus; it is possible, therefore, to include two or more polymorphic DNA versions of the VH segment VH1-69 in the locus of the disclosure. The examples below illustrate how such a transgenic locus can be constructed in which diversity is extended by extending the VH1-69 gene segment repertoire based on naturally-occurring VH1-69 polymorphic variants.

In one embodiment in any configuration of the invention, the genome has been modified to prevent or reduce the expression of fully-endogenous antibody. Examples of suitable techniques for doing this can be found in PCT/GB2010/051122, US7501552, US6673986, US6130364, WO2009/076464, EP1399559 and US6586251. In one embodiment, the non-human vertebrate VDJ region of the endogenous heavy chain immunoglobulin locus, and optionally VJ region of the endogenous light chain immunoglobulin loci (lambda and/or kappa loci), have been inactivated. For example, all or part of the non-human vertebrate VDJ region is inactivated by inversion in the endogenous heavy chain immunoglobulin locus of the mammal, optionally with the inverted region being moved upstream or downstream of the endogenous Ig locus (see, eg, WO2011004192). For example, all or part of the non-human vertebrate VJ region is inactivated by inversion in the endogenous kappa chain immunoglobulin locus of the mammal, optionally with the inverted region being moved upstream or downstream of the endogenous Ig locus. For example, all or part of the non-human vertebrate VJ region is inactivated by inversion in the endogenous lambda chain immunoglobulin locus of the mammal, optionally with the inverted region being moved upstream or downstream of the endogenous Ig locus. In one embodiment the endogenous heavy chain locus is inactivated in this way as is one or both of the endogenous kappa and lambda loci.

Additionally or alternatively, the vertebrate has been generated in a genetic background which prevents the production of mature host B and T lymphocytes, optionally a RAG-1-deficient and/or RAG-2 deficient background. See US5859301 for techniques of generating RAG-1 deficient animals.

Thus, in one embodiment of any configuration or aspect of the invention, endogenous heavy and light chain expression has been inactivated.

In one embodiment each said locus constant region is a heavy chain endogenous non-human vertebrate (optionally host mouse or rat) constant region.

In one embodiment each said locus constant region is a light chain endogenous non-human vertebrate (optionally host mouse or rat) constant region.

The disclosure provides a monoclonal or polyclonal antibody composition prepared by immunisation of at least one vertebrate (eg, mouse or rat) according to the disclosure, optionally wherein the antigen is an antigen of an infectious disease pathogen (eg, a bacterial or viral pathogen antigen), optionally wherein the same antigen is used to immunise all the vertebrates; optionally wherein the antibody or antibodies are IgG-type (eg, IgG1).

The disclosure also provides a monoclonal or polyclonal antibody mixture produced by the method of the invention or a derivative antibody or mixture thereof, eg, where one or more constant region has been changed (eg, replaced with a different constant region such as a human constant region; or mutated to enhance or ablate Fc effector function). In an aspect of the disclosure, the monoclonal or polyclonal antibody mixture is provided for therapy and/or prophylaxis of a disease or condition in a human, eg, for the treatment and/or prevention of an infectious disease, wherein optionally wherein each antibody binds an antigen of an infectious disease pathogen, preferably the same antigen.

In an aspect of the disclosure, there is provided the use of an isolated, monoclonal or polyclonal antibody according to the disclosure, or a mutant or derivative antibody thereof in the manufacture of a medicament for the treatment and/or prevention of a disease or condition in a human, eg, an infectious disease, optionally wherein the infectious disease is a disease caused by a bacterial or viral pathogen.

An example of a mutant antibody is one that bears up to 15 or 10 amino acid mutations in its variable regions relative to an isolated antibody (eg, IgG-type, such as IgG1-type, antibody) obtainable or obtained by the method of the invention. An example of a derivative is one that has been modified to replace a constant region with a different constant region such as a human constant region; or mutated to enhance or ablate Fc effector function.

Examples of infectious diseases are diseases caused or mediated by a bacterial or viral pathogen. For example, the infectious disease is selected from the group consisting of a disease caused by a pathogen selected from the group consisting of Haemophilus influenza, E coli, Neisseria meningitidis, a herpes family virus, cytomegalovirus (CMV), HIV and influenza virus.

### Tailoring V(D)J Incorporation Into Immunoglobin Loci For The Generation of Antibodies Against Infectious Disease

The inventors realised that it would be desirable to provide for vertebrates, cells, methods etc for the production of therapeutic and/or prophylactic antibodies based on natural human immune responses to antigens, such as antigens of infectious disease pathogens. In this respect, the literature observes frequently used immunoglobulin gene segments to raise anti-infective responses in humans (Table 9).

In the various configurations, aspects, embodiments and examples above, the disclosure provides the skilled addressee with the possibility of choosing immunoglobulin gene segments in a way that tailors or biases the repertoire for application to generating antibodies to treat and/or prevent infectious diseases. The inventors have categorised the following groups of gene segments for use in the disclosure according to the desired application of resultant antibodies.

### List A:

### Immunoglobulin gene segments for antibodies that bind an antigen expressed by a Pathogen

(a) a VL gene segment selected from the group consisting of a V_{λ}II gene family member, V_{X}VII 4A, V_{λ}II 2.1, V_{λ}VII 4A, a Vλ1 gene family member, a Vλ3gene family member, IGLV1S2, Vλ3-cML70, lalh2, lalvl, la3h3, Kv325, a V_{κ}I gene family member, κI-15A (KL012), V_{κ}II family member, a V_{κ}III family member, a V_{κ}I gene family member, κI-15A (KL012), V_{κ}II A2 (optionally the A2a variant), V_{κ} A27 (Humkv325) and a gene segment at least 80% identical thereto.
(b) a V_{λ} gene segment selected from a V_{λ}II gene family member, V_{λ}VII 4A, V_{λ}II 2.1, V_{λ}VII 4A, a V_{λ}1 gene family member, a V_{λ}3gene family member, IGLV1S2, V_{λ}3-cML70, lalh2, lalvl, la3h3 and a gene segment at least 80% identical thereto.
(c) a V_{κ} gene segment selected from Kv325, a V_{κ}I gene family member, κI-15A (KL012), V_{κ}II family member, a V_{κ}III family member, a V_{κ}I gene family member, κI-15A (KL012), V_{κ}II A2 (optionally the A2a variant), V_{κ} A27 (Humkv325) and a gene segment at least 80% identical thereto.
(d) a V_{H} gene segment a V_{H}III gene family member (optionally, a V_{H}IIIa or V_{H}lllb family member), a V_{H}IV gene family member, V_{H}III 9.1 (VH3-15), V_{H}III VH26 (VH3-23), V_{H}3-21, LSG6.1, LSG12.1, DP77 (V3-21), V_{H} H11, V_{H}1GRR, ha3h2, V_{H}I-ha1c1, V_{H}III-VH2-1, VH4.18, ha4h3, Hv1051, 71-2, Hv1f10, VH4.11, 71-4, VH251, VH1-69 and a gene segment at least 80% identical thereto.
(e) a J_{λ} gene segment selected from J_{λ}2, J_{λ}3 and a gene segment at least 80% identical thereto.
(f) a D gene segment selected from Dk1, Dxp'1, Dn4r, D2r and a gene segment at least 80% identical thereto.

### List A1:

### Immunoglobulin gene segments for antibodies that bind an antigen expressed by a Pathogen

(a) a V_{λ} gene segment selected from a V_{A}II gene family member, V_{λ} VII 4A, V_{λ} II 2.1, V_{λ} VII 4A and a gene segment at least 80% identical thereto.
(b) a V_{κ} gene segment selected from a V_{κ}I gene family member, κI-15A (KL012), V_{κ}II family member, a V_{κ}III family member, a V_{κ}I gene family member, κI-15A (KL012), V_{κ}II A2 (optionally the A2a variant), V_{κ} A27 (Humkv325) and a gene segment at least 80% identical thereto.
(c) a V_{H} gene segment a V_{H}3 gene family member (optionally, a V_{H}llla or V_{H}lllb family member), V_{H}III 9.1 (VH3-15), V_{H}III VH26 (VH3-23), V_{H}3-21, LSG6.1, LSG12.1, DP77 (V3-21), V_{H} H11 and a gene segment at least 80% identical thereto.
(d) a J_{λ} gene segment selected from J_{λ}2, J_{λ}3 and a gene segment at least 80% identical thereto.
(e) a J_{H} gene segment selected from J_{H}2, J_{H}3, J_{H}4 and a gene segment at least 80% identical thereto.

### List A1.1:

### Immunoglobulin gene segments for antibodies that bind an antigen expressed by H influenza

(a) a V_{λ} gene segment selected from a V_{λ}II gene family member, V_{λ}VII 4A, V_{λ}II 2.1, V_{λ}VII 4A and a gene segment at least 80% identical thereto.
(b) a V_{κ} gene segment selected from a V_{κ}II family member, a V_{κ}III family member, a V_{κ}I gene family member, κI-15A (KL012), V_{κ}II A2 (optionally the A2a variant), V_{κ} A27 (Humkv325) and a gene segment at least 80% identical thereto.
(c) a V_{H} gene segment a V_{H}3 gene family member (optionally, a V_{H}lllb family member), V_{H}III 9.1 (VH3-15), V_{H}III VH26 (VH3-23), V_{H}3-21, LSG6.1, LSG 12.1, DP77 (V3-21) and a gene segment at least 80% identical thereto.
(d) a J_{λ} gene segment selected from J_{λ}2, J_{λ}3 and a gene segment at least 80% identical thereto.

### List A1.2:

### Immunoglobulin gene segments for antibodies that bind an antigen expressed by E Coli or Neisseria meningitidis

(a) a V_{H} gene segment a V_{H}3 gene family member (optionally a V_{H}IIIa or V_{H}lllb member), V_{H}III 9.1 (VH3-15), V_{H} H11, V_{H}III VH26 (VH3-23) a gene segment at least 80% identical thereto, eg, V_{H}III 9.1 + J_{H}3; oᵣ VH H11 + JH4; or V_{H}III VH26 + J_{H}2.
(b) a V_{κ} gene segment selected from a V_{κ}I gene family member, κI-15A (KL012) and a gene segment at least 80% identical thereto.
(c) a V_{λ} gene segment selected from a V_{λ}II gene family member, V_{λ}II 2.1 and a gene segment at least 80% identical thereto.
(d) a J_{H} gene segment selected from J_{H}2, J_{H}3, J_{H}4 and a gene segment at least 80% identical thereto.

### A2:

### Immunoglobulin gene segments for antibodies that bind an antigen expressed by a viral Pathogen

(a) a V_{H} gene segment selected from a V_{H}III gene family member, a V_{H}IV gene family member, V_{H}III- VH26 (VH3-23), V_{H}1GRR, ha3h2, V_{H}I-ha1c1, V_{H}III-VH2-1, VH4.18, ha4h3, Hv1051, 71-2, Hv1f10, VH4.11, 71-4, VH251, VH1-69 and a gene segment at least 80% identical thereto.
(b) a V_{λ} gene segment selected from a V_{λ}1 gene family member, a V_{λ}3gene family member, IGLV1S2, V_{λ}3-cML70, lalh2, lalvl, la3h3 and a gene segment at least 80% identical thereto.
(c) a V_{κ} gene segment selected from Kv325 and a gene segment at least 80% identical thereto.
(d) a J_{H} gene segment selected from J_{H}3, J_{H}5, J_{H}6 and a gene segment at least 80% identical thereto.
(e) a D gene segment selected from Dk1, Dxp'1, Dn4r, D2r and a gene segment at least 80% identical thereto.
(f) a J_{λ} gene segment selected from J_{λ}2, J_{λ}3 and a gene segment at least 80% identical thereto.

### A2.1:

### Immunoglobulin gene segments for antibodies that bind an antigen expressed by Herpes Virus Family (eg, VZV or HSV)

(a) a V_{H} gene segment selected from a V_{H}III gene family member, a V_{H}IV gene family member, V_{H}III- VH26 (VH3-23), VH1GRR, ha3h2, VHI-ha1c1, V_{H}III-VH2-1, VH4.18, ha4h3, and a gene segment at least 80% identical thereto.
(b) a V_{λ} gene segment selected from a V_{λ}1 gene family member, a V_{λ}3gene family member, IGLV1S2, V_{λ}3-cML70, lalh2, lalvl, la3h3 and a gene segment at least 80% identical thereto.
(c) a J_{H} gene segment selected from J_{H}3, J_{H}5, J_{H}6 and a gene segment at least 80% identical thereto.
(d) a D gene segment selected from Dk1, Dxp'1, Dn4r, D2r and a gene segment at least 80% identical thereto.
(e) a J_{λ} gene segment selected from J_{λ}2, J_{λ}3 and a gene segment at least 80% identical thereto.

### A2.2:

### Immunoglobulin gene segments for antibodies that bind an antigen expressed by CMV

(a) a V_{H} gene segment selected from Hv1051 and a gene segment at least 80% identical thereto.
(b) a V_{κ} gene segment selected from Kv325 and a gene segment at least 80% identical thereto. A2.3:

### Immunoglobulin gene segments for antibodies that bind an antigen expressed by HIV

(a) a V_{H} gene segment selected from 71-2, Hv1f10, VH4.11, 71-4, VH251, VH1-69 and a gene segment at least 80% identical thereto.

### A2.4:

### Immunoglobulin gene segments for antibodies that bind an antigen expressed by Influenza Virus

(a) a V_{H} gene segment selected from VH1-69 and a gene segment at least 80% identical thereto.

Thus,
Where one wishes to generate an antibody or antibody mixture to treat and/or prevent an infectious disease, one or more V, D and/or or all J gene segments used in any configuration, aspect, method, example or embodiment of the disclosure described herein can be selected from List A1. Thus, for example in (a) of the first configuration of the disclosure, the recited heavy chain V gene segment is selected from the VH gene segments in List A, optionally with a D in that list.

Where one wishes to generate an antibody or antibody mixture to treat and/or prevent an infectious disease caused or mediated by a bacterial pathogen, one or more or all V, D and/or J gene segments used in any configuration, aspect, method, example or embodiment of the disclosure can be selected from List A1.

Where one wishes to generate an antibody or antibody mixture to treat and/or prevent an infectious disease caused or mediated by a viral pathogen, one or more or all V, D and/or J gene segments used in any configuration, aspect, method, example or embodiment of the disclosure can be selected from List A2.

Where one wishes to generate an antibody or antibody mixture to treat and/or prevent an infectious disease caused or mediated by *H influenza,* one or more or all V, D and/or J gene segments used in any configuration, aspect, method, example or embodiment of the disclosure can be selected from List A1.1.

Where one wishes to generate an antibody or antibody mixture to treat and/or prevent an infectious disease caused or mediated by *E Coli* or *Neisseria meningitidis,* one or more or all V, D and/or J gene segments used in any configuration, aspect, method, example or embodiment of the disclosure can be selected from List A1.2.

Where one wishes to generate an antibody or antibody mixture to treat and/or prevent an infectious disease caused or mediated by Herpes Virus Family (eg, VZV or HSV), one or more or all V, D and/or J gene segments used in any configuration, aspect, method, example or embodiment of the disclosure can be selected from List A2.1.

Where one wishes to generate an antibody or antibody mixture to treat and/or prevent an infectious disease caused or mediated by CMV, one or more or all V, D and/or J gene segments used in any configuration, aspect, method, example or embodiment of the disclosure can be selected from List A2.2.

Where one wishes to generate an antibody or antibody mixture to treat and/or prevent an infectious disease caused or mediated by HIV, one or more or all V, D and/or J gene segments used in any configuration, aspect, method, example or embodiment of the disclosure can be selected from List A2.3.

Where one wishes to generate an antibody or antibody mixture to treat and/or prevent an infectious disease caused or mediated by Influenza Virus, one or more or all V, D and/or J gene segments used in any configuration, aspect, method, example or embodiment of the disclosure can be selected from List A2.4.

Optionally each VH segment in the locus of the disclosure is selected from List A1, A2, A1.1, A1.2, A2.1, A2.2, A2.3 or A2.4.

Optionally each VL segment in the locus of the disclosure is selected from List A1, A2, A1.1, A1.2, A2.1, A2.2, A2.3 or A2.4

Optionally each D segment in the locus of the disclosure is selected from List A1, A2, A1.1, A1.2, A2.1, A2.2, A2.3 or A2.4.

Optionally each JL segment in the locus of the disclosure is selected from List A1, A2, A1.1, A1.2, A2.1, A2.2, A2.3 or A2.4.

### Antibodies For Therapy & Prophylaxis of Patients of Specific Ancestry

The inventors, having undertaken the extensive Bioinformatics analysis exercise described herein, realised that the output of that analysis has made it possible to identify specific gene segments that are useful to produce antibody- and VH domain-based drugs that are tailored specifically to a patient's ancestry (ie, genotype). That is, antibodies can be selected on the basis that they are made in vivo in a transgenic non-human vertebrate (eg, mouse or rat with transgenic IgH loci) and particularly derived from gene segments that are relatively prevalent in members of the patient's population, ie, from individuals of the same human ancestry. Since variant distributions differ across different populations (see Table 13), this presumably reflects the effects of evolution, adaptation and conservation of useful variant gene types in those populations. Thus, by tailoring the antibody- based drugs according to the disclosure, it is possible to match the drug to the population gene biases, thus with the aim of making better drugs for that specific population of humans. Better can, for example, mean more efficacious, better neutralising, higher target antigen affinity, less immunogenic, less patient reactions to the drug etc. This can be determined empirically, as is standard in drug research and development processes.

Thus, the disclosure provides the following embodiments (numbered from clause 345 onwards):-
345. An isolated antibody for administration to a Chinese patient, the antibody comprising a human heavy chain, the heavy chain comprising a variable domain that is specific for an antigen
   and a constant region, wherein the constant region is a human constant region selected from a constant region (eg, an IGHG constant region) in Table 13 found in a Chinese population and with a cumulative frequency of at least 1 or 5%; and wherein
   (i) the variable domain is derived from the recombination of said human gene segments in a non-human vertebrate (eg, in a mouse or a rat); and/or (ii) the variable domain comprises non- human vertebrate (eg, mouse or rat) AID-pattern mutations and non-human vertebrate (eg, mouse or rat) terminal deoxynucleotidyl transferase (TdT)-pattern mutations.

   In another embodiment, the disclosure provides
   An isolated antibody for administration to a Chinese patient, the antibody comprising a human heavy chain, the heavy chain comprising a variable domain that is specific for an antigen and a constant region, wherein the constant region is a human constant region selected from a constant region (eg, an IGHG constant region) present in a Chinese population with a cumulative frequency of at least 5%;, and wherein
   (i) the variable domain is derived from the recombination of said human gene segments in a non-human vertebrate (eg, in a mouse or a rat);and/ or (ii) the variable domain comprises non- human vertebrate (eg, mouse or rat) AID-pattern mutations and non-human vertebrate (eg, mouse or rat) terminal deoxynucleotidyl transferase (TdT)-pattern mutations.

   In an example, the constant region is found in the 1000 Genomes database. In an example, the constant region is found in Table 13.
346. The antibody of clause 345 wherein the constant region is a IGHG1a, IGHG2a, IGHG3a, IGHG3b or IGHG4a constant region.
347. The antibody of clause 345 or 346, wherein the variable domain is derived from the recombination of a human VH gene segment with a human D gene segment and a human JH gene segment, the VH gene segment being selected from a VH in Table 13 found in a Chinese population and with a cumulative frequency of at least 5%.
   In another embodiment, the disclosure provides
   The antibody of clause 345 or 346, wherein the variable domain is derived from the recombination of a human VH gene segment with a human D gene segment and a human JH gene segment , the VH gene segment being selected from a VH present in a Chinese population with a cumulative frequency of at least 5%.
   In an example, the gene segment is found in the 1000 Genomes database. In an example, the gene segment is found in Table 13.
348. The antibody of clause 345, 346 or 347, wherein the variable domain is derived from the recombination of a human VH gene segment with a human D gene segment and a human JH gene segment, the D gene segment being selected from a D in Table 13 found in a Chinese population and with a cumulative frequency of at least 5%.
   In another embodiment, the disclosure n provides
   The antibody of clause 345, 346 or 347, wherein the variable domain is derived from the recombination of a human VH gene segment with a human D gene segment and a human JH gene segment , the D gene segment being selected from a D present in a Chinese population with a cumulative frequency of at least 5%.
   In an example, the gene segment is found in the 1000 Genomes database. In an example, the gene segment is found in Table 13.
349. The antibody of clause 345, 346, 347 or 348 wherein the variable domain is derived from the recombination of a human VH gene segment with a human D gene segment and a human JH gene segment, the JH gene segment being selected from a JH in Table 13 found in a Chinese population and with a cumulative frequency of at least 5%.
   In another embodiment, the disclosure provides
   The antibody of clause 345, 346, 347 or 348 wherein the variable domain is derived from the recombination of a human VH gene segment with a human D gene segment and a human JH gene segment , the JH gene segment being selected from a JH present in a Chinese population with a cumulative frequency of at least 5%.
   In an example, the gene segment is found in the 1000 Genomes database. In an example, the gene segment is found in Table 13.
350. An isolated VH domain identical to a variable domain as recited in any one of clauses 347 to 349, optionally fused at its C-terminus to a polypeptide (eg, an antibody Fc).
   In an embodiment, there is provided an isolated VH domain identical to a variable domain as recited in any one of clauses 347 to 349 which is part of a conjugate, conjugated with a label (eg, for imaging in the patient) or a toxin (eg, a radioactive toxic payload, such as for cancer treatment in the patient) or a half-life-extending moiety (eg, PEG of human serum albumin).
351. A pharmaceutical composition comprising the antibody or variable domain of any one of clauses 345 to 350 together with a pharmaceutically-acceptable excipient, diluent or a medicament (eg, a further antigen-specific variable domain, antibody chain or antibody).
352. An isolated antibody for administration to a Chinese patient, the antibody comprising a human heavy chain, the heavy chain comprising a variable domain that is specific for an antigen and a constant region, wherein the variable domain is derived from the recombination of a human VH gene segment with a human D gene segment and a human JH gene segment, the VH gene segment being selected from a VH in Table 13 found in a Chinese population and with a cumulative frequency of at least 5%; and wherein
   (i) the variable domain is derived from the recombination of said human gene segments in a non-human vertebrate (eg, in a mouse or a rat); and/or (ii) the variable domain comprises non- human vertebrate (eg, mouse or rat) AID-pattern mutations and non-human vertebrate (eg, mouse or rat) terminal deoxynucleotidyl transferase (TdT)-pattern mutations.

   In another embodiment, the disclosure provides
   An isolated antibody for administration to a Chinese patient, the antibody comprising a human heavy chain, the heavy chain comprising a variable domain that is specific for an antigen and a constant region, wherein the variable domain is derived from the recombination of a human VH gene segment with a human D gene segment and a human JH gene segment, the VH gene segment being selected from a VH present in a Chinese population with a cumulative frequency of at least 5%; and wherein
   (i) the variable domain is derived from the recombination of said human gene segments in a non-human vertebrate (eg, in a mouse or a rat); and/or (ii) the variable domain comprises non- human vertebrate (eg, mouse or rat) AID-pattern mutations and non-human vertebrate (eg, mouse or rat) terminal deoxynucleotidyl transferase (TdT)-pattern mutations.
353. The antibody of clause 352, wherein the variable domain is derived from the recombination of a human VH gene segment with a human D gene segment and a human JH gene segment , the D gene segment being selected from a D in Table 13 found in a Chinese population and with a cumulative frequency of at least 5%.
   In another embodiment, the disclosure provides
   The antibody of clause 352, wherein the variable domain is derived from the recombination of a human VH gene segment with a human D gene segment and a human JH gene segment , the D gene segment being selected from a D present in a Chinese population with a cumulative frequency of at least 5%.
   In an example, the gene segment is found in the 1000 Genomes database. In an example, the gene segment is found in Table 13.
354. The antibody of clause 352 or 353, wherein the variable domain is derived from the recombination of a human VH gene segment with a human D gene segment and a human JH gene segment, the JH gene segment being selected from a JH in Table 13 found in a Chinese population and with a cumulative frequency of at least 5%.
   In another embodiment, the disclosure provides
   The antibody of clause 352 or 353, wherein the variable domain is derived from the recombination of a human VH gene segment with a human D gene segment and a human JH gene segment , the JH gene segment being selected from a JH present in a Chinese population with a cumulative frequency of at least 5%.
   In an example, the gene segment is found in the 1000 Genomes database. In an example, the gene segment is found in Table 13.
355. An isolated VH domain identical to a variable domain as recited in any one of clauses 352 to 354, optionally fused at its C-terminus to a polypeptide (eg, an antibody Fc).
   In an embodiment, there is provided a VH domain identical to a variable domain as recited in any one of clauses 352 to 354 which is part of a conjugate, conjugated with a label (eg, for imaging in the patient) or a toxin (eg, a radioactive toxic payload, such as for cancer treatment in the patient) or a half-life-extending moiety (eg, PEG of human serum albumin).
356. A pharmaceutical composition comprising the antibody or variable domain of any one of clauses 352 to 355 together with a pharmaceutically-acceptable excipient, diluent or a medicament (eg, a further antigen-specific variable domain, antibody chain or antibody).
357. An antibody heavy chain or VH domain (eg, provided as part of an antibody) for therapy and/or prophylaxis of a disease or medical condition in a Chinese patient, wherein the heavy chain is a heavy chain produced by the following steps (or is a copy of such a heavy chain):-
   (a) Selection of an antigen-specific antibody heavy chain or VH domain from a non-human vertebrate (eg, a mouse or a rat), wherein the heavy chain or VH domain is derived from the recombination of a human VH gene segment with a human D gene segment and a human JH gene segment, the VH gene segment being selected from a VH in Table 13 found in a Chinese population and with a cumulative frequency of at least 5%;
   (b) Optional humanisation of the heavy chain by combining the variable domain of the heavy chain with a human constant region; or optional humanisation of the selected VH domain by combining with a human constant region.

   In another embodiment, the disclosure provides
   An antibody heavy chain or VH domain (eg, provided as part of an antibody) for therapy and/or prophylaxis of a disease or medical condition in a Chinese patient, wherein the heavy chain is a heavy chain produced by the following steps (or is a copy of such a heavy chain):-
   (a) Selection of an antigen-specific antibody heavy chain or VH domain from a non-human vertebrate (eg, a mouse or a rat), wherein the heavy chain or VH domain is derived from the recombination of a human VH gene segment with a human D gene segment and a human JH gene segment, the VH gene segment being selected from a VH present in a Chinese population with a cumulative frequency of at least 5%;
   (b) Optional humanisation of the heavy chain by combining the variable domain of the heavy chain with a human constant region; or optional humanisation of the selected VH domain by combining with a human constant region.

   In an example, the VH gene segment is found in the 1000 Genomes database. In an example, the gene segment is found in Table 13.
358. The antibody heavy chain or VH domain of clause 357, wherein the human constant region is as recited in clause 345 or 346.
359. An antibody heavy chain or VH domain as recited in clause 357 or 358 for use in a medicament for therapy and/or prophylaxis of a disease or medical condition in a Chinese patient.
360. A method of treating and/or preventing a disease or medical condition in a Chinese patient, the method comprising administering to the patient a therapeutically or prophylactically- effective amount of the antibody heavy chain or VH domain as recited in clause 357 or 358.
361. An isolated antibody for administration to a patient of European, East Asian, West African, South Asian or Americas ancestry, the antibody comprising a human heavy chain, the heavy chain comprising a variable domain that is specific for an antigen and a constant region, wherein the constant region is a human constant region selected from a constant region (eg, an IGHG constant region) in Table 13 found in a population of European, East Asian, West African, South Asian or Americas ancestry respectively and with a cumulative frequency of at least 1 or 5%;, and wherein
   (i) the variable domain is derived from the recombination of said human gene segments in a non-human vertebrate (eg, in a mouse or a rat); or (ii) the variable domain comprises non- human vertebrate (eg, mouse or rat) AID-pattern mutations and non-human vertebrate (eg, mouse or rat) terminal deoxynucleotidyl transferase (TdT)-pattern mutations.

   In another embodiment, the disclosure provides
   An isolated antibody for administration to a patient of European, East Asian, West African, South Asian or Americas ancestry, the antibody comprising a human heavy chain, the heavy chain comprising a variable domain that is specific for an antigen and a constant region, wherein the constant region is a human constant region selected from a constant region (eg, an IGHG constant region) present in a population of European, East Asian, West African, South Asian or Americas ancestry respectively with a cumulative frequency of at least 1 or 5%;, and wherein
   (i) the variable domain is derived from the recombination of said human gene segments in a non-human vertebrate (eg, in a mouse or a rat); or (ii) the variable domain comprises non- human vertebrate (eg, mouse or rat) AID-pattern mutations and non-human vertebrate (eg, mouse or rat) terminal deoxynucleotidyl transferase (TdT)-pattern mutations.

   In an example, the constant region is found in the 1000 Genomes database. In an example, the constant region is found in Table 13.
362. The antibody of clause 361 wherein the constant region is a IGHG1a, IGHG2a, IGHG3a, IGHG3b or IGHG4a constant region and the patient is of European ancestry.
363. The antibody of clause 361 or 362, wherein the variable domain is derived from the recombination of a human VH gene segment with a human D gene segment and a human JH gene segment, the VH gene segment being selected from a VH in Table 13 found in said population and with a cumulative frequency of at least 1 or 5%.
   In another embodiment, the disclosure provides
   The antibody of clause 361 or 362, wherein the variable domain is derived from the recombination of a human VH gene segment with a human D gene segment and a human JH gene segment , the VH gene segment being selected from a VH present in a Chinese population with a cumulative frequency of at least 5%.
   In an example, the gene segment is found in the 1000 Genomes database. In an example, the gene segment is found in Table 13.
364. The antibody of clause 361, 362 or 363, wherein the variable domain is derived from the recombination of a human VH gene segment with a human D gene segment and a human JH gene segment, the D gene segment being selected from a D in Table 13 found in said population and with a cumulative frequency of at least 1 or 5%.
   In another embodiment, the disclosure provides
   The antibody of clause 361, 362 or 363, wherein the variable domain is derived from the recombination of a human VH gene segment with a human D gene segment and a human JH gene segment , the D gene segment being selected from a D present in a Chinese population with a cumulative frequency of at least 5%.
   In an example, the gene segment is found in the 1000 Genomes database. In an example, the gene segment is found in Table 13.
365. The antibody of clause 361, 362, 363 or 364 wherein the variable domain is derived from the recombination of a human VH gene segment with a human D gene segment and a human JH gene segment, the JH gene segment being selected from a JH in Table 13 found in said population and with a cumulative frequency of at least 1 or 5%.
   In another embodiment, the disclosure provides
   The antibody of clause 361, 362, 363 or 364 wherein the variable domain is derived from the recombination of a human VH gene segment with a human D gene segment and a human JH gene segment , the JH gene segment being selected from a JH present in a Chinese population with a cumulative frequency of at least 5%.
   In an example, the gene segment is found in the 1000 Genomes database. In an example, the gene segment is found in Table 13.
366. An isolated VH domain identical to a variable domain as recited in any one of clauses 363 to 365, optionally fused at its C-terminus to a polypeptide (eg, an antibody Fc).
367. A pharmaceutical composition comprising the antibody or variable domain of any one of clauses 361 to 366 together with a pharmaceutically-acceptable excipient, diluent or a medicament (eg, a further antigen-specific variable domain, antibody chain or antibody).
368. An isolated antibody for administration to a patient of European, East Asian, West African or Americas ancestry, the antibody comprising a human heavy chain, the heavy chain comprising a variable domain that is specific for an antigen and a constant region, wherein the variable domain is derived from the recombination of a human VH gene segment with a human D gene segment and a human JH gene segment , the VH gene segment being selected from a VH in Table 13 found in a population of European, East Asian, West African, South Asian or Americas ancestry respectively and with a cumulative frequency of at least 1 or 5%; and wherein
   (i) the variable domain is derived from the recombination of said human gene segments in a non-human vertebrate (eg, in a mouse or a rat); or (ii) the variable domain comprises non- human vertebrate (eg, mouse or rat) AID-pattern mutations and non-human vertebrate (eg, mouse or rat) terminal deoxynucleotidyl transferase (TdT)-pattern mutations.

   In another embodiment the disclosure provides:-
   An isolated antibody for administration to a patient of European, East Asian, West African or Americas ancestry, the antibody comprising a human heavy chain, the heavy chain comprising a variable domain that is specific for an antigen and a constant region, wherein the variable domain is derived from the recombination of a human VH gene segment with a human D gene segment and a human JH gene segment , the VH gene segment being selected from a VH present in a population of European, East Asian, West African, South Asian or Americas ancestry respectively with a cumulative frequency of at least 1 or 5%; and wherein
   (i) the variable domain is derived from the recombination of said human gene segments in a non-human vertebrate (eg, in a mouse or a rat); or (ii) the variable domain comprises non- human vertebrate (eg, mouse or rat) AID-pattern mutations and non-human vertebrate (eg, mouse or rat) terminal deoxynucleotidyl transferase (TdT)-pattern mutations.

   In an example, the VH gene segment is found in the 1000 Genomes database. In an example, the gene segment is found in Table 13.
369. The antibody of clause 368, wherein the variable domain is derived from the recombination of a human VH gene segment with a human D gene segment and a human JH gene segment , the D gene segment being selected from a D in Table 13 found in said population and with a cumulative frequency of at least 1 or 5%.
   In another example there is provided
   The antibody of clause 368, wherein the variable domain is derived from the recombination of a human VH gene segment with a human D gene segment and a human JH gene segment , the D gene segment being selected from a D present in said population with a cumulative frequency of at least 1 or 5%.
   In an example, the D gene segment is found in the 1000 Genomes database. In an example, the gene segment is found in Table 13.
370. The antibody of clause 368 or 369, wherein the variable domain is derived from the recombination of a human VH gene segment with a human D gene segment and a human JH gene segment, the JH gene segment being selected from a JH in Table 13 found in said population and with a cumulative frequency of at least 1 or 5%.
   In another example there is provided
   The antibody of clause 368 or 369, wherein the variable domain is derived from the recombination of a human VH gene segment with a human D gene segment and a human JH gene segment , the JH gene segment being selected from a JH present in said population and with a cumulative frequency of at least 1 or 5%.
   In an example, the JH gene segment is found in the 1000 Genomes database. In an example, the gene segment is found in Table 13.
371. An isolated VH domain identical to a variable domain as recited in any one of clauses 368 to 370, optionally fused at its C-terminus to a polypeptide (eg, an antibody Fc).
372. A pharmaceutical composition comprising the antibody or variable domain of any one of clauses 368 to 371 together with a pharmaceutically-acceptable excipient, diluent or a medicament (eg, a further antigen-specific variable domain, antibody chain or antibody).
373. An antibody heavy chain or VH domain (eg, provided as part of an antibody) for therapy and/or prophylaxis of a disease or medical condition in a patient of European, East Asian, West African, South Asian or Americas ancestry, wherein the heavy chain is a heavy chain produced by the following steps (or is a copy of such a heavy chain):-
   (a) Selection of an antigen-specific antibody heavy chain or VH domain from a non-human vertebrate (eg, a mouse or a rat), wherein the heavy chain or VH domain is derived from the recombination of a human VH gene segment with a human D gene segment and a human JH gene segment, the VH gene segment being selected from a VH in Table 13 found in said population and with a cumulative frequency of at least 1 or 5%;
   (b) Optional humanisation of the heavy chain by combining the variable domain of the heavy chain with a human constant region; or optional humanisation of the selected VH domain by combining with a human constant region.

   In another embodiment, there is provided:-
   An antibody heavy chain or VH domain (eg, provided as part of an antibody) for therapy and/or prophylaxis of a disease or medical condition in a patient of European, East Asian, West African, South Asian or Americas ancestry, wherein the heavy chain is a heavy chain produced by the following steps (or is a copy of such a heavy chain):-
   (a) Selection of an antigen-specific antibody heavy chain or VH domain from a non-human vertebrate (eg, a mouse or a rat), wherein the heavy chain or VH domain is derived from the recombination of a human VH gene segment with a human D gene segment and a human JH gene segment, the VH gene segment being selected from a VH present in said population with a cumulative frequency of at least 1 or 5%;
   (b) Optional humanisation of the heavy chain by combining the variable domain of the heavy chain with a human constant region; or optional humanisation of the selected VH domain by combining with a human constant region.

   In an example, the VH gene segment is found in the 1000 Genomes database. In an example, the gene segment is found in Table 13.
374. The antibody heavy chain or VH domain of clause 373, wherein the human constant region is as recited in clause 361 or 362.
375. An antibody heavy chain or VH domain as recited in clause 373 or 374 for use in a medicament for therapy and/or prophylaxis of a disease or medical condition in a patient of said ancestry.
376. A method of treating and/or preventing a disease or medical condition in a patient of European, East Asian, West African, South Asian or Americas ancestry, the method comprising administering to the patient a therapeutically or prophylactically-effective amount of the antibody heavy chain or VH domain as recited in clause 373 or 374.

In embodiments herein, a Chinese patient can be a Han Chinese patient.

In embodiments herein, a patient of European ancestry can be a patient of Northern or Western European ancestry, Italian ancestry, British or Scottish ancestry, Finnish ancestry or Iberian ancestry.

In embodiments herein, a patient of East Asian ancestry can be a patient of Han Chinese ancestry, Japanese ancestry Chinese Dai ancestry, Vietnamese ancestry or Kinh ancestry.

In embodiments herein, a patient of West African ancestry can be a patient of Yoruba ancestry, Luhya ancestry, Gambian ancestry or Malawian ancestry.

In embodiments herein, a patient of Americas ancestry can be a patient of African American ancestry, African Caribbean ancestry, Mexican ancestry, Puerto Rican ancestry, Colombian ancestry or Peruvian ancestry.

In embodiments herein, a patient of South Asian ancestry can be a patient of Ahom ancestry, Kayadtha ancestry, Reddy ancestry, Maratha ancestry, or Punjabi ancestry.

In an example of any aspect, the cumulative frequency is at least 1, 2, 3, 4, 5, 6, 7, 8, 9, 10, 15, 20, 25, 30, 35, 40, 45, 50, 55, 60, 65, 70, 75, 80, 85, 90 or 95%.

It will be understood that particular embodiments described herein are shown by way of illustration and not as limitations of the invention. The principal features of this invention can be employed in various embodiments without departing from the scope of the invention. Those skilled in the art will recognize, or be able to ascertain using no more than routine study, numerous equivalents to the specific procedures described herein. Such equivalents are considered to be within the scope of this invention and are covered by the claims. All publications and patent applications mentioned in the specification are indicative of the level of skill of those skilled in the art to which this invention pertains. The use of the word "a" or "an" when used in conjunction with the term "comprising" in the claims and/or the specification may mean "one," but it is also consistent with the meaning of "one or more," "at least one," and "one or more than one." The use of the term "or" in the claims is used to mean "and/or" unless explicitly indicated to refer to alternatives only or the alternatives are mutually exclusive, although the disclosure supports a definition that refers to only alternatives and "and/or." Throughout this application, the term "about" is used to indicate that a value includes the inherent variation of error for the device, the method being employed to determine the value, or the variation that exists among the study subjects.

As used in this specification and claim(s), the words "comprising" (and any form of comprising, such as "comprise" and "comprises"), "having" (and any form of having, such as "have" and "has"), "including" (and any form of including, such as "includes" and "include") or "containing" (and any form of containing, such as "contains" and "contain") are inclusive or open-ended and do not exclude additional, unrecited elements or method steps.

The term "or combinations thereof" as used herein refers to all permutations and combinations of the listed items preceding the term. For example, "A, B, C, or combinations thereof is intended to include at least one of: A, B, C, AB, AC, BC, or ABC, and if order is important in a particular context, also BA, CA, CB, CBA, BCA, ACB, BAC, or CAB. Continuing with this example, expressly included are combinations that contain repeats of one or more item or term, such as BB, AAA, MB, BBC, AAABCCCC, CBBAAA, CABABB, and so forth. The skilled artisan will understand that typically there is no limit on the number of items or terms in any combination, unless otherwise apparent from the context.

Any part of this disclosure may be read in combination with any other part of the disclosure, unless otherwise apparent from the context.

All of the compositions and/or methods disclosed and claimed herein can be made and executed without undue experimentation in light of the present disclosure. While the compositions and methods of this invention have been described in terms of preferred embodiments, it will be apparent to those of skill in the art that variations may be applied to the compositions and/or methods and in the steps or in the sequence of steps of the method described herein without departing from the concept and scope of the invention. All such similar substitutes and modifications apparent to those skilled in the art are deemed to be within the scope and concept of the invention as defined by the appended claims.

The present invention is described in more detail in the following non limiting prophetic Examples.

### EXAMPLES

### EXAMPLE 1

### "Recombineered BAC Vectors to add Polymorphic V-regions to the Mouse Genome"

Figure 1 through 3 depict recombineering methods (see references above) that can be used to introduce polymorphic V-gene regions into genomic DNA. In one embodiment, a genomic fragment from the human heavy chain region is inserted into a bacterial artificial chromosome (BAC) vector by standard techniques. Preferably, such a BAC, which can range in size from 20-kb to 200-kb or more, can be isolated from libraries of BACs by standard techniques including sequence searches of commercially available libraries or by hybridization to bacterial colonies containing BACs to identify those with a BAC of interest.

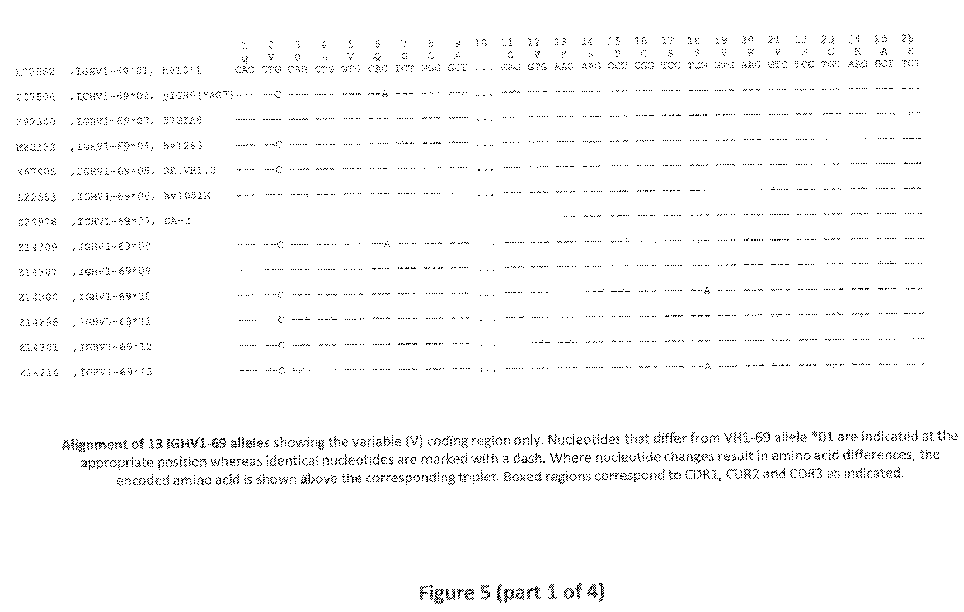

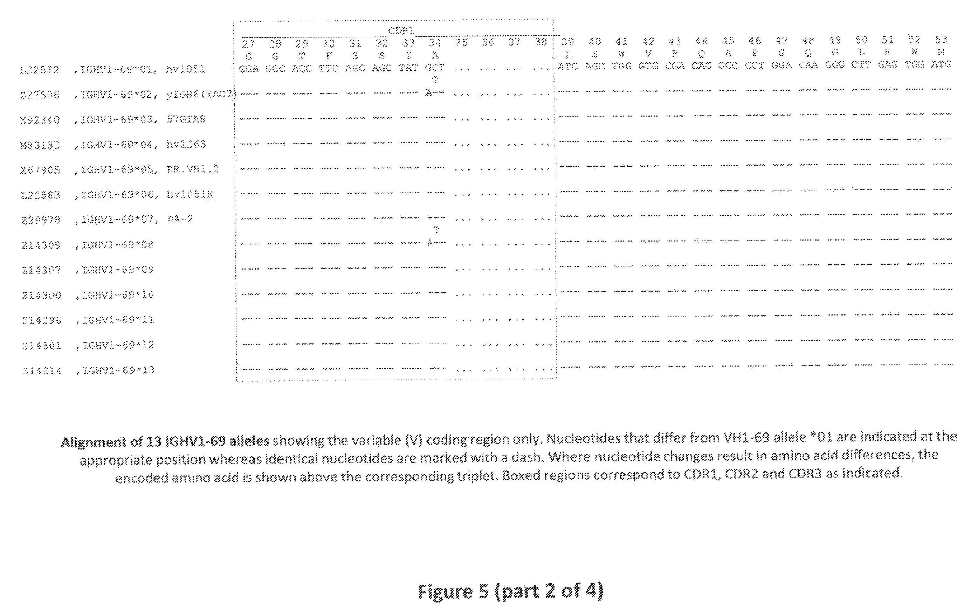

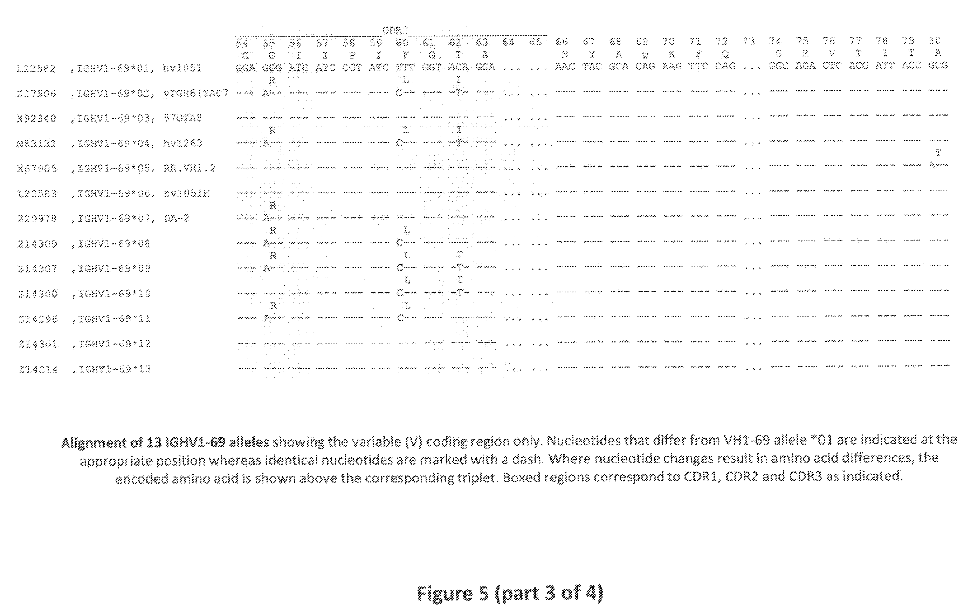

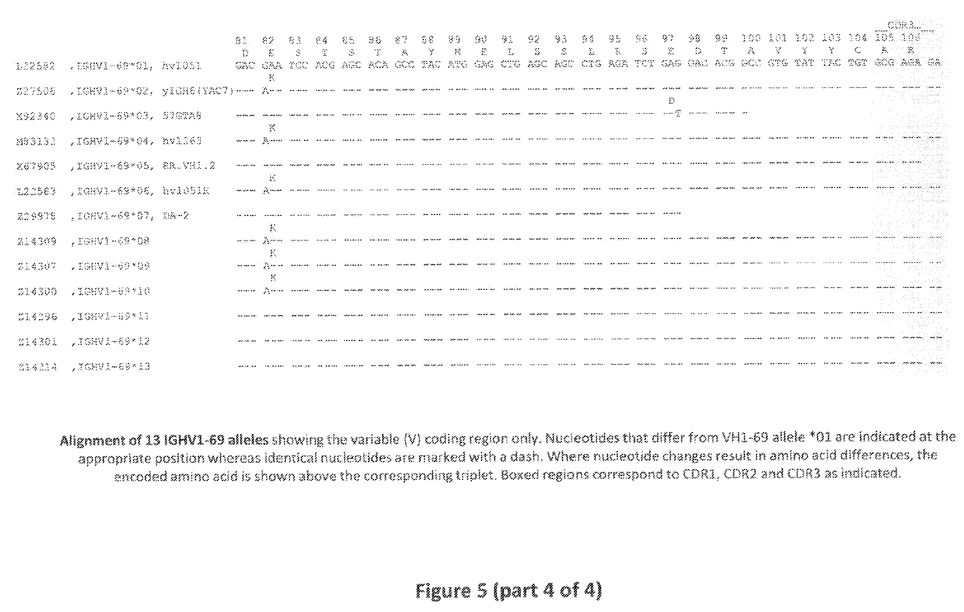

A BAC is chosen that has several VH gene segments; in Figure 1, these are generically identified as VH[a] through VH[z] for example. One skilled in the art will readily identify appropriate genomic fragments, for example, an approximately 120-kb fragment from human VH5-78 through VH1-68 which includes 5 endogenous active VH gene segments and 7 VH pseudogenes. Using recombineering techniques, the endogenous VH gene segments can be replaced by polymorphic VH or VL gene segments. In this example, two steps are required. The first step replaces the V-region coding exon of an endogenous VH gene segment with a positive-negative selection operon, in this example, an operon encoding an ampicillin resistance gene (*Amp*) and a streptomycin-sensitizing ribosomal protein (*rpsL*). Certain strains of bacteria can be selected for the absence of the rpsL gene by resistance to streptomycin. Short stretches of DNA homologous to sequences flanking
the endogenous VH gene exon are placed 5' and 3' of the rpsL-Amp operon. In the presence of appropriate recombination factors per standard recombineering techniques (see references above) recombination between the operon fragment and the BAC will result in replacement of the endogenous VH gene exon with the operon (Figure 1a) which are selected by resistance to ampicillin. The second step uses the same homologous sequences in order to replace the inserted operon with a desired polymorphic VH gene segment. In this example, a human VH1-69 gene is inserted (Figure 1b and 1c). In particular the *02 variant of VH1-69 is used [ref IMGT and Figure 5]. Successful integrations of the polymorphic VH gene segment are selected in bacteria that become resistant to streptomycin due to the loss of the operon, specifically the *rpsL* portion.

In this example, the two step process as described can be repeated for each of the endogenous VH gene segments or for as many endogenous gene segments that one wishes to replace with polymorphic V gene segments (Figure 1d).

As is apparent, any polymorphic V gene segment can be inserted in this manner and any endogenous V gene segment can act as a target, including pseudogenes. V gene segments in each of the heavy chain and two light chain loci can be replaced using this technique with appropriate genomic fragments available as BAC inserts.

Figure 2 depicts another method for creating a genomic fragment encoding polymorphic V gene segments. In this example, polymorphic V gene segments are inserted into a region of genomic DNA devoid of other genes, control elements or other functions. Such 'desert' regions can be selected based on sequence analysis and corresponding DNA fragments cloned into BACs or identified in existing BAC libraries. Starting with such a genomic fragment, recombineering techniques can be used to insert polymorphic V gene segments at intervals of, for example, 10-kb. In this example, a 150-kb genomic fragment might accommodate insertion of up to 15 polymorphic V gene segments. Insertion of the segments is a two-step process. The first recombineering step inserts the *rpsL-Amp* operon at a specific site. Sequences homologous to a specific site are used to flank the operon. These are used by the recombineering system to insert the element specifically into the BAC genomic fragment and positive events are selected by resistance to ampicillin (Figure 2a). The second step replaces the operon in the genomic fragment with a polymorphic V gene segment by a similar recombineering step using the same sequence homology (Figure 2b). In this example, both
exons and promoter element of a polymorphic VH gene segment are inserted, resulting in replacement of the *rpsL-Amp* operon and therefore resistance to streptomycin (Figure 2c).

The two step technique for inserting polymorphic V gene segments into a specific site on the genomic fragment can be repeated multiple times resulting in a BAC genomic fragment with several polymorphic gene segments, including their promoter elements. It is apparent that the examples shown in Figures 1 and 2 can be combined wherein the technique for insertion can be used to add extra polymorphic V gene segments to a BAC genomic fragment as depicted in Figure 1. One might choose to add these extra segments to an IG genomic fragment since such a fragment would be more amenable to proper IG gene expression once inserted into a non-human mammal's genome. It is known that a genomic fragment can have elements such as enhancers or elements that contribute to certain chromatin conformations, both important in wild-type gene expression.

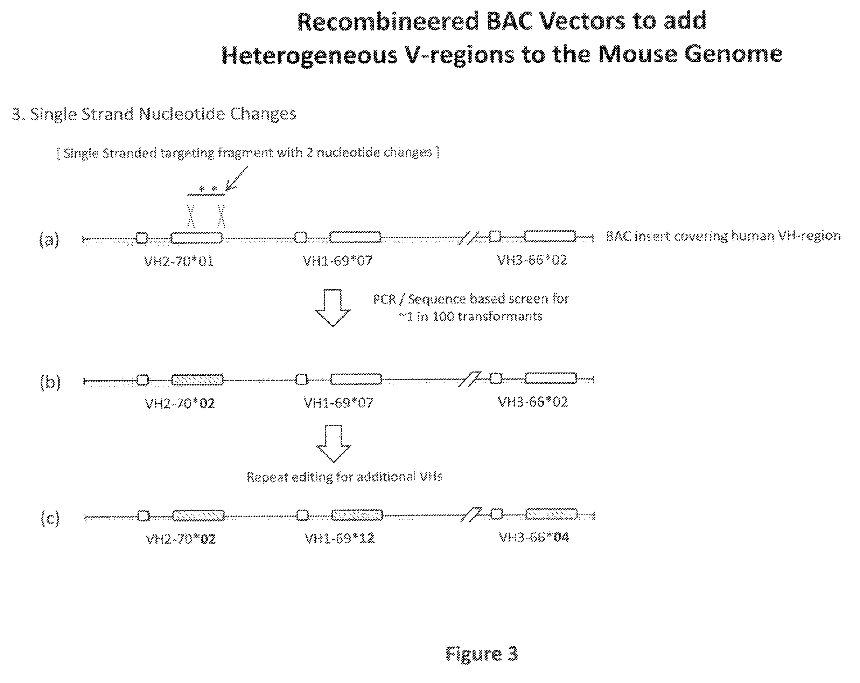

Figure 3 depicts an additional method to create genomic fragments with polymorphic V gene segments. This method depends upon the efficiency with which short (around 50 to 150 bases, preferably 100 bases) single stranded DNA fragments recombine with a homologous sequence using recombineering (Nat Rev Genet. 2001 Oct;2(10):769-79; Recombineering: a powerful new tool for mouse functional genomics; Copeland NG, Jenkins NA, Court DL). The recombinases used in recombineering preferentially bind and use such short single-stranded fragments of DNA as a substrate for initiating homologous recombination. The efficiency can be as high as 10-2, that is, a positive event can be found in approximately 100 randomly picked (not selected) clones resulting from recombineering. A positive event in this example occurring when one or more single nucleotide changes introduced into the single-stranded fragment get transferred to the BAC insert containing V gene segments and surrounding genomic DNA, said nucleotide change or changes occurring at a homologous sequence on the BAC.

Polymorphic V gene segments can differ from endogenous V gene segments by only 1 or 2, or up to 10 or 15 nucleotide changes, for example. An example of such nucleotide polymorphisms are depicted in Figure 5. Short single stranded regions that encompass the polymorphic nucleotide changes can be chemically synthesized using standard techniques. The resulting single stranded DNA fragments are introduced into bacteria and via recombineering techniques approximately 1 in 100 BAC fragments will have incorporated the polymorphic nucleotides via homologous incorporation of
the single stranded fragment (Figure 3a). BACs with the desired nucleotide change can be identified by screening for example several hundred individual clones by polymerase chain reaction (PCR) amplification and sequencing, both by standard techniques. In the example, two nucleotide changes will convert a VH1-69*01 gene segment into a VH1-69*02 gene segment (Figure 3b).

It is clear that this process can be repeated for multiple endogenous V gene segments contained on a single BAC genomic fragment. In addition, the techniques depicted in Figure 2 can be used to add additional polymorphic V gene segments by insertion into regions between existing V gene segments. As would be evident to one skilled in the art, a combination of these techniques can be used to create numerous variations of both polymorphic and endogenous human V gene segments. And it would be evident that several different genomic fragments with engineered polymorphic V gene segments and endogenous human V gene segments can be combined to create even more variations.

### EXAMPLE 2

### "Adding Polymorphic V-regions to the Genome using SRMCE of Modified BACs"

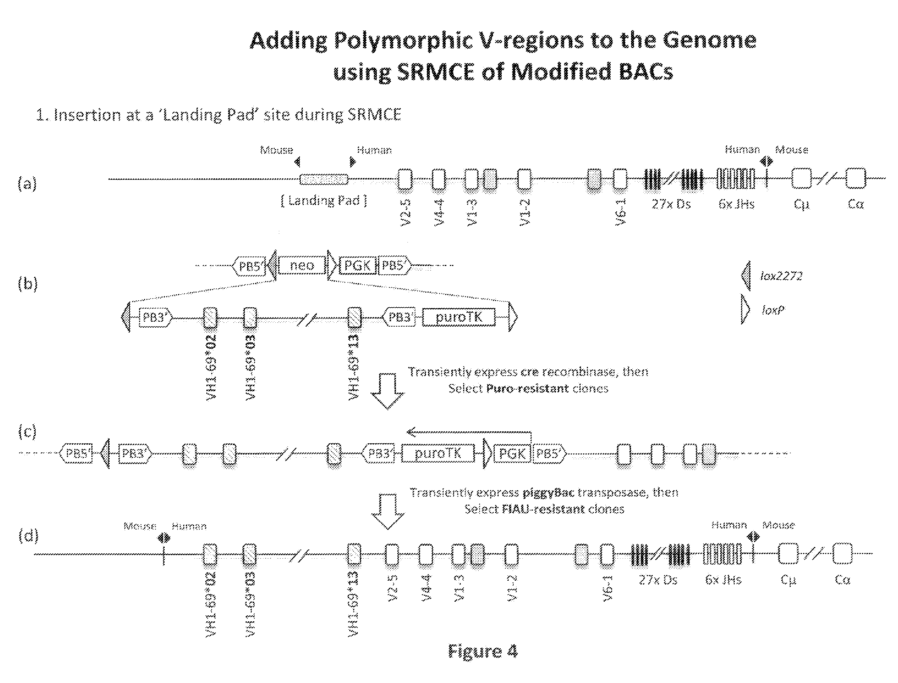

Modified BACs with polymorphic V gene segments created using the methods described in Example 1 can be used to alter the genome of non-human mammals. These alterations can result in an intact IG locus in which normal immunoglobulin region recombination results in VDJ or VJ combinations which includes the human V gene segments. An example of how such an animal can be created is by altering the genome of, for example, mouse embryonic stem (ES) cells using the strategy outlined in Figure 4.

One technique to integrate modified BACs with polymorphic V gene segments into a genome is sequential recombinase mediated cassette exchange (SRMCE). The technique is described in WO2011004192 (Genome Research Limited)

SRMCE provides for a locus modified with a 'landing pad' inserted at a specific location. This insertion can either be *de novo* via homologous recombination or as a consequence of a previous BAC insertion. In this example, the landing pad is inserted in the mouse IGH locus between the most 3' J gene segment and the Cµ gene segment and a previous BAC insertion *via* SRMCE techniques have resulted in the addition of 5 human V gene segments and 2 V region pseudogenes. The landing pad has elements as shown in Figure 4 that will allow the selection of correct insertion of a second targeting BAC fragment. The specificity of this insertion is provided by cre recombinase-mediated exchange between permissive *lox* sites. A *lox* site is permissive for recombination only with a compatible lox site. In this example, the *loxP* site will only recombine with *loxP* and *lox*2272 will only recombine with *lox*2272*.* This provides directionality to the insertion of the BAC fragment as depicted in Figure 4b and 4c.

ES cell clones with correct insertions are selected from a pool of clones without insertions or with non-productive insertions by resistance to puromycin. Resistance to puromycin results from the juxtaposition of an active promoter element, PGK, with the *puroTK* coding region. Correct insertions are verified by standard techniques including PCR of junctions, PCR of internal elements, Southern blotting, comparative genomic hybridization (CGH), sequencing and etc. In the example, correct *lox*2272*-lox*2272 and *loxP-loxP* recombination also results in two intact sets of piggyBac elements that did not exist prior to insertion. An intact piggyBac element is comprised of a set of inverted repeats which are depicted in the figure by "PB5"' and "PB3"'. An appropriated oriented set of *piggyBac* elements are the substrate of piggyBac transposase which can catalyse recombination between the elements, resulting in deletion of intervening sequences as well as both elements. The DNA remaining after a *piggyBac* transposition is left intact and is lacking any remnant of the *piggyBac* element. In the example, ES cell clones with successful *piggyBac* transposition are selected by loss of the active *puroTK* element which renders the cells resistant to the drug FIAU (Figure 4c and 4d).

The final product of the SRMCE method in this example is a IGH locus with several polymorphic V gene segments inserted along with a set of endogenous unmodified VH gene segments between sequences of the mouse genome on the 5' side and the mouse IGH constant region gene segments on the 3' side. The polymorphic V gene segments are positioned such that they can participate in the recombination events associated with B cell maturation yielding VDJ gene segments. These gene segments can then be transcribed and spliced to the mouse constant region. Translation of these transcripts will result in the production of an antibody heavy chain encoded by the polymorphic V gene segment, a human DH gene segment, a human JH gene segment and a mouse constant heavy chain gene segment.

As is well known to those skilled in the art, an ES cell clone can be used to create a line of genetically modified mice via injection of said cells into a mouse blastocyst embryo, transferring the injected embryo to a suitable recipient and breeding the chimeric offspring that result. The modified gene locus can be propagated through breeding and made either heterozygous or homozygous depending on the genetic cross.

It is evident from the structure of the IGH locus provided in this example and by knowledge of the mechanisms involved in B cell receptor (BCR) and antibody gene rearrangements that a large set of different combinations of polymorphic V gene segments with various DH and JH gene segments will result and these can contribute to a large repertoire of functional antibody genes in a population of B cells in genetically modified animals. In this example, several different human VH1-69 polymorphs are incorporated to provide superhuman VH diversity. This particular VH gene segment is known to be prevalent in antibodies that bind infectious disease pathogens (such as influenza virus) and therefore the antibody repertoire of a mouse with the genetic modification of this example would be expected to produce antibodies with a bias in favour of those that bind infectious disease pathogens. The repertoire, in other words, would have a larger subset of antibodies with superior affinities for pathogen antigens. Examples of such pathogens include influenza virus, hepatitis C virus (HCV) and human immunodeficiency virus-1 (HIV-1) (see also table above).

### EXAMPLE 3

### "Alignment of 13 VH1-69 Alleles"

Building a more diverse antibody repertoire by incorporating additional V gene segment polymorphs requires availability of polymorphic variants of V gene segments. One source of such variants include sequence databases. In this example, 13 distinct variants of the VH1-69 gene segment are provided.

These variant sequences and comparisons are drawn from the "IMmunoGeneTics" IMGT Information System (www.imgt.com) database. Figure 5 is a diagram of the alignment of variants *02 through *13 with the *01 variant. The VH1-69*01 nucleotide and amino acid sequence is provided at the top of the figure. Where the remaining variants are identical to the *01 variant sequence a dash is inserted below the sequence. Nucleotide differences are noted alongside the appropriate variant and if the sequence change results in a protein coding change, the amino acid change is indicated above the triplet.

Figure 5 depicts between 1 and 4 amino acid changes for each variant in comparison to the *01 variant. All of the amino acid changes occur in the part of the heavy chain protein encoding the complementarity determining regions (CDRs). These regions are responsible for antigen specificity and the affinity of the antibody for the antigen. It is evident that providing additional polymorphic CDRs in a repertoire of antibodies will increase the likelihood of there being an antibody with superior binding characteristics for various antigens. In several reports, it has been observed that the VH1-69-encoded variable region of the heavy chain is often found in antibodies that bind influenza virus, HCV and HIV-1 antigens (see table above). Therefore incorporating the polymorphic V gene segments of this example into a transgenic animal model using the methods of Examples 1 and 2 would likely result in an antibody repertoire in said transgenic animal with more antibodies that bind to antigens associated with these and other pathogens. And as is known in the art, a larger repertoire increases the probability of finding monoclonal antibodies using, for example, hybridoma technology, that bind with high affinity and specificity to a desired antigen.

This disclosure therefore describes in these examples a transgenic mouse model which can be immunized with pathogen or other antigens. Plasma B cells from such an immunized mouse can be used to make a hybridoma library that can be screened for production of antibodies that bind the pathogen antigens. This library will be superior to libraries from traditional transgenic mice for finding such antibodies given the addition of polymorphic VH1-69 gene segments to the IGH locus in said transgenic mouse.

These examples are not limiting to the human polymorphic V gene segments that can be chosen or to the methods used to introduce them into an animal model. The method can be used to construct a transgenic locus with immunoglobulin D and/or J segments. The V, D, J segments can be from a plurality of human sources (optionally more than one human ethnic population).

### EXAMPLE 4

### Human IgH JH Gene Variants Selected from the 1000 Genomes Database

Data is presented for human JH2, 5 and 6 variants. In Tables 10A, 11A and 12A samples from humans from various populations are listed where the sequence analysis of the inventors has revealed the presence of polymorphisms in one or both IgH JH alleles. The population codes are explained in Table 8 above. The polymorphisms are nucleotide variants from JH2, 5 and 6 reference sequences (SEQ ID NOs: 1, 2 and 3 respectively; see below). All references are sequences taken from the Ensembl database (www.ensembl.org). The JH5 reference is human IgH J5-001 disclosed in that database. The JH6 reference is human IgH J6-001 disclosed in that database. The JH2 reference is human IgH J2-001 disclosed in that database.

The reference nucleotide and encoded amino acid sequences are shown on the next page. Alignments with encoded amino acid sequences are also provided, including the corresponding position numbers on human chromosome 14.

Variant Frequencies are shown in Tables 10A, 11A and 12A and these relate to the frequency of the variants in the 1000 Genomes Database (release current at October 2011).

Tables 10B, 11B and 12B show the non-synonymous nucleotide polymorphisms in the human JH variants, as sorted by the present inventors from the 1000 Genomes database. Position numbers corresponding to nucleotide positions on human chromosome 14 are shown for variant positions (chromosome 14 being the chromosome bearing the IgH locus in humans). Thus, for example, the first entry in Table 11B is "14:106330027:A/C" which refers to a position in a variant JH5 sequence wherein the position corresponds to position 106,330,027 on human chromosome 14, such position being A (adenine) in the reference sequence. The "C" indicates that the present inventors observed a mutation to cytosine at this position in the variants found in the 1000 Genomes database. This change leads to a change at the amino acid level of the encoded sequence (i.e., a "non-synonymous" change), in this case a change from a serine (found in the reference) to an alanine in the variant.

### Example 5:

### Human Antibody Gene Segment Variant Identification & Population Analysis

The genomic coding region coordinates for each target gene for variant analysis were identified from the Ensembl WWW site (www.ensembl.org) using coordinates from the GRCh.p8 Human Genome assembly (www.ncbi.nlm.nih.gov/projects/genome/assembly/grc). Using the collected gene location coordinates, variant data was extracted from the public ftp site of the 1000 Genomes Project using the Perl 'Variant Pattern Finder' (VPF - www.1000genomes.org/variation-pattern- finder-api-documentation).

Data extracted by VPF was post processed using software to extract all non-synonymous (NSS) variants with their associated genotype calls. Genotypes calls were assembled to form unique haplotypes, representing groups of NSS variants associated with 1000 Genome population groups and frequency of occurrence within those populations.

The output of the analysis results in tables such as in Table 13. The main body of the table describes each haplotype in turn giving a unique ID for that gene (in the range a-z,aa-zz), the population frequencies and occurrence in individuals and unique population groups; one or more subsequent columns describe the DNA base calls at each location that form the haplotype giving both the base from the reference sequence or the variant base call.

Table 13 was constructed in this manner. The table can be read as follows:

The first four columns (left to right) consist of (1) the haplotype ID letter ('ref' indicates reference - the DNA base call at each genomic location from the GRCh37 Human Reference Assembly) (2) the observed cumulative frequency of the haplotype among the different populations (3) the number of individuals in which a specific haplotype was observed (4) the number of unique population groups that the identified individuals belong to (the actual population group identifiers are displayed as a string of ID's in the most right hand column for each haplotype. For example haplotype 'a' has a population ID string of '3,4,9,13').

The populations are numbered as follows (population labels being according to 1000 Genomes Project nomenclature)
1= ASW;
2= CEU;
3=CHB;
4=CHS;
5=CLM;
6=FIN;
7=GBR;
8=IBS;
9=JPT;
10=LWK;
11=MXL;
12=PUR;
13=TSI;
14=YRI.

Subsequent columns detail a single point variant and have the following format (top to bottom) (1) the human genomic location of the variant (format [chromosome number]: [location] e.g. '14:106204113'); (2) The identifier for the point variant as defined in DbSNP (www.ncbi.nlm.nih.gov/projects/SNP/); (3) One or additional rows show the amino acid change as result of the variant for a specific transcript (denoted by the Ensembl transcript ID in the most right- hand column for each row), the format is the amino acid in the reference sequence followed by '->' and the amino acid caused by the substitution of the variant in the reference sequence (e.g. 'Gly- >Arg' means a that the translated reference sequence would result in a glycine at that location, whereas the substitution of the identified variant would result in translated protein containing arginine) using the IUPAC three letter amino acid codes (http://pac.iupac.org/publications/pac/pdf/1972/pdf/3104x0639.pdf). Subsequent rows (one per haplotype) show the DNA base at each location, bases matching the reference sequence are shown in black on white back ground, bases varying from the reference are shown as white text on a black background.

The most right-hand column contains the Ensembl transcript ID's (e.g. 'ENST00000390542') for each of the gene transcript and relates to the amino acid changes to the left of this column. Because the transcripts are differing lengths each variant position may or may not have an associated amino acid change at the that position.

### Example 6:

### Transgenic Mice. B-cells. Hybridomas. Antibodies & Heavy Chains Based on Human JH6*02

A functional human gene segment repertoire (from V_{H}2-26 to J_{H}6, see the IMGT database for the structure of the human IgH locus;
http://www.imgt.org/IMGTrepertoire/index.php?section=LocusGenes&repertoire=locus&species=h uman&group=IGK) was sectored by the inventors to produce two different transgenic heavy chain alleles (denoted S2F and S3F) and corresponding mice. The transgenic alleles were expressed in the mice and the heavy chain repertoires were assessed at the RNA transcript level. Deep sequence
analysis was carried out using Bioinformatics methods to assess V, D and JH gene usage, including in variable domain sequences having a HCDR3 length of at least 20 amino acids. Endogenous, mouse variable region gene segments were inactivated by inversion (as per the method described in WO2011004192).

### Sequencing of Human Donor DNA Samples: Identification of Conserved JH6*02 Variant

DNA samples from 9 anonymised consenting human donors were obtained by taking cheek swabs.

The samples were processed and the DNA Samples were extracted follow the protocol of QIAamp DNA Mini Kit (Cat.No.51304, Qiagen).

PCR reactions were set up to amplify the JH6 region and PCR products were sequenced (PCR Oligos sequence: Fwd. 5'-AGGCCAGCAGAGGGTTCCATG-3' (SEQ ID NO: 444), Rev. 5'-GGCTCCCAGATCCTCAAGGCAC-3' (SEQ ID NO: 445)).

Sequence analysis was carried out by comparing to the JH6 reference sequence from IMGT annotated database (http://www.imgt.org/), and this identified that all 9 donor genomes contained the human JH6*02 variant, with this variant being in the homozygous state in 7 out of the 9 donors. The inventors also consulted the genomic sequences publicly available for Jim Watson and Craig Venter at Ensembl human genome database [http://www.ensembl.org/]. These too contained the human JH6*02 variant. This confirmed to the inventors that human JH6*02 is a common, conserved variant in humans, and thus a good candidate for construction of a transgenic IgH locus as per the invention

### Identification of Suitable Human DNA Sepuence BACs

A series of human bacterial artificial chromosome (BAC) clones were identified from Ensemble (http://www.ensembl.org/index.html) or UCSC (http://genome.ucsc.edu/) human database searches based on gene name (IGH) or location (chromosome 14: 106026574-107346185). Seven human RP11 BAC clones (see an extract of the UCSC database in Figure 10, identified BACs being circled) were selected, RP11-1065N8 BAC carrying human JH6*02. In total, the following BACs were identified as sources of human IgH locus DNA: RP11-1065N8, RP11-659B19, RP11-141I7, RP-112H5, RP11-101G24, RP11-12F16 and RP11-47P23.

With a similar approach, different BAC clones (eg, different RP11 clone IDs or different sources from RP11) or genetically engineered BACs can be selected for insertion into the mouse IGH locus to provide different sets of human repertoires in the transgenic mouse.

### Construction of Transgenic IgH Loci

Insertion of human heavy gene segments from a 1st IGH BAC (RP11-1065N8) into the IGH locus of mouse AB2.1 ES cells (Baylor College of Medicine) was performed to create a heavy chain allele denoted the S1 allele. The inserted human sequence corresponds to the sequence of human chromosome 14 from position 106494908 to position 106328951 and comprises functional heavy gene segments V_{H}2-5, V_{H}7-4-1, V_{H}4-4, V_{H}1-3, V_{H}1-2, V_{H}6-1, D1-1, D2-2, D3-9, D3-10, D4-11, D5-12, D6-13, D1-14, D2-15, D3-16, D4-17, D5-18, D6-19, D1-20, D2-21, D3-22, D4-23, D5-24, D6-25, D1-26, D7-27, J_{H}1, J_{H}2, J_{H}3, J_{H}4, J_{H}5 and J_{H}6 (in 5' to 3' order), wherein the JH6 was chosen to be the human JH6*02 variant. The insertion was made between positions 114666435 and 114666436 on mouse chromosome 12, which is upstream of the mouse Cµ region. The mouse V_{H}, D and J_{H} gene segments were retained in the locus, immediately upstream of (5' of) the inserted human heavy chain DNA.

A second allele, S2 was constructed in which more human functional VH gene segments were inserted upstream (5') of the 5'-most V_{H} inserted in the S1 allele by the sequential insertion of human DNA from a second BAC (BAC2). The inserted human sequence from BAC2 corresponds to the sequence of human chromosome 14 from position 106601551 to position 106494909 and comprises functional heavy chain gene segments V_{H}3-13, V_{H}3-11, V_{H}3-9, V_{H}1-8, V_{H}3-7. The mouse V_{H}, D and J_{H} gene segments were retained in the locus, immediately upstream of (5' of) the inserted human heavy chain DNA. In a subsequent step, these were inverted to inactivate them, thereby producing S2F mice in which only the human heavy chain variable region gene segments are active.

A third allele, S3 was constructed in which more human functional V_{H} gene segments were inserted upstream (5') of the 5'-most V_{H} inserted in the S2 allele by the sequential insertion of human DNA from a third BAC (BAC3). The inserted sequence corresponds to the sequence of human chromosome 14 from position 106759988 to position 106609301, and comprises functional heavy chain gene segments, V_{H}2-26, V_{H}1-24, V_{H}3-23, V_{H}3-21, V_{H}3-20, V_{H}1-18, and V_{H}3-15. The mouse V_{H}, D and J_{H} gene segments were retained in the locus, immediately upstream of (5' of) the inserted human heavy chain DNA. In a subsequent step, these were inverted to inactivate them, thereby producing S3F mice in which only the human heavy chain variable region gene segments are active.

Mice bearing either the S2F or S3F insertion into an endogenous heavy chain locus were generated from the ES cells using standard procedures. The other endogenous heavy chain locus was inactivated in the mice by insertion of an inactivating sequence comprising neoR into the mouse JH- Cµ intron (to produce the "HA" allele).

### Immunisation procedure

Transgenic mice of the S2F or S3F genotype were primed with 20-40ug recombinant proteins obtained commercially or produced in house with Antigen 1 (OVA (Sigma A7641); Antigen 2 (a human infectious disease pathogen antigen) and Antigen 3 (a human antigen) via the ip route in complete Freunds adjuvant (Sigma F 5881) and 10ug/animal CpG (CpG oligo; Invivogen, San Diego, California, USA) and then boosted twice in about two weekly intervals with about half the amount of antigen in incomplete Freunds adjuvant (Sigma F 5506) and 10ug/animal CpG. Final boosts were administered two weeks later iv without any adjuvant and contained 5-10 ug protein in PBS.

### Hybridoma fusion procedure

Spleens were taken 3 days after the final boost and spleenocytes were treated with CpG (25 ^m final concentration) for and left until the following day. Cells were then fused with SP0/2 Ag14 myeloma cells (HPA Cultures Cat No 85072401) using a BTX ECM2001 electrofusion instrument. Fused cells were left to recover for 20 minutes then seeded in a T75 flask until next morning.

Then the cells were spun down and plated out by dilution series on 96-well culture plates and left for about 10 days before screening. Media was changed 1-3 times during this period.

### Screening

Culture supernatants of the hybridoma wells above were screened using homogenious time resolved fluorescence assay (htrf) using Europium cryptate labelled anti-mouse IgG (Cisbio anti-mouse Ig Europium Cryptate) and a biotin tagged target antigen with a commercially available streptavidin conjucated donor (Cisbio; streptaviding conjugated D2) or by IgG-specific 384 well ELISA. Positive wells identified by htrf were scaled to 24-well plates or immediately counterscreened using an IgG- specific detection ELISA method. Positives identified by primary ELISA screen were immediately expanded to 24-well plates. Once cultures were expanded to 24-well stage and reached conflueny, supernatants were re-tested using htrf or IgG-specific ELISA to confirm binding to target antigen. Supernatant of such confirmed cultures were then also analysed by surface plasmon resonance using a BioRad ProteOn XPR36 instrument. For this, antibody expressed in the hybridoma cultures was captured on a biosensor GLM chip (BioRad 176-512) which had an anti-mouse IgG (GE Healthcare BR-1008-38) covalently coupled the biosensor chip surface. The antigen was then used as the analyte and passed over the captured hybridoma antibody surface. For Antigen 2 and Antigen 3, concentrations of 256nM, 64nM, 16nM, 4nM and 1nM were typically used, for Antigen 1, concentrations of 1028nM, 256nM, 64nM, 16nM and 4nM were typically used, binding curves were double referenced using a 0nM injection (i.e. buffer alone). Kinetics and overall affinities were determined using the 1:1 model inherent to the BioRad ProteOn XPR36 analysis software.

Any clones with confirmed binding activity were used for preparing total RNA and followed by PCR to recover the heavy chain variable region sequences. Standard 5'-RACE was carried out to analyse RNA transcripts from the transgenic heavy chain loci in the S2F and S3F mice. Additionally, deep sequence analysis of almost 2000 sequences produced by the mice was carried out.

### Bioinformatics Analysis

Sequences for analysis were obtained from two different methods:
- The first is from RNA extracted from the spleen: first cDNA strand was synthesized using an oligo based on the Cmu region of the mouse IGH locus as a PCR template. PCR was performed using this oligo with an oligo dT-anchor primer. Then PCR product was cloned into pDrive vector (Qiagen) and then sequenced.
- The second is from hybridomas generated through electro-fusion: total RNA was extracted from hybridoma lines of interest using standard Trizol methods and frozen at -80 oC for long term storage. cDNA was generated from 100ng total RNA using standard Superscript III reverse transcriptase and a gene-specific reverse primer binding to all mouse IgG isotypes for heavy chain and a mouse kappa constant region primer for the light chain amplification. 2-3 ul of cDNA were then used as template in a PCR reaction using Pfu DNA polymerase and a panel of degenerate forward primers annealing to the leader sequence of the human immunoglobulin variable domain as well as one mouse pan-IgG reverse primer. PCR products were run out of a 1% agarose gel and bands of approximately 350-450 basepairs extracted and purified. DNA was then sequenced.

The sequences from the first method can either be from IgM from Naive mice or IgG from immunised mice. The samples from the second method are all from IgG from immunised mice, and specific to the immunising antigen. Almost 2000 sequences were analysed.

The sequences were obtained as a pair of forward and reverse reads. These were first trimmed to remove low-quality base calls from the ends of the reads (trimmed from both ends until a 19 nucleotide window had an average quality score of 25 or more). The reads were combined together by taking the reverse complement of the reverse read, and aligning it against the forward read. The alignment scoring was 5 for a match, -4 for a mismatch, a gap open penalty of 10 and a gap extension penalty of 1. A consensus sequence was then produced by stepping through the alignment and comparing bases. When there was a disagreement the base with the highest quality value from sequencing was used.

The LAST© (Basic Local Alignment Search Tool) (Camacho C., Coulouris G., Avagyan V., Ma N., Papadopoulos J., Bealer K., & Madden T.L. (2008) "BLAST©: architecture and applications." BMC Bioinformatics 10:421 http://www.ncbi.nlm.nih.gov/pubmed/20003500) program 'blastn' was then used to find the germline J and V segments used in each sequence. A wordsize of 30 was used for V matching, and 15 for J matching. The database searched against was constructed from the NGS sequencing of the BACs which were used to generate the Kymouse.

If a sequence matched both a V and a J segment, the sequence between the two was then compared to a database of germline D segments in the mouse using 'blastn' with a wordsize of 4 and the options 'blastn-short' and 'ungapped'. This was used to assign a D segment, if possible. The CDR3 was identified by searching for the conserved "TATTACTGT" sequence in the V segment, and the "CTGGGG" in the J segment. If these motifs were not found, then up to 4 mismatches were allowed. The IMGT definition of CDR3 was used, so the CDR3 length is calculated from after the "TGT" in the V to before the "TGG" in the J. Sequences with an out of frame junction (those which do not have a CDR3 nucleotide length divisible by 3) or which contained a stop codon ("TAA", "TAG" or "TGA") were excluded.

The identity of the matching V, J and D segments as well as the CDR3 length from this assignment were then saved as a table for downstream analysis. The ratio of IGHJ6*02 used increased from the naive to immunised mice, as well as being enriched in the sub-population of sequences with a long HCDR3 (defined as consisting of 20 or more amino acids):

| | All | | HCDR3>20 | | % HCDR3>20 |
|---|---|---|---|---|---|
| | JH6*02% | Total Count | JH6*02% | Total Count | |
| Naive | 22.31% | 1340 | 91.11% | 45 | 3.36% |
| Immunised | 37.50% | 256 | 66.67% | 9 | 3.52% |
| Hybridoma | 36.13% | 119 | 63.64% | 11 | 9.24% |

This shows that the JH6*02 gene segment is selected for by immunisation, as the proportion of JH6*02 usage increases after immunisation. JH6*02 is also used in the majority of antibodies with a long HCDR3 length, which is desirable for targets which are specifically bound by long HCDR3 length antibodies.

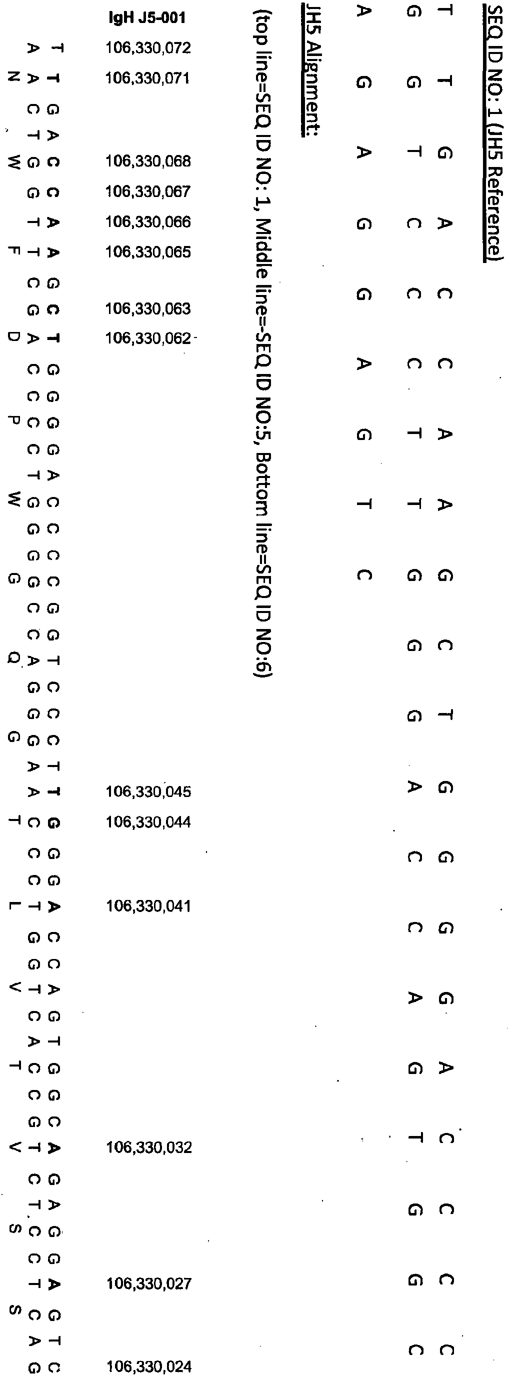

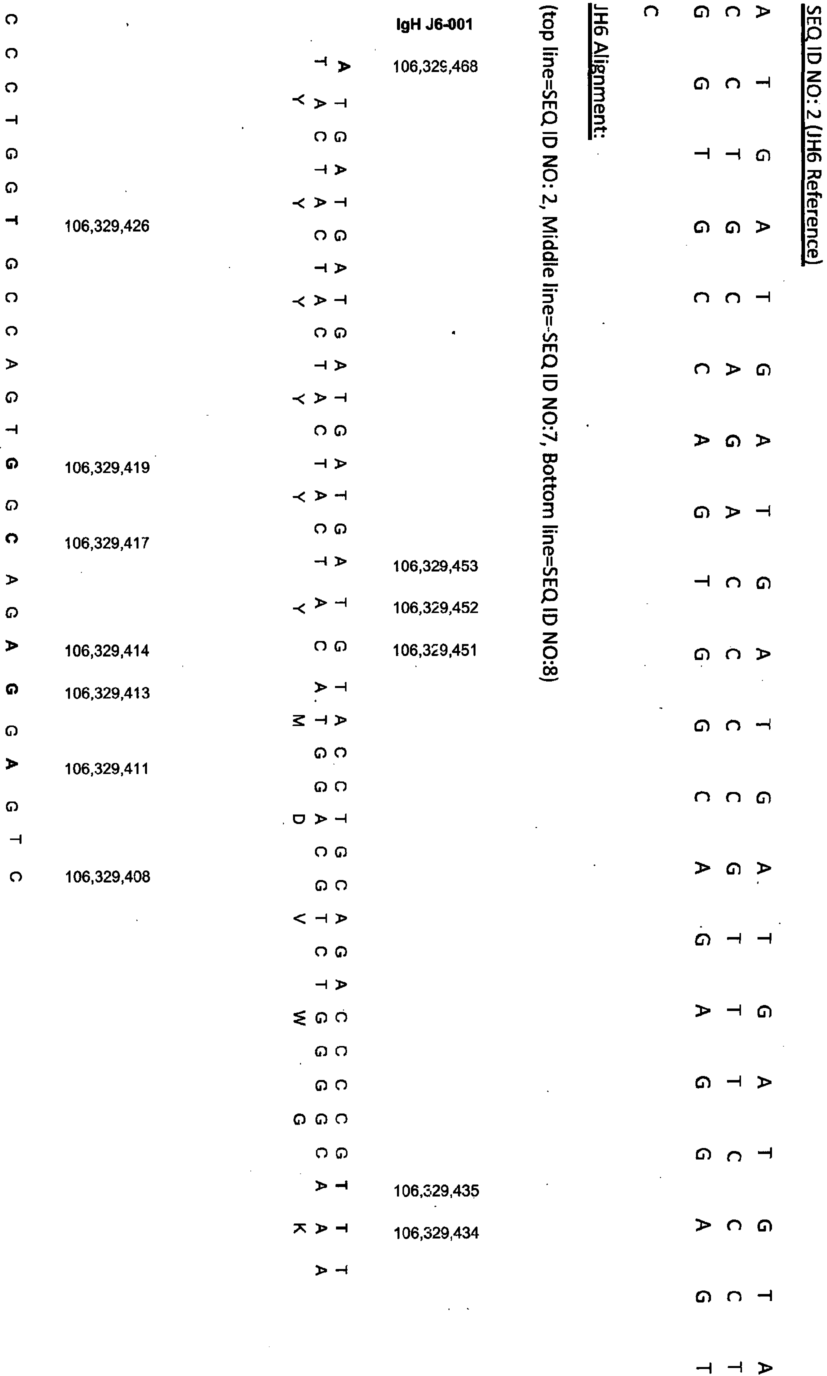

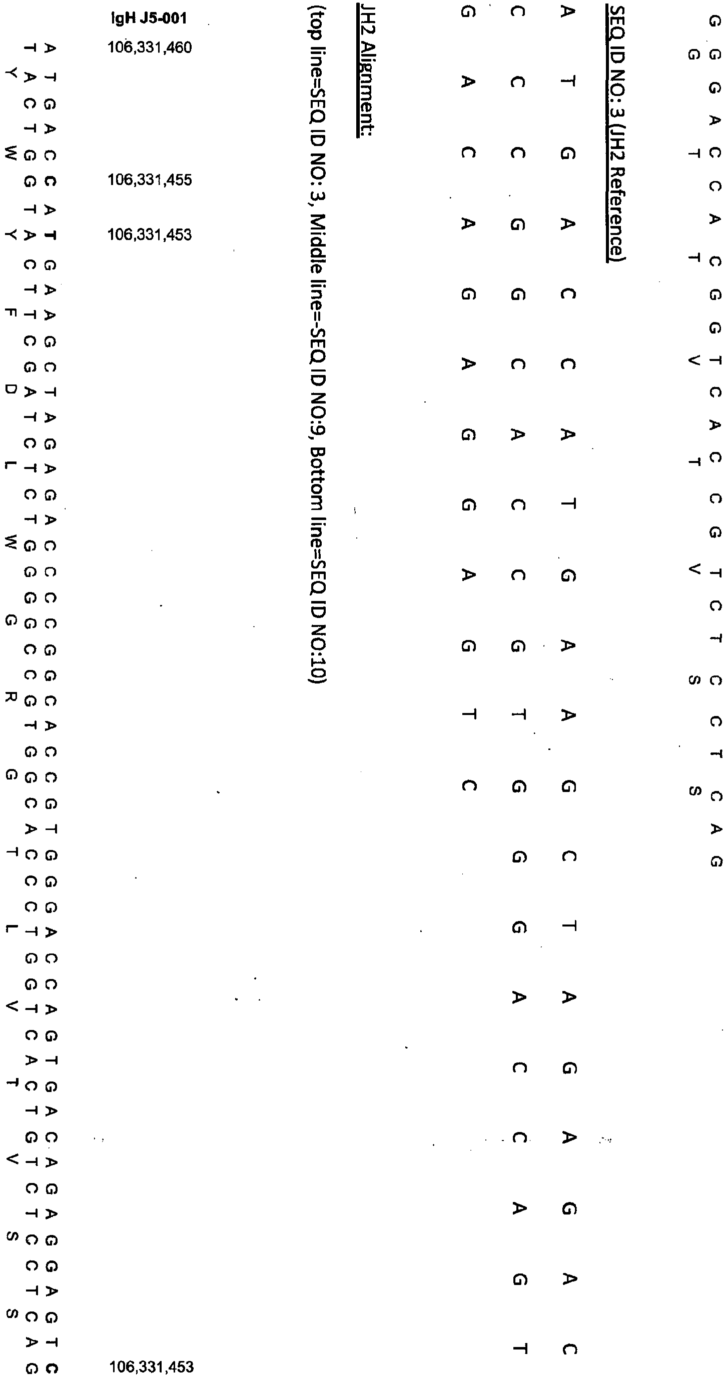

**TABLES**

| In the tables, the notation is illustrated by the following example | | | |
|---|---|---|---|
| IGLV1-40 | V140*02 | X53936 | g9>c\|c10>g,L4>V\| |

Polymorphic variant IGV lambda V1-40*02 has Genbank Accession No. X53936 and when compared to the *01 variant, the V1-40*02 variant has mutations at positions 9, 10 and 4. For example, at position 9, a "C" appears instead of a "G". that is present in the *01 variant. The "|" is simply a notation separator, and does not indicate any mutation. For example the "g282 I" notation indicates no change (ie, position 282 is a g). "del#" means that the residue at that position is absent.

**Table 1: Human IgH V Polymorphic Variants**

| All variant sequences appearing in Genbank under the listed Accession Numbers are incorporated herein in their entirety by reference as though explicitly recited herein and for possible inclusion in any of the claims herein. | | | | |
|---|---|---|---|---|
| **subgroup** | **IGHV** | **IGHV** | **Accession number** | **Description of mutations** |
| 1 | IGHV1-2 | IGHV1-2*01 | X07448 | SEQ ID NO: 11 |
| | | IGHV1-2*02 | X62106 | \|c163>t,R55>W\| \|g233>t,t234>g,S78>M\| t299>c,V100>A\| |
| | | IGHV1-2*03 | X92208 | |
| | | IGHV1-2*04 | Z12310 | |
| | IGHV1-3 | IGHV1-3*01 | X62109 | SEQ ID NO: 12 c6 \|t12 \|t167,I56 \|a208,K70 \|a291 \|c296,T99 \| |
| | | IGHV1-3*02 | X62107 | c6>t \|t12>g \| t167>g,I56>S \|a208>g,K70>E \|a291>g\| c296>t,T99>M\| |
| | IGHV1-8 | IGHV1-8*01 | M99637 | SEQ ID NO: 13 |
| | IGHV1-18 | IGHV1-18*01 | M99641 | g282 \| |
| | | IGHV1-18*02 | X60503 | g282>a\| |
| | IGHV1-24 | IGHV1-24*01 | M99642 | SEQ ID NO: 14 |
| | IGHV1-45 | IGHV1-45*01 | X92209 | SEQ ID NO: 15 g139 ,G47 \|t237 \| |
| | | IGHV1-45*02 | AB019438 | \|t237>c\| |
| | | IGHV1-45*03 | Z17391 | g139>a,G47>R\|t237>c\| |
| | IGHV1-46 | IGHV1-46*01 | X92343 | SEQ ID NO: 16 c92 ,T31 \|g315 \| |
| | | IGHV1-46*02 | J00240 | c92>a,T31>N\| |
| | | IGHV1-46*03 | L06612 | \|g315>t\| |
| | IGHV1-58 | IGHV1-58*01 | M29809 | SEQ ID NO: 17 g115 ,V39 \| |
| | | IGHV1-58*02 | AB019438 | g115>a,V39>M\| |
| | IGHV1-69 | IGHV1-69*01 | L22582 | SEQ ID NO:18 |
| | | IGHV1-69*02 | Z27506 | |
| | | IGHV1-69*03 | X92340 | \|g291>t,E97>D\| |
| | | IGHV1-69*04 | M83132 | |
| | | IGHV1-69*05 | X67905 | g6>c\| \|g238>a,A80>T\| |
| | | IGHV1-69*06 | L22583 | \|g244>a,E82>K\| |
| | | IGHV1-69*07 | Z29978 | \|g163>a,G55>R\| |
| | | IGHV1-69*08 | Z14309 | |
| | | IGHV1-69*09 | Z14307 | |
| | | IGHV1-69*10 | Z14300 | |
| | | IGHV1-69*11 | Z14296 | g6>c\| \|g163>a,G55>R\|t178>c,F60>L\| |
| | | IGHV1-69*12 | Z14301 | g6>c |
| | | IGHV1-69*13 | Z14214(st) | g6>c\| \|g54>a\| |
| | IGHV1-c | IGHV1-c*01 | Z18904 | SEQ ID NO: 19 |
| | IGHV1-f | IGHV1-f*01 | Z12305 | SEQ ID NO: 20 c201 |
| | | IGHV1-f*02 | Z29977 | c201>t\| |
| 2 | IGHV2-5 | IGHV2-5*01 | X62111 | SEQ ID NO: 21 |
| | | IGHV2-5*02 | Z14072 | \|a175>g,N59>D\| |
| | | IGHV2-5*03 | X93619 | \|a175>g,N59>D\| \|c234>t\| |
| | | IGHV2-5*04 | L21963 | \|c299>g,A100>G\|c314>t,A105>V\|a317>g,c318>g,H106>R\| |
| | | IGHV2-5*05 | L21964 | \|a175>g,N59>D\|a202>g,S68>G\| |
| | | IGHV2-5*06 | L21966 | \|g39>a\| \|a175>g,N59>D\|a202>g,S68>G\| |
| | | IGHV2-5*07 | L21968 | \|c299>g,A100>G \|c314>t,A105>V\| |
| | | IGHV2-5*08 | L21971 | |
| | | IGHV2-5*09 | L21972 | |
| | | IGHV2-5*10 | X69690 | |
| | IGHV2-26 | IGHV2-26*01 | M99648 | SEQ ID NO: 22 |
| | IGHV2-70 | IGHV2-70*01 | L21969 | SEQ ID NO: 23 |
| | | | | |
| | | IGHV2-70*02 | X92241 | \|a297>g\| \|a301>g,c302>t,T101>V\| |
| | | IGHV2-70*03 | X92238 | |
| | | IGHV2-70*04 | Z12330 | |
| | | IGHV2-70*05 | Z27502 | |
| | | IGHV2-70*06 | X92239 | |
| | | IGHV2-70*07 | X92243 | |
| | | IGHV2-70*08 | X92245 | |
| | | IGHV2-70*09 | L21962 | |
| | | IGHV2-70*10 | L21965 | |
| | | IGHV2-70*11 | L21967 | |
| | | IGHV2-70*12 | L21970 | |
| | | IGHV2-70*13 | AB019437 | \|c309>t\| |
| 3 | IGHV3-7 | IGHV3-7*01 | M99649 | SEQ ID NO: 24 |
| | | IGHV3-7*02 | X92288 | g144>a\| |
| | IGHV3-9 | IGHV3-9*01 | M99651 | SEQ ID NO: 25 |
| | IGH3-11 | IGHV3-11*01 | M99652 | SEQ ID NO: 26 |
| | | | | |
| | | IGHV3-11*02 | M15496 | |
| | | IGHV3-11*03 | X92287 | |
| | IGHV3-13 | IGHV3-13*01 | X92217 | SEQ ID NO: 27 |
| | | IGHV3-13*02 | M99653 | |
| | | IGHV3-13*03 | U29582 | \|c77>g,S26>C\| \|g224>a,R75>Q\| |
| | IGHV3-15 | IGHV3-15*01 | X92216 | SEQ ID NO: 28 |
| | | IGHV3-15*02 | M99654 | \|g32>c,G11>A\| |
| | | IGHV3-15*03 | M99408 | |
| | | IGHV3-15*04 | M99402 | \|a169>g,K57>E\| |
| | | IGHV3-15*05 | M99403 | \|c279>t\| |
| | | IGHV3-15*06 | M99404 | \|t159>c\| \|g196>a,D66>N\| |
| | | IGHV3-15*07 | M99406 | \|a81>t\| \|g119>a,S40>N\|t159>c\| |
| | | IGHV3-15*08 | M99400 | |
| | IGHV3-16 | IGHV3-16*01 | M99655 | SEQ ID NO: 29 |
| | | | | a6 \|a9 \| |
| | | IGHV3-16*02 | AB019440 | a6>g\|a9>g\| |
| | IGHV3-19 | IGHV3-19*01 | M99656 | SEQ ID NO: 30 |
| | IGHV3-20 | IGHV3-20*01 | M99657 | SEQ ID NO: 31 |
| | IGHV3-21 | IGHV3-21*01 | AB019439 | SEQ ID NO: 32 |
| | | | | g9 \| |
| | | IGHV3-21*02 | M99658 | g9>a\| |
| | IGHV3-23 | IGHV3-23*01 | M99660 | SEQ ID NO: 33 |
| | | | | |
| | | IGHV3-23*02 | M35415 | \|c203>g,A68>G\|c237>a\| |
| | | IGHV3-23*03 | U29481 | |
| | | IGHV3-23*04 | AJ879486 | t13>g\| |
| | | IGHV3-23*05 | AY757302 | d154>t, g155>a, S>Y\|g157>a, t159>a, G>S\|g166>a, G>5\| |
| | IGHV3-30 | IGHV3-30*01 | M83134 | SEQ ID NO: 34 |
| | | IGHV3-30*02 | L26401 | |
| | | IGHV3-30*03 | M99663 | |
| | | IGHV3-30*04 | L06615 | \|a150>g\| |
| | | IGHV3-30*05 | M77323 | |
| | | IGHV3-30*06 | L06617 | \|c75>g\| \|c101>c,t102>c,A34>G\| |
| | | IGHV3-30*07 | L06614 | \|t288>c\| |
| | | IGHV3-30*08 | M62737 | g18>c,E6>D\| \|g80>c,G27>A\| |
| | | IGHV3-30*09 | M77300 | \|a150>g\| \|a229>g,T77>A\| |
| | | IGHV3-30*10 | M77326 | \| g202>a,A68>T\| |
| | | IGHV3-30*11 | M77331 | \|c75>g\| |
| | | IGHV3-30*12 | M77338 | |
| | | IGHV3- 30*13 | M77339 | |
| | | IGHV3-30*14 | M77324 | \|a150>g\| \|g267>t\| |
| | | IGHV3-30*15 | M77327 | \|a275>g,N92>S\| |
| | | IGHV3-30*16 | M77328 | \|t135>c\| |
| | | IGHV3-30*17 | M77329 | \|a138>g\| |
| | | IGHV3-30*18 | X92214 | |
| | | IGHV3-30*19 | L06616 | \|c75>g\| \|c101>g,t102>g,A34>G\| \|a150>g\| |
| | IGHV3-30-3 | IGHV3-30-3*01 | X92283 | SEQ ID NO: 35 |
| | | | | c75 \|gA17 ,R106 \| |
| | | IGHV3-30-3*02 | M77302 | c75>g\|g317>a,R106>K\| |
| | IGHV3-33 | IGHV3-33*01 | AB019439 | SEQ ID NO: 36 |
| | | | | |
| | | IGHV3-33*02 | M99665 | g6>a\| \|t212>c,V71>A\| \|a251>c,K84>T\|a263>t,Y88>F\| |
| | | IGHV3-33*03 | M77305 | \|t246>c\| \|g317>a,R106>K\| |
| | | IGHV3-33*04 | M77335 | \|g150>a\| \|t177>c\| |
| | | IGHV3-33*05 | M77334 | \|g170>c,g171>a,W57>S\| |
| | IGHV3-35 | IGHV3-35*01 | M99666 | SEQ ID NO: 37 |
| | IGHV3-38 | IGHV3-38*01 | M99669 | SEQ ID NO: 38 |
| | | | | c302 ,A101 \| |
| | | IGHV3-38*02 | AB019439 | c302>t,A101>V \| |
| | IGHV3-43 | IGHV3-43*01 | M99672 | SEQ ID NO: 39 |
| | | | | t32 ,V11 \|a100 ,T34 \|g138 \|t172 ,W58 \| |
| | | IGHV3-43*02 | Z18901 | t32>g,V11>G\|a100>g,T34>A\|g138>a\|t172>g,W58>G\| |
| | IGHV3-47 | IGHV3-47*01 | Z18900 | SEQ ID NO: 40 |
| | | | | c58 \|c101 ,A34 \|t149 ,L50 \|t267 ,L89 \|t270 ,H90 \|t310 \| |
| | | IGHV3-47*02 | AB019438 | c58>a\|c101>t,A34>V\|t149>c,L50>P\| \|t270>a,H90>Q\| |
| | | IGHV3-47*03 | M99674 | |
| | IGHV3-48 | IGHV3-48*01 | M99675 | SEQ ID NO: 41 |
| | | | | |
| | | IGHV3-48*02 | AB019438 | \|c287>a,A96>D\| |
| | | IGHV3-48*03 | U03893 | |
| | IGHV3-49 | IGHV3-49*01 | M99676 | SEQ ID NO: 42 |
| | | | | |
| | | IGHV3-49*02 | M99401 | |
| | | IGHV3-49*03 | AB019438 | \|a202>g,T68>A\|g245>a,G82>D\| |
| | | IGHV3-49*04 | AM940220 | \|t124>g,F42>V\|a202>g,T68>A\|g245>a,G82>D\| |
| | | IGHV3-49*05 | AM940221 | \|a202>g,T68>A\|g245>a,G82>D\| |
| | IGHV3-53 | IGHV3-53*01 | M99679 | t19 ,S7 \|g133 ,A45 \|c210 \|g315 \|a318 \| |
| | | IGHV3-53*02 | Z12342 | SEQ ID NO: 43 |
| | | | | t19>a,S7>T\| |
| | | IGHV3-53*03 | J03617 | \|g133>c,A45>P\|c210>t\|g315>t\|a318>g\| |
| | IGHV3-64 | IGHV3-64*01 | M99682 | SEQ ID NO: 44 |
| | | | | |
| | | IGHV3-64*02 | AB019437 | \|g26>a,G9>E\| \|a205>g,N69>D\| |
| | | IGHV3-64*03 | M77298 | |
| | | IGHV3-64*04 | M77299 | |
| | | IGHV3-64*05 | M77301 | |
| | IGHV3-66 | IGHV3-66*01 | X92218 | SEQ ID NO: 45 |
| | | | | g24 \|g37 ,V13 \|a81 \|g175 ,G59 \|a223 \|c288 \|g319 \| |
| | | IGHV3-66*02 | Z27504 | \|a223>c\|c288>t\| |
| | | IGHV3-66*03 | AB019437 | g24>a\|g37>a,V13>I\|a81>g\|g175>t,G59>C\|a223>c\|c288>t\| |
| | | IGHV3-66*04 | X70208 | \|g319>c\| |
| | IGHV3-72 | IGHV3-72*01 | X92206 | SEQ ID NO: 46 |
| | | | | t186 \| |
| | | IGHV3-72*02 | Z29979 | t186>c\| |
| | IGHV3-73 | IGHV3-73*01 | X70197 | SEQ ID NO: 47 |
| | | | | t21 \| |
| | | IGHV3-73*02 | AB019437 | t21>c\| |
| | IGHV3-74 | IGHV3-74*01 | L33851 | SEQ ID NO: 48 |
| | | | | c21 \|g197 ,c198 ,S66 \| |
| | | IGHV3-74*02 | Z17392 | c21>t\| |
| | | IGHV3-74*03 | J00239 | \|g197>c,c198>g,S66>T\| |
| | IGHV3-d | IGHV3-d*01 | Z18898 | SEQ ID NO: 49 |
| | IGHV3-h | IGHV3-h*01 | AJ879484 | SEQ ID NO: 50 |
| | | IGHV3-h*02 | AJ879485 | \|g303>t\| |
| 4 | IGHV4-4 | IGHV4-4*01 | X05713 | SEQ ID NO: 51 |
| | | IGHV4-4*02 | X92232 | |
| | | IGHV4-4*03 | X92252 | \|g308>a,C103>Y\| |
| | | IGHV4-4*04 | X92253 | |
| | | IGHV4-4*05 | X92254 | |
| | | IGHV4-4*06 | Z75355 | |
| | | IGHV4-4*07 | X62112 | |
| | IGHV4-28 | IGHV4-28*01 | X05714 | SEQ ID NO: 52 g48 \|g49 ,c51 ,D17 \|c185 ,T62 \|g297 \|c299 ,A100 \|a319 \| |
| | | IGHV4-28*02 | M83133 | g48>a\|g49>c,c51>g,D17>Q\|c185>t,T62>I\| |
| | | IGHV4-28*03 | X92233 | \|a319>g\| |
| | | IGHV4-28*04 | X56358 | g297>c\|c299>g,A100>G\| |
| | | IGHV4-28*05 | X92260 | \|c185>t,T62>I\| |
| | IGHV4-30-2 | IGHV4-30-2*01 | L10089 | SEQ ID NO: 53 |
| | | | | |
| | | IGHV4-30-2*02 | M95122 | \|C288>t\| \|c315>g\| |
| | | IGHV4-30-2*03 | X92229 | |
| | | IGHV4-30-2*04 | Z75351 | \|g245>c,R82>T\| \|g291>a\| \|c315>g\| |
| | IGHV4-30-4 | IGHV4-30-4*01 | Z14238 | SEQ ID NO: 54 |
| | | | | |
| | | IGHV4-30-4*02 | Z14239 | \|a48>g\|c49>g,g51>c,Q17>D\| \|c288>a\| |
| | | IGHV4-30-4*03 | X92274 | \|a291>g\| |
| | | IGHV4-30-4*04 | X92275 | g18>c,E6>D\| \|a166>t,156>F\| |
| | | IGHV4-30-4*05 | Z75353 | \|t228>c\| |
| | | IGHV4-30-4*06 | Z75360 | \|c134>a,P45>H\| |
| | IGHV4-31 | IGHV4-31*01 | L10098 | SEQ ID NO: 55 |
| | | | | |
| | | IGHV4-31*02 | M99683 | \|c69>t\| \|t224>g,L75>R\| |
| | | IGHV4-31*03 | Z14237 | \|t224>g,L75>R\| |
| | | IGHV4-31*04 | M95120 | a8>g,Q3>R\| \|t224>g,L75>R\| |
| | | IGHV4-31*05 | M95121 | |
| | | IGHV4-31*06 | X92270 | \|g100>a,G34>S\| \|t224>g,L75>R\| |
| | | IGHV4-31*07 | X92271 | \|c84>a\| \|t224>g,L75>R\| |
| | | IGHV4-31*08 | X92272 | |
| | | IGHV4-31*09 | X92273 | \|t224>g,L75>R\| \| ,c245>a,T82>K\|t249>c\|t285>c\| |
| | | IGHV4-31*10 | Z14235 | |
| | IGHV4-34 | IGHV4-34*01 | AB019439 | SEQ ID NO: 56 |
| | | | | |
| | | IGHV4-34*02 | M99684 | \|g15>a\| |
| | | IGHV4-34*03 | X92255 | \|t300>c\| |
| | | IGHV4-34*04 | X92236 | |
| | | IGHV4-34*05 | X92237 | |
| | | IGHV4-34*06 | X92256 | \|a274>g,S92>G\| \|t300>c\| |
| | | IGHV4-34*07 | X92258 | |
| | | IGHV4-34*08 | M95113 | \|t85>a,S29>T\| |
| | | IGHV4-34*09 | Z14241 | |
| | | IGHV4-34*10 | Z14242 | |
| | | IGHV4-34*11 | X05716 | |
| | | IGHV4-34*12 | X56591 | \| a170>t ,N57>I\| |
| | | IGHV4-34*13 | Z75356 | \|g207>c\| |
| | IGHV4-39 | IGHV4-39*01 | AB019439 | SEQ ID NO: 57 a2 ,Q1 \|t56 ,L19 \|c237 \|g258 ,Q86 \|t262 ,S88 \|a291 \|t300 \|c319 \| |
| | | IGHV4-39*02 | X05715 | \|g258>c,Q86>H\| \|c319>g\| |
| | | IGHV4-39*03 | X92259 | \|t300>c\| |
| | | IGHV4-39*04 | X92297 | \|a291>g\| |
| | | IGHV4-39*05 | M95116 | \|t56>c,L19>P\| |
| | | IGHV4-39*06 | Z14236 | a2>g,Q1>R\| \|c237>a\| \|t262>c,S88>P\|a291>g\|t300>c\| |
| | | IGHV4-39*07 | AM940222 | \|c237>a\| \|a291>g\|t300>c\| c319>g |
| | IGHV4-55 | IGHV4-55*01 | M99685 | SEQ ID NO: 58 |
| | | | | |
| | | IGHV4-55*02 | X92223 | \|c237>a\| |
| | | IGHV4-55*03 | X92263 | \|c237>a\| \|a263>c,Y88>S\| |
| | | IGHV4-55*04 | X92265 | c44>t,P15>L\|c237>a\| |
| | | IGHV4-55*05 | X92266 | c44>t,P15>L\| |
| | | IGHV4-55*06 | X92267 | \|c255>g,N85>K\| \|c288>t\| |
| | | IGHV4-55*07 | X92268 | \|a251>g,K84>R\| \|a263>c,Y88>S\| \|g291>a\| |
| | | IGHV4-55*08 | X92234 | \|c237>a\| \|t319>g\| |
| | | IGHV4-55*09 | X92235 | \|a263>c,Y88>S\| \|c290>t,A97>V\| \|t319>a\| |
| | IGHV4-59 | IGHV4-59*01 | AB019438 | SEQ ID NO: 59 |
| | | | | |
| | | IGHV4-59*02 | M29812 | \|a88>g,I30>V\| |
| | | IGHV4-59*03 | M95114 | \|g258>a\| |
| | | IGHV4-59*04 | M95117 | |
| | | IGHV4-59*05 | M95118 | |
| | | IGHV4-59*06 | M95119 | |
| | | IGHV4-59*07 | X56360 | \|g51>c,E17>D\| |
| | | IGHV4-59*08 | X87091 | \|t288>c\| g291>a\| |
| | | IGHV4-59*09 | Z75359 | \|a320>g\| |
| | | IGHV4-59*10 | Z14243 | |
| | IGHV4-61 | IGHV4-61*01 | M29811 | SEQ ID NO: 60 |
| | | IGHV4-61*02 | L10097 | |
| | | IGHV4-61*03 | X92230 | \|g258>c,Q86>H\| |
| | | IGHV4-61*04 | X92250 | \|g162>a\| \|g289-g291>del(3nt)\| |
| | | IGHV4-61*05 | X56356 | |
| | | IGHV4-61*06 | Z75347 | \|t288>c\| \|g315>c\| |
| | | IGHV4-61*07 | Z75348 | \|g315>c\| |
| | | IGHV4-61*08 | AB019437 | \|a100>g,S34>G\| |
| | IGHV4-b | IGHV4-b*01 | Z12367 | SEQ ID NO: 61 |
| | | IGHV4-b*02 | X56365 | g70>a,A24>T\| |
| 5 | IGHV5-51 | IGHV5-51*01 | M99686 | SEQ ID NO: 62 |
| | | IGHV5-51*02 | M18806 | \|t116>c,I39>T\| \|c148>t\| |
| | | IGHV5-51*03 | X56368 | c45>g\| |
| | | IGHV5-51*04 | X56367 | c45>g\| \|t247>c,S83>P\| |
| | | IGHV5-51*05 | Z27449 | \|g139>a,G47>R\| |
| | IGHV5-a | IGHV5-a*01 | X92227 | SEQ ID NO: 63 t21 \|c148 \|c225 ,H75 \|c288 ,A96 \| |
| | | IGHV5-a*02 | X92279 | \|c148>t\| \|c288>del#,A96>del#\| |
| | | IGHV5-a*03 | X56375 | t21>c\| |
| | | IGHV5-a*04 | X56376 | \|c225>g,H75>Q\| |
| 6 | IGHV6-1 | IGHV6-1*01 | X92224/J04 097 | SEQ ID NO: 64 |
| | | IGHV6-1*02 | Z14223 | a27>g\| |
| 7 | IGHV7-4-1 | IGHV7-4-1*01 | L10057 | SEQ ID NO: 65 |
| | | IGHV7-4-1*02 | X62110 | t274>a,C92>S\| |
| | | IGHV7-4-1*03 | X92290 | t274>a,C92>S\|g278>c,c279>g,S93>T\| |
| | IGHV7-81 | IGHV7-81*01 | AB019437 | SEQ ID NO: 66 |

**Table 2: Human IgH D Polymorphic Variants**

| All variant sequences appearing in Genbank under the listed Accession Numbers are incorporated herein in their entirety by reference as though explicitly recited herein and for possible inclusion in any of the claims herein. | | | | |
|---|---|---|---|---|
| **IGHD subgro up** | **IGHD name** | **Variant name** | **Genbank Accession number** | **Sequence** |
| **1** | **IGHD1-1** | IGHD1-1*01 | X97051 | SEQ ID NO: 67 |
| | **IGHD1-7** | IGHD1-7*01 | X13972 | SEQ ID NO: 68 |
| | **IGHD1-14** | IGHD1-14*01 | X13972 | SEQ ID NO: 69 |
| | **IGHD1-20** | IGHD1-20*01 | X97051 | SEQ ID NO: 70 |
| | **IGHD1-26** | IGHD1-26*01 | X97051 | SEQ ID NO: 71 |
| 2 | **IGHD2-2** | IGHD2-2*01 | J00232 | SEQ ID NO: 72 AGGATATTGTAGTAGTACCAGCTGCTATGCC |
| | | IGHD2-2*02 | X97051 | G>A |
| | | IGHD2-2*03 | M35648 | A>T |
| | **IGHD2-8** | IGHD2-8*01 | X13972 | SEQ ID NO: 73 AGGATATTGTACTAATGGTGTATGCTATACC |
| | | IGHD2-8*02 | J00233 | AA>GG |
| | **IGHD2-15** | IGHD2-15*01 | J00234 | SEQ ID NO: 74 |
| | **IGHD2-21** | IGHD2-21*01 | J00235 | SEQ ID NO: 75 AGCATATTGTGGTGGTGATTGCTATTCC |
| | | IGHD2- 21*02 | X97051 | T>C |
| 3 | **IGHD3-3** | IGHD3-3*01 | X13972 | SEQ ID NO: 76 GTATTACGATTTTTGGAGTGGTTATTATACC |
| | | IGHD3-3*02 | X93618 | CG>GC |
| | **IGHD3-9** | IGHD3-9*01 | X13972 | SEQ ID NO: 77 |
| | **IGHD3-10** | IGHD3-10*01 | X13972 | SEQ ID NO: 78 GTATTACTATGGTTCGGGGAGTTATTATAAC |
| | | IGHD3-10*02 | X93615 | G>[OMITTED] |
| | **IGHD3-16** | IGHD3-16*01 | X93614 | SEQ ID NO: 79 GTATTATGATTACGTTTGGGGGAGTTATGCTTATACC |
| | | IGHD3-16*02 | X97051 | GC>CG |
| | **IGHD3-22** | IGHD3-22*01 | X93616 | SEQ ID NO: 80 |
| 4 | **IGHD4-4** | IGHD4-4*01 | X13972 | SEQ ID NO: 81 |
| | **IGHD4-11** | D4-11*01 | X13972 | SEQ ID NO: 82 |
| | **IGHD4-17** | IGHD4-17*01 | X97051 | SEQ ID NO: 83 |
| | **IGHD4-23** | IGHD4-23*01 | X97051 | SEQ ID NO: 84 |
| 5 | **IGHD5-5** | IGHD5-5*01 | X13972 | SEQ ID NO: 85 |
| | **IGHD5-12** | IGHD5-12*01 | X13972 | SEQ ID NO: 86 |
| | **IGHD5-**18 | IGHD5-18*01 | X97051</A<< B> | SEQ ID NO: 87 |
| | **IGHD5-24** | IGHD5-24*01 | X97051 | SEQ ID NO: 88 |
| 6 | **IGHD6-**6 | IGHD6-6*01 | X13972 | SEQ ID NO: 89 |
| | **IGHD6-13** | IGHD6-13*01 | X13972 | SEQ ID NO: 90 |
| | **IGHD6-19** | IGHD6-19*01 | X97051 | SEQ ID NO: 91 |
| | **IGHD6-25** | IGHD6-25*01 | X97051 | SEQ ID NO: 92 |
| 7 | **IGHD7-27** | IGHD7-27*01 | J00256 | SEQ ID NO: 93 |

Underlined nucleotides in the *01 variant are positions that are different in other variants, eg, GC>CG for IGHD3-16*02 indicates that GC in the *01 variant is instead CG in the *02 variant. All other positions are identical to between the *01 and other variant. Similar notation is used in other tables below to denote changes between variants of gene segments.

**Table 3: Human IgH J Polymorphic Variants**

| All variant sequences appearing in Genbank under the listed Accession Numbers are incorporated herein in their entirety by reference as though explicitly recited herein and for possible inclusion in any of the claims herein. | | | |
|---|---|---|---|
| **IGHV name** | **Variant name** | **Genbank Accession number** | **Sequence** |
| **IGHJ1** | J1*01 | J00256 | SEQ ID NO: 94 |
| **IGHJ2** | J2*01 | J00256 | SEQ ID NO: 95 |
| **IGHJ3** | | | SEQ ID NO: 96 |
| | J3*01 | J00256 | |
| | J3*02 | X86355 | G>A |
| **IGHJ4** | | | SEQ ID NO: 97 |
| | J4*01 | J00256 | |
| | | | AA>AG |
| | J4*02 | X86355 | |
| | | | AC>GC |
| | | | GA>GG |
| | J4*03 | M25625 | |
| **IGHJ5** | | | SEQ ID NO: 98 |
| | J5*01 | J00256 | |
| | | | T>C |
| | | | A>G |
| | J5*02 | X86355 | |
| **IGHJ6** | | | SEQ 10 NO: 99 |
| | J6*01 | J00256 | |
| | | | GG>GC |
| | J6*02 | X86355 | |
| | | | |
| | J6*03 | X86356 | GGT>TAC |
| | | | GG>GC |
| | | | CA>AA |
| | | | GG>GC |
| | | | CA>AA |
| | J6*04 | AJ8794887 | |

**Table 4: Human Ig Vk Polymorohic Variants**

| All variant sequences appearing in Genbank under the listed Accession Numbers are incorporated herein in their entirety by reference as though explicitly recited herein and for possible inclusion in any of the claims herein. | | | | |
|---|---|---|---|---|
| **IGKV subgroup** | **IGKV name** | **Variant name** | **Accession number** | **Sequence** |
| 1 | IGKV1-5 | IGKV1-5*01 | Z00001 | SEQ ID NO: 100 |
| | | | | |
| | | | | |
| | | IGKV1-5*02 | M23851 | AC>AT |
| | | IGKV1-5*03 | X72813 | GAT>AAG |
| | | | | GCC>GCG |
| | | | | TCC>TCT |
| | | | | TTG>TTA |
| | IGKV1-6 | IGKV1-6*01 | M64858 | SEQ ID NO: 7.01 |
| | IGKV1-8 | IGKV1-8*01 | Z00014 | SEQ ID NO: 102 |
| | IGKV1D-8 | IGKV1D-8*01 | Z00008 | SEQ ID NO: 103 |
| | IGKV1-9 | IGKV1-9*01 | Z00013 | SEQ ID NO: 104 |
| | IGKV1-12 | IGKV1-12*01 | V01577 | SEQ ID NO: 105 |
| | IGKV1D-12 | IGKV1D-12*01 | X17263 | TCC>TCT |
| | | | | ACC>ACT |
| | IGKV1-12/IGKV1D-12 (1) | IGKV1-12*02/1D-12*02 | V01576 | CC>TC |
| | IGKV1-13 | IGKV1-13*01 | Z00010 | SEQ ID NO: 106 |
| | | | | |
| | | | | |
| | | IGKV1-13*02 | Z00006 | TGA>TGG AAT>AGT |
| | IGKV1D-13 | IGKV1D-13*01 | X17262 | TGA>TGG |
| | IGKV1-16 | IGKV1-16*01 | J00248 | SEQ ID NO: 107 |
| | | | | |
| | | IGKV1-16*02 | FM164406 | AGG>AAG |
| | IGKV1D-16 | IGKV1D-16*01 | K01323 | SEQ ID NO: 108 |
| | | | | |
| | | | | |
| | | IGKV1D-16*02 | V00558 | AGT>AGG |
| | IGKV1-17 | IGKV1-17*01 | X72808 | SEQ ID NO: 109 |
| | | | | |
| | | IGKV1-17*02 | D88255 | AGC> AAC |
| | IGKV1D-17 | IGKV1D-17*01 | X63392 | SEQ ID NO: 110 |
| | | | | |
| | | IGKV1D-17*02 | FM164407 | AAC>GAC CAC>CGC |
| | IGKV1-27 | IGKV1-27*01 | X63398 | SEQ ID NO: 111 |
| | IGKV1-33 | IGKV1-33*01 | M64856 | SEQ ID NO: 112 |
| | IGKV1D-33 | IGKV1D-33*01 | M64855 | SEQ ID NO: 113 |
| | IGKV1-37 | IGKV1-37*01 | X59316 | SEQ ID NO: 114 |
| | IGKV1D-37 | IGKV1D-37*01 | X71893 | SEQ ID NO: 115 |
| | IGKV1-39 | IGKV1-39*01 | X59315 | SEQ ID NO:116 |
| | | | | |
| | | IGKV1-39*02 | X59318 | TCC>TTC |
| | | | | TAC>TAT |
| | | | | CAA CAG AGT> CAG TGT GGT |
| | | | | ACC>ACA |
| | IGKV1D-39 | IGKV1D-39*01 | X59312 | SEQ ID NO: 117 |
| | IGKV1D-42 | IGKV1D-42*01 | X72816 | SEQ ID NO: 118 |
| | IGKV1D-43 | IGKV1D-43*01 | X72817 | SEQ ID NO: 119 |
| 2 | IGKV2-24 | IGKV2-24*01 | X12684 | SEQ ID NO: 120 |
| | IGKV2D-24 | IGKV2D-24*01 | X63401 | SEQ ID NO: 121 |
| | IGKV2-28 | IGKV2-28*01 | X63397 | SEQ ID NO: 122 |
| | IGKV2D-28 | IGKV2D-28*01 | X12691 | SEQ ID NO: 123 |
| | IGKV2-29 | IGKV2-29*01 | X63396 | SEQ ID NO: 124 |
| | | | | |
| | | | | |
| | | IGKV2-29*02 | U41645 | CTG> CTA |
| | | | | TGA> TGC |
| | | IGKV2-29*03 | AJ783437 | TGA> TGC |
| | IGKV2D-29 | IGKV2D-29*01 | M31952 | SEQ ID NO: 125 |
| | | | | |
| | | IGKV2D-29*02 | U41644 | CCT>TCT |
| | IGKV2-30 | IGKV2-30*01 | X63403 | SEQ ID NO: 126 |
| | | | | |
| | | | | |
| | | IGKV2-30*02 | FM164408 | TAC>CAC |
| | IGKV2D-30 | IGKV2D-30*01 | X63402 | SEQ ID NO: 127 |
| | IGKV2-40 | IGKV2-40*01 | X59314 | SEQ ID NO: 128 |
| | | IGKV2-40*02 | X59317 | GAC TGG>GAT TGT |
| | | | | GGC>GAC |
| | IGKV2D-40 | IGKV2D-40*01 | X59311 | SEQ ID NO: 129 |
| 3 | IGKV3-7 | IGKV3-7*01 | X02725 | SEQ ID NO: 130 |
| | | | | |
| | | IGKV3-7*02 | X72812 | ACC>TCC |
| | | | | TAT>TAC |
| | | | | GGC>GGG |
| | | | | GCG>GCT |
| | | | | AGC>GGC |
| | | | | ACA>AGA |
| | | | | CAT>TAT |
| | | IGKV3-7*03 | K02769 | SEQ ID NO: 131 |
| | | | | |
| | | | | |
| | | IGKV3-7*04 | FM164409 | CAT>TAT |
| | IGKV3D-7 | IGKV3D-7*01 | X72820 | SEQ ID NO: 132 |
| | IGKV3-11 | IGKV3-11*01 | X01668 | SEQ ID NO: 133 |
| | | | | |
| | | IGKV3-11*02 | K02768 | ACA>AGA |
| | IGKV3D-11 | IGKV3D-11*01 | X17264 | SEQ ID NO:134 |
| | IGKV3-15 | IGKV3-15*01 | M23090 | SEQ ID NO: 135 |
| | IGKV3D-15 | IGKV3D-15*01 | X72815 | SEQ ID NO:136 |
| | | IGKV3D-15*02 | M23091 | ACG>ATG |
| | | | | TGG>TGA |
| | IGKV3-20 | IGKV3-20*01 | X12686 | SEQ ID NO:137 |
| | | | | |
| | | IGKV3-20*01, Tou-kv325 | X56593 | ACC>CCC |
| | | | | CTG>TGT |
| | | | | TCT> CTT |
| | | | | TTG> TGT |
| | | | | TCT> CTC |
| | | | | |
| | | IGKV3-20*02 | L37729 | ACG>ACA |
| | | | | GGC>GCC |
| | | | | GAC>GCA |
| | IGKV3D-20 | IGKV3D-20*01 | X12687 | SEQ ID NO: 138 |
| 4 | IGKV4-1 | IGKV4-1*01 | Z00023 | SEQ ID NO: 139 |
| 5 | IGKV5 2 | IGKV5-2*01 | X02485 | SEQ ID NO: 140 |
| 6 | IGKV6-21 | IGKV6-21*01 | X63399 | SEQ ID NO:141 |
| | IGKV6D-21 | IGKV6D-21*01 | X12683 | SEQ ID NO: 142 |
| | IGKV6D-41 | IGKV6D-41*01 | M27751 (3) | SEQ ID NO: 143 |

**Table 5: Human le Vλ Polymorphic Variants**

| All variant sequences appearing in Genbank under the listed Accession Numbers are incorporated herein in their entirety by reference as though explicitly recited herein and for possible inclusion in any of the claims herein. | | | | |
|---|---|---|---|---|
| | | | | |

| **IGLV subgroup** | **IGLV Gene name** | **IGLV variant name** | **Accession number** | **Sequences & Description of mutations** |
|---|---|---|---|---|
| 1 | IGLV1-36 | V1-36^{*}01 | Z73653 | SEQ ID NO: 144 |
| | IGLV1-40 | V1-40*01 | M94116 | SEQ ID NO: 145 |
| | | V1-40*02 | X53936 | g9>c\|c10>g,L4>V\| |
| | | V1-40*03 | Z22192 | g9>c\|c10>g,L4>V\|a253>g,T85>A\| |
| | IGLV1-41 | V-41*01 | M94118 | SEQ ID NO: 146 |
| | | V-41*02 | D87010 | g295>t,E99>*\|c332>t,P111>L\| |
| | | | | |
| | IGLV1-44 | V1-44*01 | Z73654 | SEQ ID NO: 147 |
| | IGLV1-47 | V1-47*01 | Z73663 | SEQ ID NO: 148 |
| | | V1-47*02 | D87016 | g168>t,R56>S\| |
| | IGLV1-50 | V1-50*01 | M94112 | SEQ ID NO: 149 |
| | IGLV1-51 | V1-51*01 | Z73661 | SEQ ID NO: 150 |
| | | V-51*02 | M30446 | t162>c\|c168>a,D56>E\| |
| 2 | IGLV2-8 | V2-8*01 | X97462 | SEQ ID NO: 151 |
| | | V2-8*02 | L27695 | g37>a,G13>R\| |
| | | V2-8*03 | Y12418 | \|c230>t,S77>F\| |
| | IGLV2-11 | V2-11*01 | Z73657 | SEQ ID NO: 152 |
| | | V2-11*02 | Z22198 | t96>g\| |
| | | V2-11*03 | Y12415 | \|g132>a\| |
| | IGLV2-14 | V2-14*01 | Z73664 | SEQ ID NO: 153 |
| | | V2-14*02 | L27822 | |
| | | V2-14*03 | Y12412 | \|g132>a\|g168>t,E56>D\| |
| | | V2-14*04 | Y12413 | \|g168>t,E56>D\| |
| | IGLV2-18 | V2-18*01 | Z73642 | SEQ ID NO: 154 |
| | | | | |
| | | V2-18*02 | L27697 | \|t317>c,L106>S\| |
| | | V2-18*03 | L27694 | \|t272>c,I91>T\|t317>c,L106>S\| |
| | | V2-18*04 | L27692 | t227>c,F76>S\|t249>c\| \|t317>c,L106>S\| |
| | IGLV2-23 | V2-23*01 | X14616 | SEQ ID NO: 155 |
| | | V2-23*02 | Z73665 | g170>t,G57>V\|a339>c,L113>F\| |
| | | V2-23*03 | D86994 | \|a339>c,L113>F\| |
| | IGLV2-33 | V2-33*01 | Z73643 | SEQ ID NO: 156 |
| | | V2-33*02 | L27823 | a3>g\| |
| | | V2-33*03 | L27691 | \|t96>c\|a256>g,M86>V\| |
| 3 | IGLV3-1 | V3-1*01 | X57826 | SEQ ID NO: 157 |
| | IGLV3-9 | V3-9*01 | X97473 | a52 ,T18 \|a82 ,I28 \|a88 ,g89 ,S30 \|a95 ,N32 \|g173 ,S58 \| |
| | | V3-9*02 | X74288 | |
| | | V3-9*03 | X51754 | |
| | IGLV3-10 | V3-10*01 | X97464 | SEQ ID NO: 158 |
| | | V3-10*02 | L29166 | |
| | IGLV3-12 | V3-12*01 | Z73658 | SEQ ID NO: 159 |
| | | V3-12*02 | D86998 | a259>g,T87>A\| |
| | | | | |
| | IGLV3-16 | V3-16*01 | X97471 | SEQ ID NO: 160 |
| | IGLV3-19 | V3-19*01 | X56178 | SEQ ID NO: 161 |
| | IGLV3-21 | V3-21*01 | X71966 | SEQ ID NO: 162 |
| | | V3-21*02 | D87007 | a27>g\|a49>c,K17>Q\|a160>g,I54>V\|t166>g,Y56>D\|c324>t\| |
| | | V3-21*03 | M94115 | a27>g\| \|a160>g,I54>V\|t166>g,Y56>D\|c324>t\| |
| | IGLV3-22 | V3-22*01 | Z73666 | SEQ ID NO: 163 |
| | | V3-22*02 | X71967 | |
| | IGLV3-25 | V3-25*01 | X97474 | SEQ ID NO: 164 |
| | | V3-25*02 | D86994 | t14>c,g15>a,M5>T\| |
| | | V3-25*03 | L29165 | t14>c,g15>a,M5>T\|t294>c\| |
| | IGLV3-27 | V3-27*01 | D86994 | SEQ ID NO: 165 |
| | IGLV3-32 | V3-32*01 | Z73645 | SEQ ID NO: 166 |
| 4 | IGLV4-3 | V4-3*01 | X57828 | SEQ ID NO: 167 |
| | IGLV4-60 | V4-60*01 | Z73667 | SEQ ID NO: 168 |
| | | V4-60*02 | D87000 | a288>t,L96>F\| |
| | | V4-60*03 | AF073885 | t287>c,a288>t,L96>S\| |
| | IGLV4-69 | V4-69*01 | Z73648 | SEQ ID NO: 169 |
| | | | | |
| | | V4-69*02 | U03868 | c198>t\| |
| 5 | IGLV5-37 | V5-37*01 | Z73672 | SEQ ID NO: 170 |
| | IGLV5-39 | V5-39*01 | Z73668 | t81 ,g135>t\| |
| | | V5-39*02 | AF216776 | t81>c,g135>t\| |
| | IGLV5-45 | V5-45*01 | Z73670 | SEQ ID NO: 171 |
| | | V5-45*02 | Z73671 | g22>t,A8>S\|g75>a\| |
| | | V5-45*03 | D86999 | g22>t,A8>S\| |
| | IGLV5-48 | V5-48*01 | Z73649 | SEQ ID NO: 172 |
| | IGLV5-52 | V5-52*01 | Z73669 | SEQ ID NO: 173 |
| 6 | IGLV6-57 | V6-57*01 | Z73673 | SEQ ID NO: 174 |
| 7 | IGLV7-43 | V7-43*01 | X14614 | SEQ ID NO: 175 |
| | IGLV7-46 | V7-46*01 | Z73674 | SEQ ID NO: 176 |
| | | V7-46*02 | D86999 | \|c275>t,S92>L\| |
| | | V7-46*03 | Z22210 | c271>del#\|L91>del#\| |
| 8 | IGLV8-61 | V8-61*01 | Z73650 | SEQ ID NO: 177 |
| | | V8-61*02 | U03637 | \|c223>t,R75>C\| |
| | | V8-61*03 | AF266511 | g210>c\| |
| 9 | IGLV9-49 | V9-49*01 | Z73675 | SEQ ID NO: 178 |
| | | V9-49*02 | Z73675 | \|g291>a\| |
| | | V9-49*03 | U03869 | g153>a\| |
| 10 | IGLV10 -54 | V10-54*01 | Z73676 | SEQ ID NO: 179 |
| | | V10-54*02 | D86996 | \|a86>t,N29>l\| \|a228>c,L76>F\|g320>t,W107>L\| |
| | | V10-54*03 | S70116 | g33>c\| \|t125>c,g126>t,L42>P\|c127>g,Q43>E\| |
| 11 | IGLV11 -55 | V11-55*01 | D86996 | SEQ ID NO: 180 |
| | | | | |
| | | | | |
| | | | | |
| | | | | |

**Table 6: Human IgH Jk Polymorphic Variants**

| All variant sequences appearing in Genbank under the listed Accession Numbers are incorporated herein in their entirety by reference as though explicitly recited herein and for possible inclusion in any of the claims herein. | | | |
|---|---|---|---|
| | | | |

| **IGKJ gene name** | **Variant name** | **Accession number** | **Sequence** |
|---|---|---|---|
| **IGKJ1** | IGKJ1*01 | J00242 | SEQ ID NO: 181 |
| **IGKJ2** | IGKJ2*01 | J00242 | SEQ ID NO: 182 |
| | | | TG TAC ACT TTT GGC CAG GGG ACC AAG CTG GAG ATC AAA C |
| | IGKJ2*02 | Z70260 #c | TAC>TGC |
| | IGKJ2*03 | U95246 #c | ACT>AGT |
| | IGKJ2*04 | L40735 #c | TAC>TGC |
| | | | ACT>AGT |
| **IGKJ3** | IGKJ3*01 | J00242 | SEQ ID NO: 183 |
| **IGKJ4** | IGKJ4*01 | J00242 | SEQ ID NO: 184 |
| | | | G CTC ACT TTC GGC GGA GGG ACC AAG GTG GAG ATC AAA C |
| | IGKJ4*02 | AF103571 #c | ACT>ACG |
| **IGKJ5** | IGKJ5*01 | J00242 | SEQ ID NO: 185 |

**Table 7: Human IgHJλ Polymorphic Variants**

| All variant sequences appearing in Genbank under the listed Accession Numbers are incorporated herein in their entirety by reference as though explicitly recited herein and for possible inclusion in any of the claims herein. | | | |
|---|---|---|---|
| **IGLJ gene name** | **Variant name** | **Accession number** | **Sequence** |
| **IGLJ1** | J1*01 | X04457 | SEQ ID NO: 186 |
| **IGLJ2** | J2*01 | M15641 | SEQ ID NO: 187 |
| **IGU3** | J3*01 | M15642 | SEQ ID NO: 188 |
| | | | T GTG GTA TTC GGC GGA GGG ACC AAG CTG ACC GTC CTA G |
| | J3*02 | D87023(1) | GTG> TGG |
| | | | GTA> GTG |
| **IGLJ4** | J4*01 | X51755 | SEQ ID NO:189 |
| **IGLJ5** | J5*01 | X51755 | SEQ ID NO: 190 |
| | | | C TGG GTG TTT GGT GAG GGG ACC GAG CTG ACC GTC CTA G |
| | J5*02 | D87017 | ACC>ACG |
| **IGU6** | J6*01 | M18338 | SEQ ID NO: 191 |
| | J7*01 | X51755 | SEQ ID NO: 192 |
| **IGU7** | | | T GCT GTG TTC GGA GGA GGC ACC CAG CTG ACC GTC CTC G |
| | J7*02 | D87017 | GTC>GCC |

**TABLE 8:1000 GENOMES PROJECT HUMAN POPULATIONS**

| |
|---|
| Below is a summary of the ethnic population origin of samples that the 1000 Genomes Project sequences. |

| **Population** |
|---|
| **European ancestry** |
| Utah residents (CEPH) with Northern and Western European ancestry (CEU) |
| Toscani in Italia (TSI) |
| British from England and Scotland (GBR) |
| Finnish from Finland (FIN) |
| Iberian populations in Spain (IBS) |
| |

| **East Asian ancestry** |
|---|
| Han Chinese in Beijing, China (CHB) |
| Japanese in Toyko, Japan (JPT) |
| Han Chinese South (CHS) |
| Chinese Dai in Xishuangbanna (CDX) |
| Kinh in Ho Chi Minh City, Vietnam (KHV) |
| Chinese in Denver, Colorado (CHD) (pilot 3 only) |
| |

| **West African ancestry** |
|---|
| Yoruba in Ibadan, Nigeria (YRI) |
| Luhya in Webuye, Kenya (LWK) |
| Gambian in Western Division, The Gambia (GWD) |

| **Malawian in Blantyre, Malawi (MAB)** |
|---|
| **Population** |
| West African Population (TBD) |

| **Americas** |
|---|
| African Ancestry in Southwest US (ASW) |
| African American in Jackson, MS (AJM) |
| African Caribbean in Barbados (ACB) |
| Mexican Ancestry in Los Angeles, CA (MXL) |
| Puerto Rican in Puerto Rico (PUR) |
| Colombian in Medellin, Colombia (CLM) |
| Peruvian in Lima, Peru (PEL) |
| |

| **South Asian ancestry** |
|---|
| Ahom in the State of Assam, India |
| Kayadtha in Calcutta, India |
| Reddy in Hyderabad, India |
| Maratha in Bombay, India |
| Punjabi in Lahore, Pakistan |

**Table 9: Immunoglobulin Gene Usage in Human Antibody Responses to Infectious Disease Pathogens**

| | | | |
|---|---|---|---|
| | | | |
| KAPPA V GENES | Haemophilus influenzae type b polysaccharide (Hib PS) | Haemophilus influenzae | 1. Lonberg, Nat Biotech 2005; [human PBMCs] |
| • Vk II germline gene A2 + JK3 | | | |
| • Vk II family gene + JK4 | | | 2. Adderson et al, J Clin Invest 1992; [Human PBLs] |
| • 94% identical to the A27 (Humkv325) germ line gene | | | |
| | | | 3. Chung et al, J Immunol 1993 |
| • a VκI gene family member; κI-15A (KL012) | | | 4. Nadel et al, J Immunol 1998 |
| | | | 5. Feeney et al, J Clin Invest 1996 |
| | | | 6. Lucas et al, Infect Immun 1994; [Human PBLs] |
| LAMBDA V GENES | | | |
| • Four Vλ VII family members that are 96-98% identical to each other | | | 7. Adderson et al, J Clin Invest 1993; [Human PBLs] |
| | | | 8. Granoff et al, J Clin Invest 1993; [human PBLs] |
| • Vλ II family members (82,89 and 91% homologous to Vλ2.1 gene) + VHIII segments closely homologous to germline gene 9.1 | | | |
| | | | 9. Azmi et al, Infect Immun 1994; [human tonsil cells] |
| • V_{λ}VII 4A | | | |
| • All with Jλ homologous to germline Jλ2 and Jλ3 | | | |
| | | | |
| VH GENES | | | |
| • VH 96% identical to the VH germ line gene segment DP77 (V3-21) | | | |
| • LSG6.1, LSG12.1, V_{H}III VH26, V_{H}III 9.1 | | | |
| | | | |
| VH and VL COMBINATIONS | | | |
| • V_{H}III 9.1+ V_{λ}VII 4A | | | |
| • V_{H}III 9.1 + V_{λ}II 2.1 | | | |
| • V_{H}III 9.1 + V_{κ}II A2 | | | |
| • V_{H}III VH26 + V_{λ}II 2.1 | | | |
| • V_{H}III 9.1; VHIII H11; V_{H}III VH26 | • Polysaccharide capsule of E coli | • E coli K1 | 9. Azmi et al, Infect Immun 1994 |
| • κI 15A | K1 | | |
| • Vλ2.1 | | | |
| | • Meningococcal B polysaccharide; Poly[α(2→8)-N-acetylneuramic acid | • Neisseria meningitidis Group B | |
| | | | |
| • VHIII or VHIV family member | | • Herpes family virus | 10. Huang et al, J Clin Invest 1992; |
| • VλI or Vλ3 member | • HSV 120-kD glycoprotein | • Herpes simplex virus (HSV); HSV-1; HSV-2 | [human tonsils] |
| • VH26 + Dk1 + JH6 with IGLV1S2 + Jλ2 | • 116-, 105-, 64-kD glycoproteins of VZV | • Varicella zoster virus (VZV) | |
| | | | |
| • VH4.18 | | | |
| • VH2-1 (VH3) + D region Dxp'1 + JH5 with Vλ3 cML70 + Jλ3 | | | |
| | | | |
| • VH1GRR+JH3 + Dn4r or D2r with IGLV1S2 + JλZ | | | |
| • For VZV Abs: ha3h2 (VH3) with Ialh2 (Vλ); or halcl (VH1) with Ialvl (Vλ1) | | | |
| • For VZV Abs: ha4h3 (VH4) with Ia3h3(Vλ3) | | | |
| • Hv1051 (VH) | | • Cytomegalovirus (CMV) | 10. Huang et al, J Clin Invest 1992; |
| • Kv325 (Vk) | | | |
| • 71-2 (VH) | | • HIV | 10. Huang et al, J Clin Invest 1992; |
| • Hv1f10 (VH) | | | 11. Wang & Palese, Science 2011 |
| • VH4.11 | | | |
| • 71-4 (VH) | | | |
| • VH251 | | | |
| • VH1-69 | | | |
| • VH1-69 | Haemagglutinin (HA) | • Influenza virus, eg, Group 1 and/or Group 2 Infulenza A virus; eg, H1N1, H2N2, or H3N2 or H7N2 or H7N7 influenza virus | 12. Ekiert et al, Science 2009 |
| | | | 13. Throsby et al, PLoS One 2008 |
| | | | 14. Sui et al, Nat Struct Mol Biol 2009 |
| | | | 15. Ekiert et al, Science 2011 |

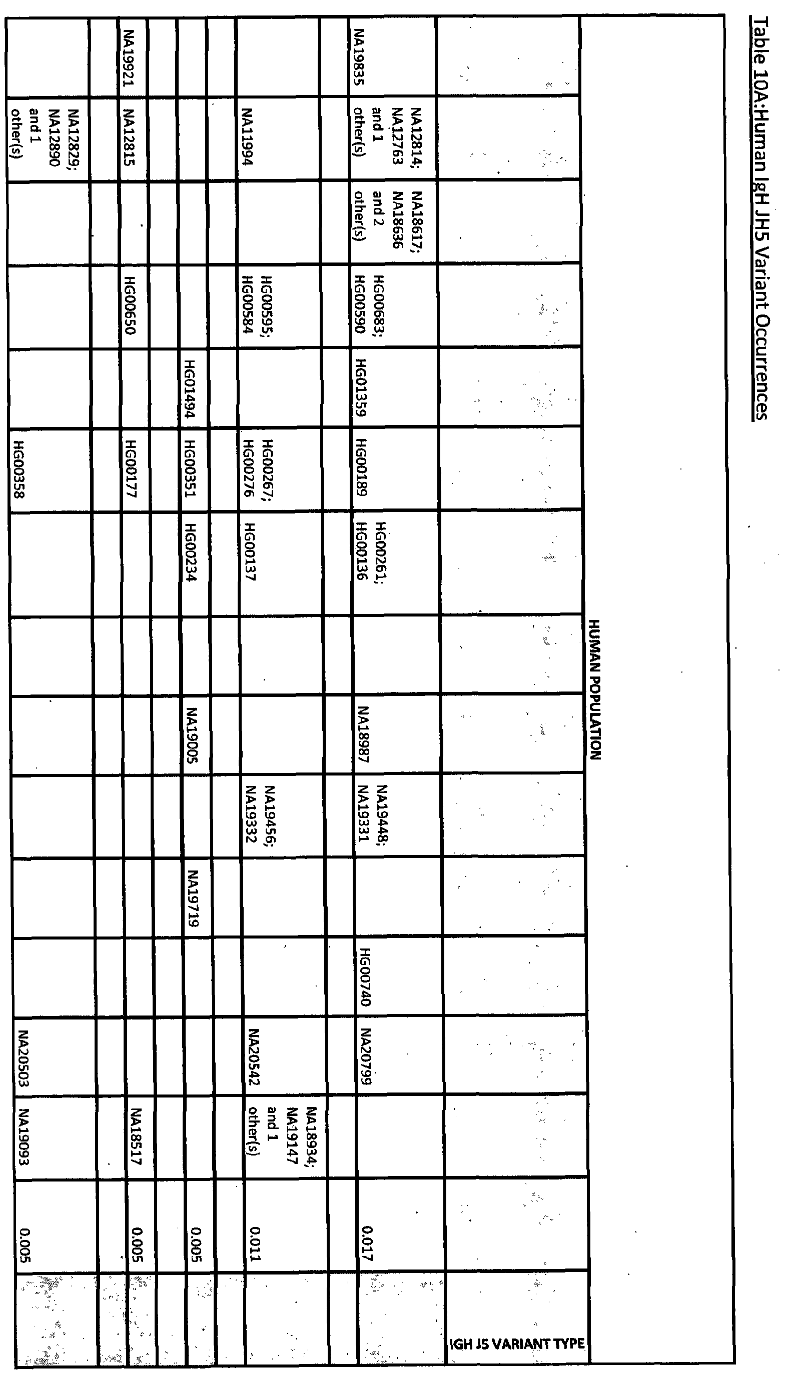

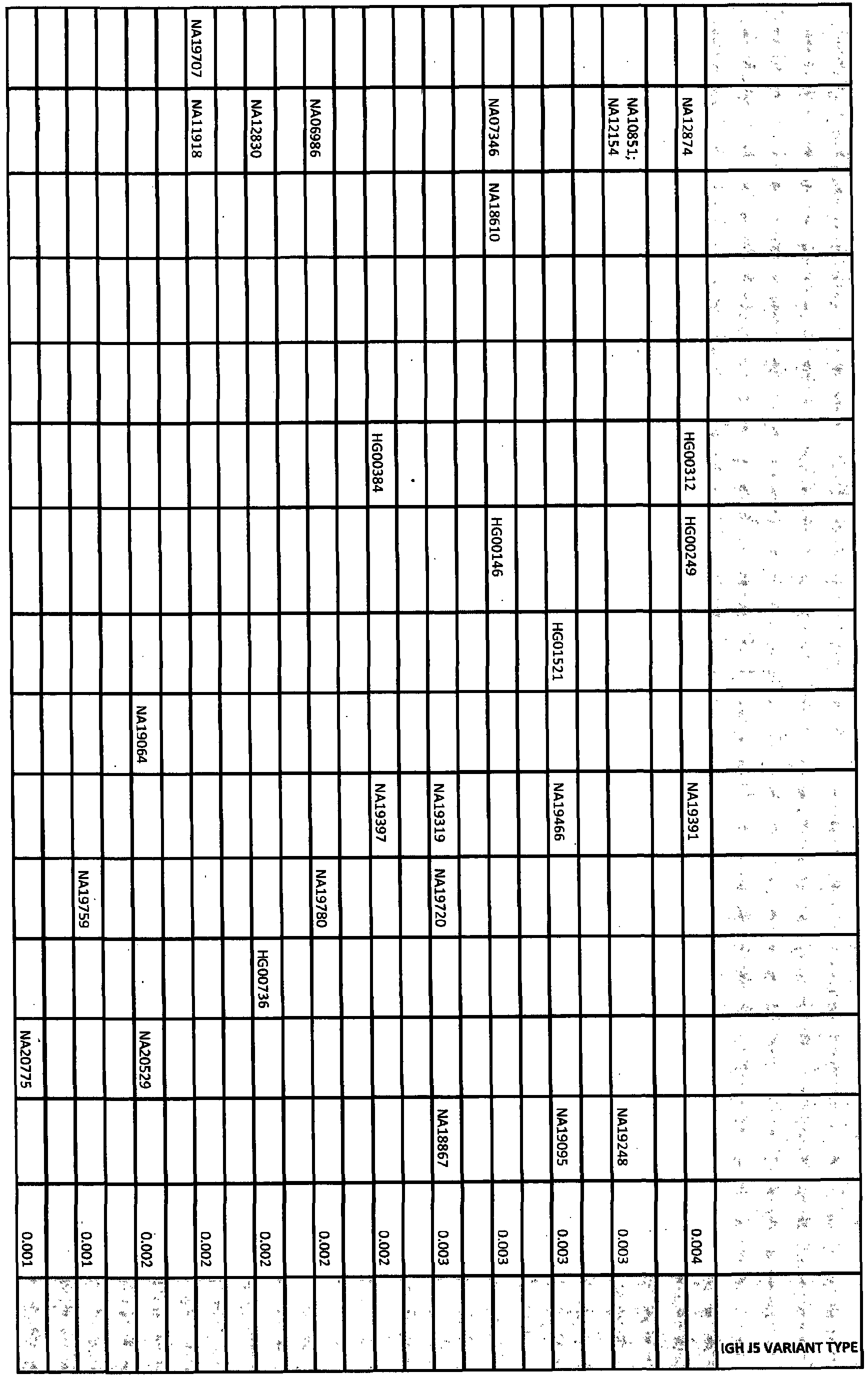

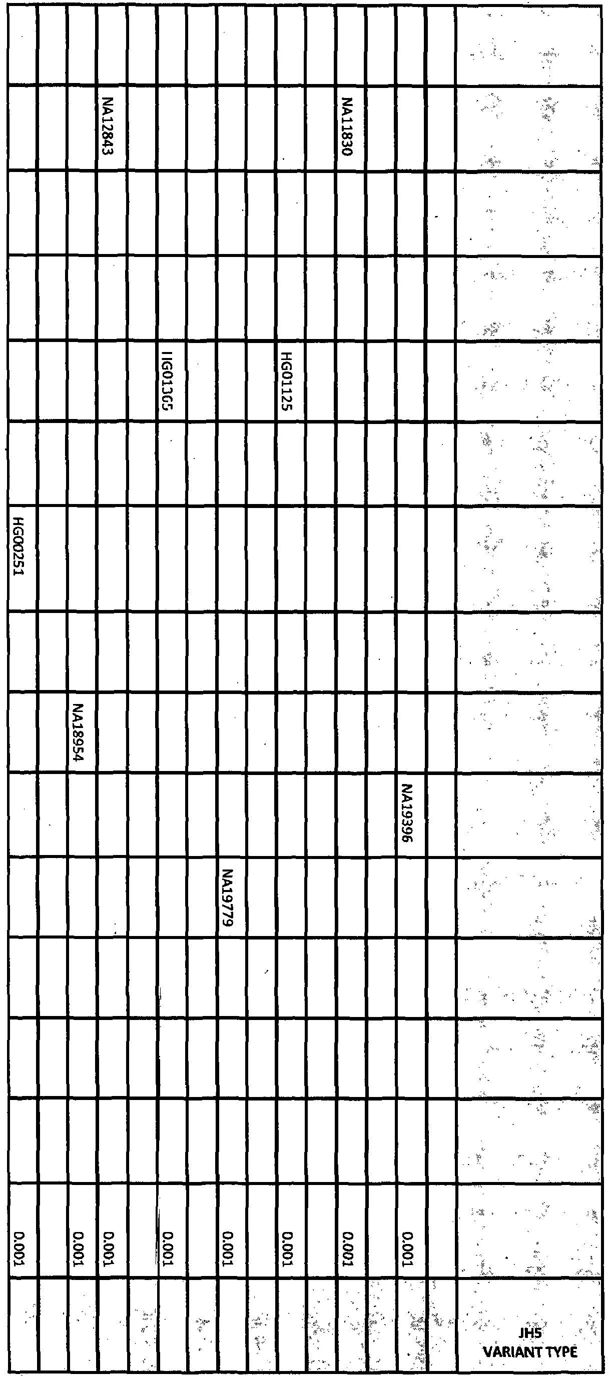

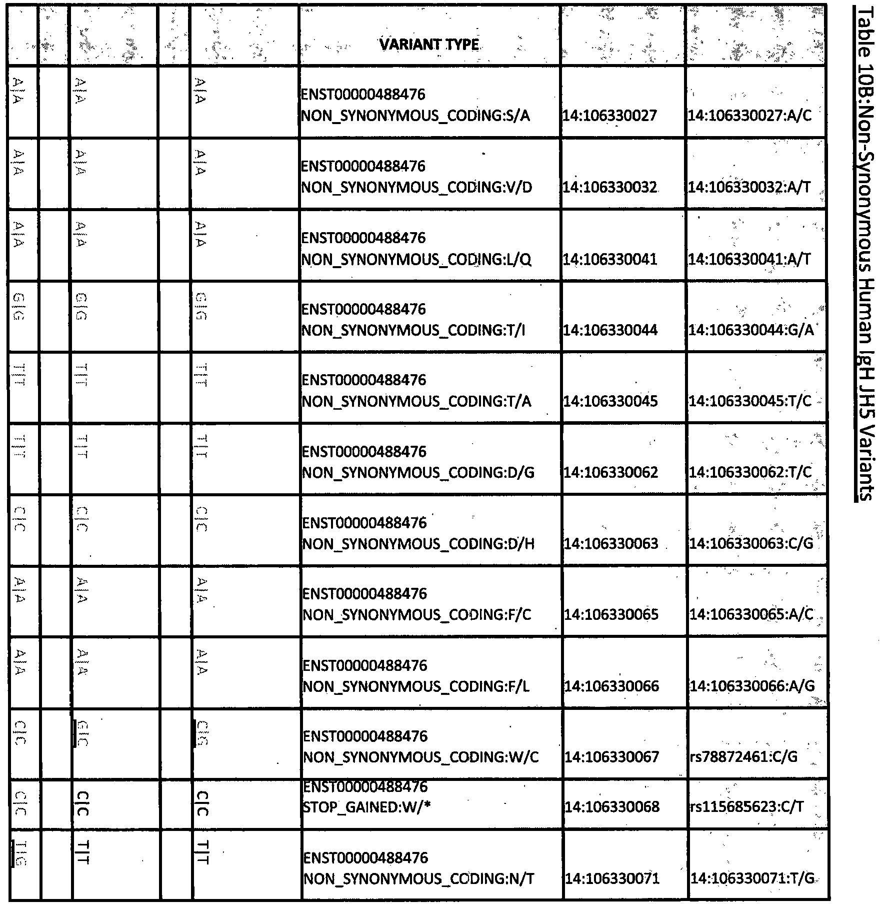

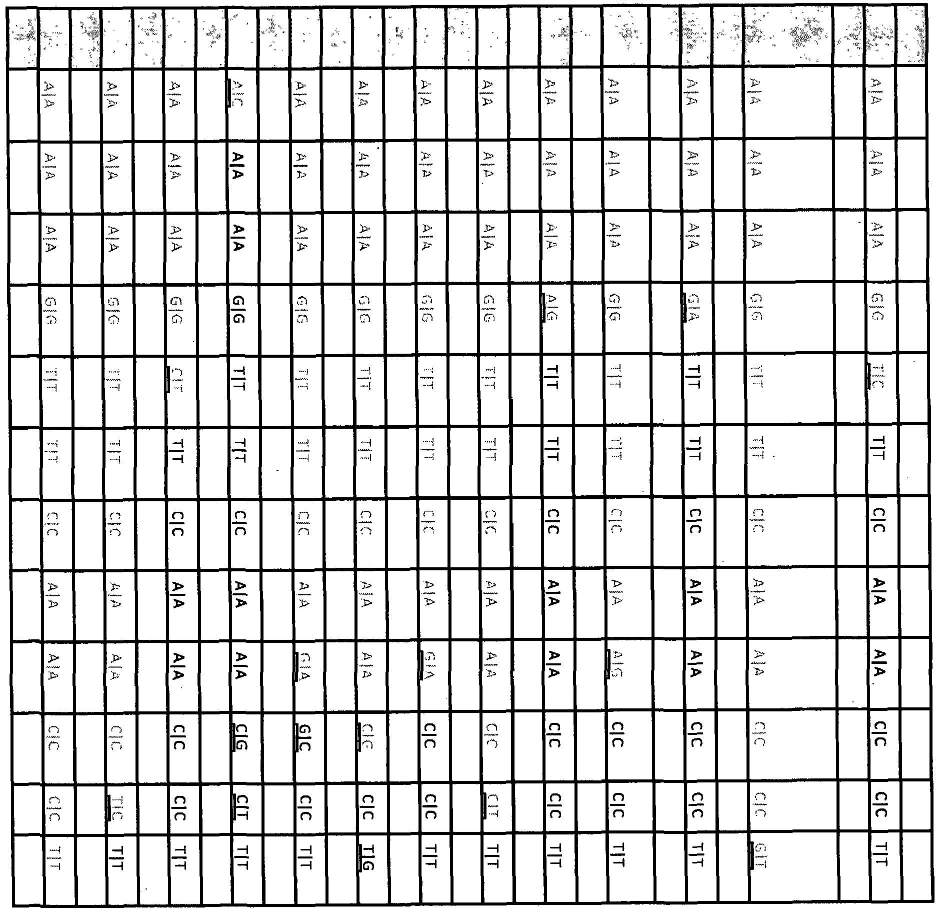

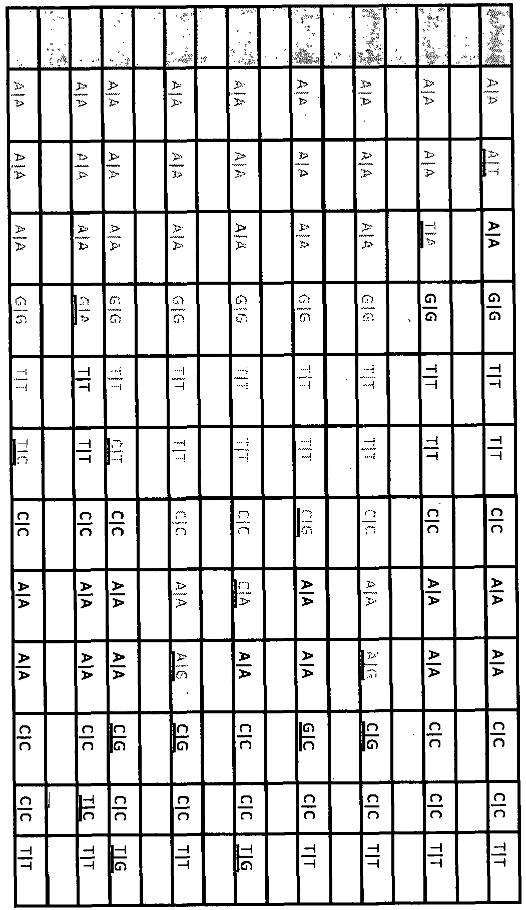

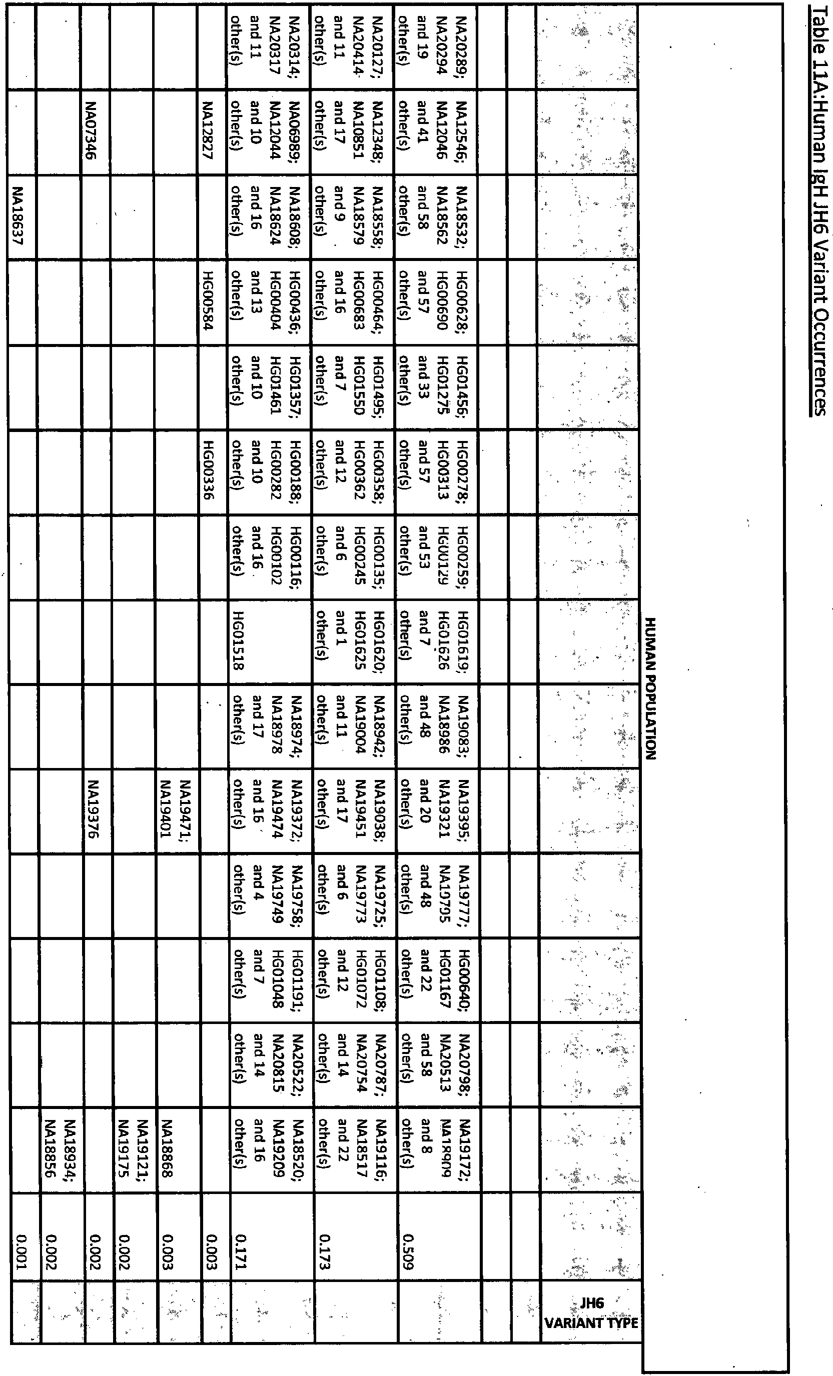

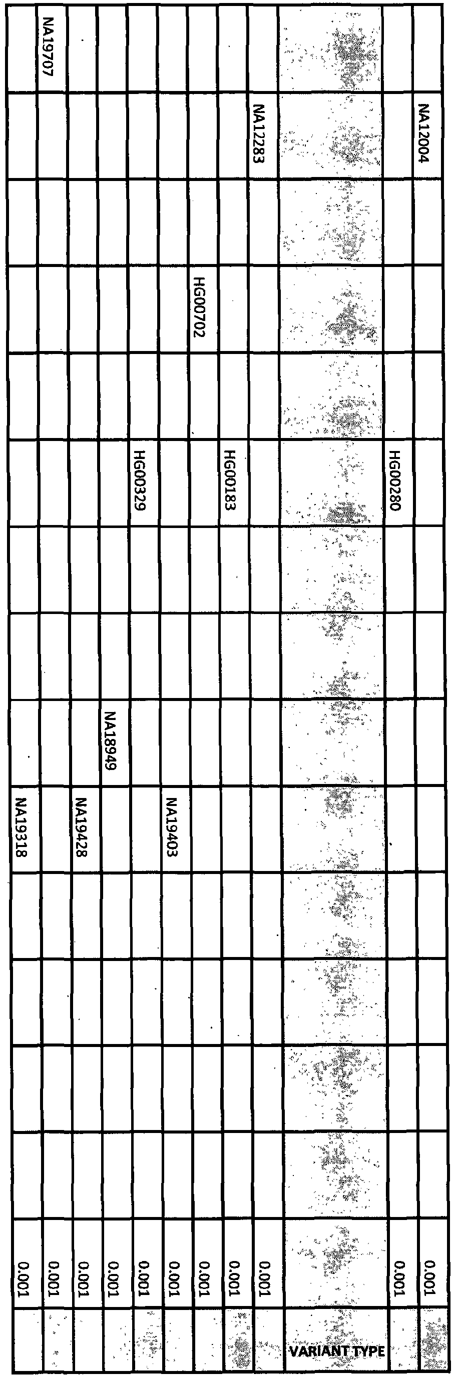

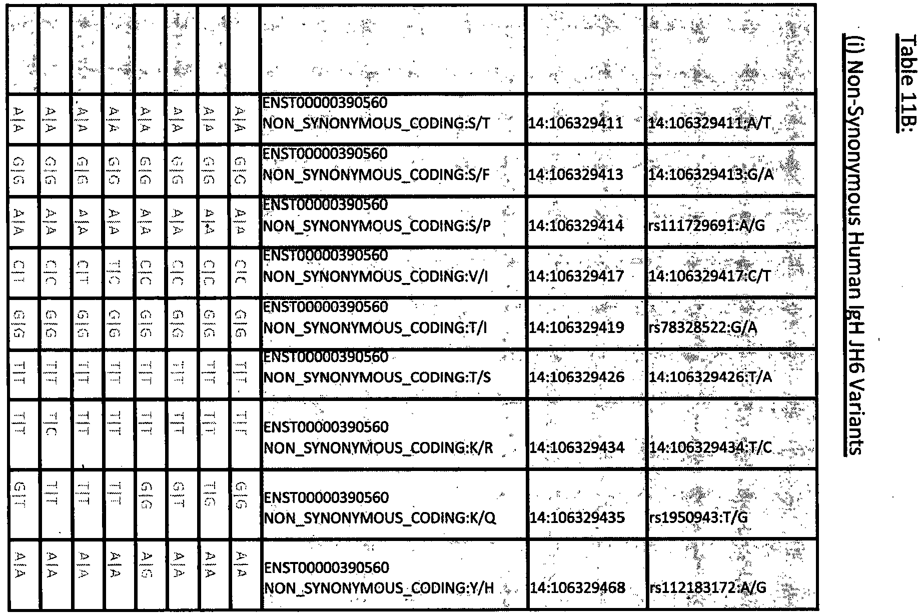

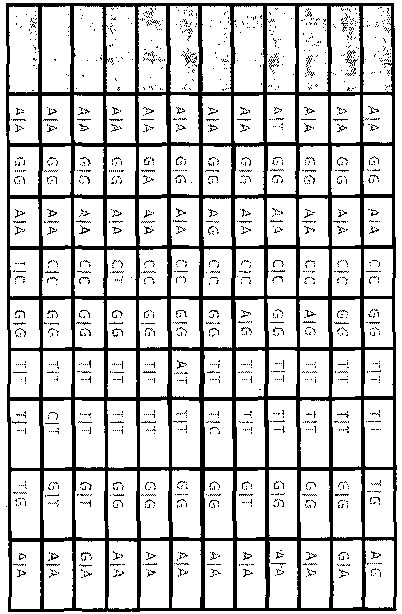

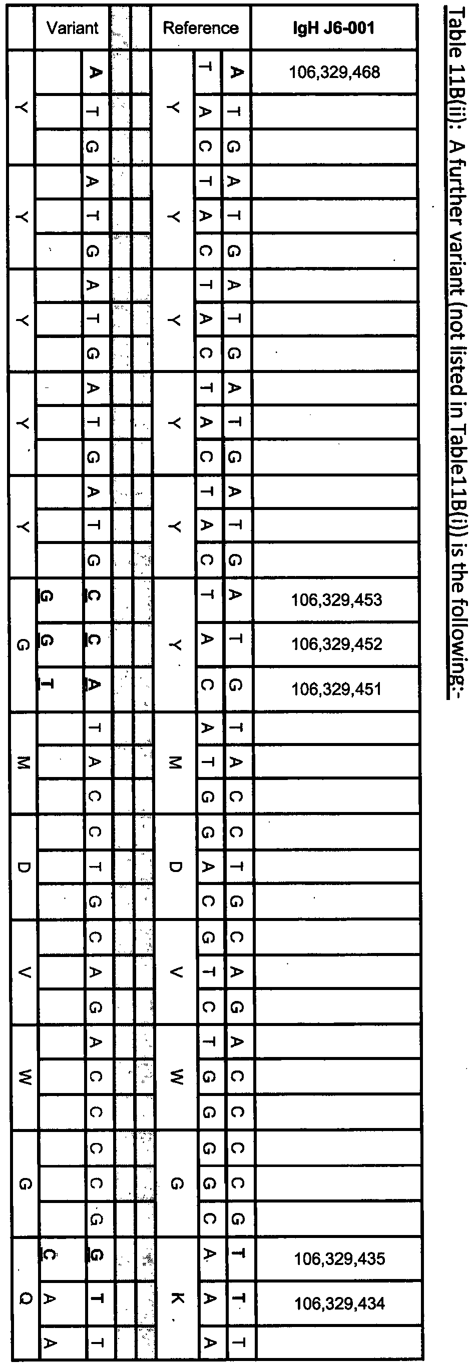

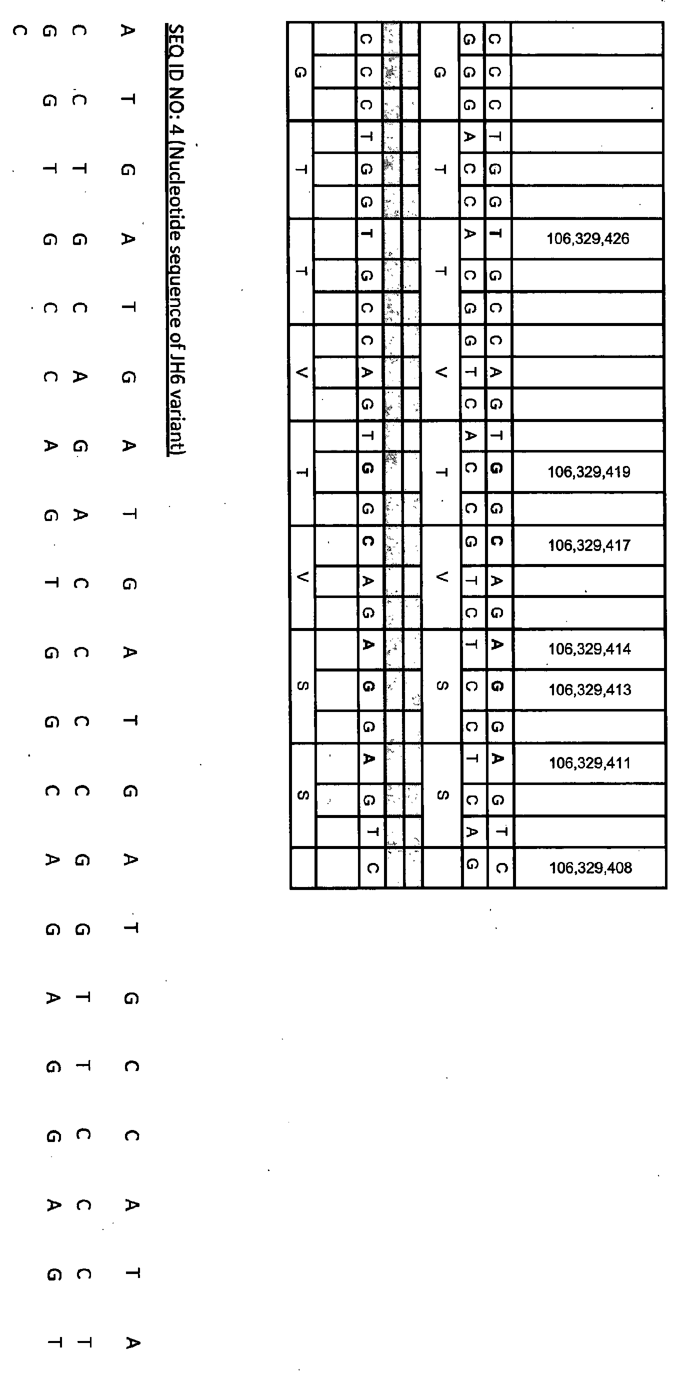

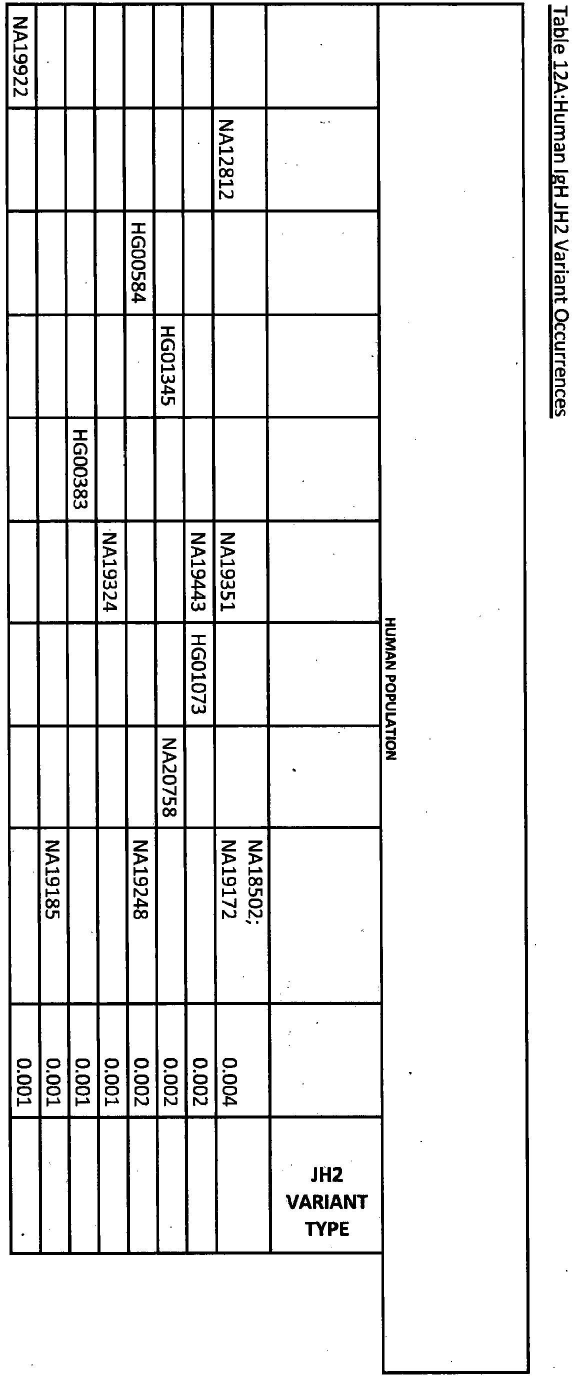

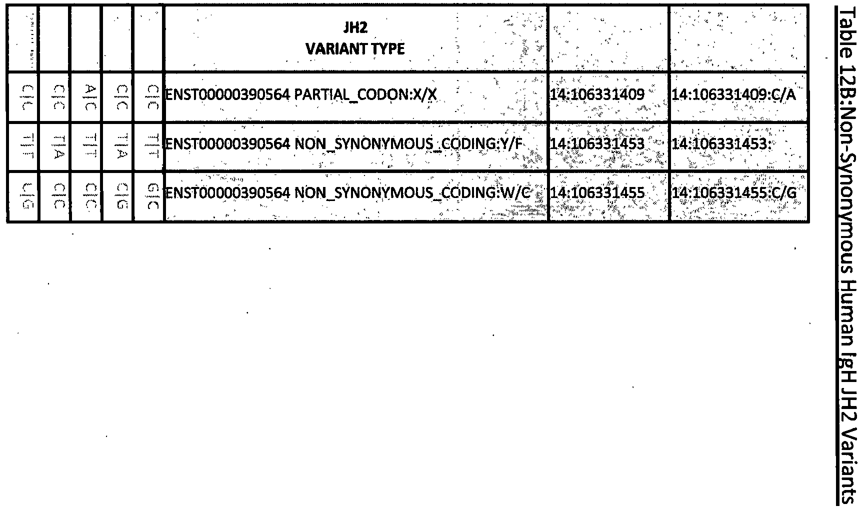

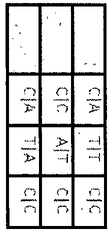

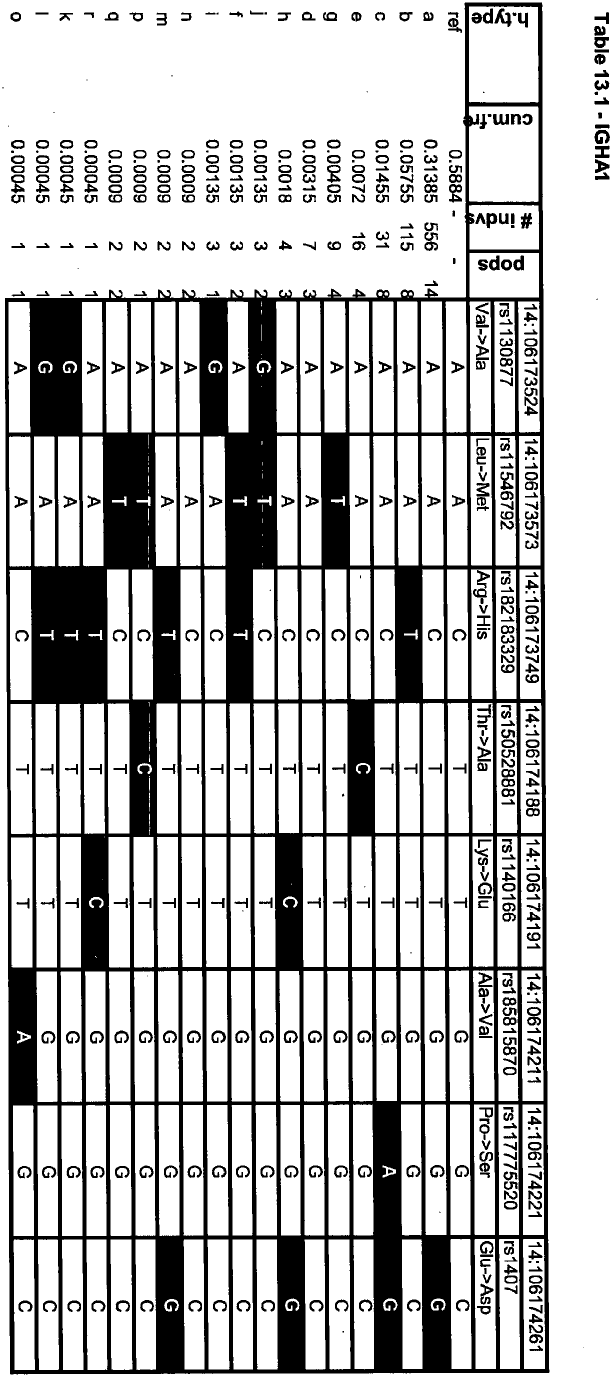

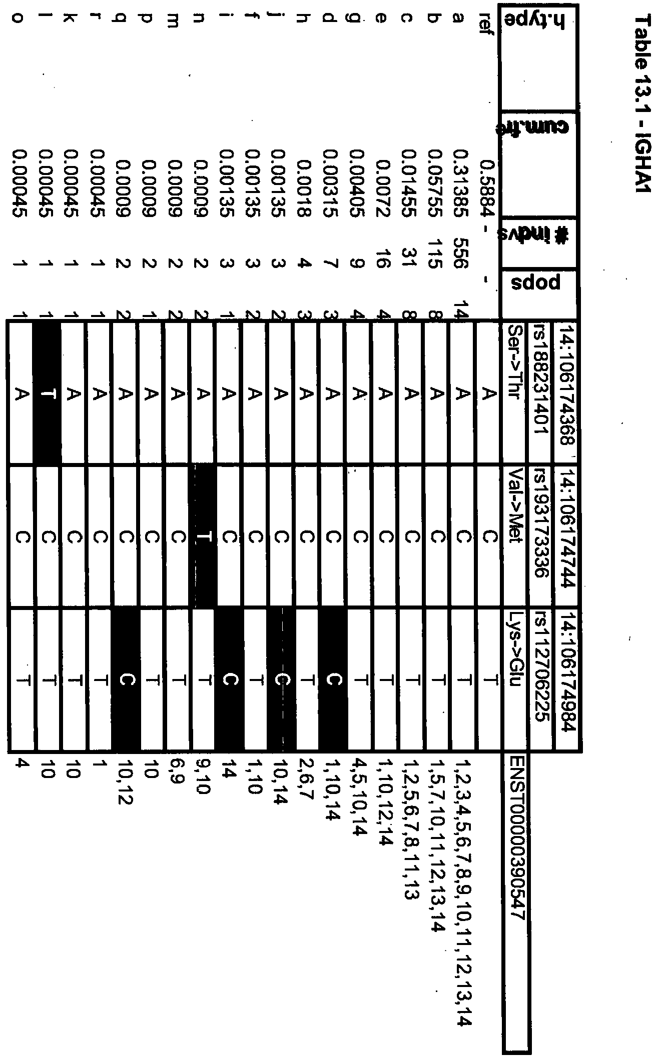

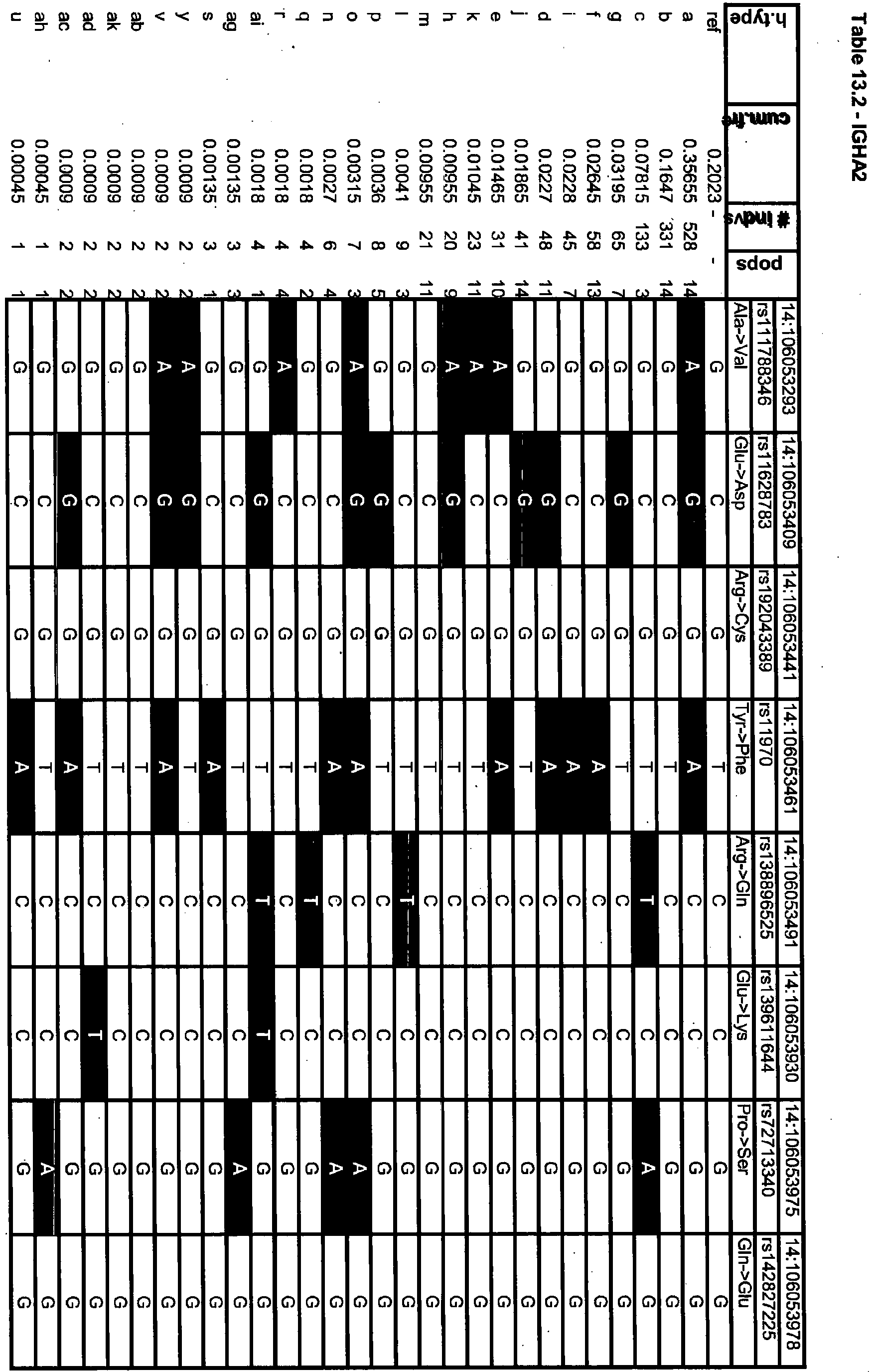

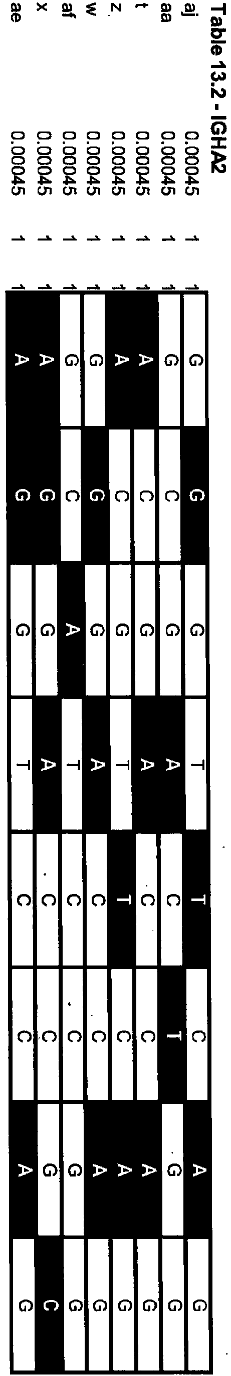

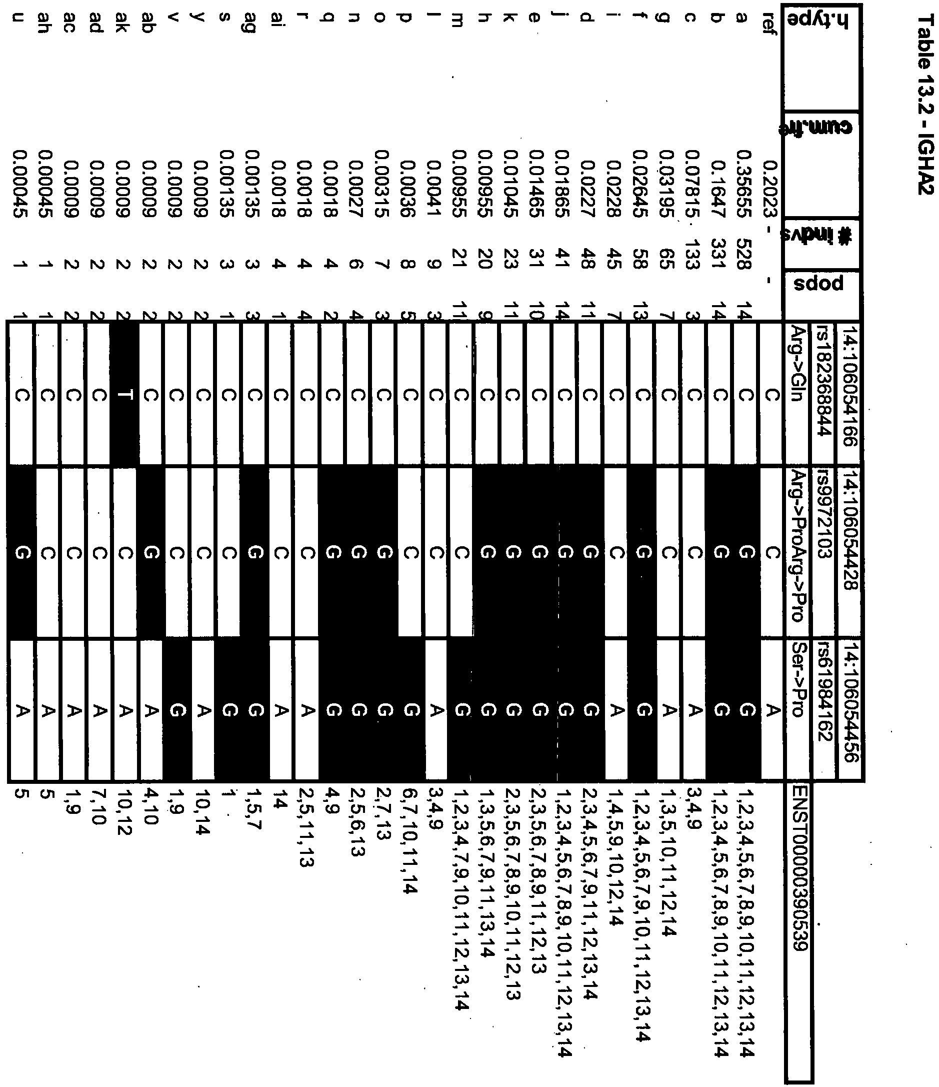

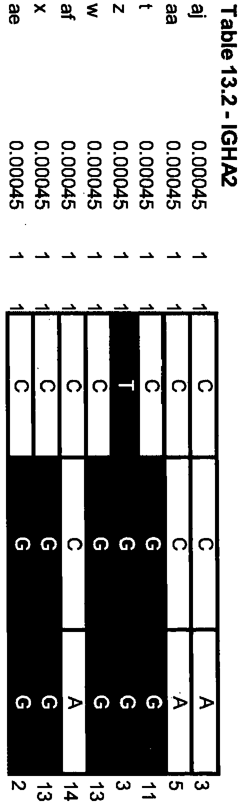

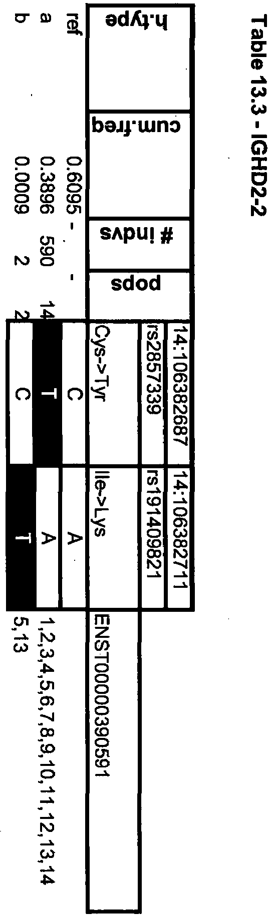

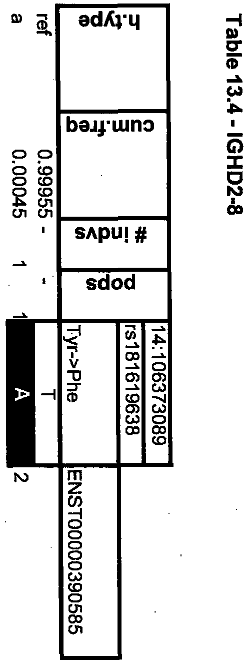

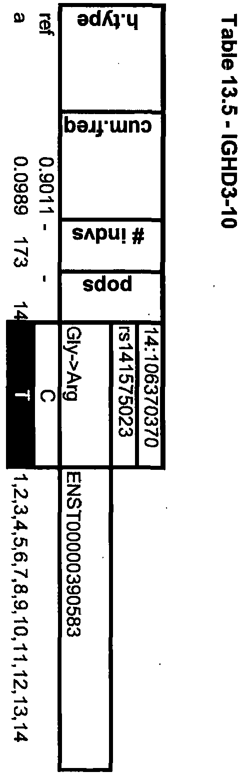

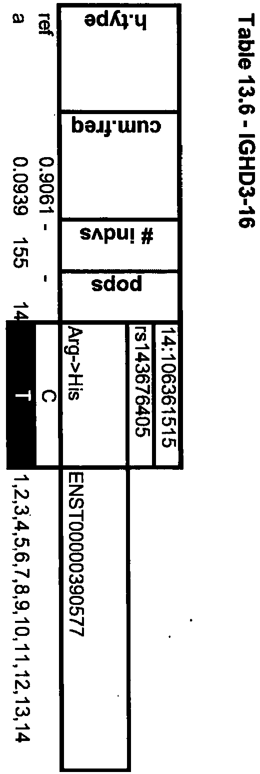

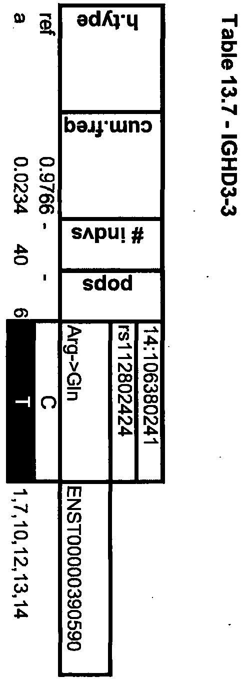

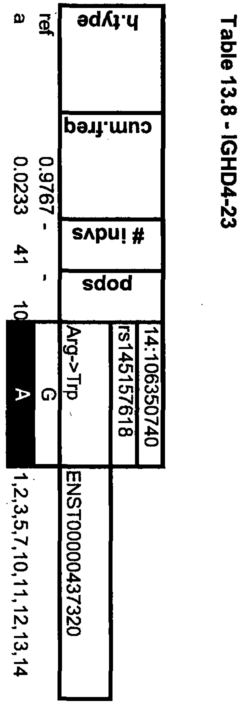

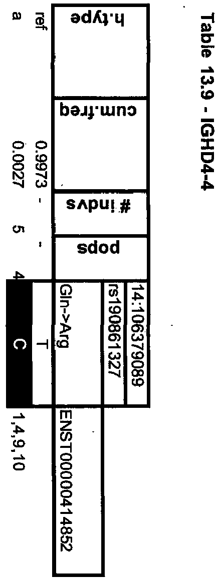

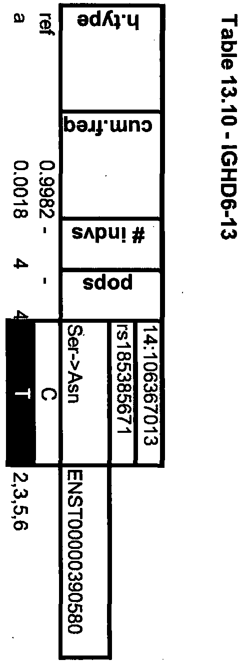

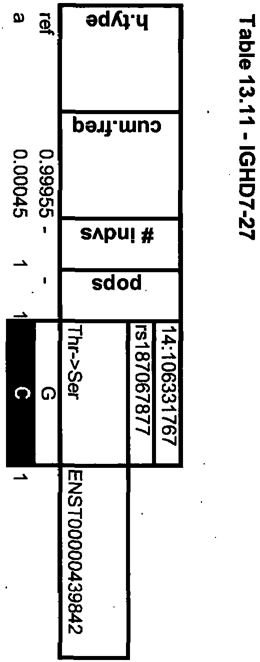

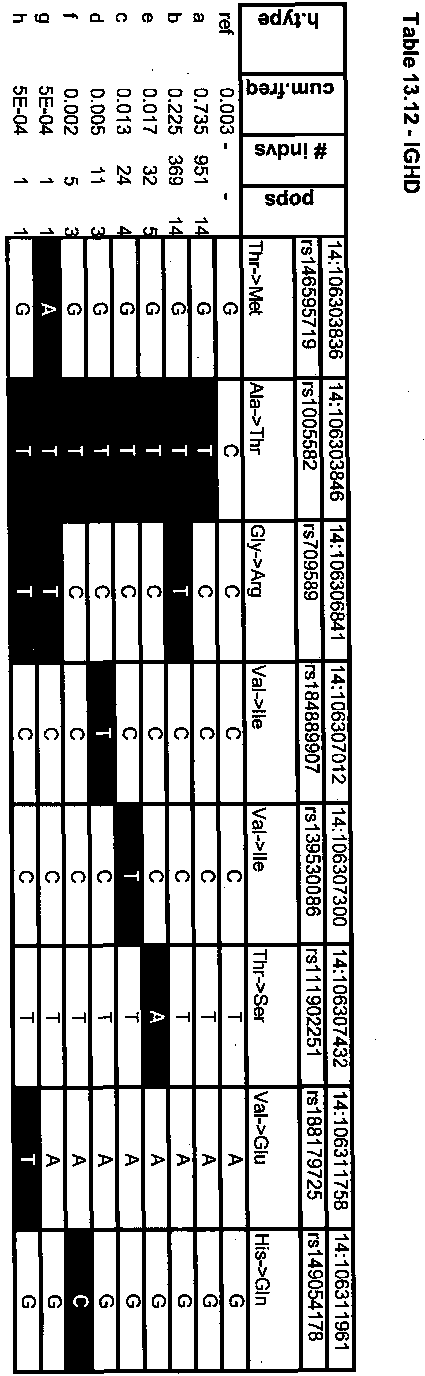

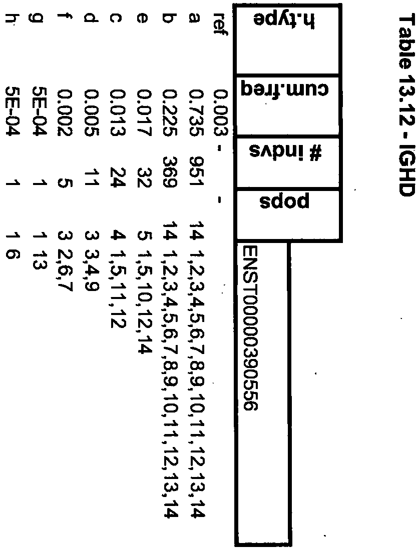

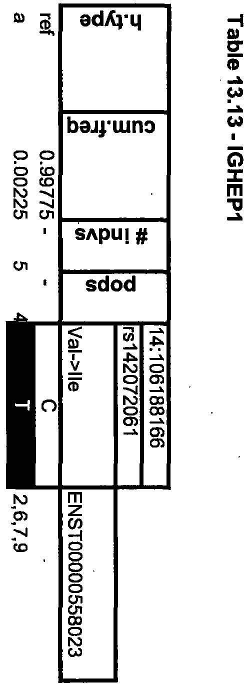

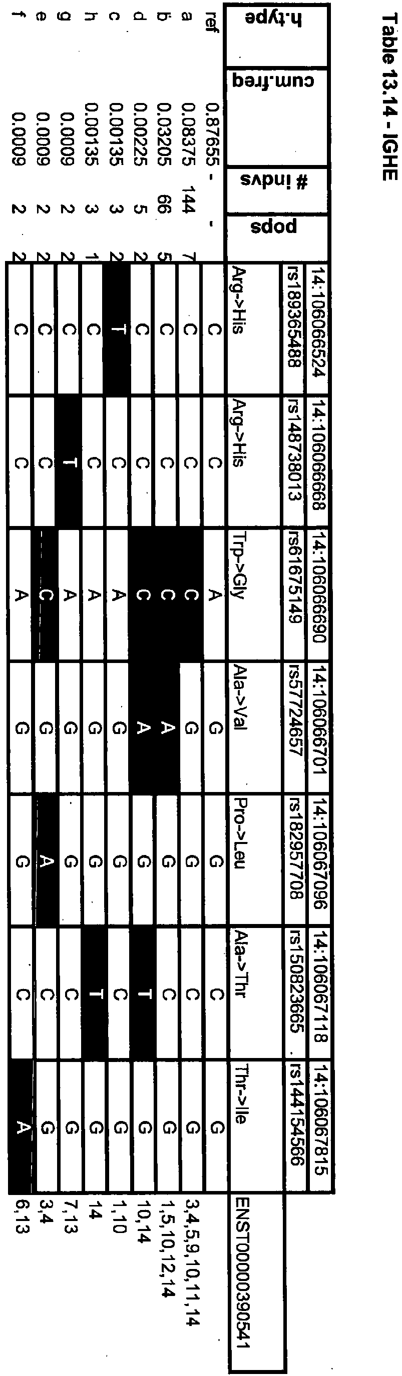

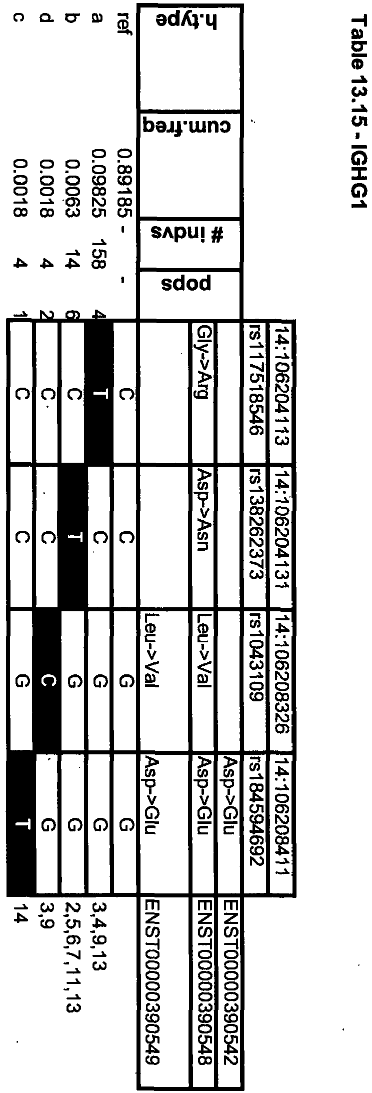

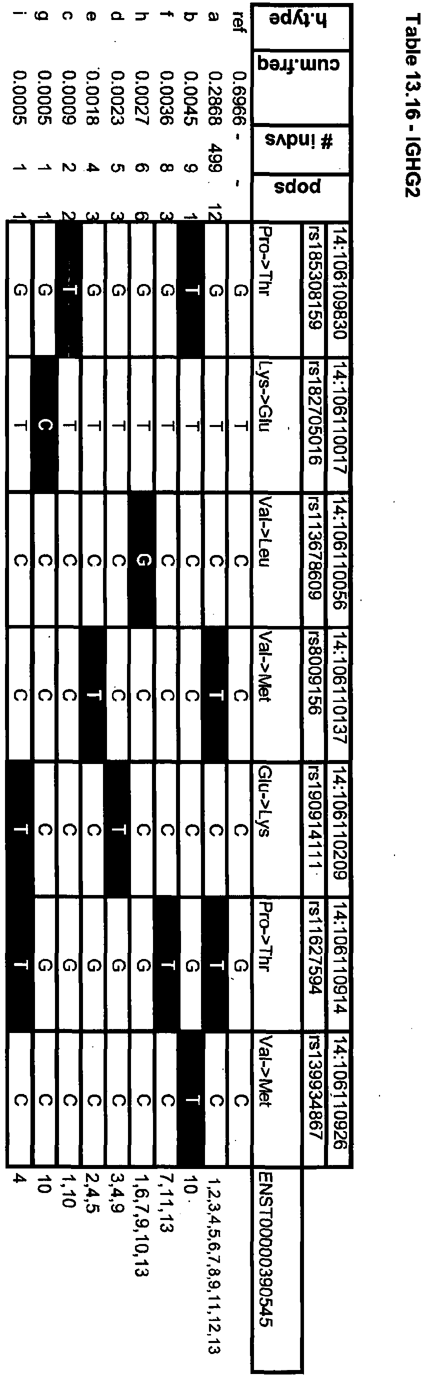

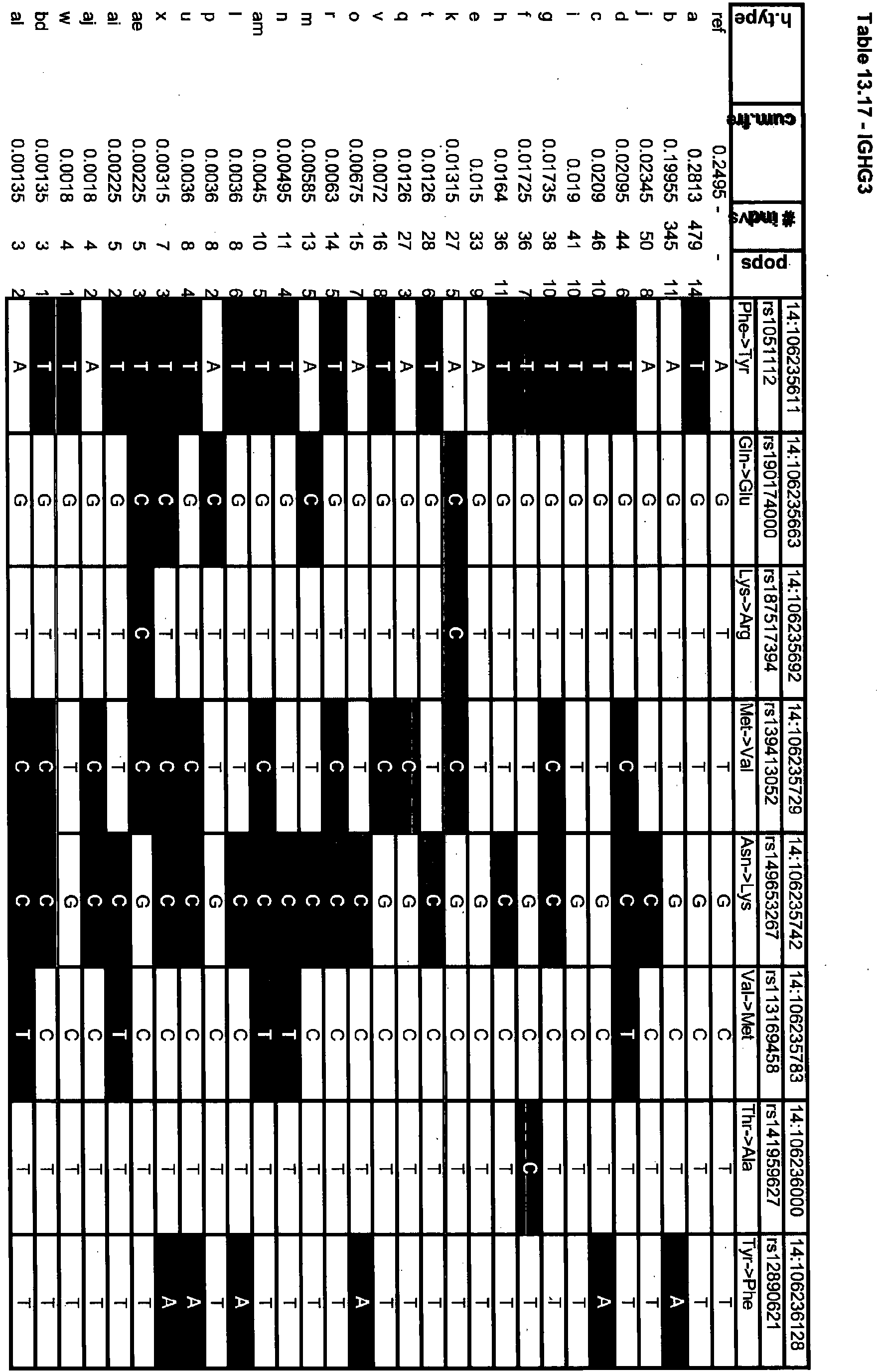

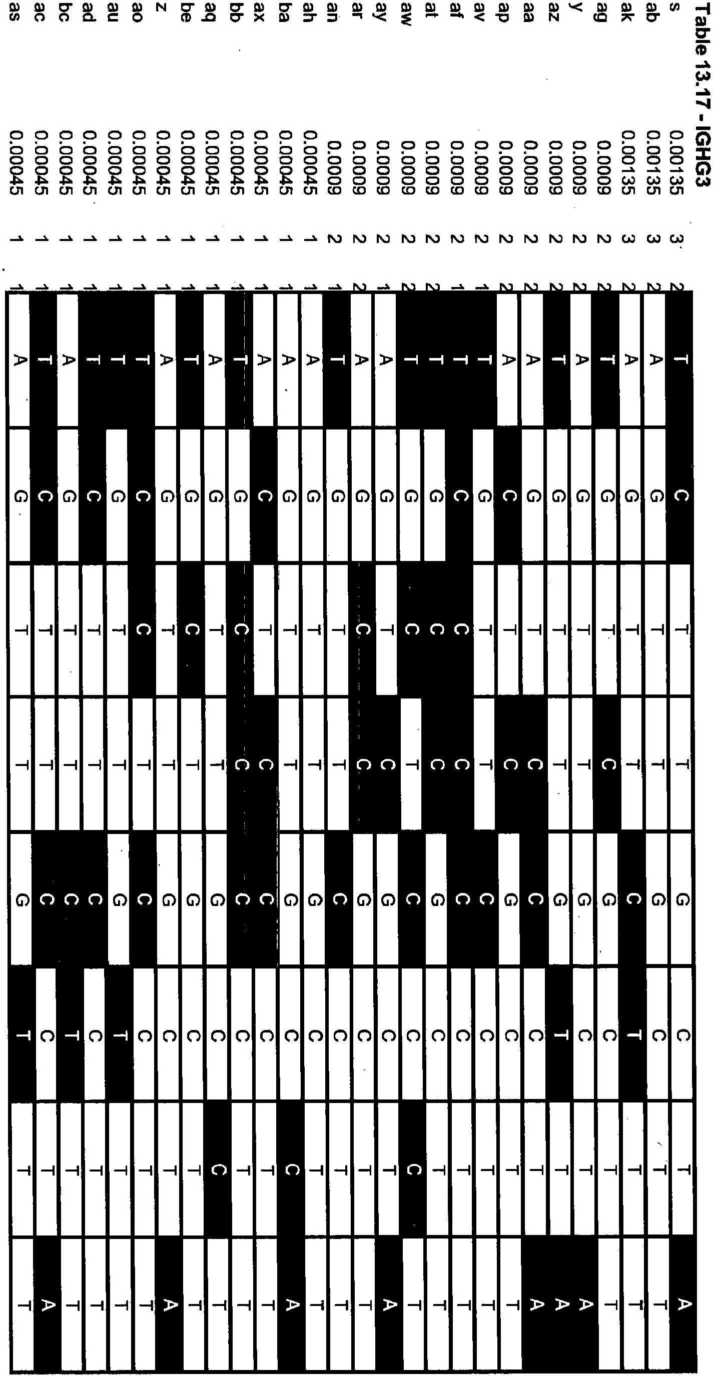

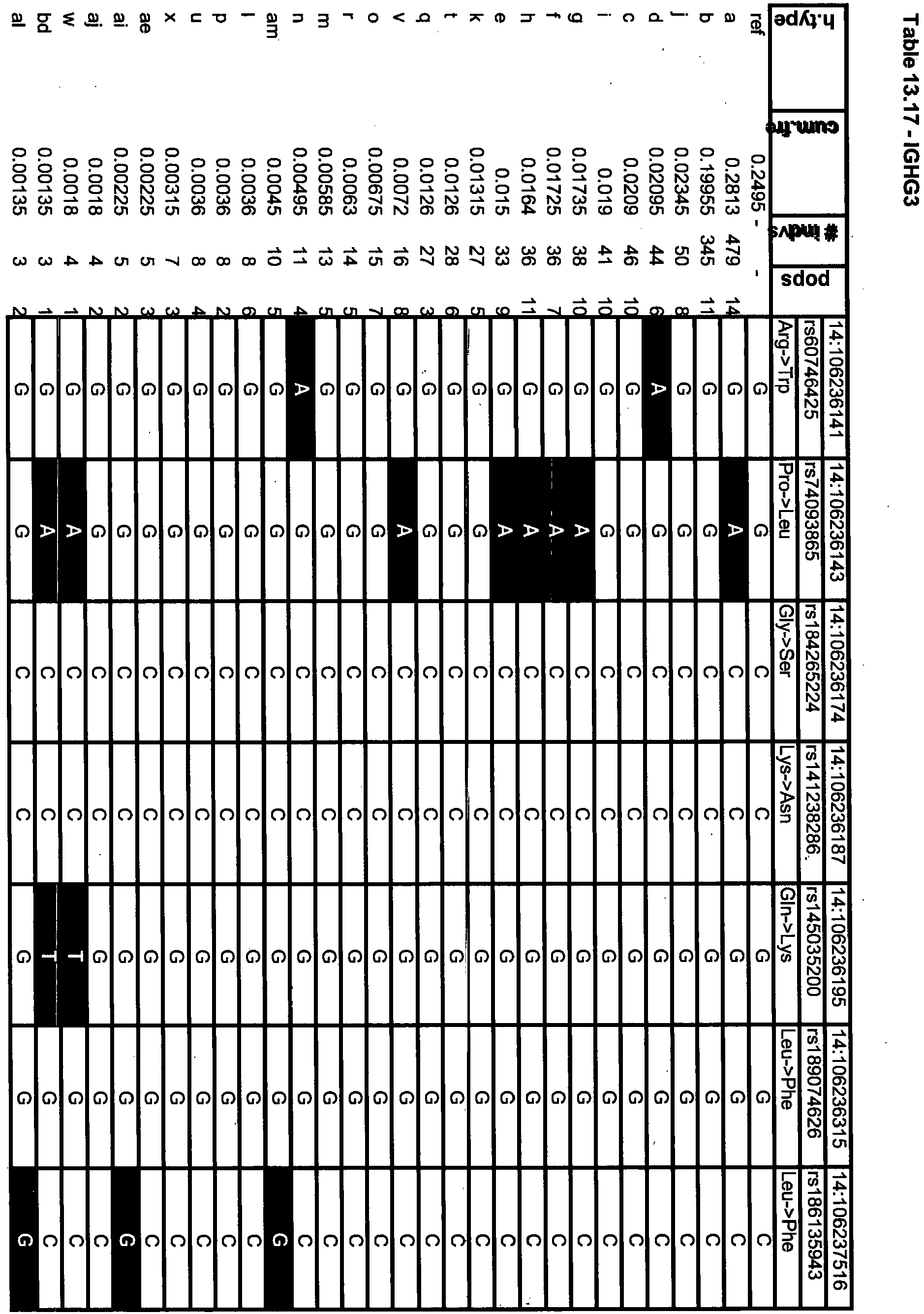

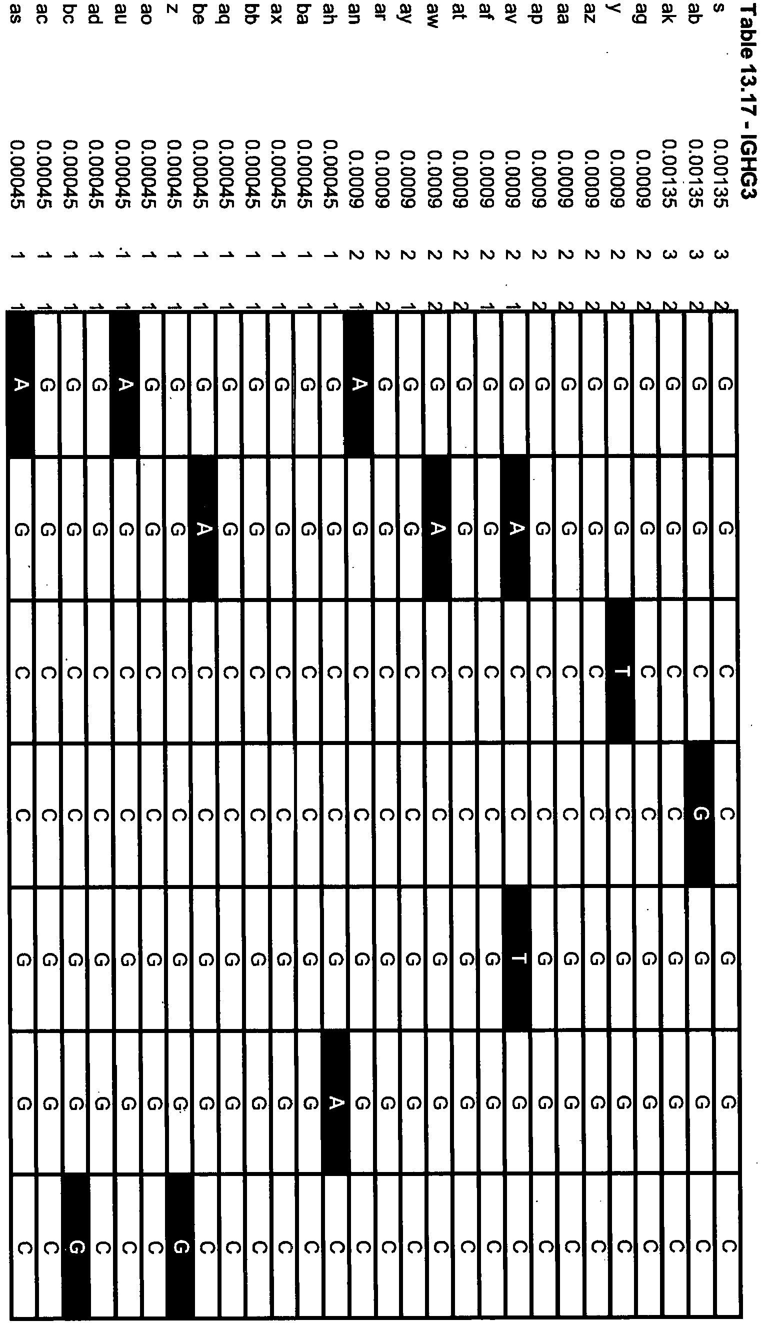

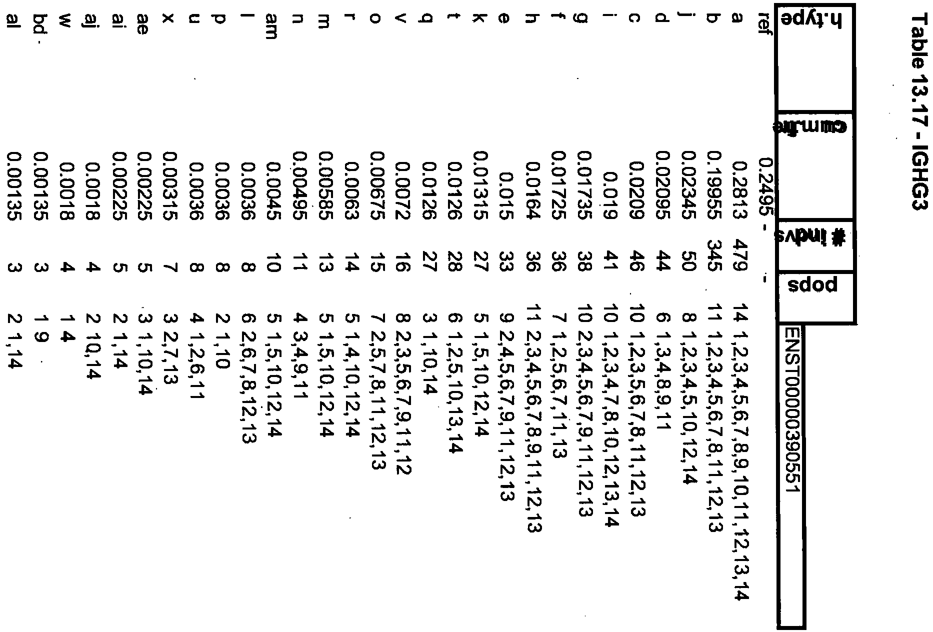

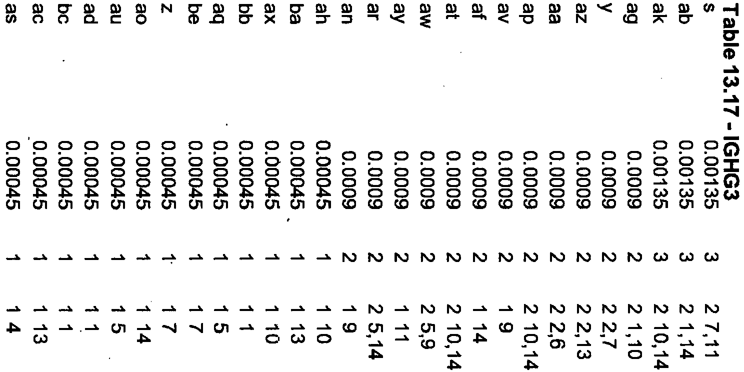

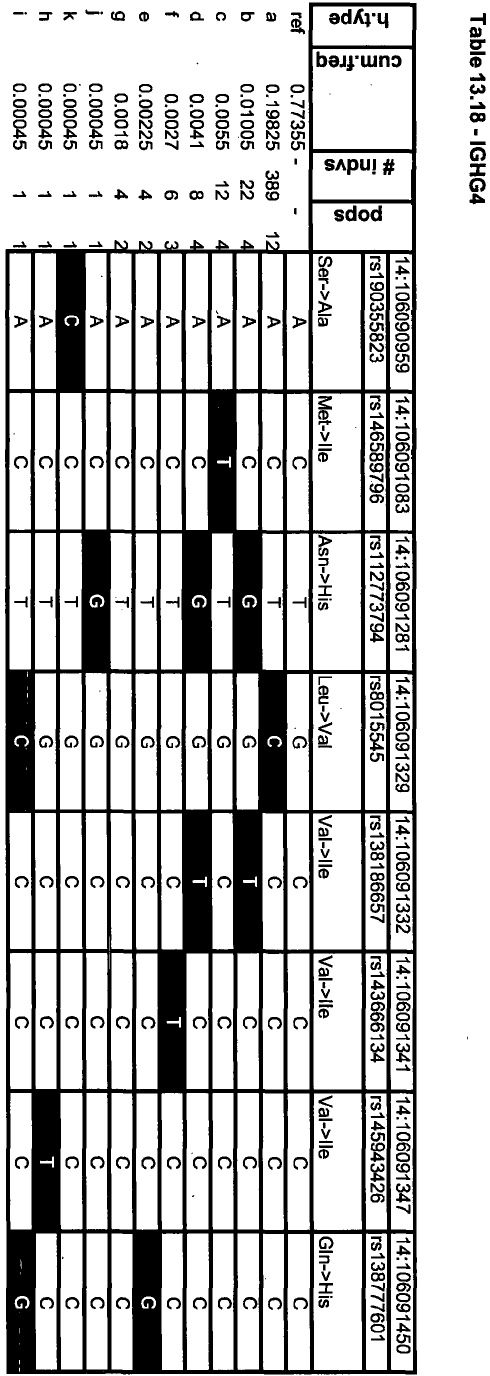

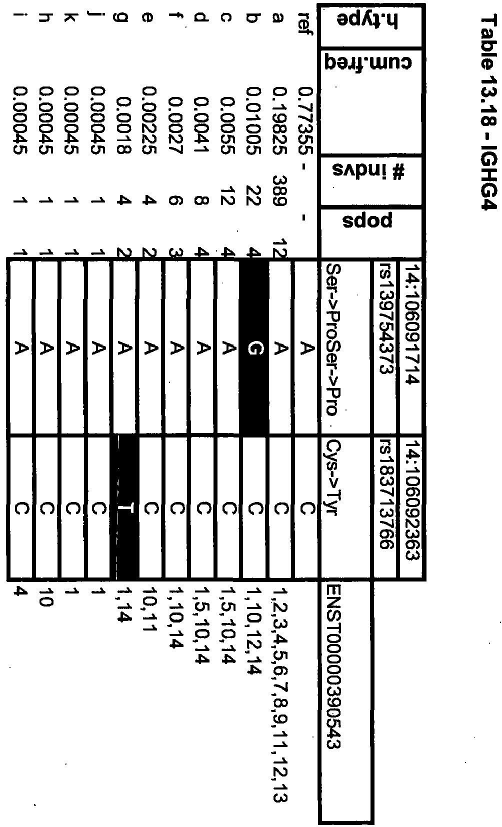

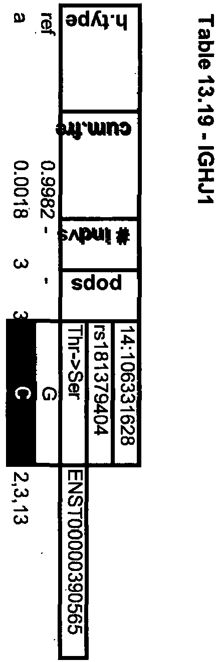

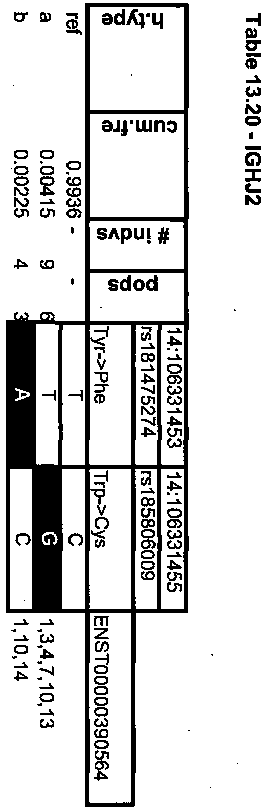

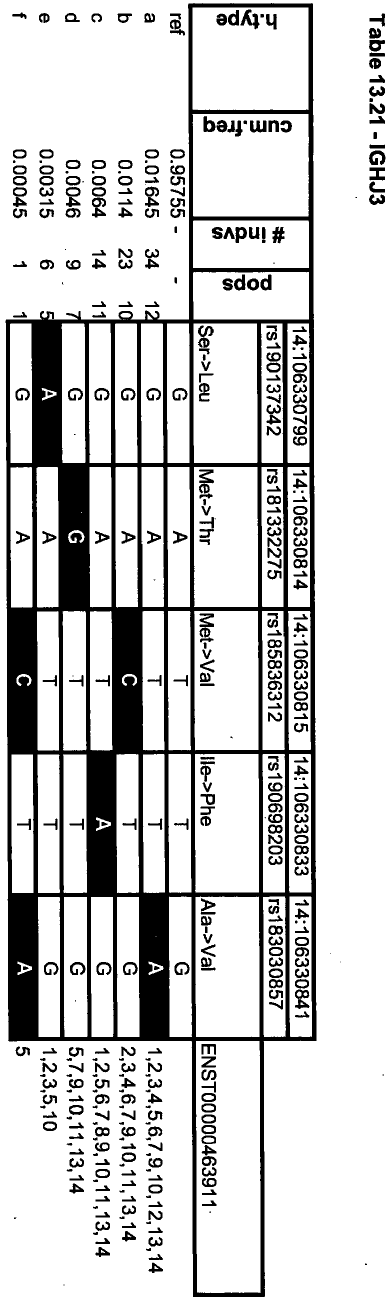

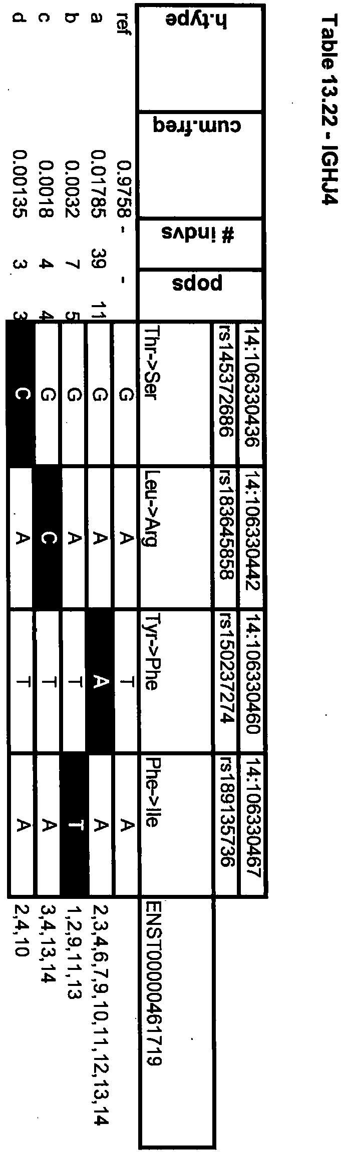

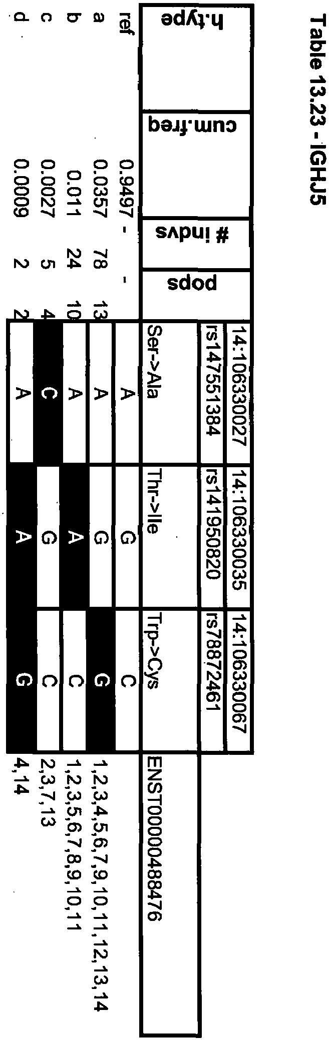

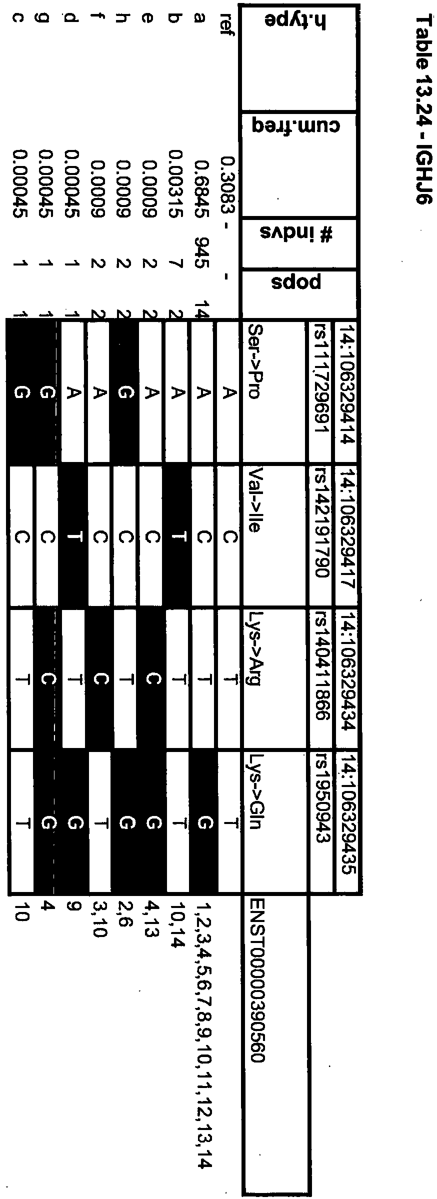

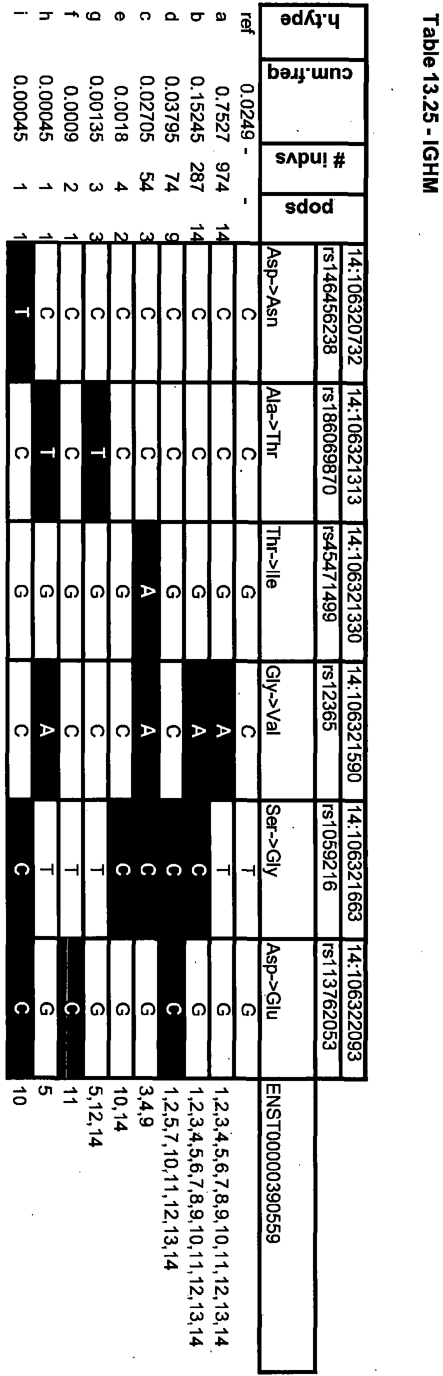

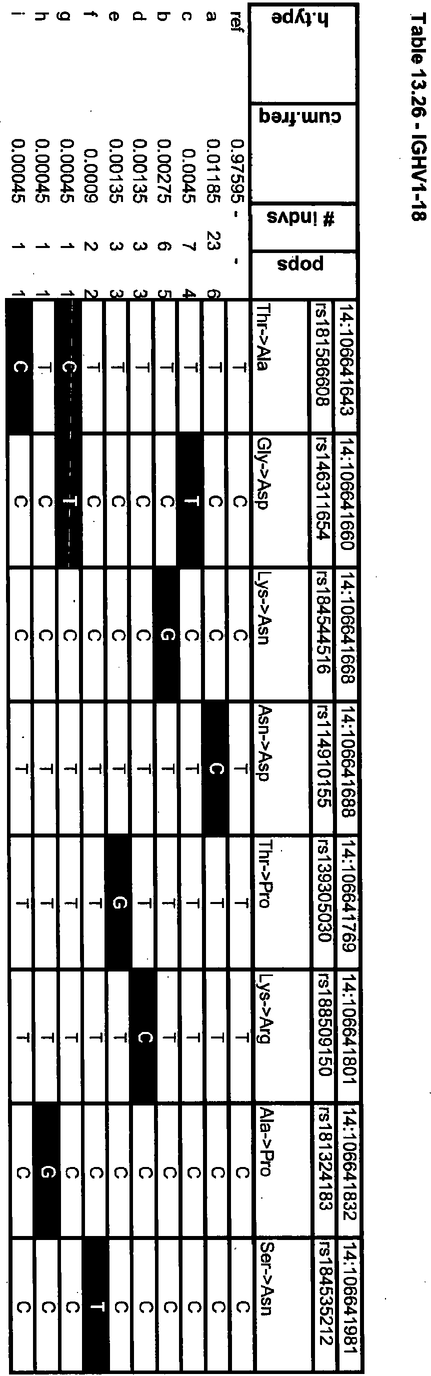

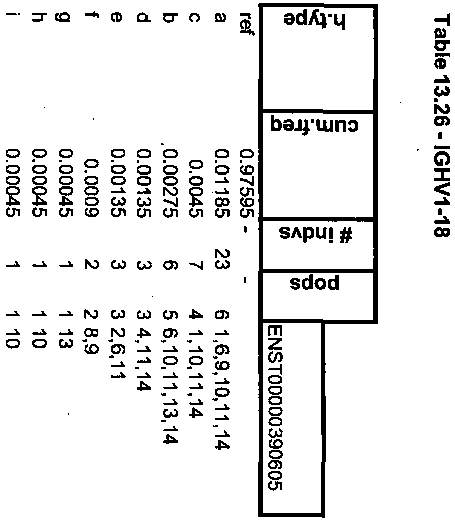

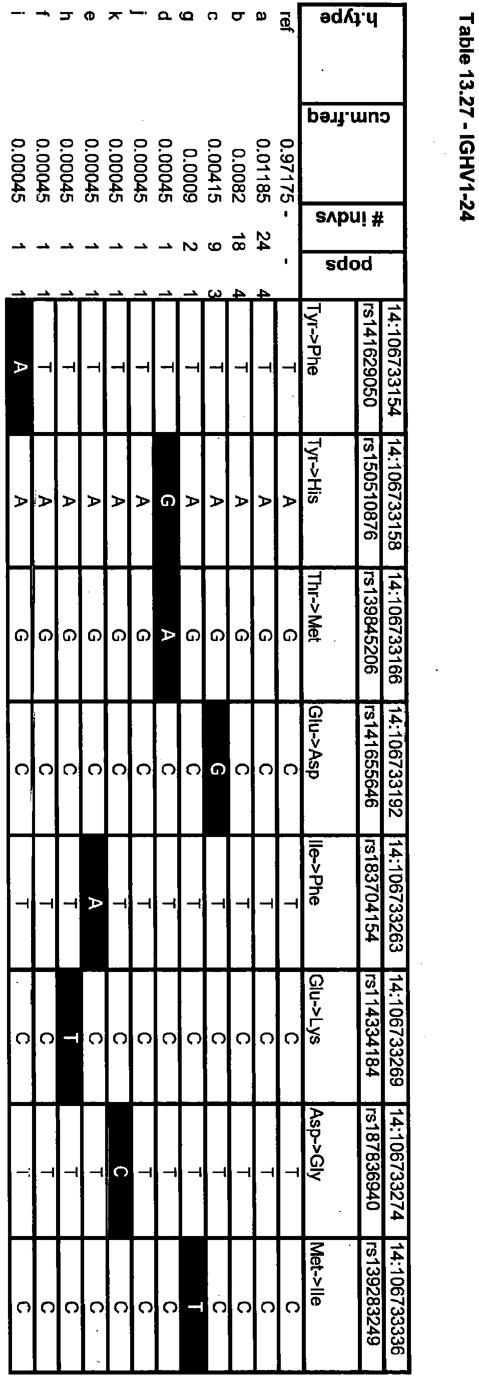

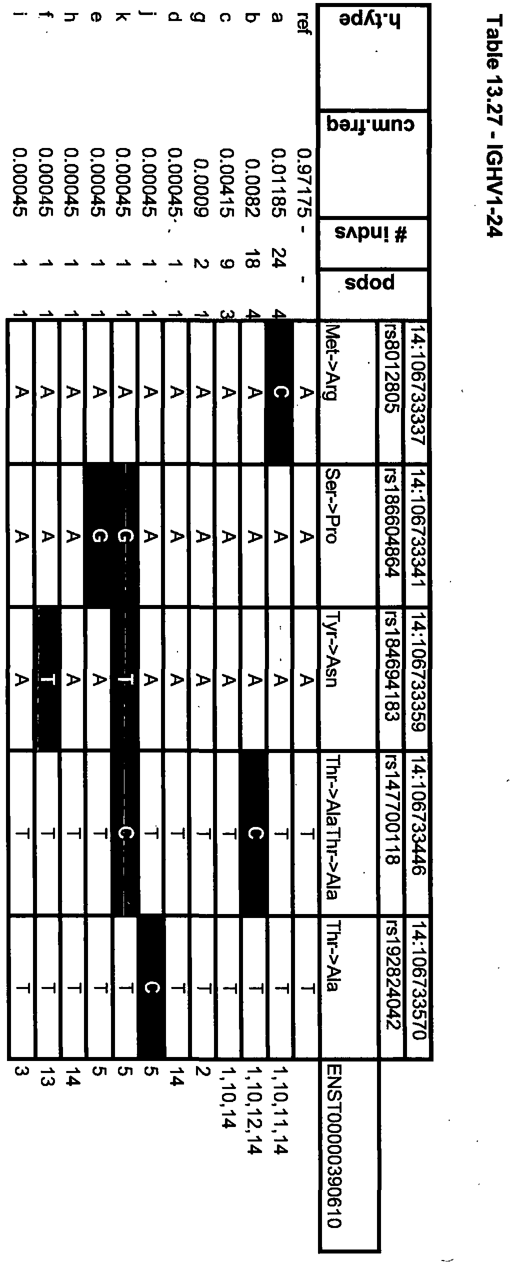

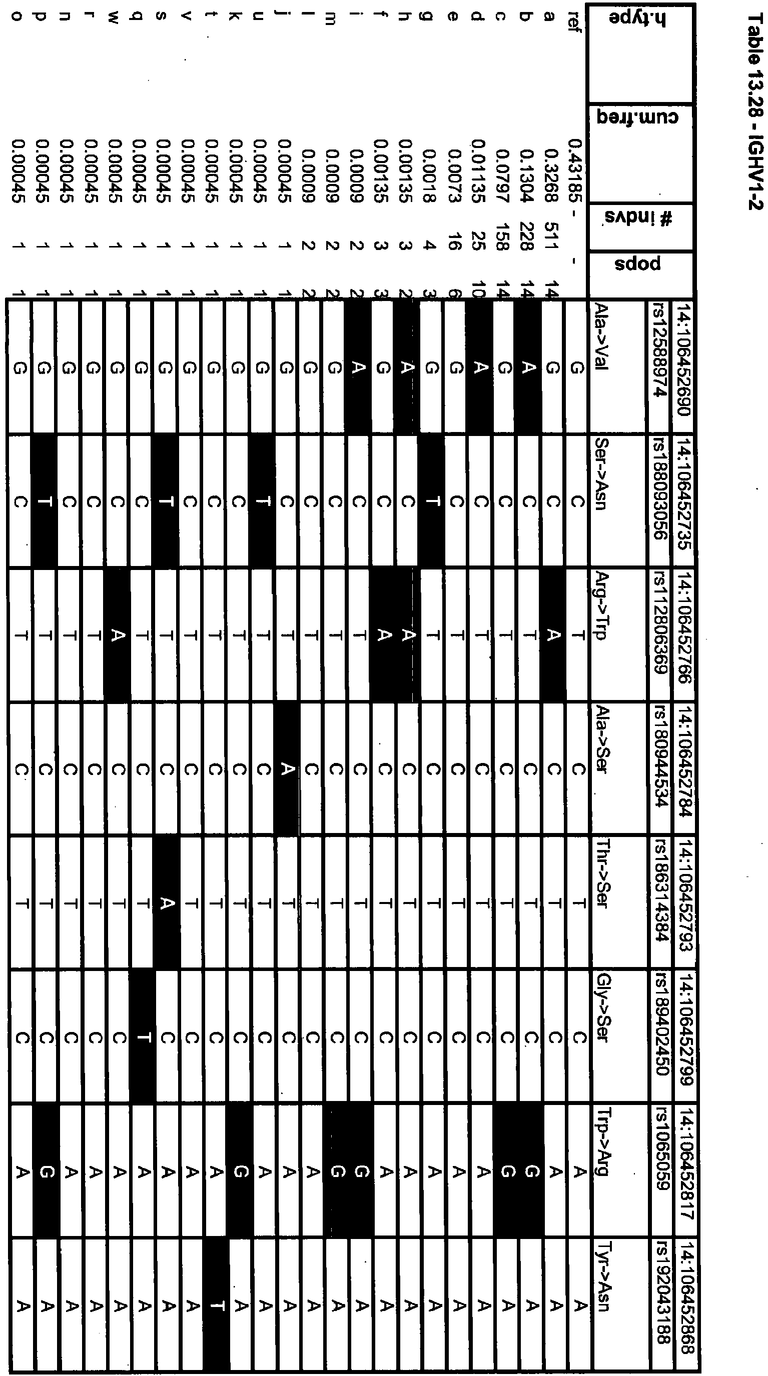

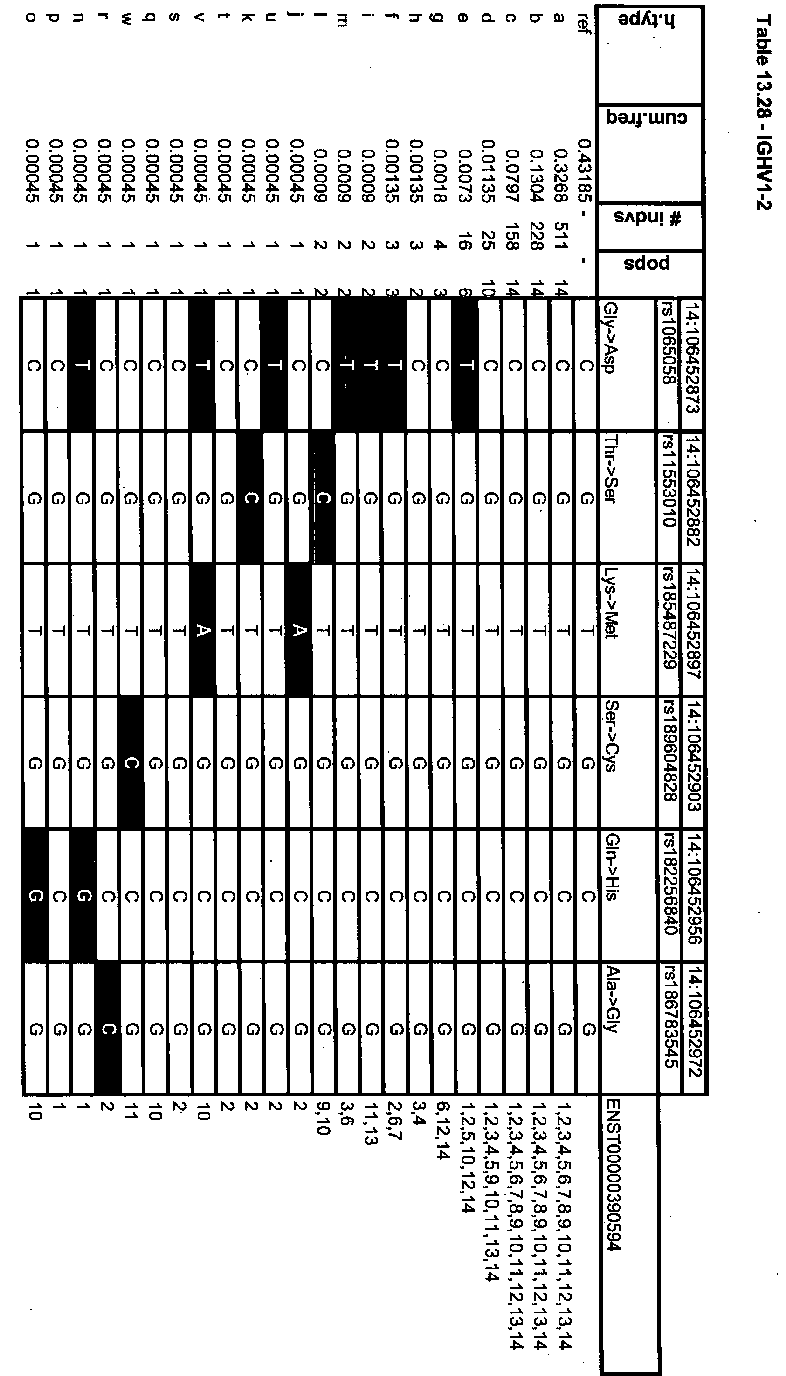

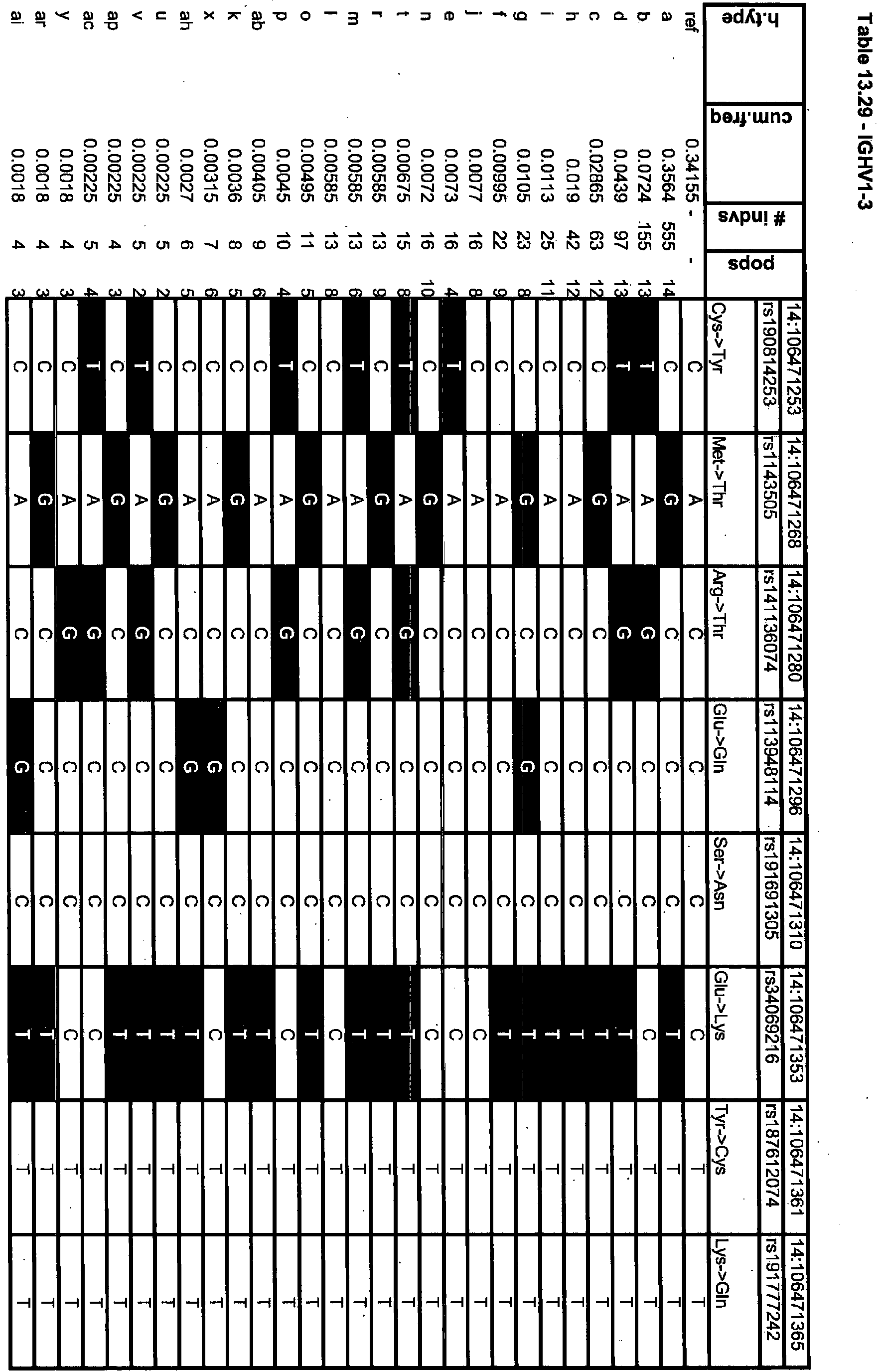

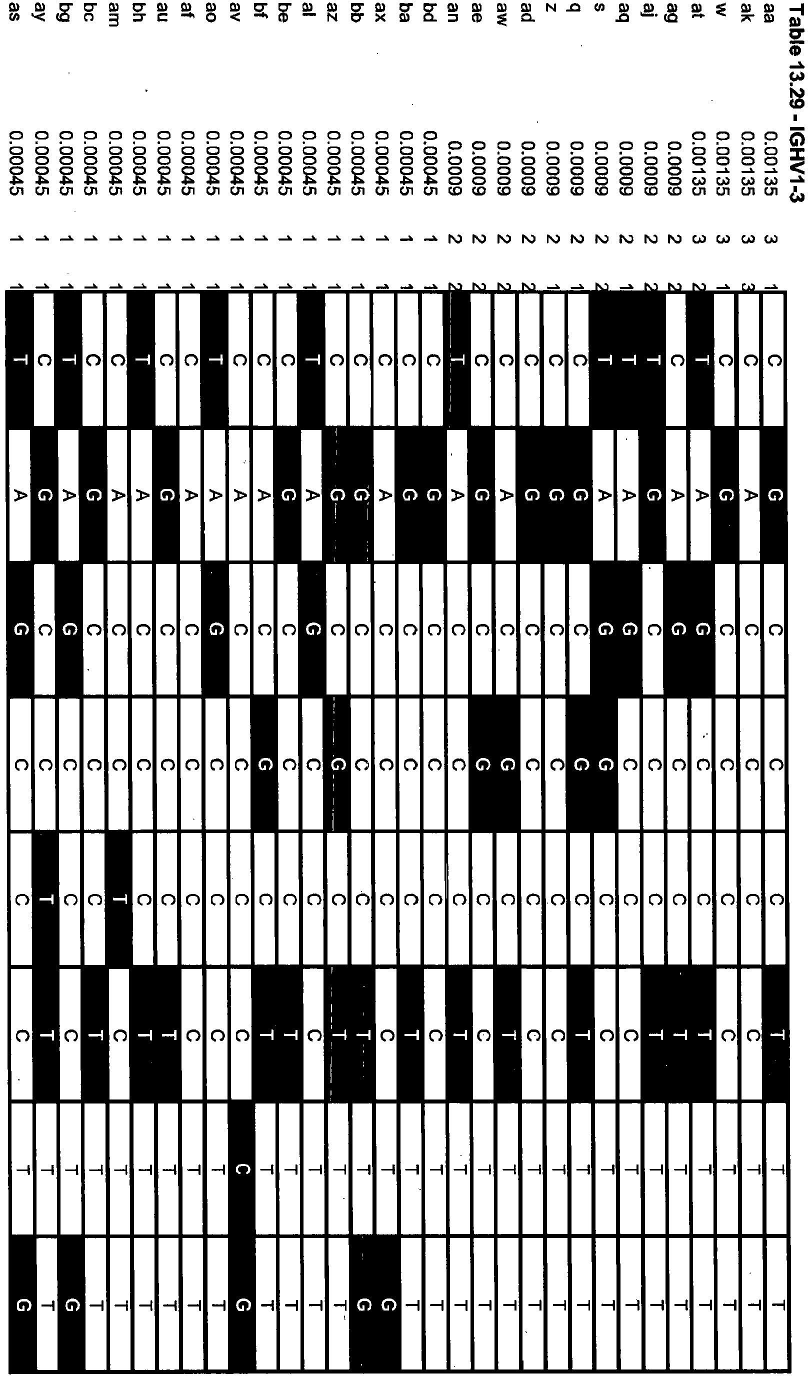

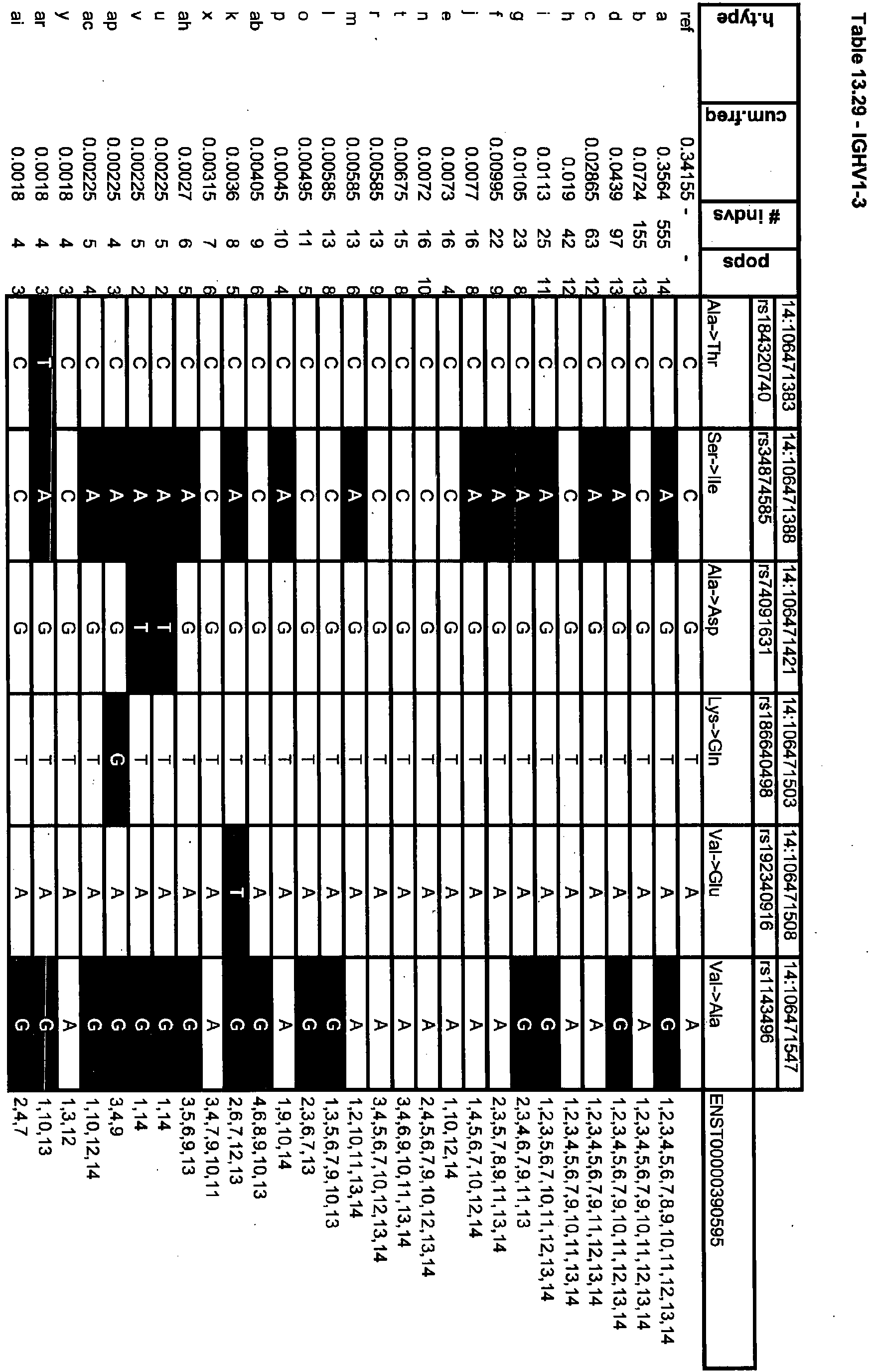

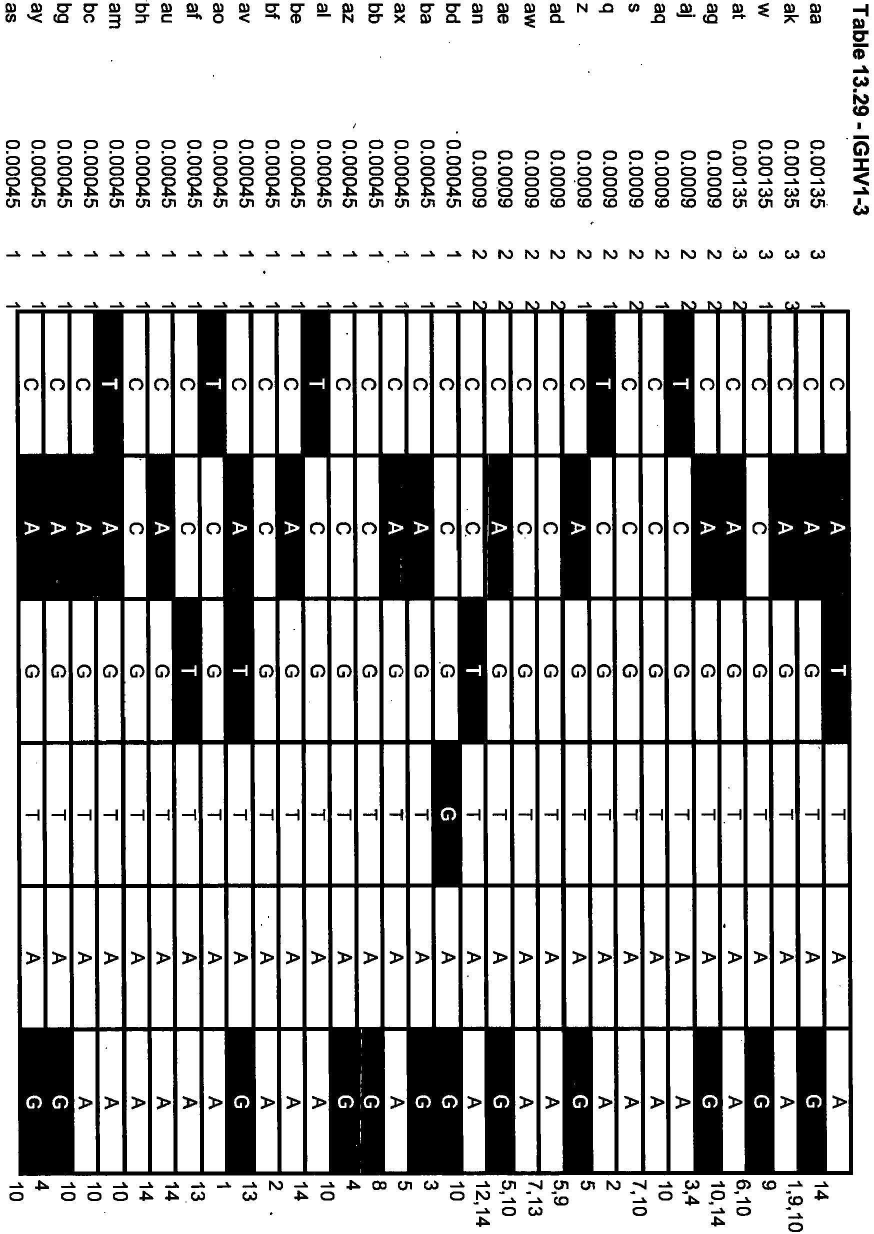

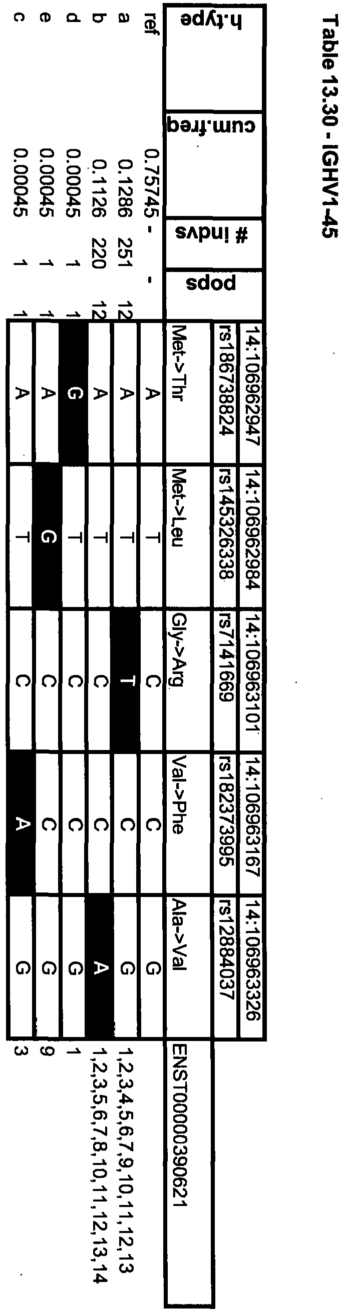

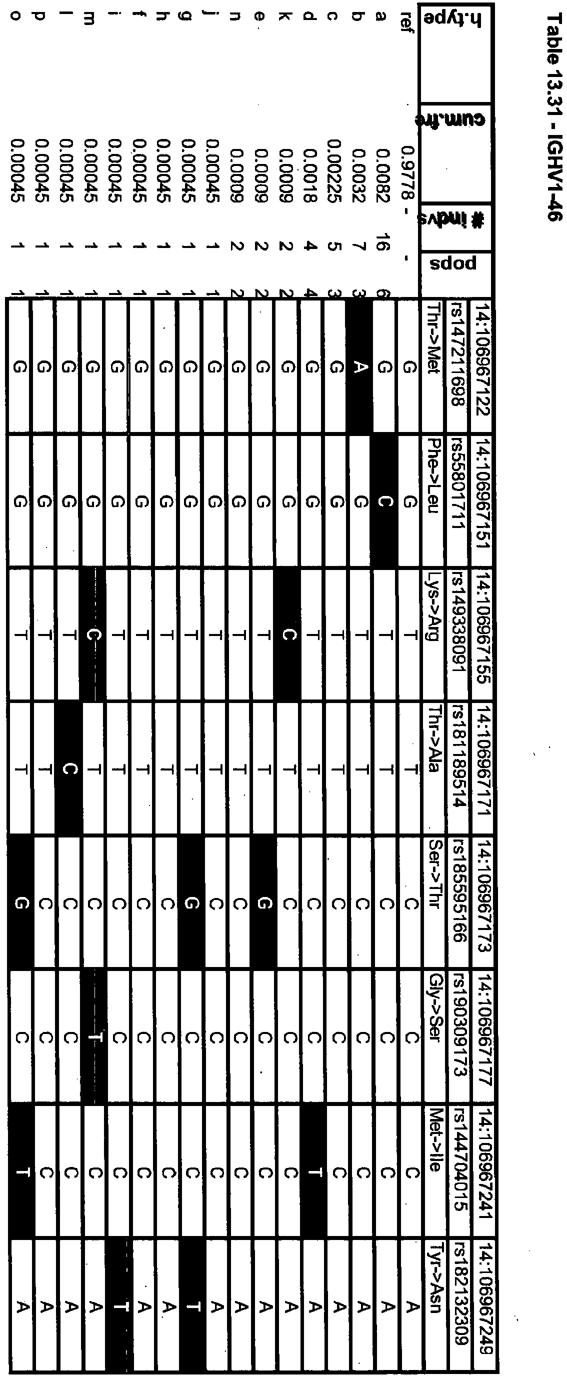

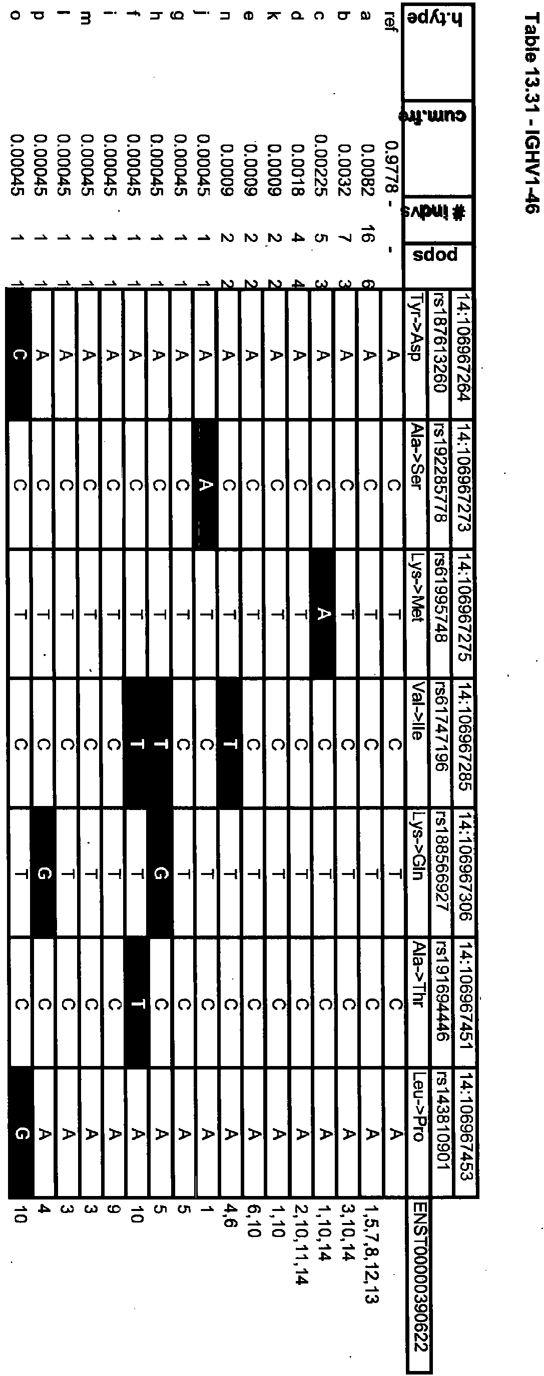

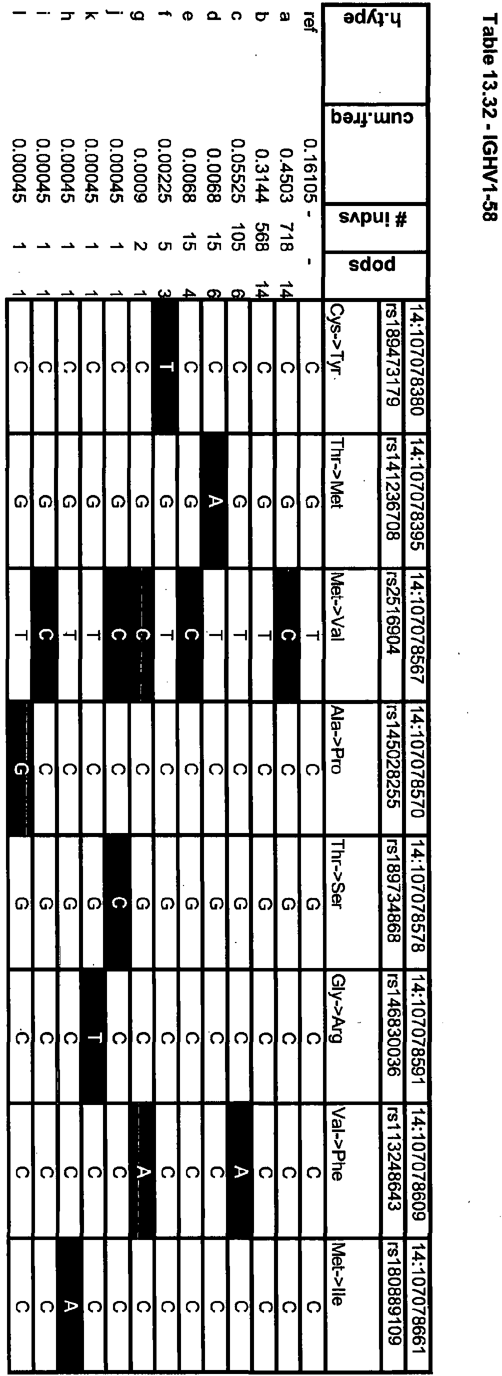

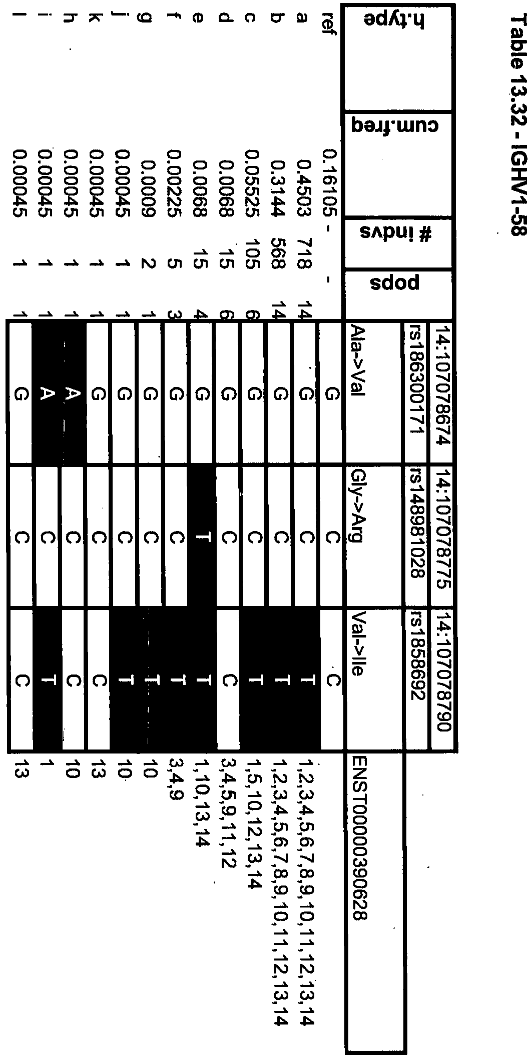

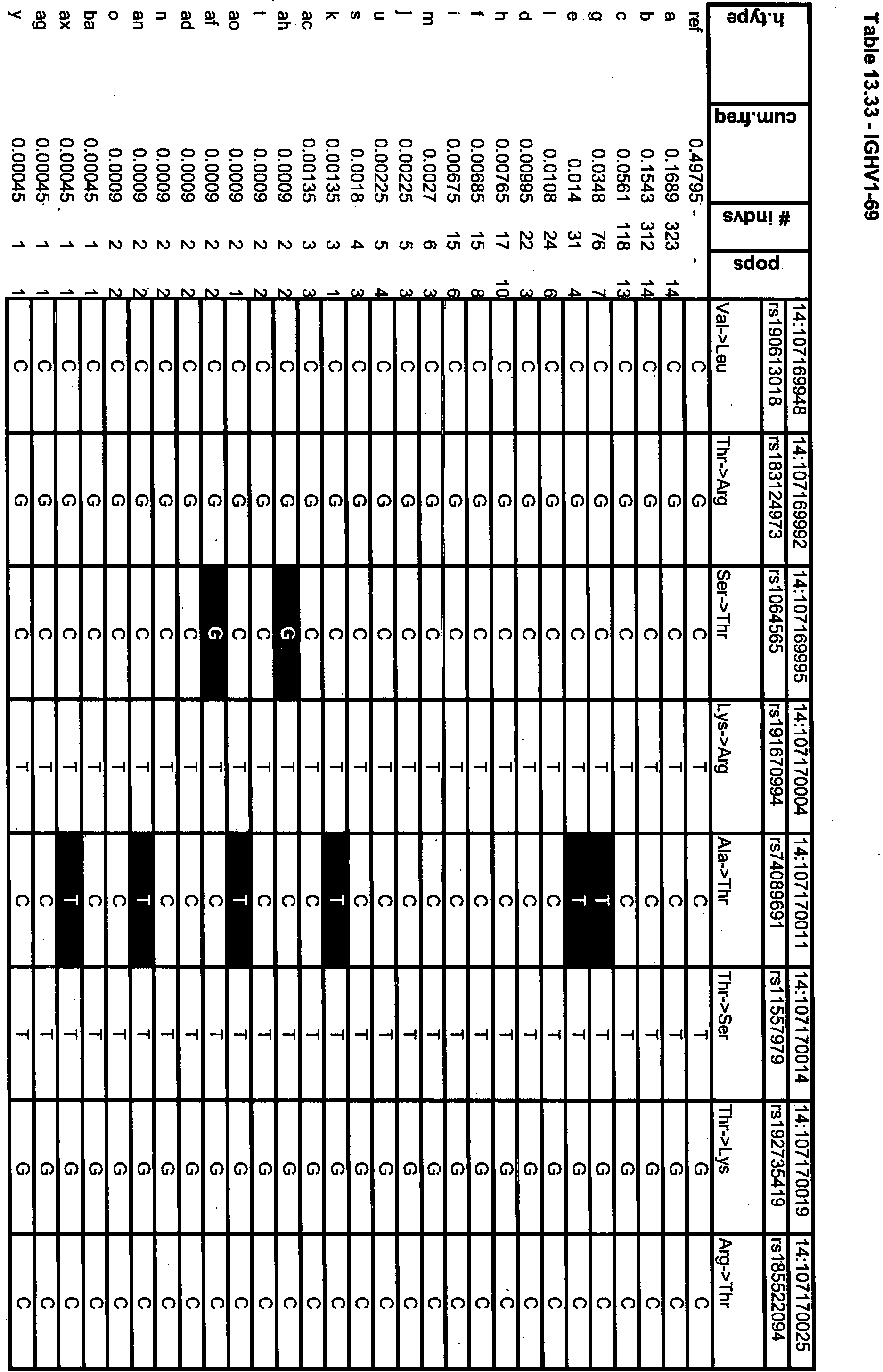

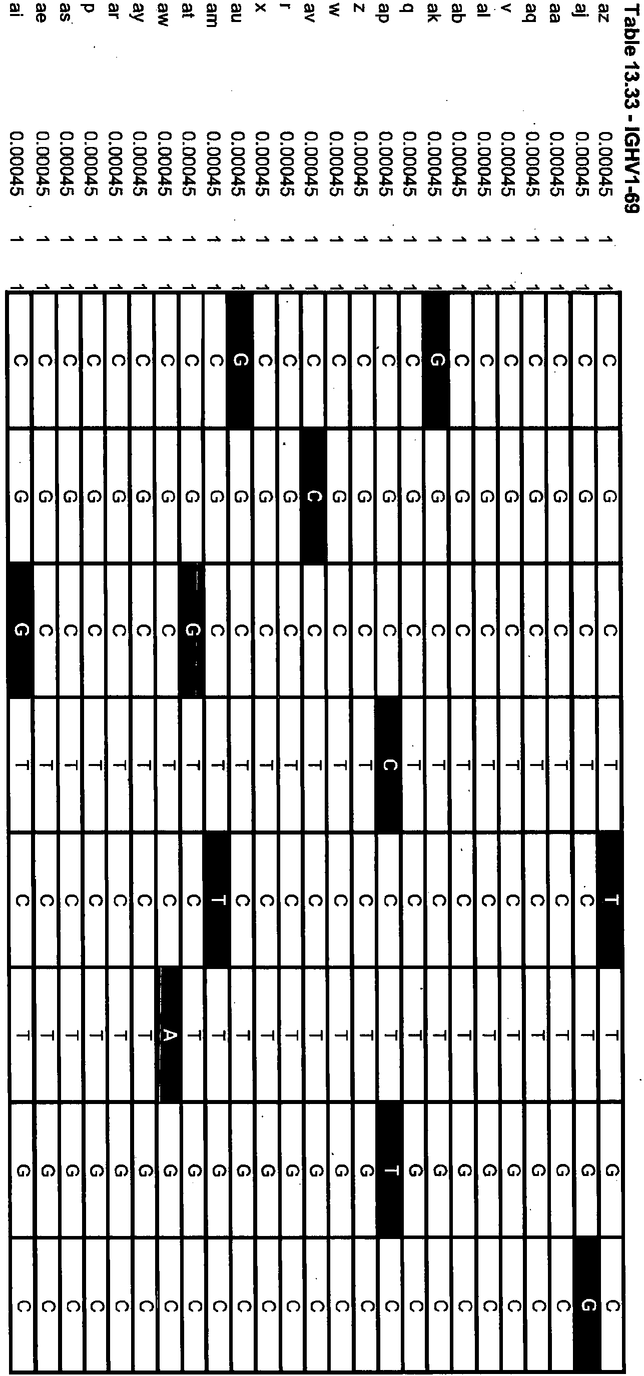

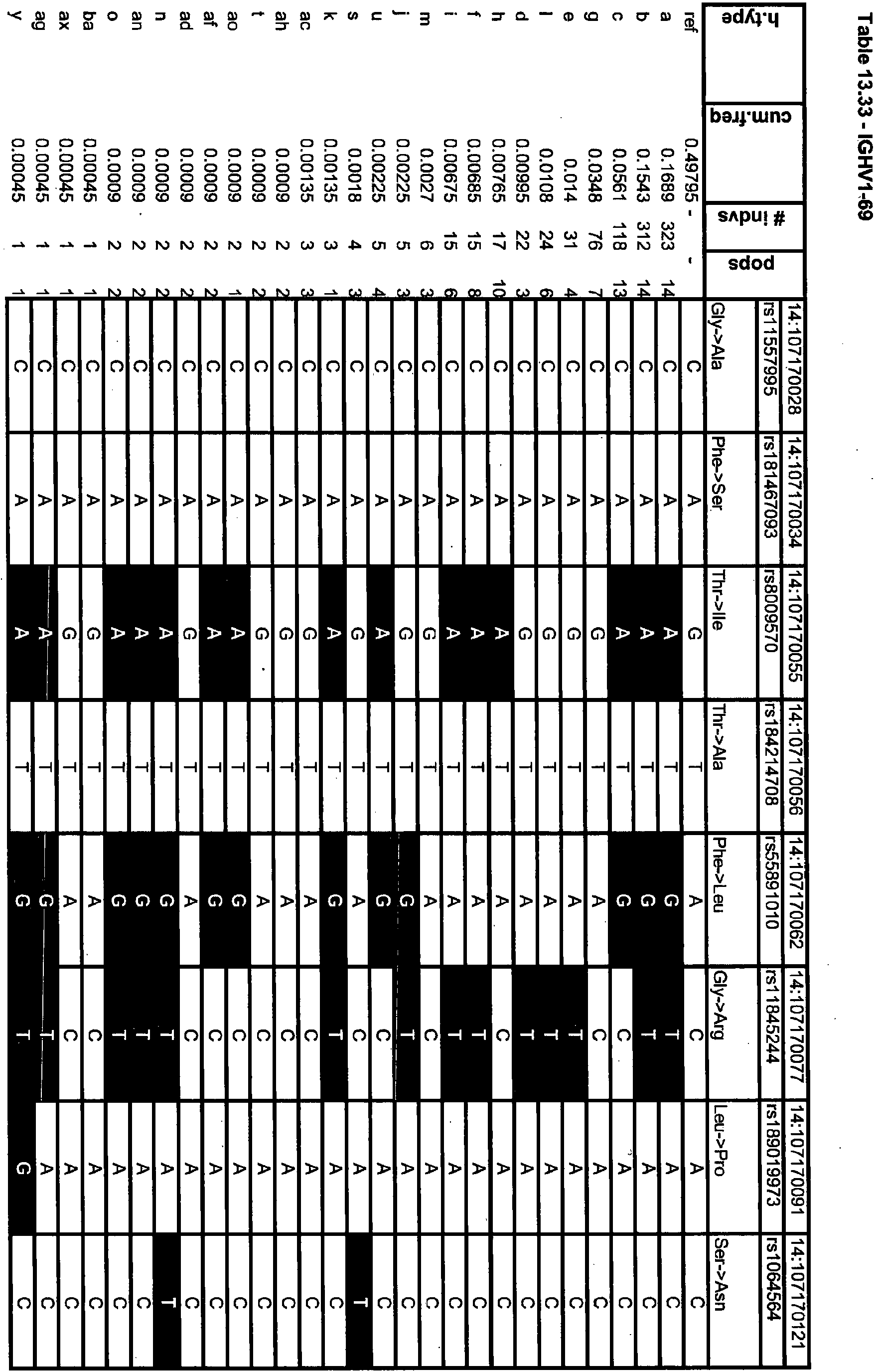

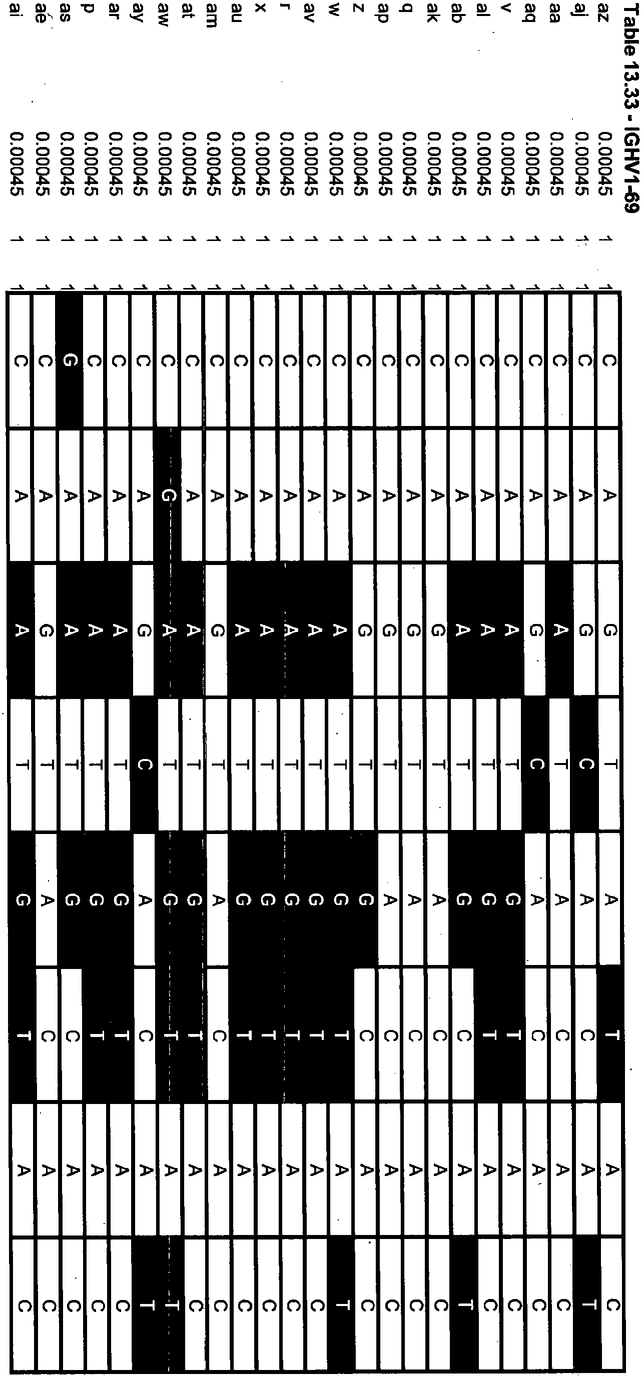

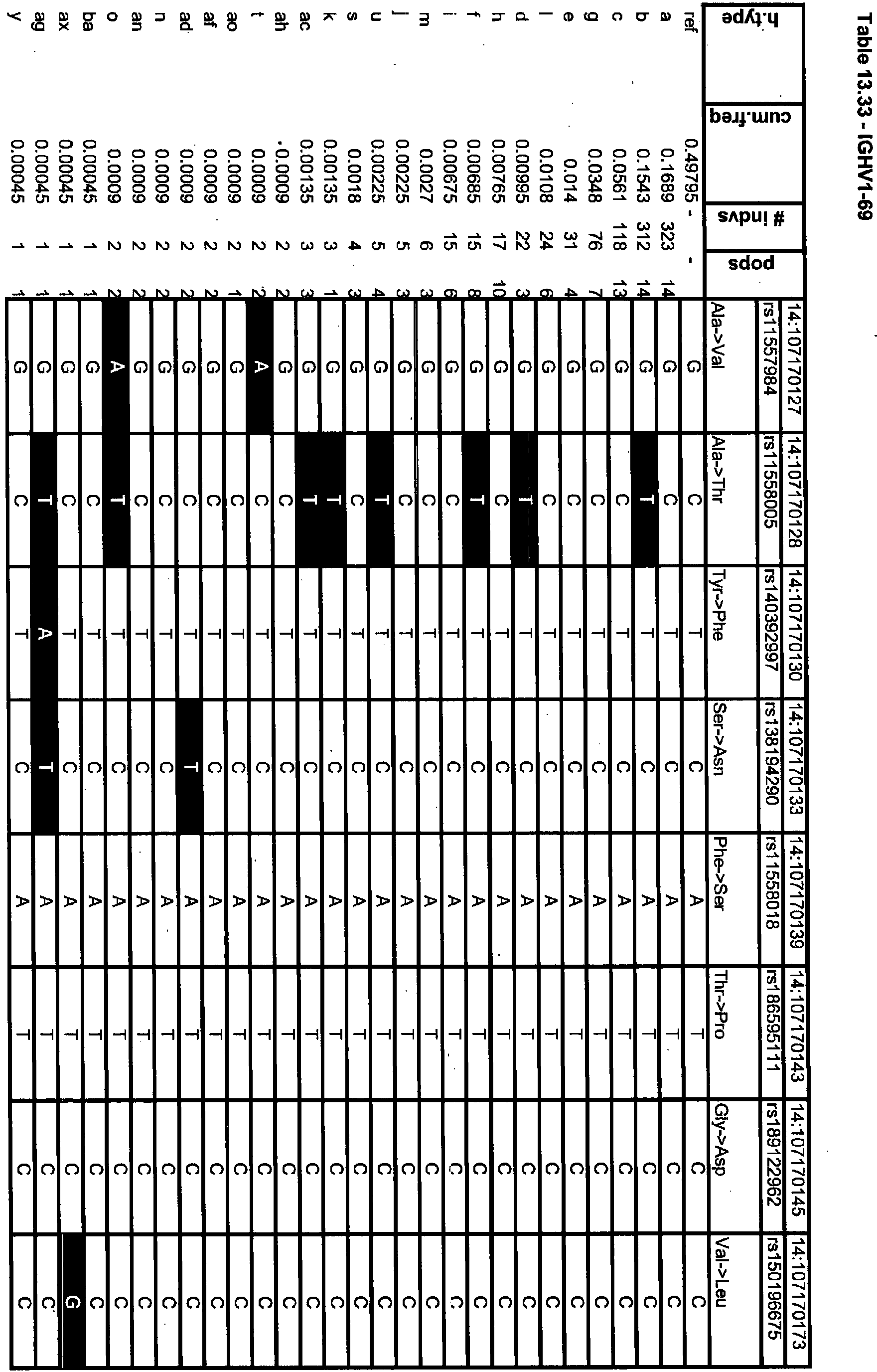

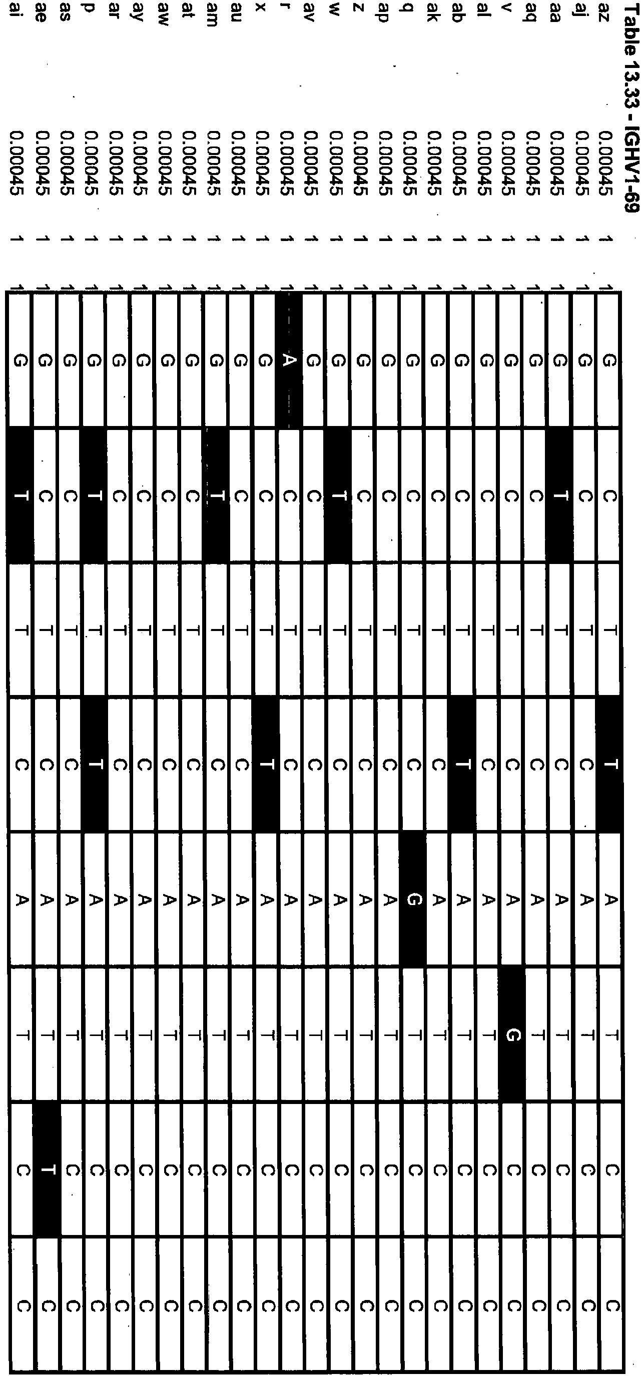

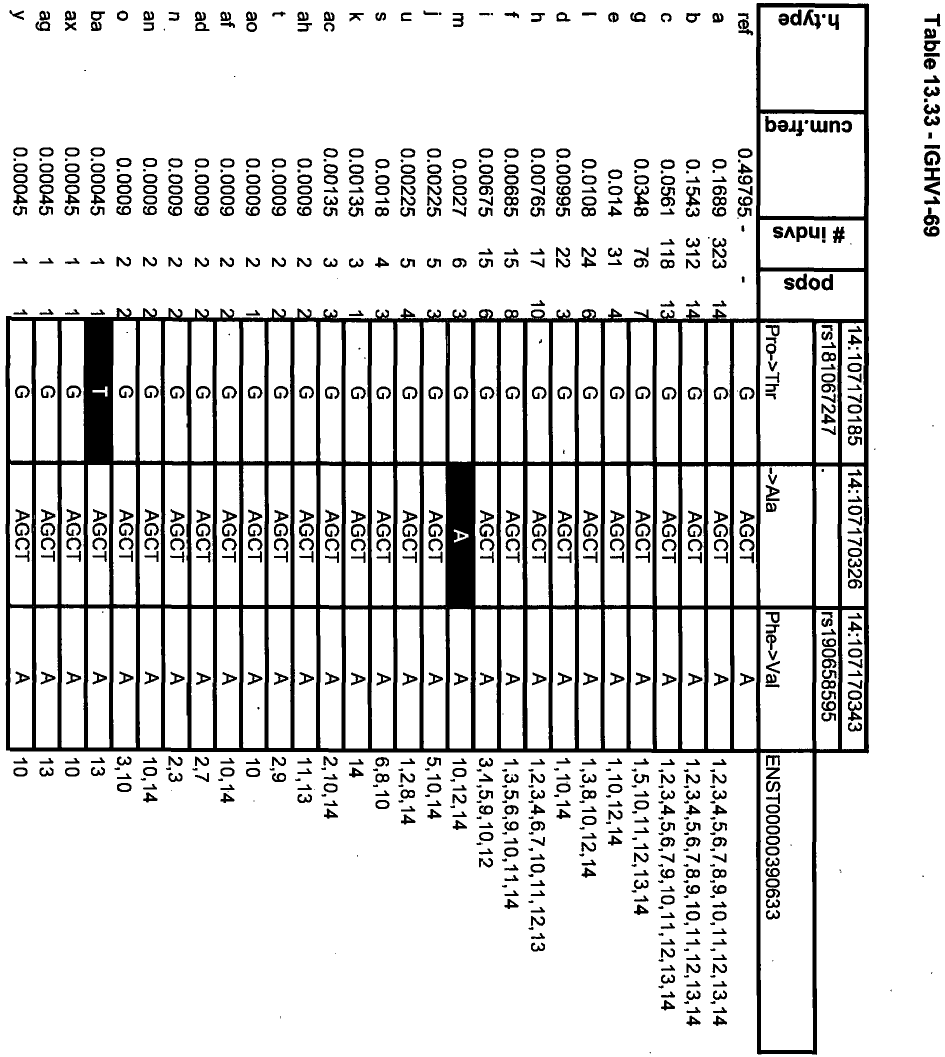

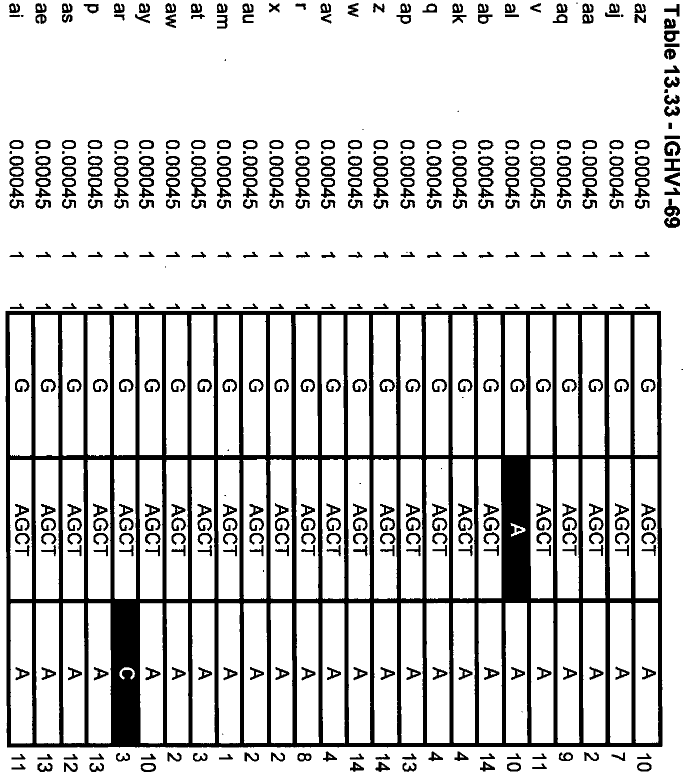

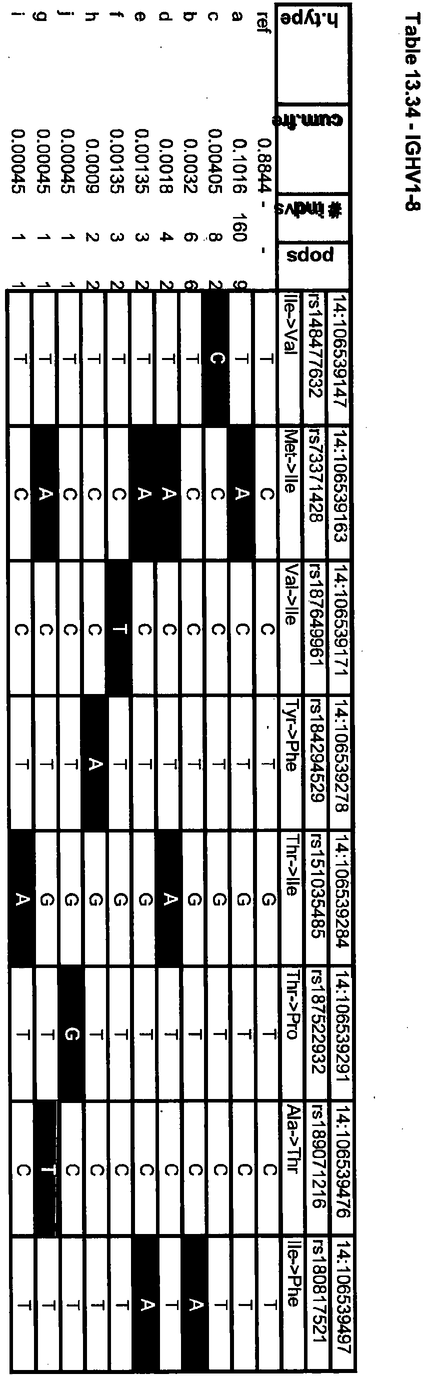

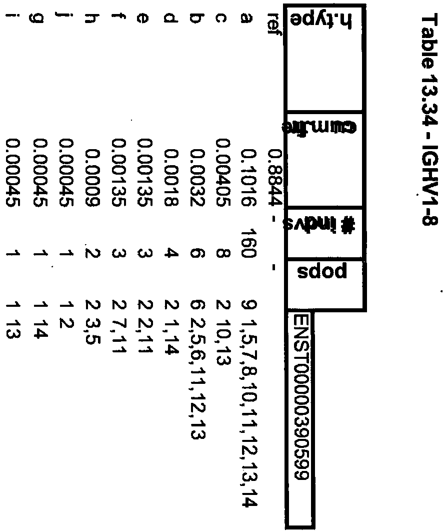

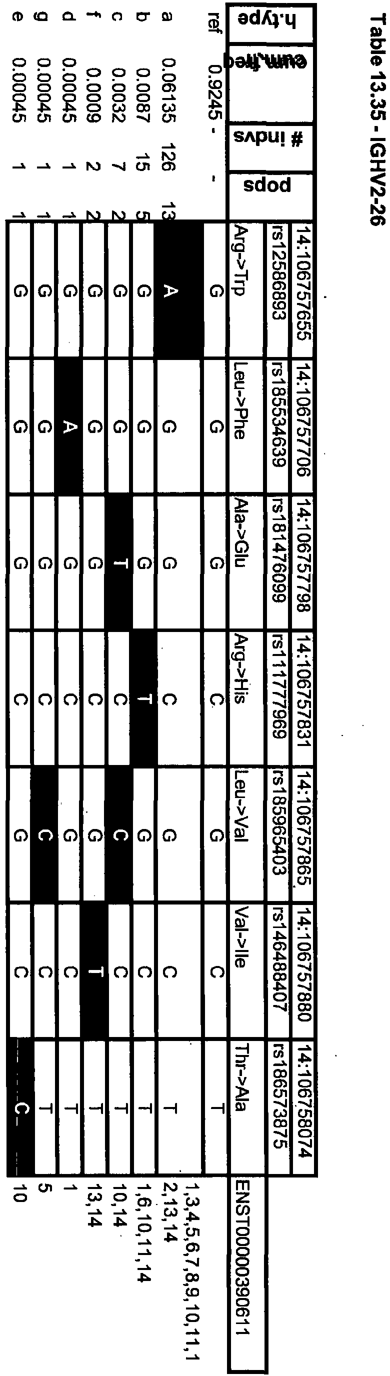

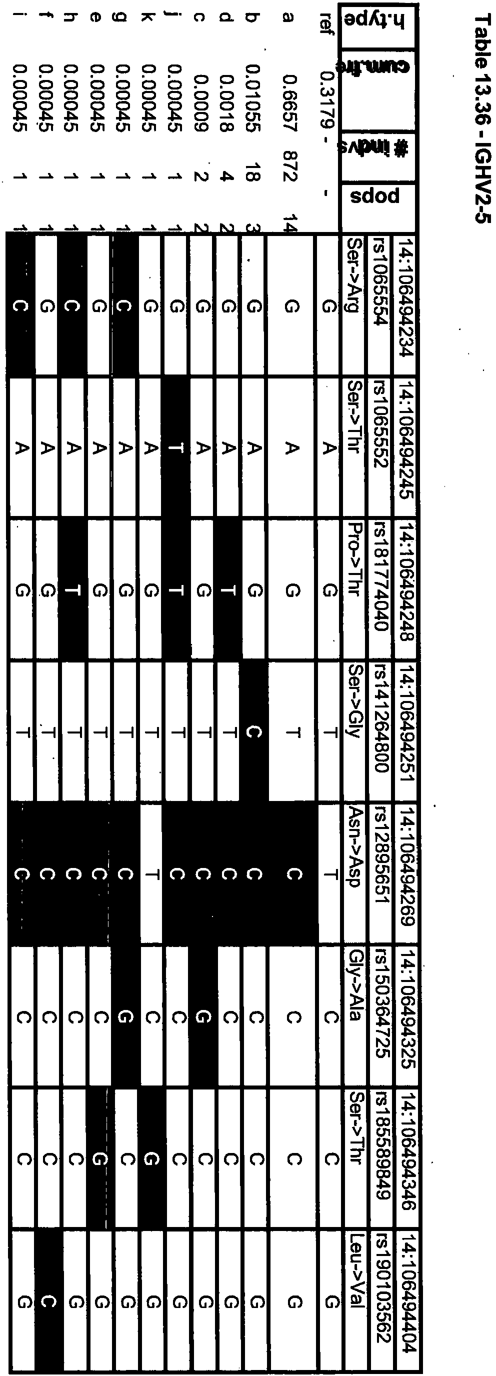

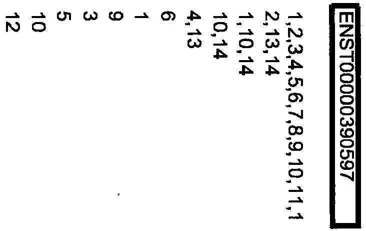

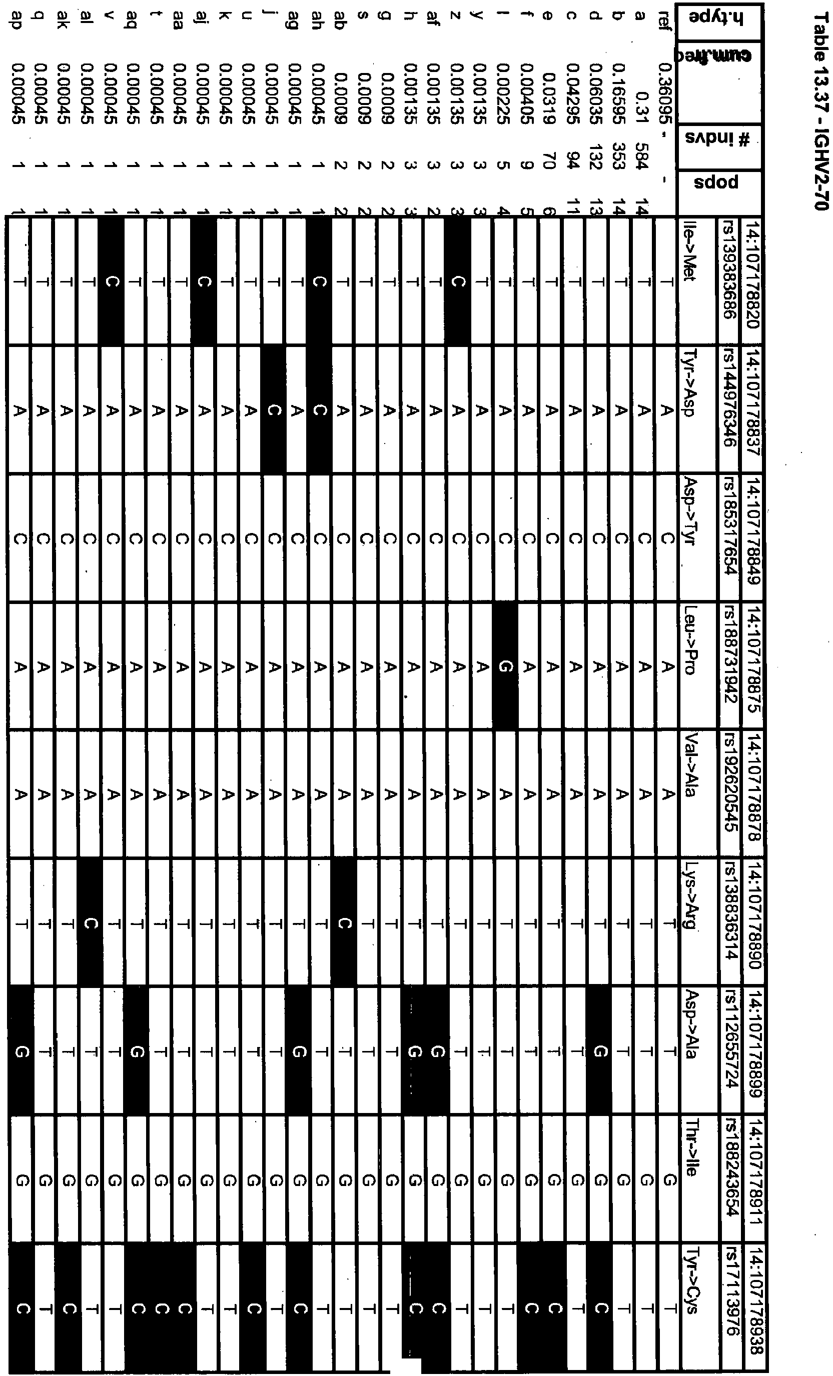

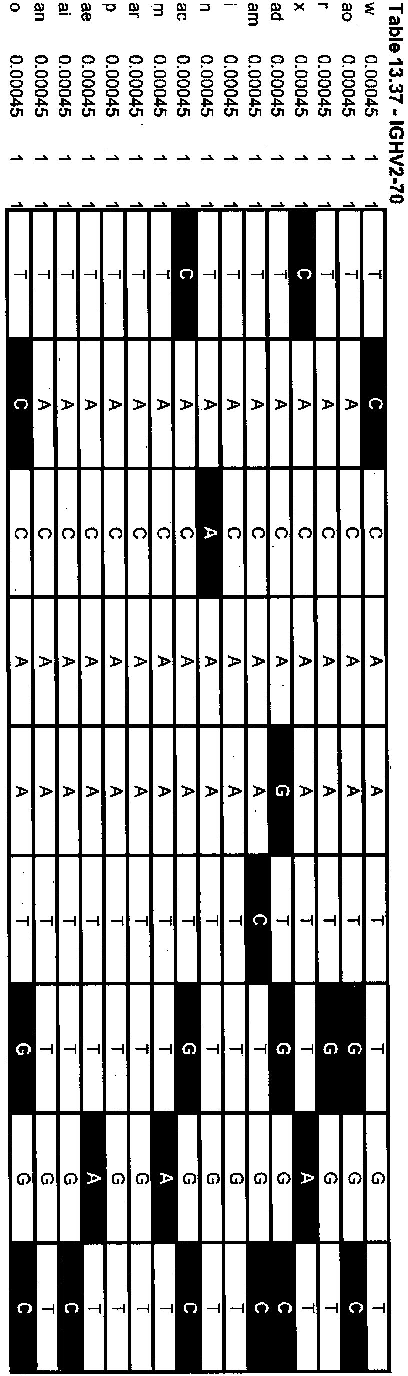

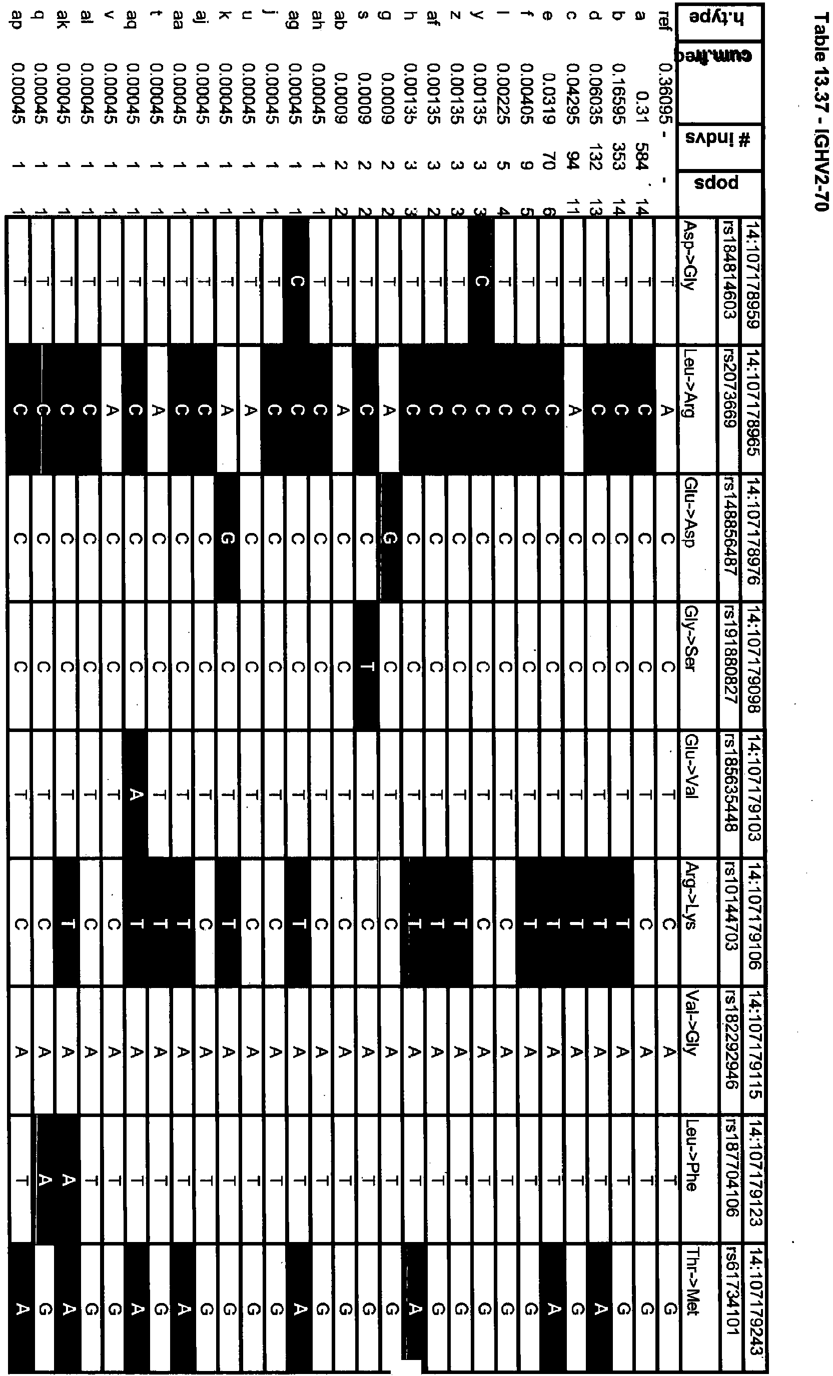

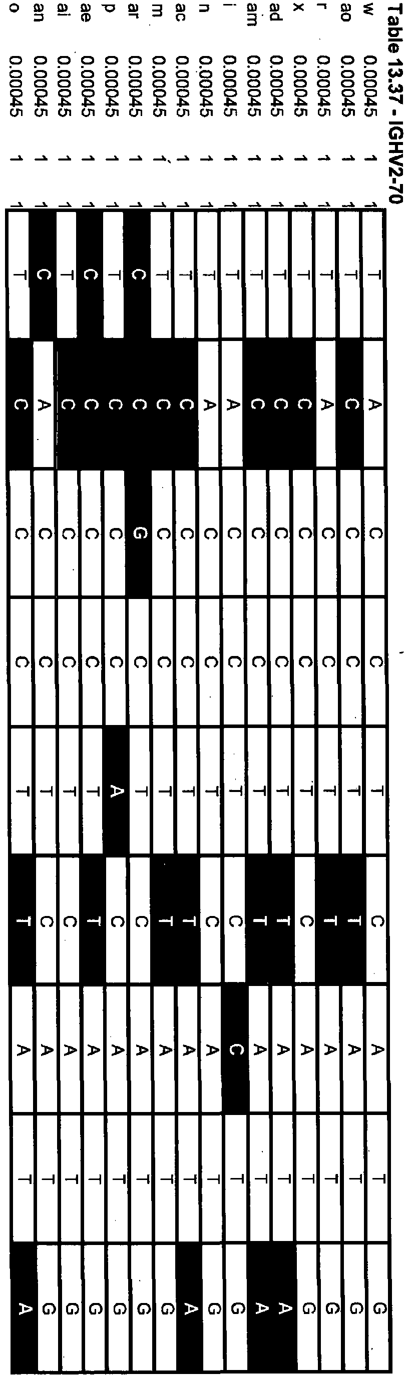

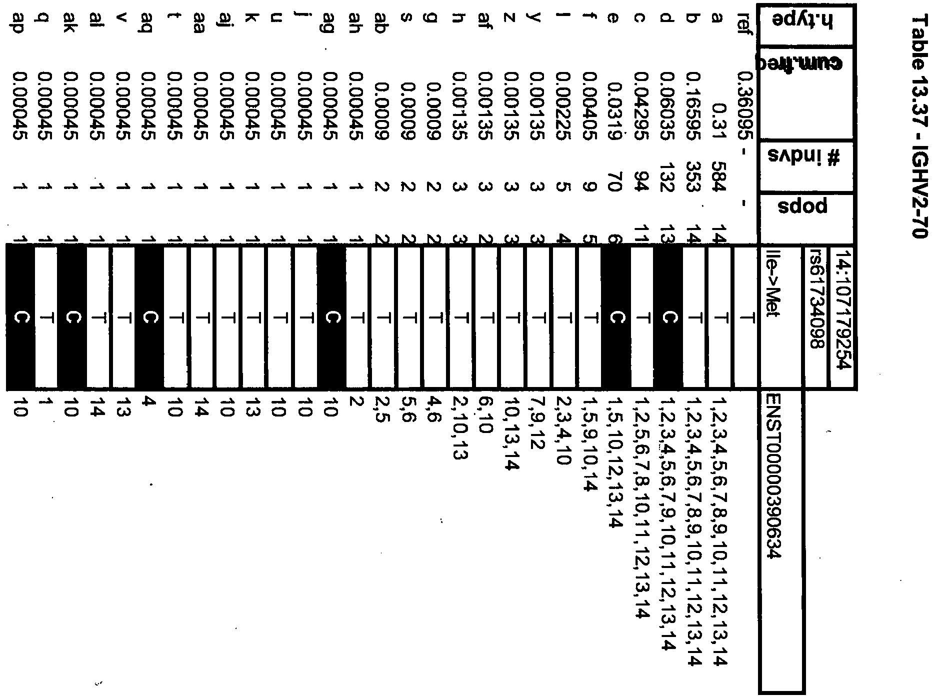

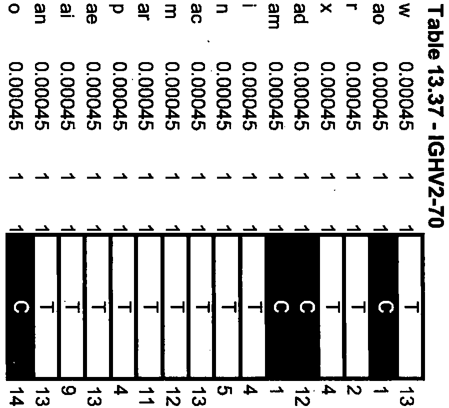

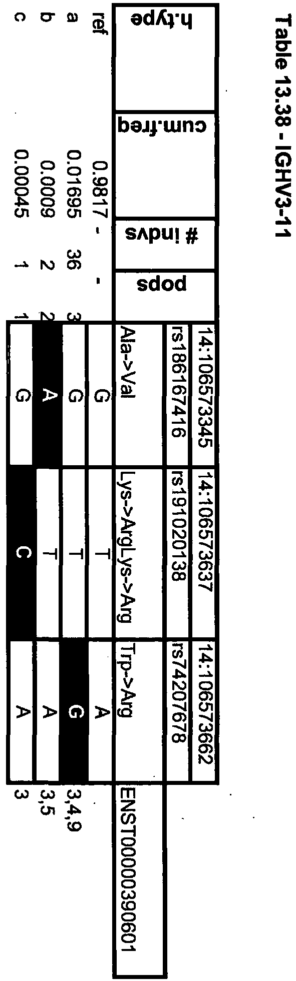

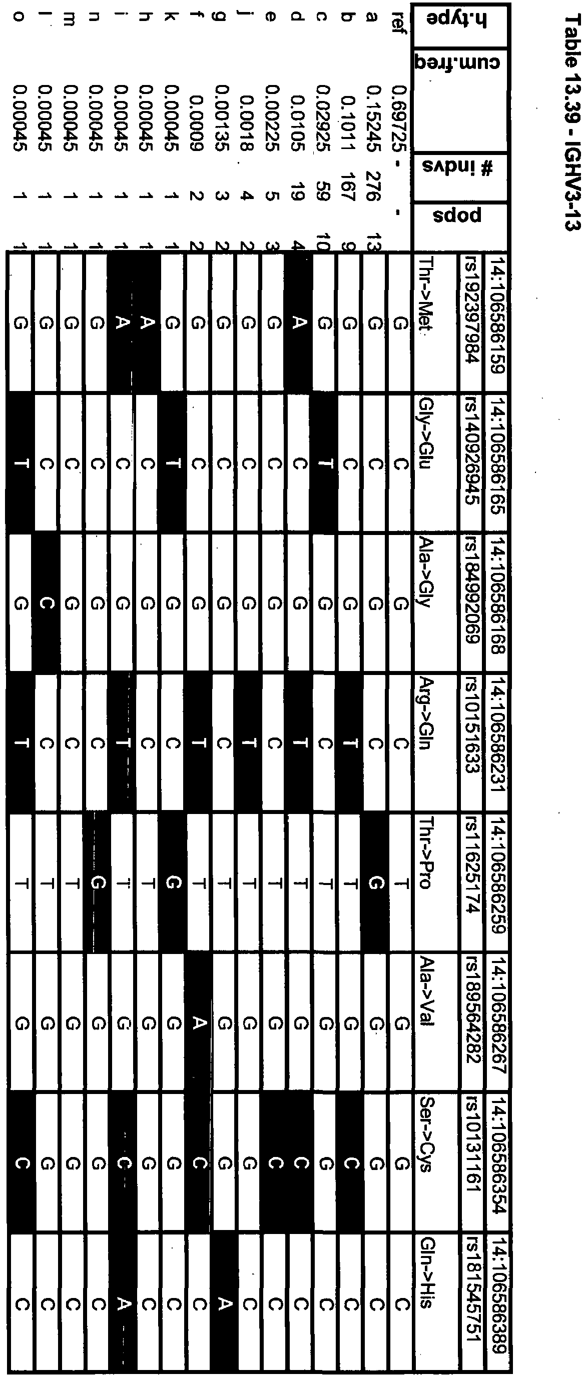

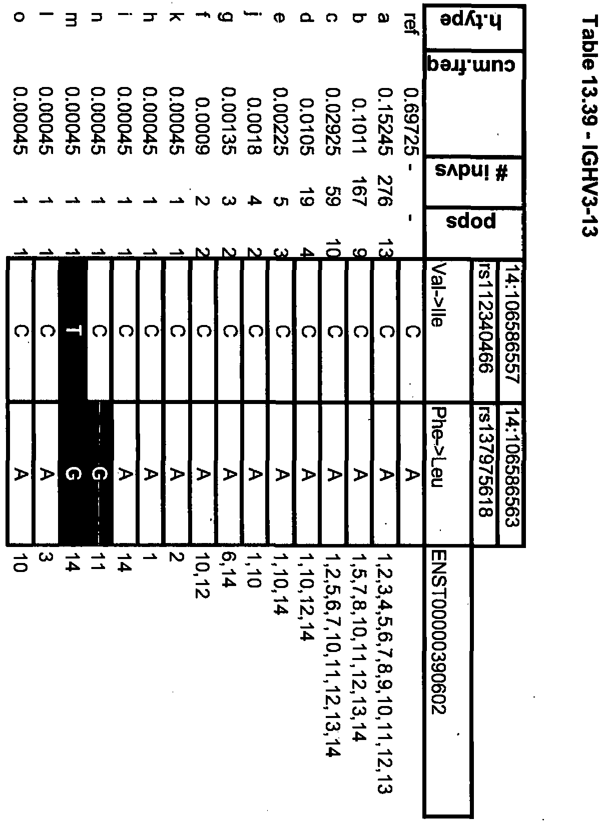

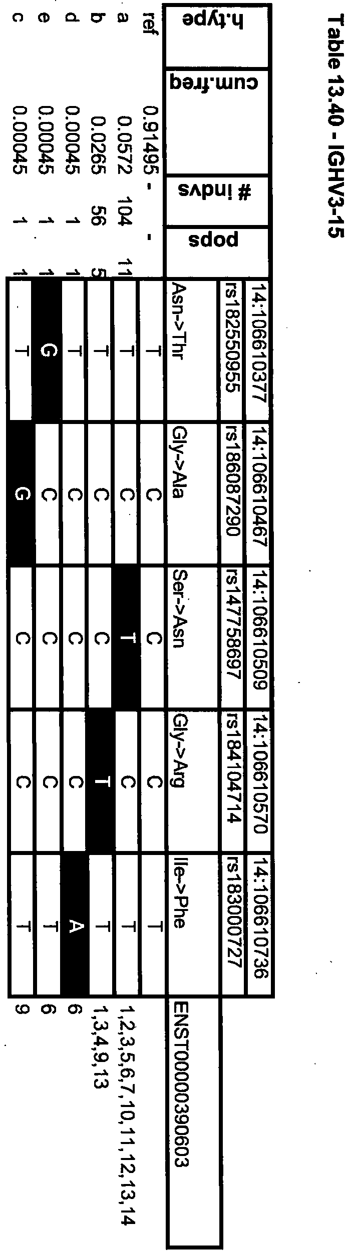

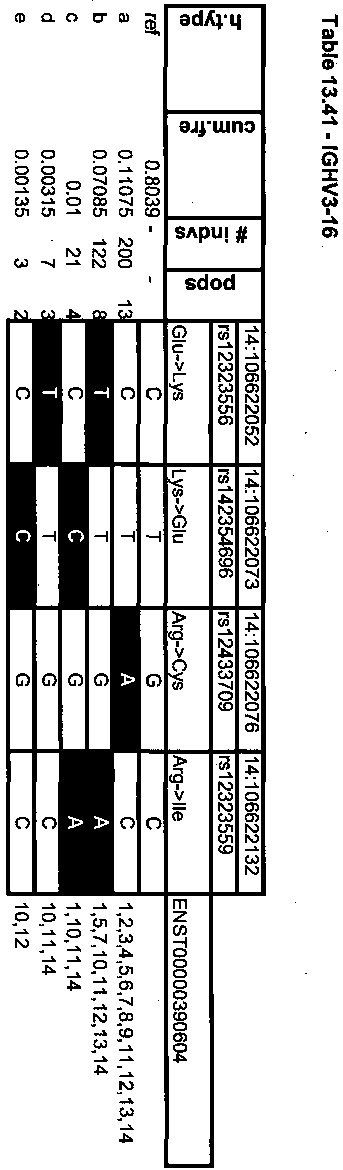

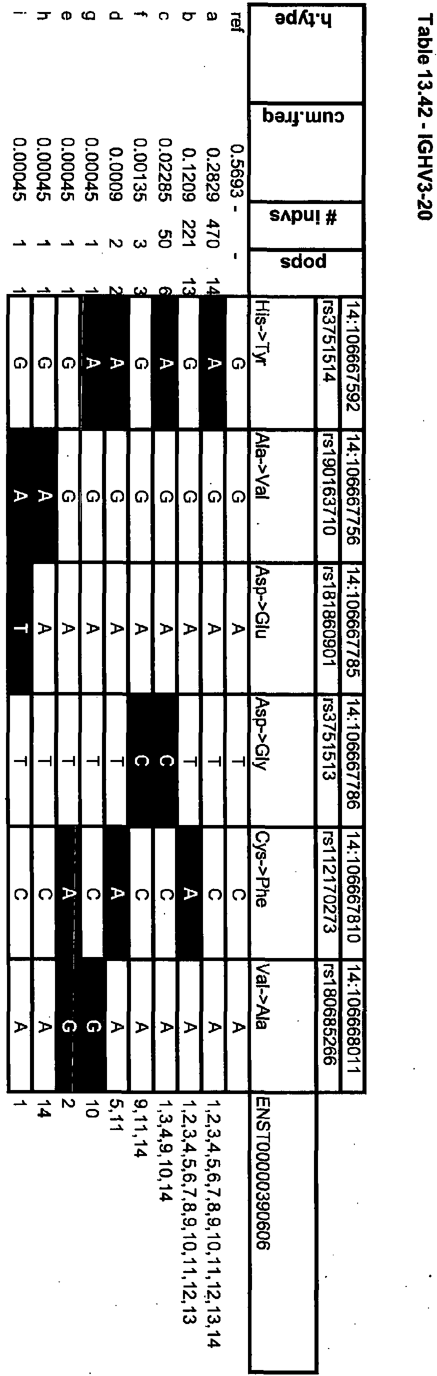

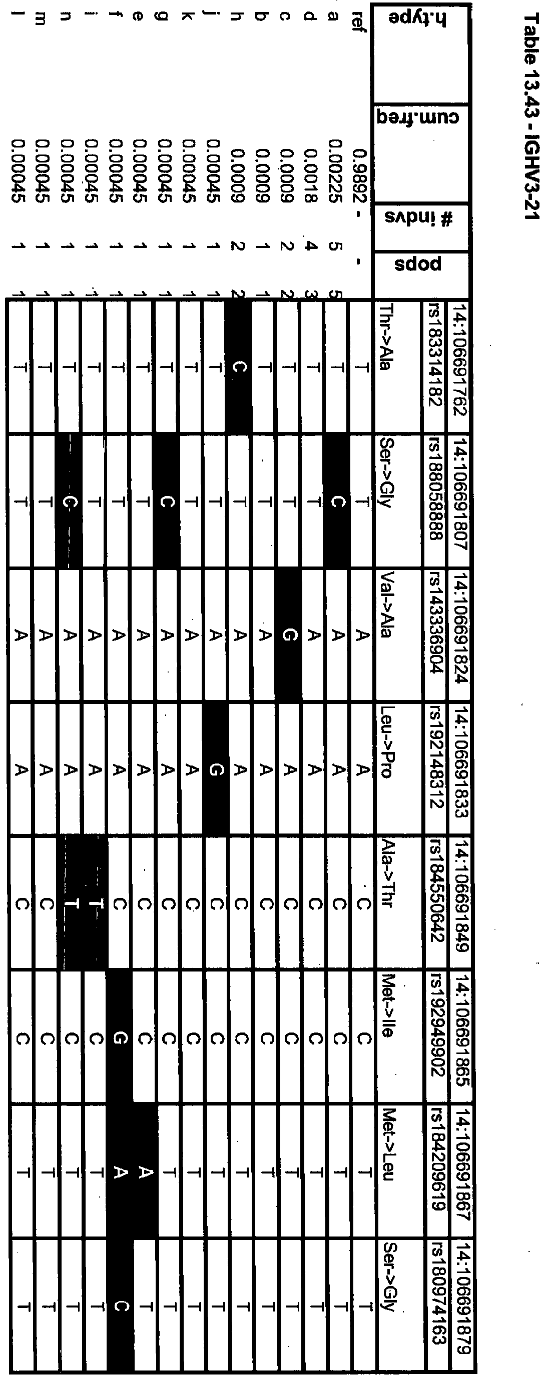

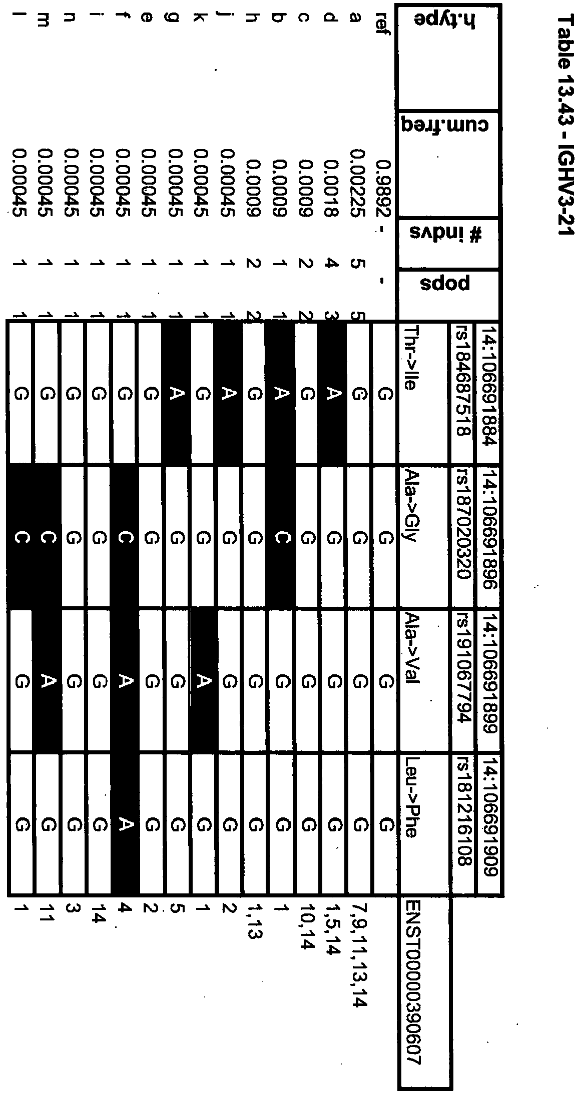

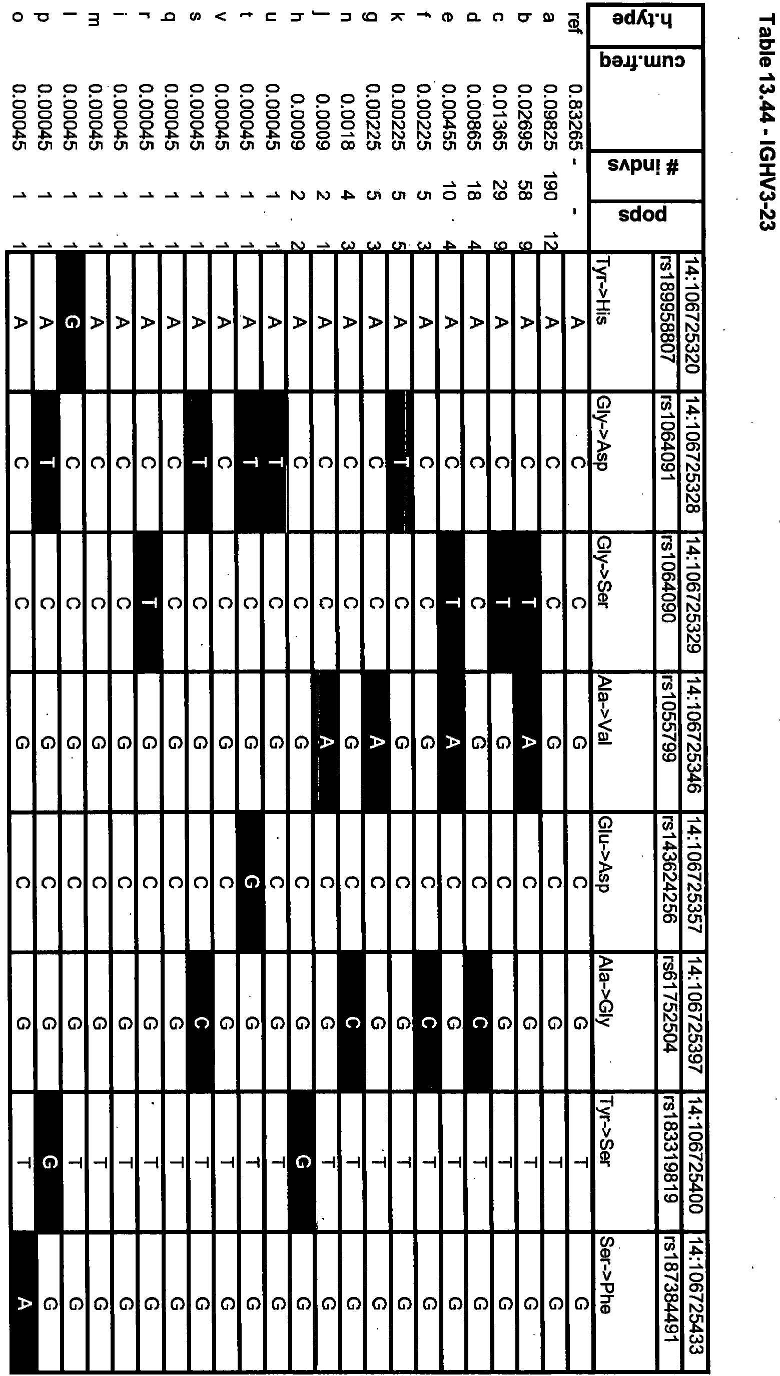

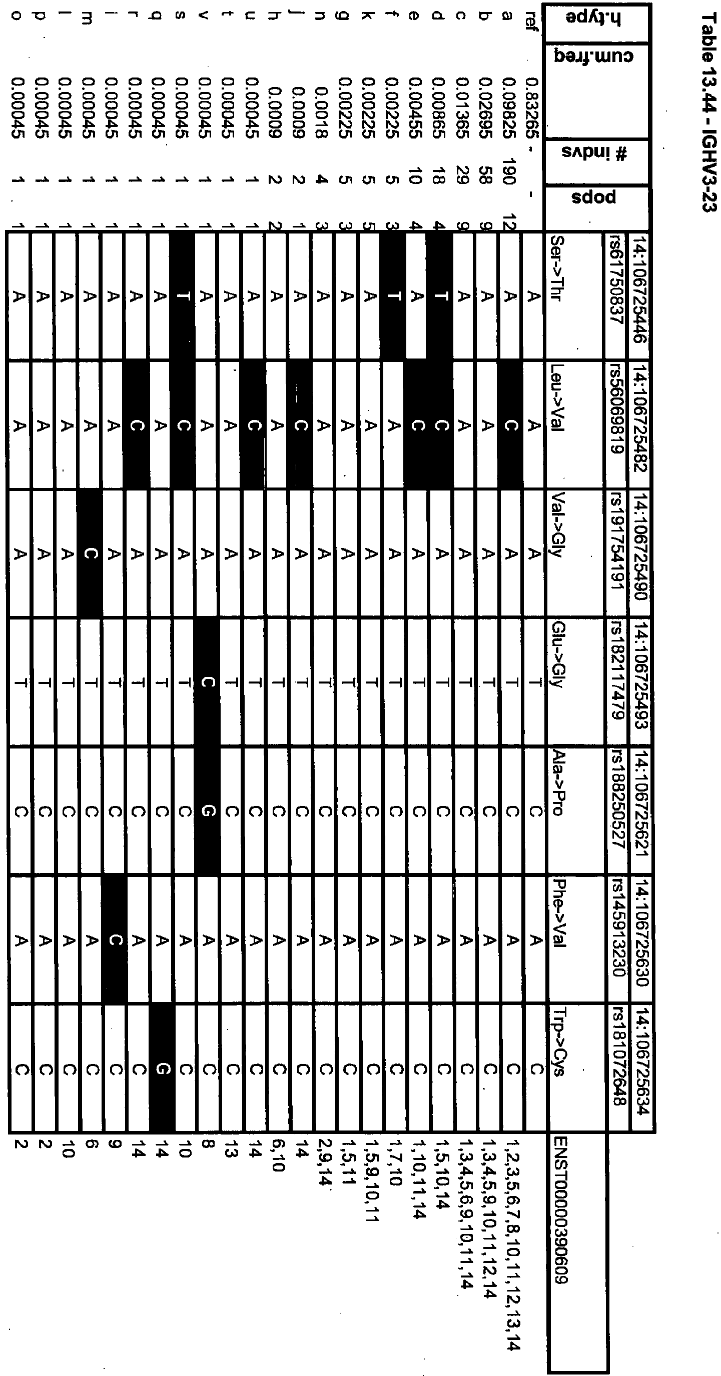

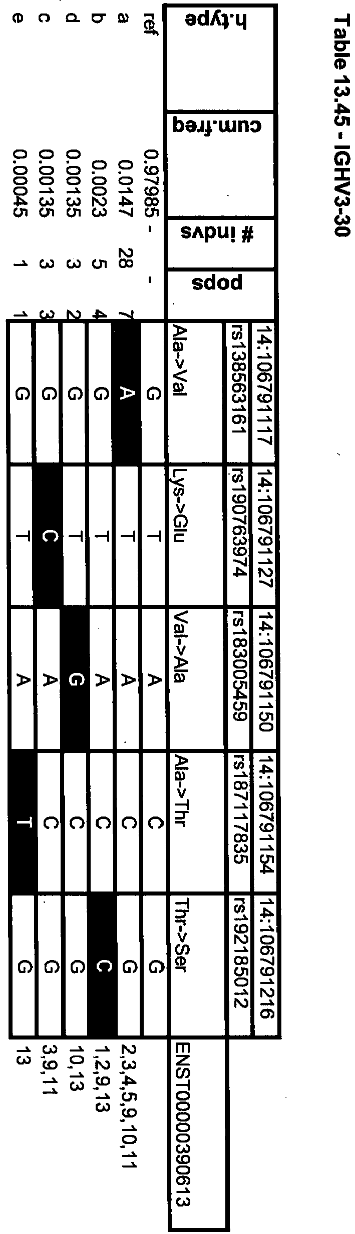

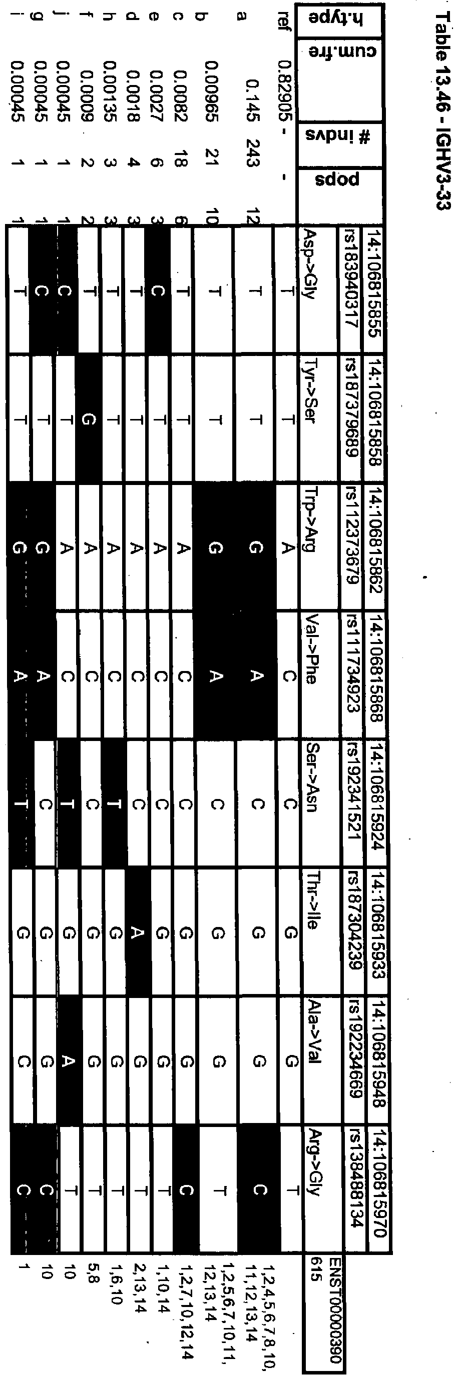

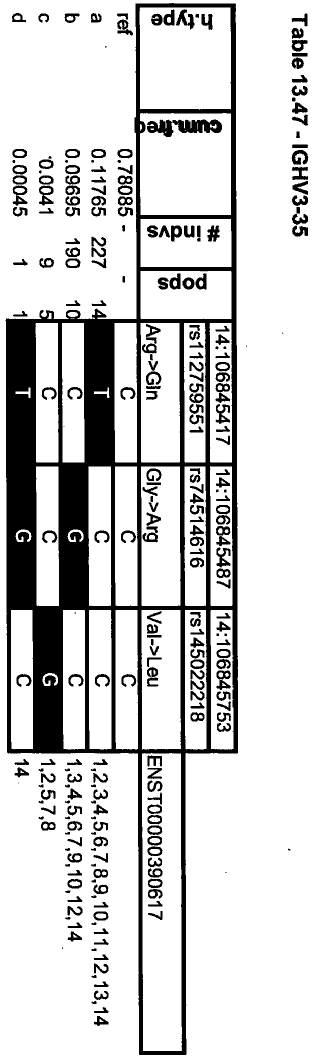

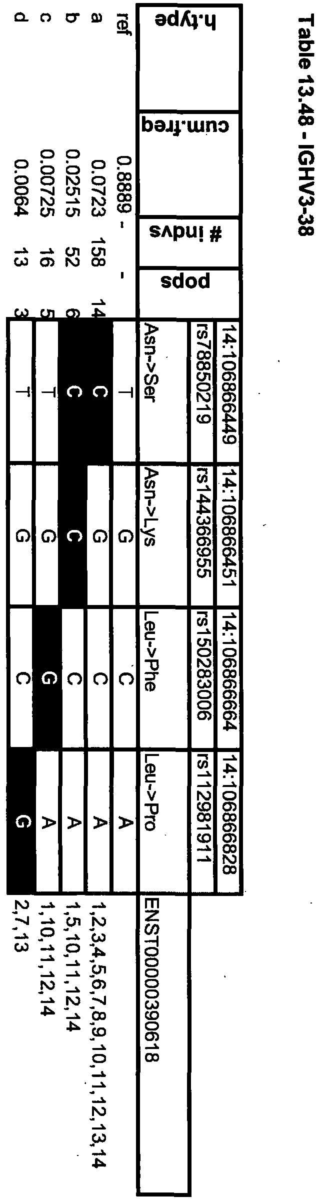

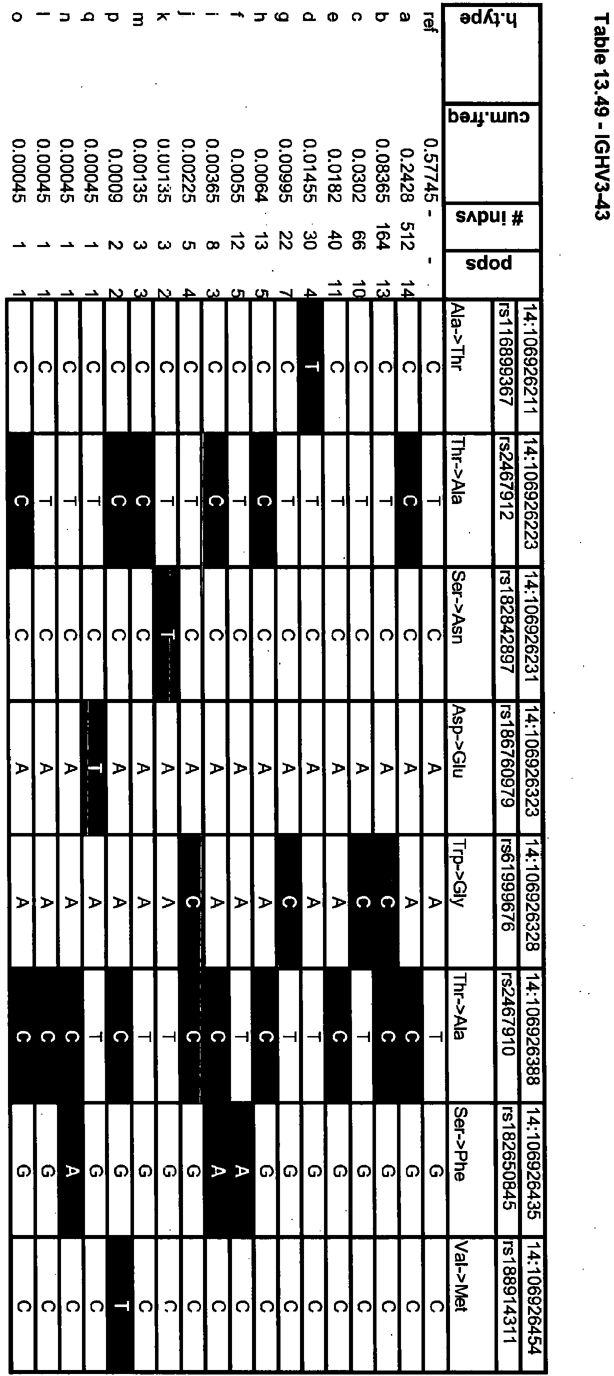

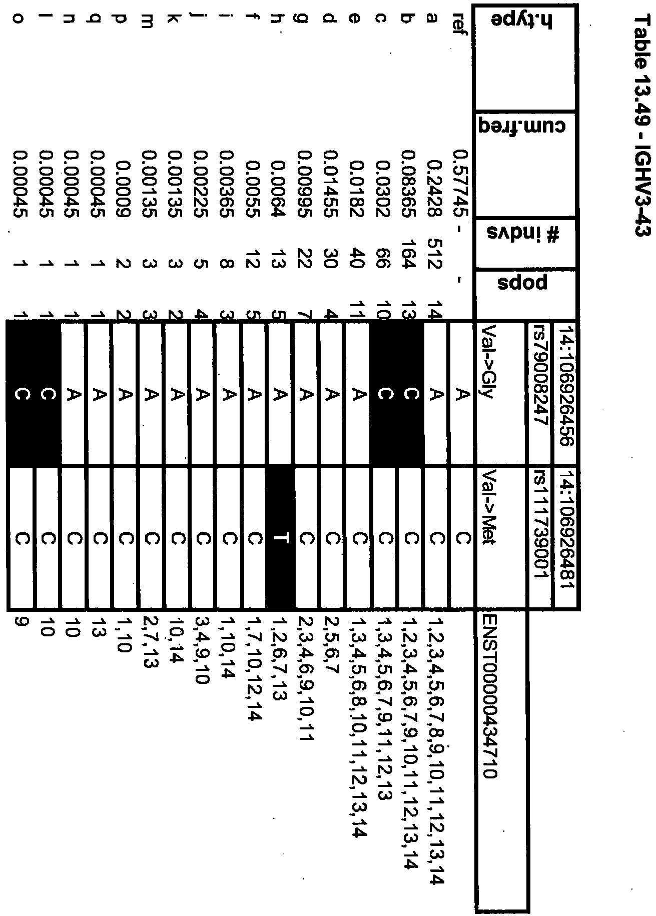

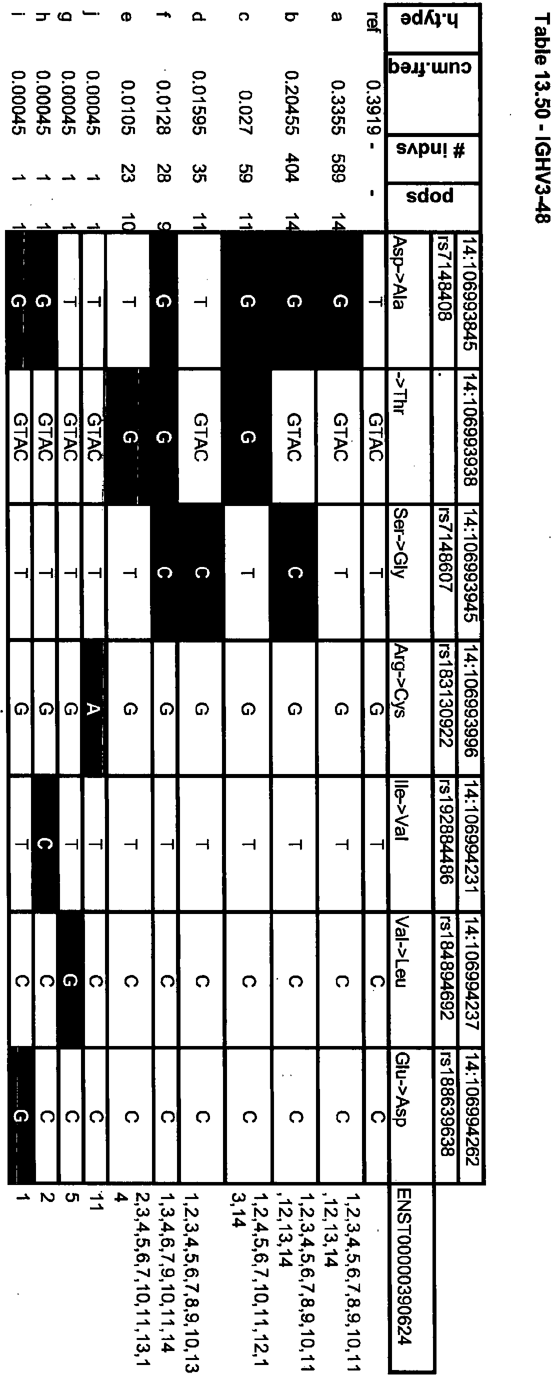

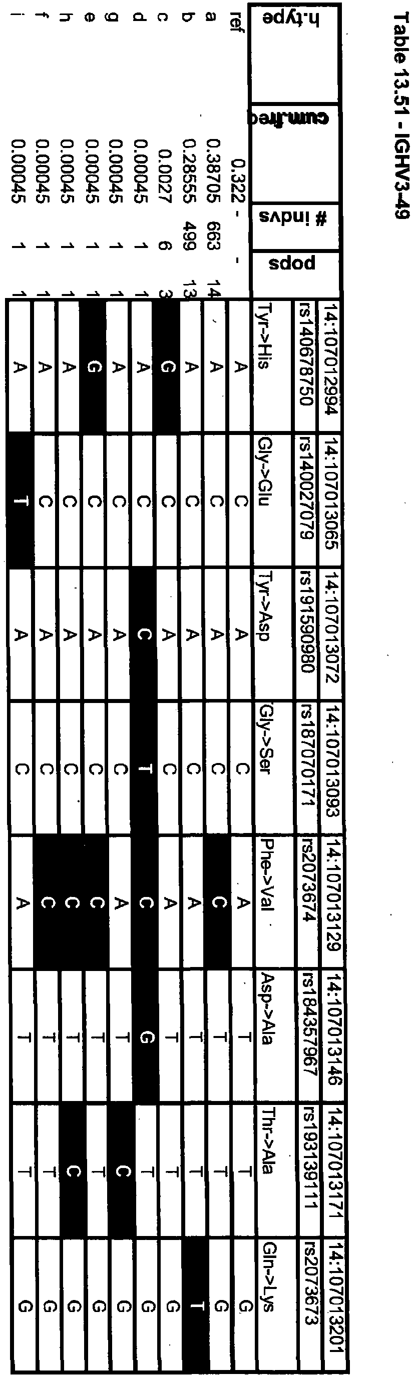

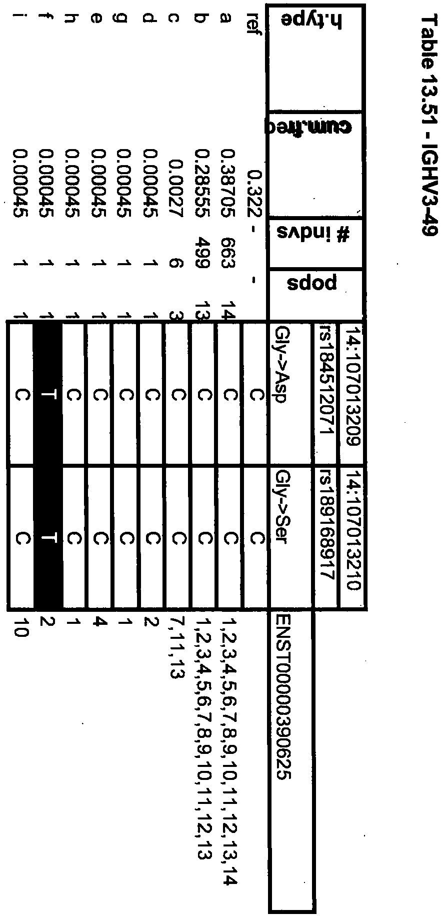

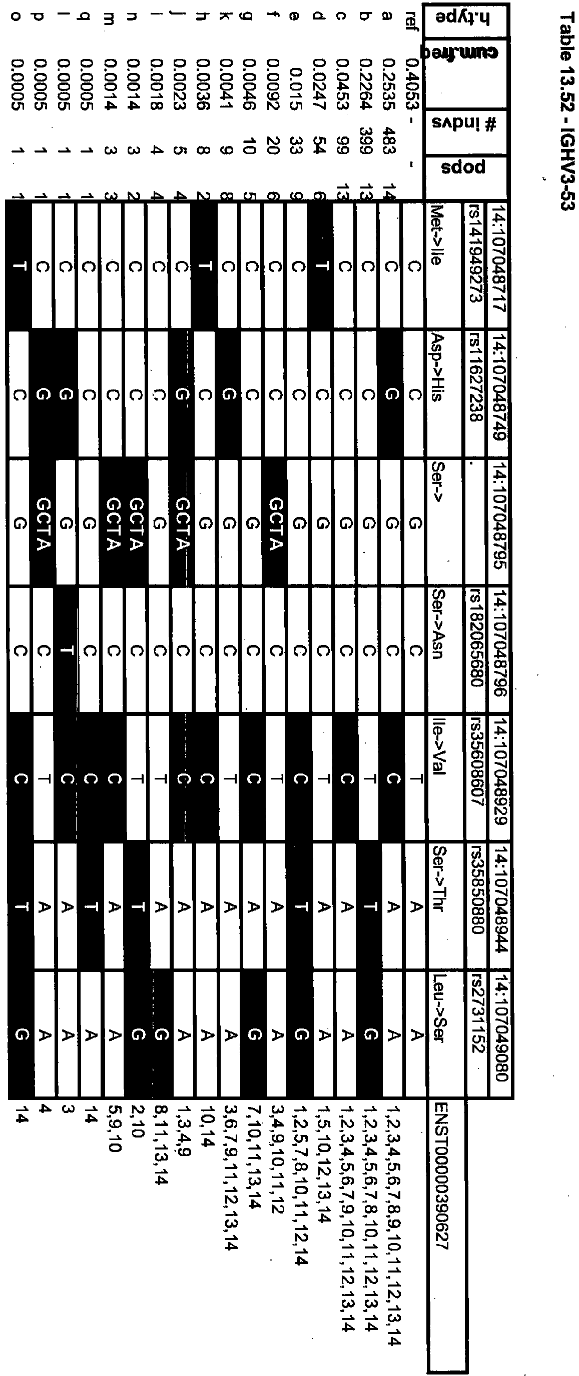

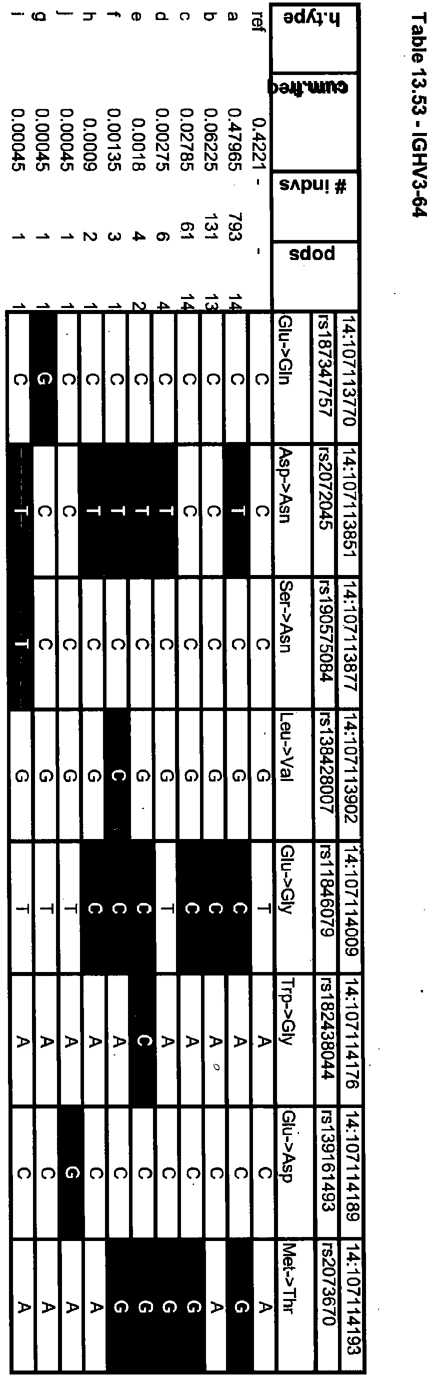

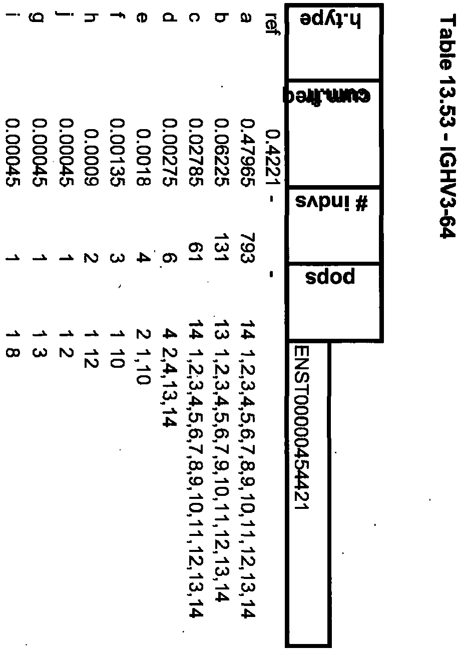

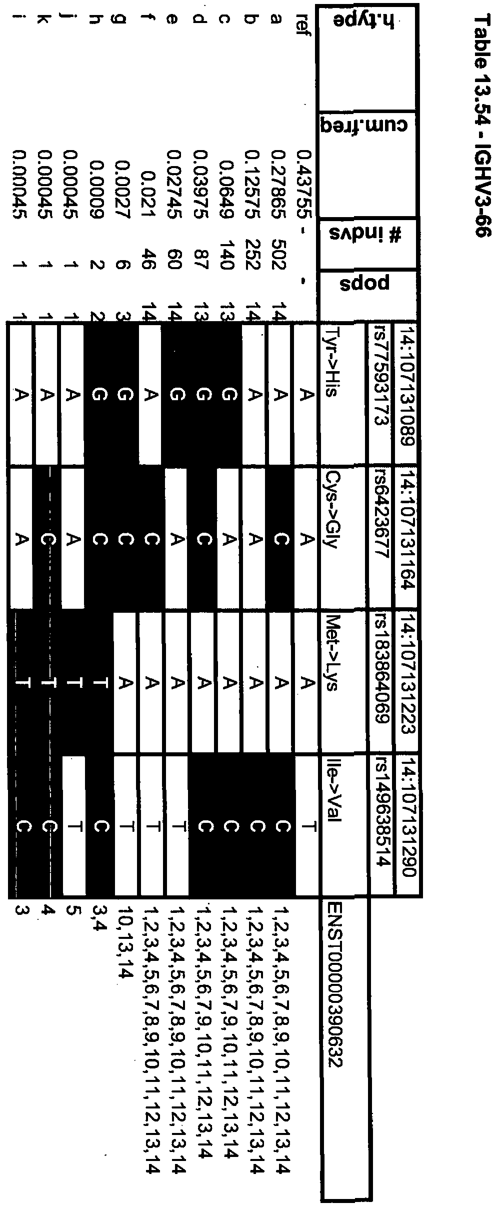

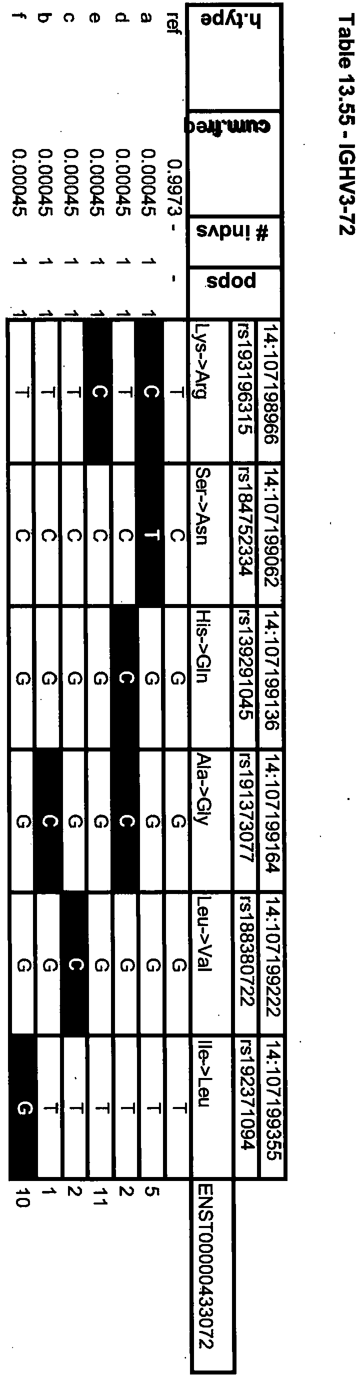

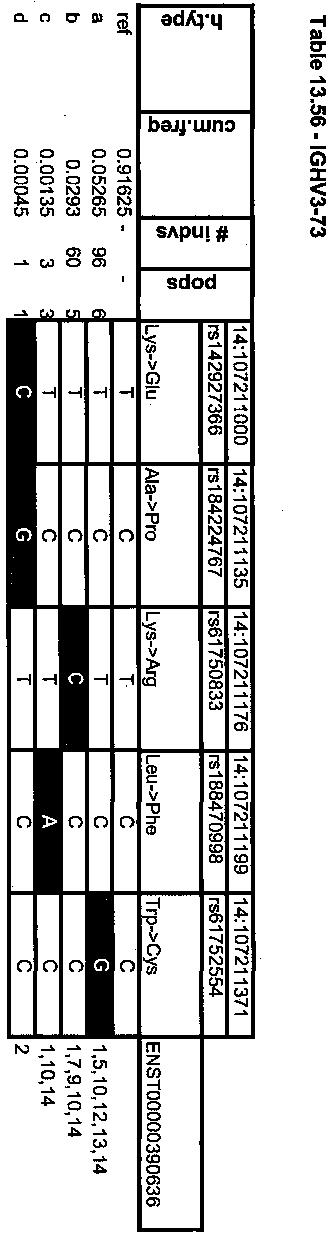

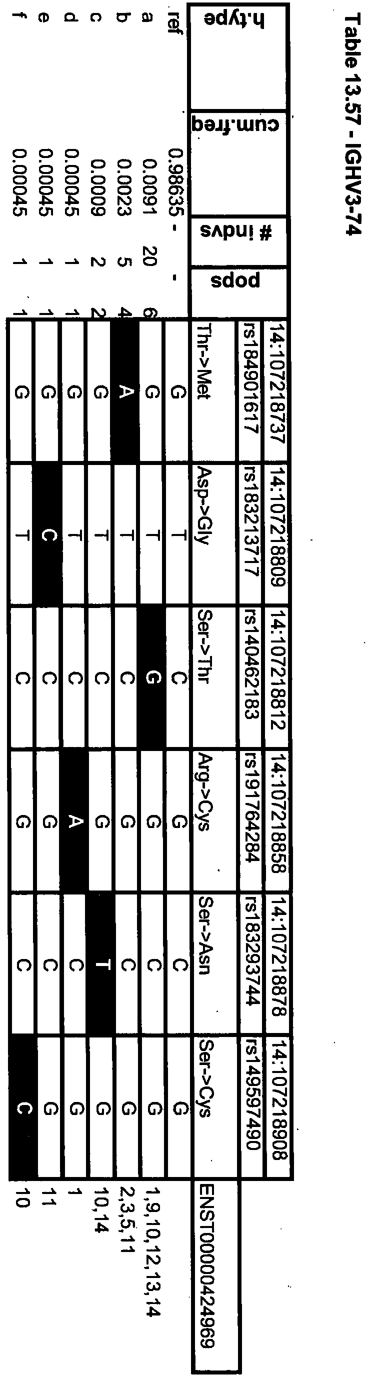

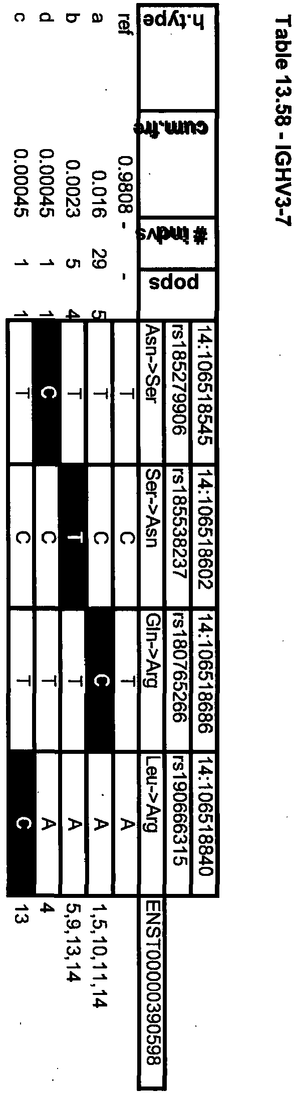

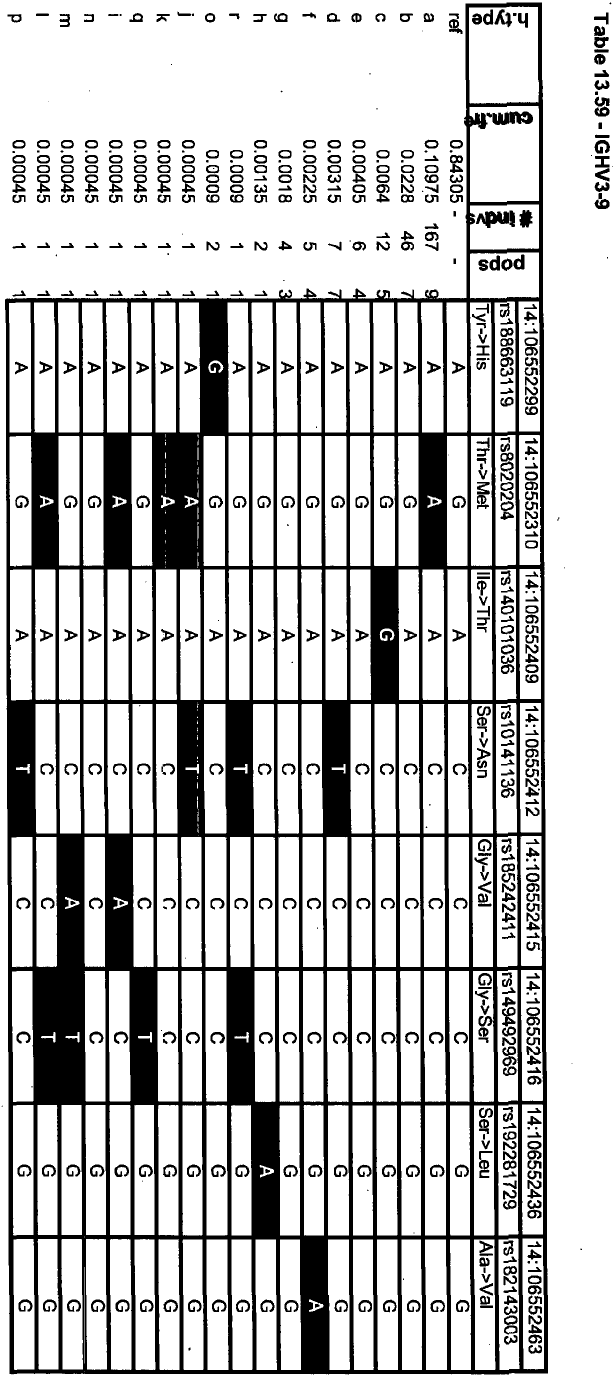

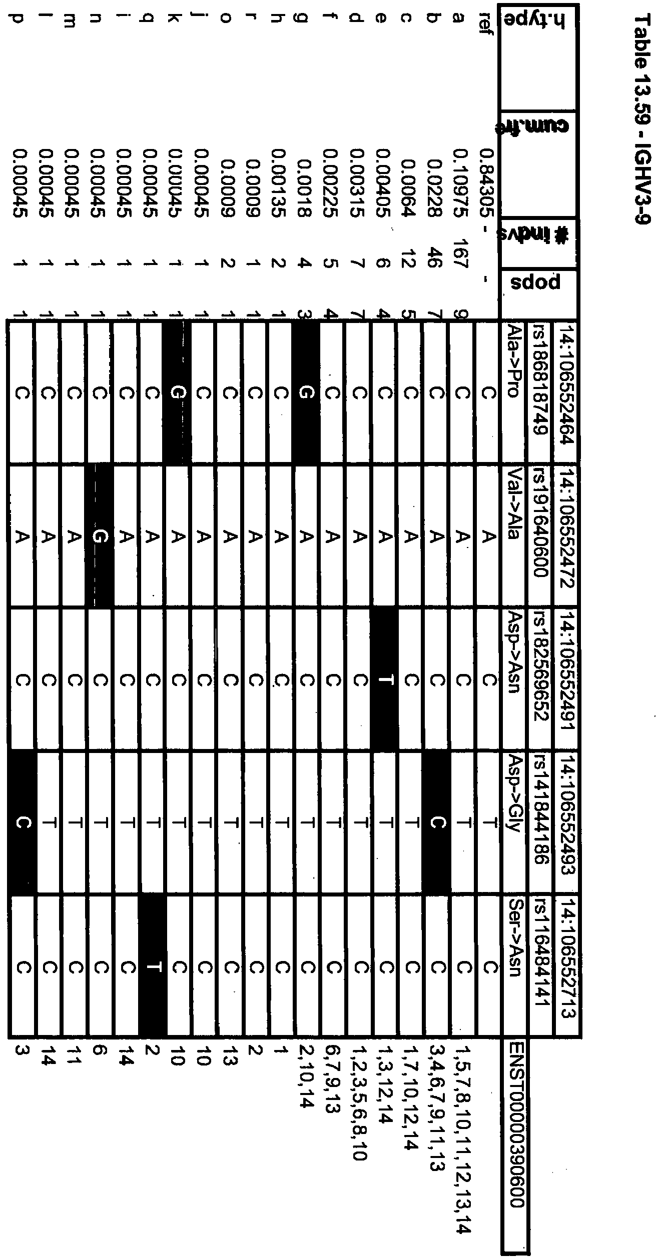

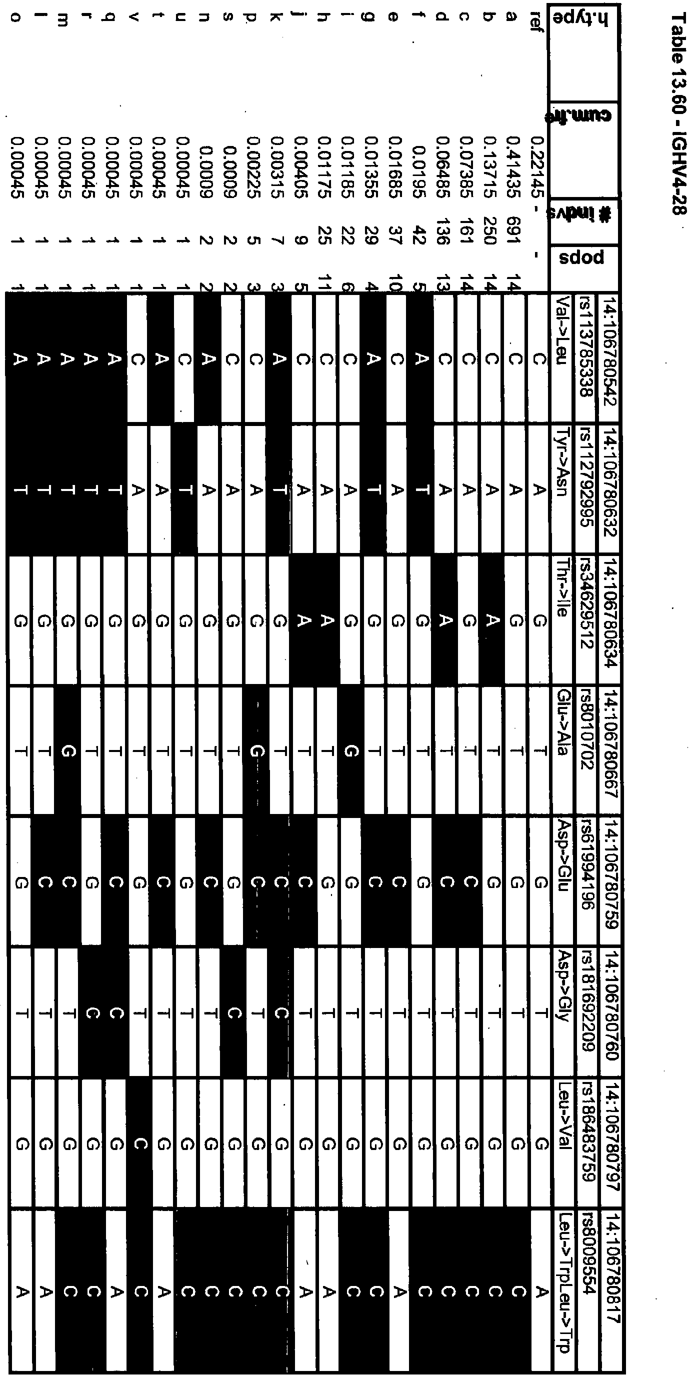

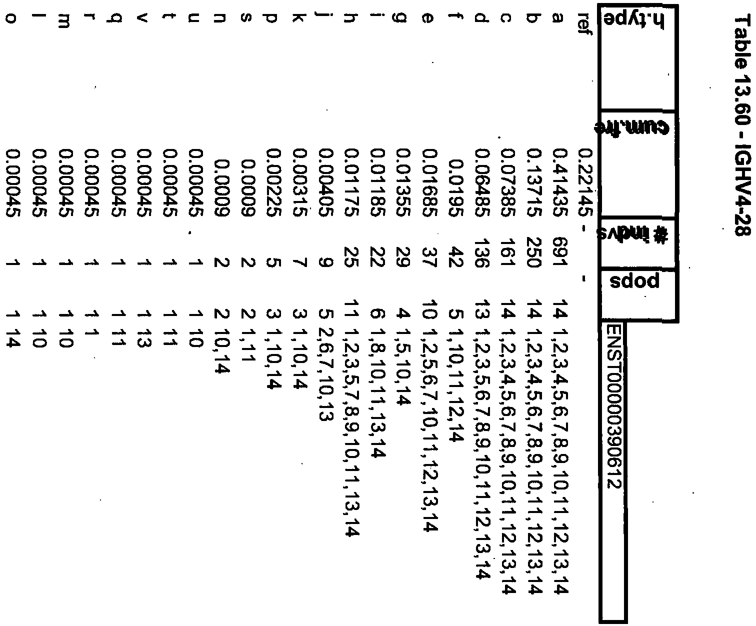

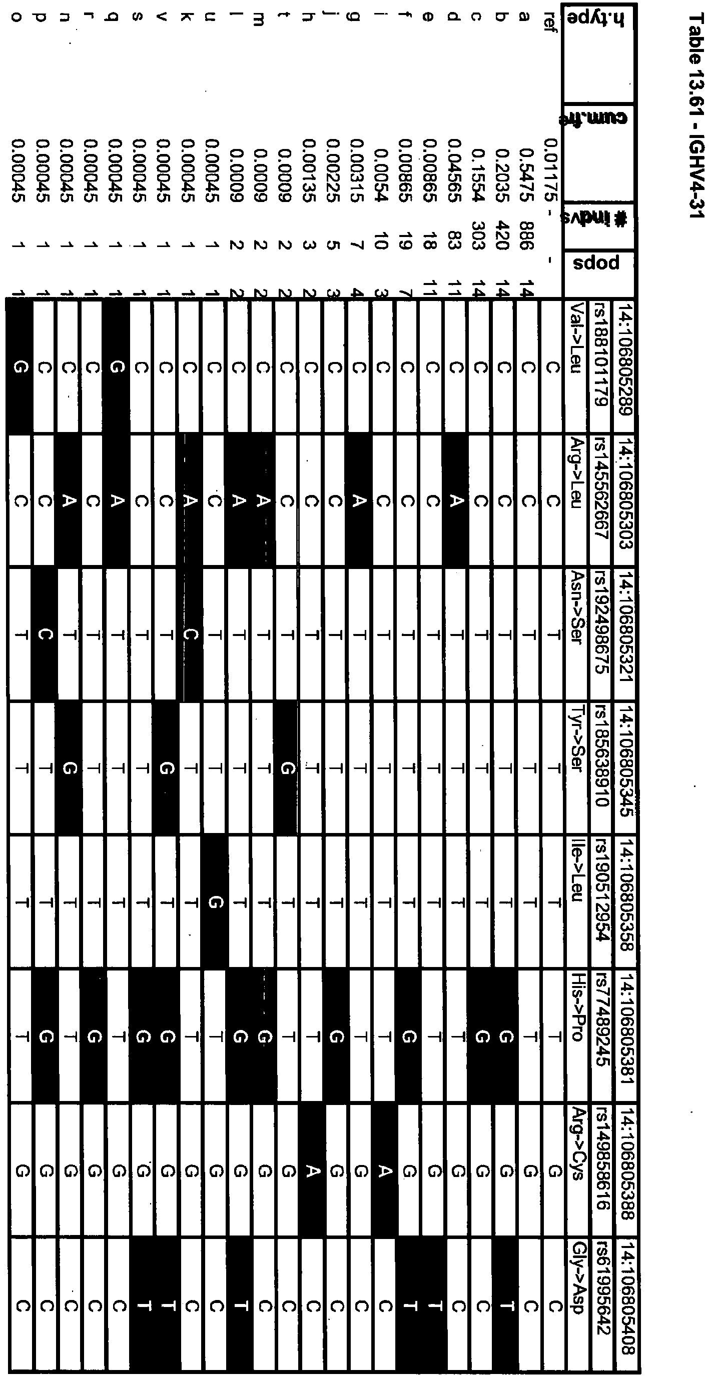

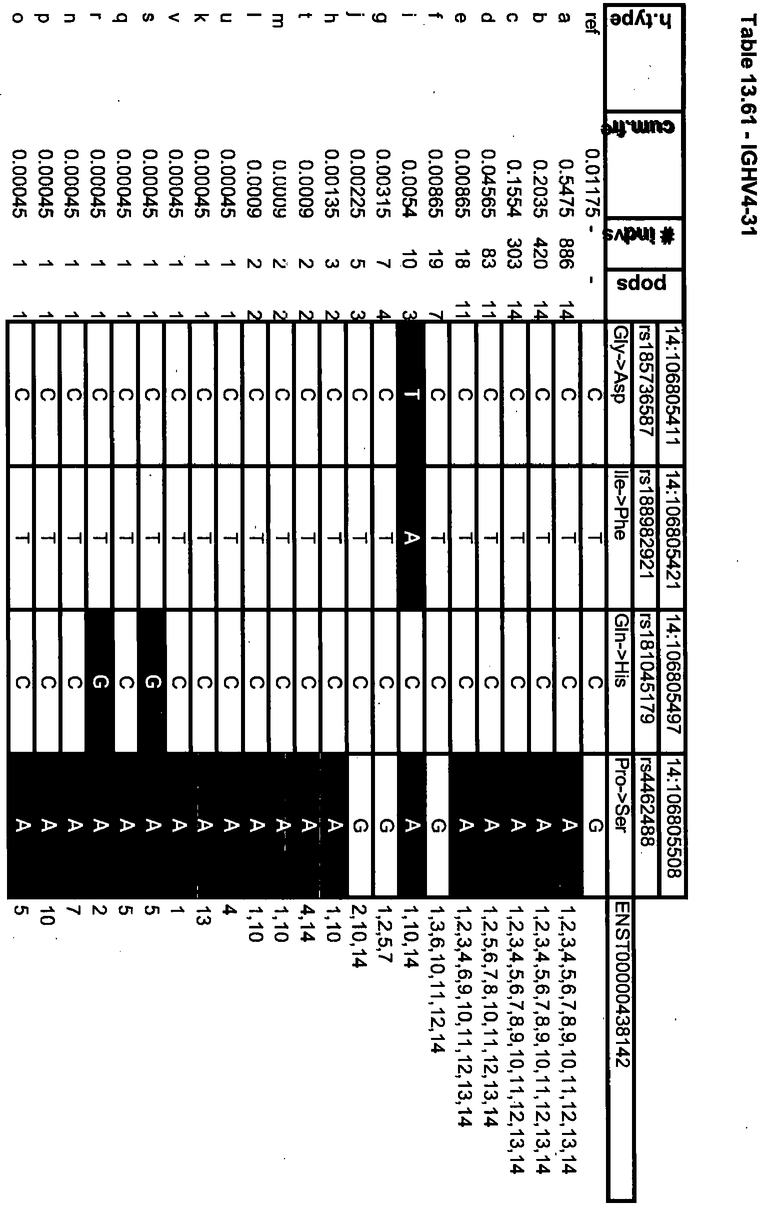

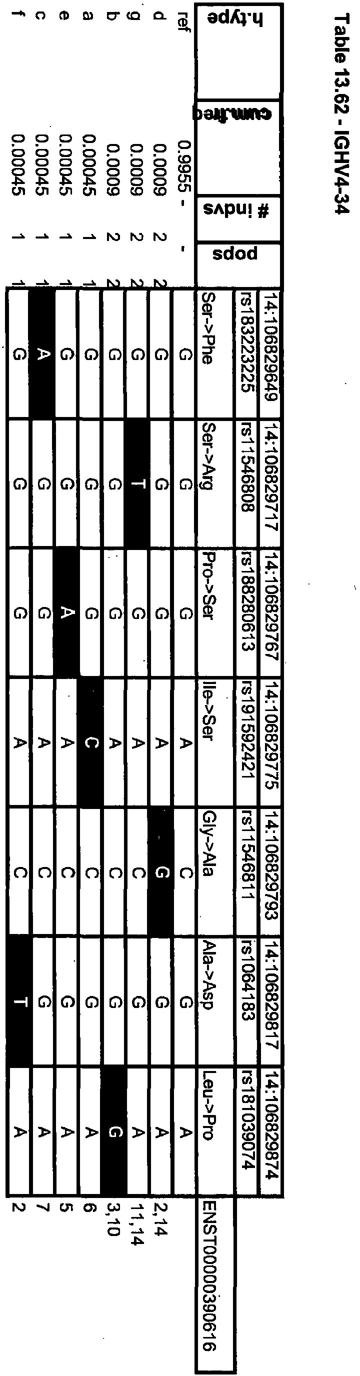

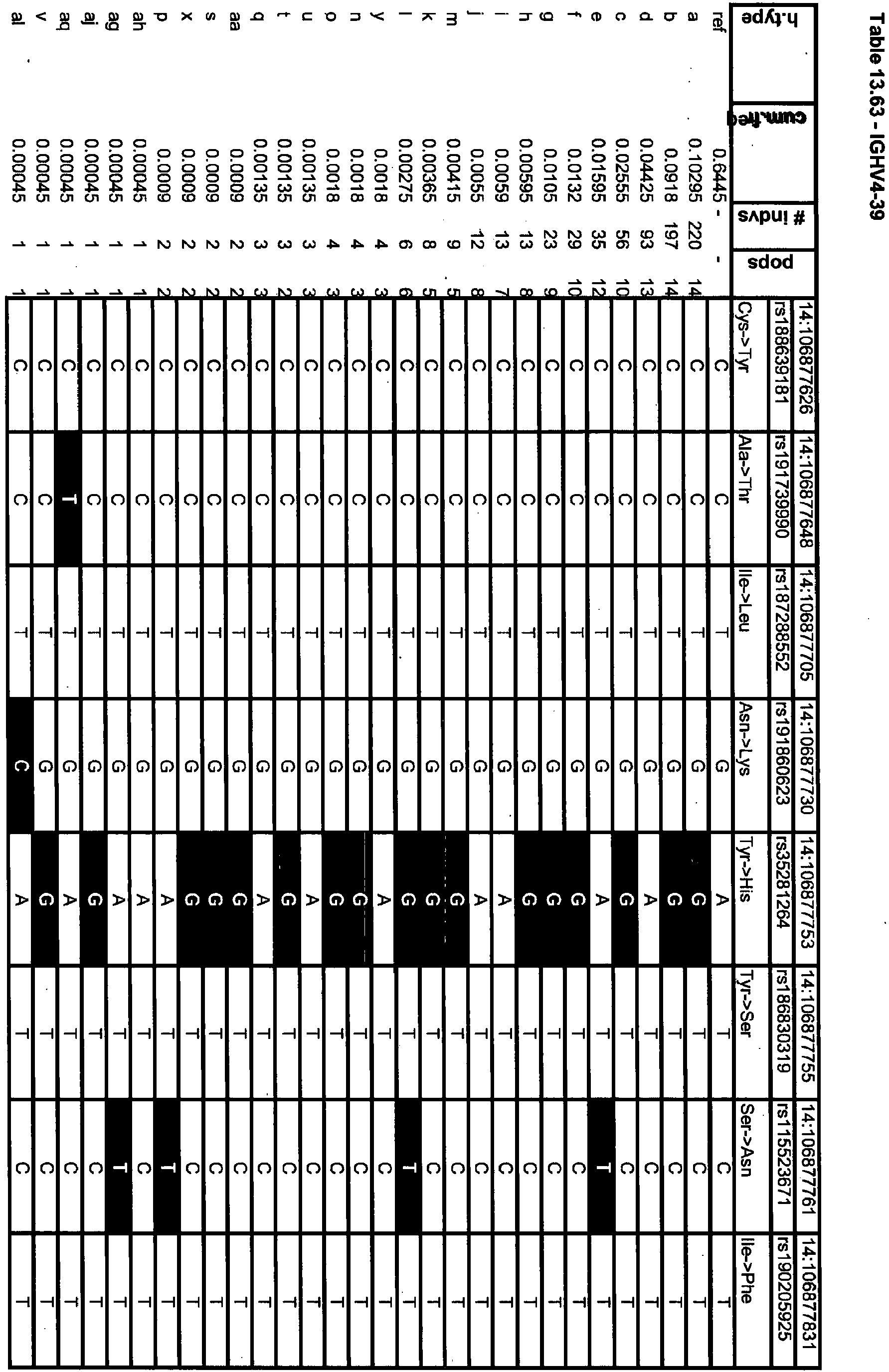

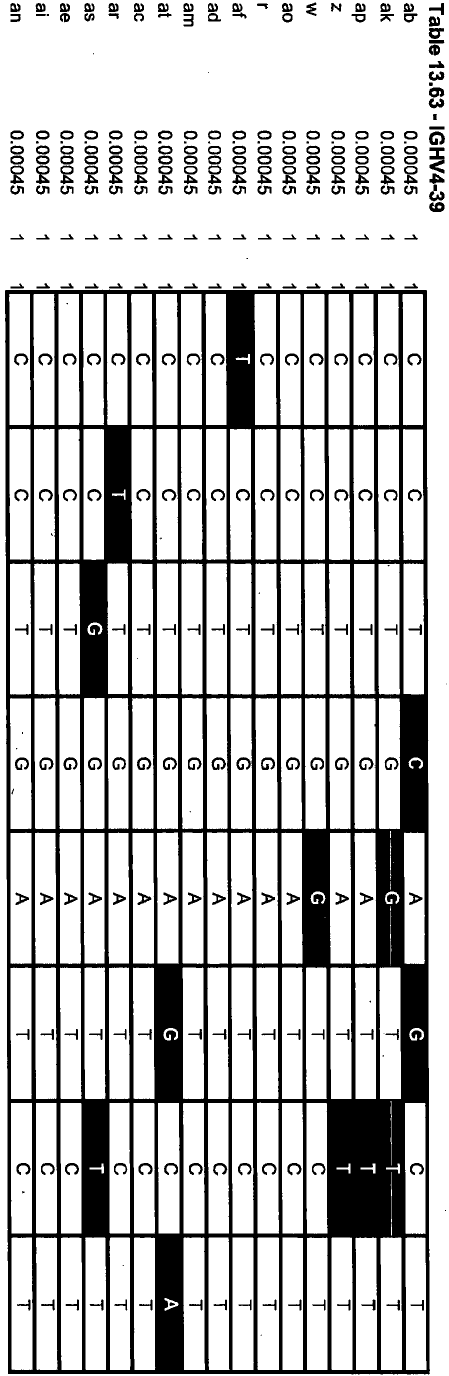

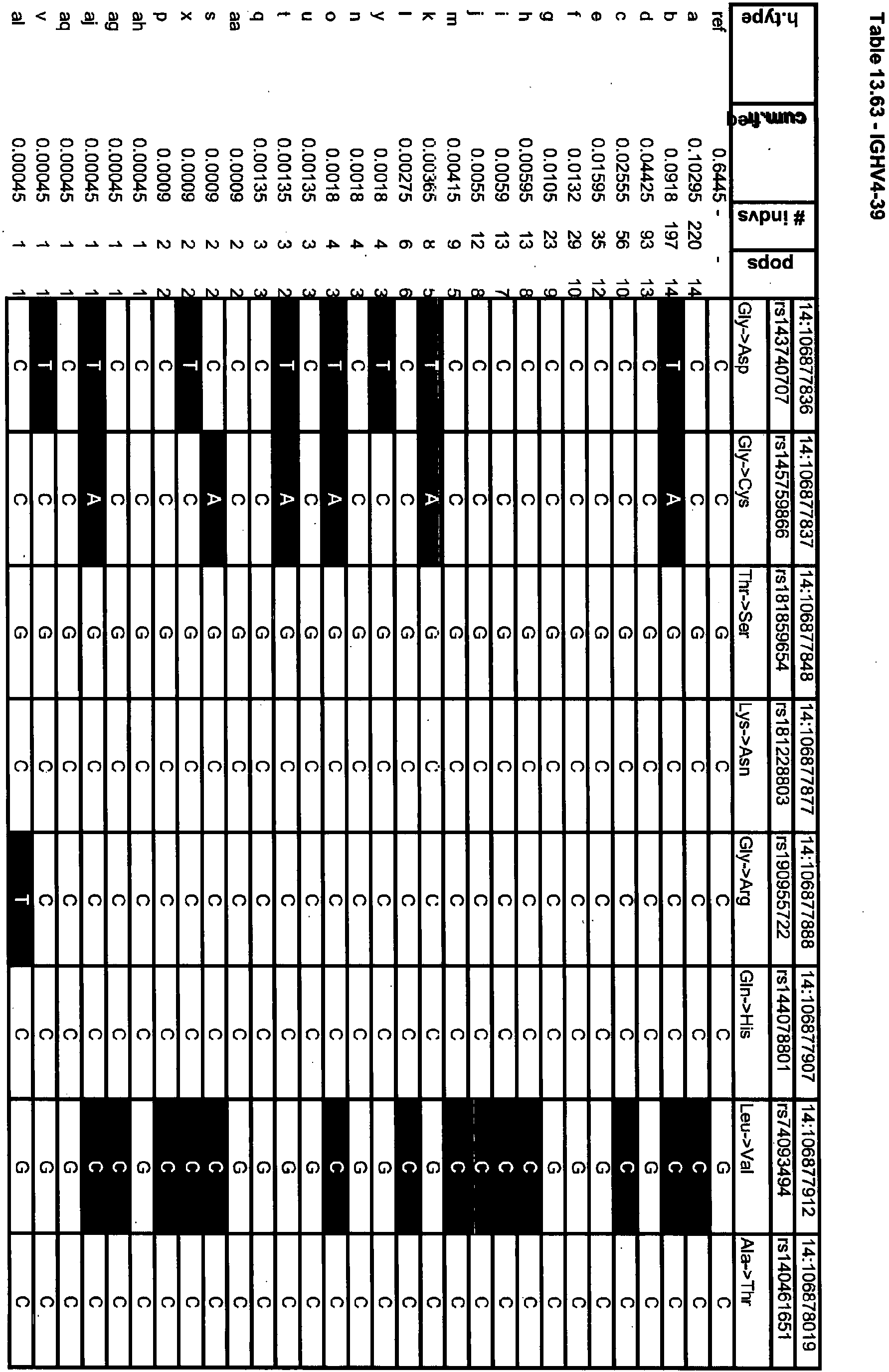

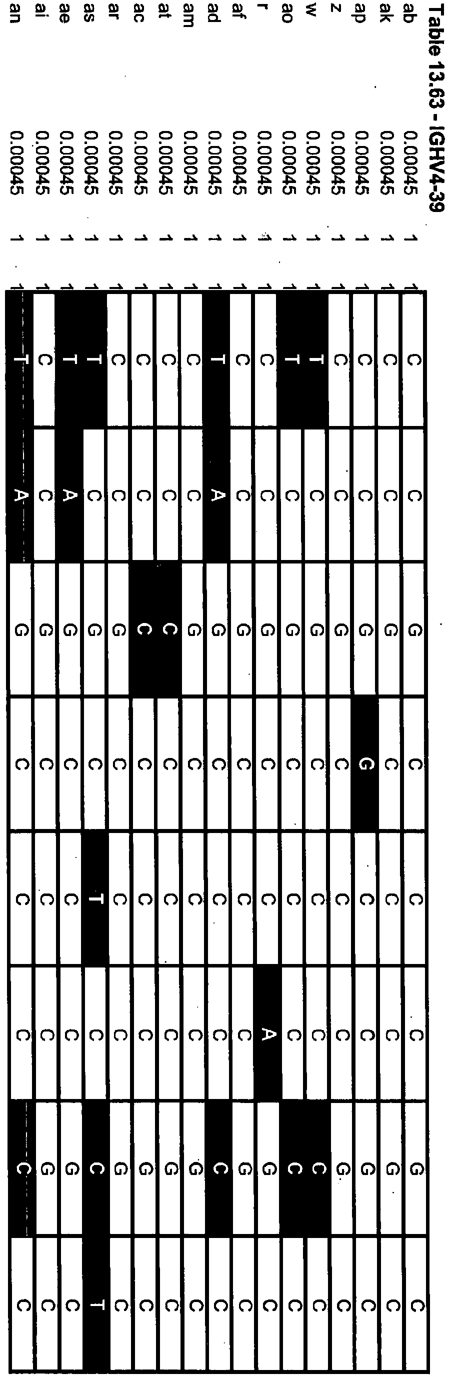

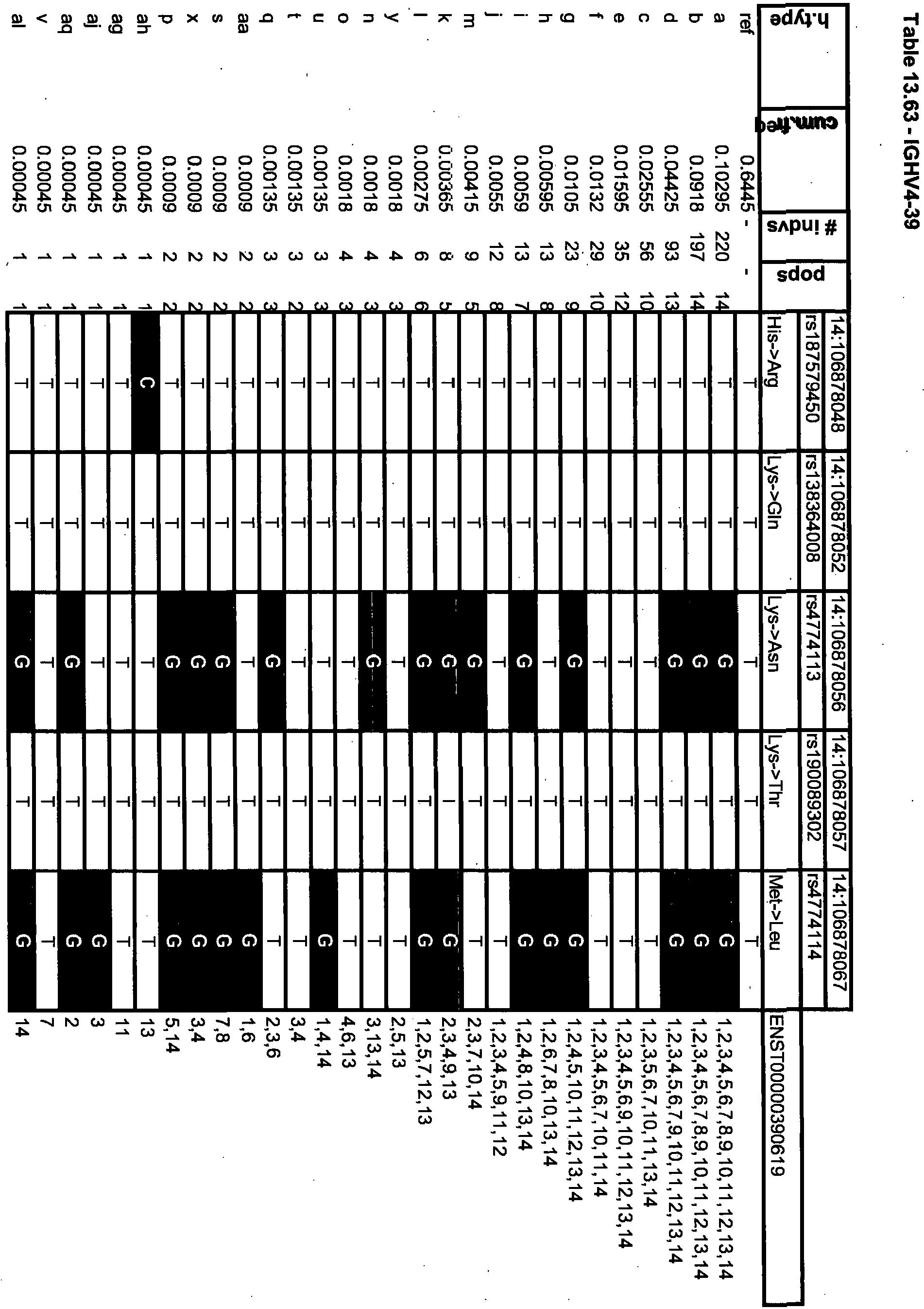

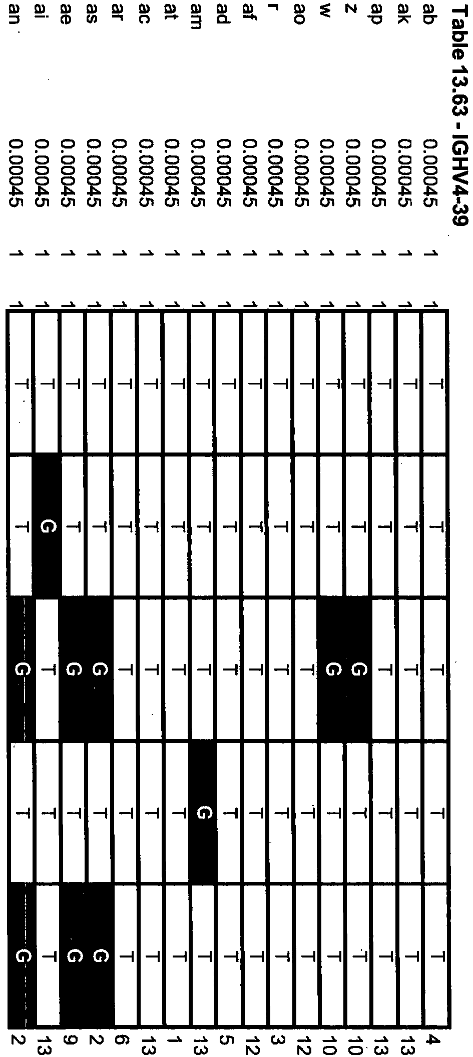

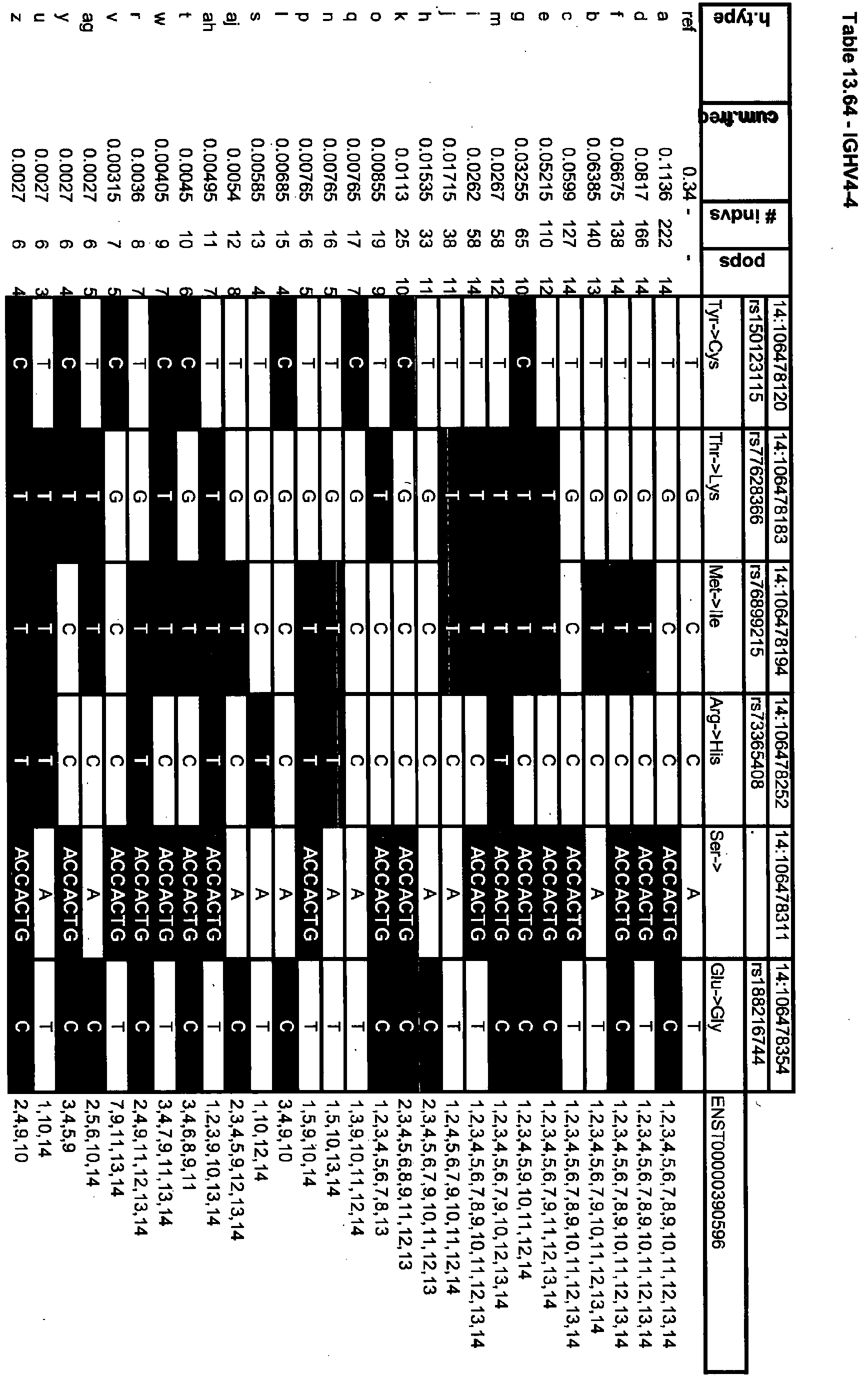

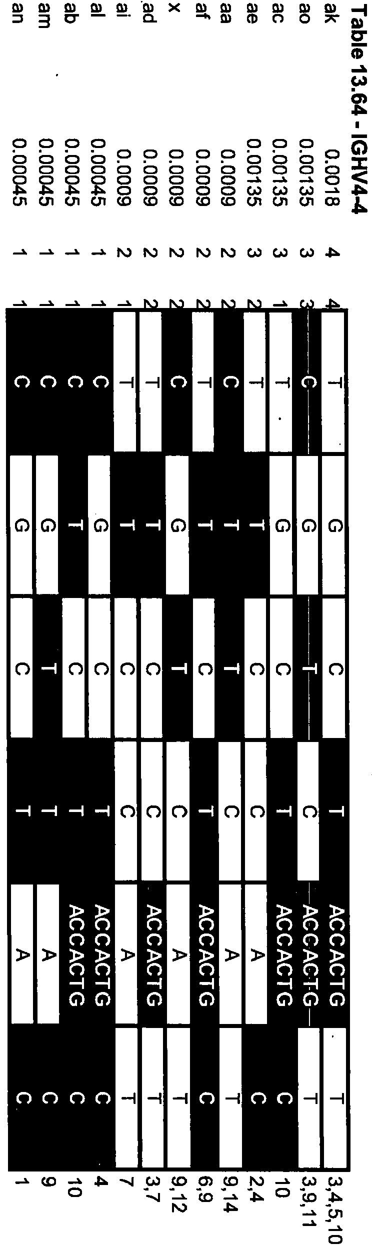

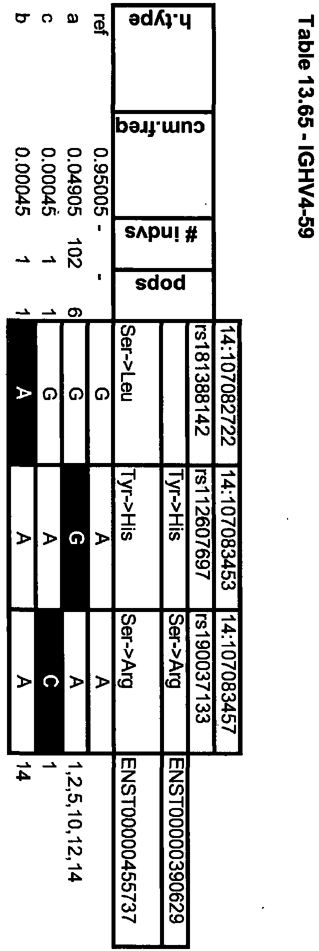

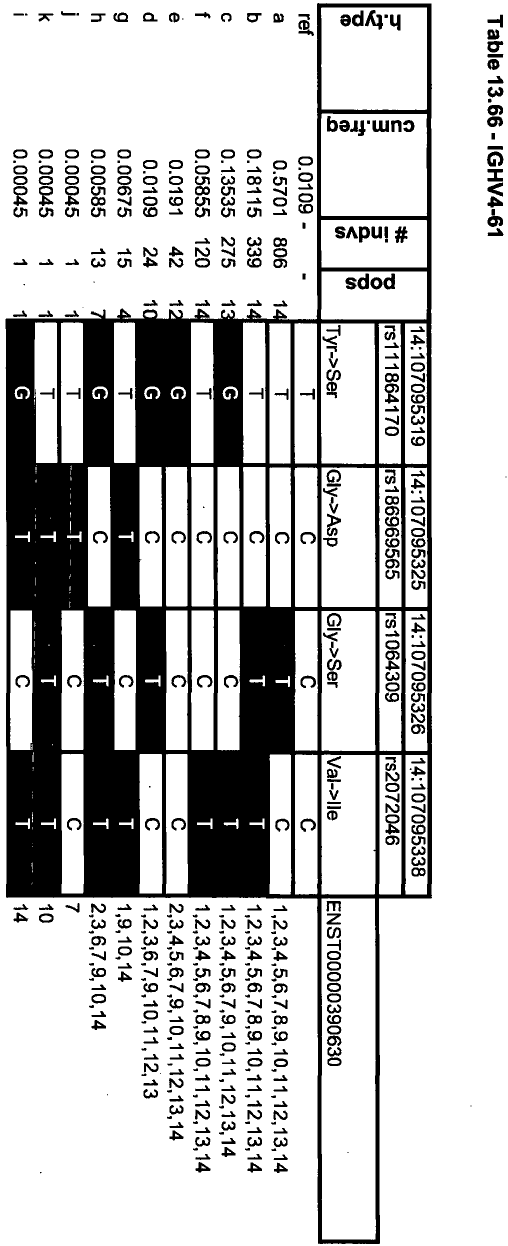

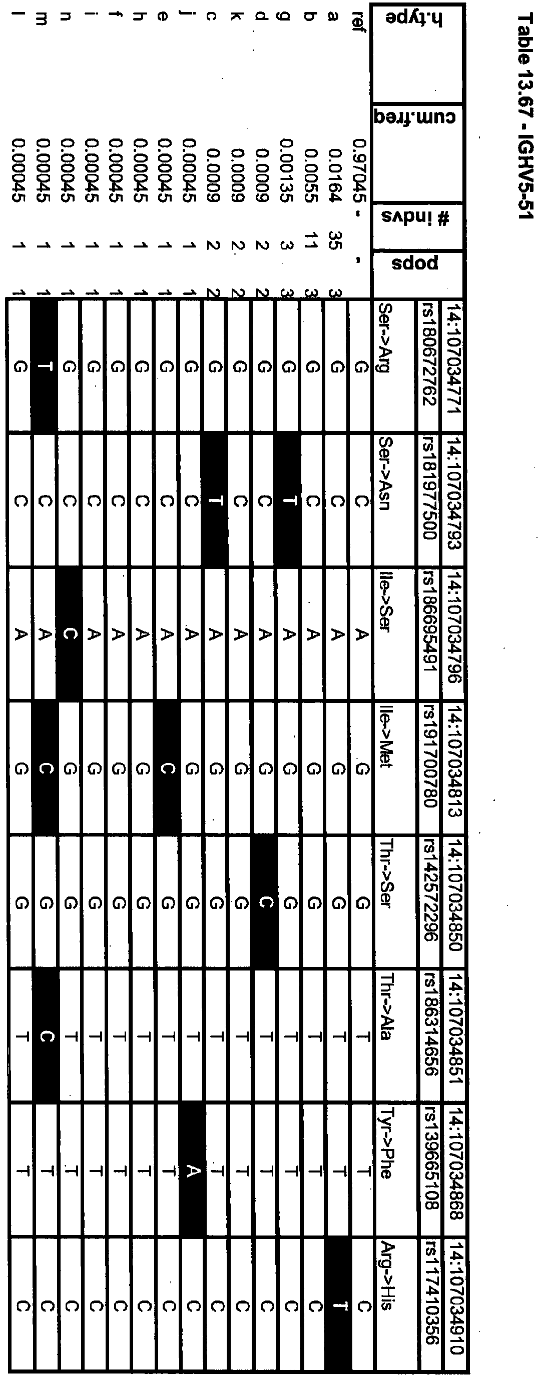

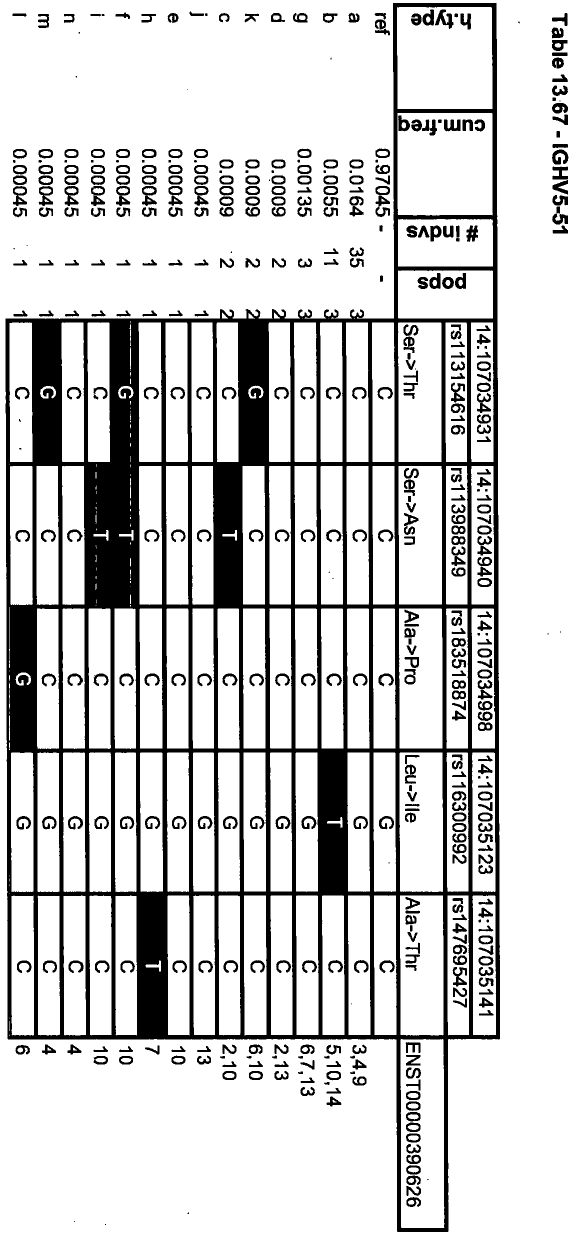

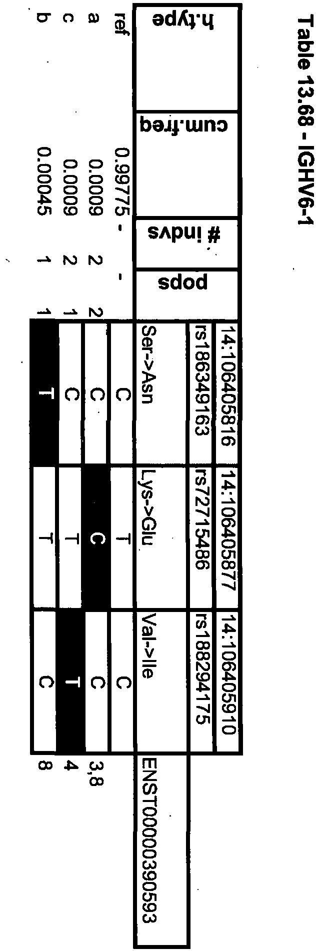

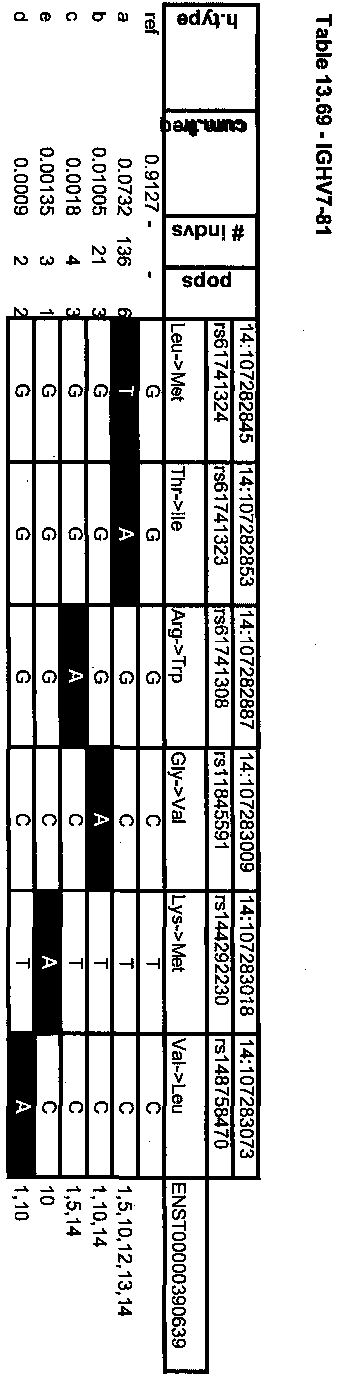

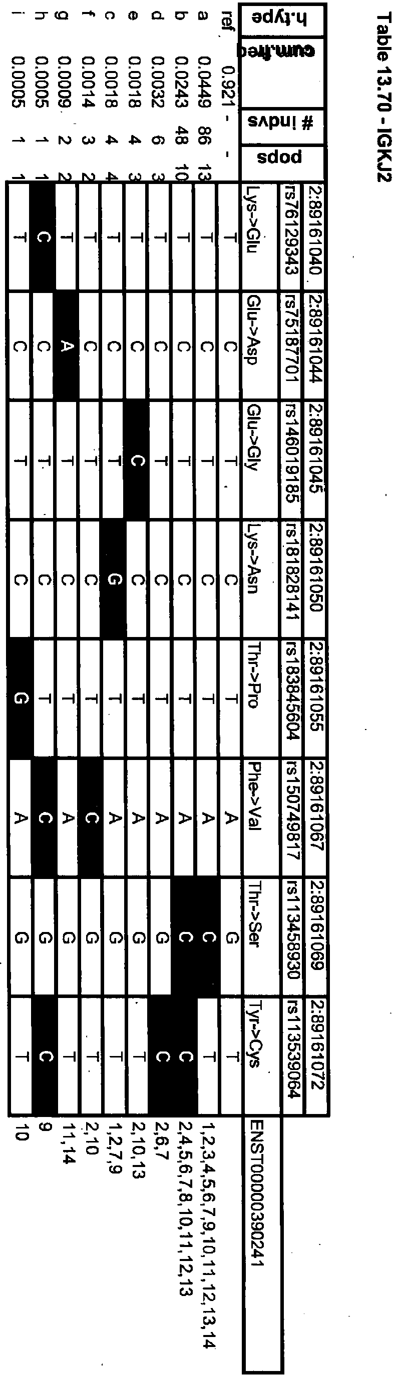

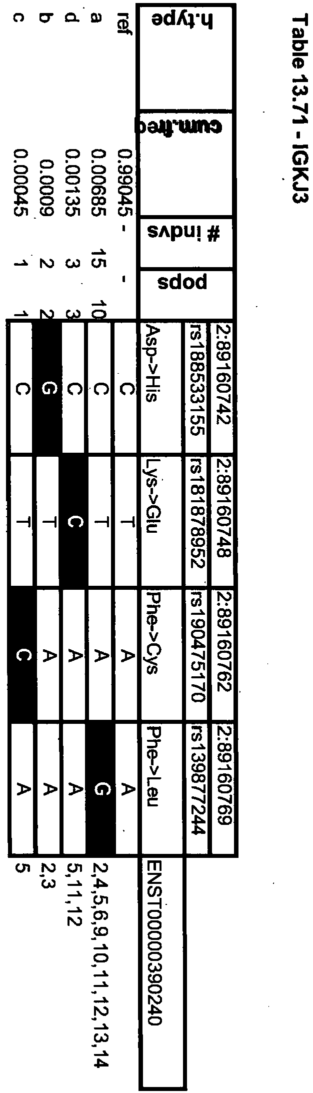

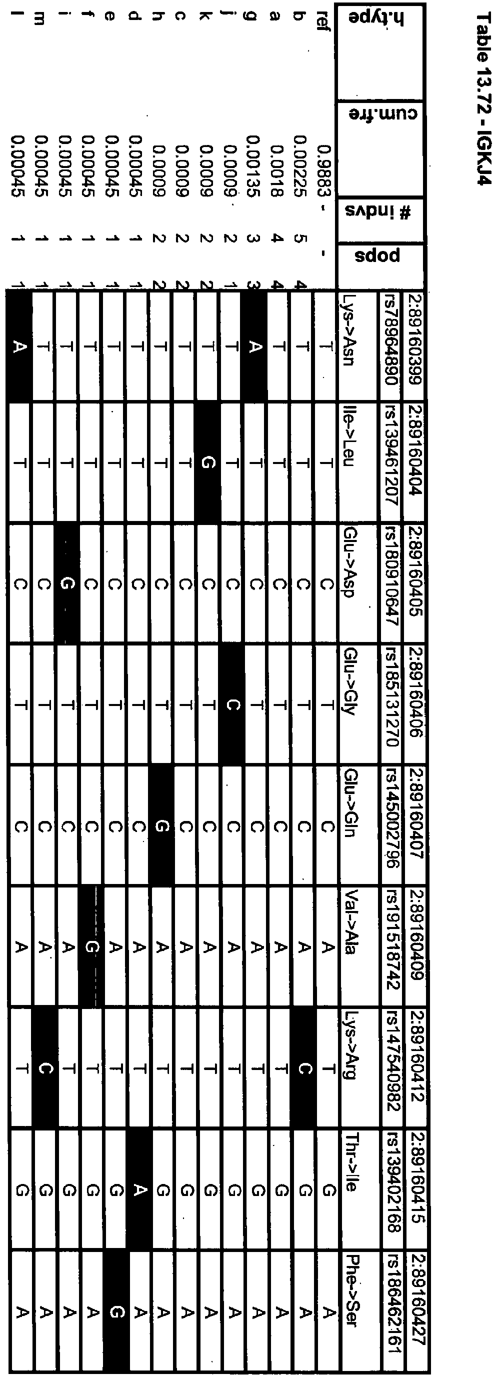

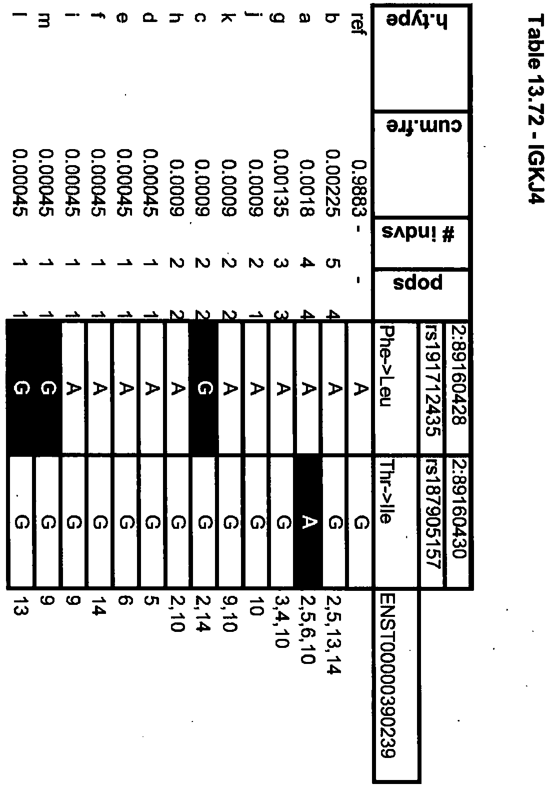

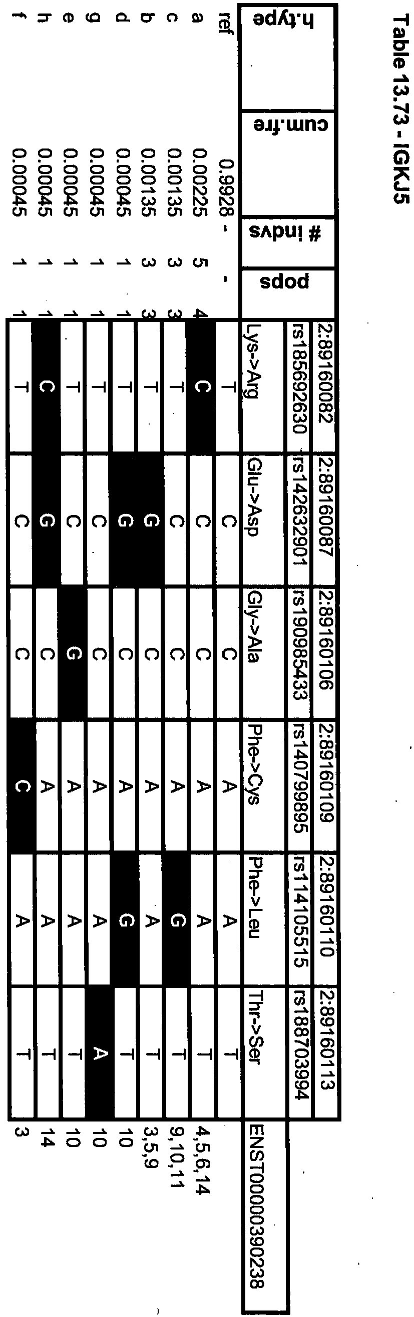

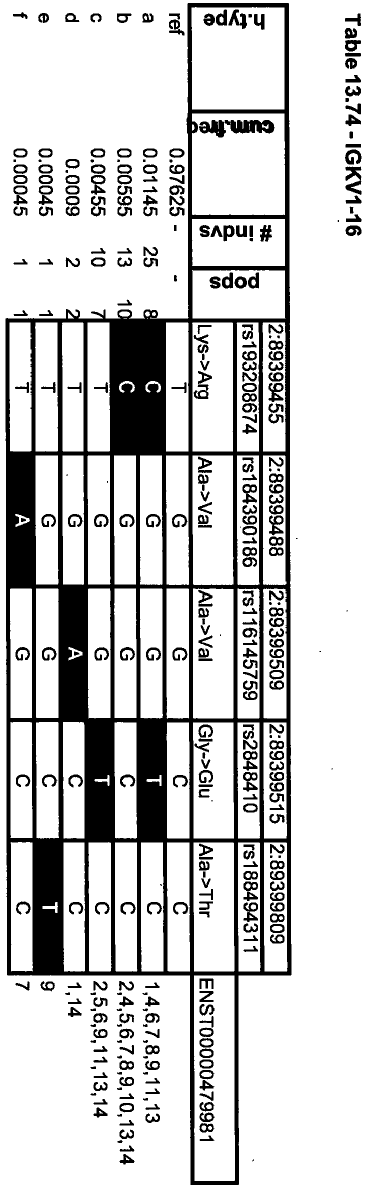

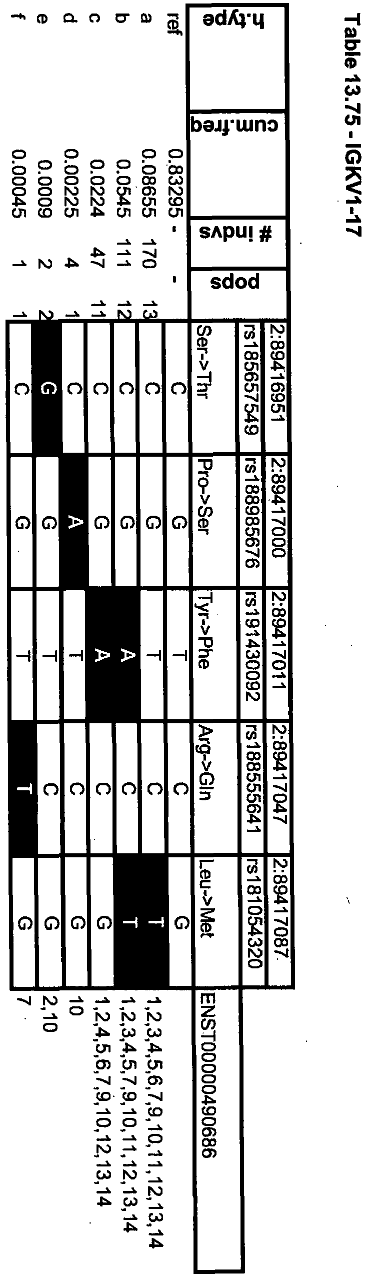

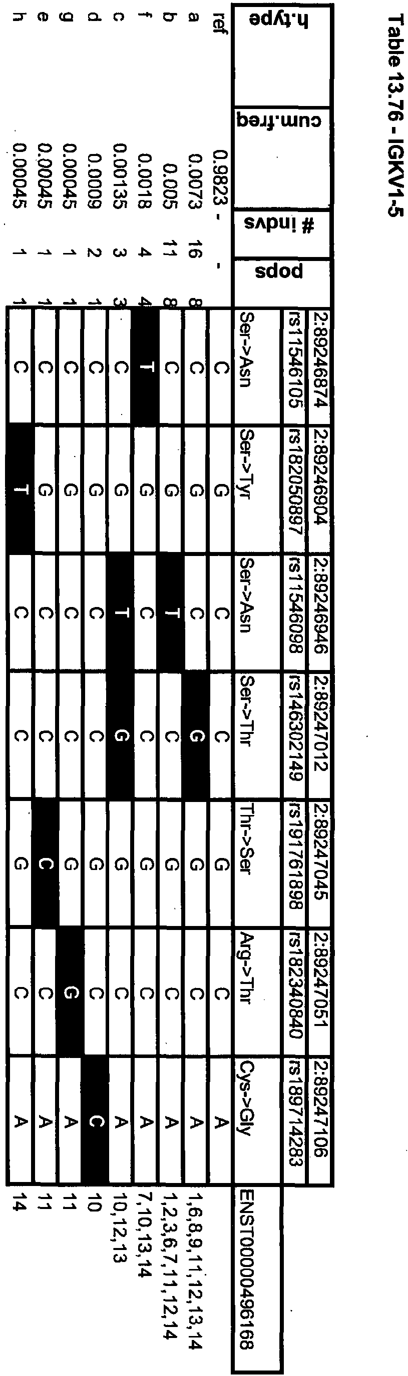

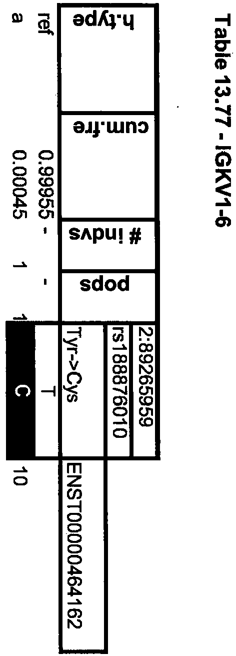

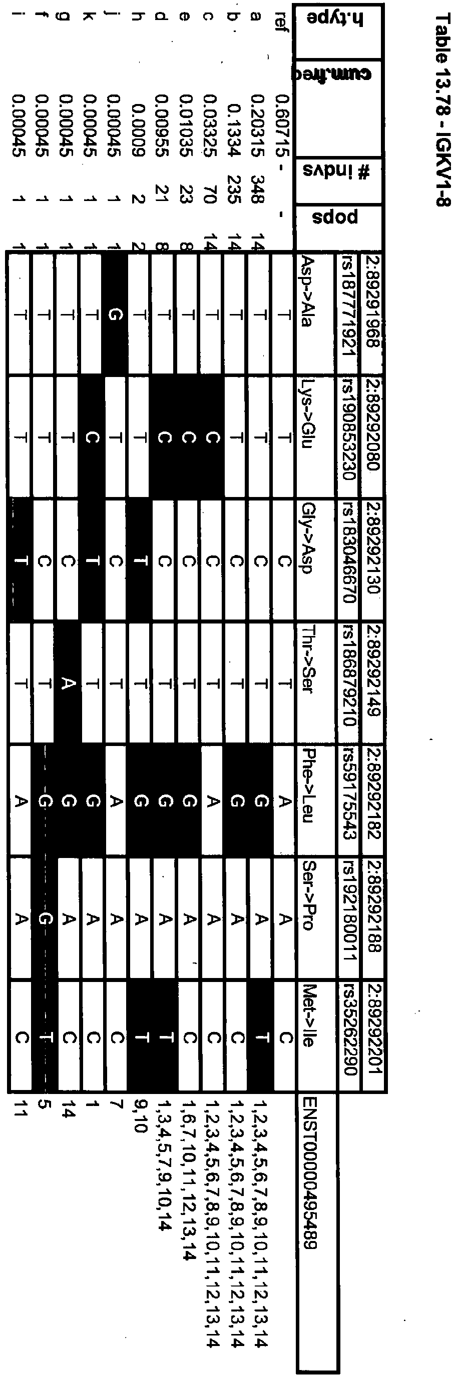

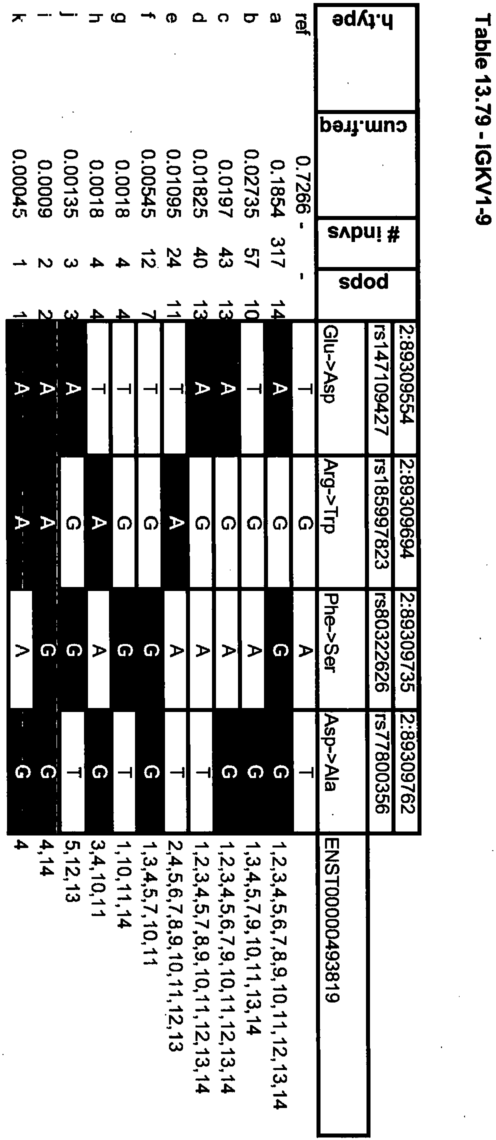

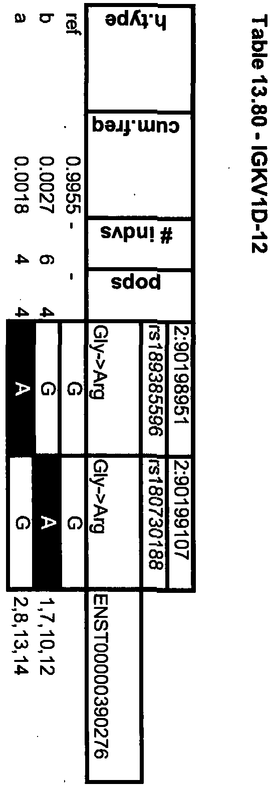

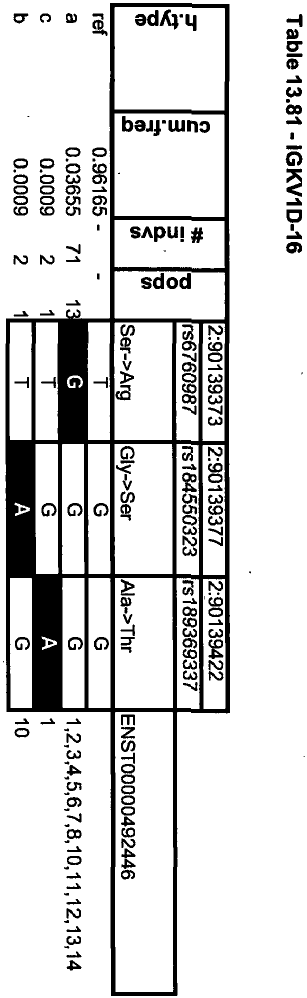

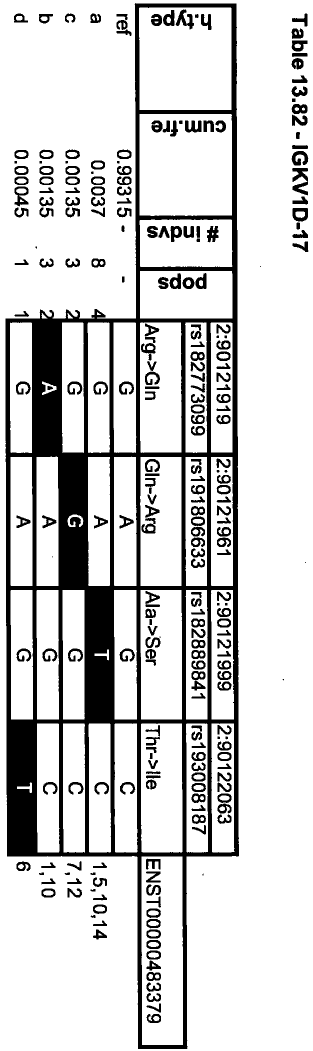

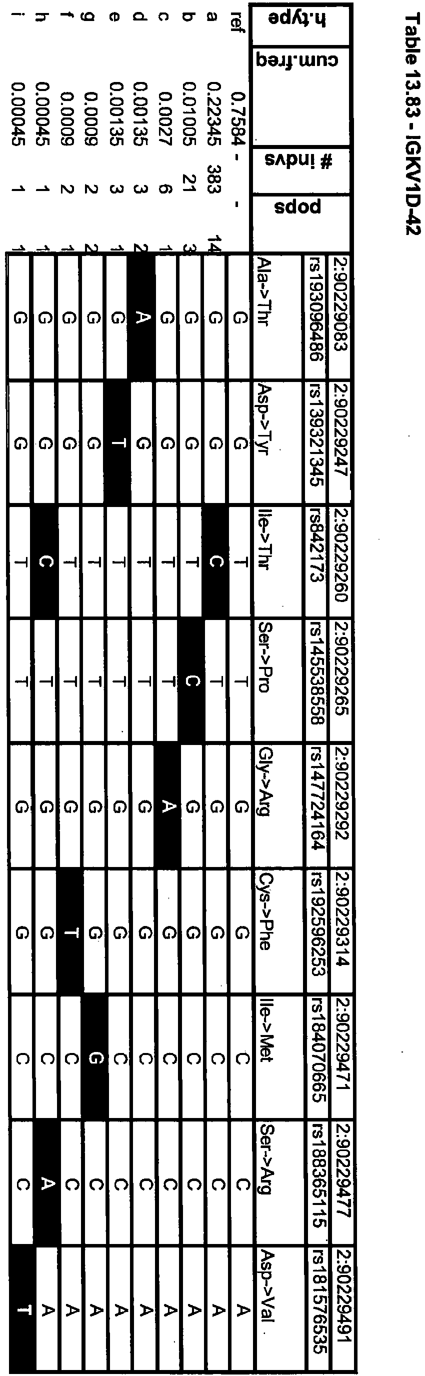

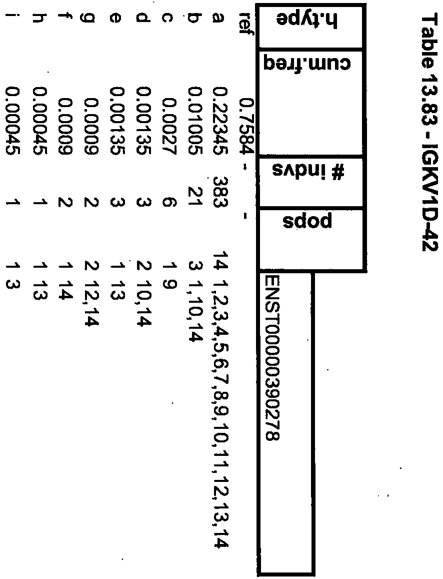

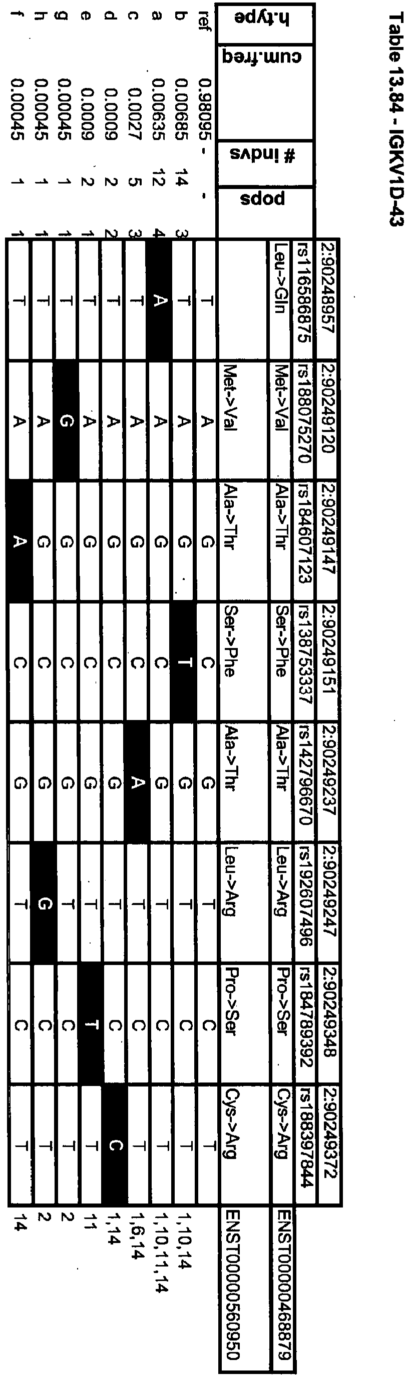

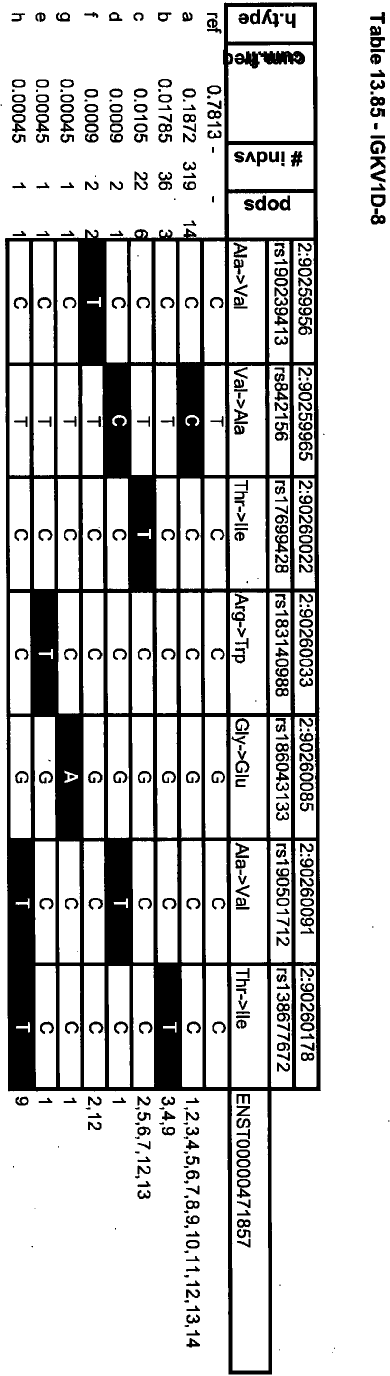

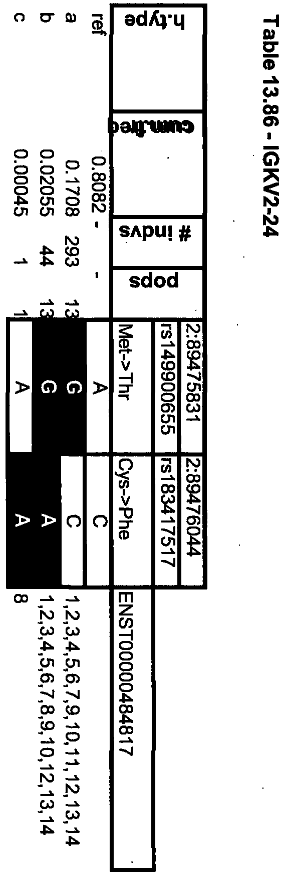

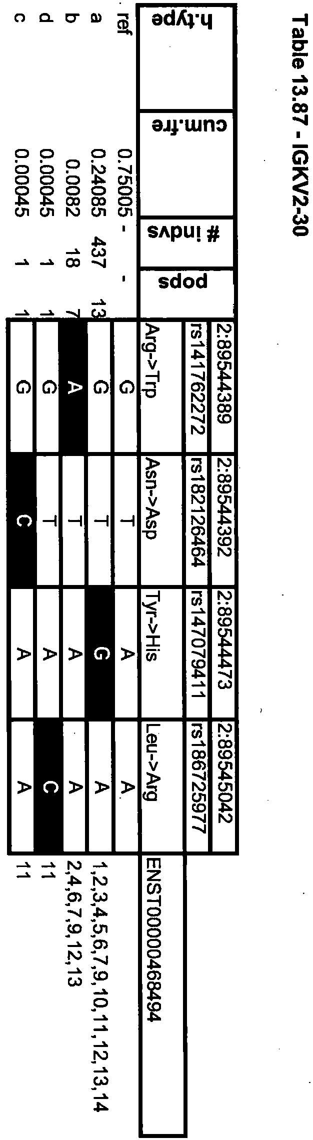

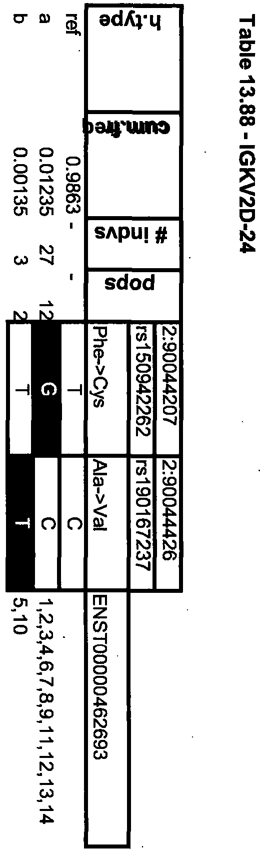

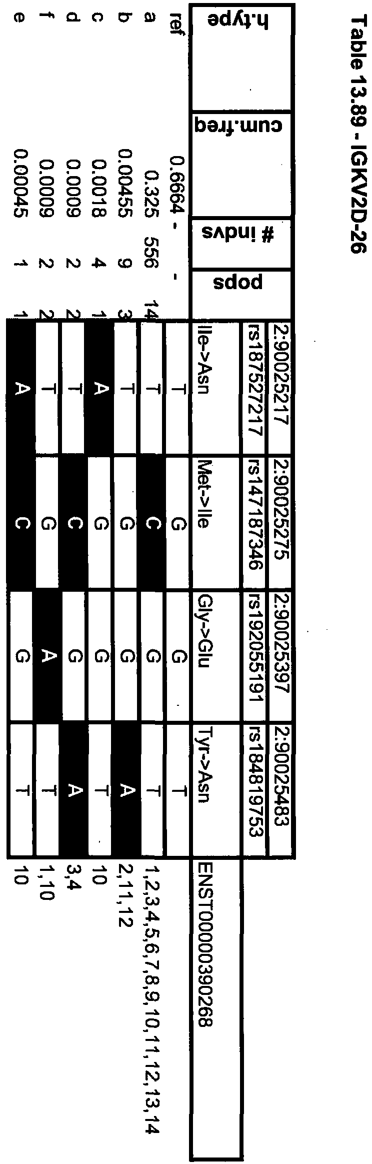

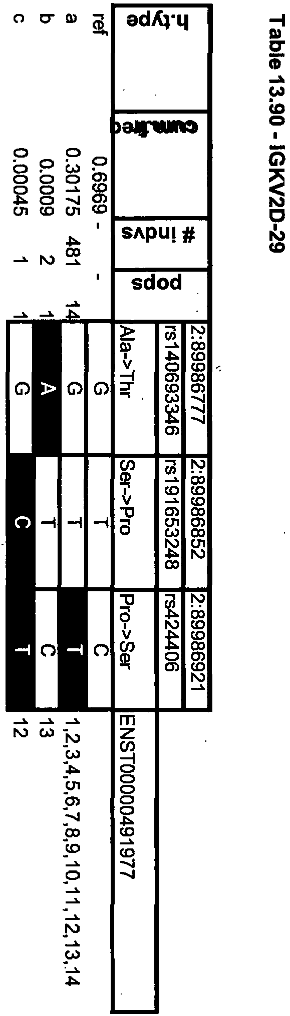

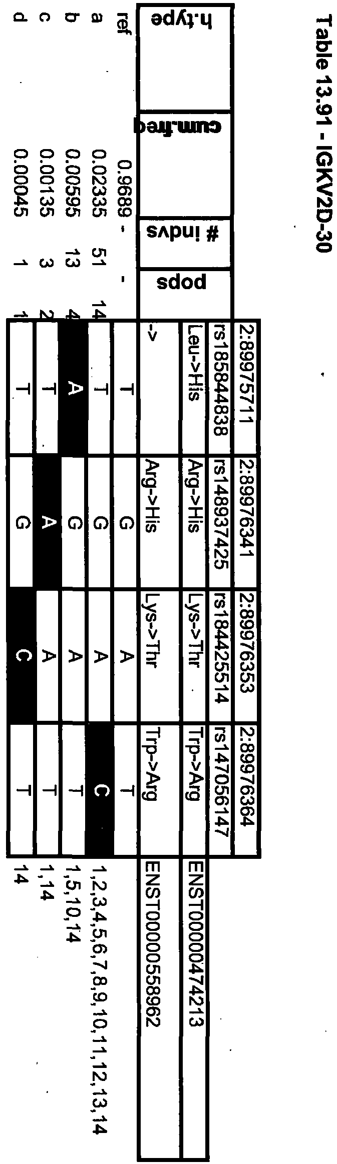

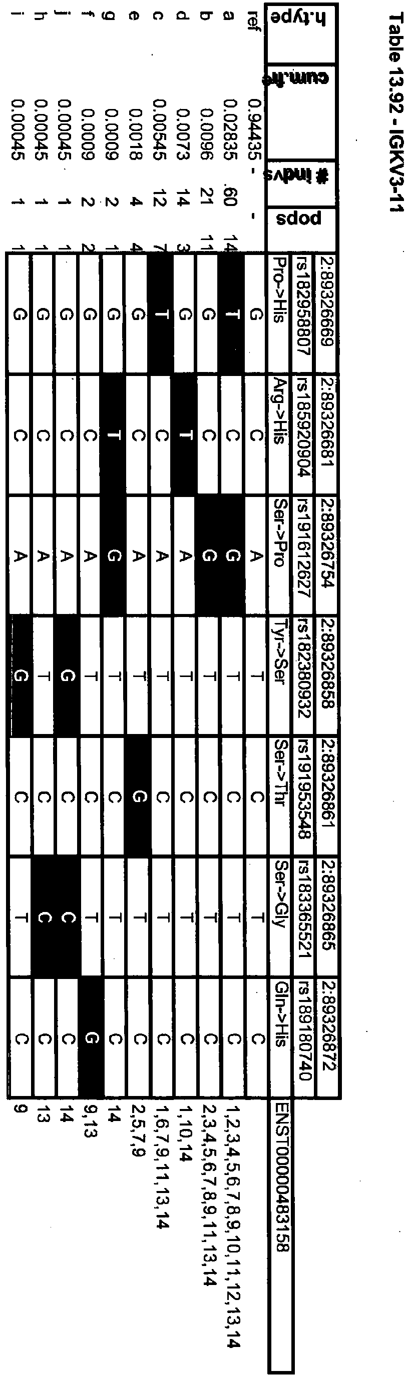

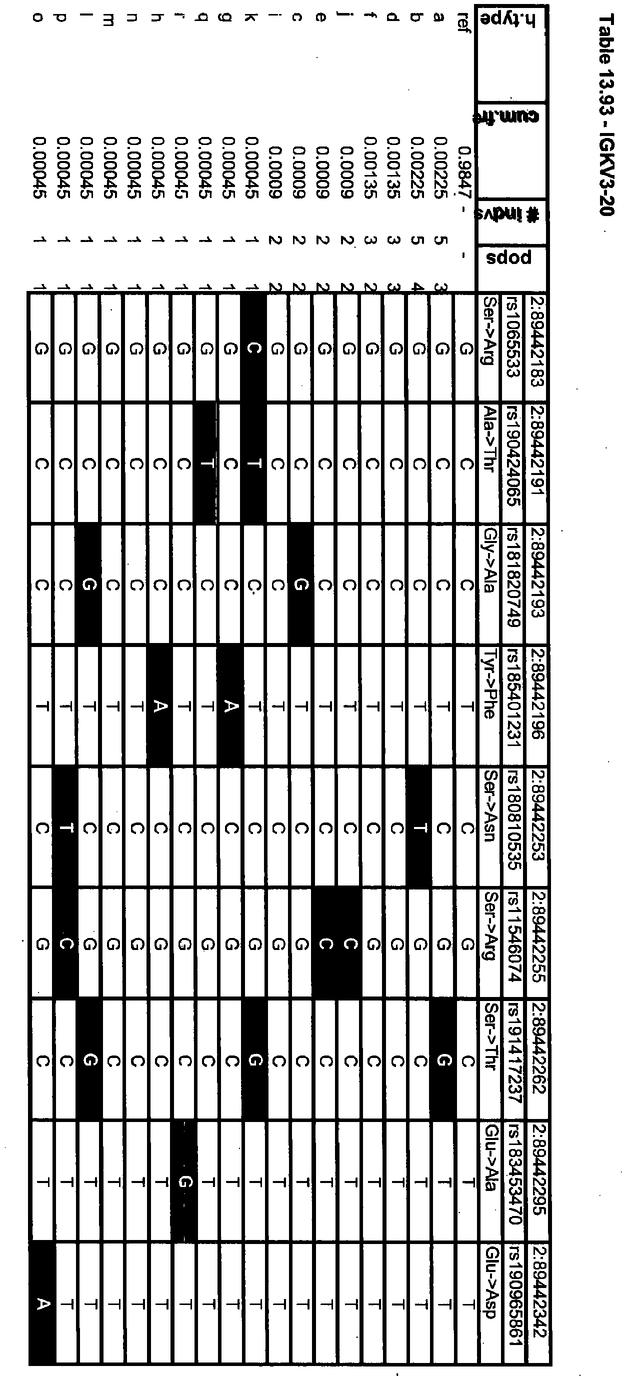

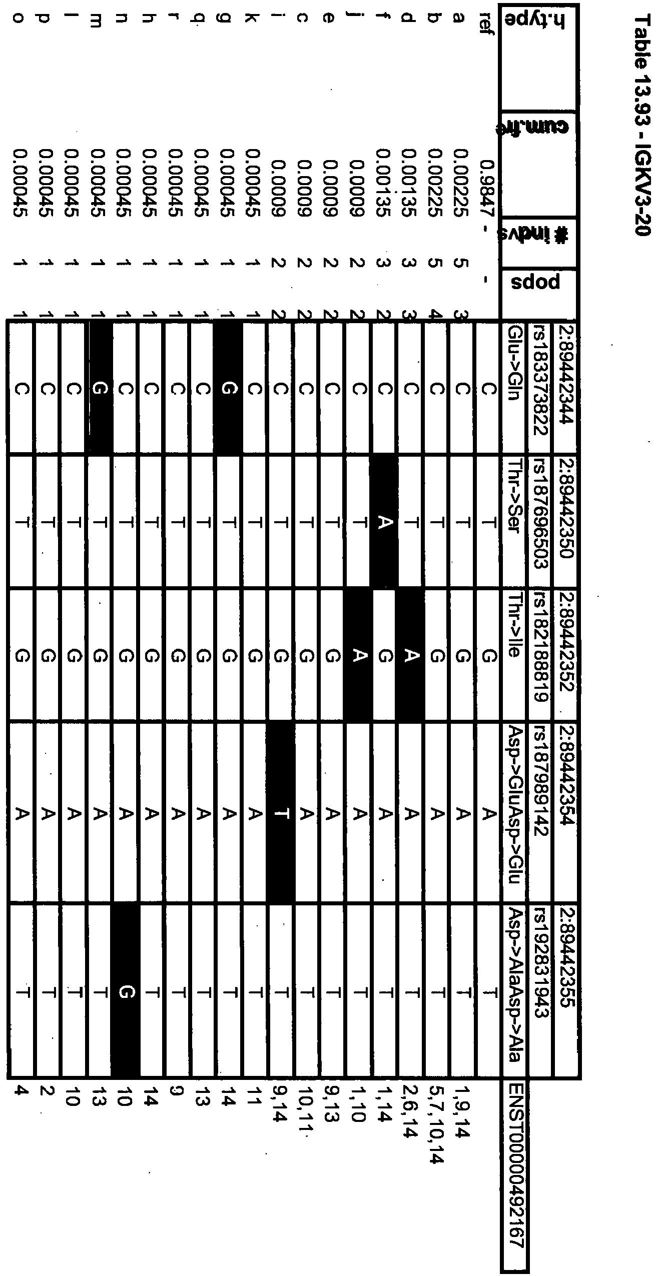

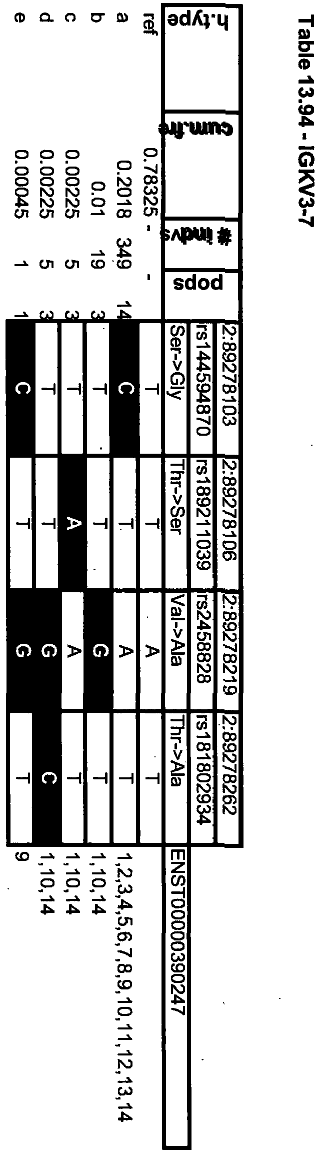

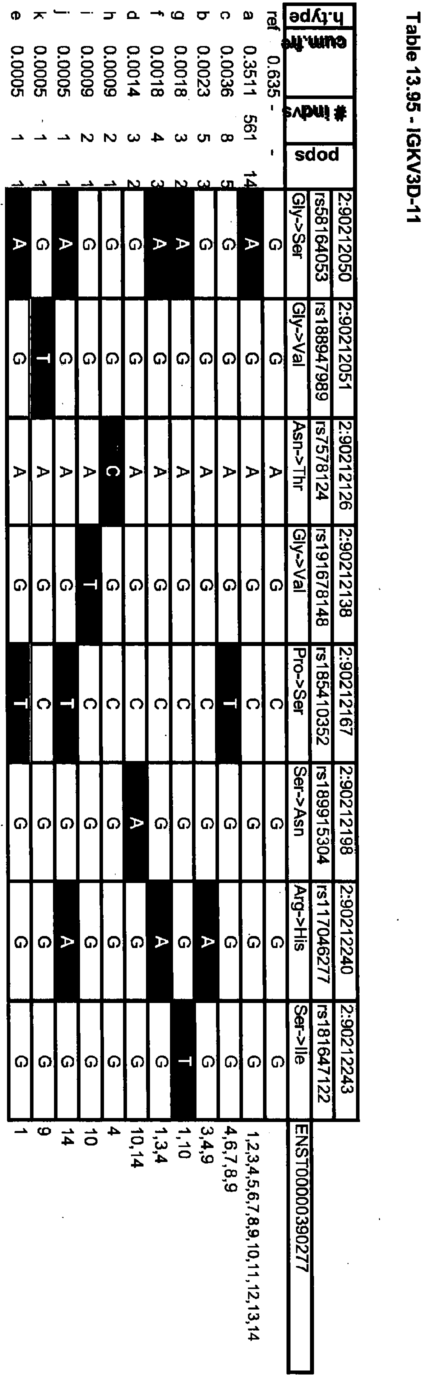

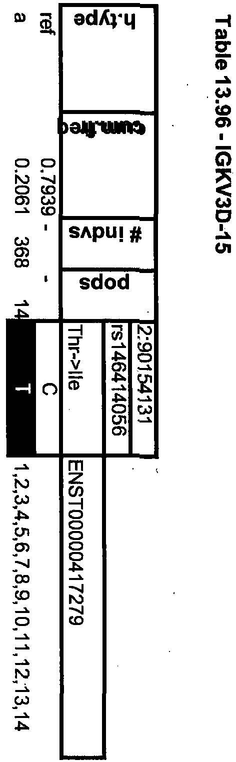

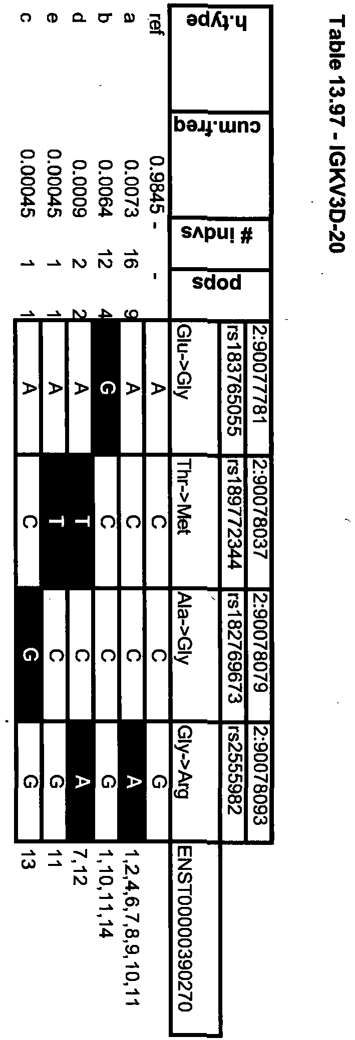

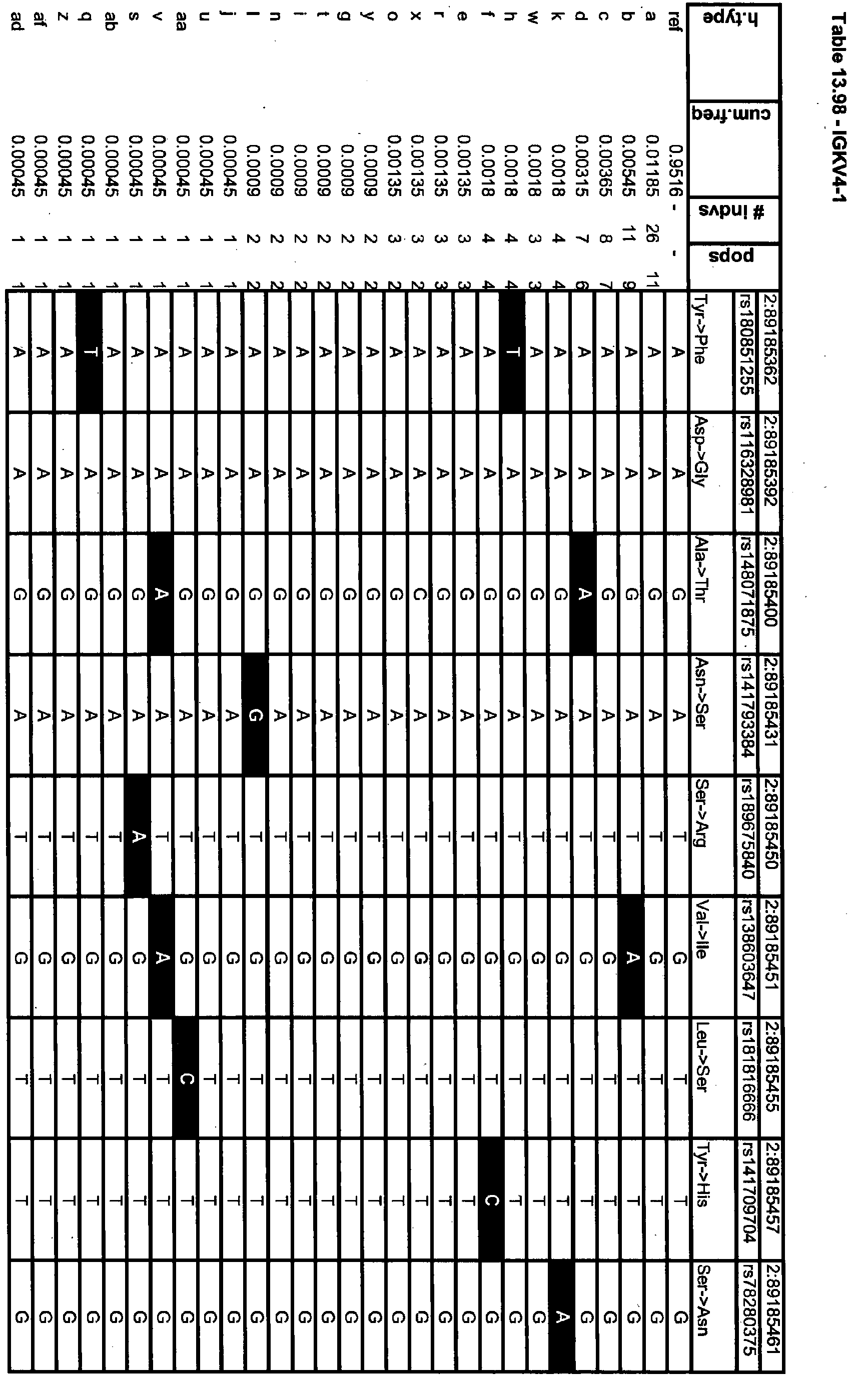

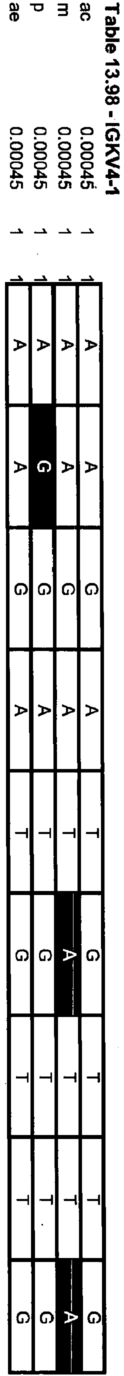

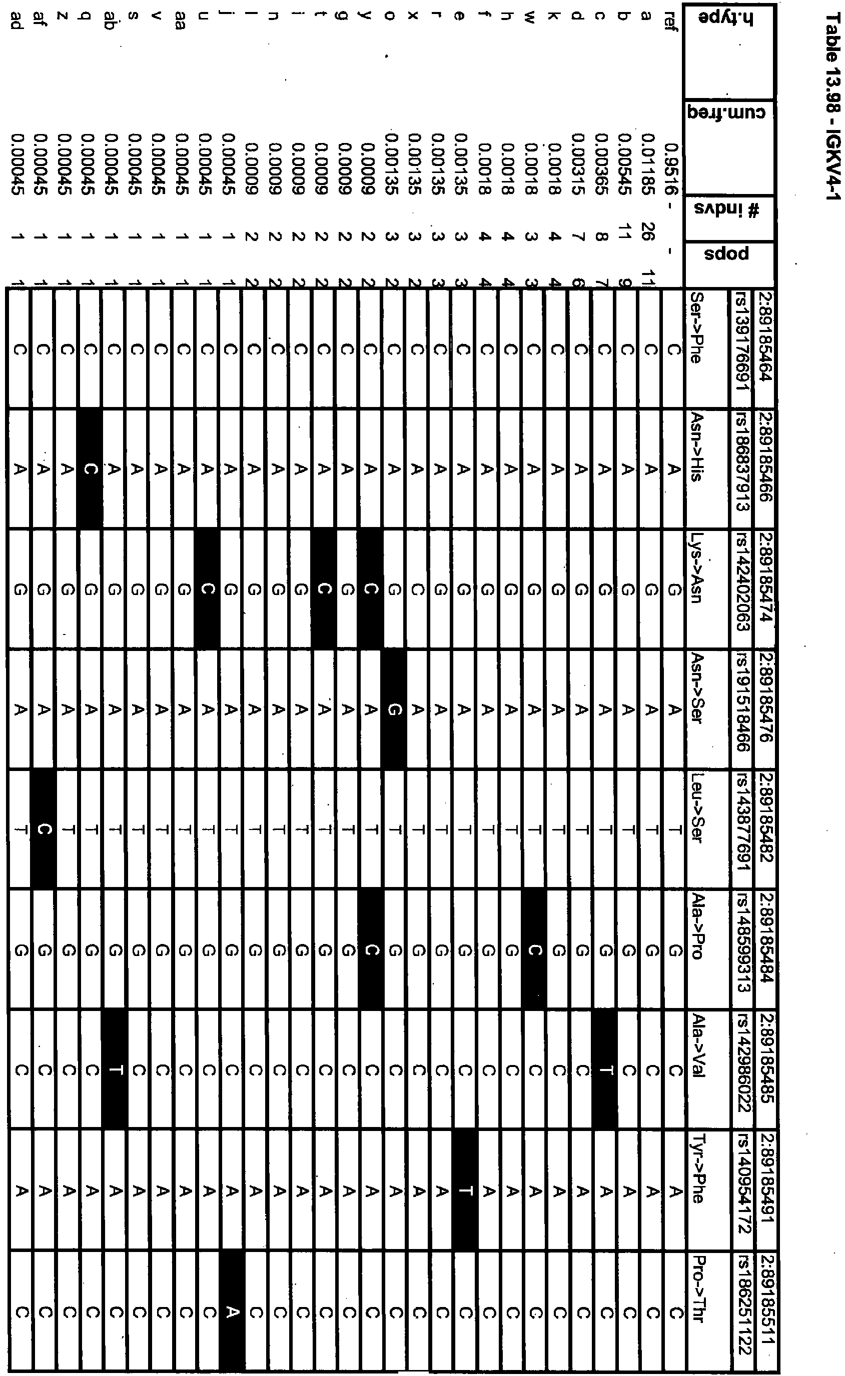

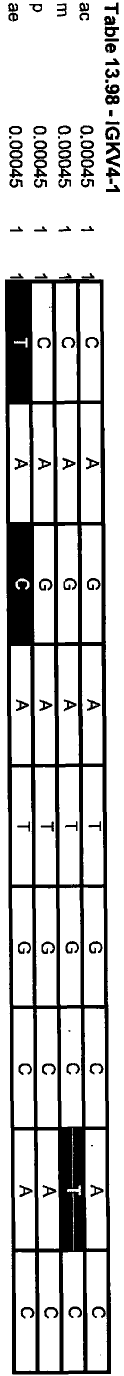

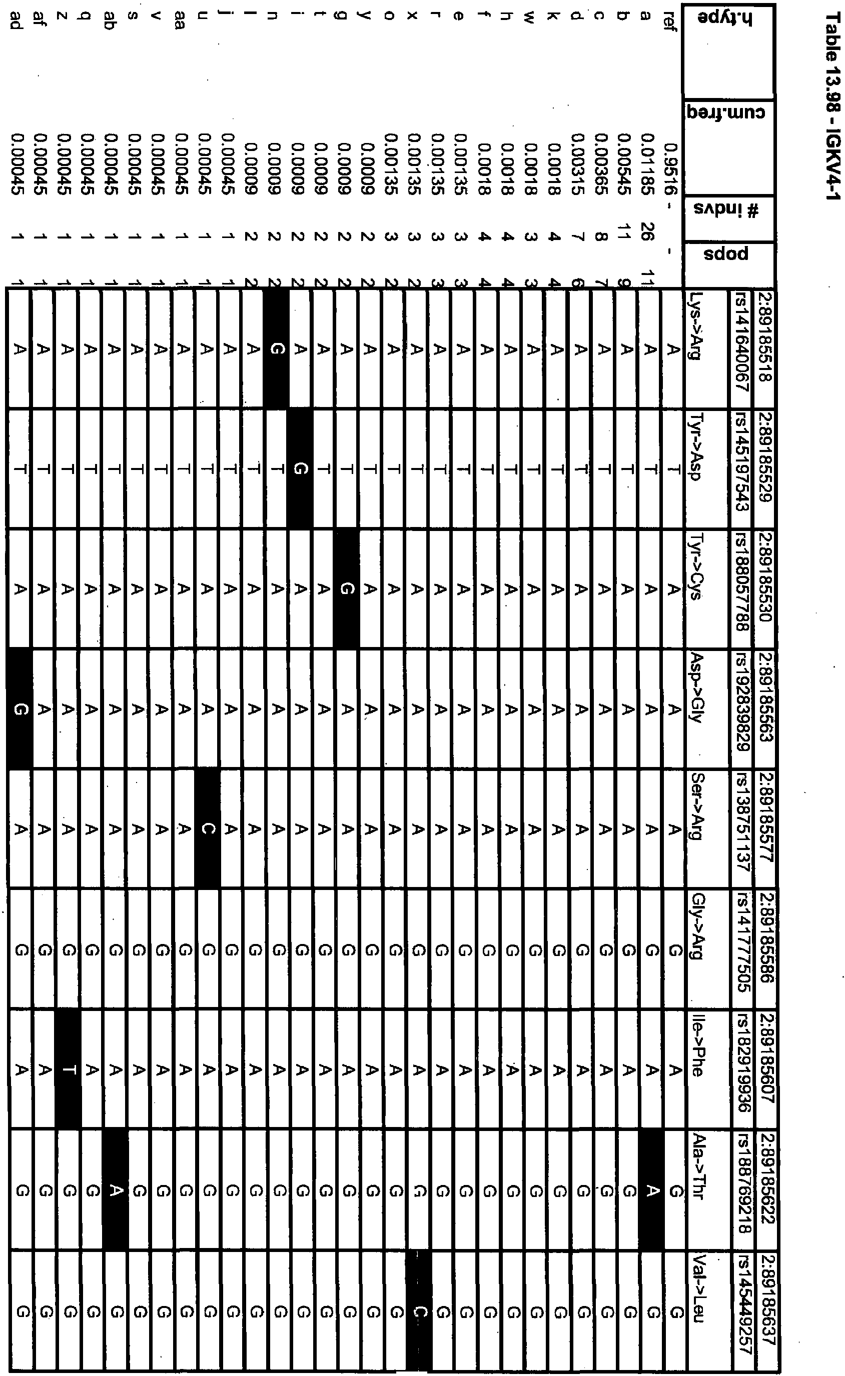

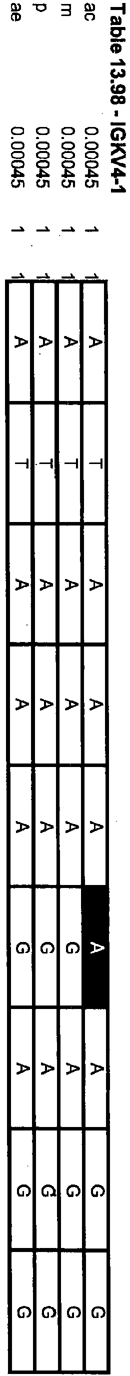

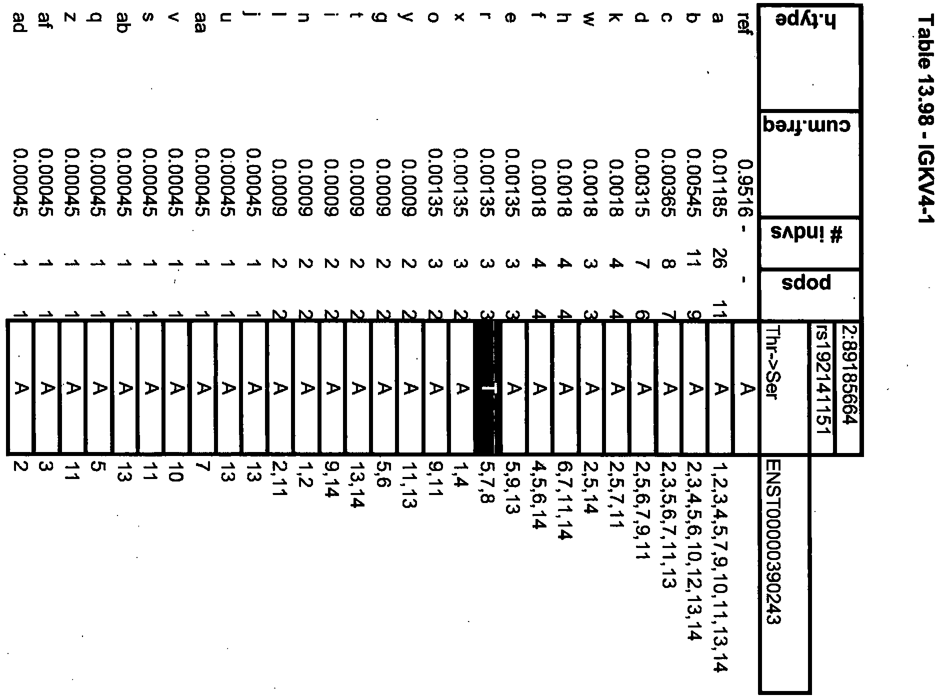

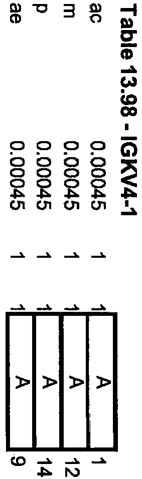

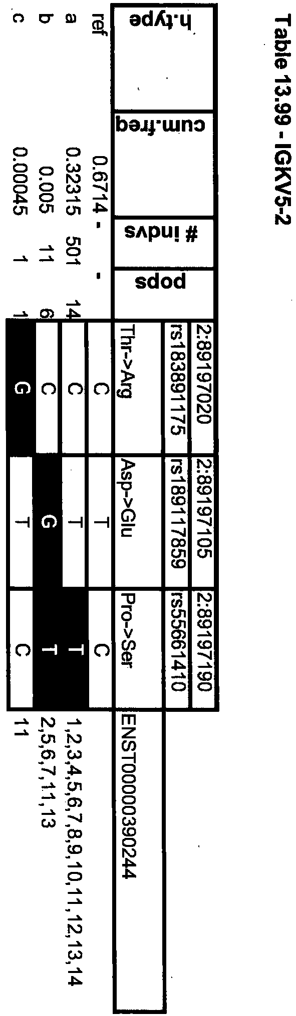

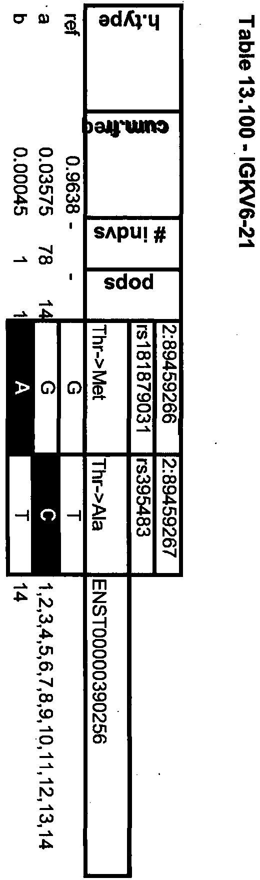

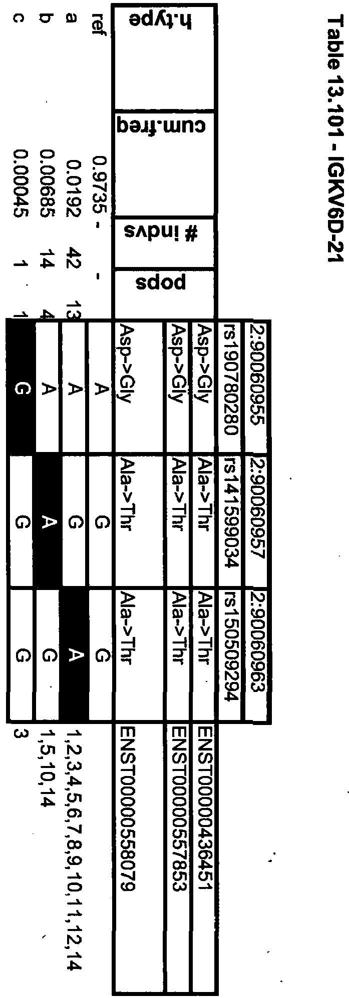

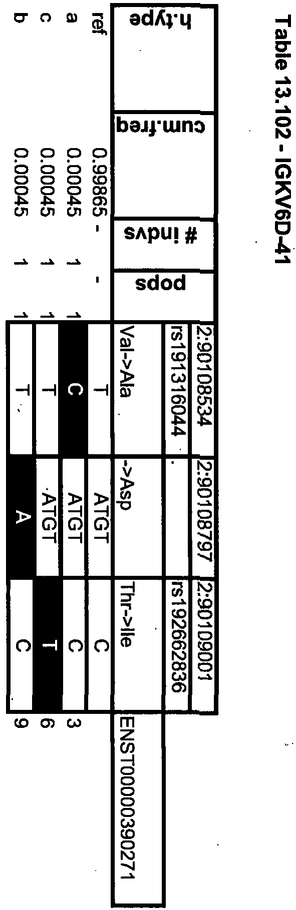

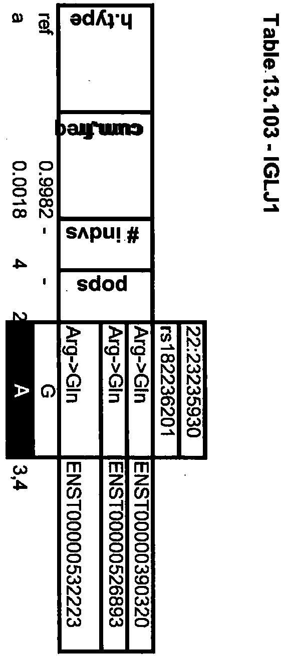

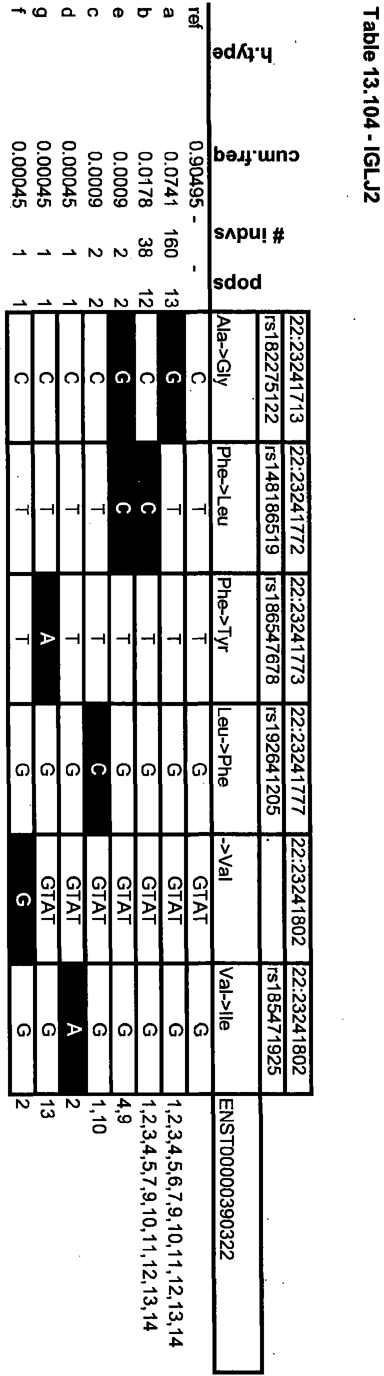

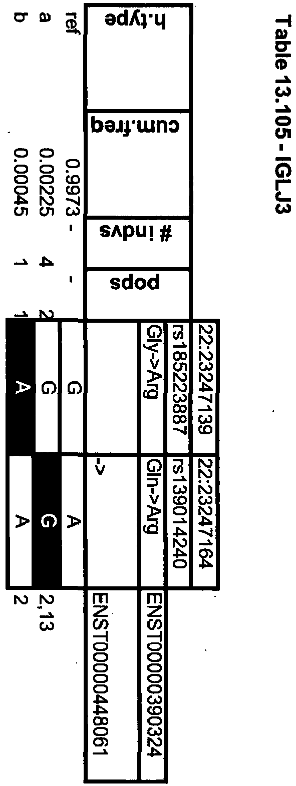

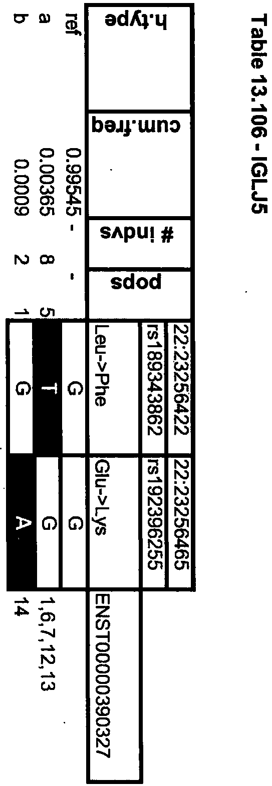

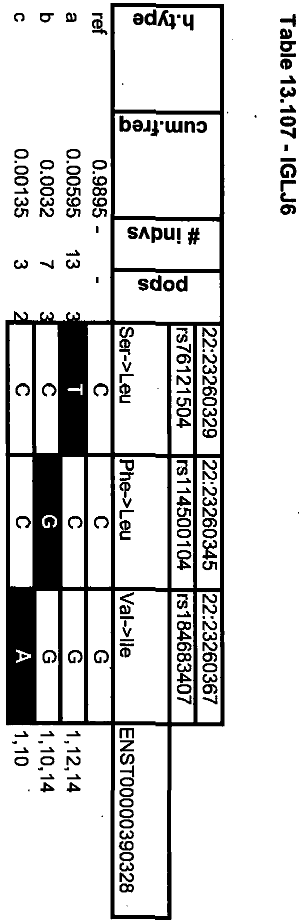

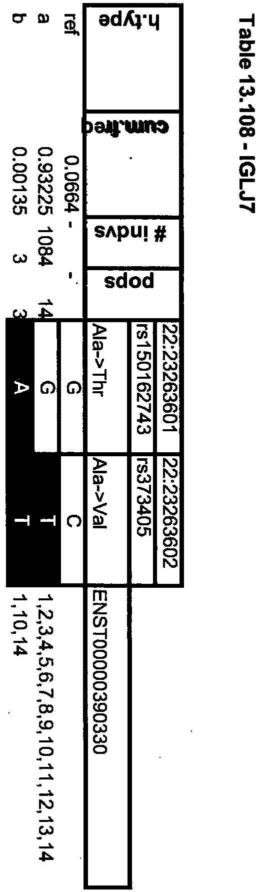

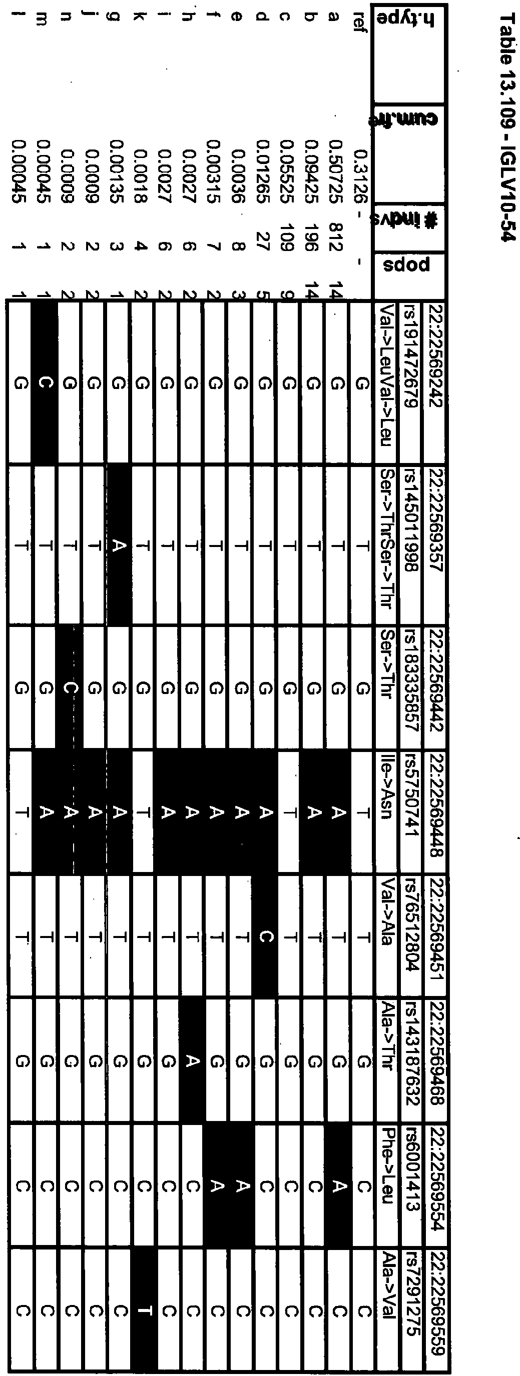

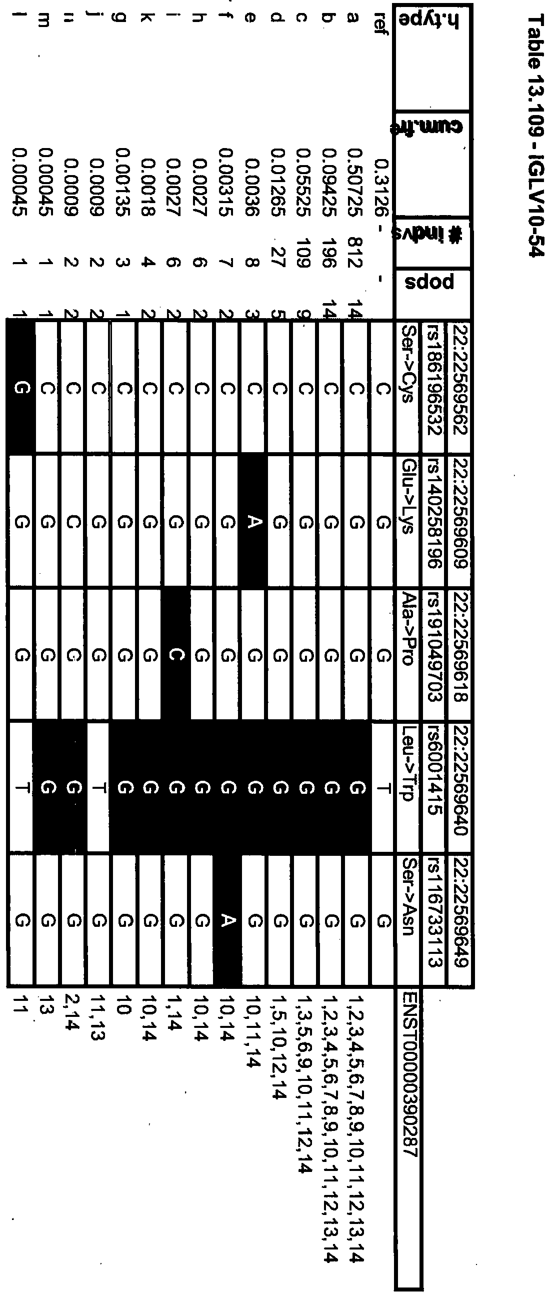

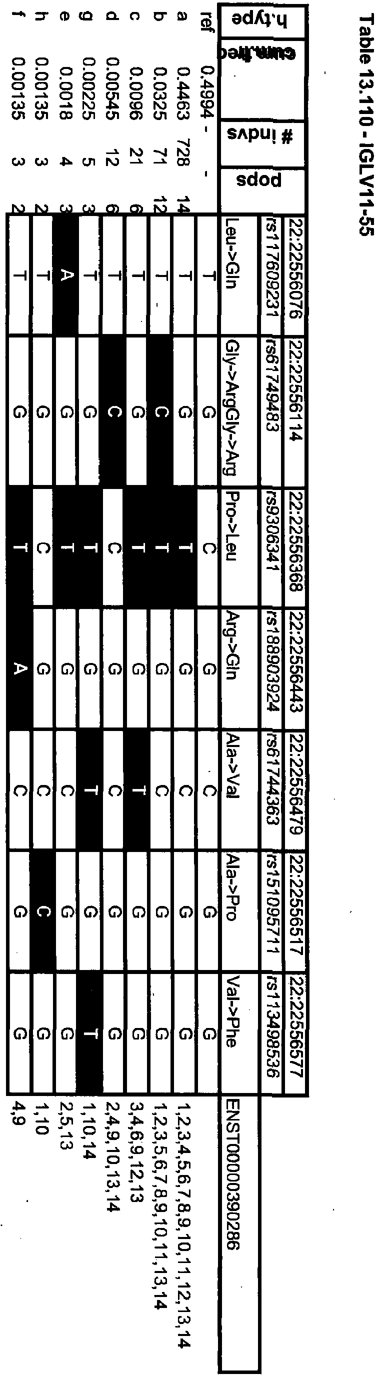

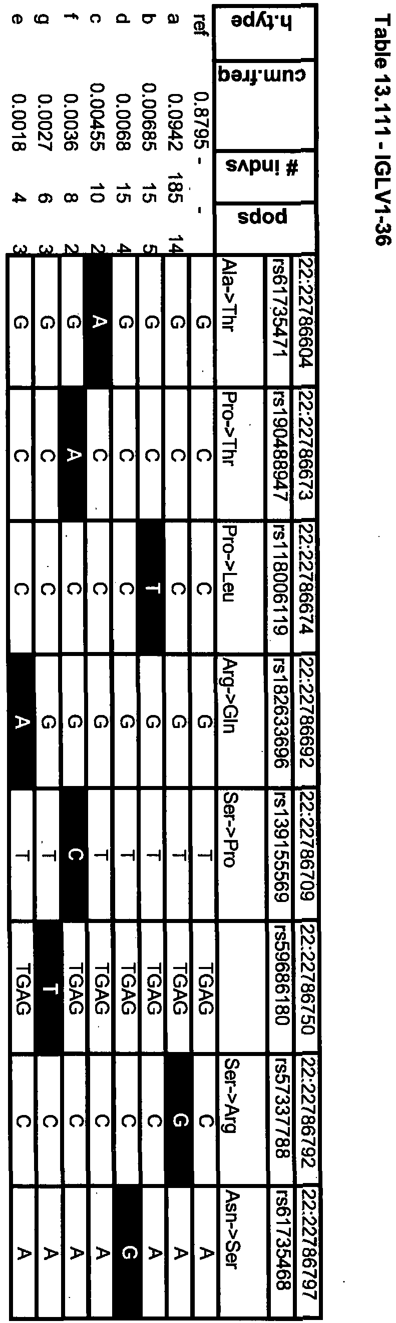

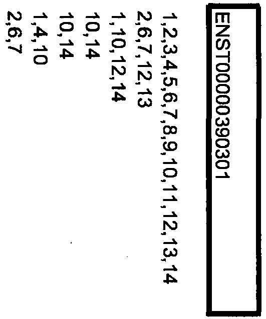

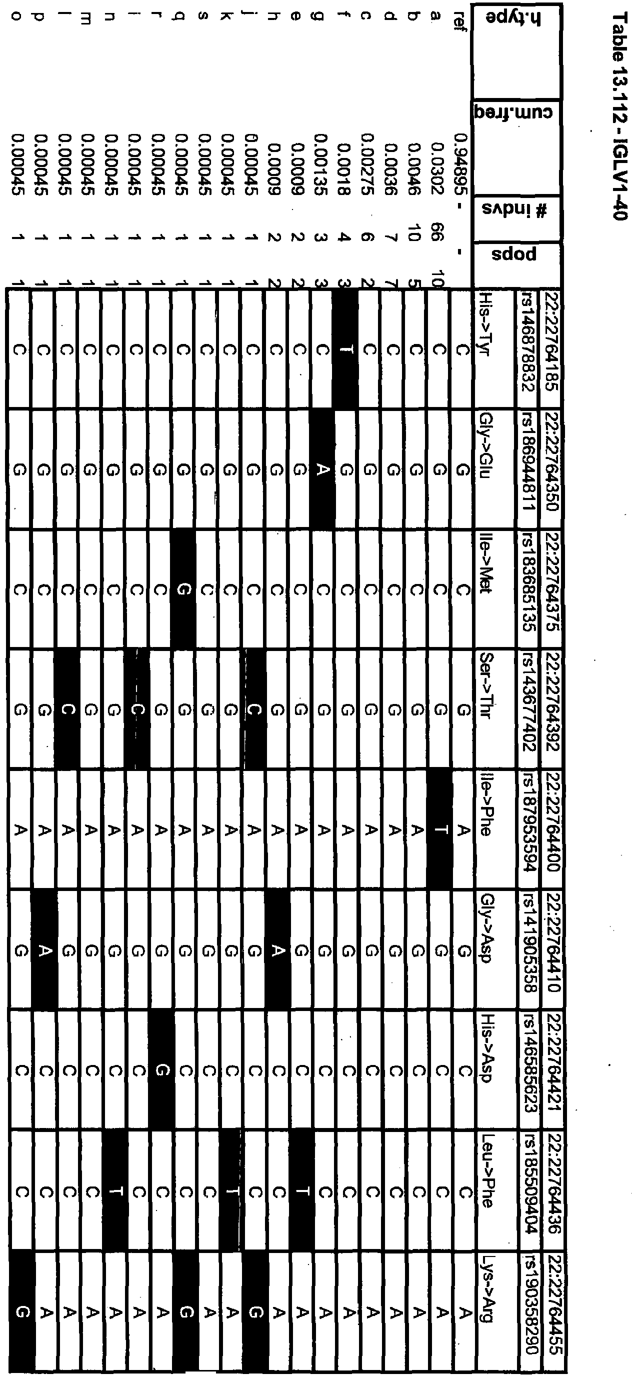

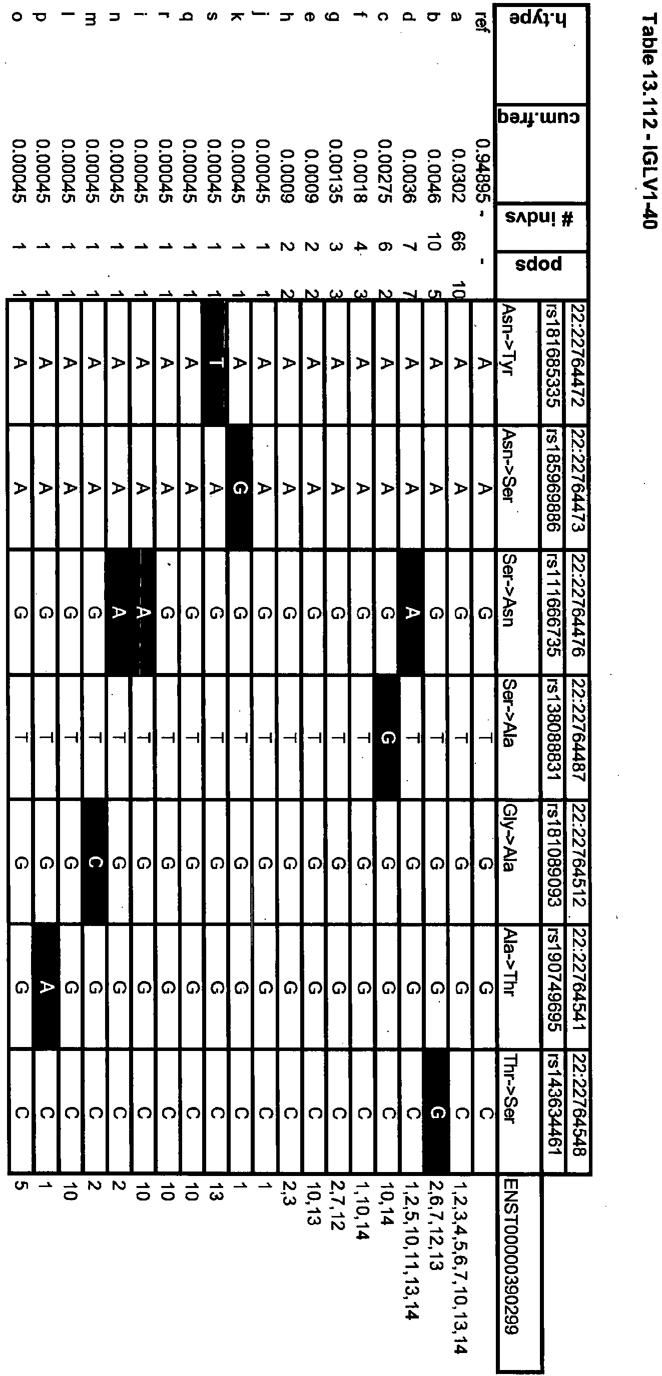

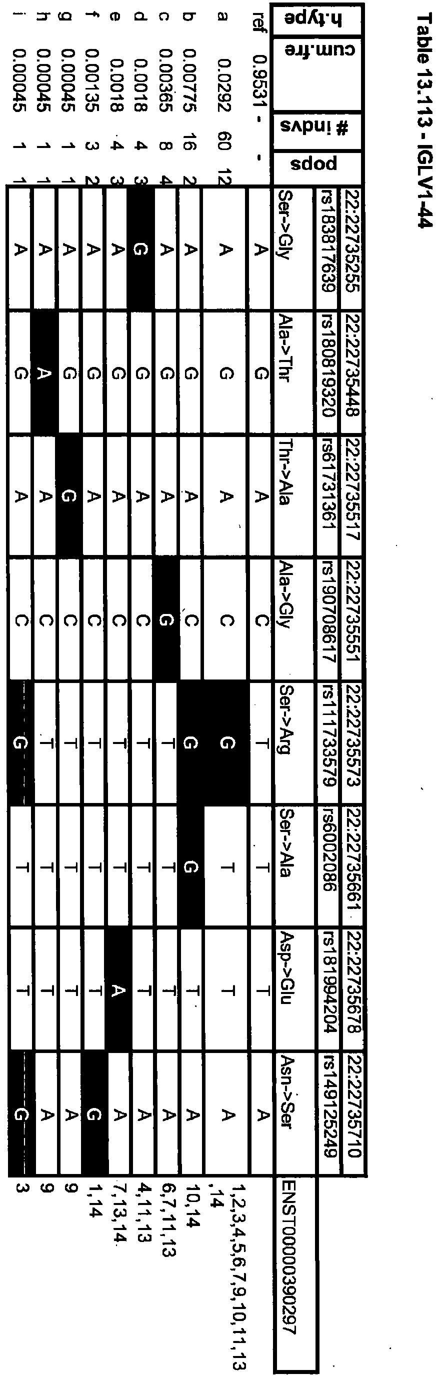

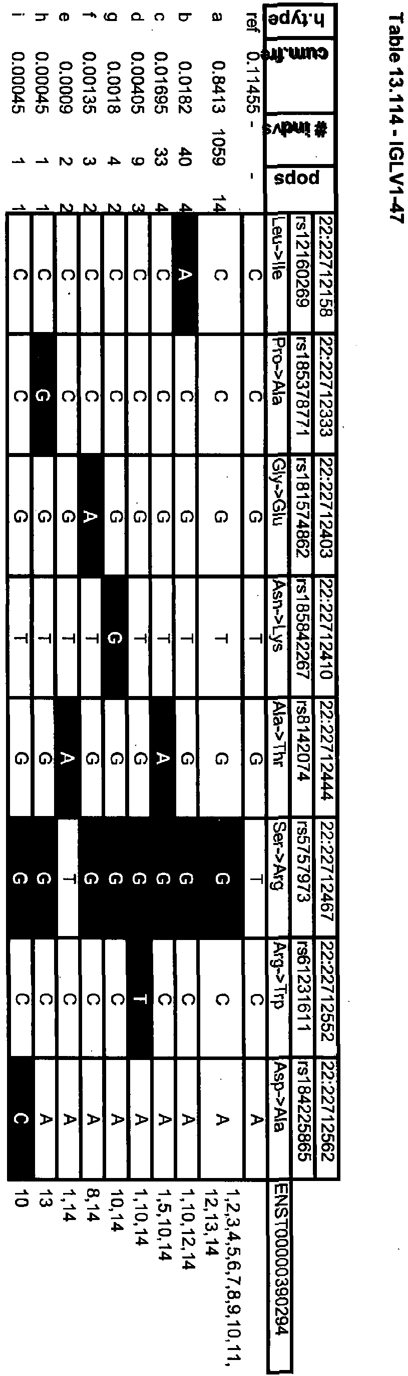

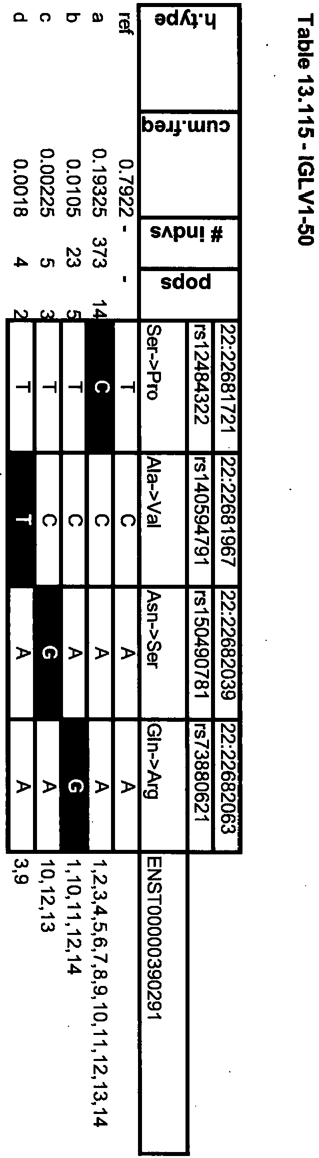

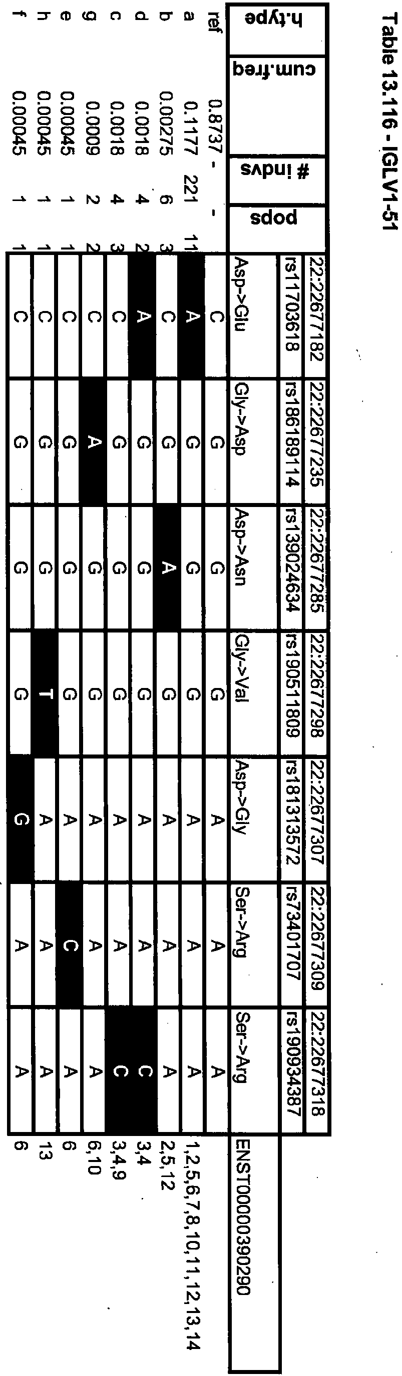

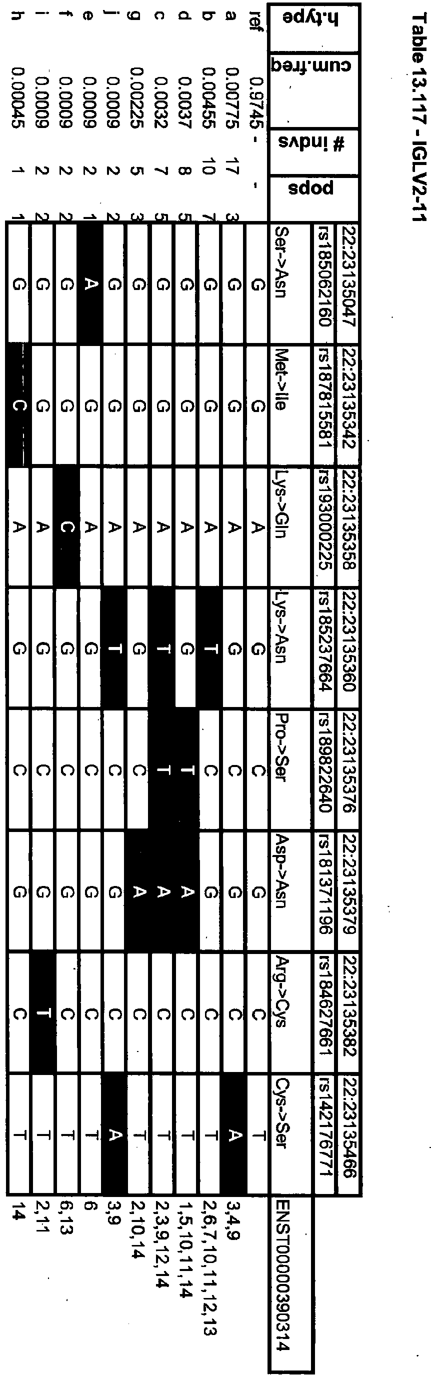

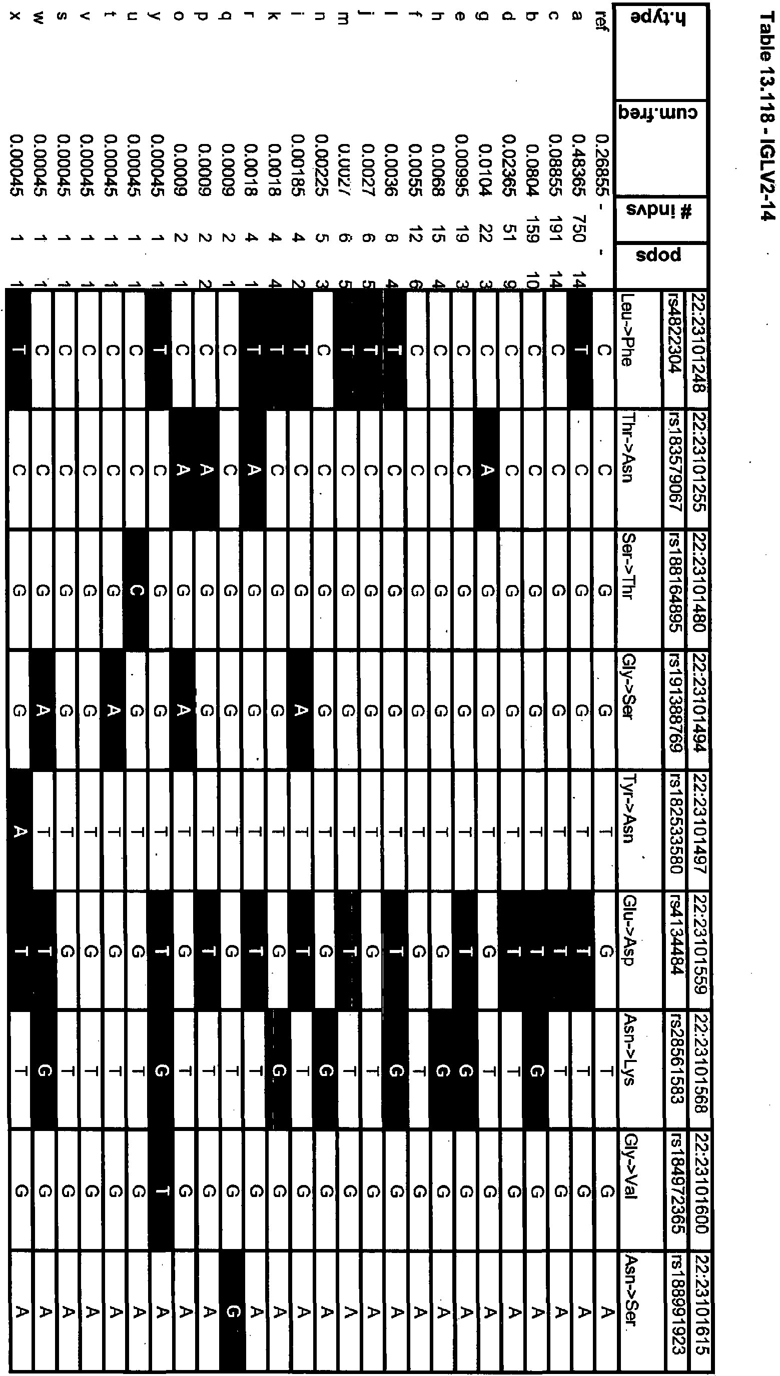

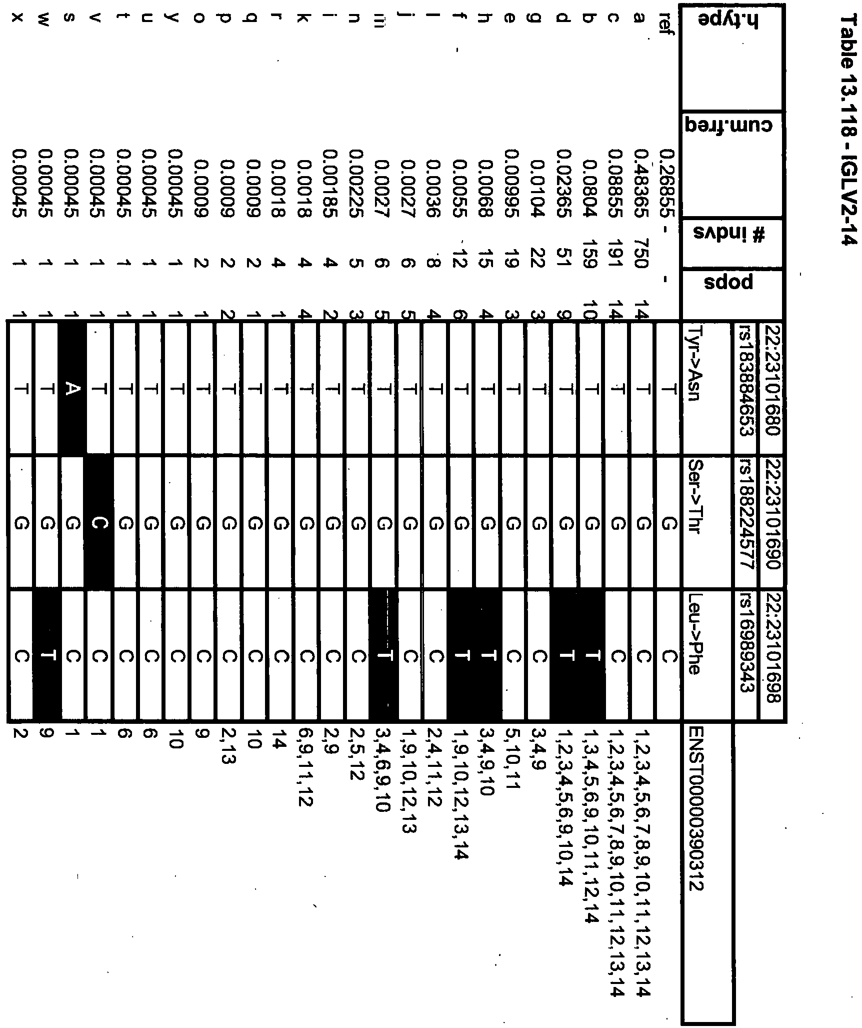

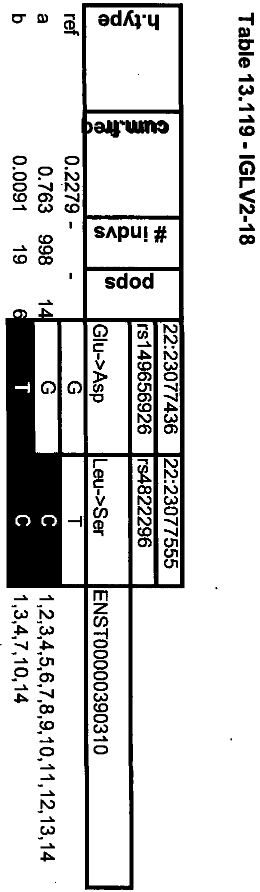

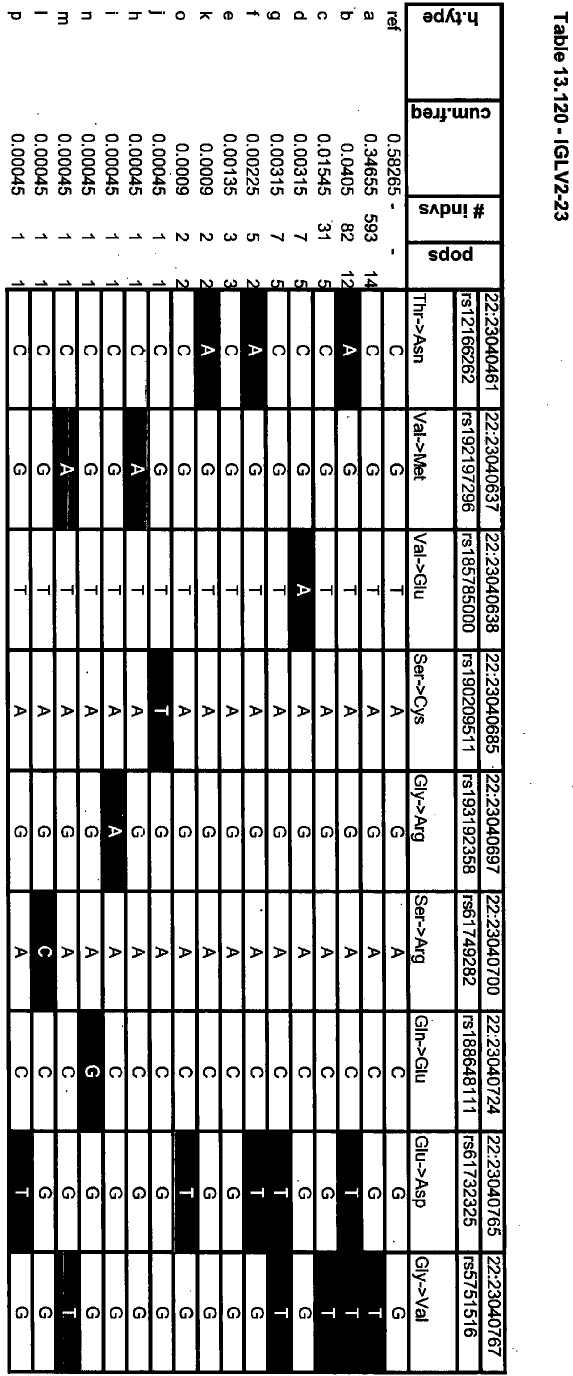

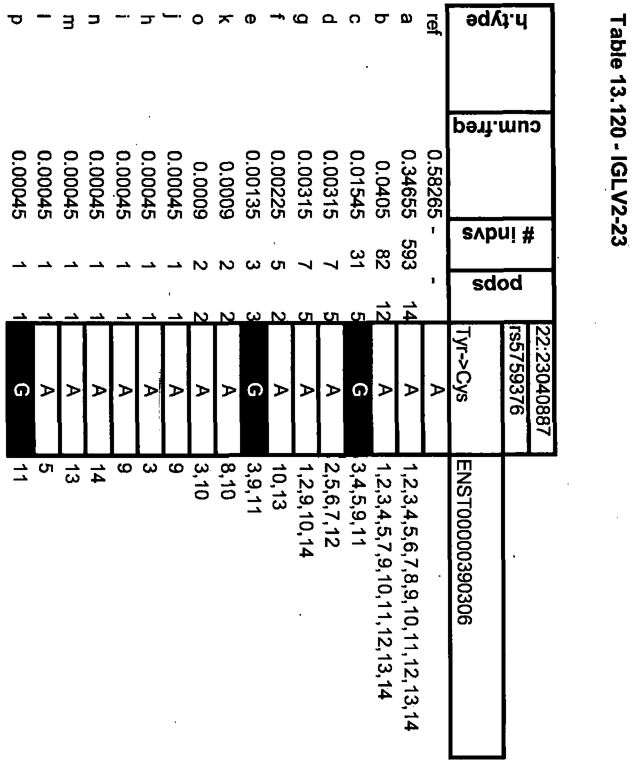

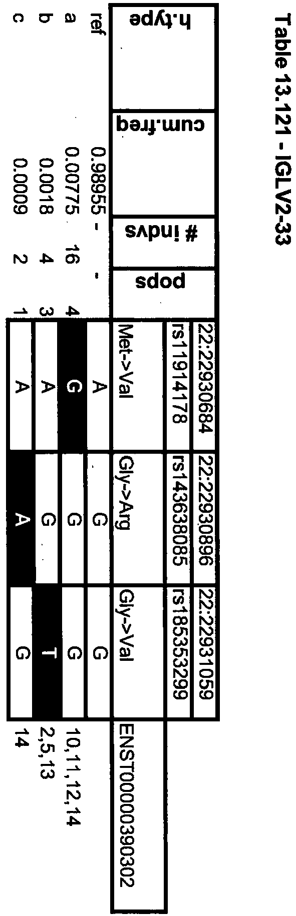

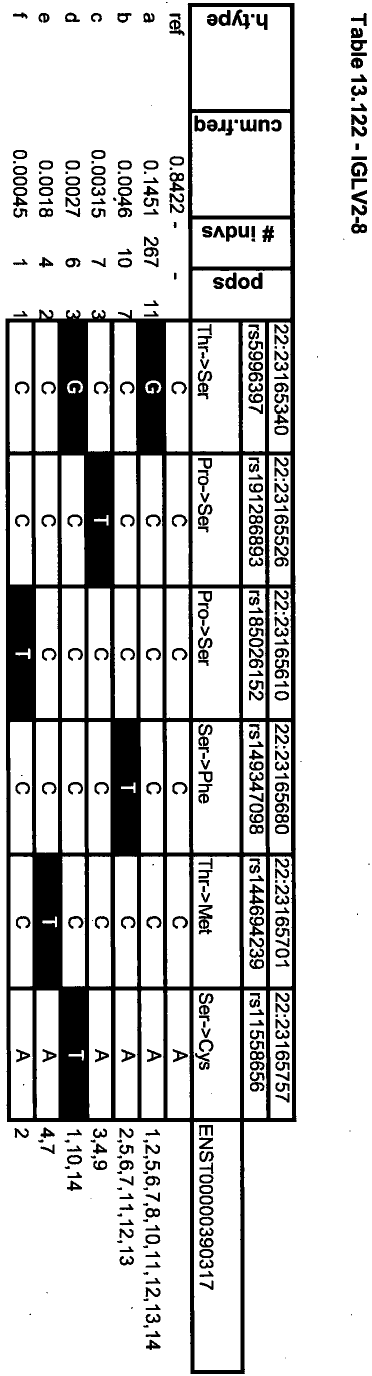

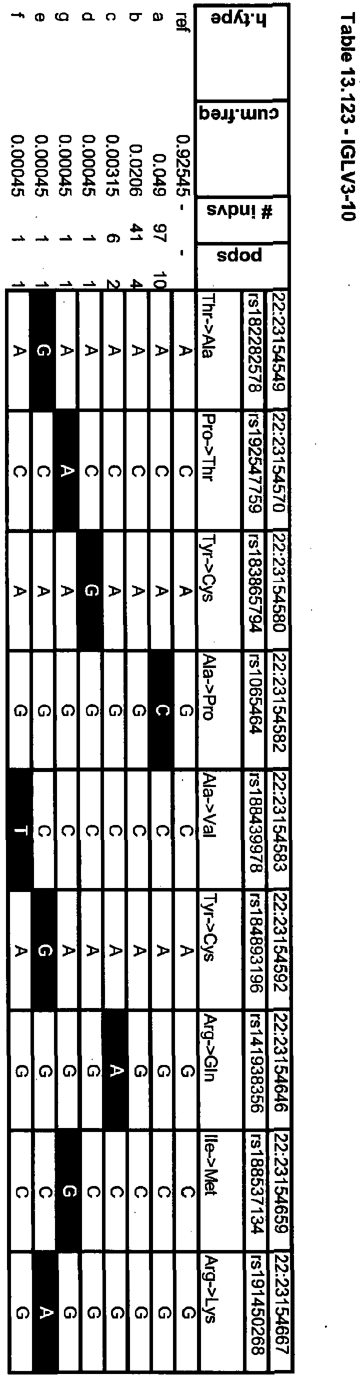

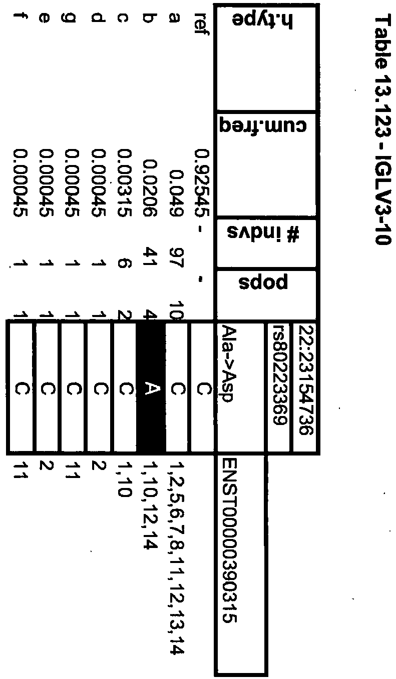

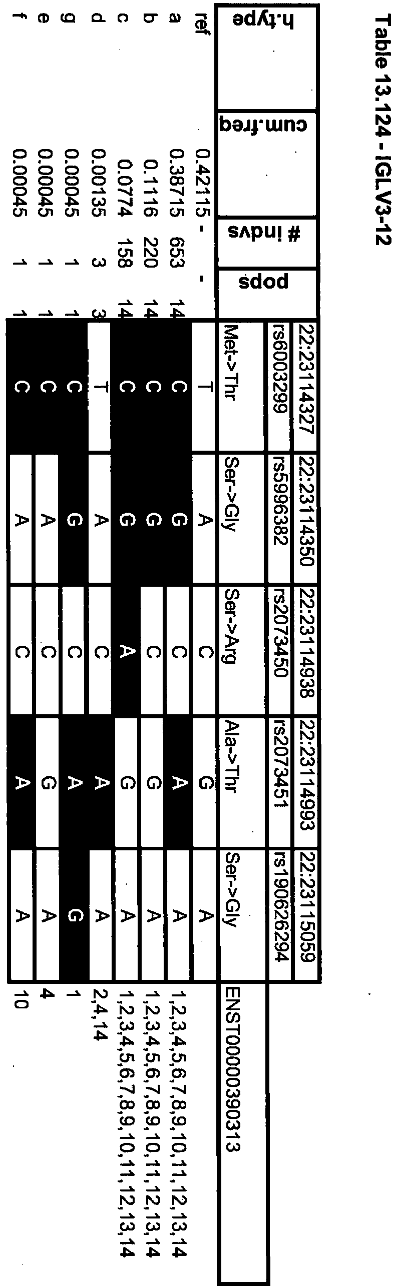

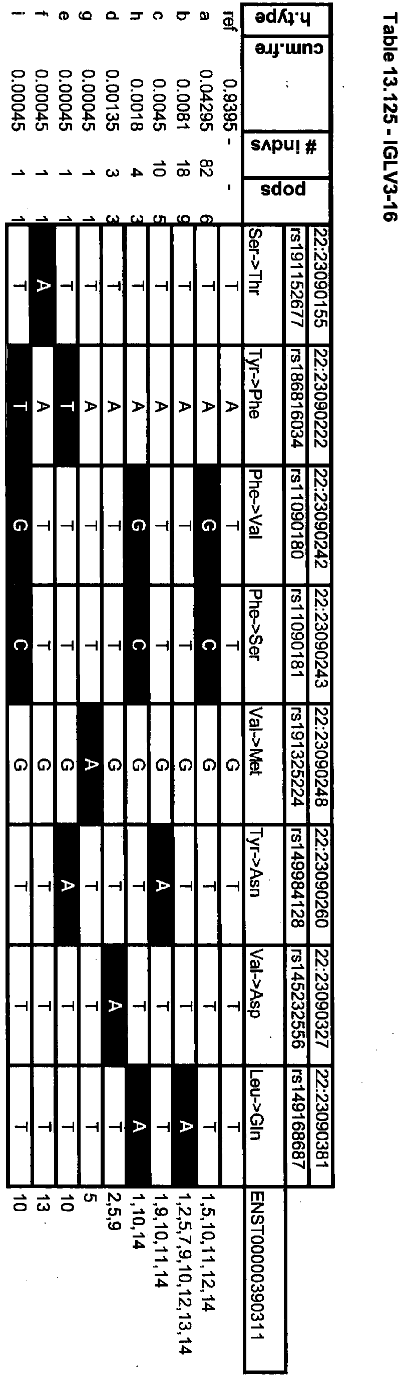

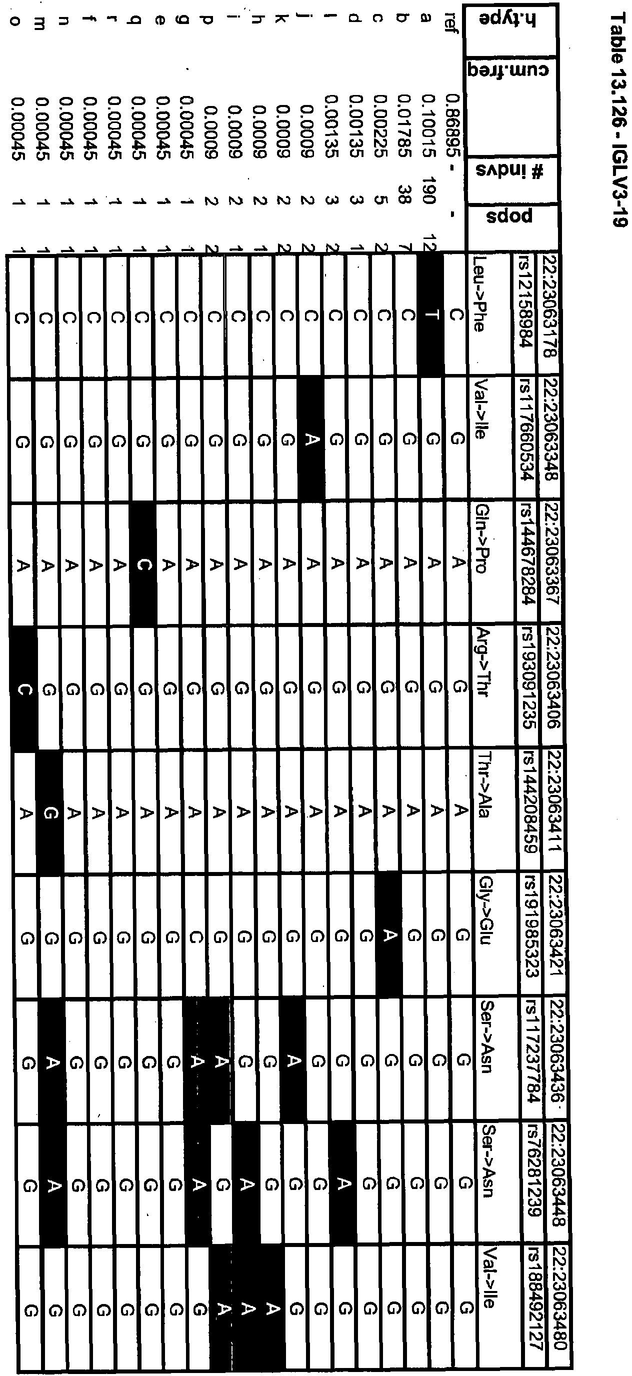

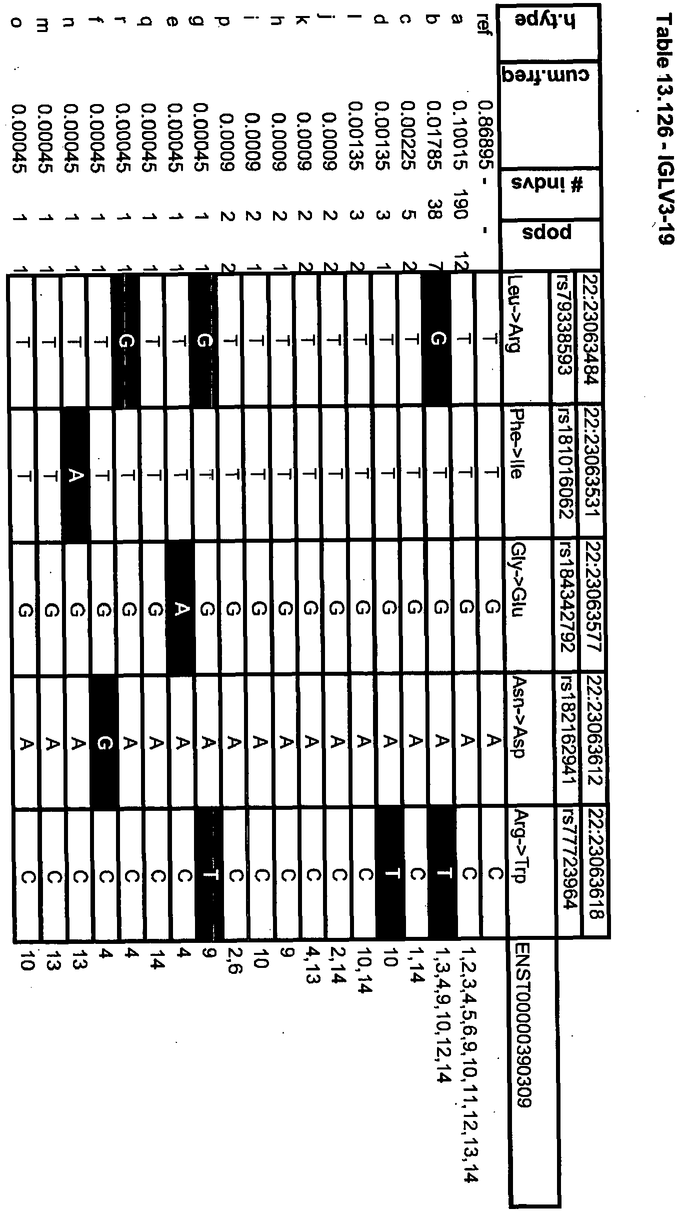

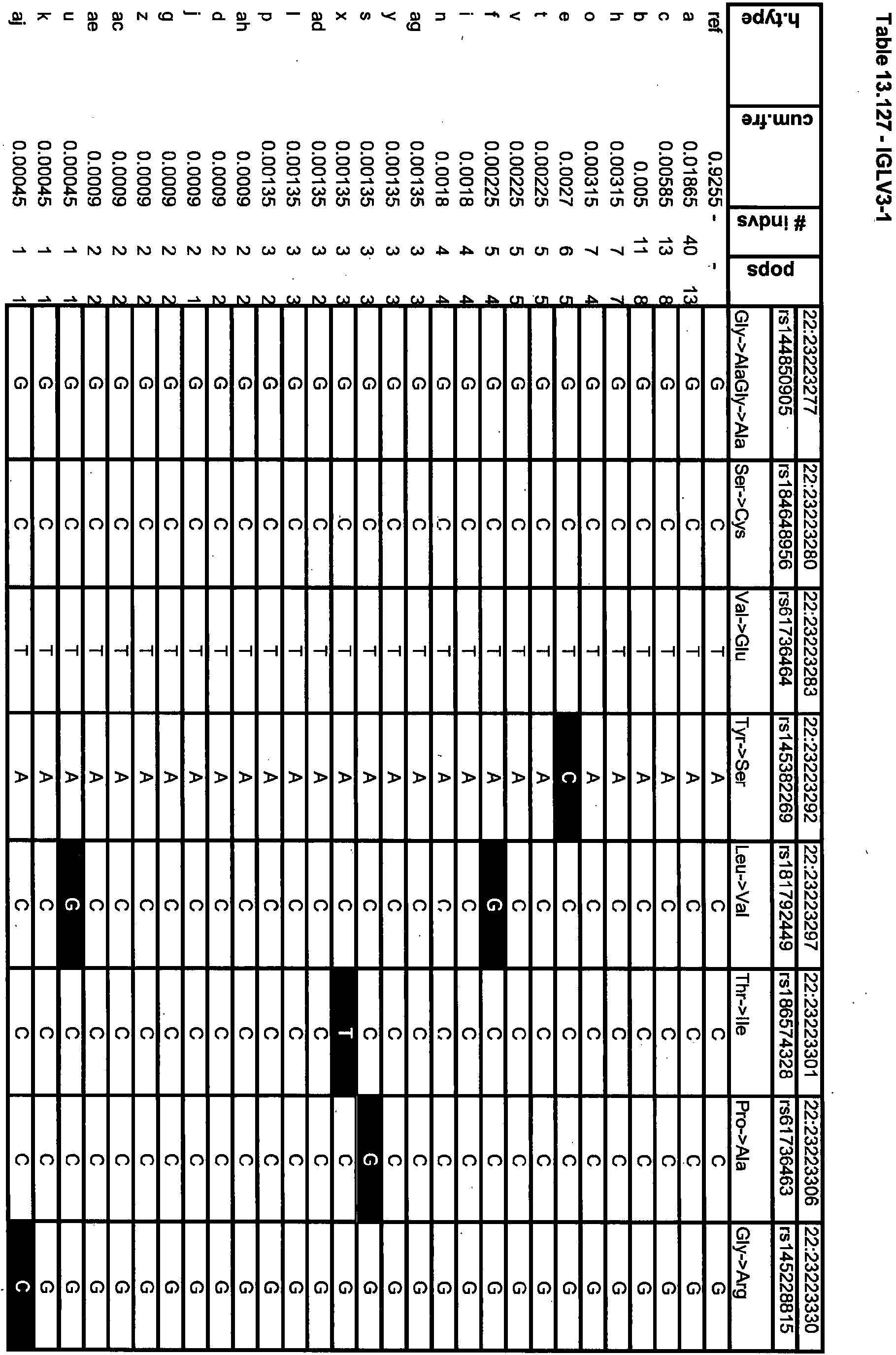

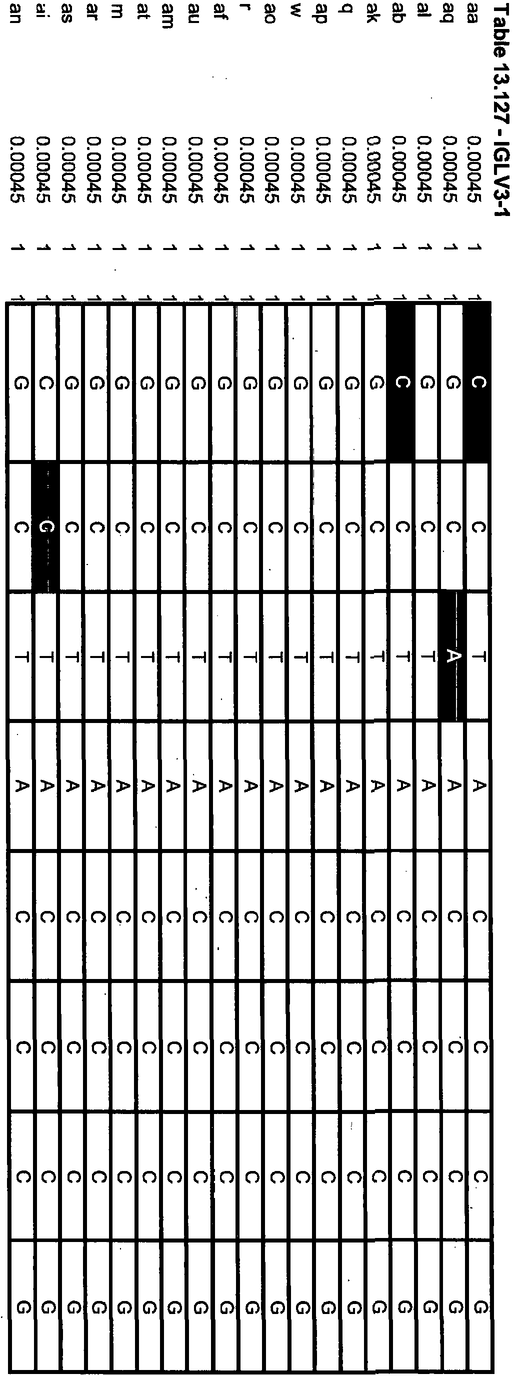

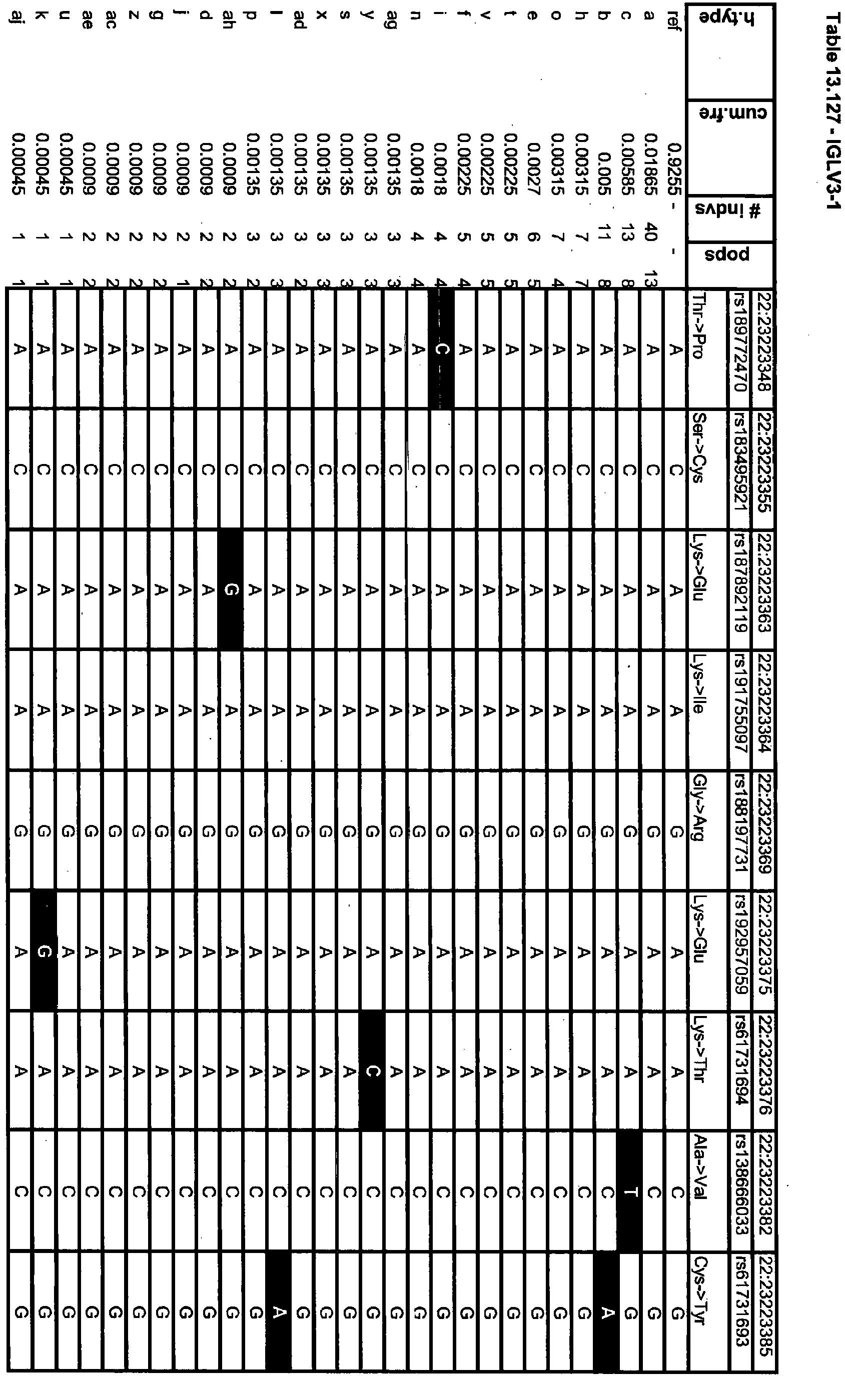

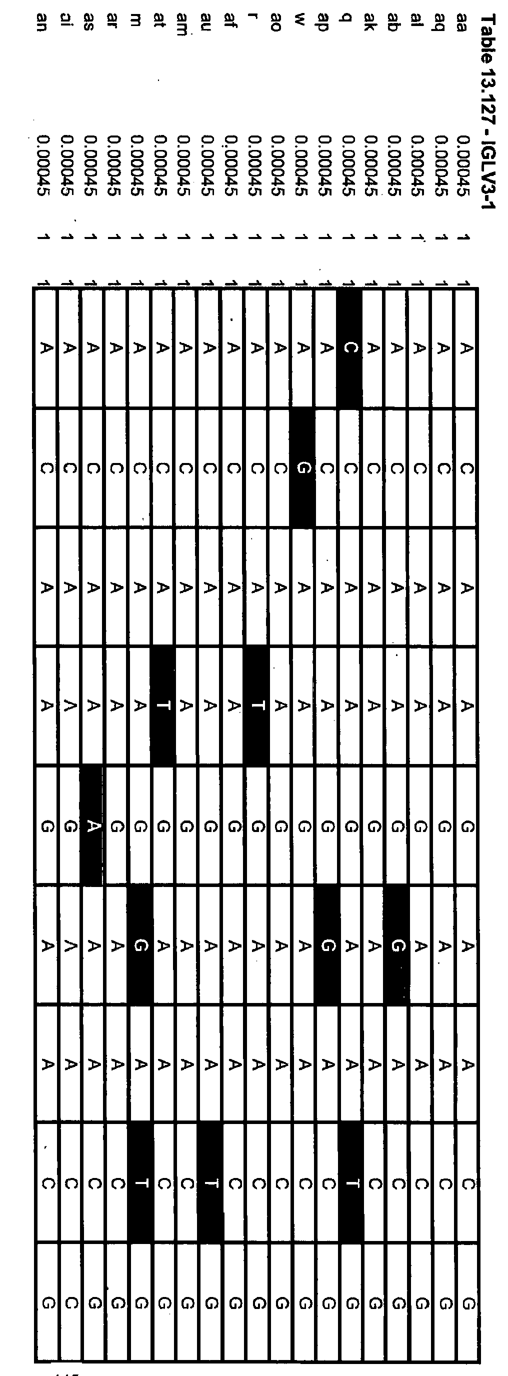

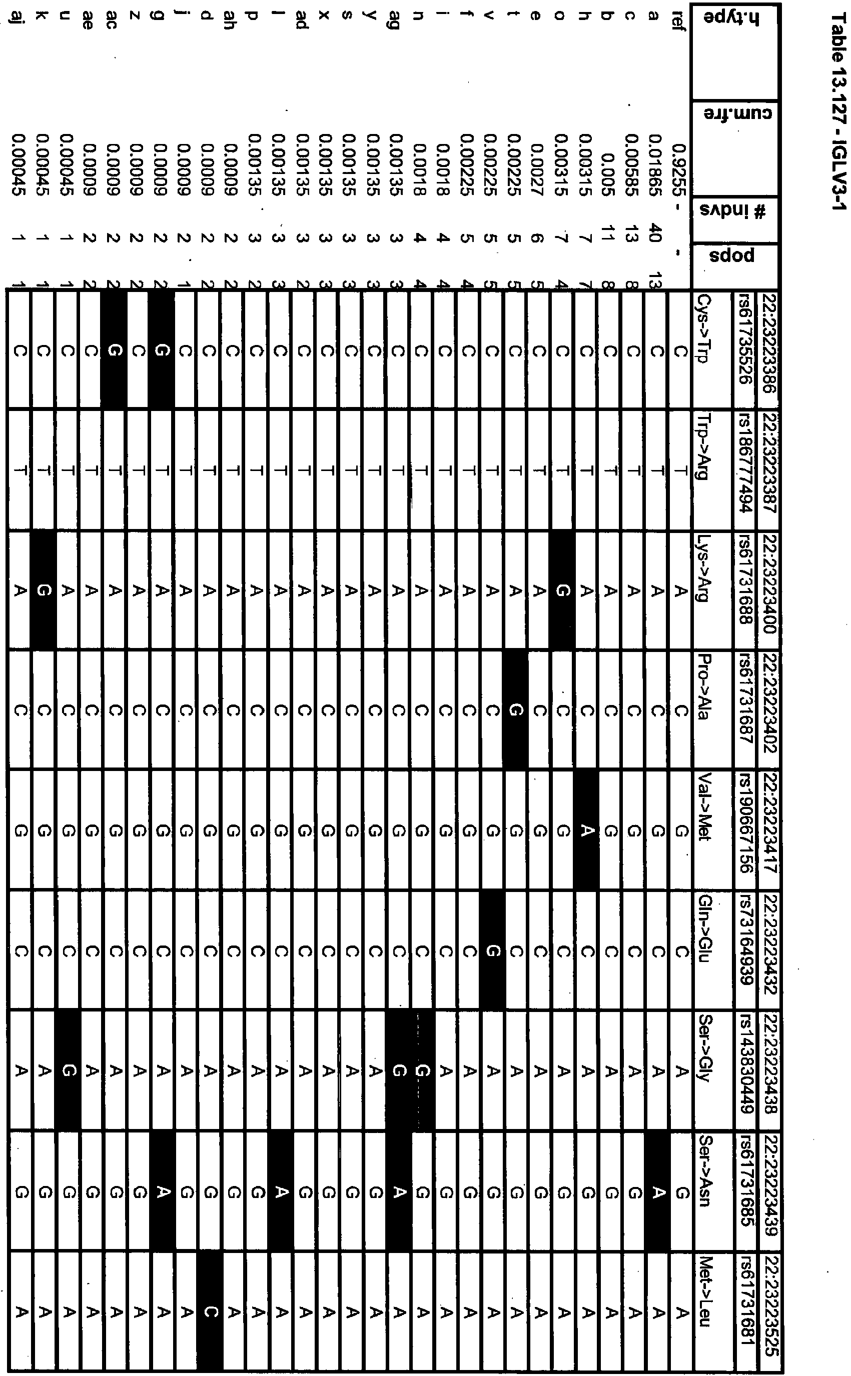

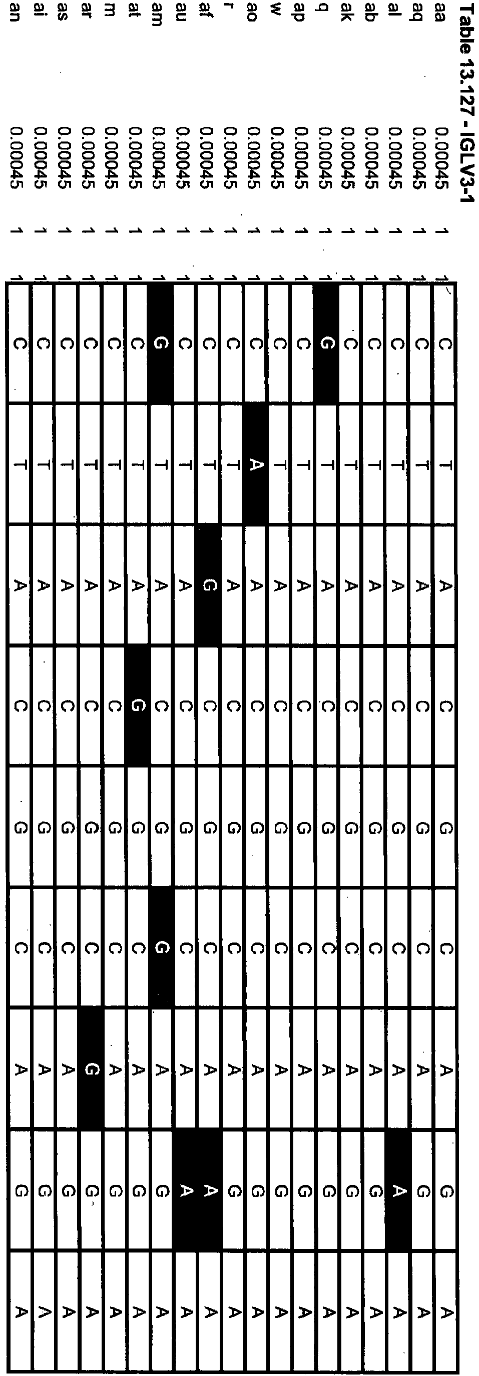

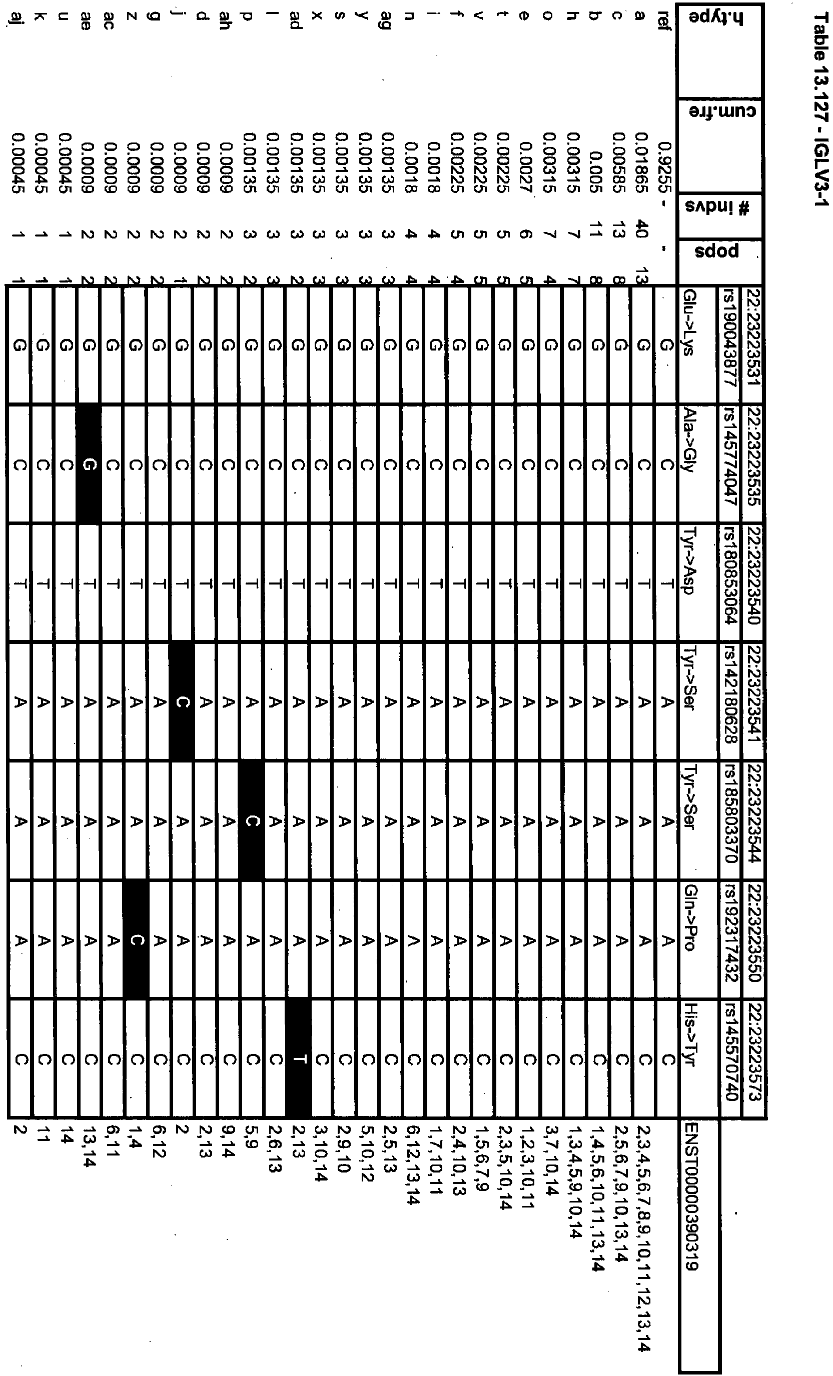

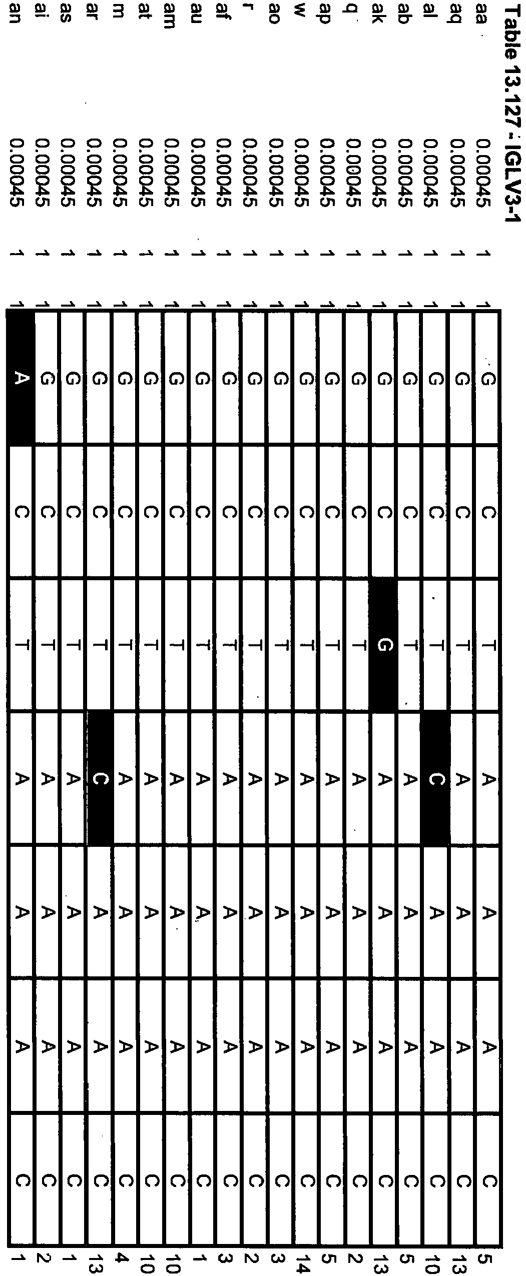

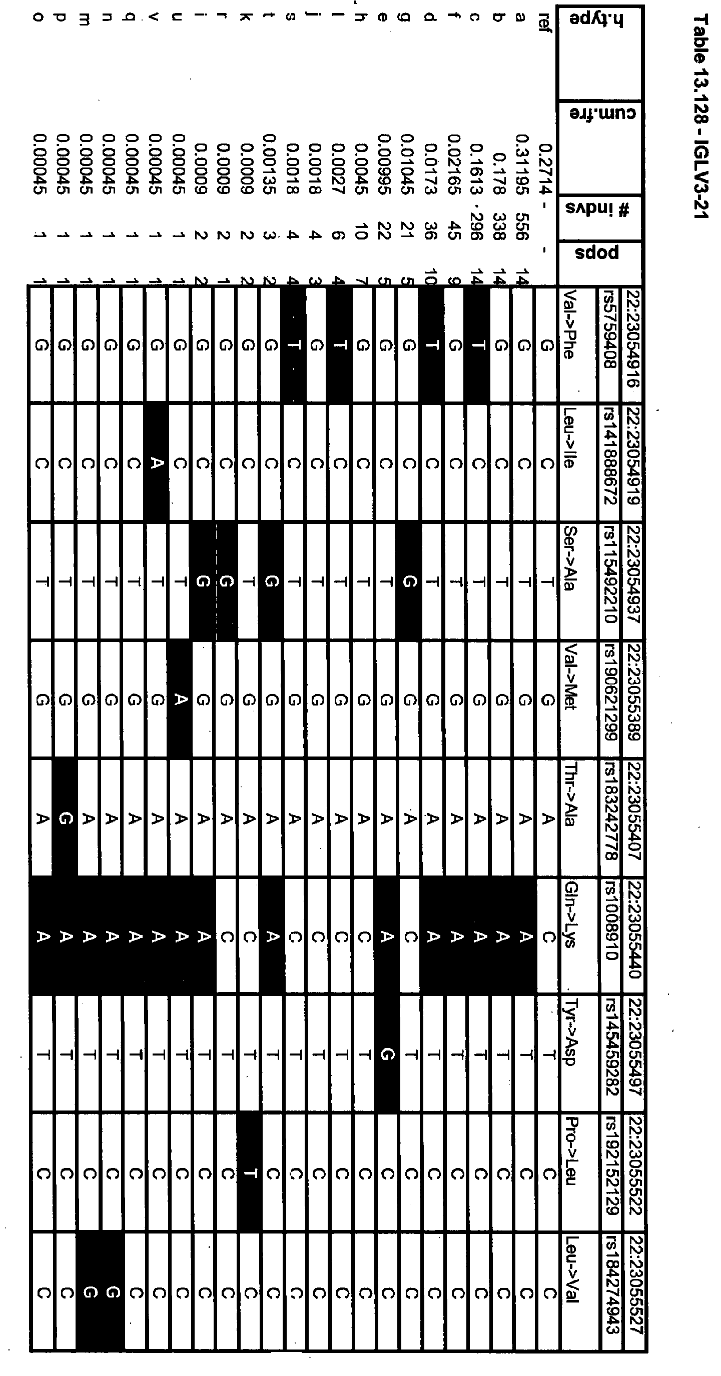

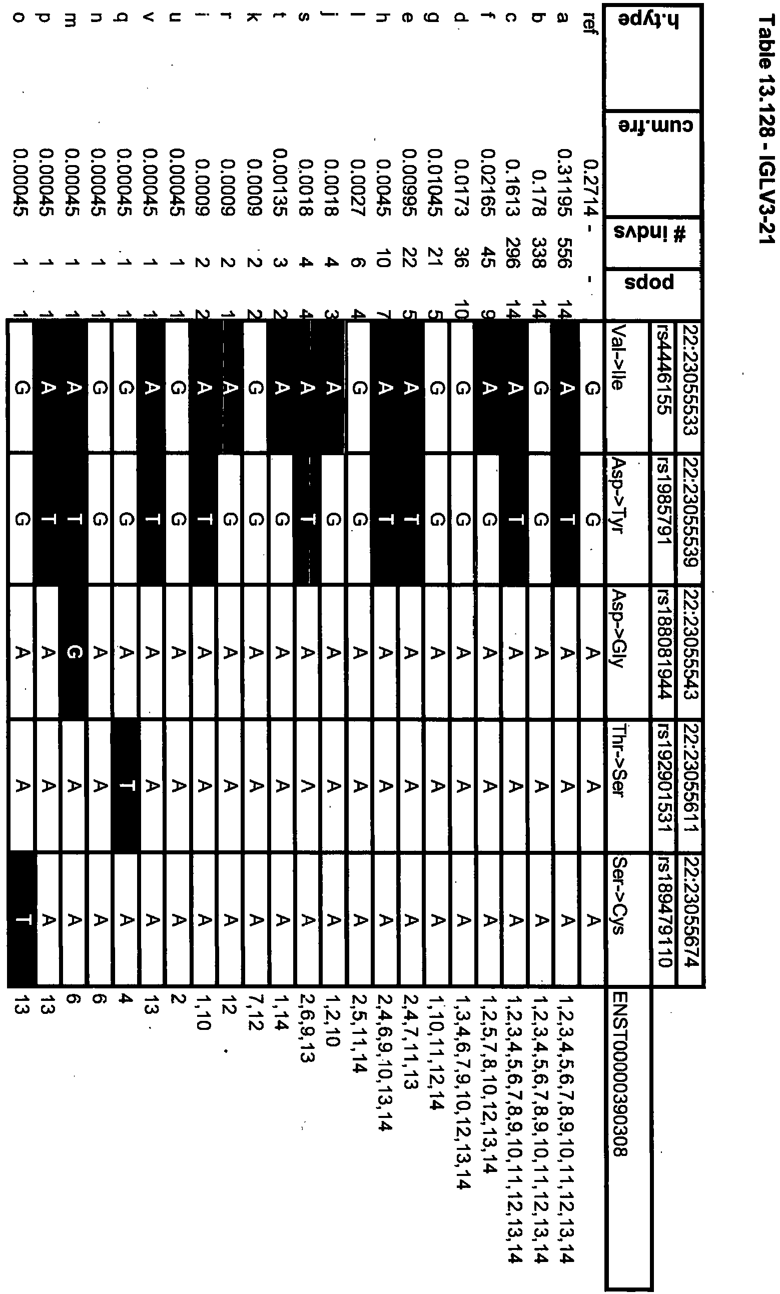

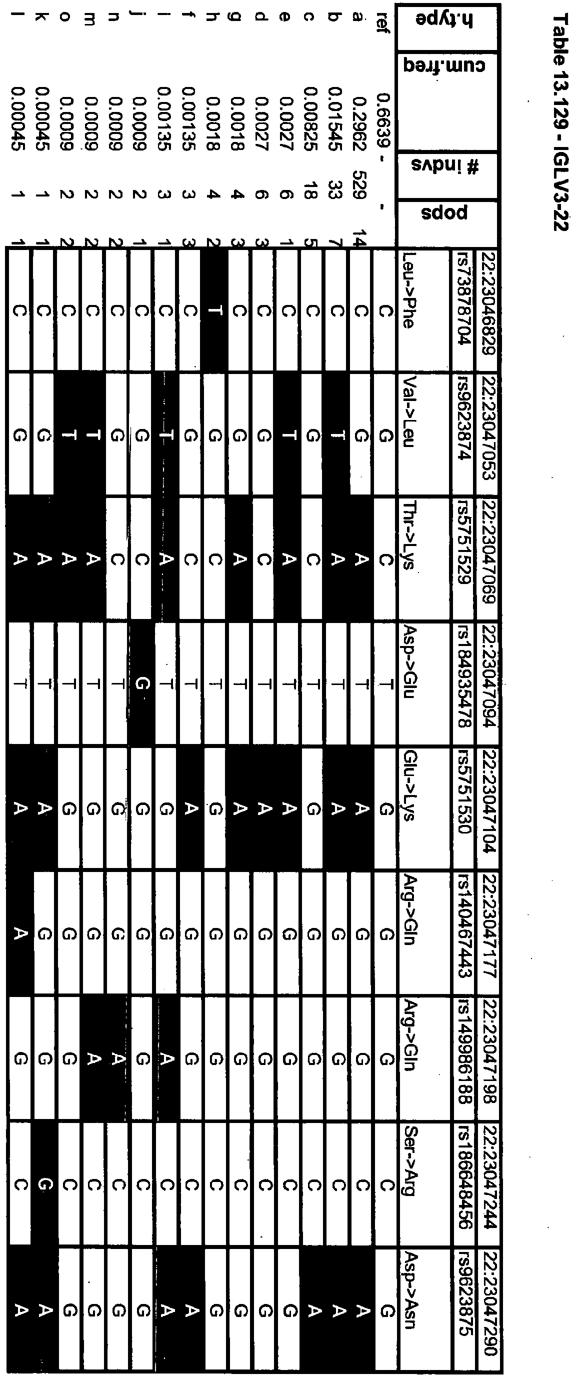

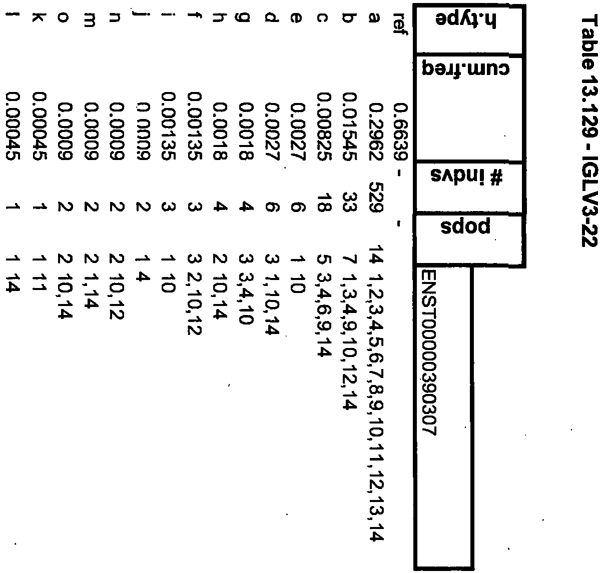

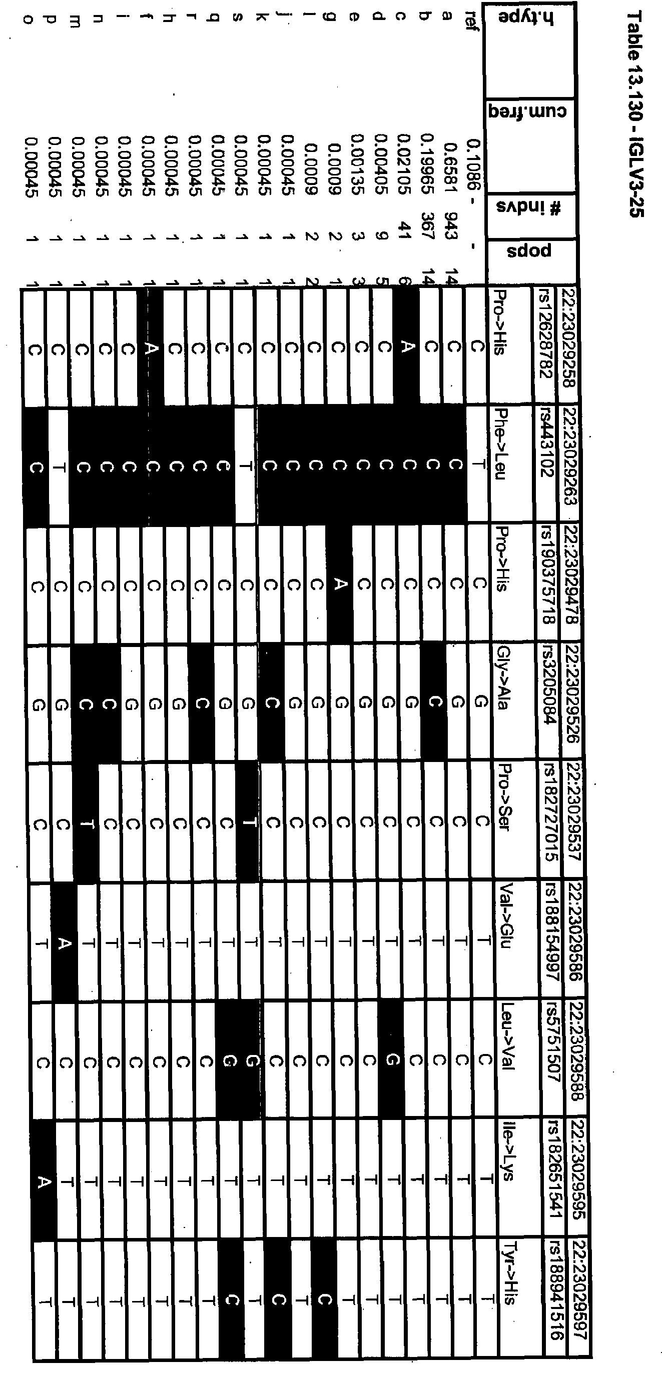

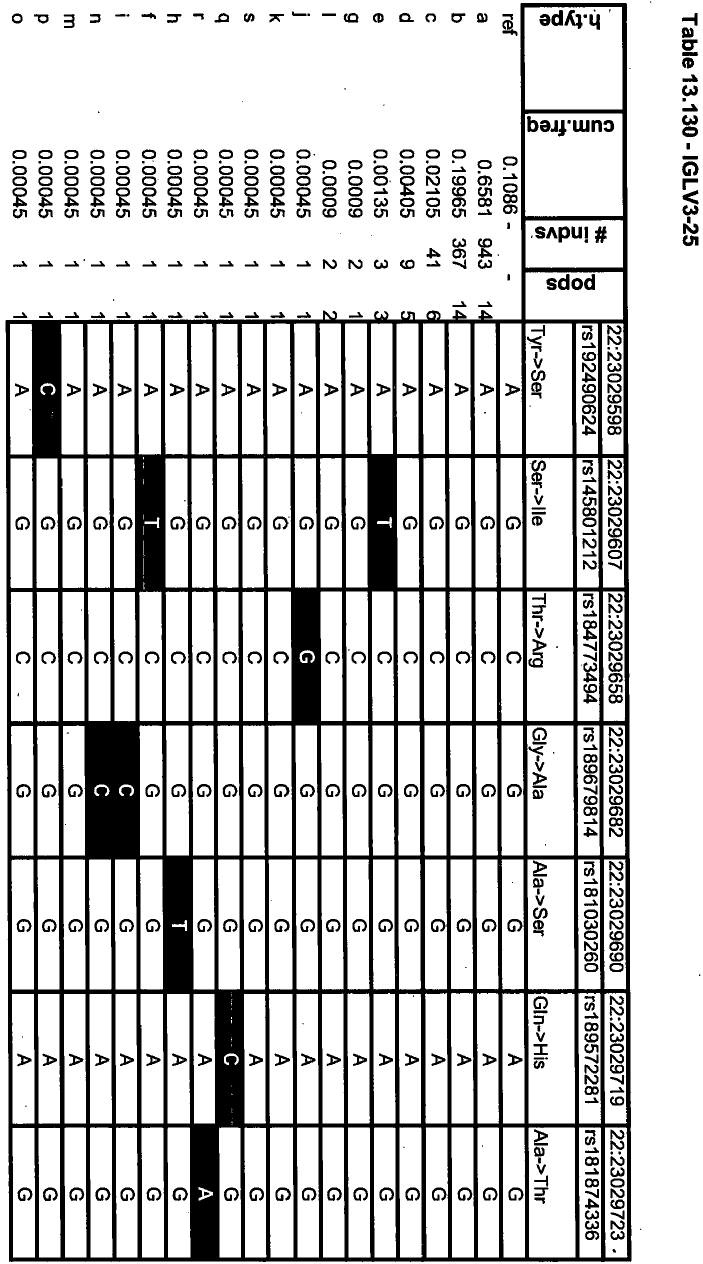

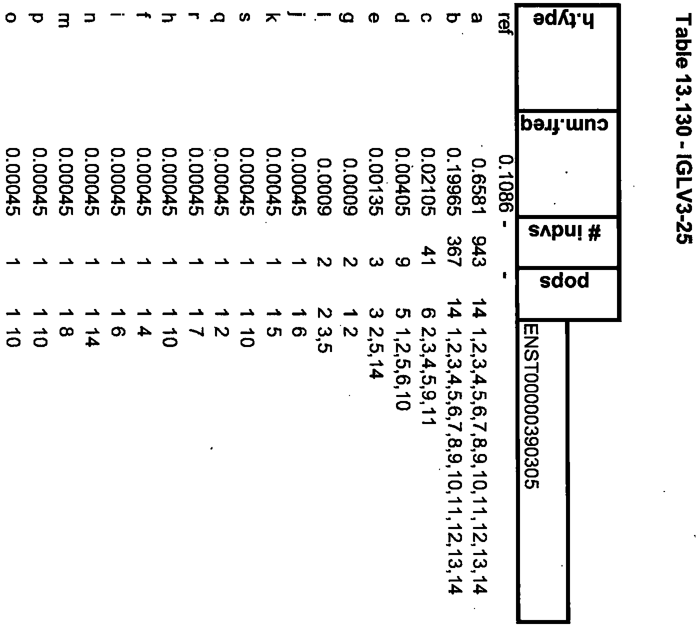

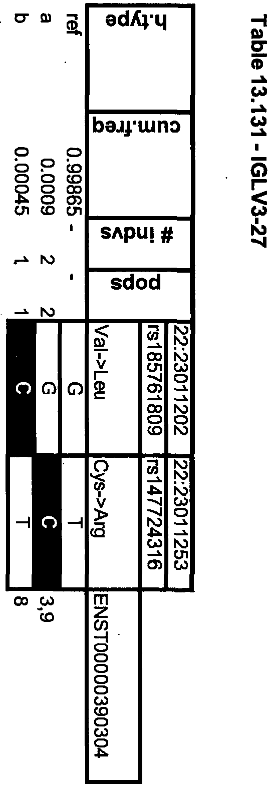

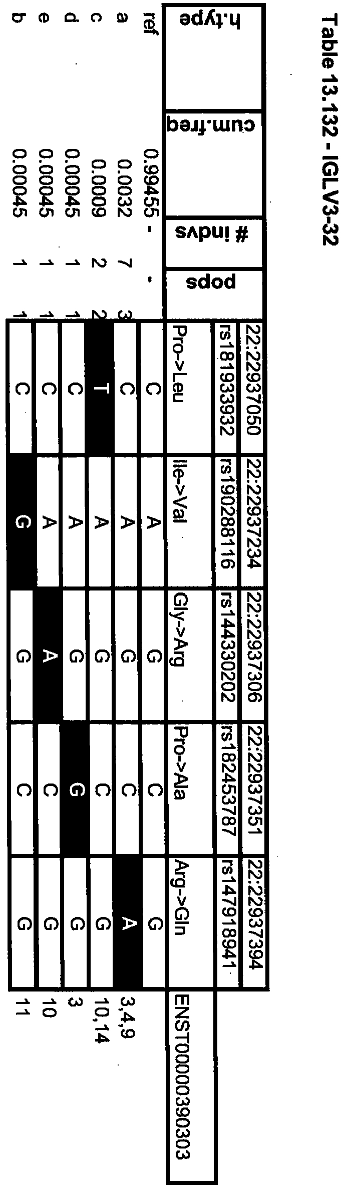

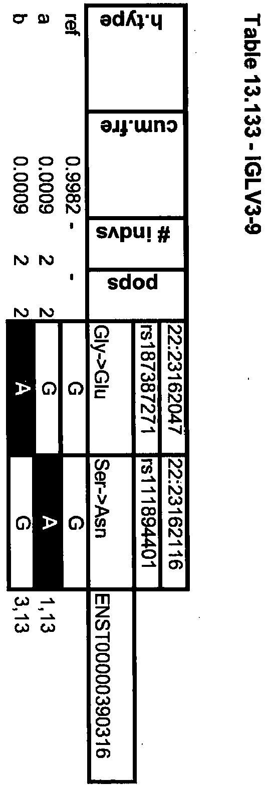

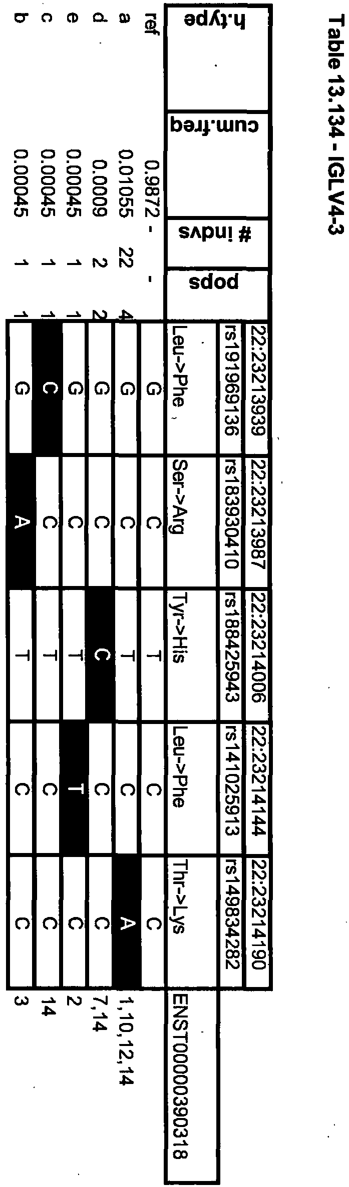

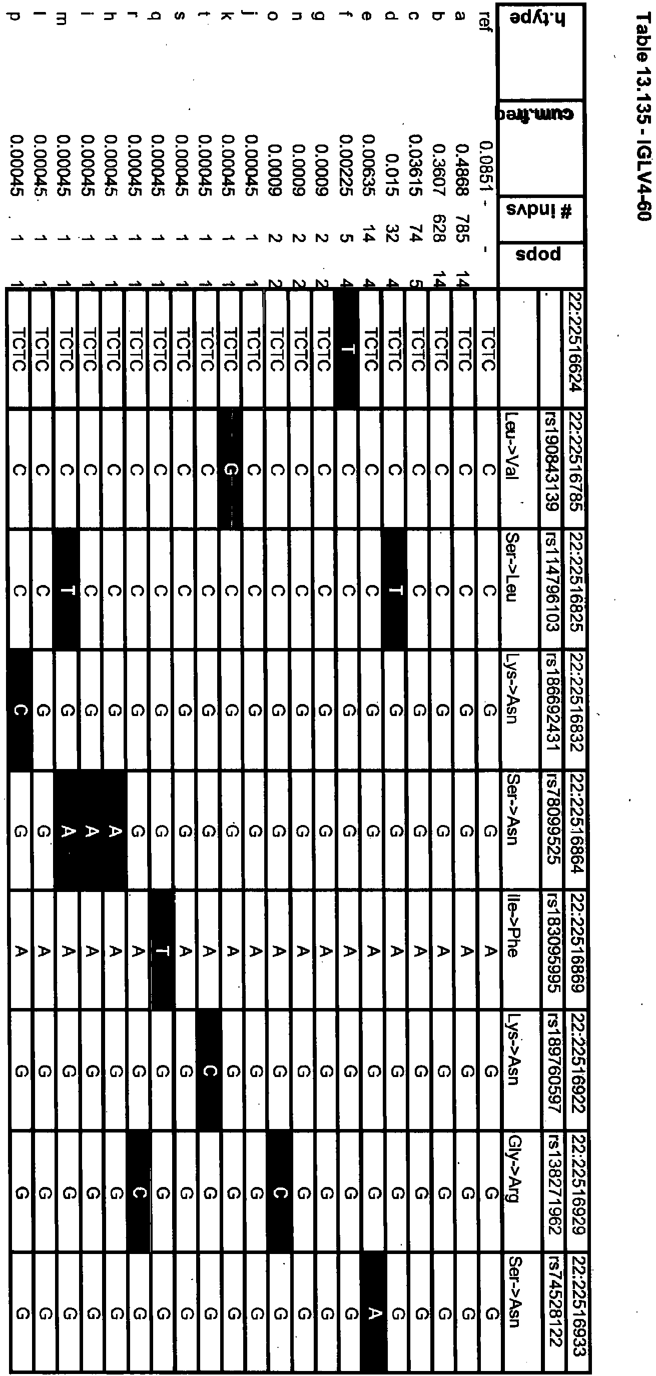

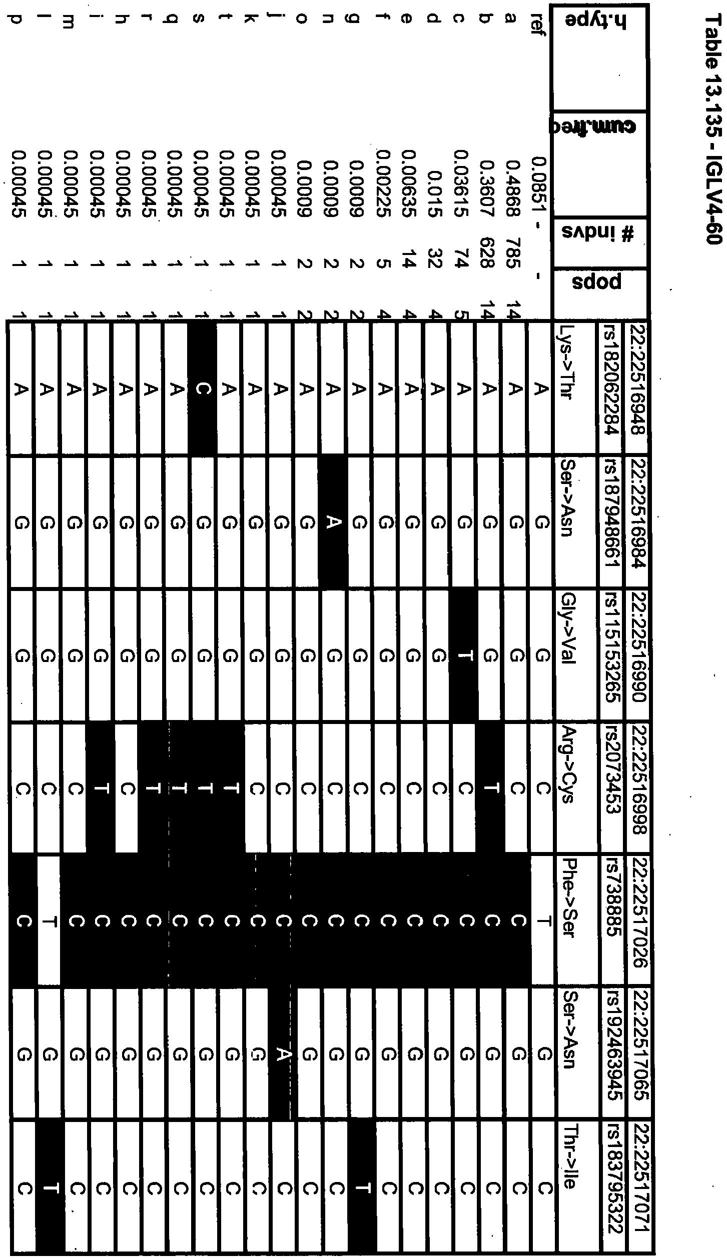

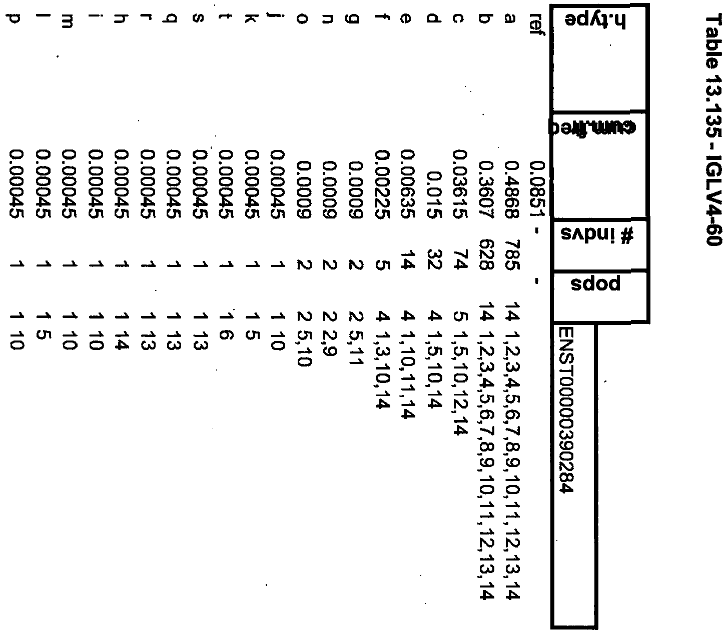

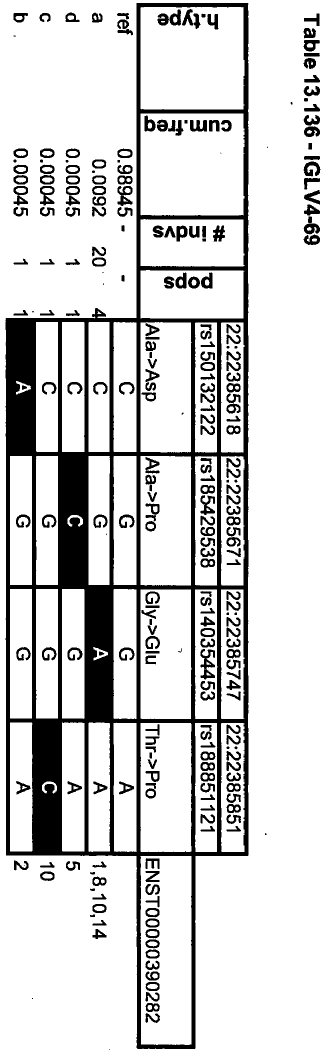

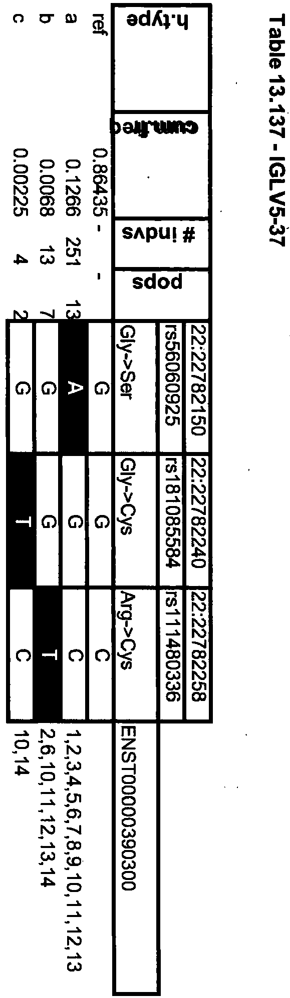

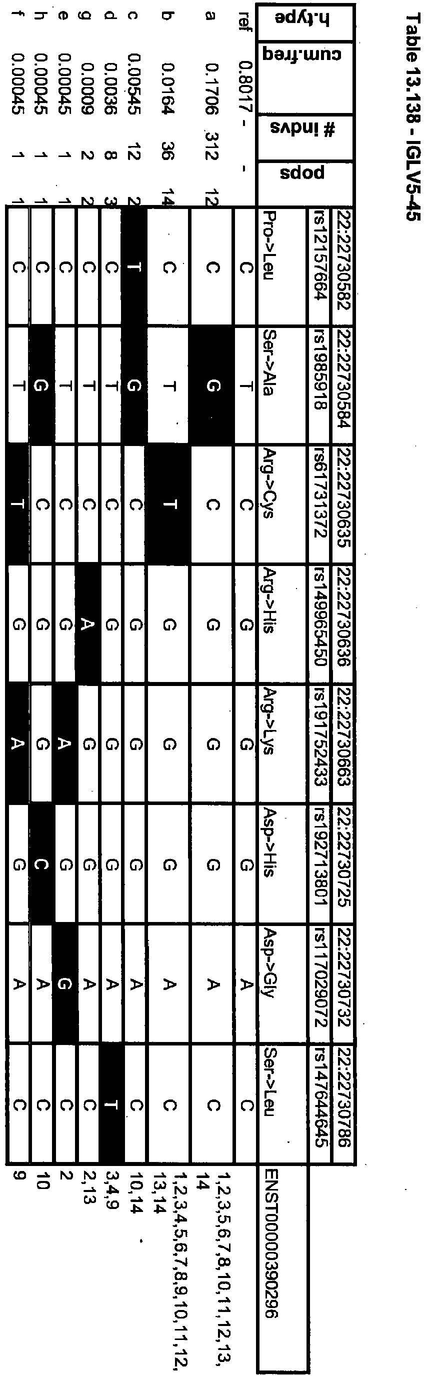

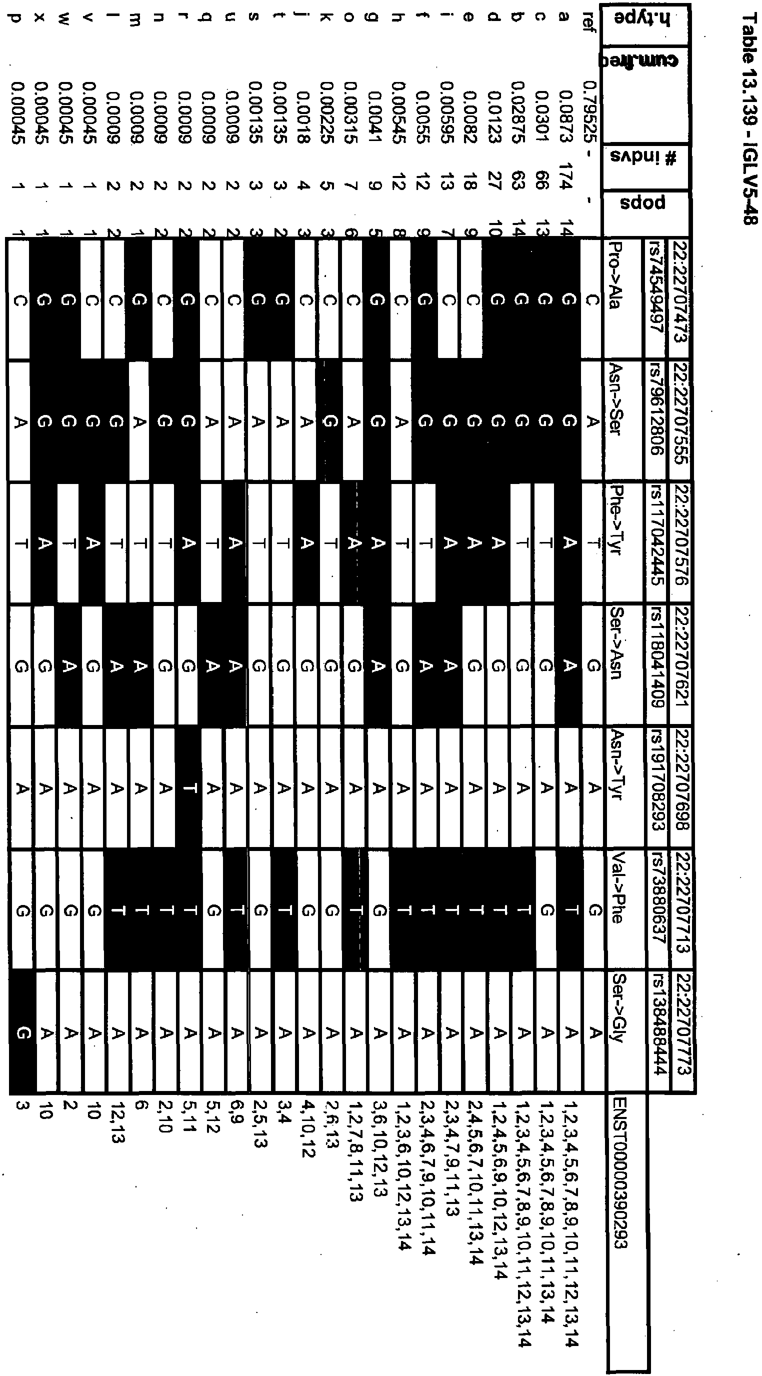

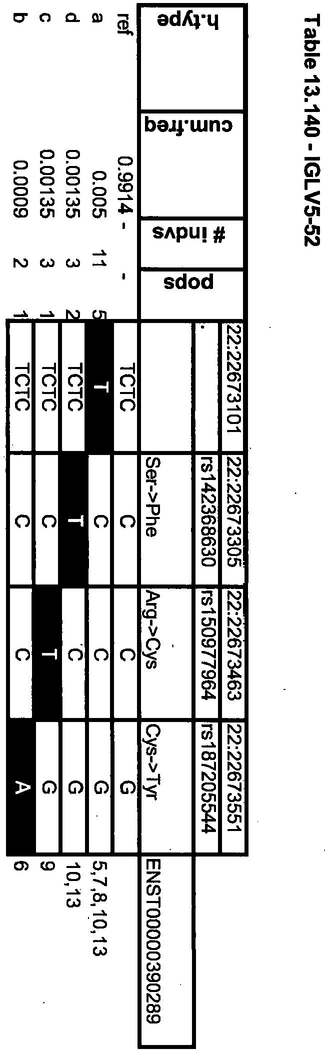

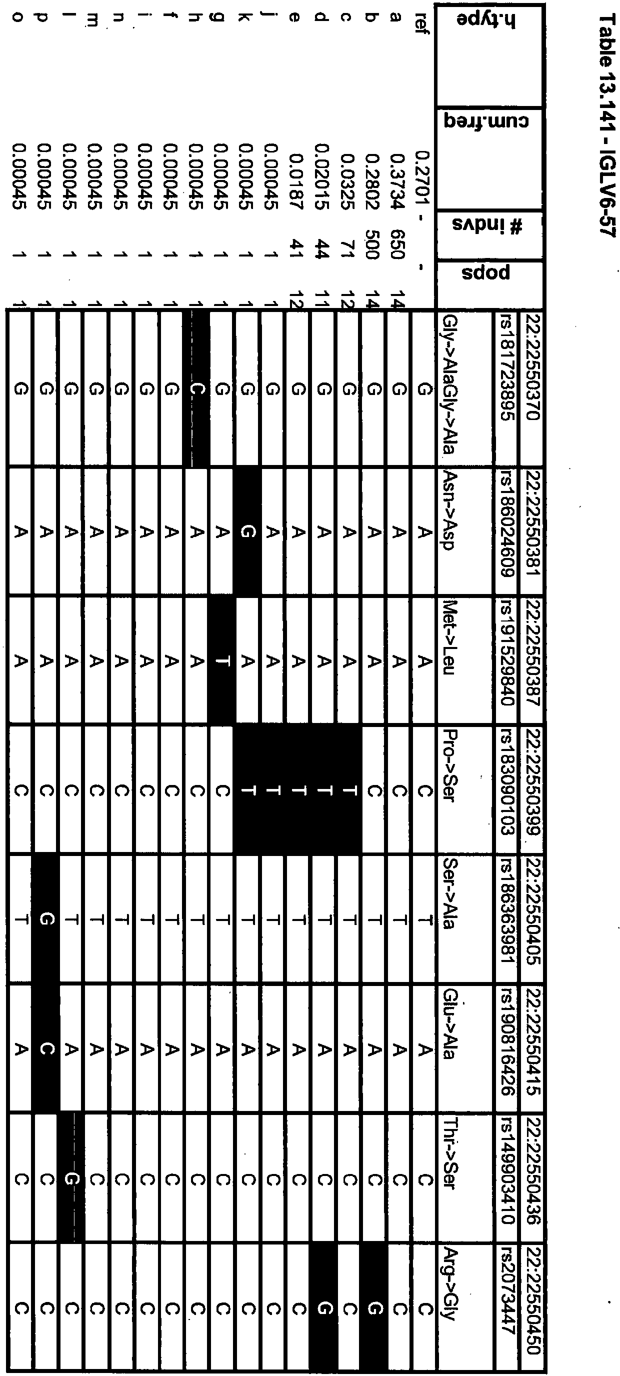

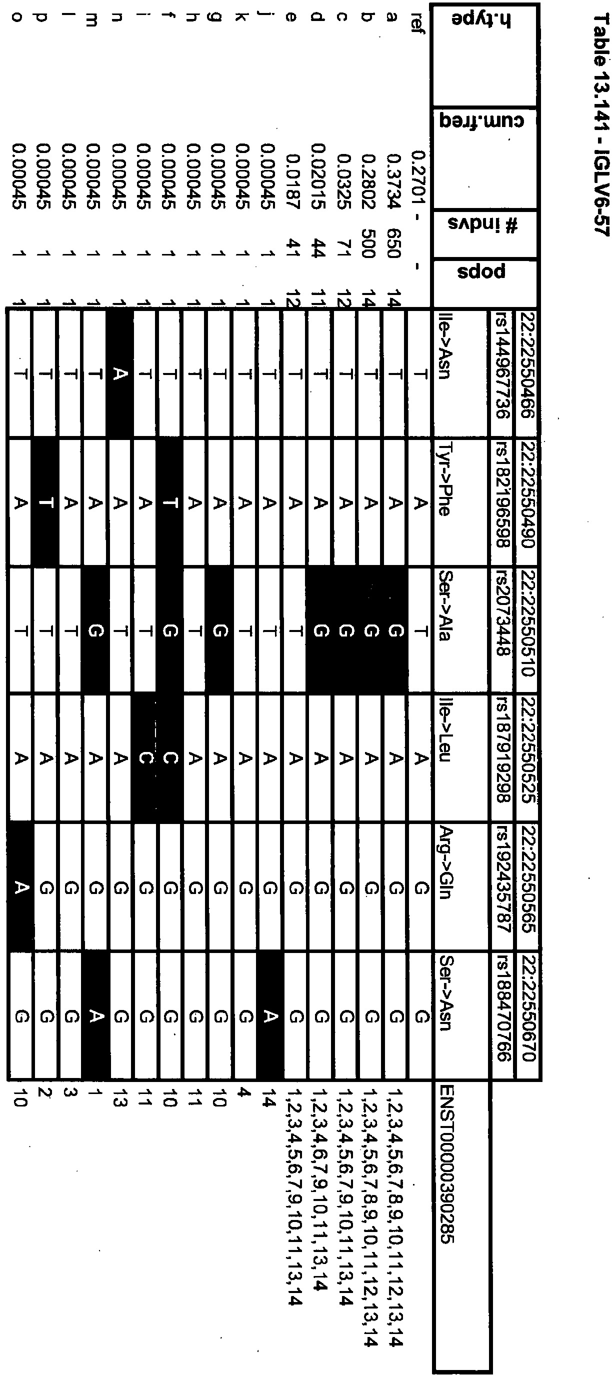

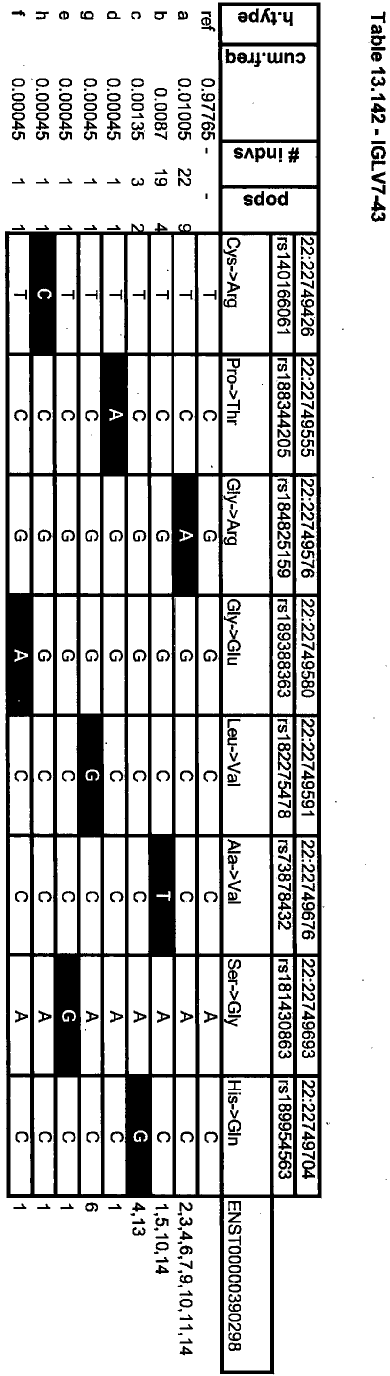

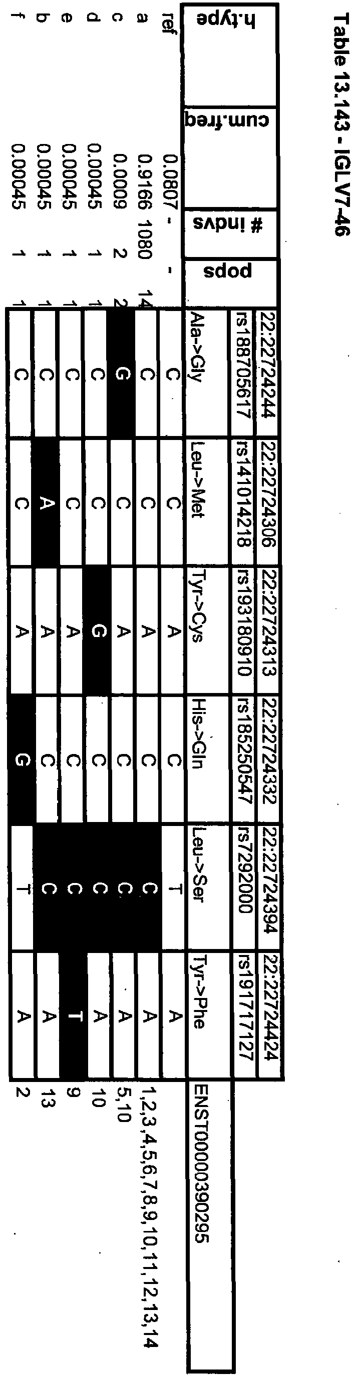

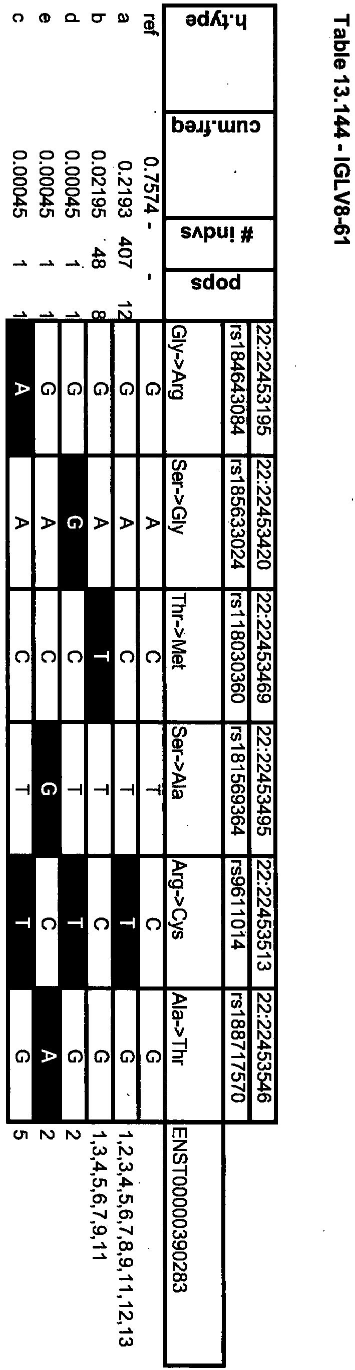

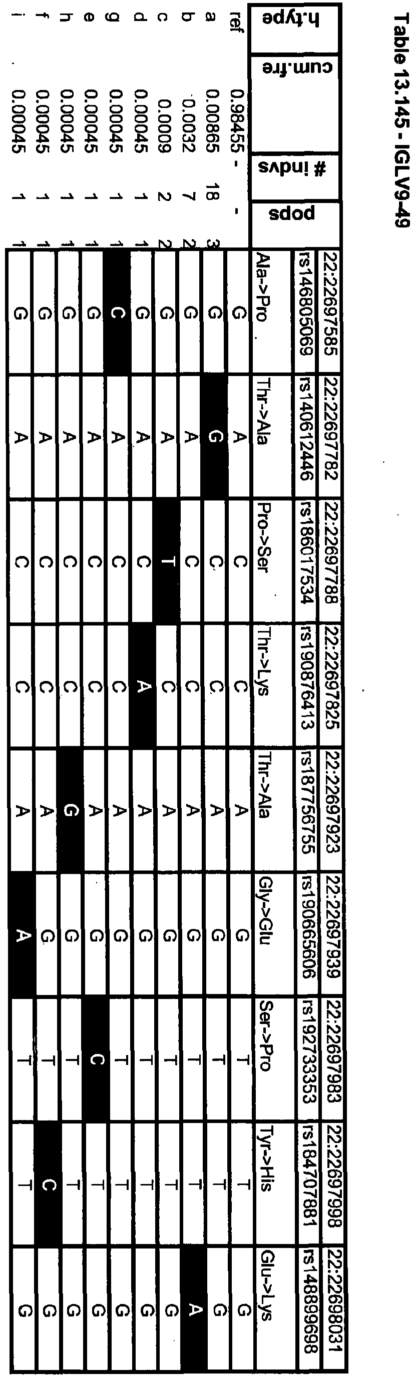

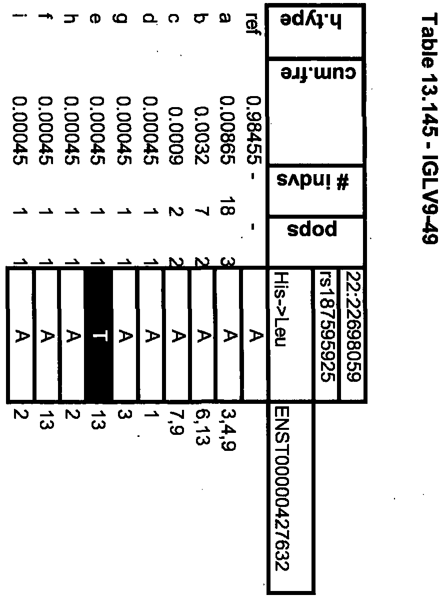

**Table 14A: Variants Having a Cumulative Frequency Greater than 5% or 10%**

| **GENE SEGMENT** | **VARIANT NAME** | **CUMULATIVE FREQUENCY** | **CUMULATIVE FREQUENCY >10%** | **NO UNIQUE POPULATIONS** |
|---|---|---|---|---|
| **HeavyC** | | | | |
| IGHA | IGHA1-a | 0.31385 | Yes | 14 |
| IGHA | IGHA2-a | 0.35655 | Yes | 14 |
| IGHA | IGHA2-b | 0.1647 | Yes | 14 |
| IGHG | IGHG2-a | 0.28675 | Yes | 12 |
| IGHG | IGHG3-a | 0.2813 | Yes | 14 |
| IGHG | IGHG3-b | 0.19955 | Yes | 11 |
| IGHG | IGHG4-a | 0.19825 | Yes | 12 |
| IGHM | IGHM-a | 0.7527 | Yes | 14 |
| IGHM | IGHM-b | 0.15245 | Yes | 14 |
| **Heavy J** | | | | |
| IGHJ | IGHJ6-a | 0.6845 | Yes | 14 |
| **Heavy V** | | | | |
| IGHV | IGHV1-2-a | 0.3268 | Yes | 14 |
| IGHV | IGHV1-2-b | 0.1304 | Yes | 14 |
| IGHV | IGHV1-2-c | 0.0797 | No | 14 |
| IGHV | IGHV1-3-a | 0.3564 | Yes | 14 |
| IGHV | IGHV1-3-b | 0.0724 | No | 13 |
| IGHV | IGHV1-45-a | 0.1286 | Yes | 12 |
| IGHV | IGHV1-45-b | 0.1126 | Yes | 12 |
| IGHV | IGHV1-58-a | 0.4503 | Yes | 14 |
| IGHV | IGHV1-58-b | 0.3144 | Yes | 14 |
| IGHV | IGHV1-69-a | 0.1689 | Yes | 14 |
| IGHV | IGHV1-69-b | 0.1543 | Yes | 14 |
| IGHV | IGHV1-69-c | 0.0561 | No | 13 |
| IGHV | IGHV2-26-a | 0.06135 | No | 13 |
| IGHV | IGHV2-5-a | 0.6657 | Yes | 14 |
| IGHV | IGHV2-70-a | 0.31 | Yes | 14 |
| IGHV | IGHV2-70-b | 0.16595 | Yes | 14 |
| IGHV | IGHV2-70-d | 0.06035 | No | 13 |
| IGHV | IGHV3-13-a | 0.15245 | Yes | 13 |
| IGHV | IGHV3-15-a | 0.0572 | No | 11 |
| IGHV | IGHV3-16-a | 0.11075 | Yes | 13 |
| IGHV | IGHV3-20-a | 0.2829 | Yes | 14 |
| IGHV | IGHV3-20-b | 0.1209 | Yes | 13 |
| IGHV | IGHV3-23-a | 0.09825 | No | 12 |
| IGHV | IGHV3-33-a | 0.145 | Yes | 12 |
| IGHV | IGHV3-35-a | 0.11765 | Yes | 14 |
| IGHV | IGHV3-35-b | 0.09695 | No | 10 |
| IGHV | IGHV3-38-a | 0.0723 | No | 14 |
| IGHV | IGHV3-43-a | 0.2428 | Yes | 14 |
| IGHV | IGHV3-43-b | 0.08365 | No | 13 |
| IGHV | IGHV3-48-a | 0.3355 | Yes | 14 |
| IGHV | IGHV3-48-b | 0.20455 | Yes | 14 |
| IGHV | IGHV3-49-a | 0.38705 | Yes | 14 |
| IGHV | IGHV3-49-b | 0.28555 | Yes | 13 |
| IGHV | IGHV3-53-a | 0.2535 | Yes | 14 |
| IGHV | IGHV3-53-b | 0.2264 | Yes | 13 |
| IGHV | IGHV3-64-a | 0.47965 | Yes | 14 |
| IGHV | IGHV3-64-b | 0.06225 | No | 13 |
| IGHV | IGHV3-66-a | 0.27865 | Yes | 14 |
| IGHV | IGHV3-66-b | 0.12575 | Yes | 14 |
| IGHV | IGHV3-66-c | 0.0649 | No | 13 |
| IGHV | IGHV4-28-a | 0.41435 | Yes | 14 |
| IGHV | IGHV4-28-b | 0.13715 | Yes | 14 |
| IGHV | IGHV4-28-c | 0.07385 | No | 14 |
| IGHV | IGHV4-28-d | 0.06485 | No | 13 |
| IGHV | IGHV4-31-a | 0.5475 | Yes | 14 |
| IGHV | IGHV4-31-b | 0.2035 | Yes | 14 |
| IGHV | IGHV4-31-c | 0.1554 | Yes | 14 |
| IGHV | IGHV4-39-a | 0.10295 | Yes | 14 |
| IGHV | IGHV4-39-b | 0.0918 | No | 14 |
| IGHV | IGHV4-4-a | 0.1136 | Yes | 14 |
| IGHV | IGHV4-4-d | 0.0817 | No | 14 |
| IGHV | IGHV4-4-f | 0.06675 | No | 14 |
| IGHV | IGHV4-4-b | 0.06385 | No | 13 |
| IGHV | IGHV4-4-c | 0.0599 | No | 14 |
| IGHV | IGHV4-4-e | 0.05215 | No | 12 |
| IGHV | IGHV4-61-a | 0.5701 | Yes | 14 |
| IGHV | IGHV4-61-b | 0.18115 | Yes | 14 |
| IGHV | IGHV4-61-c | 0.13535 | Yes | 13 |
| IGHV | IGHV4-61-f | 0.05855 | No | 14 |
| **Kappa C** | | | | |
| IGKC | IGKC-a | 0.2427 | Yes | 14 |
| **Kappa V** | | | | |
| IGKV | IGKV1-17-a | 0.08655 | No | 13 |
| IGKV | IGKV1-17-b | 0.0545 | No | 12 |
| IGKV | IGKV1-8-a | 0.20315 | Yes | 14 |
| IGKV | IGKV1-8-b | 0.1334 | Yes | 14 |
| IGKV | IGKV1-9-a | 0.1854 | Yes | 14 |
| IGKV | IGKV1D-42-a | 0.22345 | Yes | 14 |
| IGKV | IGKV1D-8-a | 0.1872 | Yes | 14 |
| IGKV | IGKV2-24-a | 0.1708 | Yes | 13 |
| IGKV | IGKV2-30-a | 0.24085 | Yes | 13 |
| IGKV | IGKV2D-26-a | 0.325 | Yes | 14 |
| IGKV | IGKV2D-29-a | 0.30175 | Yes | 14 |
| IGKV | IGKV3-7-a | 0.2018 | Yes | 14 |
| IGKV | IGKV3D-11-a | 0.35105 | Yes | 14 |
| IGKV | IGKV3D-15-a | 0.2061 | Yes | 14 |
| IGKV | IGKV5-2-a | 0.32315 | Yes | 14 |
| **Lambda C** | | | | |
| IGLC | IGLC7-a | 0.0751 | No | 14 |
| **Lambda J** | | | | |
| IGU | IGU2-a | 0.0741 | No | 13 |
| IGU | IGU7-a | 0.93225 | Yes | 14 |
| **Lambda V** | | | | |
| IGLV | IGLV10-54-a | 0.50725 | Yes | 14 |
| IGLV | IGLV10-54-b | 0.09425 | No | 14 |
| IGLV | IGLV11-55-a | 0.4463 | Yes | 14 |
| IGLV | IGLV1-36-a | 0.0942 | No | 14 |
| IGLV | IGLV1-47-a | 0.8413 | Yes | 14 |
| IGLV | IGLV1-50-a | 0.19325 | Yes | 14 |
| IGLV | IGLV1-51-a | 0.1177 | Yes | 11 |
| IGLV | IGLV2-14-a | 0.48365 | Yes | 14 |
| IGLV | IGLV2-14-c | 0.08855 | No | 14 |
| IGLV | IGLV2-14-b | 0.0804 | No | 10 |
| IGLV | IGLV2-18-a | 0.763 | Yes | 14 |
| IGLV | IGLV2-23-a | 0.34655 | Yes | 14 |
| IGLV | IGLV2-8-a | 0.1451 | Yes | 11 |
| IGLV | IGLV3-12-a | 0.38715 | Yes | 14 |
| IGLV | IGLV3-12-b | 0.1116 | Yes | 14 |
| IGLV | IGLV3-12-c | 0.0774 | No | 14 |
| IGLV | IGLV3-19-a | 0.10015 | Yes | 12 |
| IGLV | IGLV3-21-a | 0.31195 | Yes | 14 |
| IGLV | IGLV3-21-b | 0.178 | Yes | 14 |
| IGLV | IGLV3-21-c | 0.1613 | Yes | 14 |
| IGLV | IGLV3-22-a | 0.2962 | Yes | 14 |
| IGLV | IGLV3-25-a | 0.6581 | Yes | 14 |
| IGLV | IGLV3-25-b | 0.19965 | Yes | 14 |
| IGLV | IGLV4-60-a | 0.4868 | Yes | 14 |
| IGLV | IGLV4-60-b | 0.3607 | Yes | 14 |
| IGLV | IGLV5-37-a | 0.1266 | Yes | 13 |
| IGLV | IGLV5-45-a | 0.1706 | Yes | 12 |
| IGLV | IGLV5-48-a | 0.0873 | No | 14 |
| IGLV | IGLV6-57-a | 0.3734 | Yes | 14 |
| IGLV | IGLV6-57-b | 0.2802 | Yes | 14 |
| IGLV | IGLV7-46-a | 0.9166 | Yes | 14 |
| IGLV | IGLV8-61-a | 0.2193 | Yes | 12 |

**Table 14B: Variants Having a Cumulative Frequency Greater than 20%**

| **GENE SEGMENT** | **VARIANT NAME** | **CUMULATIAVE FREQUENCY** | **NO UNIQUE POPULATIONS** |
|---|---|---|---|
| **Heavy C** | | | |
| IGHA | IGHA1-a | 0.31385 | 14 |
| IGHA | IGHA2-a | 0.35655 | 14 |
| IGHG | IGHG2-a | 0.28675 | 12 |
| IGHG | IGHG3-a | 0.2813 | 14 |
| IGHM | IGHM-a | 0.7527 | 14 |
| **Heavy J** | | | |
| IGHJ | IGHJ6-a | 0.6845 | 14 |
| **Heavy V** | | | |
| IGHV | IGHV1-2-a | 0.3268 | 14 |
| IGHV | IGHV1-3-a | 0.3564 | 14 |
| IGHV | IGHV1-58-a | 0.4503 | 14 |
| IGHV | IGHV1-58-b | 0.3144 | 14 |
| IGHV | IGHV2-5-a | 0.6657 | 14 |
| IGHV | IGHV2-70-a | 0.31 | 14 |
| IGHV | IGHV3-20-a | 0.2829 | 14 |
| IGHV | IGHV3-43-a | 0.2428 | 14 |
| IGHV | IGHV3-48-a | 0.3355 | 14 |
| IGHV | IGHV3-48-b | 0.20455 | 14 |
| IGHV | IGHV3-49-a | 0.38705 | 14 |
| IGHV | IGHV3-49-b | 0.28555 | 13 |
| IGHV | IGHV3-53-a | 0.2535 | 14 |
| IGHV | IGHV3-53-b | 0.2264 | 13 |
| IGHV | IGHV3-64-a | 0.47965 | 14 |
| IGHV | IGHV3-66-a | 0.27865 | 14 |
| IGHV | IGHV4-28-a | 0.41435 | 14 |
| IGHV | IGHV4-31-a | 0.5475 | 14 |
| IGHV | IGHV4-31-b | 0.2035 | 14 |
| IGHV | IGHV4-61-a | 0.5701 | 14 |
| **Kappa C** | | | |
| IGKC | IGKC-a | 0.2427 | 14 |
| | IGKV1-8-a | 0.20315 | 14 |
| **Kappa V** | | | |
| IGKV | | | |
| IGKV | IGKV1D-42-a | 0.22345 | 14 |
| IGKV | IGKV2-30-a | 0.24085 | 13 |
| IGKV | IGKV2D-26-a | 0.325 | 14 |
| IGKV | IGKV2D-29-a | 0.30175 | 14 |
| IGKV | IGKV3-7-a | 0.2018 | 14 |
| IGKV | IGKV3D-11-a | 0.35105 | 14 |
| IGKV | IGKV3D-15-a | 0.2061 | 14 |
| IGKV | IGKV5-2-a | 0.32315 | 14 |
| **Lambda J** | | | |
| IGU | IGU7-a | 0.93225 | 14 |
| **Lambda V** | | | |
| IGLV | IGLV10-54-a | 0.50725 | 14 |
| IGLV | IGLV11-55-a | 0.4463 | 14 |
| IGLV | IGLV1-47-a | 0.8413 | 14 |
| IGLV | IGLV2-14-a | 0.48365 | 14 |
| IGLV | IGLV2-18-a | 0.763 | 14 |
| IGLV | IGLV2-23-a | 0.34655 | 14 |
| IGLV | IGLV3-12-a | 0.38715 | 14 |
| IGLV | IGLV3-21-a | 0.31195 | 14 |
| IGLV | IGLV3-22-a | 0.2962 | 14 |
| IGLV | IGLV3-25-a | 0.6581 | 14 |
| IGLV | IGLV4-60-a | 0.4868 | 14 |
| IGLV | IGLV4-60-b | 0.3607 | 14 |
| IGLV | IGLV6-57-a | 0.3734 | 14 |
| IGLV | IGLV6-57-b | 0.2802 | 14 |
| IGLV | IGLV7-46-a | 0.9166 | 14 |
| IGLV | IGLV8-61-a | 0.2193 | 12 |

**Table 14C: Variants Having a Cumulative Frequency Greater than 30%**

| **GENE SEGMENT** | **VARIANT NAME** | **CUMULATIVE FREQUENCY** | **NO UNIQUE POPULATIONS** |
|---|---|---|---|
| **Heavy C** | | | |
| IGHA | IGHA1-a | 0.31385 | 14 |
| IGHA | IGHA2-a | 0.35655 | 14 |
| IGHM | IGHM-a | 0.7527 | 14 |
| **Heavy J** | | | |
| IGHJ | IGHJ6-a | 0.6845 | 14 |
| | | | |
| **Heavy V** | | | |
| IGHV | IGHV1-2-a | 0.3268 | 14 |
| IGHV | IGHV1-3-a | 0.3564 | 14 |
| IGHV | IGHV1-58-a | 0.4503 | 14 |
| IGHV | IGHV1-58-b | 0.3144 | 14 |
| IGHV | IGHV2-5-a | 0.6657 | 14 |
| IGHV | IGHV2-70-a | 0.31 | 14 |
| IGHV | IGHV3-48-a | 0.3355 | 14 |
| IGHV | IGHV3-49-a | 0.38705 | 14 |
| IGHV | IGHV3-64-a | 0.47965 | 14 |
| IGHV | IGHV4-28-a | 0.41435 | 14 |
| IGHV | IGHV4-31-a | 0.5475 | 14 |
| IGHV | IGHV4-61-a | 0.5701 | 14 |
| IGKV | IGKV2D-26-a | 0.325 | 14 |
| IGKV | IGKV2D-29-a | 0.30175 | 14 |
| IGKV | IGKV3D-11-a | 0.35105 | 14 |
| IGKV | IGKV5-2-a | 0.32315 | 14 |
| **Lambda J** | | | |
| IGLJ | IGU7-a | 0.93225 | 14 |
| **Lambda V** | | | |
| IGLV | IGLV10-54-a | 0.50725 | 14 |
| IGLV | IGLV11-55-a | 0.4463 | 14 |
| IGLV | IGLV1-47-a | 0.8413 | 14 |
| IGLV | IGLV2-14-a | 0.48365 | 14 |
| IGLV | IGLV2-18-a | 0.763 | 14 |
| IGLV | IGLV2-23-a | 0.34655 | 14 |
| IGLV | IGLV3-12-a | 0.38715 | 14 |
| IGLV | IGLV3-21-a | 0.31195 | 14 |
| IGLV | IGLV3-25-a | 0.6581 | 14 |
| IGLV | IGLV4-60-a | 0.4868 | 14 |
| IGLV | IGLV4-60-b | 0.3607 | 14 |
| IGLV | IGLV6-57-a | 0.3734 | 14 |
| IGLV | IGLV7-46-a | 0.9166 | 14 |

**Table 14D: Variants Having a Cumulative Frequency Greater than 40%**

| **GENE SEGMENT** | **VARIANT NAME** | **CUMULATIAVE FREQUENCY** | **NO UNIQUE POPULATIONS** |
|---|---|---|---|
| **Heavy C** | | | |
| IGHM | IGHM-a | 0.7527 | 14 |
| **Heavy J** | | | |
| IGHJ | IGHJ6-a | 0.6845 | 14 |
| | | | |
| **Heavy** V IGHV | IGHV1-58-a | 0.4503 | 14 |
| IGHV | IGHV2-5-a | 0.6657 | 14 |
| IGHV | IGHV3-64-a | 0.47965 | 14 |
| IGHV | IGHV4-28-a | 0.41435 | 14 |
| IGHV | IGHV4-31-a | 0.5475 | 14 |
| IGHV | IGHV4-61-a | 0.5701 | 14 |
| **Lambda J** | | | |
| IGU | IGU7-a | 0.93225 | 14 |
| **Lambda V** | | | |
| IGLV | IGLV10-54-a | 0.50725 | 14 |
| IGLV | IGLV11-55-a | 0.4463 | 14 |
| IGLV | IGLV1-47-a | 0.8413 | 14 |
| IGLV | IGLV2-14-a | 0.48365 | 14 |
| IGLV | IGLV2-18-a | 0.763 | 14 |
| IGLV | IGLV3-25-a | 0.6581 | 14 |
| IGLV | IGLV4-60-a | 0.4868 | 14 |
| IGLV | IGLV7-46-a | 0.9166 | 14 |

**Table 14E: Variants Having a Cumulative Frequency Greater than 50%**

| **GENE SEGMENT** | **VARIANT NAME** | **CUMULATIVE FREQUENCY** | **NO UNIQUE POPULATIONS** |
|---|---|---|---|
| **Heavy C** | | | |
| IGHM | IGHM-a | 0.7527 | 14 |
| **Heavy J** | | | |
| IGHJ | IGHJ6-a | 0.6845 | 14 |
| | | | |
| **Heavy V** | | | |
| IGHV | IGHV2-5-a | 0.6657 | 14 |
| IGHV | IGHV4-31-a | 0.5475 | 14 |
| IGHV | IGHV4-61-a | 0.5701 | 14 |
| **Lambda J** | | | |
| IGU | IGU7-a | 0.93225 | 14 |
| **Lambda V** | IGLV10-54-a | 0.50725 | 14 |
| IGLV | | | |
| IGLV | IGLV1-47-a | 0.8413 | 14 |
| IGLV | IGLV2-18-a | 0.763 | 14 |
| IGLV | IGLV3-25-a | 0.6581 | 14 |
| IGLV | IGLV7-46-a | 0.9166 | 14 |

**Table 14F: Variants Having a Cumulative Frequency Greater than 60%**

| **GENE SEGMENT** | **VARIANT NAME** | **CUMULATIVE FREQUENCY** | **NO UNIQUE POPULATIONS** |
|---|---|---|---|
| **Heavy C** | | | |
| IGHM | IGHM-a | 0.7527 | 14 |
| **Heavy J** | | | |
| IGHJ | IGHJ6-a | 0.6845 | 14 |
| | | | |
| **Heavy V** | | | |
| IGHV | IGHV2-5-a | 0.6657 | 14 |
| **Lambda J** | | | |
| IGLJ | IGU7-a | 0.93225 | 14 |
| **Lambda V** | | | |
| IGLV | IGLV1-47-a | 0.8413 | 14 |
| IGLV | IGLV2-18-a | 0.763 | 14 |
| IGLV | IGLV3-25-a | 0.6581 | 14 |
| IGLV | IGLV7-46-a | 0.9166 | 14 |

**Table 14G: Variants Having a Cumulative Frequency Greater than 70%**

| **GENE SEGMENT** | **VARIANT NAME** | **CUMULATIVE FREQUENCY** | **NO UNIQUE POPULATIONS** |
|---|---|---|---|
| **Heavy C** | | | |
| IGHM | IGHM-a | 0.7527 | 14 |
| **Lambda J** | | | |
| IGU | IGU7-a | 0.93225 | 14 |
| **Lambda** V | | | |
| IGLV | IGLV1-47-a | 0.8413 | 14 |
| IGLV | IGLV2-18-a | 0.763 | 14 |
| IGLV | IGLV7-46-a | 0.9166 | 14 |

**Table 14H: Variants Having a Cumulative Frequency Greater than 80%**

| **GENE SEGMENT** | **VARIANT NAME** | **CUMULATIVE FREQUENCY** | **NO UNIQUE POPULATIONS** |
|---|---|---|---|
| **Lambda J** | | | |
| IGU | IGU7-a | 0.93225 | 14 |
| **Lambda V** | IGLV1-47-a | 0.8413 | 14 |
| IGLV | | | |
| IGLV | IGLV7-46-a | 0.9166 | 14 |

**Table 14I: Variants Having a Cumulative Frequency Greater than 90%**

| **GENE SEGMENT** | **VARIANT NAME** | **CUMULATIVE FREQUENCY** | **NO UNIQUE POPULATIONS** |
|---|---|---|---|
| **Lambda J** | | | |
| IGLJ | IGU7-a | 0.93225 | 14 |
| **Lambda** V | | | |
| IGLV | IGLV7-46-a | 0.9166 | 14 |

**Table 15: NAIVE HEAVY CHAIN REPERTOIRES**

| **TABLE 15A:** **All Naïve** - **J** **Usage** | | | |
|---|---|---|---|
| J | Average HCDR3 Length | Count | % |
| IGHJ1*01 | 11 | 12 | 0.90% |
| IGH2*01 | 12 | 47 | 3.51% |
| IGHJ3*02 | 12 | 205 | 15.30% |
| IGHJ4*02 | 11 | 689 | 51.42% |
| IGHJ5*02 | 13 | 88 | 6.57% |
| **IGHJ6*02** | **16** | **299** | **22.31%** |
| | | | |

| **Naive HCDR3>=20** | | | |
|---|---|---|---|
| J | Average HCDR3 Length | Count | % |
| IGHJ3 *02 | 20 | 1 | 2.22% |
| IGHJ4*02 | 20 | 1 | 2.22% |
| IGHJ5*02 | 20 | 2 | 4.44% |
| **IGHJ6*02** | **21** | **41** | **91.11%** |
| | | | |

| **Naïve** - **Long HCDR3 using IGHJ6*02** | | | |
|---|---|---|---|
| HCDR3Length | | Count | |
| 20 | | 19 | |
| 21 | | 10 | |
| 22 | | 7 | |
| 23 | | 3 | |
| 24 | | 1 | |
| 26 | | 1 | |

| **TABLE 15B: All Naïve - J & D Usage** | | | |
|---|---|---|---|
| J | D | Average HCDR3 Length | Count |
| IGHJ6*02 | IGHD3-9*01 | 19 | 21 |
| IGHJ6*02 | IGHV4-17*0 | 18 | 7 |
| IGHJ6*02 | IGHD3-10*01 | 17 | 98 |
| IGHJ6*02 | IGHD2-2*02 | 17 | 4 |
| IGHJ6*02 | IGHD5-24*01 | 17 | 1 |
| IGHJ6*02 | IGHD6-19*01 | 16 | 30 |
| IGHJ6*02 | IGHD3-22*01 | 16 | 5 |
| IGHJ6*02 | IGHD6-13*01 | 16 | 33 |
| IGHJ6*02 | IGHD5-12*01 | 15 | 7 |
| IGHJ6*02 | IGHD1-26*01 | 15 | 25 |
| IGHJ6*02 | IGHD1-20*01 | 14 | 6 |
| IGHJ6*02 | IGHD5-18*01 | 14 | 3 |
| IGHJ6*02 | IGHD3-16*02 | 14 | 6 |
| IGHJ6*02 | IGHD2-21*02 | 13 | 5 |
| IGHJ6*02 | IGHD1-14*01 | 13 | 4 |
| IGHJ6*02 | IGHD7-27*02 | 12 | 8 |
| IGHJ6*02 | IGHD-1*01 | 12 | 8 |
| IGHJ6*02 | IGHD6-25*01 | 12 | 3 |
| IGHJ6*02 | IGHD4-23*01 | 11 | 2 |

| **Naive HCDR3>=20** | | | |
|---|---|---|---|
| J | D | Average HCDR3 Length | Count |
| IGHJ6*02 | IGHD6-19*01 | 23 | 1 |
| IGHJ6*02 | IGHD4-17*01 | 22 | 2 |
| IGHJ6*02 | IGHD3-9*01 | 21 | 11 |
| IGHJ6*02 | IGHD3-10*01 | 21 | 25 |
| IGHJ6*02 | IGHD6-13*01 | 20 | 1 |
| IGHJ6*02 | IGHD3-22*01 | 20 | 1 |

| **TABLE 15C: All Naïve - V & J Usage** | | | |
|---|---|---|---|
| V | J | Average HCDR3 Length | Count |
| IGHV3-21*03 | IGHJ6*02 | 20 | 5 |
| IGHV1-18*01 | IGHJ6*02 | 20 | 10 |
| IGHV1-2*02 | IGHJ6*02 | 19 | 5 |
| IGHV3-13*01 | IGHJ6*02 | 19 | 3 |
| IGHV3-7*01 | IGHJ6*02 | 19 | 6 |
| IGHV-8*01 | IGHJ6*02 | 19 | 21 |
| IGH7-4-1*01 | IGHJ6*02 | 19 | 9 |
| IGHV3-9*01 | IGHJ6*02 | 18 | 13 |
| IGHV4-61*1 | IGHJ6*02 | 17 | 20 |
| IGHV3-23*04 | IGHJ6*02 | 17 | 6 |
| IGHV4-4*02 | IGHJ6*02 | 16 | 34 |
| IGHV-3*01 | IGHJ6*02 | 16 | 39 |
| IGHV3-20*d01 | IGH6*02 | 16 | 1 |
| IGHV6-1*01 | IGHJ6*02 | 14 | 69 |
| IGHV2-5*10 | IGHJ6*02 | 14 | 8 |
| IGHV3-11*01 | IGHJ6*02 | 14 | 3 |
| IGHV3-66*03 | IGHJ6*02 | 13 | 21 |
| IGHV3-15*01 | IGHJ6*02 | 12 | 3 |
| | | | |
| **Naive HCDR3>=20** | | | |

| V | J | Average HCDR3 Length | Count |
|---|---|---|---|
| IGHV3-21*03 | IGHJ6*02 | 23 | 3 |
| IGHV3-13*01 | IGHJ6*02 | 22 | 1 |
| IGHV3-7*01 | IGHJ6*02 | 22 | 3 |
| IGH6-1*01 | IGHJ6*02 | 21 | 3 |
| IGHV1-8*01 | IGHJ6*02 | 21 | 10 |
| IGHV1-2*02 | IGHJ6*02 | 21 | 3 |
| IGH7-4-1*01 | IGHJ6*02 | 21 | 3 |
| IGHV-3*01 | IGHJ6*02 | 21 | 4 |
| IGHV1-18*01 | IGHJ6*02 | 21 | 5 |
| IGHV4-4*02 | IGHJ6*02 | 20 | 3 |
| IGHV3-9*01 | IGHJ6*02 | 20 | 2 |
| IGHV3-23*04 | IGHJ6*02 | 20 | 1 |

**TABLE 16: IMMUNISED HEAVY CHAIN REPERTOIRES**

| **TABLE 16A:** **All Immunised** - **J** **Usage** | | | |
|---|---|---|---|
| J | HCDR3 Length | Count | % |
| IGHJ*01 | 11 | 2 | 0.78% |
| IGHJ2*01 | 14 | 7 | 2.73% |
| IGHJ3*02 | 15 | 12 | 4.69% |
| IGHJ4*02 | 15 | 120 | 46.88% |
| IGHJ5*02 | 15 | 19 | 7.42% |
| **IGHJ6*02** | **17** | **96** | **37.50%** |
| | | | |

| **HCDR3>20** | | | |
|---|---|---|---|
| J | HCDR3 Length | Count | % |
| IGHJ4*02 | 22 | 2 | 22.22% |
| IGHV5*02 | 25 | 1 | 11.11% |
| **IGHJ6*02** | **21** | **6** | **66.67%** |

**Immunised - Long HCDR3 using IGHJ6*02**

| HCDR3Length | Count |
|---|---|
| 20 | 4 |
| 21 | 1 |
| 24 | 1 |

**TABLE 16B: All Immunised - J & D Usage**

| J | D | HCDR3 Length | Count |
|---|---|---|---|
| IGHJ6*02 | IGHD4-17*01 | 18 | 1 |
| IGHJ6*02 | IGHD-26*01 | 18 | 5 |
| IGHJ6*02 | IGHD6-19*01 | 17 | 27 |
| IGHJ6*02 | IGHD3-10*01 | 17 | 42 |
| IGHJ6*02 | IGHD6-13*01 | 16 | 2 |
| IGHJ6*02 | IGHD5-18*01 | 15 | 2 |
| IGHJ6*02 | IGHD4-23*01 | 15 | 5 |
| IGHJ6*02 | IGHD5-12*01 | 14 | 3 |
| IGHJ6*02 | IGHD3-16*02 | 14 | 3 |
| IGHJ6*02 | IGHD3-22*01 | 13 | 3 |
| IGHJ6*02 | IGHD3-9*01 | 12 | 1 |
| IGH6*02 | IGHD1-20*01 | 11 | 1 |
| | | | |

| **HCDR3>20** | | | |
|---|---|---|---|
| J | D | HCDR3 Length | Count |
| IGHJ6*02 | IGHD3-10*01 | 21 | 4 |
| IGHJ6*02 | IGHD6-19*01 | 20 | 1 |
| IGHJ6*02 | IGHD1-26*01 | 20 | 1 |

**TABLE 16C: All Immunised - V & J Usage**

| V | J | HCDR3 Length | Count |
|---|---|---|---|
| IGHV3-7*01 | IGHJ6*02 | 18 | 33 |
| IGHV-8*01 | IGHJ6*02 | 17 | 3 |
| IGHV3-9*01 | IGHJ6*02 | 17 | 22 |
| IGHV3-13*01 | IGHJ6*02 | 17 | 5 |
| IGHV1-2*02 | IGHJ6*02 | 16 | 8 |
| IGHV3-11*01 | IGHJ6*02 | 16 | 3 |
| IGHV4-4*02 | IGHJ6*02 | 15 | 9 |
| IGHV6-1*01 | IGHJ6*02 | 15 | 6 |
| IGHV1-3*01 | IGHJ6*02 | 15 | 6 |

| **HCDR3>20** | | | |
|---|---|---|---|
| V | J | HCDR3 Length | Count |
| IGHV3-7*01 | IGHJ6*02 | 21 | 4 |
| IGHV3-11*01 | IGHJ6*02 | 21 | 1 |
| IGHV4-4*02 | IGHJ6*02 | 20 | 1 |

**TABLE 17: ANTIGEN-SPECIFIC HEAVY CHAIN REPERTOIRES**

| **TABLE 17A:** **All Antigen**-**Specific** - **J** **Usage** | | | |
|---|---|---|---|
| J | HCDR3 Length | Count | % |
| IGHJ3*02 | 12 | 2 | 1.68% |
| IGHJ3*02 | 17 | 4 | 3.36% |
| IGHJ4*02 | 13 | 64 | 53.78% |
| IGHJ5*02 | 19 | 6 | 5.04% |
| **IGHJ6*02** | **17** | **43** | **36.13%** |
| | | | |
| **HCDR3>20** | | | |

| | | | |
|---|---|---|---|
| J | HCDR3 Length | Count | % |
| IGHJ4*02 | 22 | 1 | 9.09% |
| IGHJ5*02 | 21 | 3 | 27.27% |
| **IGHJ6*02** | **21** | **7** | **63**.**64%** |

**Immunised - Long HCDR3 using IGHJ6*02**

| HCDR3Length | Count |
|---|---|
| 20 | 4 |
| 21 | 1 |
| 22 | 1 |
| 24 | 1 |

| **TABLE 17B:** **All Antigen-Specific** - **J & D** **Usage** | | | |
|---|---|---|---|
| J | D | HCDR3 Length | Count |
| IGHJ6*02 | IGHD3-9*01 | 19 | 6 |
| IGHJ6*02 | IGHD3-10*01 | 19 | 12 |
| IGHJ6*02 | IGHD6-19*01 | 17 | 13 |
| IGHJ6*02 | IGHD5-12*01 | 16 | 2 |
| IGHJ6*02 | IGHD2-15*01 | 16 | 1 |
| IGHJ6*02 | IGHD1-26*01 | 15 | 7 |
| IGHJ6*02 | IGHD3-16*02 | 12 | 1 |
| IGHJ6*02 | IGHD5-18*01 | 11 | 1 |
| | | | |

| **HCDR3>20** | | | |
|---|---|---|---|
| J | D | HCDR3 Length | Count |
| IGHJ6*02 | IGHD3-9*01 | 21 | 1 |
| IGHJ6*02 | IGHD3-10*01 | 21 | 6 |

| **TABLE E 17C:** **All Antigen-Specific** - **V &** **J Usag**e | | | |
|---|---|---|---|
| V | J | HCDR3 Length | Count |
| IGHV-3*01 | IGHJ6*02 | 18 | 1 |
| IGHV3-7*01 | IGHJ6*02 | 18 | 6 |
| IGHV3-9*01 | IGHJ6*02 | 18 | 6 |
| IGHV-8*01 | IGHJ6*02 | 17 | 6 |
| IGHV3-20*d01 | IGHJ6*02 | 17 | 3 |
| IGH3-11*01 | IGHJ6*02 | 17 | 8 |
| IGHV4-4*02 | IGHJ6*02 | 17 | 5 |
| IGH3-15*01 | IGHJ6*02 | 15 | 5 |
| IGHV3-13*01 | IGHJ6*02 | 15 | 3 |
| | | | |

| **HCDR3>20** | | | |
|---|---|---|---|
| V | J | HCDR3 Length | Count |
| IGHV4-4*02 | IGHJ6*02 | 20 | 2 |
| IGHV-8*01 | IGHJ6*02 | 22 | 2 |
| IGHV3-9*01 | IGHJ6*02 | 20 | 1 |
| IGH3-11*01 | IGHJ6*02 | 21 | 1 |
| IGHV3-20*d01 | IGHJ6*02 | 22 | 1 |

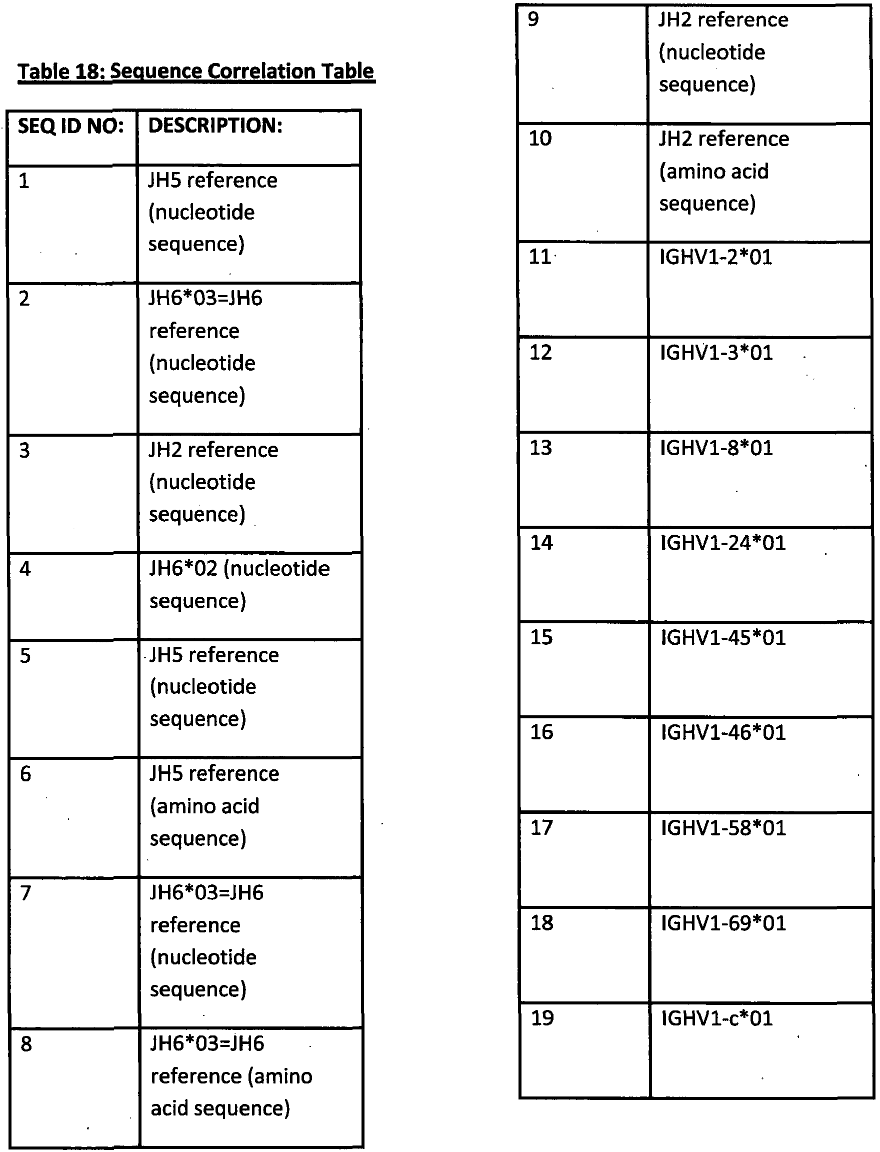

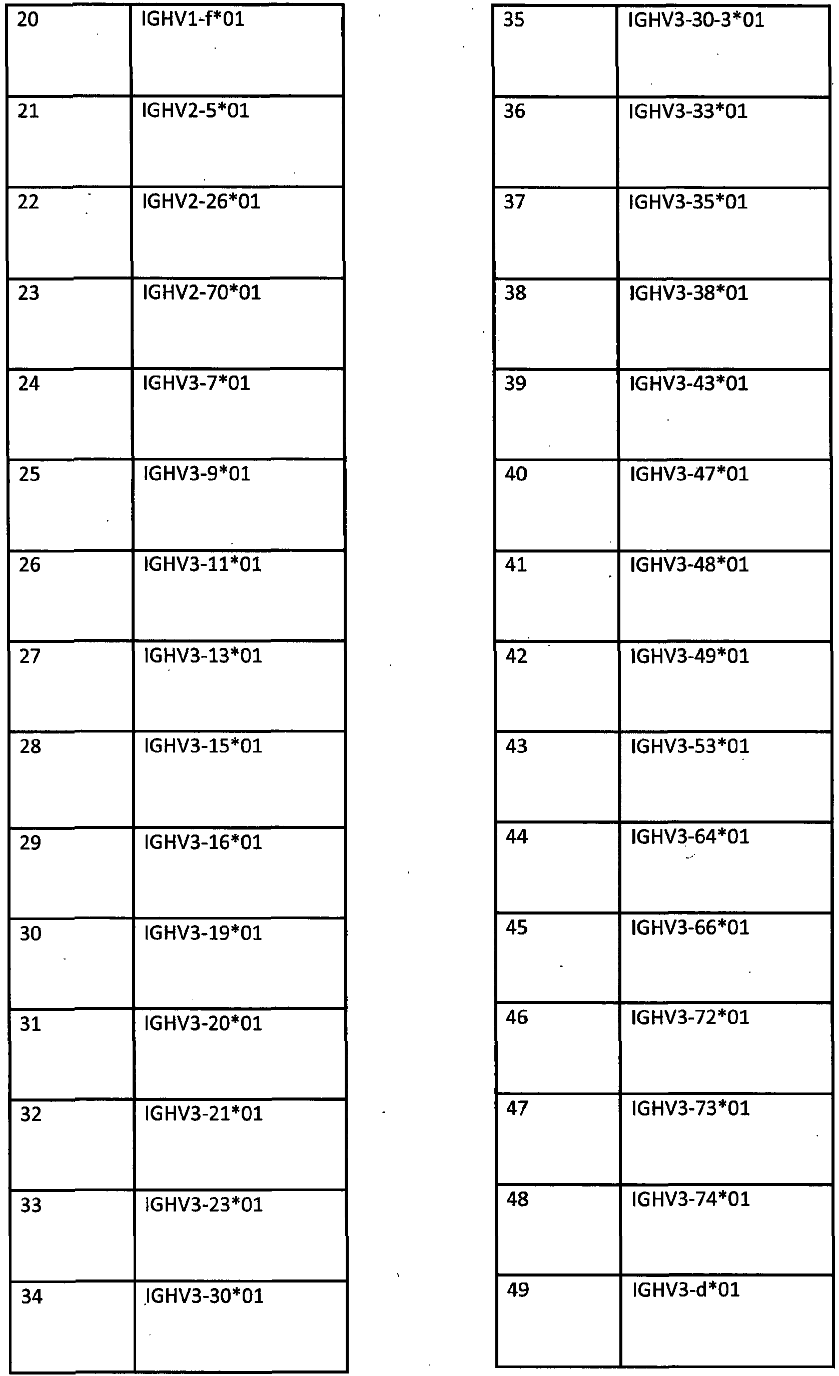

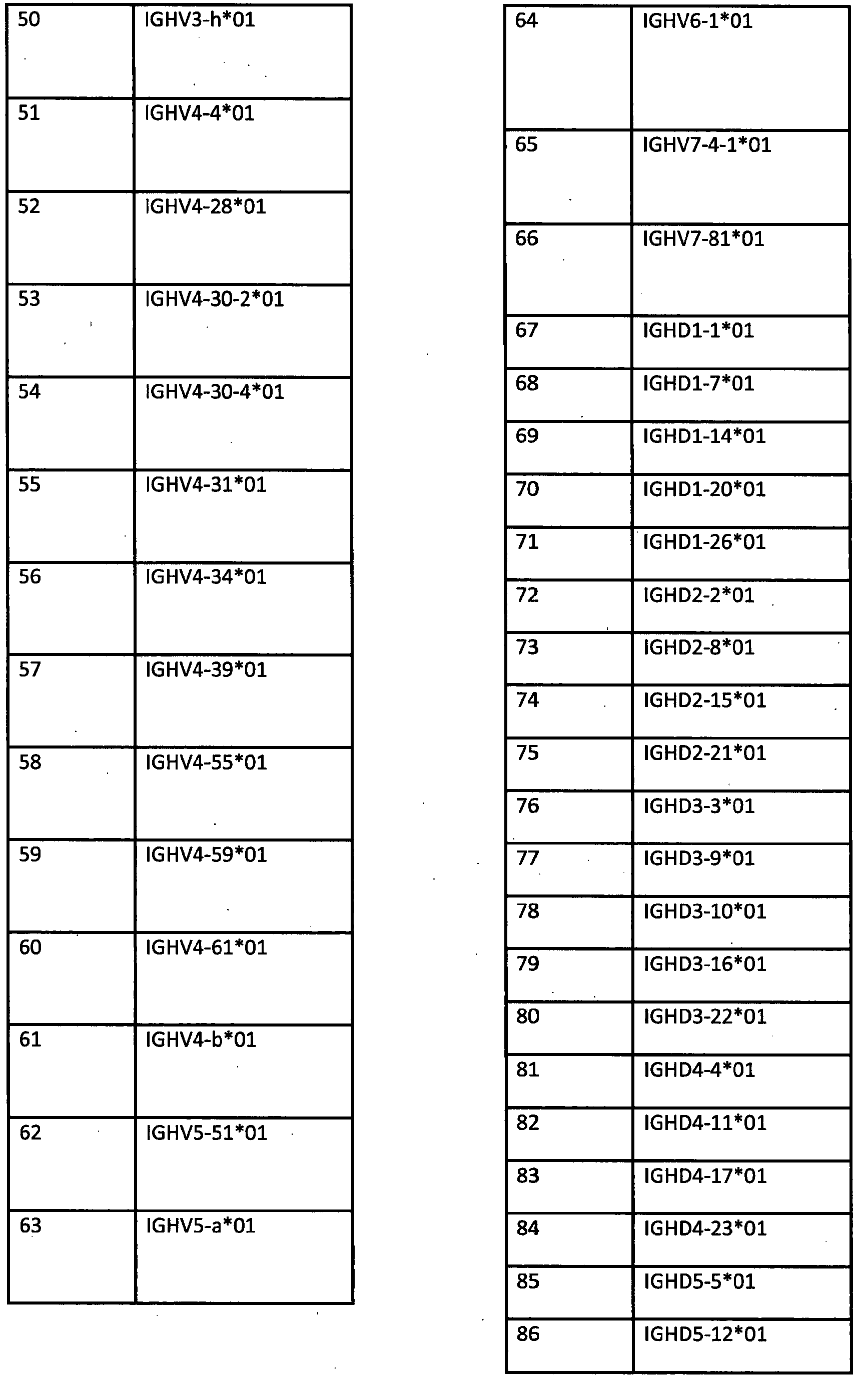

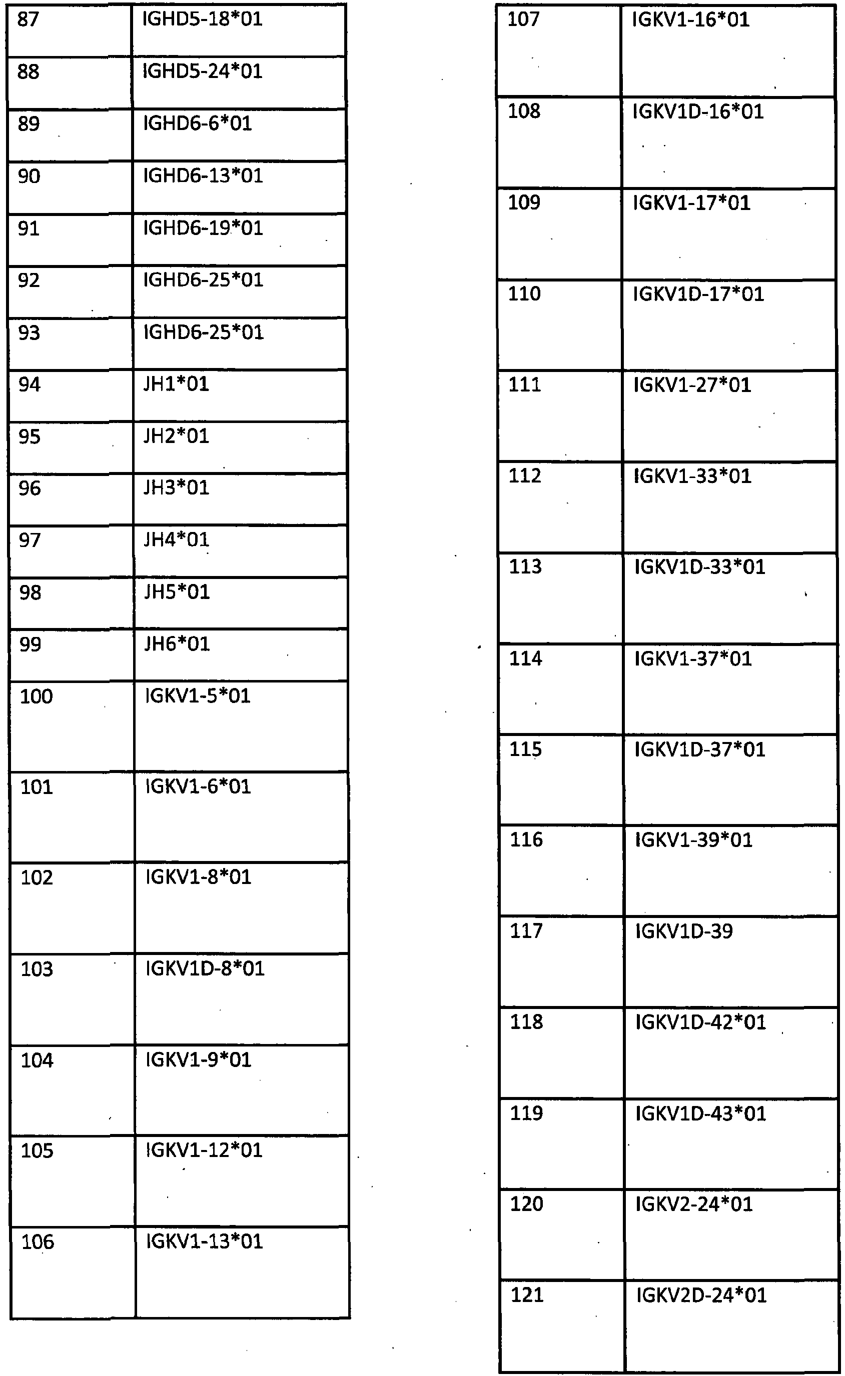

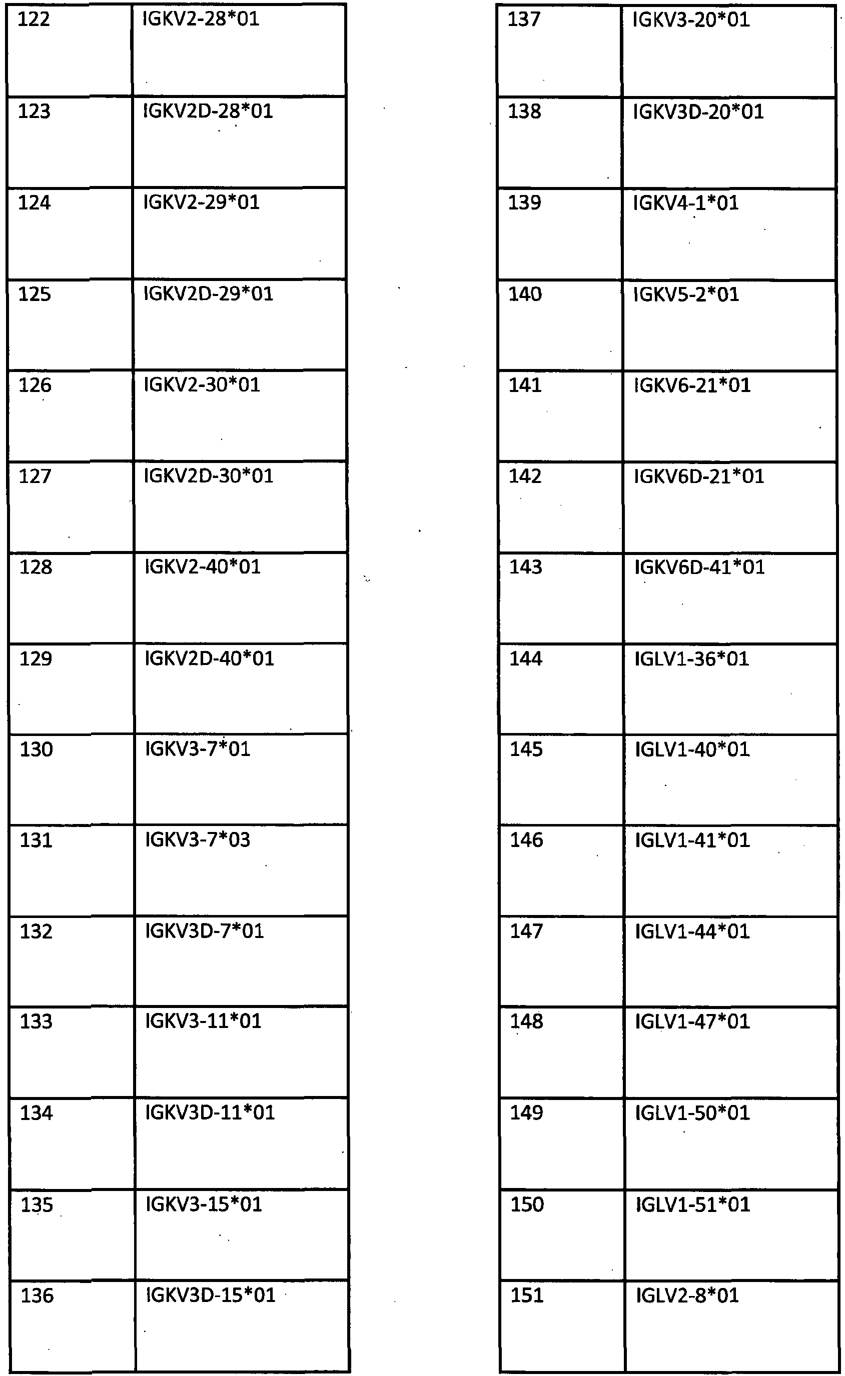

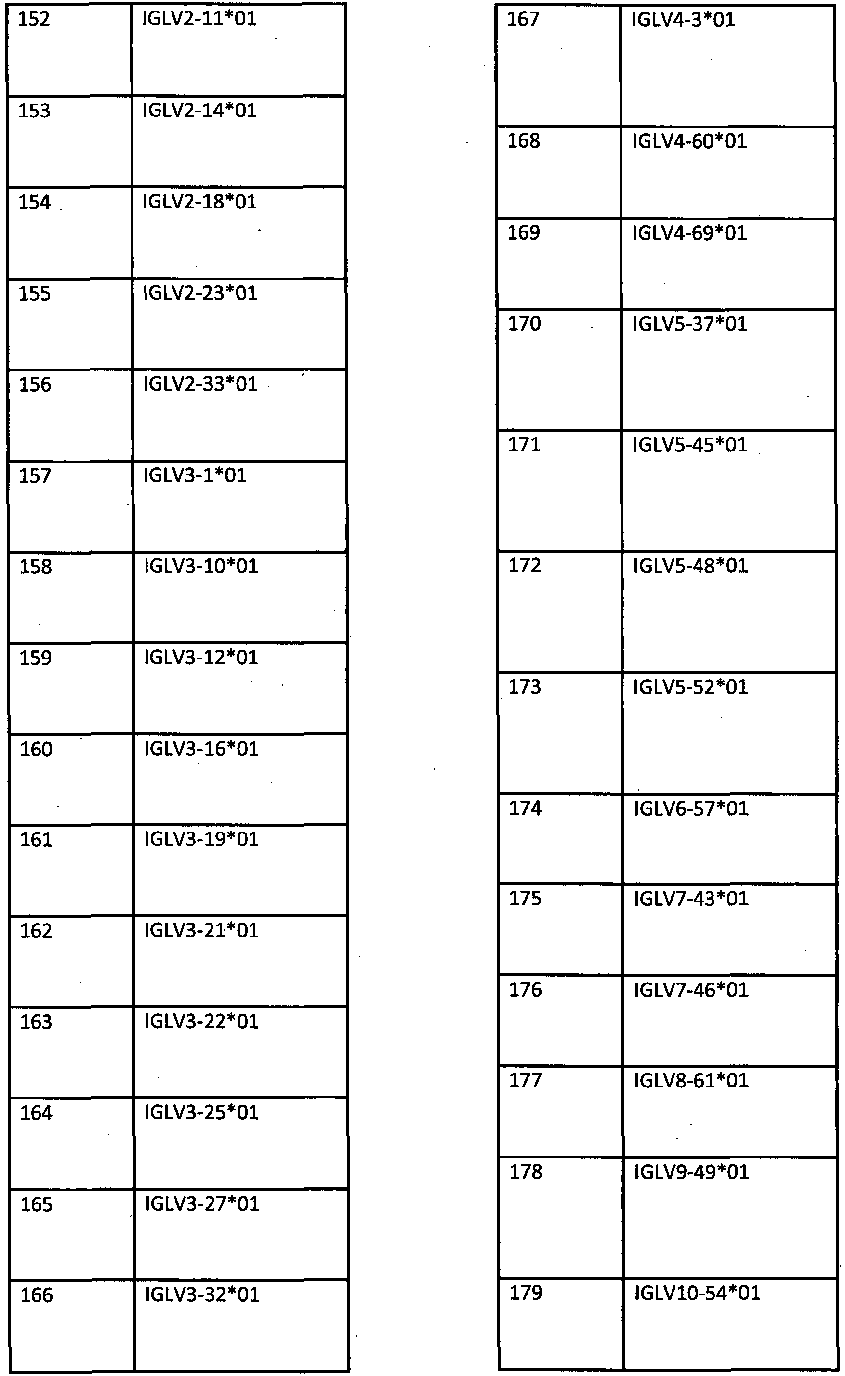

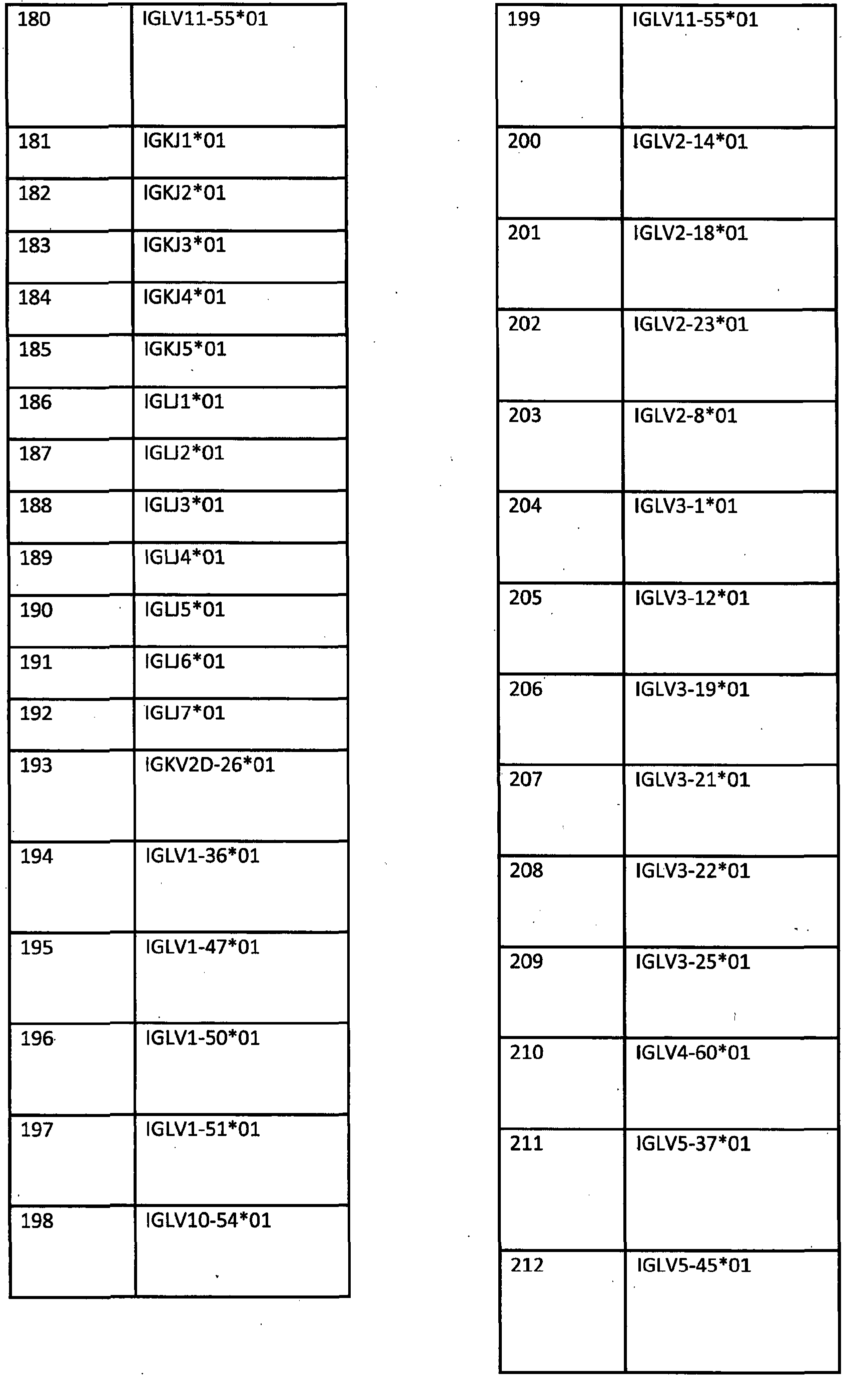

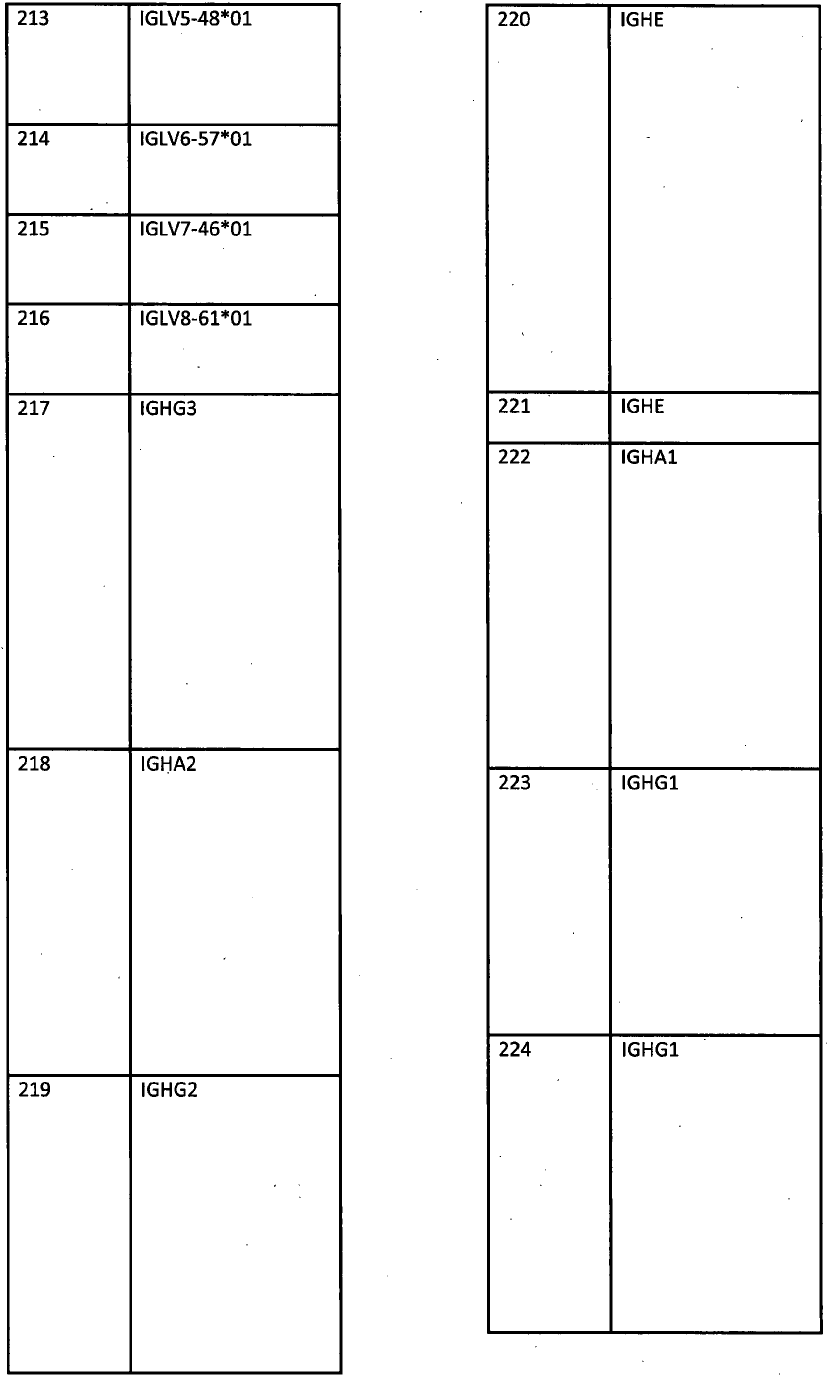

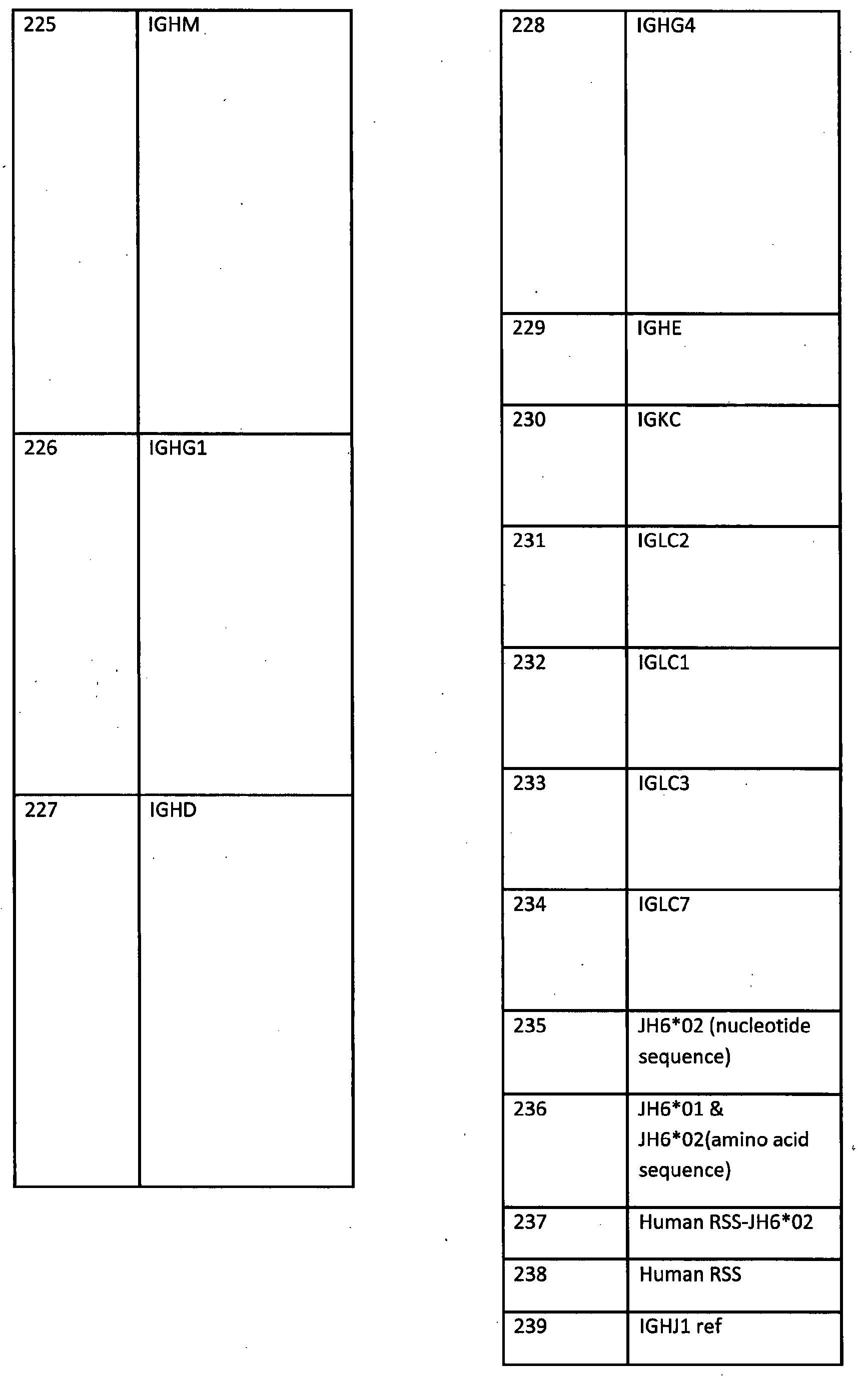

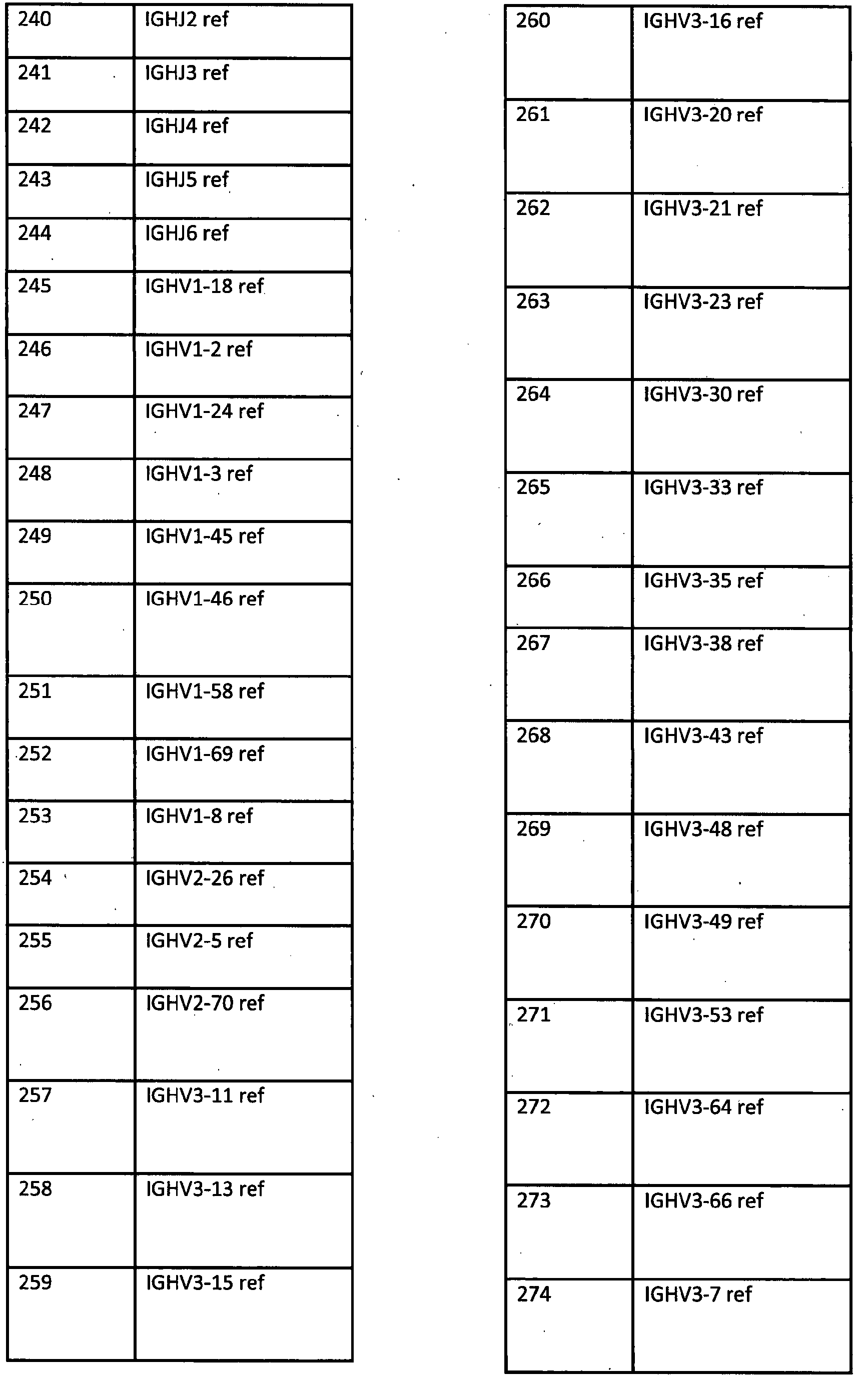

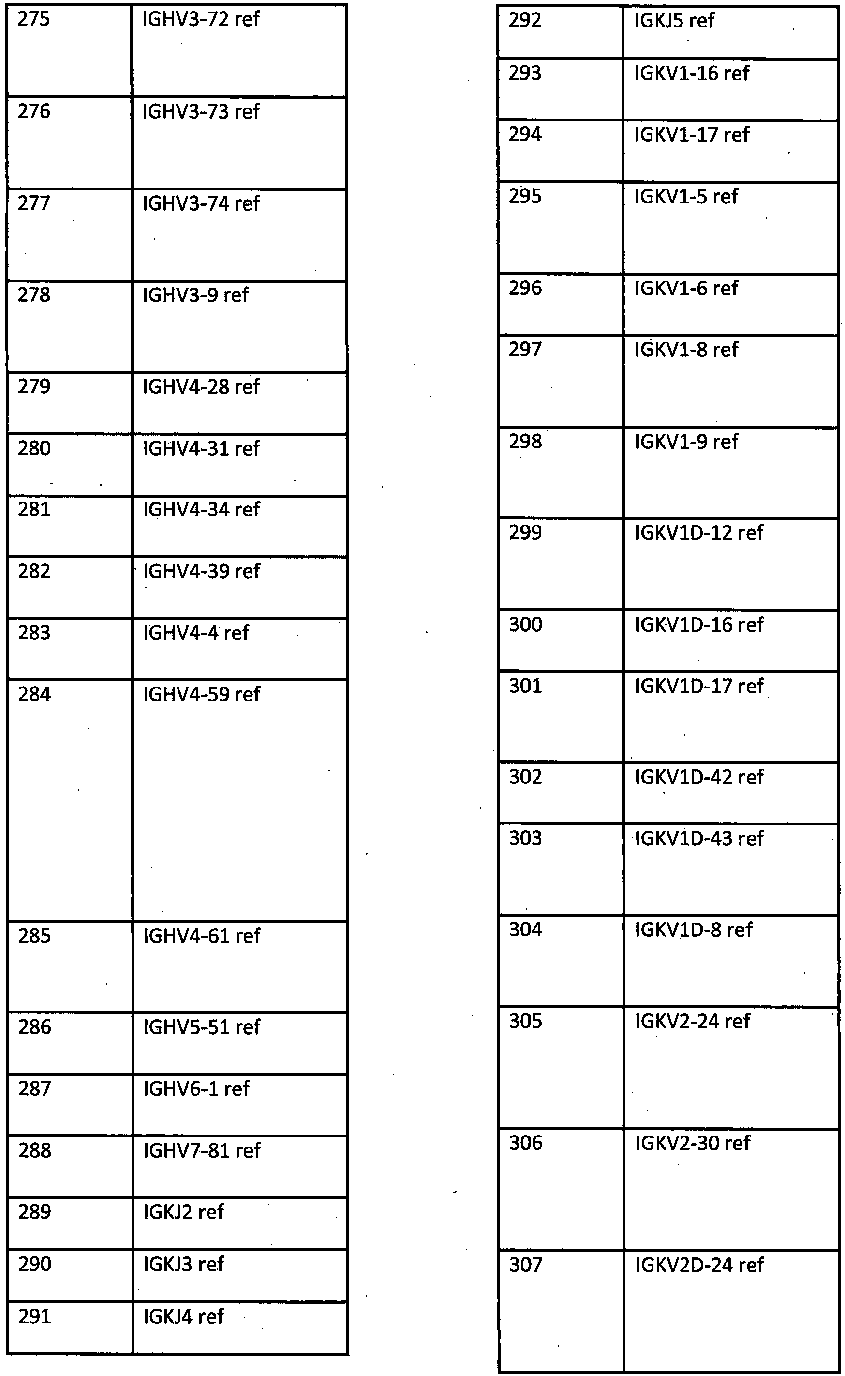

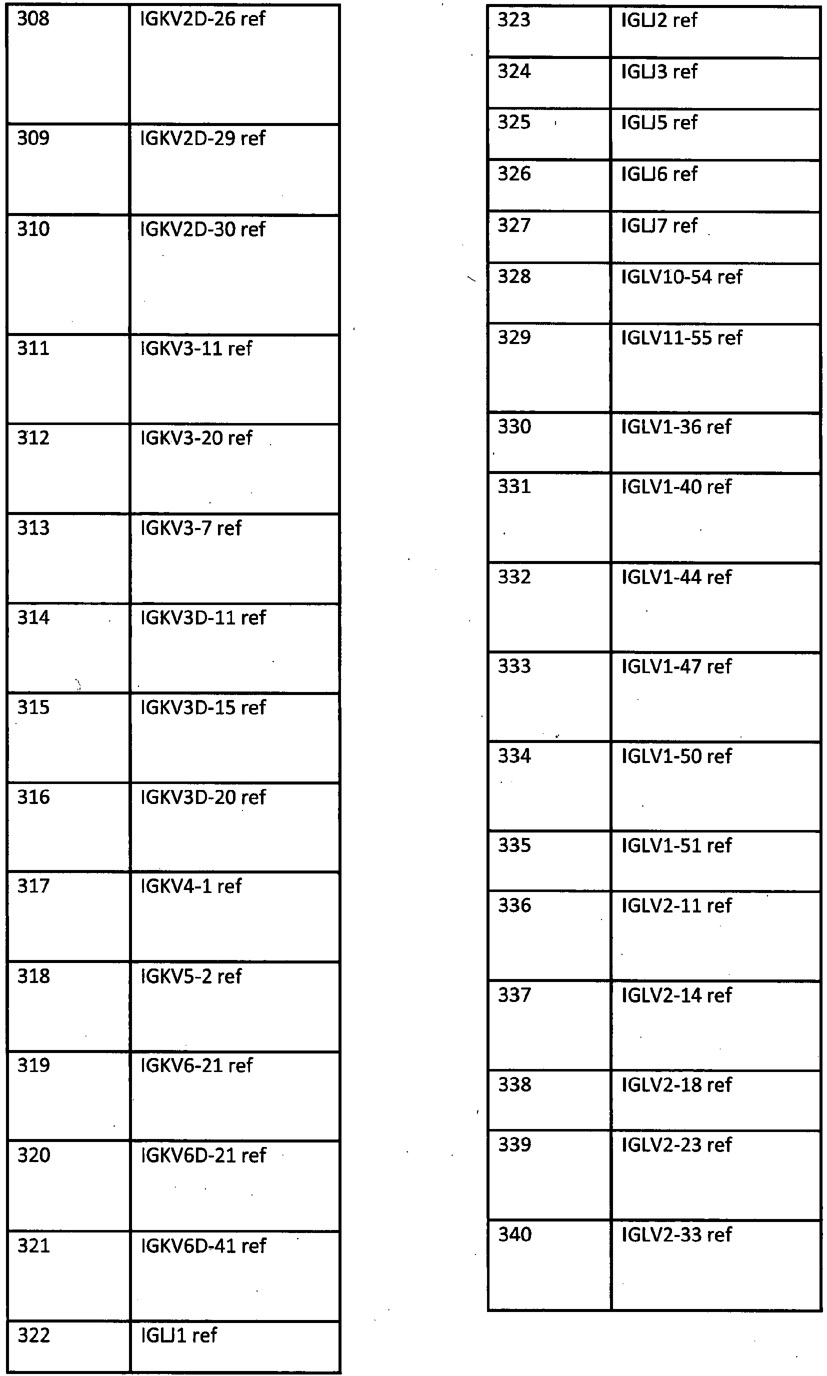

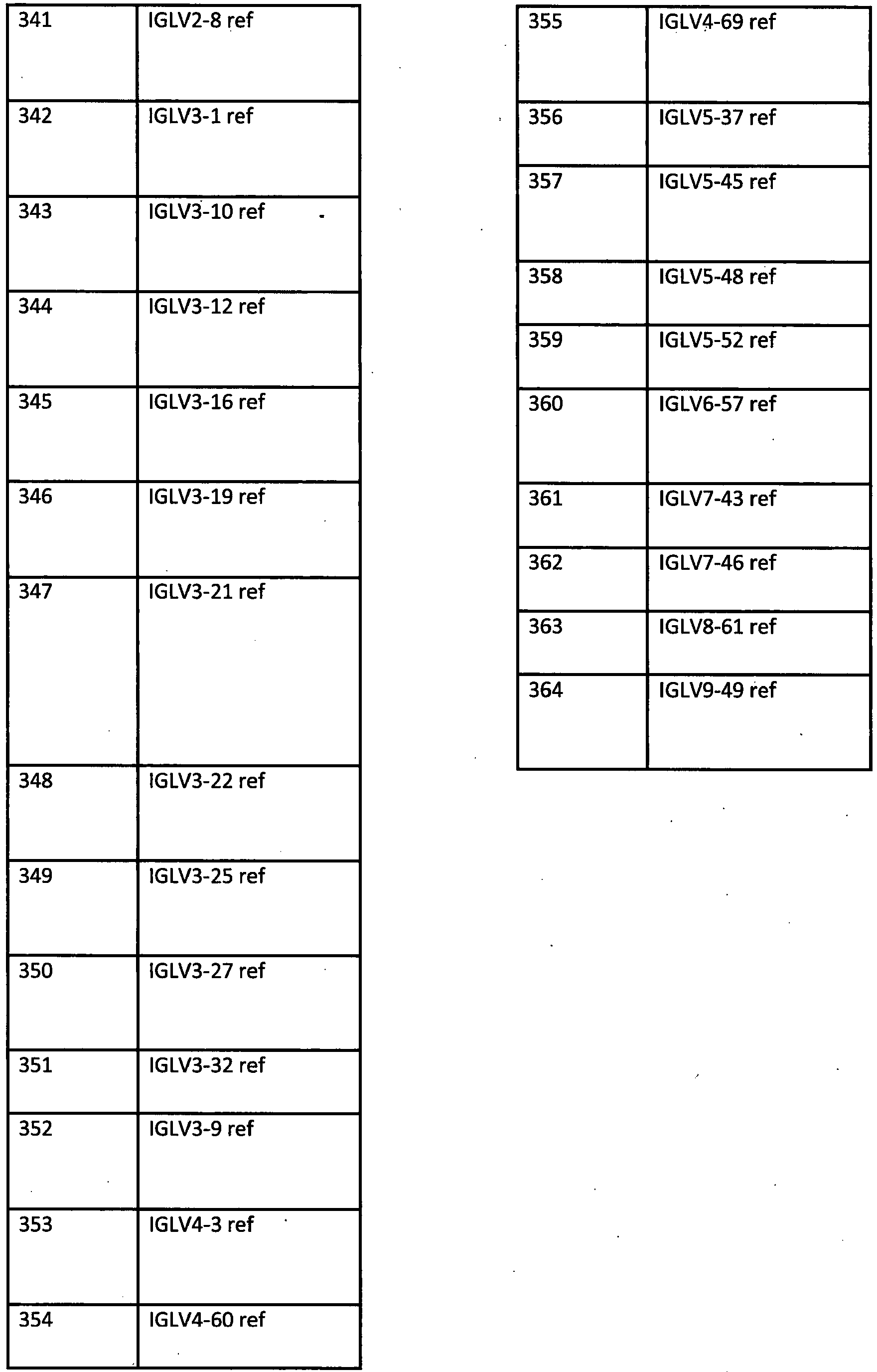

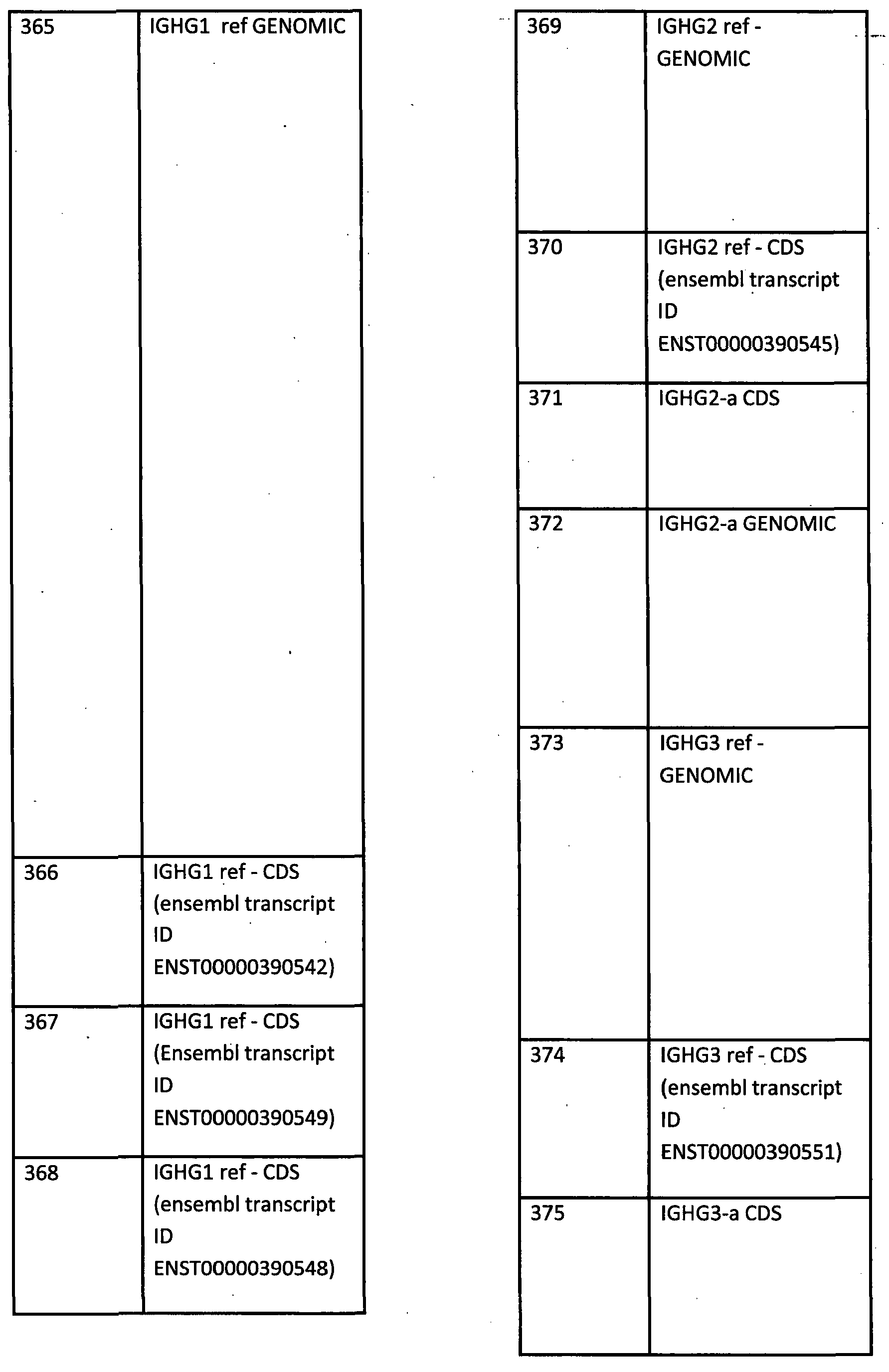

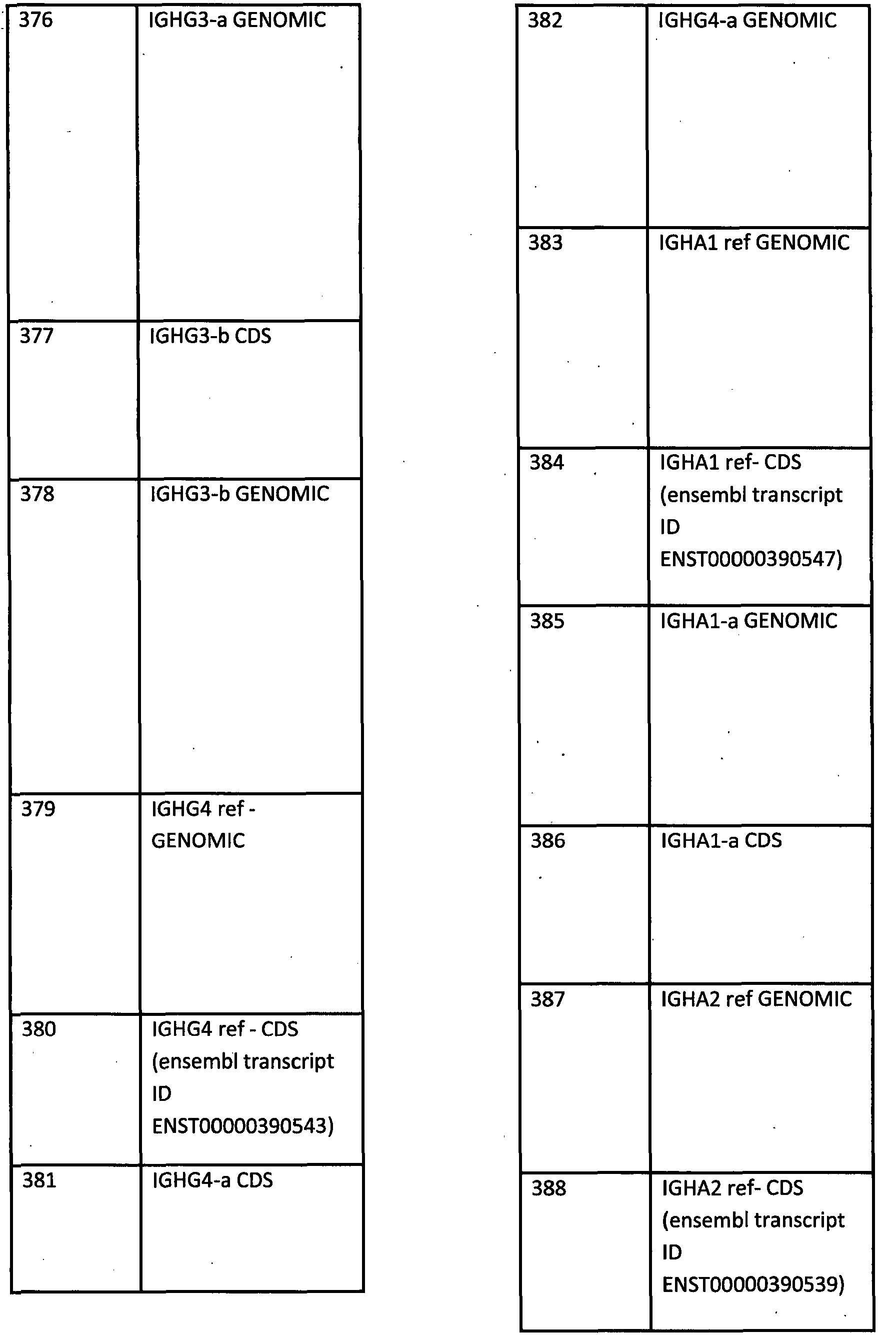

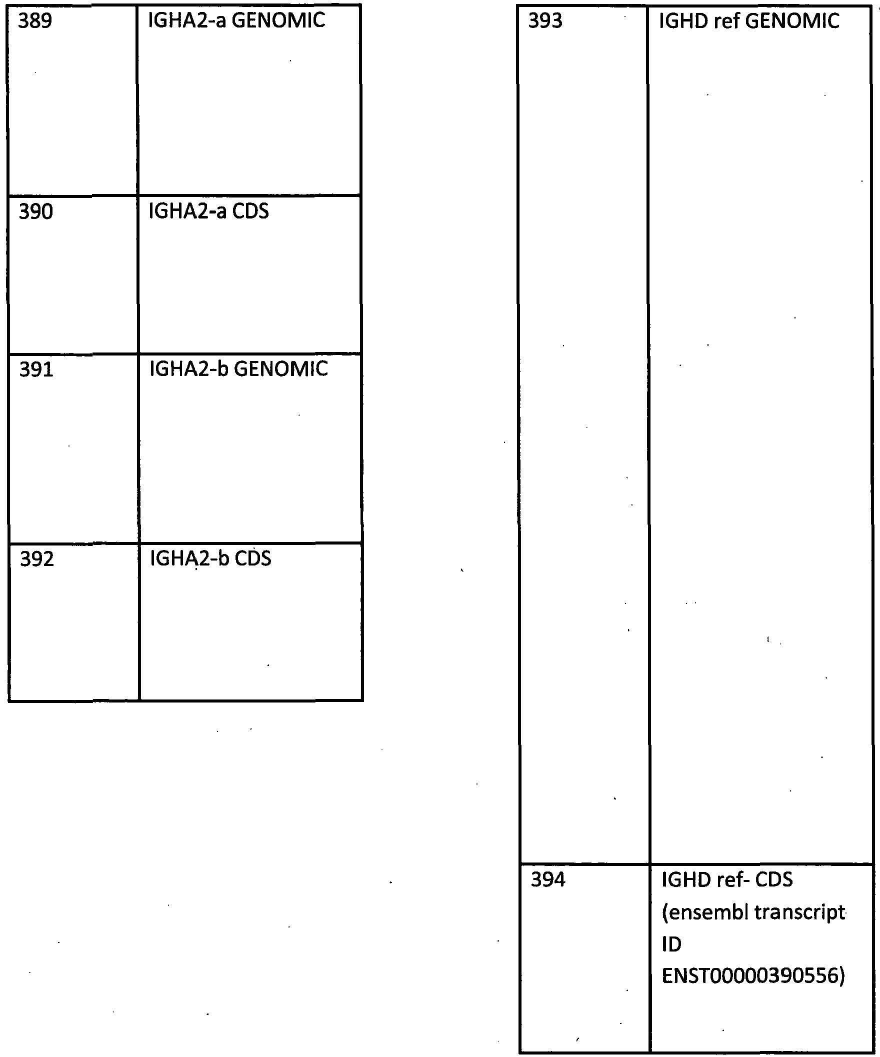

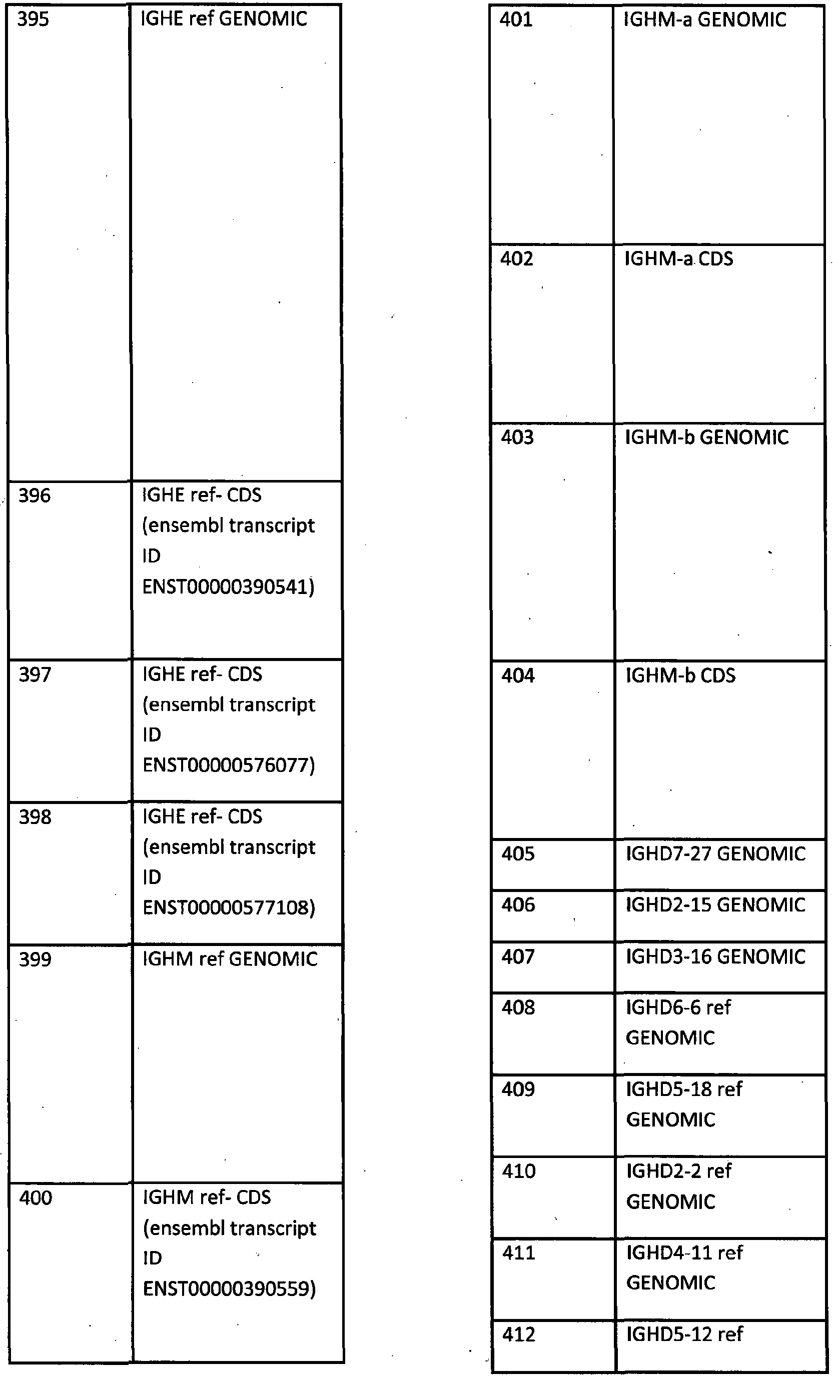

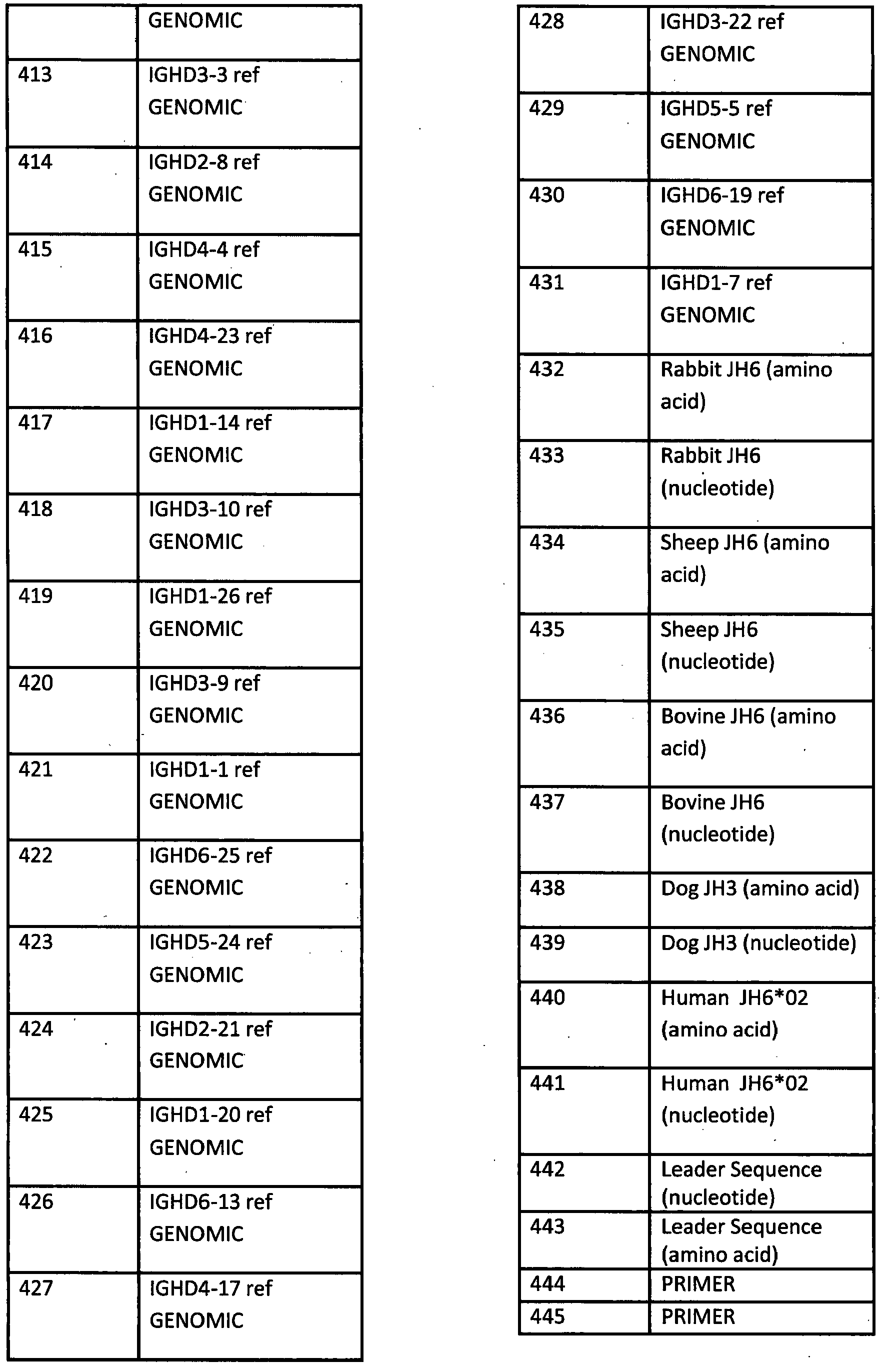

**Table 19**

| | IgG1 | IgG2 | IgG3 | IgG4 |
|---|---|---|---|---|
| Comp lement activation | | | | |
| Classical pathway | +++ | + | +++ | - |
| Alternative pathway | - | + | - | - |
| | | | | |
| Fc receptor recognition | | | | |
| FcγRI | +++ | - | +++ | ++ |
| | | | | |
| FcγRIIa, 131R/R | ++ | - | ++ | - |
| FcγRIIa, 131H/H | + | + | ++ | - |
| FcγRIIb | ++ | - | ++ | + |
| | | | | |
| FcγRIII | + | +/- | + | +/- |

### Table 20

**Table 20A: Variants Found In No More Than 3 Human Populations**

| **TYPE** | **VARIANT** | **C.FREQ** | **C.FREQ (%)** | **#INDIVIDUALS** | **#POPULATIONS** |
|---|---|---|---|---|---|
| IGKV | IGKV1D-8-b | 0.01785 | 1.79% | 36 | 3 |
| IGHV | IGHV3-11-a | 0.01695 | 1.70% | 36 | 3 |
| IGHV | IGHV5-51-a | 0.0164 | 1.64% | 35 | 3 |
| IGHV | IGHV2-5-b | 0.01055 | 1.06% | 18 | 3 |
| IGLV | IGLV2-14-g | 0.0104 | 1.04% | 22 | 3 |
| IGHV | IGHV7-81-b | 0.01005 | 1.01% | 21 | 3 |
| IGKV | IGKV1D-42-b | 0.01005 | 1.01% | 21 | 3 |
| IGKV | IGKV3-7-b | 0.01 | 1.00% | 19 | 3 |
| IGHV | IGHV1-69-d | 0.00995 | 1.00% | 22 | 3 |
| IGLV | IGLV2-14-e | 0.00995 | 1.00% | 19 | 3 |
| IGLV | IGLV9-49-a | 0.00865 | 0.87% | 18 | 3 |
| IGLV | IGLV1-44-b | 0.00775 | 0.78% | 16 | 2 |
| IGLV | IGLV2-11-a | 0.00775 | 0.78% | 17 | 3 |
| IGKV | IGKV3-11-d | 0.0073 | 0.73% | 14 | 3 |
| IGKV | IGKV1D-43-b | 0.00685 | 0.69% | 14 | 3 |
| IGHV | IGHV3-38-d | 0.0064 | 0.64% | 13 | 3 |
| IGLJ | IGU6-a | 0.00595 | 0.60% | 13 | 3 |
| IGHV | IGHV5-51-b | 0.0055 | 0.55% | 11 | 3 |
| IGLV | IGLV5-45-c | 0.00545 | 0.55% | 12 | 2 |
| IGHV | IGHV4-31-i | 0.0054 | 0.54% | 10 | 3 |
| IGKV | IGKV2D-26-b | 0.00455 | 0.46% | 9 | 3 |
| IGLV | IGLV1-36-c | 0.00455 | 0.46% | 10 | 2 |
| IGHV | IGHV1-24-c | 0.00415 | 0.42% | 9 | 3 |
| IGHV | IGHV1-8-c | 0.00405 | 0.41% | 8 | 2 |
| IGLV | IGLV1-47-d | 0.00405 | 0.41% | 9 | 3 |
| IGHV | IGHV3-43-i | 0.00365 | 0.37% | 8 | 3 |
| IGHV | IGHV3-53-h | 0.0036 | 0.36% | 8 | 2 |
| IGLV | IGLV10-54-e | 0.0036 | 0.36% | 8 | 3 |
| IGLV | IGLV1-36-f | 0.0036 | 0.36% | 8 | 2 |
| IGLV | IGLV5-45-d | 0.0036 | 0.36% | 8 | 3 |
| IGHV | IGHV1-46-b | 0.0032 | 0.32% | 7 | 3 |
| IGHV | IGHV2-26-c | 0.0032 | 0.32% | 7 | 2 |
| IGU | IGU6-b | 0.0032 | 0.32% | 7 | 3 |
| IGLV | IGLV3-32-a | 0.0032 | 0.32% | 7 | 3 |
| IGLV | IGLV9-49-b | 0.0032 | 0.32% | 7 | 2 |
| IGHJ | IGHJ6-b | 0.00315 | 0.32% | 7 | 2 |
| IGHV | IGHV3-16-d | 0.00315 | 0.32% | 7 | 3 |
| IGHV | IGHV4-28-k | 0.00315 | 0.32% | 7 | 3 |
| IGKJ | IGKJ2-d | 0.00315 | 0.32% | 6 | 3 |
| IGLV | IGLV10-54-f | 0.00315 | 0.32% | 7 | 2 |
| IGLV | IGLV2-8-c | 0.00315 | 0.32% | 7 | 3 |
| IGLV | IGLV3-10-c | 0.00315 | 0.32% | 6 | 2 |
| IGLV | IGLV1-40-c | 0.00275 | 0.28% | 6 | 2 |
| IGLV | IGLV1-51-b | 0.00275 | 0.28% | 6 | 3 |
| IGHV | IGHV1-69-m | 0.0027 | 0.27% | 6 | 3 |
| IGHV | IGHV3-33-e | 0.0027 | 0.27% | 6 | 3 |
| IGHV | IGHV3-49-c | 0.0027 | 0.27% | 6 | 3 |
| IGHV | IGHV3-66-g | 0.0027 | 0.27% | 6 | 3 |
| IGHV | IGHV4-4-u | 0.0027 | 0.27% | 6 | 3 |
| IGKV | IGKV1D-42-c | 0.0027 | 0.27% | 6 | 1 |
| IGKV | IGKV1D-43-c | 0.0027 | 0.27% | 5 | 3 |
| IGLV | IGLV10-54-h | 0.0027 | 0.27% | 6 | 2 |
| IGLV | IGLV10-54-i | 0.0027 | 0.27% | 6 | 2 |
| IGLV | IGLV1-36-g | 0.0027 | 0.27% | 6 | 3 |
| IGLV | IGLV2-8-d | 0.0027 | 0.27% | 6 | 3 |
| IGLV | IGLV3-22-e | 0.0027 | 0.27% | 6 | 1 |
| IGLV | IGLV3-22-d | 0.0027 | 0.27% | 6 | 3 |
| IGHJ | IGHJ2-b | 0.00225 | 0.23% | 4 | 3 |
| IGHV | IGHV1-3-u | 0.00225 | 0.23% | 5 | 2 |
| IGHV | IGHV1-3-v | 0.00225 | 0.23% | 5 | 2 |
| IGHV | IGHV1-3-ap | 0.00225 | 0.23% | 4 | 3 |
| IGHV | IGHV1-46-c | 0.00225 | 0.23% | 5 | 3 |
| IGHV | IGHV1-58-f | 0.00225 | 0.23% | 5 | 3 |
| IGHV | IGHV1-69-j | 0.00225 | 0.23% | 5 | 3 |
| IGHV | IGHV3-13-e | 0.00225 | 0.23% | 5 | 3 |
| IGHV | IGHV3-23-f | 0.00225 | 0.23% | 5 | 3 |
| IGHV | IGHV3-23-g | 0.00225 | 0.23% | 5 | 3 |
| IGHV | IGHV4-28-p | 0.00225 | 0.23% | 5 | 3 |
| IGHV | IGHV4-31-j | 0.00225 | 0.23% | 5 | 3 |
| IGKV | IGKV1-17-d | 0.00225 | 0.23% | 4 | 1 |
| IGKV | IGKV3-20-a | 0.00225 | 0.23% | 5 | 3 |
| IGKV | IGKV3-7-c | 0.00225 | 0.23% | 5 | 3 |
| IGKV | IGKV3-7-d | 0.00225 | 0.23% | 5 | 3 |
| IGKV | IGKV3D-11-b | 0.00225 | 0.23% | 5 | 3 |
| IGU | IGU3-a | 0.00225 | 0.23% | 4 | 2 |
| IGLV | IGLV11-55-g | 0.00225 | 0.23% | 5 | 3 |
| IGLV | IGLV1-50-c | 0.00225 | 0.23% | 5 | 3 |
| IGLV | IGLV2-11-g | 0.00225 | 0.23% | 5 | 3 |
| IGLV | IGLV2-14-n | 0.00225 | 0.23% | 5 | 3 |
| IGLV | IGLV2-23-f | 0.00225 | 0.23% | 5 | 2 |
| IGLV | IGLV3-19-c | 0.00225 | 0.23% | 5 | 2 |
| IGLV | IGLV5-37-c | 0.00225 | 0.23% | 4 | 2 |
| IGLV | IGLV5-48-k | 0.00225 | 0.23% | 5 | 3 |
| IGLV | IGLV2-14-i | 0.00185 | 0.19% | 4 | 2 |
| IGHJ | IGHJ1-a | 0.0018 | 0.18% | 3 | 3 |
| IGHV | IGHV1-2-g | 0.0018 | 0.18% | 4 | 3 |
| IGHV | IGHV1-3-y | 0.0018 | 0.18% | 4 | 3 |
| IGHV | IGHV1-3-ar | 0.0018 | 0.18% | 4 | 3 |
| IGHV | IGHV1-3-ai | 0.0018 | 0.18% | 4 | 3 |
| IGHV | IGHV1-69-s | 0.0018 | 0.18% | 4 | 3 |
| IGHV | IGHV1-8-d | 0.0018 | 0.18% | 4 | 2 |
| IGHV | IGHV2-5-d | 0.0018 | 0.18% | 4 | 2 |
| IGHV | IGHV3-13-j | 0.0018 | 0.18% | 4 | 2 |
| IGHV | IGHV3-21-d | 0.0018 | 0.18% | 4 | 3 |
| IGHV | IGHV3-23-n | 0.0018 | 0.18% | 4 | 3 |
| IGHV | IGHV3-33-d | 0.0018 | 0.18% | 4 | 3 |
| IGHV | IGHV3-64-e | 0.0018 | 0.18% | 4 | 2 |
| IGHV | IGHV3-9-g | 0.0018 | 0.18% | 4 | 3 |
| IGHV | IGHV4-39-y | 0.0018 | 0.18% | 4 | 3 |
| IGHV | IGHV4-39-n | 0.0018 | 0.18% | 4 | 3 |
| IGHV | IGHV4-39-o | 0.0018 | 0.18% | 4 | 3 |
| IGHV | IGHV7-81-c | 0.0018 | 0.18% | 4 | 3 |
| IGKJ | IGKJ2-e | 0.0018 | 0.18% | 4 | 3 |
| IGKV | IGKV2D-26-c | 0.0018 | 0.18% | 4 | 1 |
| IGKV | IGKV3D-11-g | 0.0018 | 0.18% | 3 | 2 |
| IGKV | IGKV3D-11-f | 0.0018 | 0.18% | 4 | 3 |
| IGKV | IGKV4-1-w | 0.0018 | 0.18% | 3 | 3 |
| IGU | IGLI1-a | 0.0018 | 0.18% | 4 | 2 |
| IGLV | IGLV10-54-k | 0.0018 | 0.18% | 4 | 2 |
| IGLV | IGLV11-55-e | 0.0018 | 0.18% | 4 | 3 |
| IGLV | IGLV1-36-e | 0.0018 | 0.18% | 4 | 3 |
| IGLV | IGLV1-40-f | 0.0018 | 0.18% | 4 | 3 |
| IGLV | IGLV1-44-d | 0.0018 | 0.18% | 4 | 3 |
| IGLV | IGLV1-44-e | 0.0018 | 0.18% | 4 | 3 |
| IGLV | IGLV1-47-g | 0.0018 | 0.18% | 4 | 2 |
| IGLV | IGLV1-50-d | 0.0018 | 0.18% | 4 | 2 |
| IGLV | IGLV1-51-d | 0.0018 | 0.18% | 4 | 2 |
| IGLV | IGLV1-51-c | 0.0018 | 0.18% | 4 | 3 |
| IGLV | IGLV2-14-r | 0.0018 | 0.18% | 4 | 1 |
| IGLV | IGLV2-33-b | 0.0018 | 0.18% | 4 | 3 |
| IGLV | IGLV2-8-e | 0.0018 | 0.18% | 4 | 2 |
| IGLV | IGLV3-16-h | 0.0018 | 0.18% | 4 | 3 |
| IGLV | IGLV3-21-j | 0.0018 | 0.18% | 4 | 3 |
| IGLV | IGLV3-22-g | 0.0018 | 0.18% | 4 | 3 |
| IGLV | IGLV3-22-h | 0.0018 | 0.18% | 4 | 2 |
| IGLV | IGLV5-48-j | 0.0018 | 0.18% | 4 | 3 |
| IGHJ | IGHJ4-d | 0.00135 | 0.14% | 3 | 3 |
| IGHV | IGHV1-18-d | 0.00135 | 0.14% | 3 | 3 |
| IGHV | IGHV1-18-e | 0.00135 | 0.14% | 3 | 3 |
| IGHV | IGHV1-2-h | 0.00135 | 0.14% | 3 | 2 |
| IGHV | IGHV1-2-f | 0.00135 | 0.14% | 3 | 3 |
| IGHV | IGHV1-3-aa | 0.00135 | 0.14% | 3 | 1 |
| IGHV | IGHV1-3-ak | 0.00135 | 0.14% | 3 | 3 |
| IGHV | IGHV1-3-w | 0.00135 | 0.14% | 3 | 1 |
| IGHV | IGHV1-3-at | 0.00135 | 0.14% | 3 | 2 |
| IGHV | IGHV1-69-k | 0.00135 | 0.14% | 3 | 1 |
| IGHV | IGHV1-69-ac | 0.00135 | 0.14% | 3 | 3 |
| IGHV | IGHV1-8-e | 0.00135 | 0.14% | 3 | 2 |
| IGHV | IGHV1-8-f | 0.00135 | 0.14% | 3 | 2 |
| IGHV | IGHV2-70-y | 0.00135 | 0.14% | 3 | 3 |
| IGHV | IGHV2-70-z | 0.00135 | 0.14% | 3 | 3 |
| IGHV | IGHV2-70-af | 0.00135 | 0.14% | 3 | 2 |
| IGHV | IGHV2-70-h | 0.00135 | 0.14% | 3 | 3 |
| IGHV | IGHV3-13-g | 0.00135 | 0.14% | 3 | 2 |
| IGHV | IGHV3-16-e | 0.00135 | 0.14% | 3 | 2 |
| IGHV | IGHV3-20-f | 0.00135 | 0.14% | 3 | 3 |
| IGHV | IGHV3-30-d | 0.00135 | 0.14% | 3 | 2 |
| IGHV | IGHV3-30-c | 0.00135 | 0.14% | 3 | 3 |
| IGHV | IGHV3-33-h | 0.00135 | 0.14% | 3 | 3 |
| IGHV | IGHV3-43-k | 0.00135 | 0.14% | 3 | 2 |
| IGHV | IGHV3-43-m | 0.00135 | 0.14% | 3 | 3 |
| IGHV | IGHV3-53-n | 0.00135 | 0.14% | 3 | 2 |
| IGHV | IGHV3-53-m | 0.00135 | 0.14% | 3 | 3 |
| IGHV | IGHV3-64-f | 0.00135 | 0.14% | 3 | 1 |
| IGHV | IGHV3-73-c | 0.00135 | 0.14% | 3 | 3 |
| IGHV | IGHV3-9-h | 0.00135 | 0.14% | 2 | 1 |
| IGHV | IGHV4-31-h | 0.00135 | 0.14% | 3 | 2 |
| IGHV | IGHV4-39-u | 0.00135 | 0.14% | 3 | 3 |
| IGHV | IGHV4-39-t | 0.00135 | 0.14% | 3 | 2 |
| IGHV | IGHV4-39-q | 0.00135 | 0.14% | 3 | 3 |
| IGHV | IGHV4-4-ao | 0.00135 | 0.14% | 3 | 3 |
| IGHV | IGHV4-4-ac | 0.00135 | 0.14% | 3 | 1 |
| IGHV | IGHV4-4-ae | 0.00135 | 0.14% | 3 | 2 |
| IGHV | IGHV5-51-g | 0.00135 | 0.14% | 3 | 3 |
| IGHV | IGHV7-81-e | 0.00135 | 0.14% | 3 | 1 |
| IGKJ | IGKJ2-f | 0.00135 | 0.14% | 3 | 2 |
| IGKJ | IGKJ3-d | 0.00135 | 0.14% | 3 | 3 |
| IGKJ | IGKJ4-g | 0.00135 | 0.14% | 3 | 3 |
| IGKJ | IGKJ5-c | 0.00135 | 0.14% | 3 | 3 |
| IGKJ | IGKJ5-b | 0.00135 | 0.14% | 3 | 3 |
| IGKV | IGKV1-5-c | 0.00135 | 0.14% | 3 | 3 |
| IGKV | IGKV1-9-j | 0.00135 | 0.14% | 3 | 3 |
| IGKV | IGKV1D-17-c | 0.00135 | 0.14% | 3 | 2 |
| IGKV | IGKV1D-17-b | 0.00135 | 0.14% | 3 | 2 |
| IGKV | IGKV1D-42-e | 0.00135 | 0.14% | 3 | 1 |
| IGKV | IGKV1D-42-d | 0.00135 | 0.14% | 3 | 2 |
| IGKV | IGKV2D-24-b | 0.00135 | 0.14% | 3 | 2 |
| IGKV | IGKV2D-30-c | 0.00135 | 0.14% | 3 | 2 |
| IGKV | IGKV3-20-d | 0.00135 | 0.14% | 3 | 3 |
| IGKV | IGKV3-20-f | 0.00135 | 0.14% | 3 | 2 |
| IGKV | IGKV3D-11-d | 0.00135 | 0.14% | 3 | 2 |
| IGKV | IGKV4-1-r | 0.00135 | 0.14% | 3 | 3 |
| IGKV | IGKV4-1-x | 0.00135 | 0.14% | 3 | 2 |
| IGKV | IGKV4-1-e | 0.00135 | 0.14% | 3 | 3 |
| IGKV | IGKV4-1-o | 0.00135 | 0.14% | 3 | 2 |
| IGU | IGLI6-c | 0.00135 | 0.14% | 3 | 2 |
| IGU | IGU7-b | 0.00135 | 0.14% | 3 | 3 |
| IGLV | IGLV10-54-g | 0.00135 | 0.14% | 3 | 1 |
| IGLV | IGLV11-55-h | 0.00135 | 0.14% | 3 | 2 |
| IGLV | IGLV11-55-f | 0.00135 | 0.14% | 3 | 2 |
| IGLV | IGLV1-40-g | 0.00135 | 0.14% | 3 | 3 |
| IGLV | IGLV1-44-f | 0.00135 | 0.14% | 3 | 2 |
| IGLV | IGLV1-47-f | 0.00135 | 0.14% | 3 | 2 |
| IGLV | IGLV2-23-e | 0.00135 | 0.14% | 3 | 3 |
| IGLV | IGLV3-12-d | 0.00135 | 0.14% | 3 | 3 |
| IGLV | IGLV3-16-d | 0.00135 | 0.14% | 3 | 3 |
| IGLV | IGLV3-19-d | 0.00135 | 0.14% | 3 | 1 |
| IGLV | IGLV3-19-1 | 0.00135 | 0.14% | 3 | 2 |
| IGLV | IGLV3-1-ag | 0.00135 | 0.14% | 3 | 3 |
| IGLV | IGLV3-1-y | 0.00135 | 0.14% | 3 | 3 |
| IGLV | IGLV3-1-s | 0.00135 | 0.14% | 3 | 3 |
| IGLV | IGLV3-1-x | 0.00135 | 0.14% | 3 | 3 |
| IGLV | IGLV3-1-ad | 0.00135 | 0.14% | 3 | 2 |
| IGLV | IGLV3-1-1 | 0.00135 | 0.14% | 3 | 3 |
| IGLV | IGLV3-1-p | 0.00135 | 0.14% | 3 | 2 |
| IGLV | IGLV3-21-t | 0.00135 | 0.14% | 3 | 2 |
| IGLV | IGLV3-22-f | 0.00135 | 0.14% | 3 | 3 |
| IGLV | IGLV3-22-i | 0.00135 | 0.14% | 3 | 1 |
| IGLV | IGLV3-25-e | 0.00135 | 0.14% | 3 | 3 |
| IGLV | IGLV5-48-t | 0.00135 | 0.14% | 3 | 2 |
| IGLV | IGLV5-48-s | 0.00135 | 0.14% | 3 | 3 |
| IGLV | IGLV5-52-d | 0.00135 | 0.14% | 3 | 2 |
| IGLV | IGLV5-52-c | 0.00135 | 0.14% | 3 | 1 |
| IGLV | IGLV7-43-c | 0.00135 | 0.14% | 3 | 2 |
| IGHJ | IGHJ5-d | 0.0009 | 0.09% | 2 | 2 |
| IGHJ | IGHJ6-e | 0.0009 | 0.09% | 2 | 2 |
| IGHJ | IGHJ6-h | 0.0009 | 0.09% | 2 | 2 |
| IGHJ | IGHJ6-f | 0.0009 | 0.09% | 2 | 2 |
| IGHV | IGHV1-18-f | 0.0009 | 0.09% | 2 | 2 |
| IGHV | IGHV1-24-g | 0.0009 | 0.09% | 2 | 1 |
| IGHV | IGHV1-2-i | 0.0009 | 0.09% | 2 | 2 |
| IGHV | IGHV1-2-m | 0.0009 | 0.09% | 2 | 2 |
| IGHV | IGHV1-2-l | 0.0009 | 0.09% | 2 | 2 |
| IGHV | IGHV1-3-ag | 0.0009 | 0.09% | 2 | 2 |
| IGHV | IGHV1-3-aj | 0.0009 | 0.09% | 2 | 2 |
| IGHV | IGHV1-3-aq | 0.0009 | 0.09% | 2 | 1 |
| IGHV | IGHV1-3-s | 0.0009 | 0.09% | 2 | 2 |
| IGHV | IGHV1-3-q | 0.0009 | 0.09% | 2 | 1 |
| IGHV | IGHV1-3-z | 0.0009 | 0.09% | 2 | 1 |
| IGHV | IGHV1-3-ad | 0.0009 | 0.09% | 2 | 2 |
| IGHV | IGHV1-3-aw | 0.0009 | 0.09% | 2 | 2 |
| IGHV | IGHV1-3-ae | 0.0009 | 0.09% | 2 | 2 |
| IGHV | IGHV1-3-an | 0.0009 | 0.09% | 2 | 2 |
| IGHV | IGHV1-46-k | 0.0009 | 0.09% | 2 | 2 |
| IGHV | IGHV1-46-e | 0.0009 | 0.09% | 2 | 2 |
| IGHV | IGHV1-46-n | 0.0009 | 0.09% | 2 | 2 |
| IGHV | IGHV1-58-g | 0.0009 | 0.09% | 2 | 1 |
| IGHV | IGHV1-69-ah | 0.0009 | 0.09% | 2 | 2 |
| IGHV | IGHV1-69-t | 0.0009 | 0.09% | 2 | 2 |
| IGHV | IGHV1-69-ao | 0.0009 | 0.09% | 2 | 1 |
| IGHV | IGHV1-69-af | 0.0009 | 0.09% | 2 | 2 |
| IGHV | IGHV1-69-ad | 0.0009 | 0.09% | 2 | 2 |
| IGHV | IGHV1-69-n | 0.0009 | 0.09% | 2 | 2 |
| IGHV | IGHV1-69-an | 0.0009 | 0.09% | 2 | 2 |
| IGHV | IGHV1-69-o | 0.0009 | 0.09% | 2 | 2 |
| IGHV | IGHV1-8-h | 0.0009 | 0.09% | 2 | 2 |
| IGHV | IGHV2-26-f | 0.0009 | 0.09% | 2 | 2 |
| IGHV | IGHV2-5-c | 0.0009 | 0.09% | 2 | 2 |
| IGHV | IGHV2-70-g | 0.0009 | 0.09% | 2 | 2 |
| IGHV | IGHV2-70-s | 0.0009 | 0.09 % | 2 | 2 |
| IGHV | IGHV2-70-ab | 0.0009 | 0.09% | 2 | 2 |
| IGHV | IGHV3-11-b | 0.0009 | 0.09% | 2 | 2 |
| IGHV | IGHV3-13-f | 0.0009 | 0.09% | 2 | 2 |
| IGHV | IGHV3-20-d | 0.0009 | 0.09% | 2 | 2 |
| IGHV | IGHV3-21-c | 0.0009 | 0.09% | 2 | 2 |
| IGHV | IGHV3-21-h | 0.0009 | 0.09% | 2 | 2 |
| IGHV | IGHV3-23-j | 0.0009 | 0.09% | 2 | 1 |
| IGHV | IGHV3-23-h | 0.0009 | 0.09% | 2 | 2 |
| IGHV | IGHV3-33-f | 0.0009 | 0.09% | 2 | 2 |
| IGHV | IGHV3-43-p | 0.0009 | 0.09% | 2 | 2 |
| IGHV | IGHV3-64-h | 0.0009 | 0.09% | 2 | 1 |
| IGHV | IGHV3-66-h | 0.0009 | 0.09% | 2 | 2 |
| IGHV | IGHV3-74-c | 0.0009 | 0.09% | 2 | 2 |
| IGHV | IGHV3-9-o | 0.0009 | 0.09% | 2 | 1 |
| IGHV | IGHV4-28-s | 0.0009 | 0.09% | 2 | 2 |
| IGHV | IGHV4-28-n | 0.0009 | 0.09% | 2 | 2 |
| IGHV | IGHV4-31-t | 0.0009 | 0.09% | 2 | 2 |
| IGHV | IGHV4-31-m | 0.0009 | 0.09% | 2 | 2 |
| IGHV | IGHV4-31-l | 0.0009 | 0.09% | 2 | 2 |
| IGHV | IGHV4-34-d | 0.0009 | 0.09% | 2 | 2 |
| IGHV | IGHV4-34-g | 0.0009 | 0.09% | 2 | 2 |
| IGHV | IGHV4-34-b | 0.0009 | 0.09% | 2 | 2 |
| IGHV | IGHV4-39-aa | 0.0009 | 0.09% | 2 | 2 |
| IGHV | IGHV4-39-s | 0.0009 | 0.09% | 2 | 2 |
| IGHV | IGHV4-39-x | 0.0009 | 0.09% | 2 | 2 |
| IGHV | IGHV4-39-p | 0.0009 | 0.09% | 2 | 2 |
| IGHV | IGHV4-4-aa | 0.0009 | 0.09% | 2 | 2 |
| IGHV | IGHV4-4-af | 0.0009 | 0.09% | 2 | 2 |
| IGHV | IGHV4-4-x | 0.0009 | 0.09% | 2 | 2 |
| IGHV | IGHV4-4-ad | 0.0009 | 0.09% | 2 | 2 |
| IGHV | IGHV4-4-ai | 0.0009 | 0.09% | 2 | 1 |
| IGHV | IGHV5-51-d | 0.0009 | 0.09% | 2 | 2 |
| IGHV | IGHV5-51-k | 0.0009 | 0.09% | 2 | 2 |
| IGHV | IGHV5-51-c | 0.0009 | 0.09% | 2 | 2 |
| IGHV | IGHV6-1-a | 0.0009 | 0.09% | 2 | 2 |
| IGHV | IGHV6-1-c | 0.0009 | 0.09% | 2 | 1 |
| IGHV | IGHV7-81-d | 0.0009 | 0.09% | 2 | 2 |
| IGKJ | IGKJ2-g | 0.0009 | 0.09% | 2 | 2 |
| IGKJ | IGKJ3-b | 0.0009 | 0.09% | 2 | 2 |
| IGKJ | IGKJ4-j | 0.0009 | 0.09% | 2 | 1 |
| IGKJ | IGKJ4-c | 0.0009 | 0.09% | 2 | 2 |
| IGKJ | IGKJ4-k | 0.0009 | 0.09% | 2 | 2 |
| IGKJ | IGKJ4-h | 0.0009 | 0.09% | 2 | 2 |
| IGKV | IGKV1-16-d | 0.0009 | 0.09% | 2 | 2 |
| IGKV | IGKV1-17-e | 0.0009 | 0.09% | 2 | 2 |
| IGKV | IGKV1-5-d | 0.0009 | 0.09% | 2 | 1 |
| IGKV | IGKV1-8-h | 0.0009 | 0.09% | 2 | 2 |
| IGKV | IGKV1-9-i | 0.0009 | 0.09% | 2 | 2 |
| IGKV | IGKV1D-16-c | 0.0009 | 0.09% | 2 | 1 |
| IGKV | IGKV1D-16-b | 0.0009 | 0.09% | 2 | 1 |
| IGKV | IGKV1D-42-g | 0.0009 | 0.09% | 2 | 2 |
| IGKV | IGKV1D-42-f | 0.0009 | 0.09% | 2 | 1 |
| IGKV | IGKV1D-43-e | 0.0009 | 0.09% | 2 | 1 |
| IGKV | IGKV1D-43-d | 0.0009 | 0.09% | 2 | 2 |
| IGKV | IGKV1D-8-d | 0.0009 | 0.09% | 2 | 1 |
| IGKV | IGKV1D-8-f | 0.0009 | 0.09% | 2 | 2 |
| IGKV | IGKV2D-26-d | 0.0009 | 0.09% | 2 | 2 |
| IGKV | IGKV2D-26-f | 0.0009 | 0.09% | 2 | 2 |
| IGKV | IGKV2D-29-b | 0.0009 | 0.09% | 2 | 1 |
| IGKV | IGKV3-11-g | 0.0009 | 0.09% | 2 | 1 |
| IGKV | IGKV3-11-f | 0.0009 | 0.09% | 2 | 2 |
| IGKV | IGKV3-20-j | 0.0009 | 0.09% | 2 | 2 |
| IGKV | IGKV3-20-i | 0.0009 | 0.09% | 2 | 2 |
| IGKV | IGKV3-20-e | 0.0009 | 0.09% | 2 | 2 |
| IGKV | IGKV3-20-c | 0.0009 | 0.09% | 2 | 2 |
| IGKV | IGKV3D-11-h | 0.0009 | 0.09% | 2 | 1 |
| IGKV | IGKV3D-11-i | 0.0009 | 0.09% | 2 | 1 |
| IGKV | IGKV3D-20-d | 0.0009 | 0.09% | 2 | 2 |
| IGKV | IGKV4-1-y | 0.0009 | 0.09% | 2 | 2 |
| IGKV | IGKV4-1-g | 0.0009 | 0.09% | 2 | 2 |
| IGKV | IGKV4-1-t | 0.0009 | 0.09% | 2 | 2 |
| IGKV | IGKV4-1-i | 0.0009 | 0.09% | 2 | 2 |
| IGKV | IGKV4-1-n | 0.0009 | 0.09% | 2 | 2 |
| IGKV | IGKV4-1-l | 0.0009 | 0.09% | 2 | 2 |
| IGU | IGU2-e | 0.0009 | 0.09% | 2 | 2 |
| IGU | IGU2-c | 0.0009 | 0.09% | 2 | 2 |
| IGU | IGU5-b | 0.0009 | 0.09% | 2 | 1 |
| IGLV | IGLV10-54-n | 0.0009 | 0.09% | 2 | 2 |
| IGLV | IGLV10-54-j | 0.0009 | 0.09% | 2 | 2 |
| IGLV | IGLV1-40-h | 0.0009 | 0.09% | 2 | 2 |
| IGLV | IGLV1-40-e | 0.0009 | 0.09% | 2 | 2 |
| IGLV | IGLV1-47-e | 0.0009 | 0.09% | 2 | 2 |
| IGLV | IGLV1-51-g | 0.0009 | 0.09% | 2 | 2 |
| IGLV | IGLV2-11-j | 0.0009 | 0.09% | 2 | 2 |
| IGLV | IGLV2-11-f | 0.0009 | 0.09% | 2 | 2 |
| IGLV | IGLV2-11-i | 0.0009 | 0.09% | 2 | 2 |
| IGLV | IGLV2-11-e | 0.0009 | 0.09% | 2 | 1 |
| IGLV | IGLV2-14-p | 0.0009 | 0.09% | 2 | 2 |
| IGLV | IGLV2-14-q | 0.0009 | 0.09% | 2 | 1 |
| IGLV | IGLV2-14-o | 0.0009 | 0.09% | 2 | 1 |
| IGLV | IGLV2-23-k | 0.0009 | 0.09% | 2 | 2 |
| IGLV | IGLV2-23-o | 0.0009 | 0.09% | 2 | 2 |
| IGLV | IGLV2-33-c | 0.0009 | 0.09% | 2 | 1 |
| IGLV | IGLV3-19-j | 0.0009 | 0.09% | 2 | 2 |
| IGLV | IGLV3-19-k | 0.0009 | 0.09% | 2 | 2 |
| IGLV | IGLV3-19-h | 0.0009 | 0.09% | 2 | 1 |
| IGLV | IGLV3-19-i | 0.0009 | 0.09% | 2 | 1 |
| IGLV | IGLV3-19-p | 0.0009 | 0.09% | 2 | 2 |
| IGLV | IGLV3-1-ah | 0.0009 | 0.09% | 2 | 2 |
| IGLV | IGLV3-1-d | 0.0009 | 0.09% | 2 | 2 |
| IGLV | IGLV3-1-j | 0.0009 | 0.09% | 2 | 1 |
| IGLV | IGLV3-1-g | 0.0009 | 0.09% | 2 | 2 |
| IGLV | IGLV3-1-z | 0.0009 | 0.09% | 2 | 2 |
| IGLV | IGLV3-1-ac | 0.0009 | 0.09% | 2 | 2 |
| IGLV | IGLV3-1-ae | 0.0009 | 0.09% | 2 | 2 |
| IGLV | IGLV3-21-k | 0.0009 | 0.09% | 2 | 2 |
| IGLV | IGLV3-21-r | 0.0009 | 0.09% | 2 | 1 |
| IGLV | IGLV3-21-i | 0.0009 | 0.09% | 2 | 2 |
| IGLV | IGLV3-22-j | 0.0009 | 0.09% | 2 | 1 |
| IGLV | IGLV3-22-n | 0.0009 | 0.09% | 2 | 2 |
| IGLV | IGLV3-22-m | 0.0009 | 0.09% | 2 | 2 |
| IGLV | IGLV3-22-o | 0.0009 | 0.09% | 2 | 2 |
| IGLV | IGLV3-25-g | 0.0009 | 0.09 % | 2 | 1 |
| IGLV | IGLV3-25-l | 0.0009 | 0.09 % | 2 | 2 |
| IGLV | IGLV3-27-a | 0.0009 | 0.09% | 2 | 2 |
| IGLV | IGLV3-32-c | 0.0009 | 0.09% | 2 | 2 |
| IGLV | IGLV3-9-a | 0.0009 | 0.09% | 2 | 2 |
| IGLV | IGLV3-9-b | 0.0009 | 0.09% | 2 | 2 |
| IGLV | IGLV4-3-d | 0.0009 | 0.09% | 2 | 2 |
| IGLV | IGLV4-60-g | 0.0009 | 0.09% | 2 | 2 |
| IGLV | IGLV4-60-n | 0.0009 | 0.09% | 2 | 2 |
| IGLV | IGLV4-60-o | 0.0009 | 0.09% | 2 | 2 |
| IGLV | IGLV5-45-g | 0.0009 | 0.09% | 2 | 2 |
| IGLV | IGLV5-48-u | 0.0009 | 0.09% | 2 | 2 |
| IGLV | IGLV5-48-q | 0.0009 | 0.09% | 2 | 2 |
| IGLV | IGLV5-48-r | 0.0009 | 0.09% | 2 | 2 |
| IGLV | IGLV5-48-n | 0.0009 | 0.09% | 2 | 2 |
| IGLV | IGLV5-48-m | 0.0009 | 0.09% | 2 | 1 |
| IGLV | IGLV5-48-1 | 0.0009 | 0.09% | 2 | 2 |
| IGLV | IGLV5-52-b | 0.0009 | 0.09% | 2 | 1 |
| IGLV | IGLV7-46-c | 0.0009 | 0.09% | 2 | 2 |
| IGLV | IGLV9-49-c | 0.0009 | 0.09% | 2 | 2 |

**Table 20B: Variants Found In No More Than 3 Human Populations & In At Least 10 Individuals**

| **TYPE** | **VARIANT** | **C.FREQ** | **C.FREQ(%)** | **#INDIVIDUALS** | **#POPULATIONS** |
|---|---|---|---|---|---|
| IGKV | IGKV1D-8-b | 0.01785 | 1.79% | 36 | 3 |
| IGHV | IGHV3-11-a | 0.01695 | 1.70% | 36 | 3 |
| IGHV | IGHV5-51-a | 0.0164 | 1.64% | 35 | 3 |
| IGHV | IGHV2-5-b | 0.01055 | 1.06% | 18 | 3 |
| IGLV | IGLV2-14-g | 0.0104 | 1.04% | 22 | 3 |
| IGHV | IGHV7-81-b | 0.01005 | 1.01% | 21 | 3 |
| IGKV | IGKV1D-42-b | 0.01005 | 1.01% | 21 | 3 |
| IGKV | IGKV3-7-b | 0.01 | 1.00% | 19 | 3 |
| IGHV | IGHV1-69-d | 0.00995 | 1.00% | 22 | 3 |
| IGLV | IGLV2-14-e | 0.00995 | 1.00% | 19 | 3 |
| IGLV | IGLV9-49-a | 0.00865 | 0.87% | 18 | 3 |
| IGLV | IGLV1-44-b | 0.00775 | 0.78% | 16 | 2 |
| IGLV | IGLV2-11-a | 0.00775 | 0.78% | 17 | 3 |
| IGKV | IGKV3-11-d | 0.0073 | 0.73% | 14 | 3 |
| IGKV | IGKV1D-43-b | 0.00685 | 0.69% | 14 | 3 |
| IGHV | IGHV3-38-d | 0.0064 | 0.64% | 13 | 3 |
| IGU | IGU6-a | 0.00595 | 0.60% | 13 | 3 |
| IGHV | IGHV5-51-b | 0.0055 | 0.55% | 11 | 3 |
| IGLV | IGLV5-45-c | 0.00545 | 0.55% | 12 | 2 |
| IGHV | IGHV4-31-i | 0.0054 | 0.54% | 10 | 3 |
| IGLV | IGLV1-36-c | 0.00455 | 0.46% | 10 | 2 |

**Table 20C: Variants Found In No More Than 3 Human Populations & In At Least 20 Individuals**

| **TYPE** | **VARIANT** | **C.FREQ** | **C.FREQ (%)** | **#INDIVIDUALS** | **#POPULATIONS** |
|---|---|---|---|---|---|
| IGKV | IGKV1D-8-b | 0.01785 | 1.79% | 36 | 3 |
| IGHV | IGHV3-11-a | 0.01695 | 1.70% | 36 | 3 |
| IGHV | IGHV5-51-a | 0.0164 | 1.64% | 35 | 3 |
| IGLV | IGLV2-14-g | 0.0104 | 1.04% | 22 | 3 |
| IGHV | IGHV7-81-b | 0.01005 | 1.01% | 21 | 3 |
| IGKV | IGKV1D-42-b | 0.01005 | 1.01% | 21 | 3 |
| IGHV | IGHV1-69-d | 0.00995 | 1.00% | 22 | 3 |

**Table 20D: Variants Found In No More Than 3 Human Populations & In At Least 30 Individuals**

| **TYPE** | **VARIANT** | **C.FREQ** | **C.FREQ (%)** | **#INDIVIDUALS** | **#POPULATIONS** |
|---|---|---|---|---|---|
| IGKV | IGKV1D-8-b | 0.01785 | 1.79% | 36 | 3 |
| IGHV | IGHV3-11-a | 0.01695 | 1.70% | 36 | 3 |
| IGHV | IGHV5-51-a | 0.0164 | 1.64% | 35 | 3 |

**Table 21**

| **Seq ID#** | **Associated Gene Name** | **Ensembl Gene ID** | **Gene Location [Chromosome Name:Gene Chr Start (bp)-GeneChr End (bp)]** | **Ensembl Transcript ID** | **Exon Positions [Chromosome Name:Exon Chr Start (bp)-Exon Chr End (bp)]** |
|---|---|---|---|---|---|
| 239 | IGHJ1 | ENSG00000211905 | 14:106331617-106331668 | ENST00000390565 | 14:106331617-106331668 |
| 240 | IGHJ2 | ENSG00000211904 | 14:106331409-106331460 | ENST00000390564 | 14:106331409-106331460 |
| 241 | IGHJ3 | ENSG00000242887 | 14:106330797-106330845 | ENST00000463911 | 14:106330797-106330845 |
| 242 | IGHJ4 | ENSG00000240041 | 14:106330425-106330470 | ENST00000461719 | 14:106330425-106330470 |
| 243 | IGHJ5 | ENSG00000242472 | 14:106330024-106330072 | ENST00000488476 | 14:106330024-106330072 |
| 244 | IGHJ6 | ENSG00000211900 | 14:106329408-106329468 | ENST00000390560 | 14:106329408-106329468 |
| 245 | IGHV1-18 | ENSG00000211945 | 14:106641563-106642056 | ENST00000390605 | 14:106641952-106642056 14:106641563-106641867 |
| 246 | IGHV1-2 | ENSG00000211934 | 14:106452671-106453170 | ENST00000390594 | 14:106453061-106453170 14:106452671-106452975 |
| 247 | IGHV1-24 | ENSG00000211950 | 14:106733144-106733639 | ENST00000390610 | 14:106733534-106733639 14:106733144-106733448 |
| 248 | IGHV1-3 | ENSG00000211935 | 14:106471246-106471723 | ENST00000390595 | 14:106471636-106471723 14:106471246-106471550 |
| 249 | IGHV1-45 | ENSG00000211961 | 14:106962931-106963424 | ENST00000390621 | 14:106963321-106963424 14:106962931-106963235 |
| 250 | IGHV1-46 | ENSG00000211962 | 14:106967049-106967788 | ENST00000390622 | 14:106967439-106967788 14:106967049-106967353 |
| 251 | IGHV1-58 | ENSG00000211968 | 14:107078373-107078869 | ENST00000390628 | 14:107078763-107078869 14:107078373-107078677 |
| 252 | IGHV1-69 | ENSG00000211973 | 14:107169931-107170428 | ENST00000390633 | 14:107170322-107170428 14:107169931-107170235 |
| 253 | IGHV1-8 | ENSG00000211939 | 14:106539079-106539577 | ENST00000390599 | 14:106539470-106539577 14:106539079-106539383 |
| 254 | IGHV2-26 | ENSG00000211951 | 14:106757650-106758116 | ENST00000390611 | 14:106758047-106758116 14:106757650-106757960 |
| 255 | IGHV2-5 | ENSG00000211937 | 14:106494135-106494597 | ENST00000390597 | 14:106494532-106494597 14:106494135-106494445 |
| 256 | IGHV2-70 | ENSG00000211974 | 14:107178820-107179338 | ENST00000390634 | 14:107179217-10717933814:107178820-107179130 |
| 257 | IGHV3-11 | ENSG00000211941 | 14:106573233-106573800 | ENST00000390601 | 14:106573635-106573800 14:106573233-106573537 |
| 258 | IGHV3-13 | ENSG00000211942 | 14:106586137-106586667 | ENST00000390602 | 14:106586542-106586667 14:106586137-106586438 |
| 259 | IGHV3-15 | ENSG00000211943 | 14:106610313-106610852 | ENST00000390603 | 14:106610727-106610852 14:106610313-106610623 |
| 260 | IGHV3-16 | ENSG00000211944 | 14:106621894-106622419 | ENST00000390604 | 14:106622300-106622419 14:106621894-106622198 |
| 261 | IGHV3-20 | ENSG00000211946 | 14:106667581-106668095 | ENST00000390606 | 14:106667988-106668095 14:106667581-106667885 |
| 262 | IGHV3-21 | ENSG00000211947 | 14:106691673-106692203 | ENST00000390607 | 14:106692079-106692203 14:106691673-106691977 |
| 263 | IGHV3-23 | ENSG00000211949 | 14:106725201-106725733 | ENST00000390609 | 14:106725609-106725733 14:106725201-106725505 |
| 264 | IGHV3-30 | ENSG00000211953 | 14:106791005-106791536 | ENST00000390613 | 14:106791411-106791536 14:106791005-106791309 |
| 265 | IGHV3-33 | ENSG00000211955 | 14:106815722-106816253 | ENST00000390615 | 14:106816128-106816253 14:106815722-106816026 |
| 266 | IGHV3-35 | ENSG00000211957 | 14:106845323-106845789 | ENST00000390617 | 14:106845729-10684578914:106845323-106845627 |
| 267 | IGHV3-38 | ENSG00000211958 | 14:106866406-106866934 | ENST00000390618 | 14:106866811-106866934 14:106866406-106866707 |
| 268 | IGHV3-43 | ENSG00000232216 | 14:106926188-106926724 | ENST00000434710 | 14:106926599-106926724 14:106926188-106926495 |
| 269 | IGHV3-48 | ENSG00000211964 | 14:106993814-106994346 | ENST00000390624 | 14:106994222-106994346 14:106993814-106994118 |
| 270 | IGHV3-49 | ENSG00000211965 | 14:107012938-107013477 | ENST00000390625 | 14:107013352-107013477 14:107012938-107013248 |
| 271 | IGHV3-53 | ENSG00000211967 | 14:107048672-107049341 | ENST00000390627 | 14:107049075-10704934114:107048672-107048973 |
| 272 | IGHV3-64 | ENSG00000223648 | 14:107113741-107114274 | ENST00000454421 | 14:107114149-107114274 14:107113741-107114045 |
| 273 | IGHV3-66 | ENSG00000211972 | 14:107131033-107131560 | ENST00000390632 | 14:107131436-107131560 14:107131033-107131334 |
| 274 | IGHV3-7 | ENSG00000211938 | 14:106518400-106518932 | ENST00000390598 | 14:106518808-106518932 14:106518400-106518704 |
| 275 | IGHV3-72 | ENSG00000225698 | 14:107198932-107199471 | ENST00000433072 | 14:107199346-10719947114:107198932-107199242 |
| 276 | IGHV3-73 | ENSG00000211976 | 14:107210932-107211471 | ENST00000390636 | 14:107211346-10721147114:107210932-107211242 |
| 277 | IGHV3-74 | ENSG00000224650 | 14:107218676-107219365 | ENST00000424969 | 14:107219084-107219365 14:107218676-107218980 |
| 278 | IGHV3-9 | ENSG00000211940 | 14:106552285-106552809 | ENST00000390600 | 14:106552684-106552809 14:106552285-106552592 |
| 279 | IGHV4-28 | ENSG00000211952 | 14:106780513-106781017 | ENST00000390612 | 14:106780900-106781017 14:106780513-106780817 |
| 280 | IGHV4-31 | ENSG00000231475 | 14:106805209-106805716 | ENST00000438142 | 14:106805599-10680571614:106805209-106805516 |
| 281 | IGHV4-34 | ENSG00000211956 | 14:106829594-106830076 | ENST00000390616 | 14:106829979-106830076 14:106829594-106829895 |
| 282 | IGHV4-39 | ENSG00000211959 | 14:106877619-106878126 | ENST00000390619 | 14:106878010-106878126 14:106877619-106877926 |
| 283 | IGHV4-4 | ENSG00000211936 | 14:106478110-106478603 | ENST00000390596 | 14:106478494-106478603 14:106478110-106478411 |
| 284 | IGHV4-59 | ENSG00000224373 | 14:107081806-107083830 | ENST00000390629 | 14:107083640-107083830 14:107083256-107083557 |
| 284 | IGHV4-59 | ENSG00000224373 | 14:107081806-107083830 | ENST00000455737 | 14:107083640-107083725 14:107083240-107083557 |
| | | | | | 14:107081806-107082728 |
| 285 | IGHV4-61 | ENSG00000211970 | 14:107095126-107095662 | ENST00000390630 | 14:107095516-107095662 14:107095126-107095433 |
| 286 | IGHV5-51 | ENSG00000211966 | 14:107034729-107035221 | ENST00000390626 | 14:107035117-107035221 14:107034729-107035033 |
| 287 | IGHV6-1 | ENSG00000211933 | 14:106405611-106406108 | ENST00000390593 | 14:106406008-106406108 14:106405611-106405924 |
| 288 | IGHV7-81 | ENSG00000211979 | 14:107282792-107283280 | ENST00000390639 | 14:107283181-107283280 14:107282792-107283096 |
| 289 | IGKJ2 | ENSG00000211596 | 2:89161037-89161074 | ENST00000390241 | 2:89161037-89161074 |
| 290 | IGKJ3 | ENSG00000211595 | 2:89160733-89160770 | ENST00000390240 | 2:89160733-89160770 |
| 291 | IGKJ4 | ENSG00000211594 | 2:89160398-89160434 | ENST00000390239 | 2:89160398-89160434 |
| 292 | IGKJ5 | ENSG00000211593 | 2:89160080-89160117 | ENST00000390238 | 2:89160080-89160117 |
| 293 | IGKV1-16 | ENSG00000240864 | 2:89399352-89399854 | ENST00000479981 | 2:89399773-89399854 2:89399352-89399647 |
| 294 | IGKV1-17 | ENSG00000240382 | 2:89416833-89417335 | ENST00000490686 | 2:89417254-89417335 2:89416833-89417128 |
| 295 | IGKV1-5 | ENSG00000243466 | 2:89246819-89247475 | ENST00000496168 | 2:89247240-89247475 2:89246819-89247114 |
| 296 | IGKV1-6 | ENSG00000239855 | 2:89265781-89266286 | ENST00000464162 | 2:89266203-89266286 2:89265781-89266076 |
| 297 | IGKV1-8 | ENSG00000240671 | 2:89291928-89292450 | ENST00000495489 | 2:89292349-89292450 2:89291928-89292223 |
| 298 | IGKV1-9 | ENSG00000241755 | 2:89309479-89310012 | ENST00000493819 | 2:89309900-89310012 2:89309479-89309774 |
| 299 | IGKV1D-12 | ENSG00000240834 | 2:90198535-90199190 | ENST00000390276 | 2:90198535-90198770 2:90198895-90199190 |
| 300 | IGKV1D-16 | ENSG00000241244 | 2:90139078-90139580 | ENST00000492446 | 2:90139078-90139159 2:90139285-90139580 |
| 301 | IGKV1D-17 | ENSG00000242766 | 2:90121477-90122133 | ENST00000483379 | 2:90121477-90121712 2:90121838-90122133 |
| 302 | IGKV1D-42 | ENSG00000211633 | 2:90229045-90229531 | ENST00000390278 | 2:90229045-90229119 2:90229236-90229531 |
| 303 | IGKV1D-43 | ENSG00000242580 | 2:90248739-90249395 | ENST00000468879 | 2:90248739-90248974 2:90249100-90249395 |
| 304 | IGKV1D-8 | ENSG00000239819 | 2:90259593-90260248 | ENST00000471857 | 2:90259593-90259828 2:90259953-90260248 |
| 305 | IGKV2-24 | ENSG00000241294 | 2:89475812-89476644 | ENST00000484817 | 2:89476566-89476644 2:89475812-89476122 |
| 306 | IGKV2-30 | ENSG00000243238 | 2:89544264-89545079 | ENST00000468494 | 2:89545001-89545079 2:89544264-89544574 |
| 307 | IGKV2D-24 | ENSG00000241566 | 2:90043607-90044439 | ENST00000462693 | 2:90043607-90043685 2:90044129-90044439 |
| 308 | IGKV2D-26 | ENSG00000211623 | 2:90024732-90025512 | ENST00000390268 | 2:90024732-90024810 2:90025202-90025512 |
| 309 | IGKV2D-29 | ENSG00000243264 | 2:89986322-89987079 | ENST00000491977 | 2:89986322-89986400 2:89986769-89987079 |
| 310 | IGKV2D-30 | ENSG00000239571 | 2:89975669-89976489 | ENST00000474213 | 2:89975669-89975752 2:89976179-89976489 |
| 311 | IGKV3-11 | ENSG00000241351 | 2:89326668-89327228 | ENST00000483158 | 2:89327133-89327228 2:89326668-89326963 |
| 312 | IGKV3-20 | ENSG00000239951 | 2:89442057-89442643 | ENST00000492167 | 2:89442543-89442643 2:89442057-89442355 |
| 313 | IGKV3-7 | ENSG00000243063 | 2:89277987-89278600 | ENST00000390247 | 2:89278455-89278600 2:89277987-89278285 |
| 314 | IGKV3D-11 | ENSG00000211632 | 2:90211643-90212253 | ENST00000390277 | 2:90211643-90211788 2:90211958-90212253 |
| 315 | IGKV3D-15 | ENSG00000224041 | 2:90153696-90154258 | ENST00000417279 | 2:90153696-90153793 2:90153963-90154258 |
| 316 | IGKV3D-20 | ENSG00000211625 | 2:90077680-90078311 | ENST00000390270 | 2:90077680-90077825 2:90078013-90078311 |
| 317 | IGKV4-1 | ENSG00000211598 | 2:89184913-89185669 | ENST00000390243 | 2:89184913-89185136 2:89185356-89185669 |
| 318 | IGKV5-2 | ENSG00000211599 | 2:89196748-89197300 | ENST00000390244 | 2:89196748-89196859 2:89197005-89197300 |
| 319 | IGKV6-21 | ENSG00000211611 | 2:89459235-89459850 | ENST00000390256 | 2:89459741-89459850 2:89459235-89459530 |
| 320 | IGKV6D-21 | ENSG00000225523 | 2:90060377-90060995 | ENST00000436451 | 2:90060377-90060489 2:90060700-90060995 |
| 321 | IGKV6D-41 | ENSG00000211626 | 2:90108504-90109080 | ENST00000390271 | 2:90108504-90108578 2:90108785-90109080 |
| 322 | IGLI1 | ENSG00000211674 | 22:23235872-23235998 | ENST00000390320 | 22:23235872-23235998 |
| 323 | IGU2 | ENSG00000211676 | 22:23241661-23241835 | ENST00000390322 | 22:23241661-23241835 |
| 324 | IGU3 | ENSG00000211678 | 22:23247030-23247205 | ENST00000390324 | 22:23247030-23247205 |
| 325 | IGLI5 | ENSG00000211681 | 22:23256408-23256479 | ENST00000390327 | 22:23256408-23256479 |
| 326 | IGU6 | ENSG00000211682 | 22:23260304-23260373 | ENST00000390328 | 22:23260304-23260373 |
| 327 | IGU7 | ENSG00000211684 | 22:23263562-23263607 | ENST00000390330 | 22:23263562-23263607 |
| 328 | IGLV10-54 | ENSG00000211642 | 22:22569184-22569660 | ENST00000390287 | 22:22569184-22569242 22:22569355-22569660 |
| 329 | IGLV11-55 | ENSG00000211641 | 22:22556057-22556600 | ENST00000390286 | 22:22556057-22556114 22:22556233-22556600 |
| 330 | IGLV1-36 | ENSG00000211655 | 22:22786296-22786802 | ENST00000390301 | 22:22786296-22786381 22:22786497-22786802 |
| 331 | IGLV1-40 | ENSG00000211653 | 22:22764098-22764614 | ENST00000390299 | 22:22764098-22764194 22:22764305-22764614 |
| 332 | IGLV1-44 | ENSG00000211651 | 22:22735135-22735715 | ENST00000390297 | 22:22735135-22735294 22:22735410-22735715 |
| 333 | IGLV1-47 | ENSG00000211648 | 22:22712087-22712608 | ENST00000390294 | 22:22712087-22712188 22:22712304-22712608 |
| 334 | IGLV1-50 | ENSG00000211645 | 22:22681658-22682172 | ENST00000390291 | 22:22681658-22681754 22:22681865-22682172 |
| 335 | IGLV1-51 | ENSG00000211644 | 22:22676828-22677336 | ENST00000390290 | 22:22676828-22676909 22:22677019-22677336 |
| 336 | IGLV2-11 | ENSG00000211668 | 22:23134980-23135496 | ENST00000390314 | 22:23134980-23135067 22:23135185-23135496 |
| 337 | IGLV2-14 | ENSG00000211666 | 22:23101189-23101707 | ENST00000390312 | 22:23101189-23101275 22:23101393-23101707 |
| 338 | IGLV2-18 | ENSG00000211664 | 22:23077095-23077584 | ENST00000390310 | 22:23077095-23077153 22:23077270-23077584 |
| 339 | IGLV2-23 | ENSG00000211660 | 22:23040274-23040892 | ENST00000390306 | 22:23040274-2304048122:23040599-23040892 |
| 340 | IGLV2-33 | ENSG00000211656 | 22:22930626-22931145 | ENST00000390302 | 22:22930626-22930729 22:22930852-22931145 |
| 341 | IGLV2-8 | ENSG00000211671 | 22:23165153-23165787 | ENST00000390317 | 22:23165153-23165360 22:23165476-23165787 |
| 342 | IGLV3-1 | ENSG00000211673 | 22:23222886-23223576 | ENST00000390319 | 22:23222886-23222983 22:23223277-23223576 |
| 343 | IGLV3-10 | ENSG00000211669 | 22:23154244-23154782 | ENST00000390315 | 22:23154244-23154324 22:23154478-23154782 |
| 344 | IGLV3-12 | ENSG00000211667 | 22:23114317-23115079 | ENST00000390313 | 22:23114317-2311437122:23114775-23115079 |
| 345 | IGLV3-16 | ENSG00000211665 | 22:23089870-23090398 | ENST00000390311 | 22:23089870-23089953 22:23090108-23090398 |
| 346 | IGLV3-19 | ENSG00000211663 | 22:23063108-23063630 | ENST00000390309 | 22:23063108-23063193 22:23063340-23063630 |
| 347 | IGLV3-21 | ENSG00000211662 | 22:23054174-23055688 | ENST00000390308 | 22:23054174-23054290 22:23054770-23054949 22:23055384-23055688 |
| 348 | IGLV3-22 | ENSG00000211661 | 22:23046750-23047307 | ENST00000390307 | 22:23046750-23046859 22:23047009-23047307 |
| 349 | IGLV3-25 | ENSG00000211659 | 22:23029190-23029735 | ENST00000390305 | 22:23029190-23029278 22:23029445-23029735 |
| 350 | IGLV3-27 | ENSG00000211658 | 22:23010758-23011276 | ENST00000390304 | 22:23010758-23010841 22:23010984-23011276 |
| 351 | IGLV3-32 | ENSG00000211657 | 22:22936998-22937501 | ENST00000390303 | 22:22936998-22937079 22:22937226-22937501 |
| 352 | IGLV3-9 | ENSG00000211670 | 22:23161507-23162253 | ENST00000390316 | 22:23161507-23161667 22:23161954-23162253 |
| 353 | IGLV4-3 | ENSG00000211672 | 22:23213686-23214214 | ENST00000390318 | 22:23213686-23213763 22:23213890-23214214 |
| 354 | IGLV4-60 | ENSG00000211639 | 22:22516592-22517074 | ENST00000390284 | 22:22516592-22516643 22:22516765-22517074 |
| 355 | IGLV4-69 | ENSG00000211637 | 22:22385332-22385870 | ENST00000390282 | 22:22385332-22385440 22:22385561-22385870 |
| 356 | IGLV5-37 | ENSG00000211654 | 22:22781876-22782371 | ENST00000390300 | 22:22781876-22781926 22:22782049-22782371 |
| 357 | IGLV5-45 | ENSG00000211650 | 22:22730355-22730874 | ENST00000390296 | 22:22730355-22730428 22:22730552-22730874 |
| 358 | IGLV5-48 | ENSG00000211647 | 22:22707289-22707781 | ENST00000390293 | 22:22707289-22707334 22:22707459-22707781 |
| 359 | IGLV5-52 | ENSG00000211643 | 22:22673082-22673581 | ENST00000390289 | 22:22673082-22673132 22:22673254-22673581 |
| 360 | IGLV6-57 | ENSG00000211640 | 22:22550113-22550686 | ENST00000390285 | 22:22550113-22550244 22:22550370-22550686 |
| 361 | IGLV7-43 | ENSG00000211652 | 22:22749356-22749827 | ENST00000390298 | 22:22749356-22749435 22:22749523-22749827 |
| 362 | IGLV7-46 | ENSG00000211649 | 22:22723982-22724454 | ENST00000390295 | 22:22723982-2272406122:22724151-22724454 |
| 363 | IGLV8-61 | ENSG00000211638 | 22:22453110-22453622 | ENST00000390283 | 22:22453110-22453216 22:22453316-22453622 |
| 364 | IGLV9-49 | ENSG00000223350 | 22:22697539-22698084 | ENST00000427632 | 22:22697539-2269762122:22697759-22698084 |

### References:

1. Nat Biotechnol. 2005 Sep;23(9):1117-25; Human antibodies from transgenic animals; Lonberg N.
2. J Clin Invest. 1992 Mar;89(3):729-38; Immunoglobulin light chain variable region gene sequences for human antibodies to Haemophilus influenzae type b capsular polysaccharide are dominated by a limited number of V kappa and V lambda segments and VJ combinations; Adderson EE, Shackelford PG, Insel RA, Quinn A, Wilson PM, Carroll WL.
3. J Immunol. 1993 Oct 15;151(8):4352-61; Clonal characterization of the human IgG antibody repertoire to Haemophilus influenzae type b polysaccharide. V. In vivo expression of individual antibody clones is dependent on Ig CH haplotypes and the categories of antigen; Chung GH, Scott MG, Kim KH, Kearney J, Siber GR, Ambrosino DM, Nahm MH.
4. J Immunol. 1998 Dec 1;161(11):6068-73; Decreased frequency of rearrangement due to the synergistic effect of nucleotide changes in the heptamer and nonamer of the recombination signal sequence of the V kappa gene A2b, which is associated with increased susceptibility of Navajos to Haemophilus influenzae type b disease; Nadel B, Tang A, Lugo G, Love V, Escuro G, Feeney AJ.
5. J Clin Invest. 1996 May 15;97(10):2277-82; A defective Vkappa A2 allele in Navajos which may play a role in increased susceptibility to haemophilus influenzae type b disease; Feeney AJ, Atkinson MJ, Cowan MJ, Escuro G, Lugc G.
6. Infect Immun. 1994 Sep;62(9):3873-80; Variable region sequences of a protective human monoclonal antibody specific for the Haemophilus influenzae type b capsular polysaccharide; Lucas AH, Larrick JW, Reason DC.
7. J Clin Invest. 1993 Jun;91(6):2734-43; Restricted immunoglobulin VH usage and VDJ combinations in the human response to Haemophilus influenzae type b capsular polysaccharide. Nucleotide sequences of monospecific anti-Haemophilus antibodies and polyspecific antibodies cross-reacting with self antigens; Adderson EE, Shackelford PG, Quinn A, Wilson PM, Cunningham MW, Insel RA, Carroll WL.
8. J Clin Invest. 1993 Mar;91(3):788-96; Variable region expression in the antibody responses of infants vaccinated with Haemophilus influenzae type b polysaccharide-protein conjugates. Description of a new lambda light chain-associated idiotype and the relation between idiotype expression, avidity, and vaccine formulation. The Collaborative Vaccine Study Group; Granoff DM, Shackelford PG, Holmes SJ, Lucas AH.
9. Infect Immun. 1994 May;62(5):1776-86; Variable region sequences and idiotypic expression of a protective human immunoglobulin M antibody to capsular polysaccharides of Neisseria meningitidis group B and Escherichia coli K1; Azmi FH, Lucas AH, Raff HV, Granoff DM.
10. J Clin Invest. 1992 Dec;90(6):2197-208; Sequence analyses of three immunoglobulin G antivirus antibodies reveal their utilization of autoantibody-related immunoglobulin Vh genes, but not V lambda genes; Huang DF, Olee T, Masuho Y, Matsumoto Y, Carson DA, Chen PP.
11. Science. 2011 Aug 12;333(6044):834-5, Biochemistry. Catching a moving target, Wang TT, Palese P
12. Science. 2009 Apr 10;324(5924):246-51. Epub 2009 Feb 26; Antibody recognition of a highly conserved influenza virus epitope; Ekiert DC, Bhabha G, Elsliger MA, Friesen RH, Jongeneelen M, Throsby M, Goudsmit J, Wilson IA.
13. PLoS One. 2008;3(12):e3942. Epub 2008 Dec 16; Heterosubtypic neutralizing monoclonal antibodies cross-protective against H5N1 and H1N1 recovered from human IgM+ memory B cells; Throsby M, van den Brink E, Jongeneelen M, Poon LL, Alard P, Cornelissen L, Bakker A, Cox F, van Deventer E, Guan Y, Cinatl J, ter Meulen J, Lasters I, Carsetti R, Peiris M, de Kruif J, Goudsmit J.
14. Nat Struct Mol Biol. 2009 Mar;16(3):265-73. Epub 2009 Feb 22,Structural and functional bases for broad-spectrum neutralization of avian and human influenza A viruses, Sui J, Hwang WC, Perez S, Wei G, Aird D, Chen LM, Santelli E, Stec B, Cadwell G, Ali M, Wan H, Murakami A, Yammanuru A, Han T, Cox NJ, Bankston LA, Donis RO, Liddington RC, Marasco WA.
15. Science. 2011 Aug 12;333(6044):843-50. Epub 2011 Jul 7, A highly conserved neutralizing epitope on group 2 influenza A viruses, Ekiert DC, Friesen RH, Bhabha G, Kwaks T, Jongeneelen M, Yu W, Ophorst C, Cox F, Korse HJ, Brandenburg B, Vogels R, Brakenhoff JP, Kompier R, Koldijk MH, Cornelissen LA, Poon LL, Peiris M, Koudstaal W, Wilson IA, Goudsmit J.

## Claims

1. A method for producing a heavy chain, VH domain or an antibody specific to a target antigen, wherein the heavy chain, VH domain or antibody comprises an HCDR3 at least 20 amino acids in length, the method comprising
providing a non human vertebrate, optionally a mouse or a rat, that has a genome comprising an immunoglobulin heavy chain locus comprising unrearranged human gene segment JH6*02, one or more VH gene segments and one or more D gene segments upstream of a constant region; wherein the gene segments in the heavy chain locus are operably linked to the constant region thereof so that the vertebrate is capable of producing an antibody heavy chain produced by recombination of the human JH6*02 with a human D segment and a human VH segment,
wherein the JH6*02 gene segment comprises the following nucleotide sequence: ATTACTA CTACTACTAC GGTATGGACG TCTGGGGCCA AGGGACCACG GTCACCGTCT CCTCAG, and
wherein the vertebrate has been immunised with the target antigen and produces an antibody heavy chain specific for the target antigen wherein the variable domain of the heavy chain is the product of recombination between a human VH, D and JH6*02 and wherein the HCDR3 length is at least 20 amino acids, and
isolating from the non-human vertebrate the heavy chain, VH domain or an antibody specific to the target antigen or a cell producing the heavy chain, VH domain or antibody, wherein the heavy chain, VH domain or antibody comprises a HCDR3 that is derived from the recombination of human JH6*02 with a human VH gene segment and a human D gene segment,
optionally wherein the constant region of the locus is a non-human vertebrate (e.g., mouse or rat) constant region, and the non-human constant region of the isolated heavy chain or antibody is replaced with a human constant region.

2. The method of claim 1 comprising isolating a heavy chain, VH domain or an antibody, wherein the HCDR3 length is at least 20 amino acids.

3. The method of claim 1 or 2, comprising isolating a B-cell or hybridoma expressing a heavy chain VH domain that is identical to the VH domain of the heavy chain of claim 2.

4. The method of any one of claims 1-3, wherein the constant region is mouse or rat.

5. The method of claim 1, wherein the locus comprises one, more or all human D gene segments D3-9; D4-17; D3-10; D2-2; D5-24; D6-19; D3-22; D6-13; D5-12; D1- 26; D1-20; D5-18; D3-16; D2-21; D1-14; D7-27; D1-1; D6-25; D2-14; and D4-23, optionally wherein the locus comprises one, more or all human D gene segments D3-9, D3-10, D6-19, D4-17, D6-13, D3-22, D2-2, D2-25 and D3-3.

6. The method of claim 1 or 5, wherein i) the JH6*02 is the only JH6-type gene segment in the genome or ii) the JH6*02 is the closest JH gene segment to the constant region in the locus.

7. The method of claim 1, 5 or 6, wherein the locus comprises a plurality of human D gene segments and the JH6*02 is in human germline configuration with respect to the 3'-most human D gene segment.

8. The method of claim 1, 5, 6 or 7, wherein the locus comprises one, more or all of human gene segments selected from V3-21, V3-13, V3-7, V6-1, V1-8, V1-2, V7-4-1, V1-3, V1- 18, V4-4, V3-9, V3-23, V3-11, V3-20, D3-9*01, D3-10*01, D6-19*01, D6-13*01, D1-26*01, IGHV1-8*01, IGHV4-61*01, IGHV6-1*01, IGHV4-4*02, IGHV1-3*01, IGHV3-66*03, IGHV3-7*01 and IGHV3-9*01.

9. The method of any one of claims 1 and 5 to 8, wherein the antibody heavy chain is a product of the recombination of JH6*02 with a human VH gene segment recited in claim 8 and/or a D gene segment recited in claim 5.

10. The method of any of claims 1 and 5 to 9, wherein all endogenous non-human vertebrate heavy chain variable region gene segments have been inactivated in the genome and/or wherein the genome is homozygous for said heavy chain locus.

11. The method of claim 1, wherein the vertebrate is a mouse comprising functional heavy gene segments VH2-5, VH7-4-1, VH4-4, VHI-3, VHI-2, VH6-1, DI-1, D2-2, D3-9, D3-10, D4-11, D5-12, D6-13, DI-14, D2-15, D3-16, D4-17, D5-18, D6-19, DI-20, D2-21, D3-22, D4-23, D5-24, D6-25, DI-26, D7-27, JHI, JH2, JH3, JH4, JH5 and JH6, in 5' to 3' order,
wherein the JH6 is the human JH6*02 variant,
wherein the insertion of human DNA is made between positions 114666435 and 114666436 on mouse chromosome 12 ;
wherein human functional heavy chain gene segments VH3-13, VH3-11, VH3-9, VH1-8, VH3-7 are inserted upstream of VH2-5;
wherein the mouse VH, D and JH gene segments are retained in the locus, immediately upstream of (5' of) the inserted human heavy chain DNA;
and wherein the mouse VH, D and J segments are inverted to inactivate them, thereby producing mice in which only the human heavy chain variable region gene segments are active.

12. The method of claim 11 additionally comprising VH2-26, V H 1- 24, VH 3 - 23, VH 3 - 21, VH3-20, VH1- 18, and VH 3 - 15 inserted upstream (5') of the 5'-most VH.

13. The method of any preceding claim, wherein
(i) the target antigen is an antigen of an infectious disease pathogen;
(ii) the target antigen is a receptor, and the antibody heavy chain specifically binds a receptor cleft; or
(iii) the target antigen is an enzyme, and the antibody heavy chain specifically binds the enzyme active site.

14. The method of claim 13, wherein the target antigen is a bacterial or viral pathogen antigen.

15. The method of any preceding claim, comprising removing B lymphocytes from the vertebrate and selecting one or more B lymphocytes expressing antibodies that bind to the antigen, optionally immortalising said selected B lymphocytes or progeny thereof, optionally by producing hybridomas therefrom, and isolating an antibody expressed by the B lymphocytes.

16. The method of claim 15, further comprising isolating from said B lymphocytes nucleic acid encoding said antibody.

17. The method of any preceding claim, wherein the constant region of the locus is a non-human vertebrate (e.g., mouse or rat) constant region, and the method further comprises replacing the non-human constant region of the isolated heavy chain or antibody with a human constant region.

18. The method of claim 16, further comprising exchanging the heavy chain constant region nucleotide sequence of the antibody with a nucleotide sequence encoding a human or humanised heavy chain constant region, and optionally affinity maturing the variable region of said antibody.

19. The method of claim 16 or claim 18, further comprising inserting said nucleic acid into an expression vector and optionally a host cell.

20. The method of claim 19, comprising expressing the heavy chain, VH domain or antibody from the host cell and providing an isolated heavy chain, VH domain or antibody.

21. Use of human JH6*02 for generating a HCDR3 of at least 20 amino acids in a non-human vertebrate whose genome comprises an immunoglobulin heavy chain locus comprising human gene segment JH6*02, one or more human VH gene segments and one or more human D gene segments upstream of a constant region; wherein the gene segments in the heavy chain locus are operably linked to the constant region thereof so that the vertebrate is capable of producing an antibody heavy chain produced by recombination of the human JH6*02 with a human D segment and a human VH segment and wherein the JH6*02 gene segment comprises the following nucleotide sequence: ATTACTA CTACTACTAC GGTATGGACG TCTGGGGCCA AGGGACCACG GTCACCGTCT CCTCAG.

22. The use according to claim 21 wherein the antibody heavy chain specifically binds a target antigen.

23. The use of claim 21, wherein the vertebrate is immunised with a target antigen.

24. The use of claim 21, wherein the vertebrate comprises a mouse Eµ and Sµ in the heavy chain locus between the JH6*02 and the constant region.

25. The use of claim 21 wherein the Eµ and Sµ are Eµ and Sµ of a mouse 129-derived genome or of a mouse C57BL/6-derived genome.

## Patentansprüche

1. Verfahren zur Herstellung einer schweren Kette, VH-Domäne oder eines zu einem Target-Antigen spezifischen Antikörpers, wobei die schwere Kette, VH-Domäne oder Antikörper eine mindestens 20 Aminosäuren lange HCDR3 umfasst, wobei das Verfahren umfasst:
Bereitstellen eines nicht-humanen Wirbeltiers, gegebenenfalls eine Maus oder eine Ratte, welches ein Genom hat, das einen Locus einer schweren Kette eines Immoglobulins umfasst, umfassend ein nicht-rearrangiertes humanes Gensegment JH6*02, ein oder mehrere VH-Gensegmente und ein oder mehrere D-Gensegmente stromaufwärts einer konstanten Region, wobei die Gensegmente in dem Locus der schweren Kette funktionsfähig mit der konstanten Region derselben verknüpft sind, so dass das Wirbeltier fähig ist, eine schwere Kette eines Antikörpers durch Rekombination des humanen JH6*02 mit einem humanen D-Segment und einem humanen VH-Segment zu produzieren,
wobei das JH6*02 Gensegment die folgenden Nukleotidsequenzen umfasst: ATTACTA CTACTACTAC GGTATGGACG TCTGGGGCCA AGGGACCACG GTCACCGTCT CCTCAG, und
wobei das Wirbeltier mit dem Target-Antigen immunisiert worden ist und eine für das Target-Antigen spezifische schwere Kette eines Antikörpers produziert, wobei die variable Domäne der schweren Kette das Produkt aus einer Rekombination zwischen einer humanen VH, D und JH6*02 ist und wobei die Länge der HCDR3 mindestens 20 Aminosäuren ist, und
Isolieren der schweren Kette, VH-Domäne oder eines zu dem Target-Antigen spezifischen Antikörpers oder einer Zelle, die die schwere Kette, VH-Domäne oder Antikörper produziert, aus dem nicht humanen Wirbeltier, wobei die schwere Kette, VH-Domäne oder Antikörper eine HCDR3 umfasst, das abgeleitet ist aus der Rekombination von humanem JH6*02 mit einem humanen VH-Gensegment und einem humanen D-Gensegment,
wobei gegebenenfalls die konstante Region des Locus eine konstante Region eines nicht-humanen Wirbeltiers (z.B. Maus oder Ratte) ist und die nicht-humane konstante Region der isolierten schweren Kette oder Antikörpers durch eine humane konstante Region ersetzt ist.

2. Verfahren nach Anspruch 1, umfassend Isolieren einer schweren Kette, VH-Domäne oder eines Antikörpers, wobei die Länge der HCDR3 mindestens 20 Aminosäuren ist.

3. Verfahren nach Anspruch 1 oder 2, umfassend Isolieren einer B-Zelle oder Hybridomen, die ein VH-Domäne einer schweren Kette exprimieren, welche identisch ist zu der VH-Domäne der schweren Kette aus Anspruch 2.

4. Verfahren nach einem der Ansprüche 1 - 3, wobei die konstante Region Maus oder Ratte ist.

5. Verfahren nach Anspruch 1, wobei der Locus ein, mehrere oder alle humane D-Gensegmente D3-9; D4-17; D3-10; D2-2; D5-24; D6-19; D3-22; D6-13; D5-12; D1-26; D1-20; D5-18; D3-16; D2-21; D1-14; D7-27; D1-1; D6-25; D2-14; und D4-23 umfasst, wobei gegebenenfalls der Locus ein, mehrere oder alle D-Gensegmente D3-9, D3-10, D6-19, D4-17, D6-13, D3-22, D2-2, D2-25 und D3-3 umfasst.

6. Verfahren nach Anspruch 1 oder 5, wobei i) das JH6*02 das einzige Gensegment des JH6-Typs in dem Genom ist oder ii) wobei das JH6*02 das zu der konstanten Region am nächsten gelegene JH-Gensegment in dem Locus ist.

7. Verfahren nach Anspruch 1, 5 oder 6, wobei der Locus eine Mehrzahl der humanen D-Gensegmente umfasst und das JH6*02 in der humanen Keimbahnkonfiguration ist in Bezug auf das am ehesten humane 3' D-Gensegment.

8. Verfahren nach Anspruch 1, 5, 6 oder 7, wobei der Locus ein, mehrere oder alle der humanen Gensegmente, ausgewählt aus V3-21, V3-13, V3-7, V6-1, V1-8, V1-2, V7-4-1, V1-3, V1-18, V4-4, V3-9, V3-23, V3-11, V3-20, D3-9*01, D3-10*01, D6-19*01, D6-13*01, D1-26*01, IGHV1-8*01, IGHV4-61*01, IGHV6-1*01, IGHV4-4*02, IGHV1-3*01, IGHV3-66*03, IGHV3-7*01 und IGVH3-9*01, umfasst.

9. Verfahren nach einem der Ansprüche 1 und 5 bis 8, wobei die schwere Kette des Antikörpers ein Produkt ist aus der Rekombination von JH6*02 mit einem humanen VH-Gensegment, wie zitiert in Anspruch 8, und/oder einem D-Gensegment, wie zitiert in Anspruch 5.

10. Verfahren nach einem der Ansprüche 1 und 5 bis 9, wobei alle endogenen Gensegmente der variablen Region der nicht-humanen schweren Kette eines Wirbeltiers in dem Genom inaktiviert worden sind und/oder wobei das Genom homozygot für den Locus der schweren Kette ist.

11. Verfahren nach Anspruch 1, wobei das Wirbeltier eine Maus ist, umfassen die funktionalen schweren Gensegmente VH2-5, VH7-4-1, VH4-4, VHI-3, VHI-2, VH6-1, DI-1, D2-2, D3-9, D3-10, D4-11, D5-12, D6-13, DI-14, D2-15, D3-16, D4-17, D5-18, D6-19, DI-20, D2-21, D3-22, D4-23, D5-24, D6-25, DI-26, D7-27, JHI, JH2, JH3, JH4, JH5 und JH6, in der 5' zu 3' Reihenfolge,
wobei das JH6 die humane Variante des JH6*02 ist,
wobei die Einfügung der humanen DNA zwischen den Positionen 114666435 und 114666436 am Mauschromosom 12 durchgeführt wird;
wobei funktionale Gensegmente VH3-13, VH3-11, VH3-9, VH1-8, VH3-7 der schweren Kette stromaufwärts von VH2-5 eingefügt werden;
wobei die Maus VH, D und JH-Gensegmente in dem Locus unmittelbar stromaufwärts der (5' der) eingefügten humanen DNA der schweren Kette erhalten bleiben,
und wobei die Maus VH, D und J-Segmente invertiert sind, um diese zu inaktivieren, wodurch Mäuse produziert werden, in welchen nur die Gensegmente der variablen Region der humanen schweren Kette aktiv sind.

12. Verfahren nach Anspruch 11, zusätzlich umfassend VH2-26, V H 1 - 24, VH 3 - 23, VH 3 - 21, VH3-20, VH1 - 18 und VH 3 - 15, die stromaufwärts (5') der 5'-ehesten VH eingefügt sind.

13. Verfahren nach einem der vorherigen Ansprüche, wobei
(i) das Target-Antigen ein Antigen eines infektiösen Krankheitserregers ist;
(ii) das Target-Antigen ein Rezeptor ist und wobei die schwere Kette des Antikörpers spezifisch einen Rezeptorspalt bindet; oder
(iii) das Target-Antigen ein Enzym ist und die schwere Kette des Antikörpers spezifisch das aktive Zentrum des Enzyms bindet.

14. Verfahren nach Anspruch 13, wobei das Target-Antigen ein bakterielles oder virales Erreger-Antigen ist.

15. Verfahren nach einem der vorherigen Ansprüche, umfassend Entfernen von B Lymphozyten aus dem Wirbeltier und Auswählen eines oder mehrerer B Lymphozyten, die Antikörper, welche an das Antigen binden, exprimieren, gegebenenfalls Immortalisieren der ausgewählten B Lymphozyten oder Nachkommen derselben, gegebenenfalls durch Herstellen von Hybridomen davon und Isolieren eines Antikörpers, der durch die B Lymphozyten exprimiert wird.

16. Verfahren nach Anspruch 15, ferner umfassend Isolieren aus der Nukleinsäure der B Lymphozyten, die den Antikörper kodiert.

17. Verfahren nach einem der vorherigen Ansprüche, wobei die konstante Region des Locus eine konstante Region eines nicht-humanen Wirbeltiers (z.B. Maus oder Ratte) ist und wobei das Verfahren ferner das Ersetzen der nicht-humanen konstanten Region der isolierten schweren Kette oder des Antikörpers mit einer humanen konstanten Region umfasst.

18. Verfahren nach Anspruch 16, ferner umfassend Austauschen der Nukleotidsequenz der konstanten Region der schweren Kette des Antikörpers mit einer Nukleotidsequenz, die eine konstante Region einer humanen oder humanisierten schweren Kette kodiert und gegebenenfalls Affinitätsreifung der variablen Region des Antikörpers.

19. Verfahren nach Anspruch 16 oder Anspruch 18, ferner umfassend Einfügen der Nukleinsäure in einen Expressionsvektor und gegebenenfalls eine Wirtszelle.

20. Verfahren nach Anspruch 19, umfassen Exprimieren der schweren Kette, VH-Domäne oder des Antikörpers aus der Wirtszelle und Bereitstellen einer isolierten schweren Kette, VH-Domäne oder Antikörper.

21. Verwendung eines humanen JH6*02 zur Generierung einer HCDR3 von mindestens 20 Aminosäuren Länge in einem nicht-humanen Wirbeltier, wessen Genom einen Locus einer schweren Kette eines Immunoglobulins umfasst, umfassend ein humanes Gensegment JH6*02, ein oder mehrere humane VH-Gensegmente und ein oder mehrere humane D-Gensegmente stromaufwärts einer konstanten Region; wobei die Gensegmente in dem Locus der schweren Kette funktionsfähig verknüpft sind mit der konstanten Region derselben, so dass das Wirbeltier fähig ist, eine schwere Kette eines Antikörpers durch Rekombination des humanen JH6*02 mit einem humanen D-Segment und einem humanen VH-Segment zu produzieren und wobei das JH6*02 Gensegment die folgende Nukleotidsequenz umfasst: ATTACTA CTACTACTAC GGTATGGACG TCTGGGGCCA AGGGACCACG GTCACCGTCT CCTCAG.

22. Verwendung nach Anspruch 21, wobei die schwere Kette des Antikörpers spezifisch ein Target-Antigen bindet.

23. Verwendung nach Anspruch 21, wobei das Wirbeltier mit einem Target-Antigen immunisiert ist.

24. Verwendung nach Anspruch 21, wobei das Wirbeltier ein Maus Eµ und Sµ in dem Locus der schweren Kette zwischen dem JH6*02 und der konstanten Region umfasst.

25. Verwendung nach Anspruch 21, wobei die Eµ und Sµ die Eµ und Sµ eines Maus 129-abgeleiteten Genoms oder eines Maus C57BL/6-abgeleiteten Genoms sind.

## Revendications

1. Procédé de production d'une chaîne lourde, d'un domaine VH ou d'un anticorps spécifique d'un antigène cible, dans lequel la chaîne lourde, le domaine VH ou l'anticorps comprend une HCDR3 d'une longueur d'au moins 20 acides aminés, le procédé comprenant
la fourniture d'un vertébré non humain, éventuellement une souris ou un rat, qui a un génome comprenant un emplacement de chaîne lourde d'immunoglobuline comprenant un segment de gène humain non réarrangé JH6*02, un ou plusieurs segments de gène VH et un ou plusieurs segments de gène D en amont d'une région constante ; dans lequel les segments de gène dans l'emplacement de chaîne lourde sont liés de façon opérationnelle à la région constante de ceux-ci de sorte que le vertébré soit capable de produire une chaîne lourde d'anticorps produite par recombinaison du JH6*02 humain avec un segment D humain et un segment VH humain,
dans lequel le segment de gène JH6*02 comprend la séquence de nucléotides suivante : dans lequel le vertébré a été immunisé avec l'antigène cible et produit une chaîne lourde d'anticorps spécifique de l'antigène cible dans lequel le domaine variable de la chaîne lourde est le produit de recombinaison entre un VH, D et JH6*02 humains et dans lequel la longueur de la HCDR3 est d'au moins 20 acides aminés, et
l'isolement du vertébré non humain de la chaîne lourde, du domaine VH ou d'un anticorps spécifique de l'antigène cible ou d'une cellule produisant la chaîne lourde, le domaine VH ou l'anticorps, dans lequel la chaîne lourde, le domaine VH ou l'anticorps comprend une HCDR3 qui est dérivée de la recombinaison du JH6*02 humain avec un segment de gène VH humain et un segment de gène D humain,
éventuellement dans lequel la région constante de l'emplacement est une région constante de vertébré non humaine (par exemple, une souris ou un rat), et la région constante non humaine de la chaîne lourde ou de l'anticorps isolé est remplacée par une région constante humaine.

2. Procédé selon la revendication 1 comprenant l'isolement d'une chaîne lourde, d'un domaine VH ou d'un anticorps, dans lequel la longueur de la HCDR3 est d'au moins 20 acides aminés.

3. Procédé selon la revendication 1 ou 2, comprenant l'isolement d'une cellule B ou d'un hybridome exprimant un domaine VH de chaîne lourde qui est identique au domaine VH de la chaîne lourde de la revendication 2.

4. Procédé selon l'une quelconque des revendications 1 à 3, dans lequel la région constante est une souris ou un rat.

5. Procédé selon la revendication 1, dans lequel l'emplacement comprend un, plusieurs ou tous les segments de gène D humains D3-9 ; D4-17 ; D3-10 ; D2-2 ; D5-24 ; D6-19 ; D3-22 ; D6-13 ; D5-12 ; D1-26; D1-20; D5-18 ; D3-16 ; D2-21 ; D1-14 ; D7-27 ; D1-1 ; D6-25 ; D2-14 ; et D4-23, éventuellement dans lequel l'emplacement comprend un, plusieurs ou tous les segments de gène D humains D3-9, D3-10, D6-19, D4-17, D6-13, D3-22, D2-2, D2-25 et D3-3.

6. Procédé selon la revendication 1 ou 5, dans lequel i) le JH6*02 est le seul segment de gène de type JH6 dans le génome ou ii) le JH6*02 est le segment de gène JH le plus proche de la région constante dans l'emplacement.

7. Procédé selon la revendication 1, 5 ou 6, dans lequel l'emplacement comprend une pluralité de segments de gène D humains et le JH6*02 est selon une configuration de lignée germinale humaine par rapport au segment de gène D humain le plus 3'.

8. Procédé selon la revendication 1, 5, 6 ou 7, dans lequel l'emplacement comprend un, plusieurs ou tous les segments de gène humains choisis parmi V3-21, V3-13, V3-7, V6-1, V1-8, V1-2, V7-4-1, V1-3, V1-18, V4-4, V3-9, V3-23, V3-11, V3-20, D3-9*01, D3-10*01, D6-19*01, D6-13*01, D1-26*01, IGHVI1-8*01, IGHV4-61*01, IGHV6-1*01, IGHV4-4*02, IGHV1-3*01, IGHV3-66*03, IGHV3-7*01 et IGHV3-9*01.

9. Procédé selon l'une quelconque des revendications 1 et 5 à 8, dans lequel la chaîne lourde de l'anticorps est un produit de la recombinaison de JH6*02 avec un segment de gène VH humain selon la revendication 8 et/ou un segment de gène D selon la revendication 5.

10. Procédé selon l'une quelconque des revendications 1 et 5 à 9, dans lequel tous les segments de gène de la région variable de la chaîne lourde du vertébré non humains ont été inactivés dans le génome et/ou dans lequel le génome est homozygote pour ledit emplacement de la chaîne lourde.

11. Procédé selon la revendication 1, dans lequel le vertébré est une souris comprenant des segments de gène lourds fonctionnels VH2-5, VH7-4-1, VH4-4, VHI-3, VHI-2, VH6-1, DI-1, D2-2, D3-9, D3-10, D4-11, D5-12, D6-13, DI-14, D2-15, D3-16, D4-17, D5-18, D6-19, DI-20, D2-21, D3-22, D4-23, D5-24, D6-25, DI-26, D7-27, JHI, JH2, JH3, JH4, JH5 et JH6, dans l'ordre 5' à 3'.
dans lequel le JH6 est la variante JH6*02 humaine,
dans lequel l'insertion d'ADN humain est réalisée entre les positions 114666435 et 114666436 dans le chromosome 12 de souris ;
dans lequel les segments de gène de chaîne lourde fonctionnels humains VH3-13, VH3-11, VH3-9, VH1-8, VH3-7 sont insérés en amont de VH2-5 ;
dans lequel les segments de gène VH, D et JH de souris sont maintenus dans l'emplacement, immédiatement en amont de (5' de) l'ADN de chaîne lourde humain inséré ;
et dans lequel les segments VH, D et J de souris sont inversés pour les inactiver, afin de produire des souris dans lesquels uniquement les segments de gène de la région variable de la chaîne lourde humains sont actifs.

12. Procédé selon la revendication 11 comprenant de plus VH2-26, VH1-24, VH3-23, VH3-21, VH3-20, VH1-18 et VH3-15 insérés en amont (5') du VH le plus en 5'.

13. Procédé selon une quelconque revendication précédente, dans lequel
(i) l'antigène cible est un antigène d'un pathogène d'une maladie infectieuse ;
(ii) l'antigène cible est un récepteur, et la chaîne lourde de l'anticorps se lie spécifiquement à une fente du récepteur ; ou
(iii) l'antigène cible est une enzyme, et la chaîne lourde de l'anticorps se lie spécifiquement au site actif de l'enzyme.

14. Procédé selon la revendication 13, dans lequel l'antigène cible est un antigène pathogène bactérien ou viral.

15. Procédé selon une quelconque revendication précédente, comprenant l'élimination des lymphocytes B du vertébré et la sélection d'un ou de plusieurs lymphocytes B exprimant des anticorps qui se lient à l'antigène, éventuellement l'immortalisation desdits lymphocytes B choisis ou de la progéniture de ceux-ci, éventuellement par production d'hybridomes à partir de ceux-ci, et l'isolement d'un anticorps exprimé par les lymphocytes B.

16. Procédé selon la revendication 15, comprenant en outre l'isolement desdits lymphocytes B d'un acide nucléique codant pour ledit anticorps.

17. Procédé selon une quelconque revendication précédente, dans lequel la région constante de l'emplacement est une région constante de vertébré non humaine (par exemple, une souris ou un rat), et le procédé comprend en outre le remplacement de la région constante non humaine de la chaîne lourde ou de l'anticorps isolé par une région constante humaine.

18. Procédé selon la revendication 16, comprenant en outre l'échange de la séquence de nucléotides de la région constante de chaîne lourde de l'anticorps par une séquence de nucléotides codant pour une région constante de chaîne lourde humaine ou humanisée, et éventuellement la maturation par affinité de la région variable dudit anticorps.

19. Procédé selon la revendication 16 ou la revendication 18, comprenant en outre l'insertion dudit acide nucléique dans un vecteur d'expression et éventuellement dans une cellule hôte.

20. Procédé selon la revendication 19, comprenant l'expression de la chaîne lourde, du domaine VH ou de l'anticorps à partir de la cellule hôte et la fourniture d'une chaîne lourde, d'un domaine VH ou d'un anticorps isolé.

21. Utilisation de JH6*02 humain pour générer une HCDR3 d'au moins 20 acides aminés dans un vertébré non humain dont le génome comprend un emplacement de chaîne lourde d'immunoglobuline comprenant un segment de gène humain JH6*02, un ou plusieurs segments de gène VH humains et un ou plusieurs segments de gène D humains en amont d'une région constante ; dans laquelle les segments de gène dans l'emplacement de chaîne lourde sont liés de façon opérationnelle à la région constante de ceux-ci de sorte que le vertébré soit capable de produire une chaîne lourde d'anticorps produite par recombinaison du JH6*02 humain avec un segment D humain et un segment VH humain et dans laquelle le segment de gène de JH6*02 comprend la séquence de nucléotides suivante : ATTACTA CTACTACTAC GGTATGGACG TCTGGGGCCA AGGGACCACG GTCACCGTCT CCTCAG.

22. Utilisation selon la revendication 21, dans laquelle la chaîne lourde de l'anticorps se lie spécifiquement à un antigène cible.

23. Utilisation selon la revendication 21, dans laquelle le vertébré est immunisé avec un antigène cible.

24. Utilisation selon la revendication 21, dans laquelle le vertébré comprend un Eµ et un Sµ de souris dans l'emplacement de chaîne lourde entre le JH6*02 et la région constante.

25. Utilisation selon la revendication 21, dans laquelle l'Eµ et le Sµ sont un Eµ et un Sµ d'un génome 129-dérivé de souris ou d'un génome C57BL/6-dérivé de souris.
